# EUROPEAN PATENT SPECIFICATION

(11) **EP 3 179 992 B1**
(45) Date of publication and mention of the grant of the patent: **11.05.2022**
(21) Application number: 15762756.3
(22) Date of filing: 11.08.2015
(51) Int. Cl.: A61K 39/395, A61K 31/00, A61K 31/4155, A61K 31/416, A61K 31/454, A61K 31/519, A61K 45/06

(54) **THERAPEUTIC COMBINATIONS OF A BTK INHIBITOR, A PD-1 INHIBITOR AND/OR A PD-L1 INHIBITOR**
THERAPEUTISCHE KOMBINATIONEN EINES BTK-INHIBITORS, EINES PD-1-INHIBITORS UND/ODER EINES PD-L1-INHIBITORS
COMBINAISONS THÉRAPEUTIQUES ASSOCIANT UN INHIBITEUR DE BTK, UN INHIBITEUR DE PD-1 ET/OU UN INHIBITEUR DE PD-L1

(30) Priority: 11.08.2014 US 201462035812 P; 05.12.2014 US 201462088357 P; 12.02.2015 US 201562115489 P; 17.06.2015 US 201562181164 P
(43) Date of publication of application: 21.06.2017
(73) Proprietor: Acerta Pharma B.V., 5349 AB Oss (NL)
(72) Inventor: HAMDY, Ahmed, Santa Cruz, CA 95060 (US); ROTHBAUM, Wayne, New York, NY 10023 (US); IZUMI, Raquel, San Carlos, CA 94070 (US); LANNUTTI, Brian, Solana Beach, CA 92075 (US); COVEY, Todd, San Carlos, CA 94070 (US); ULRICH, Roger, Sammamish,WA 98075 (US); JOHNSON, Dave, Aptos, CA 95033 (US); BARF, Tjeerd, 5371 AS Ravenstein (NL); KAPTEIN, Allard, 5301 HB Zaltbommel (NL)
(74) Representative: D Young & Co LLP
(86) International application number: PCT/IB2015/056123
(87) International publication number: WO 2016/024228

(56) References cited:
- WO-A1-2013/010868
- WO-A1-2015/061752
- GRISAFI DAVIDE ET AL: "Ibrutinib: from bench side to clinical implications", MEDICAL ONCOLOGY, SCIENCE AND TECHNOLOGY LETTERS, NORTHWOOD, GB, vol. 32, no. 9, 30 July 2015 (2015-07-30), pages 1-10, XP035522374, ISSN: 1357-0560, DOI: 10.1007/S12032-015-0669-9 [retrieved on 2015-07-30]
- G. K. PHILIPS ET AL: "Therapeutic uses of anti-PD-1 and anti-PD-L1 antibodies", INTERNATIONAL IMMUNOLOGY., vol. 27, no. 1, 16 October 2014 (2014-10-16), pages 39-46, XP055217958, GB ISSN: 0953-8178, DOI: 10.1093/intimm/dxu095
- IDIT SAGIV-BARFI ET AL: "Therapeutic antitumor immunity by checkpoint blockade is enhanced by ibrutinib, an inhibitor of both BTK and ITK", PROCEEDINGS OF THE NATIONAL ACADEMY OF SCIENCES, vol. 112, no. 9, 17 February 2015 (2015-02-17), pages E966-E972, XP055217955, US ISSN: 0027-8424, DOI: 10.1073/pnas.1500712112
- E KLYUCHNIKOV ET AL: "Allogeneic hematopoietic cell transplantation for diffuse large B cell lymphoma: who, when and how?", BONE MARROW TRANSPLANTATION, vol. 49, no. 1, 27 May 2013 (2013-05-27), pages 1-7, XP055217963, GB ISSN: 0268-3369, DOI: 10.1038/bmt.2013.72
- SONGDEJ NATTHAPOL ET AL: "GIST Treatment Options after Tyrosine Kinase Inhibitors", CURRENT TREATMENT OPTIONS IN ONCOLOGY MAY 2005, SPRINGER US, BOSTON, vol. 15, no. 3, 22 June 2014 (2014-06-22), pages 493-506, XP035381545, ISSN: 1527-2729, DOI: 10.1007/S11864-014-0295-3 [retrieved on 2014-06-22]
- JENNIFER L. YORI ET AL: "Abstract LB-221: Inhibition of rapamycin-induced feedback activation of AKT with dasatinib induces complete tumor regression in a preclinical model of breast cancer.", CANCER RESEARCH, vol. 73, no. 8, Suppl. 1, 15 April 2013 (2013-04-15), - 10 April 2013 (2013-04-10), pages LB-221, XP055217951, US ISSN: 0008-5472, DOI: 10.1158/1538-7445.AM2013-LB-221
- BRIAN J. PARK ET AL: "Dasatinib synergizes with both cytotoxic and signal transduction inhibitors in heterogeneous breast cancer cell lines - Lessons for design of combination targeted therapy", CANCER LETTERS, vol. 320, no. 1, 2 February 2012 (2012-02-02), pages 104-110, XP055217954, US ISSN: 0304-3835, DOI: 10.1016/j.canlet.2012.01.039
- D'CRUZ OJ ET AL: "Novel Bruton's tyrosine kinase inhibitors currently in development", ONCOTARGETS AND THERAPY, vol. 6, 6 March 2013 (2013-03-06), pages 161-176, XP055217561, GB ISSN: 1178-6930, DOI: 10.2147/OTT.S33732
- VAN DEN AKKER EMILE ET AL: "The Btk inhibitor LFM-A13 is a potent inhibitor of Jak2 kinase activity", BIOLOGICAL CHEMISTRY, vol. 385, no. 5, May 2004 (2004-05), pages 409-413, XP008177694, ISSN: 1431-6730
- GIROTTI MARIA ROMINA ET AL: "No longer an untreatable disease: How targeted and immunotherapies have changed the management of melanoma patients", MOLECULAR ONCOLOGY, ELSEVIER, AMSTERDAM, NL, vol. 8, no. 6, 15 August 2014 (2014-08-15) , pages 1140-1158, XP029054008, ISSN: 1574-7891, DOI: 10.1016/J.MOLONC.2014.07.027
- HANTSCHEL O: "Targeting BCR-ABL and JAK2 in Ph+ ALL", BLOOD 20150226 AMERICAN SOCIETY OF HEMATOLOGY USA, vol. 125, no. 9, 26 February 2015 (2015-02-26), pages 1362-1363, XP008177695, ISSN: 0006-4971
- QINGJIE LIU ET AL: "Design and synthesis of carbazole carboxamides as promising inhibitors of Bruton's tyrosine kinase (BTK) and Janus kinase 2 (JAK2)", BIOORGANIC & MEDICINAL CHEMISTRY LETTERS, vol. 25, no. 19, 6 August 2015 (2015-08-06), pages 4265-4269, XP055218382, AMSTERDAM, NL ISSN: 0960-894X, DOI: 10.1016/j.bmcl.2015.07.102
- STEPHENS DEBORAH M ET AL: "Changing The Treatment Paradigm For Previously Treated Chronic Lymphocytic Leukemia Patients With Del(17p) Karyotype", BLOOD, vol. 122, no. 21, November 2013 (2013-11), page 2872, XP008177696, & 55TH ANNUAL MEETING OF THE AMERICAN-SOCIETY-OF-HEMATOLOGY; NEW ORLEANS, LA, USA; DECEMBER 07 -10, 2013
- D. CHIRON ET AL: "Cell-Cycle Reprogramming for PI3K Inhibition Overrides a Relapse-Specific C481S BTK Mutation Revealed by Longitudinal Functional Genomics in Mantle Cell Lymphoma", CANCER DISCOVERY, vol. 4, no. 9, 31 July 2014 (2014-07-31), pages 1022-1035, XP055218352, US ISSN: 2159-8274, DOI: 10.1158/2159-8290.CD-14-0098
- J. SCHWAMB ET AL: "B-cell receptor triggers drug sensitivity of primary CLL cells by controlling glucosylation of ceramides", BLOOD, vol. 120, no. 19, 8 November 2012 (2012-11-08), pages 3978-3985, XP055218383, US ISSN: 0006-4971, DOI: 10.1182/blood-2012-05-431783
- BIN CHEN ET AL: "Rapamycin Enhances the Anti-Cancer Effect of Dasatinib by Suppressing Src/PI3K/mTOR Pathway in NSCLC Cells", PLOS ONE, vol. 10, no. 6, 10 June 2015 (2015-06-10), page e0129663, XP055218386, US ISSN: 1932-6203, DOI: 10.1371/journal.pone.0129663
- L. A. MATHEWS GRINER ET AL: "High-throughput combinatorial screening identifies drugs that cooperate with ibrutinib to kill activated B-cell-like diffuse large B-cell lymphoma cells", PROCEEDINGS OF THE NATIONAL ACADEMY OF SCIENCES, vol. 111, no. 6, 27 January 2014 (2014-01-27), pages 2349-2354, XP055218384, US ISSN: 0027-8424, DOI: 10.1073/pnas.1311846111
- ZHANG QING ET AL: "[Effect of PI3K[delta] inhibitor CAL-101 on myeloma cell lines and preliminary study of synergistic effects with other new drugs].", ZHONGHUA XUE YE XUE ZA ZHI = ZHONGHUA XUEYEXUE ZAZHI OCT 2014, vol. 35, no. 10, October 2014 (2014-10), pages 926-930, XP008177739, ISSN: 0253-2727
- HONG D S ET AL: "A multicenter study of the Bruton's tyrosine kinase (BTK) inhibitor ibrutinib plus durvalumab in patients with relapsed/refractory (R/R) solid tumors", JOURNAL OF CLINICAL ONCOLOGY, LIPPINCOTT WILLIAMS & WILKINS, USA, vol. 36, no. 15, Supplement 1, 1 May 2018 (2018-05-01), page 2578, XP009511915, ISSN: 1527-7755, DOI: 10.1200/JCO.2018.36.15_SUPPL.2578

### SEQUENCE LISTING

<110> Acerta Pharma B.V.
<120> THERAPEUTIC COMBINATIONS OF A BTK INHIBITOR, A PI3K INHIBITOR, A JAK-2 INHIBITOR, A PD-1 INHIBITOR, AND/OR A PD-L1 INHIBITOR
<130> 055112-5014-WO
<140> PCT/IB2015/000000 <141> 2015-08-11
<150> 62/035,812 <151> 2014-08-11
<150> 62/088,357 <151> 2014-12-05
<150> 62/115,489 <151> 2015-02-12
<150> 62/181,164 <151> 2015-06-17
<160> 97
<170> PatentIn version 3.5
<210> 1
   <211> 440
   <212> PRT
   <213> Artificial sequence
<220>
   <223> Heavy Chain Amino Acid Sequence, Anti-PD-1 mAb (nivolumab, BMS936558; also 5C4 in WO 2006/121168) (SEQ ID NO:17 IN WO 2014/055648A1)
<400> 1
   Gln Val Gln Leu Val Glu Ser Gly Gly Gly Val Val Gln Pro Gly Arg
   1               5                   10                  15
   Ser Leu Arg Leu Asp Cys Lys Ala Ser Gly Ile Thr Phe Ser Asn Ser
               20                  25                  30
   Gly Met His Trp Val Arg Gln Ala Pro Gly Lys Gly Leu Glu Trp Val
           35                  40                  45
   Ala Val Ile Trp Tyr Asp Gly Ser Lys Arg Tyr Tyr Ala Asp Ser Val
       50                  55                  60
   Lys Gly Arg Phe Thr Ile Ser Arg Asp Asn Ser Lys Asn Thr Leu Phe
   65                  70                  75                  80
   Leu Gln Met Asn Ser Leu Arg Ala Glu Asp Thr Ala Val Tyr Tyr Cys
                   85                  90                  95
   Ala Thr Asn Asp Asp Tyr Trp Gly Gln Gly Thr Leu Val Thr Val Ser
   100 105 110
   Ser Ala Ser Thr Lys Gly Pro Ser Val Phe Pro Leu Ala Pro Cys Ser
   115 120 125
   Arg Ser Thr Ser Glu Ser Thr Ala Ala Leu Gly Cys Leu Val Lys Asp
   130 135 140
   Tyr Phe Pro Glu Pro Val Thr Val Ser Trp Asn Ser Gly Ala Leu Thr
   145 150 155 160
   Ser Gly Val His Thr Phe Pro Ala Val Leu Gln Ser Ser Gly Leu Tyr
   165 170 175
   Ser Leu Ser Ser Val Val Thr Val Pro Ser Ser Ser Leu Gly Thr Lys
   180 185 190
   Thr Tyr Thr Cys Asn Val Asp His Lys Pro Ser Asn Thr Lys Val Asp
   195 200 205
   Lys Arg Val Glu Ser Lys Tyr Gly Pro Pro Cys Pro Pro Cys Pro Ala
   210 215 220
   Pro Glu Phe Leu Gly Gly Pro Ser Val Phe Leu Phe Pro Pro Lys Pro
   225 230 235 240
   Lys Asp Thr Leu Met Ile Ser Arg Thr Pro Glu Val Thr Cys Val Val
   245 250 255
   Val Asp Val Ser Gln Glu Asp Pro Glu Val Gln Phe Asn Trp Tyr Val
   260 265 270
   Asp Gly Val Glu Val His Asn Ala Lys Thr Lys Pro Arg Glu Glu Gln
   275 280 285
   Phe Asn Ser Thr Tyr Arg Val Val Ser Val Leu Thr Val Leu His Gln
   290 295 300
   Asp Trp Leu Asn Gly Lys Glu Tyr Lys Cys Lys Val Ser Asn Lys Gly
   305 310 315 320
   Leu Pro Ser Ser Ile Glu Lys Thr Ile Ser Lys Ala Lys Gly Gln Pro
   325 330 335
   Arg Glu Pro Gln Val Tyr Thr Leu Pro Pro Ser Gln Glu Glu Met Thr
   340 345 350
   Lys Asn Gln Val Ser Leu Thr Cys Leu Val Lys Gly Phe Tyr Pro Ser
   355 360 365
   Asp Ile Ala Val Glu Trp Glu Ser Asn Gly Gln Pro Glu Asn Asn Tyr
   370 375 380
   Lys Thr Thr Pro Pro Val Leu Asp Ser Asp Gly Ser Phe Phe Leu Tyr
   385 390 395 400
   Ser Arg Leu Thr Val Asp Lys Ser Arg Trp Gln Glu Gly Asn Val Phe
   405 410 415
   Ser Cys Ser Val Met His Glu Ala Leu His Asn His Tyr Thr Gln Lys
   420 425 430
   Ser Leu Ser Leu Ser Leu Gly Lys
   435 440
<210> 2
   <211> 214
   <212> PRT
   <213> Artificial sequence
<220>
   <223> Light Chain Amino Acid Sequence, Anti-PD-1 mAb (nivolumab, BMS936558; 5C4 in WO 2006/121168) (SEQ ID NO:18 IN WO 2014/055648 A1)
<400> 2
   Glu Ile Val Leu Thr Gln Ser Pro Ala Thr Leu Ser Leu Ser Pro Gly
   1 5 10 15
   Glu Arg Ala Thr Leu Ser Cys Arg Ala Ser Gln Ser Val Ser Ser Tyr
   20 25 30
   Leu Ala Trp Tyr Gln Gln Lys Pro Gly Gln Ala Pro Arg Leu Leu Ile
   35 40 45
   Tyr Asp Ala Ser Asn Arg Ala Thr Gly Ile Pro Ala Arg Phe Ser Gly
   50 55 60
   Ser Gly Ser Gly Thr Asp Phe Thr Leu Thr Ile Ser Ser Leu Glu Pro
   65 70 75 80
   Glu Asp Phe Ala Val Tyr Tyr Cys Gln Gln Ser Ser Asn Trp Pro Arg
   85 90 95
   Thr Phe Gly Gln Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 3
   <211> 113
   <212> PRT
   <213> Artificial sequence
<220>
   <223> Heavy Chain Variable Region (VH) Amino Acid Sequence Anti-PD-1 mAb (nivolumab, BMS936558; 5C4 in WO 2006/121168) (SEQ ID NO:4 from WO 2006/121168) (SEQ ID NO:19 IN WO 2014/055648 A1)
<400> 3
<210> 4
   <211> 107
   <212> PRT
   <213> Artificial sequence
<220>
   <223> Light Chain Variable Region (VL) Amino Acid Sequence Anti-PD-1 mAb (nivolumab, BMS936558; 5C4 in WO 2006/121168) (SEQ ID NO:11 from WO 2006/121168) (SEQ ID NO:21 IN WO 2014/055648 A1)
<400> 4
<210> 5
   <211> 5
   <212> PRT
   <213> Artificial sequence
<220>
   <223> Heavy Chain CDR1 Amino Acid Sequence Anti-PD-1 mAb (nivolumab, BMS936558; 5C4 in WO 2006/121168) (SEQ ID NO:18 from WO 2006/121168) (SEQ ID NO:23 from WO 2014/055648 A1))
<400> 5
   Asn Ser Gly Met His
   1 5
<210> 6
   <211> 17
   <212> PRT
   <213> Artificial sequence
<220>
   <223> Heavy Chain CDR2 Amino Acid Sequence Anti-PD-1 mAb (nivolumab, BMS936558; 5C4 in WO 2006/121168) (SEQ ID NO:25 from WO 2006/121168) (SEQ ID NO:24 from WO 2014/055648 A1)
<400> 6
   Val Ile Trp Tyr Asp Gly Ser Lys Arg Tyr Tyr Ala Asp Ser Val Lys
   1 5 10 15
   Gly
<210> 7
   <211> 4
   <212> PRT
   <213> Artificial sequence
<220>
   <223> Heavy Chain CDR3 Amino Acid Sequence Anti-PD-1 mAb (nivolumab, BMS936558; 5C4 in WO 2006/121168) (SEQ ID NO:32 from WO 2006/121168) (SEQ ID NO:25 from WO 2014/055648 A1)
<400> 7
   Asn Asp Asp Tyr
   1
<210> 8
   <211> 11
   <212> PRT
   <213> Artificial sequence
<220>
   <223> Light Chain CDR1 Amino Acid Sequence Anti-PD-1 mAb (nivolumab, BMS936558; 5C4 in WO 2006/121168) (SEQ ID NO:39 from WO 2006/121168) (SEQ ID NO:26 from WO 2014/055648 A1)
<400> 8
   Arg Ala Ser Gln Ser Val Ser Ser Tyr Leu Ala
   1 5 10
<210> 9
   <211> 7
   <212> PRT
   <213> Artificial sequence
<220>
   <223> Light Chain CDR2 Amino Acid Sequence Anti-PD-1 mAb (nivolumab, BMS936558; 5C4 in WO 2006/121168) (SEQ ID NO:46 from WO 2006/121168) (SEQ ID NO:27 from WO 2014/055648 A1)
<400> 9
   Asp Ala Ser Asn Arg Ala Thr
   1 5
<210> 10
   <211> 9
   <212> PRT
   <213> Artificial sequence
<220>
   <223> Light Chain CDR3 Amino Acid Sequence Anti-PD-1 mAb (nivolumab, BMS936558; 5C4 in WO 2006/121168) (SEQ ID NO:53 from WO 2006/121168) (SEQ ID NO:28 from WO 2014/055648 A1)
<400> 10
   Gln Gln Ser Ser Asn Trp Pro Arg Thr
   1 5
<210> 11
   <211> 466
   <212> PRT
   <213> Artificial sequence
<220>
   <223> pembrolizumab 409A-H heavy chain full length (SEQ ID NO:31 from US Patent No. 8,354,509 B2)
<400> 11
   Met Ala Val Leu Gly Leu Leu Phe Cys Leu Val Thr Phe Pro Ser Cys
   1 5 10 15
   Val Leu Ser Gln Val Gln Leu Val Gln Ser Gly Val Glu Val Lys Lys
   20 25 30
   Pro Gly Ala Ser Val Lys Val Ser Cys Lys Ala Ser Gly Tyr Thr Phe
   35 40 45
   Thr Asn Tyr Tyr Met Tyr Trp Val Arg Gln Ala Pro Gly Gln Gly Leu
   50 55 60
   Glu Trp Met Gly Gly Ile Asn Pro Ser Asn Gly Gly Thr Asn Phe Asn
   65 70 75 80
   Glu Lys Phe Lys Asn Arg Val Thr Leu Thr Thr Asp Ser Ser Thr Thr
   85 90 95
   Thr Ala Tyr Met Glu Leu Lys Ser Leu Gln Phe Asp Asp Thr Ala Val
   100 105 110
   Tyr Tyr Cys Ala Arg Arg Asp Tyr Arg Phe Asp Met Gly Phe Asp Tyr
           115                 120                 125
   Trp Gly Gln Gly Thr Thr Val Thr Val Ser Ser Ala Ser Thr Lys Gly
       130                 135                 140
   Pro Ser Val Phe Pro Leu Ala Pro Cys Ser Arg Ser Thr Ser Glu Ser
   145                 150                 155                 160
   Thr Ala Ala Leu Gly Cys Leu Val Lys Asp Tyr Phe Pro Glu Pro Val
                   165                 170                 175
   Thr Val Ser Trp Asn Ser Gly Ala Leu Thr Ser Gly Val His Thr Phe
               180                 185                 190
   Pro Ala Val Leu Gln Ser Ser Gly Leu Tyr Ser Leu Ser Ser Val Val
           195                 200                 205
   Thr Val Pro Ser Ser Ser Leu Gly Thr Lys Thr Tyr Thr Cys Asn Val
       210                 215                 220
   Asp His Lys Pro Ser Asn Thr Lys Val Asp Lys Arg Val Glu Ser Lys
   225                 230                 235                 240
   Tyr Gly Pro Pro Cys Pro Pro Cys Pro Ala Pro Glu Phe Leu Gly Gly
                   245                 250                 255
   Pro Ser Val Phe Leu Phe Pro Pro Lys Pro Lys Asp Thr Leu Met Ile
               260                 265                 270
   Ser Arg Thr Pro Glu Val Thr Cys Val Val Val Asp Val Ser Gln Glu
           275                 280                 285
   Asp Pro Glu Val Gln Phe Asn Trp Tyr Val Asp Gly Val Glu Val His
       290                 295                 300
   Asn Ala Lys Thr Lys Pro Arg Glu Glu Gln Phe Asn Ser Thr Tyr Arg
   305                 310                 315                 320
   Val Val Ser Val Leu Thr Val Leu His Gln Asp Trp Leu Asn Gly Lys
                   325                 330                 335
   Glu Tyr Lys Cys Lys Val Ser Asn Lys Gly Leu Pro Ser Ser Ile Glu
               340                 345                 350
   Lys Thr Ile Ser Lys Ala Lys Gly Gln Pro Arg Glu Pro Gln Val Tyr
   355 360 365
   Thr Leu Pro Pro Ser Gln Glu Glu Met Thr Lys Asn Gln Val Ser Leu
   370 375 380
   Thr Cys Leu Val Lys Gly Phe Tyr Pro Ser Asp Ile Ala Val Glu Trp
   385 390 395 400
   Glu Ser Asn Gly Gln Pro Glu Asn Asn Tyr Lys Thr Thr Pro Pro Val
   405 410 415
   Leu Asp Ser Asp Gly Ser Phe Phe Leu Tyr Ser Arg Leu Thr Val Asp
   420 425 430
   Lys Ser Arg Trp Gln Glu Gly Asn Val Phe Ser Cys Ser Val Met His
   435 440 445
   Glu Ala Leu His Asn His Tyr Thr Gln Lys Ser Leu Ser Leu Ser Leu
   450 455 460
   Gly Lys
   465
<210> 12
   <211> 447
   <212> PRT
   <213> Artificial sequence
<220>
   <223> pembrolizumab heavy chain
<400> 12
   Gln Val Gln Leu Val Gln Ser Gly Val Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Tyr Met Tyr Trp Val Arg Gln Ala Pro Gly Gln Gly Leu Glu Trp Met
           35                  40                  45
   Gly Gly Ile Asn Pro Ser Asn Gly Gly Thr Asn Phe Asn Glu Lys Phe
       50                  55                  60
   Lys Asn Arg Val Thr Leu Thr Thr Asp Ser Ser Thr Thr Thr Ala Tyr
   65                  70                  75                  80
   Met Glu Leu Lys Ser Leu Gln Phe Asp Asp Thr Ala Val Tyr Tyr Cys
                   85                  90                  95
   Ala Arg Arg Asp Tyr Arg Phe Asp Met Gly Phe Asp Tyr Trp Gly Gln
               100                 105                 110
   Gly Thr Thr Val Thr Val Ser Ser Ala Ser Thr Lys Gly Pro Ser Val
           115                 120                 125
   Phe Pro Leu Ala Pro Cys Ser Arg Ser Thr Ser Glu Ser Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Lys Thr Tyr Thr Cys Asn Val Asp His Lys
           195                 200                 205
   Pro Ser Asn Thr Lys Val Asp Lys Arg Val Glu Ser Lys Tyr Gly Pro
       210                 215                 220
   Pro Cys Pro Pro Cys Pro Ala Pro Glu Phe Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser Gln Glu Asp Pro Glu
               260                 265                 270
   Val Gln Phe Asn Trp Tyr Val Asp Gly Val Glu Val His Asn Ala Lys
           275                 280                 285
   Thr Lys Pro Arg Glu Glu Gln Phe Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Gly Leu Pro Ser Ser Ile Glu Lys Thr Ile
                   325                 330                 335
   Ser Lys Ala Lys Gly Gln Pro Arg Glu Pro Gln Val Tyr Thr Leu Pro
   340 345 350
   Pro Ser Gln Glu Glu Met Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Arg Leu Thr Val Asp Lys Ser Arg
   405 410 415
   Trp Gln Glu Gly Asn Val Phe Ser Cys Ser Val Met His Glu Ala Leu
   420 425 430
   His Asn His Tyr Thr Gln Lys Ser Leu Ser Leu Ser Leu Gly Lys
   435 440 445
<210> 13
   <211> 130
   <212> PRT
   <213> Artificial sequence
<220>
   <223> pembrolizumab K09A-L-11 light chain variable region (SEQ ID NO:32 from US Patent No. 8,354,509 B2)
<400> 13
   Met Ala Pro Val Gln Leu Leu Gly Leu Leu Val Leu Phe Leu Pro Ala
   1               5                   10                  15
   Met Arg Cys Glu Ile Val Leu Thr Gln Ser Pro Ala Thr Leu Ser Leu
               20                  25                  30
   Ser Pro Gly Glu Arg Ala Thr Leu Ser Cys Arg Ala Ser Lys Gly Val
           35                  40                  45
   Ser Thr Ser Gly Tyr Ser Tyr Leu His Trp Tyr Gln Gln Lys Pro Gly
       50                  55                  60
   Gln Ala Pro Arg Leu Leu Ile Tyr Leu Ala Ser Tyr Leu Glu Ser Gly
   65                  70                  75                  80
   Val Pro Ala Arg Phe Ser Gly Ser Gly Ser Gly Thr Asp Phe Thr Leu
                   85                  90                  95
   Thr Ile Ser Ser Leu Glu Pro Glu Asp Phe Ala Val Tyr Tyr Cys Gln
               100                 105                 110
   His Ser Arg Asp Leu Pro Leu Thr Phe Gly Gly Gly Thr Lys Val Glu
           115                 120                 125
   Ile Lys
       130
<210> 14
   <211> 237
   <212> PRT
   <213> Artificial sequence
<220>
   <223> pembrolizumab K09A-L-11 light chain (SEQ ID NO:36 from US Patent No. 8,354,509 B2)
<400> 14
   Met Ala Pro Val Gln Leu Leu Gly Leu Leu Val Leu Phe Leu Pro Ala
   1               5                   10                  15
   Met Arg Cys Glu Ile Val Leu Thr Gln Ser Pro Ala Thr Leu Ser Leu
               20                  25                  30
   Ser Pro Gly Glu Arg Ala Thr Leu Ser Cys Arg Ala Ser Lys Gly Val
           35                  40                  45
   Ser Thr Ser Gly Tyr Ser Tyr Leu His Trp Tyr Gln Gln Lys Pro Gly
       50                  55                  60
   Gln Ala Pro Arg Leu Leu Ile Tyr Leu Ala Ser Tyr Leu Glu Ser Gly
   65                  70                  75                  80
   Val Pro Ala Arg Phe Ser Gly Ser Gly Ser Gly Thr Asp Phe Thr Leu
                   85                  90                  95
   Thr Ile Ser Ser Leu Glu Pro Glu Asp Phe Ala Val Tyr Tyr Cys Gln
               100                 105                 110
   His Ser Arg Asp Leu Pro Leu Thr Phe Gly Gly Gly Thr Lys Val Glu
           115                 120                 125
   Ile Lys Arg Thr Val Ala Ala Pro Ser Val Phe Ile Phe Pro Pro Ser
       130                 135                 140
   Asp Glu Gln Leu Lys Ser Gly Thr Ala Ser Val Val Cys Leu Leu Asn
   145                 150                 155                 160
   Asn Phe Tyr Pro Arg Glu Ala Lys Val Gln Trp Lys Val Asp Asn Ala
                   165                 170                 175
   Leu Gln Ser Gly Asn Ser Gln Glu Ser Val Thr Glu Gln Asp Ser Lys
               180                 185                 190
   Asp Ser Thr Tyr Ser Leu Ser Ser Thr Leu Thr Leu Ser Lys Ala Asp
           195                 200                 205
   Tyr Glu Lys His Lys Val Tyr Ala Cys Glu Val Thr His Gln Gly Leu
       210                 215                 220
   Ser Ser Pro Val Thr Lys Ser Phe Asn Arg Gly Glu Cys
   225                 230                 235
<210> 15
   <211> 15
   <212> PRT
   <213> Artificial sequence
<220>
   <223> pembrolizumab hPD-1.09A light chain CDR1 (SEQ ID NO:15 from US Patent No. 8,354,509 B2)
<400> 15
   Arg Ala Ser Lys Gly Val Ser Thr Ser Gly Tyr Ser Tyr Leu His
   1 5 10 15
<210> 16
   <211> 7
   <212> PRT
   <213> Artificial sequence
<220>
   <223> pembrolizumab hPD-1.09A light chain CDR2 (SEQ ID NO:16 from US Patent No. 8,354,509 B2)
<400> 16
   Leu Ala Ser Tyr Leu Glu Ser
   1 5
<210> 17
   <211> 9
   <212> PRT
   <213> Artificial sequence
<220>
   <223> pembrolizumab hPD-1.09A light chain CDR3 (SEQ ID NO:17 from US Patent No. 8,354,509 B2)
<400> 17
   Gln His Ser Arg Asp Leu Pro Leu Thr
   1 5
<210> 18
   <211> 5
   <212> PRT
   <213> Artificial sequence
<220>
   <223> pembrolizumab hPD-1.09A heavy chain CDR1 (SEQ ID NO:18 from US Patent No. 8,354,509 B2)
<400> 18
   Asn Tyr Tyr Met Tyr
   1 5
<210> 19
   <211> 16
   <212> PRT
   <213> Artificial sequence
<220>
   <223> pembrolizumab hPD-1.09A heavy chain CDR2 (SEQ ID NO:19 from US Patent No. 8,354,509 B2)
<400> 19
   Gly Ile Asn Pro Ser Asn Gly Gly Thr Asn Phe Asn Glu Lys Phe Lys
   1               5                   10                  15
<210> 20
   <211> 11
   <212> PRT
   <213> Artificial sequence
<220>
   <223> pembrolizumab hPD-1.09A heavy chain CDR3 (SEQ ID NO:20 from US Patent No. 8,354,509 B2)
<400> 20
   Arg Asp Tyr Arg Phe Asp Met Gly Phe Asp Tyr
   1               5                   10
<210> 21
   <211> 447
   <212> PRT
   <213> Artificial sequence
<220>
   <223> pidilizumab heavy chain
<400> 21
   Gln Val Gln Leu Val Gln Ser Gly Ser Glu Leu Lys Lys Pro Gly Ala
   1               5                   10                  15
   Ser Val Lys Ile Ser Cys Lys Ala Ser Gly Tyr Thr Phe Thr Asn Tyr
               20                  25                  30
   Gly Met Asn Trp Val Arg Gln Ala Pro Gly Gln Gly Leu Gln Trp Met
           35                  40                  45
   Gly Trp Ile Asn Thr Asp Ser Gly Glu Ser Thr Tyr Ala Glu Glu Phe
       50                  55                  60
   Lys Gly Arg Phe Val Phe Ser Leu Asp Thr Ser Val Asn Thr Ala Tyr
   65                  70                  75                  80
   Leu Gln Ile Thr Ser Leu Thr Ala Glu Asp Thr Gly Met Tyr Phe Cys
                   85                  90                  95
   Val Arg Val Gly Tyr Asp Ala Leu Asp Tyr Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Arg Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Ala Leu Pro Ala Pro Ile Glu Lys Thr Ile
   325 330 335
   Ser Lys Ala Lys Gly Gln Pro Arg Glu Pro Gln Val Tyr Thr Leu Pro
   340 345 350
   Pro Ser Arg Glu Glu Met Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 22
   <211> 213
   <212> PRT
   <213> Artificial sequence
<220>
   <223> pidilizumab light chain
<400> 22
   Glu Ile Val Leu Thr Gln Ser Pro Ser Ser Leu Ser Ala Ser Val Gly
   1 5 10 15
   Asp Arg Val Thr Ile Thr Cys Ser Ala Arg Ser Ser Val Ser Tyr Met
   20 25 30
   His Trp Phe Gln Gln Lys Pro Gly Lys Ala Pro Lys Leu Trp Ile Tyr
   35 40 45
   Arg Thr Ser Asn Leu Ala Ser Gly Val Pro Ser Arg Phe Ser Gly Ser
       50                  55                  60
   Gly Ser Gly Thr Ser Tyr Cys Leu Thr Ile Asn Ser Leu Gln Pro Glu
   65                  70                  75                  80
   Asp Phe Ala Thr Tyr Tyr Cys Gln Gln Arg Ser Ser Phe Pro Leu Thr
                   85                  90                  95
   Phe Gly Gly Gly Thr Lys Leu Glu Ile Lys Arg Thr Val Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn Arg Gly Glu Cys
       210
<210> 23
   <211> 117
   <212> PRT
   <213> Artificial sequence
<220>
   <223> variable heavy chain region of the PD-1 inhibitor pidilizumab (corresponding to SEQ ID NO:5 in U.S. Patent No. 8,686,119 B2)
<400> 23
   Gln Val Gln Leu Val Gln Ser Gly Ser Glu Leu Lys Lys Pro Gly Ala
   1               5                   10                  15
   Ser Val Lys Ile Ser Cys Lys Ala Ser Gly Tyr Thr Phe Thr Asn Tyr
               20                  25                  30
   Gly Met Asn Trp Val Arg Gln Ala Pro Gly Gln Gly Leu Gln Trp Met
   35 40 45
   Gly Trp Ile Asn Thr Asp Ser Gly Glu Ser Thr Tyr Ala Glu Glu Phe
   50 55 60
   Lys Gly Arg Phe Val Phe Ser Leu Asp Thr Ser Val Asn Thr Ala Tyr
   65 70 75 80
   Leu Gln Ile Thr Ser Leu Thr Ala Glu Asp Thr Gly Met Tyr Phe Cys
   85 90 95
   Val Arg Val Gly Tyr Asp Ala Leu Asp Tyr Trp Gly Gln Gly Thr Leu
   100 105 110
   Val Thr Val Ser Ser
   115
<210> 24
   <211> 106
   <212> PRT
   <213> Artificial sequence
<220>
   <223> variable light chain region of the PD-1 inhibitor pidilizumab (corresponding to SEQ ID NO:5 in U.S. Patent No. 8,686,119 B2)
<400> 24
   Glu Ile Val Leu Thr Gln Ser Pro Ser Ser Leu Ser Ala Ser Val Gly
   1 5 10 15
   Asp Arg Val Thr Ile Thr Cys Ser Ala Arg Ser Ser Val Ser Tyr Met
   20 25 30
   His Trp Phe Gln Gln Lys Pro Gly Lys Ala Pro Lys Leu Trp Ile Tyr
   35 40 45
   Arg Thr Ser Asn Leu Ala Ser Gly Val Pro Ser Arg Phe Ser Gly Ser
   50 55 60
   Gly Ser Gly Thr Ser Tyr Cys Leu Thr Ile Asn Ser Leu Gln Pro Glu
   65 70 75 80
   Asp Phe Ala Thr Tyr Tyr Cys Gln Gln Arg Ser Ser Phe Pro Leu Thr
   85 90 95
   Phe Gly Gly Gly Thr Lys Leu Glu Ile Lys
   100 105
<210> 25
   <211> 7
   <212> PRT
   <213> Homo sapiens
<220>
   <221> PEPTIDE
   <222> (1)..(7)
   <223> BC loop
<400> 25
   Ser Asn Thr Ser Glu Ser Phe
   1               5
<210> 26
   <211> 22
   <212> PRT
   <213> Artificial Sequence
<220>
   <223> Branched peptide at LYS8 with SEQ ID NO:25
<220>
   <221> SITE
   <222> (1)..(22)
   <223> Branched amino acid sequence, SEQ ID NO:25 attached to the Lys8 residue
<400> 26
   Ser Asn Thr Ser Glu Ser Phe Lys Phe Arg Val Thr Gln Leu Ala Pro
   1               5                   10                  15
   Lys Ala Gln Ile Lys Glu
               20
<210> 27
   <211> 22
   <212> PRT
   <213> Artificial Sequence
<220>
   <223> Branched peptide Sequence with NH2 modified C terminal and branching at Lys8 and a lipid attached to Lys 17
<220>
   <221> SITE
   <222> (1)..(22)
   <223> Branch amino acid SEQ ID NO:25 attached to the Lys8 residue
<220>
   <221> SITE
   <222> (1)..(22)
   <223> C16 lipid attached to Lys 17
<400> 27
   Ser Asn Thr Ser Glu Ser Phe Lys Phe Arg Val Thr Gln Leu Ala Pro
   1               5                   10                  15
   Lys Ala Gln Ile Lys Glu
               20
<210> 28
   <211> 22
   <212> PRT
   <213> Artificial Sequence
<220>
   <223> Branched peptide sequence with NH2 modified C terminal and with two branches
<220>
   <221> SITE
   <222> (1)..(22)
   <223> First branch amino acid SEQ ID NO:25 attached to the Lys8 residue
<220>
   <221> SITE
   <222> (1)..(22)
   <223> Second branch MPA-NH-CH2-CH2-O-CH2-CH2-O-CO attached to the Lys17 residue
<400> 28
   Ser Asn Thr Ser Glu Ser Phe Lys Phe Arg Val Thr Gln Leu Ala Pro
   1               5                   10                  15
   Lys Ala Gln Ile Lys Glu
               20
<210> 29
   <211> 22
   <212> PRT
   <213> Artificial Sequence
<220>
   <223> Branched peptide Sequence with NH2 modified C terminal and branching at Lys8 residue and a lipid attached to Lys 21
<220>
   <221> SITE
   <222> (1)..(22)
   <223> Branch amino acid sequence SEQ ID NO:25 attached to the Lys8 residue
<220>
   <221> SITE
   <222> (1)..(22)
   <223> C16 lipid attached to Lys 21
<400> 29
   Ser Asn Thr Ser Glu Ser Phe Lys Phe Arg Val Thr Gln Leu Ala Pro
   1               5                   10                  15
   Lys Ala Gln Ile Lys Glu
               20
<210> 30
   <211> 451
   <212> PRT
   <213> Artificial sequence
<220>
   <223> durvalumab (MEDI4736) heavy chain
<400> 30
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Trp Met Ser Trp Val Arg Gln Ala Pro Gly Lys Gly Leu Glu Trp Val
           35                  40                  45
   Ala Asn Ile Lys Gln Asp Gly Ser Glu Lys Tyr Tyr Val Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Gly Gly Trp Phe Gly Glu Leu Ala Phe Asp Tyr Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
   180 185 190
   Pro Ser Ser Ser Leu Gly Thr Gln Thr Tyr Ile Cys Asn Val Asn His
   195 200 205
   Lys Pro Ser Asn Thr Lys Val Asp Lys Arg Val Glu Pro Lys Ser Cys
   210 215 220
   Asp Lys Thr His Thr Cys Pro Pro Cys Pro Ala Pro Glu Phe Glu Gly
   225 230 235 240
   Gly Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser His
   260 265 270
   Glu Asp Pro Glu Val Lys Phe Asn Trp Tyr Val Asp Gly Val Glu Val
   275 280 285
   His Asn Ala Lys Thr Lys Pro Arg Glu Glu Gln Tyr Asn Ser Thr Tyr
   290 295 300
   Arg Val Val Ser Val Leu Thr Val Leu His Gln Asp Trp Leu Asn Gly
   305 310 315 320
   Lys Glu Tyr Lys Cys Lys Val Ser Asn Lys Ala Leu Pro Ala Ser Ile
   325 330 335
   Glu Lys Thr Ile Ser Lys Ala Lys Gly Gln Pro Arg Glu Pro Gln Val
   340 345 350
   Tyr Thr Leu Pro Pro Ser Arg Glu Glu Met Thr Lys Asn Gln Val Ser
   355 360 365
   Leu Thr Cys Leu Val Lys Gly Phe Tyr Pro Ser Asp Ile Ala Val Glu
   370 375 380
   Trp Glu Ser Asn Gly Gln Pro Glu Asn Asn Tyr Lys Thr Thr Pro Pro
   385 390 395 400
   Val Leu Asp Ser Asp Gly Ser Phe Phe Leu Tyr Ser Lys Leu Thr Val
   405 410 415
   Asp Lys Ser Arg Trp Gln Gln Gly Asn Val Phe Ser Cys Ser Val Met
   420 425 430
   His Glu Ala Leu His Asn His Tyr Thr Gln Lys Ser Leu Ser Leu Ser
   435 440 445
   Pro Gly Lys
   450
<210> 31
   <211> 265
   <212> PRT
   <213> Artificial sequence
<220>
   <223> durvalumab (MEDI4736) light chain
<400> 31
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Arg Tyr
   20 25 30
   Trp Met Ser Trp Val Arg Gln Ala Pro Gly Lys Gly Leu Glu Trp Val
   35 40 45
   Ala Asn Glu Ile Val Leu Thr Gln Ser Pro Gly Thr Leu Ser Leu Ser
   50 55 60
   Pro Gly Glu Arg Ala Thr Leu Ser Cys Arg Ala Ser Gln Arg Val Ser
   65 70 75 80
   Ser Ser Tyr Leu Ala Trp Tyr Gln Gln Lys Pro Gly Gln Ala Pro Arg
   85 90 95
   Leu Leu Ile Tyr Asp Ala Ser Ser Arg Ala Thr Gly Ile Pro Asp Arg
   100 105 110
   Phe Ser Gly Ser Gly Ser Gly Thr Asp Phe Thr Leu Thr Ile Ser Arg
   115 120 125
   Leu Glu Pro Glu Asp Phe Ala Val Tyr Tyr Cys Gln Gln Tyr Gly Ser
   130 135 140
   Leu Pro Trp Thr Phe Gly Gln Gly Thr Lys Val Glu Ile Lys Arg Thr
   145 150 155 160
   Val Ala Ala Pro Ser Val Phe Ile Phe Pro Pro Ser Asp Glu Gln Leu
   165 170 175
   Lys Ser Gly Thr Ala Ser Val Val Cys Leu Leu Asn Asn Phe Tyr Pro
               180                 185                 190
   Arg Glu Ala Lys Val Gln Trp Lys Val Asp Asn Ala Leu Gln Ser Gly
           195                 200                 205
   Asn Ser Gln Glu Ser Val Thr Glu Gln Asp Ser Lys Asp Ser Thr Tyr
       210                 215                 220
   Ser Leu Ser Ser Thr Leu Thr Leu Ser Lys Ala Asp Tyr Glu Lys His
   225                 230                 235                 240
   Lys Val Tyr Ala Cys Glu Val Thr His Gln Gly Leu Ser Ser Pro Val
                   245                 250                 255
   Thr Lys Ser Phe Asn Arg Gly Glu Cys
               260                 265
<210> 32
   <211> 121
   <212> PRT
   <213> Artificial sequence
<220>
   <223> durvalumab (MEDI4736) heavy chain variable region, SEQ ID NO:72 from US2013/0034559A1
<400> 32
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Trp Met Ser Trp Val Arg Gln Ala Pro Gly Lys Gly Leu Glu Trp Val
           35                  40                  45
   Ala Asn Ile Lys Gln Asp Gly Ser Glu Lys Tyr Tyr Val Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Gly Gly Trp Phe Gly Glu Leu Ala Phe Asp Tyr Trp Gly
               100                 105                 110
   Gln Gly Thr Leu Val Thr Val Ser Ser
   115 120
<210> 33
   <211> 108
   <212> PRT
   <213> Artificial sequence
<220>
   <223> durvalumab (MEDI4736) light chain variable region, SEQ ID NO:77 from US2013/0034559A1
<400> 33
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Arg Val Ser Ser Ser
   20 25 30
   Tyr Leu Ala Trp Tyr Gln Gln Lys Pro Gly Gln Ala Pro Arg Leu Leu
   35 40 45
   Ile Tyr Asp Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Tyr Gly Ser Leu Pro
   85 90 95
   Trp Thr Phe Gly Gln Gly Thr Lys Val Glu Ile Lys
   100 105
<210> 34
   <211> 5
   <212> PRT
   <213> Artificial sequence
<220>
   <223> durvalumab (MEDI4736) heavy chain variable region CDR1, SEQ ID NO:23 from US2013/0034559A1
<400> 34
   Arg Tyr Trp Met Ser
   1 5
<210> 35
   <211> 17
   <212> PRT
   <213> Artificial sequence
<220>
   <223> durvalumab (MEDI4736) heavy chain variable region CDR2, SEQ ID NO:24 from US2013/0034559A1
<400> 35
   Asn Ile Lys Gln Asp Gly Ser Glu Lys Tyr Tyr Val Asp Ser Val Lys
   1               5                   10                  15
   Gly
<210> 36
   <211> 12
   <212> PRT
   <213> Artificial sequence
<220>
   <223> durvalumab (MEDI4736) heavy chain variable region CDR3, SEQ ID NO:25 from US2013/0034559A1
<400> 36
   Glu Gly Gly Trp Phe Gly Glu Leu Ala Phe Asp Tyr
   1               5                   10
<210> 37
   <211> 12
   <212> PRT
   <213> Artificial sequence
<220>
   <223> durvalumab (MEDI4736) light chain variable region CDR1, SEQ ID NO:28 from US2013/0034559A1
<400> 37
   Arg Ala Ser Gln Arg Val Ser Ser Ser Tyr Leu Ala
   1               5                   10
<210> 38
   <211> 7
   <212> PRT
   <213> Artificial sequence
<220>
   <223> durvalumab (MEDI4736) light chain variable region CDR2, SEQ ID NO:29 from US2013/0034559A1
<400> 38
   Asp Ala Ser Ser Arg Ala Thr
   1               5
<210> 39
   <211> 9
   <212> PRT
   <213> Artificial sequence
<220>
   <223> durvalumab (MEDI4736) light chain variable region CDR3, SEQ ID NO:30 from US2013/0034559A1
<400> 39
   Gln Gln Tyr Gly Ser Leu Pro Trp Thr
   1 5
<210> 40
   <211> 5
   <212> PRT
   <213> Artificial sequence
<220>
   <223> durvalumab (MEDI4736) alternative heavy chain variable region CDR1, SEQ ID NO:3 from US2013/0034559A1
<400> 40
   Thr Tyr Ser Met Asn
   1 5
<210> 41
   <211> 17
   <212> PRT
   <213> Artificial sequence
<220>
   <223> durvalumab (MEDI4736) alternative heavy chain variable region CDR2, SEQ ID NO:4 from US2013/0034559A1
<400> 41
   Ser Ile Ser Ser Ser Gly Asp Tyr Ile Tyr Tyr Ala Asp Ser Val Lys
   1 5 10 15
   Gly
<210> 42
   <211> 11
   <212> PRT
   <213> Artificial sequence
<220>
   <223> durvalumab (MEDI4736) alternative heavy chain variable region CDR3, SEQ ID NO:5 from US2013/0034559A1
<400> 42
   Asp Leu Val Thr Ser Met Val Ala Phe Asp Tyr
   1 5 10
<210> 43
   <211> 11
   <212> PRT
   <213> Artificial sequence
<220>
   <223> durvalumab (MEDI4736) alternative light chain variable region CDR1, SEQ ID NO:8 from US2013/0034559A1
<400> 43
   Ser Gly Asp Ala Leu Pro Gln Lys Tyr Val Phe
   1 5 10
<210> 44
   <211> 7
   <212> PRT
   <213> Artificial sequence
<220>
   <223> durvalumab (MEDI4736) alternative light chain variable region CDR2, SEQ ID NO:9 from US2013/0034559A1
<400> 44
   Glu Asp Ser Lys Arg Pro Ser
   1 5
<210> 45
   <211> 11
   <212> PRT
   <213> Artificial sequence
<220>
   <223> durvalumab (MEDI4736) alternative light chain variable region CDR3, SEQ ID NO:10 from US2013/0034559A1
<400> 45
   Tyr Ser Thr Asp Arg Ser Gly Asn His Arg Val
   1 5 10
<210> 46
   <211> 5
   <212> PRT
   <213> Artificial sequence
<220>
   <223> durvalumab (MEDI4736) alternative heavy chain variable region CDR1, SEQ ID NO:13 from US2013/0034559A1
<400> 46
   Ser Tyr Trp Met Ser
   1 5
<210> 47
   <211> 17
   <212> PRT
   <213> Artificial sequence
<220>
   <223> durvalumab (MEDI4736) alternative heavy chain variable region CDR2, SEQ ID NO:14 from US2013/0034559A1
<400> 47
   Asn Ile Lys Gln Asp Gly Gly Glu Gln Tyr Tyr Val Asp Ser Val Lys
   1 5 10 15
   Gly
<210> 48
   <211> 11
   <212> PRT
   <213> Artificial sequence
<220>
   <223> durvalumab (MEDI4736) alternative heavy chain variable region CDR3, SEQ ID NO:15 from US2013/0034559A1
<400> 48
   Asp Trp Asn Tyr Gly Tyr Tyr Asp Met Asp Val
   1 5 10
<210> 49
   <211> 12
   <212> PRT
   <213> Artificial sequence
<220>
   <223> durvalumab (MEDI4736) alternative light chain variable region CDR1, SEQ ID NO:18 from US2013/0034559A1
<400> 49
   Arg Ala Ser Gln Ser Val Ser Ser Asn Tyr Leu Ala
   1 5 10
<210> 50
   <211> 7
   <212> PRT
   <213> Artificial sequence
<220>
   <223> durvalumab (MEDI4736) alternative light chain variable region CDR2, SEQ ID NO:19 from US2013/0034559A1
<400> 50
   Gly Thr Ser Ser Arg Ala Thr
   1 5
<210> 51
   <211> 9
   <212> PRT
   <213> Artificial sequence
<220>
   <223> durvalumab (MEDI4736) alternative light chain variable region CDR3, SEQ ID NO:20 from US2013/0034559A1
<400> 51
   Gln Gln Tyr Gly Ser Ser Ile Phe Thr
   1 5
<210> 52
   <211> 5
   <212> PRT
   <213> Artificial sequence
<220>
   <223> durvalumab (MEDI4736) alternative heavy chain variable region CDR1, SEQ ID NO:63 from US2013/0034559A1
<400> 52
   Thr Tyr Ser Met Asn
   1 5
<210> 53
   <211> 17
   <212> PRT
   <213> Artificial sequence
<220>
   <223> durvalumab (MEDI4736) alternative heavy chain variable region CDR2, SEQ ID NO:64 from US2013/0034559A1
<400> 53
   Ser Ile Ser Ser Ser Gly Asp Tyr Ile Tyr Tyr Ala Asp Ser Val Lys
   1 5 10 15
   Gly
<210> 54
   <211> 11
   <212> PRT
   <213> Artificial sequence
<220>
   <223> durvalumab (MEDI4736) alternative heavy chain variable region CDR3, SEQ ID NO:65 from US2013/0034559A1
<400> 54
   Asp Leu Val Thr Ser Met Val Ala Phe Asp Tyr
   1 5 10
<210> 55
   <211> 11
   <212> PRT
   <213> Artificial sequence
<220>
   <223> durvalumab (MEDI4736) alternative light chain variable region CDR1, SEQ ID NO:68 from US2013/0034559A1
<400> 55
   Ser Gly Asp Ala Leu Pro Gln Lys Tyr Val Phe
   1 5 10
<210> 56
   <211> 7
   <212> PRT
   <213> Artificial sequence
<220>
   <223> durvalumab (MEDI4736) alternative light chain variable region CDR2, SEQ ID NO:69 from US2013/0034559A1
<400> 56
   Glu Asp Ser Lys Arg Pro Ser
   1 5
<210> 57
   <211> 11
   <212> PRT
   <213> Artificial sequence
<220>
   <223> durvalumab (MEDI4736) alternative light chain variable region CDR3, SEQ ID NO:70 from US2013/0034559A1
<400> 57
   Tyr Ser Thr Asp Arg Ser Gly Asn His Arg Val
   1 5 10
<210> 58
   <211> 5
   <212> PRT
   <213> Artificial sequence
<220>
   <223> durvalumab (MEDI4736) alternative heavy chain variable region CDR1, SEQ ID NO:73 from US2013/0034559A1
<400> 58
   Arg Tyr Trp Met Ser
   1 5
<210> 59
   <211> 17
   <212> PRT
   <213> Artificial sequence
<220>
   <223> durvalumab (MEDI4736) alternative heavy chain variable region CDR2, SEQ ID NO:74 from US2013/0034559A1
<400> 59
   Asn Ile Lys Gln Asp Gly Ser Glu Lys Tyr Tyr Val Asp Ser Val Lys
   1 5 10 15
   Gly
<210> 60
   <211> 12
   <212> PRT
   <213> Artificial sequence
<220>
   <223> durvalumab (MEDI4736) alternative heavy chain variable region CDR3, SEQ ID NO:75 from US2013/0034559A1
<400> 60
   Glu Gly Gly Trp Phe Gly Glu Leu Ala Phe Asp Tyr
   1 5 10
<210> 61
   <211> 12
   <212> PRT
   <213> Artificial sequence
<220>
   <223> durvalumab (MEDI4736) alternative light chain variable region CDR1, SEQ ID NO:78 from US2013/0034559A1
<400> 61
   Arg Ala Ser Gln Arg Val Ser Ser Ser Tyr Leu Ala
   1 5 10
<210> 62
   <211> 7
   <212> PRT
   <213> Artificial sequence
<220>
   <223> durvalumab (MEDI4736) alternative light chain variable region CDR2, SEQ ID NO:79 from US2013/0034559A1
<400> 62
   Asp Ala Ser Ser Arg Ala Thr
   1 5
<210> 63
   <211> 9
   <212> PRT
   <213> Artificial sequence
<220>
   <223> durvalumab (MEDI4736) alternative light chain variable region CDR3, SEQ ID NO:80 from US2013/0034559A1
<400> 63
   Gln Gln Tyr Gly Ser Leu Pro Trp Thr
   1 5
<210> 64
   <211> 448
   <212> PRT
   <213> Artificial sequence
<220>
   <223> atezolizumab (MPDL3280A) heavy chain
<400> 64
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Asp Ser
   20 25 30
   Trp Ile His Trp Val Arg Gln Ala Pro Gly Lys Gly Leu Glu Trp Val
   35 40 45
   Ala Trp Ile Ser Pro Tyr Gly Gly Ser Thr Tyr Tyr Ala Asp Ser Val
   50 55 60
   Lys Gly Arg Phe Thr Ile Ser Ala Asp Thr Ser Lys Asn Thr Ala Tyr
   65 70 75 80
   Leu Gln Met Asn Ser Leu Arg Ala Glu Asp Thr Ala Val Tyr Tyr Cys
   85 90 95
   Ala Arg Arg His Trp Pro Gly Gly Phe Asp Tyr Trp Gly Gln Gly Thr
   100 105 110
   Leu Val Thr Val Ser Ser Ala Ser Thr Lys Gly Pro Ser Val Phe Pro
   115 120 125
   Leu Ala Pro Ser Ser Lys Ser Thr Ser Gly Gly Thr Ala Ala Leu Gly
   130 135 140
   Cys Leu Val Lys Asp Tyr Phe Pro Glu Pro Val Thr Val Ser Trp Asn
   145 150 155 160
   Ser Gly Ala Leu Thr Ser Gly Val His Thr Phe Pro Ala Val Leu Gln
   165 170 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Ala Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Ser Arg Glu Glu Met Thr Lys Asn Gln Val Ser Leu Thr Cys
           355                 360                 365
   Leu Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Tyr Ser Lys Leu Thr Val Asp Lys Ser
                   405                 410                 415
   Arg Trp Gln Gln Gly Asn Val Phe Ser Cys Ser Val Met His Glu Ala
               420                 425                 430
   Leu His Asn His Tyr Thr Gln Lys Ser Leu Ser Leu Ser Pro Gly Lys
   435 440 445
<210> 65
   <211> 214
   <212> PRT
   <213> Artificial sequence
<220>
   <223> atezolizumab (MPDL3280A) light chain
<400> 65
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Val Ser Thr Ala
   20 25 30
   Val Ala Trp Tyr Gln Gln Lys Pro Gly Lys Ala Pro Lys Leu Leu Ile
   35 40 45
   Tyr Ser Ala Ser Phe Leu Tyr Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Leu Tyr His Pro Ala
   85 90 95
   Thr Phe Gly Gln Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 66
   <211> 118
   <212> PRT
   <213> Artificial sequence
<220>
   <223> atezolizumab (MPDL3280A) VH region (corresponds to SEQ ID NO:20 in U.S. Patent No. 8,217,149)
<400> 66
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Asp Ser
   20 25 30
   Trp Ile His Trp Val Arg Gln Ala Pro Gly Lys Gly Leu Glu Trp Val
   35 40 45
   Ala Trp Ile Ser Pro Tyr Gly Gly Ser Thr Tyr Tyr Ala Asp Ser Val
   50 55 60
   Lys Gly Arg Phe Thr Ile Ser Ala Asp Thr Ser Lys Asn Thr Ala Tyr
   65 70 75 80
   Leu Gln Met Asn Ser Leu Arg Ala Glu Asp Thr Ala Val Tyr Tyr Cys
   85 90 95
   Ala Arg Arg His Trp Pro Gly Gly Phe Asp Tyr Trp Gly Gln Gly Thr
   100 105 110
   Leu Val Thr Val Ser Ala
   115
<210> 67
   <211> 108
   <212> PRT
   <213> Artificial sequence
<220>
   <223> atezolizumab (MPDL3280A) VL region (corresponds to SEQ ID NO:21 in U.S. Patent No. 8,217,149)
<400> 67
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Leu Tyr His Pro Ala
                   85                  90                  95
   Thr Phe Gly Gln Gly Thr Lys Val Glu Ile Lys Arg
               100                 105
<210> 68
   <211> 10
   <212> PRT
   <213> Artificial sequence
<220>
   <223> atezolizumab (MPDL3280A) HVR-H1 region (corresponds to SEQ ID NO:1 in U.S. Patent No. 8,217,149)
<220>
   <221> variant
   <222> (6).. (6)
   <223> residue is D or G
<400> 68
   Gly Phe Thr Phe Ser Xaa Ser Trp Ile His
   1               5                   10
<210> 69
   <211> 18
   <212> PRT
   <213> Artificial sequence
<220>
   <223> atezolizumab (MPDL3280A) HVR-H2 region (corresponds to SEQ ID NO:2 in U.S. Patent No. 8,217,149
<220>
   <221> variant
   <222> (4)..(4)
   <223> residue is S or L
<220>
   <221> variant
   <222> (10)..(10)
   <223> residue is T or S
<400> 69 Lys Gly
<210> 70
   <211> 9
   <212> PRT
   <213> Artificial sequence
<220>
   <223> atezolizumab (MPDL3280A) HVR-H3 region (corresponds to SEQ ID NO:3 in U.S. Patent No. 8,217,149
<400> 70
   Arg His Trp Pro Gly Gly Phe Asp Tyr
   1               5
<210> 71
   <211> 11
   <212> PRT
   <213> Artificial sequence
<220>
   <223> atezolizumab (MPDL3280A) HVR-L1 region (corresponds to SEQ ID NO:8 in U.S. Patent No. 8,217,149
<220>
   <221> variant
   <222> (5)..(5)
   <223> residue is D or V
<220>
   <221> variant
   <222> (6).. (6)
   <223> residue is V or I
<220>
   <221> variant
   <222> (7)..(7)
   <223> residue is S or N
<220>
   <221> variant
   <222> (9).. (9)
   <223> residue is A or F
<220>
   <221> variant
   <222> (10)..(10)
   <223> residue is V or L
<400> 71
   Arg Ala Ser Gln Xaa Xaa Xaa Thr Xaa Xaa Ala
   1               5                   10
<210> 72
   <211> 7
   <212> PRT
   <213> Artificial sequence
<220>
   <223> atezolizumab (MPDL3280A) HVR-L2 region (corresponds to SEQ ID NO:9 in U.S. Patent No. 8,217,149
<220>
   <221> variant
   <222> (4).. (4)
   <223> residue is F or T
<220>
   <221> variant
   <222> (6).. (6)
   <223> residue is Y or A
<400> 72
   Ser Ala Ser Xaa Leu Xaa Ser
   1               5
<210> 73
   <211> 9
   <212> PRT
   <213> Artificial sequence
<220>
   <223> atezolizumab (MPDL3280A) HVR-L3 region (corresponds to SEQ ID NO:10 in U.S. Patent No. 8,217,149
<220>
   <221> variant
   <222> (3)..(3)
   <223> residue is Y, G, F or S
<220>
   <221> variant
   <222> (4)..(4)
   <223> residue is L, Y, F, or W
<220>
   <221> variant
   <222> (5)..(5)
   <223> residue is Y, N, A, T, G, F or I
<220>
   <221> variant
   <222> (6).. (6)
   <223> residue is H, V, P, T or I
<220>
   <221> variant
   <222> (8)..(8)
   <223> residue is A, W, R, P or T
<400> 73
   Gln Gln Xaa Xaa Xaa Xaa Pro Xaa Thr
   1 5
<210> 74
   <211> 450
   <212> PRT
   <213> Artificial Sequence
<220>
   <223> avelumab (MSB0010718C) heavy chain
<400> 74
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ile Met Met Trp Val Arg Gln Ala Pro Gly Lys Gly Leu Glu Trp Val
   35 40 45
   Ser Ser Ile Tyr Pro Ser Gly Gly Ile Thr Phe Tyr Ala Asp Thr Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Ile Lys Leu Gly Thr Val Thr Thr Val Asp Tyr Trp Gly Gln
   100 105 110
   Gly Thr Leu Val Thr Val Ser Ser Ala Ser Thr Lys Gly Pro Ser Val
   115 120 125
   Phe Pro Leu Ala Pro Ser Ser Lys Ser Thr Ser Gly Gly Thr Ala Ala
   130 135 140
   Leu Gly Cys Leu Val Lys Asp Tyr Phe Pro Glu Pro Val Thr Val Ser
   145 150 155 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Gln Thr Tyr Ile Cys Asn Val Asn His Lys
   195 200 205
   Pro Ser Asn Thr Lys Val Asp Lys Lys Val Glu Pro Lys Ser Cys Asp
   210 215 220
   Lys Thr His Thr Cys Pro Pro Cys Pro Ala Pro Glu Leu Leu Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser His Glu
   260 265 270
   Asp Pro Glu Val Lys Phe Asn Trp Tyr Val Asp Gly Val Glu Val His
   275 280 285
   Asn Ala Lys Thr Lys Pro Arg Glu Glu Gln Tyr Asn Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Pro Ala Pro Ile Glu
   325 330 335
   Lys Thr Ile Ser Lys Ala Lys Gly Gln Pro Arg Glu Pro Gln Val Tyr
   340 345 350
   Thr Leu Pro Pro Ser Arg Asp Glu Leu Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Lys
   450
<210> 75
   <211> 216
   <212> PRT
   <213> Artificial Sequence
<220>
   <223> avelumab (MSB0010718C) light chain
<400> 75
   Gln Ser Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Gly Tyr
               20                  25                  30
   Asn Tyr Val Ser Trp Tyr Gln Gln His Pro Gly Lys Ala Pro Lys Leu
           35                  40                  45
   Met Ile Tyr Asp Val Ser Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Ser Tyr Thr Ser Ser
                   85                  90                  95
   Ser Thr Arg Val Phe Gly Thr Gly Thr Lys Val Thr Val Leu Gly Gln
               100                 105                 110
   Pro Lys Ala Asn Pro Thr Val Thr Leu Phe Pro Pro Ser Ser Glu Glu
           115                 120                 125
   Leu Gln Ala Asn Lys Ala Thr Leu Val Cys Leu Ile Ser Asp Phe Tyr
       130                 135                 140
   Pro Gly Ala Val Thr Val Ala Trp Lys Ala Asp Gly Ser Pro Val Lys
   145                 150                 155                 160
   Ala Gly Val Glu Thr Thr Lys Pro Ser Lys Gln Ser Asn Asn Lys Tyr
                   165                 170                 175
   Ala Ala Ser Ser Tyr Leu Ser Leu Thr Pro Glu Gln Trp Lys Ser His
               180                 185                 190
   Arg Ser Tyr Ser Cys Gln Val Thr His Glu Gly Ser Thr Val Glu Lys
           195                 200                 205
   Thr Val Ala Pro Thr Glu Cys Ser
       210                 215
<210> 76
   <211> 120
   <212> PRT
   <213> Artificial Sequence
<220>
   <223> avelumab (MSB0010718C) VH region (corresponding to SEQ ID NO:24 in U.S. Patent Application Publication No. US 2014/0341917 A1).
<400> 76
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ile Met Met Trp Val Arg Gln Ala Pro Gly Lys Gly Leu Glu Trp Val
           35                  40                  45
   Ser Ser Ile Tyr Pro Ser Gly Gly Ile Thr Phe Tyr Ala Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Ile Lys Leu Gly Thr Val Thr Thr Val Asp Tyr Trp Gly Gln
               100                 105                 110
   Gly Thr Leu Val Thr Val Ser Ser
           115                 120
<210> 77
   <211> 110
   <212> PRT
   <213> Artificial Sequence
<220>
   <223> avelumab (MSB0010718C) VL region (corresponding to SEQ ID NO:25 in U.S. Patent Application Publication No. US 2014/0341917 A1).
<400> 77
   Gln Ser Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Gly Tyr
               20                  25                  30
   Asn Tyr Val Ser Trp Tyr Gln Gln His Pro Gly Lys Ala Pro Lys Leu
           35                  40                  45
   Met Ile Tyr Asp Val Ser Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Ser Tyr Thr Ser Ser
                   85                  90                  95
   Ser Thr Arg Val Phe Gly Thr Gly Thr Lys Val Thr Val Leu
               100                 105                 110
<210> 78
   <211> 5
   <212> PRT
   <213> Artificial Sequence
<220>
   <223> avelumab (MSB0010718C) HVR-H1 region (corresponding to SEQ ID NO:15 in U.S. Patent Application Publication No. US 2014/0341917 A1).
<400> 78
   Ser Tyr Ile Met Met
   1               5
<210> 79
   <211> 17
   <212> PRT
   <213> Artificial Sequence
<220>
   <223> avelumab (MSB0010718C) HVR-H2 region (corresponding to SEQ ID NO:16 in U.S. Patent Application Publication No. US 2014/0341917 A1).
<400> 79
   Ser Ile Tyr Pro Ser Gly Gly Ile Thr Phe Tyr Ala Asp Thr Val Lys
   1               5                   10                  15
   Gly
<210> 80
   <211> 11
   <212> PRT
   <213> Artificial Sequence
<220>
   <223> avelumab (MSB0010718C) HVR-H3 region (corresponding to SEQ ID NO:17 in U.S. Patent Application Publication No. US 2014/0341917 A1).
<400> 80
   Ile Lys Leu Gly Thr Val Thr Thr Val Asp Tyr
   1 5 10
<210> 81
   <211> 14
   <212> PRT
   <213> Artificial Sequence
<220>
   <223> avelumab (MSB0010718C) HVR-L1 region (corresponding to SEQ ID NO:18 in U.S. Patent Application Publication No. US 2014/0341917 A1).
<400> 81
   Thr Gly Thr Ser Ser Asp Val Gly Gly Tyr Asn Tyr Val Ser
   1 5 10
<210> 82
   <211> 7
   <212> PRT
   <213> Artificial Sequence
<220>
   <223> avelumab (MSB0010718C) HVR-L2 region (corresponding to SEQ ID NO:19 in U.S. Patent Application Publication No. US 2014/0341917 A1).
<400> 82
   Asp Val Ser Asn Arg Pro Ser
   1 5
<210> 83
   <211> 10
   <212> PRT
   <213> Artificial Sequence
<220>
   <223> avelumab (MSB0010718C) HVR-L3 region (corresponding to SEQ ID NO:20 in U.S. Patent Application Publication No. US 2014/0341917 A1).
<400> 83
   Ser Ser Tyr Thr Ser Ser Ser Thr Arg Val
   1 5 10
<210> 84
   <211> 451
   <212> PRT
   <213> Artificial Sequence
<220>
   <223> Heavy chain amino acid sequence of the anti-CD20 monoclonal antibody rituximab.
<400> 84
   Gln Val Gln Leu Gln Gln Pro Gly Ala Glu Leu Val Lys Pro Gly Ala
   1               5                   10                  15
   Ser Val Lys Met Ser Cys Lys Ala Ser Gly Tyr Thr Phe Thr Ser Tyr
               20                  25                  30
   Asn Met His Trp Val Lys Gln Thr Pro Gly Arg Gly Leu Glu Trp Ile
           35                  40                  45
   Gly Ala Ile Tyr Pro Gly Asn Gly Asp Thr Ser Tyr Asn Gln Lys Phe
       50                  55                  60
   Lys Gly Lys Ala Thr Leu Thr Ala Asp Lys Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Ser Thr Tyr Tyr Gly Gly Asp Trp Tyr Phe Asn Val Trp Gly
               100                 105                 110
   Ala Gly Thr Thr Val Thr Val Ser Ala Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
   180 185 190
   Pro Ser Ser Ser Leu Gly Thr Gln Thr Tyr Ile Cys Asn Val Asn His
   195 200 205
   Lys Pro Ser Asn Thr Lys Val Asp Lys Lys Val Glu Pro Lys Ser Cys
   210 215 220
   Asp Lys Thr His Thr Cys Pro Pro Cys Pro Ala Pro Glu Leu Leu Gly
   225 230 235 240
   Gly Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser His
   260 265 270
   Glu Asp Pro Glu Val Lys Phe Asn Trp Tyr Val Asp Gly Val Glu Val
   275 280 285
   His Asn Ala Lys Thr Lys Pro Arg Glu Glu Gln Tyr Asn Ser Thr Tyr
   290 295 300
   Arg Val Val Ser Val Leu Thr Val Leu His Gln Asp Trp Leu Asn Gly
   305 310 315 320
   Lys Glu Tyr Lys Cys Lys Val Ser Asn Lys Ala Leu Pro Ala Pro Ile
   325 330 335
   Glu Lys Thr Ile Ser Lys Ala Lys Gly Gln Pro Arg Glu Pro Gln Val
   340 345 350
   Tyr Thr Leu Pro Pro Ser Arg Asp Glu Leu Thr Lys Asn Gln Val Ser
   355 360 365
   Leu Thr Cys Leu Val Lys Gly Phe Tyr Pro Ser Asp Ile Ala Val Glu
   370 375 380
   Trp Glu Ser Asn Gly Gln Pro Glu Asn Asn Tyr Lys Thr Thr Pro Pro
   385 390 395 400
   Val Leu Asp Ser Asp Gly Ser Phe Phe Leu Tyr Ser Lys Leu Thr Val
   405 410 415
   Asp Lys Ser Arg Trp Gln Gln Gly Asn Val Phe Ser Cys Ser Val Met
   420 425 430
   His Glu Ala Leu His Asn His Tyr Thr Gln Lys Ser Leu Ser Leu Ser
   435 440 445
   Pro Gly Lys
   450
<210> 85
   <211> 213
   <212> PRT
   <213> Artificial sequence
<220>
   <223> Light chain amino acid sequence of the anti-CD20 monoclonal antibody rituximab.
<400> 85
   Gln Ile Val Leu Ser Gln Ser Pro Ala Ile Leu Ser Ala Ser Pro Gly
   1 5 10 15
   Glu Lys Val Thr Met Thr Cys Arg Ala Ser Ser Ser Val Ser Tyr Ile
   20 25 30
   His Trp Phe Gln Gln Lys Pro Gly Ser Ser Pro Lys Pro Trp Ile Tyr
   35 40 45
   Ala Thr Ser Asn Leu Ala Ser Gly Val Pro Val Arg Phe Ser Gly Ser
   50 55 60
   Gly Ser Gly Thr Ser Tyr Ser Leu Thr Ile Ser Arg Val Glu Ala Glu
   65 70 75 80
   Asp Ala Ala Thr Tyr Tyr Cys Gln Gln Trp Thr Ser Asn Pro Pro Thr
   85 90 95
   Phe Gly Gly Gly Thr Lys Leu Glu Ile Lys Arg Thr Val Ala Ala Pro
   100 105 110
   Ser Val Phe Ile Phe Pro Pro Ser Asp Glu Gln Leu Lys Ser Gly Thr
   115 120 125
   Ala Ser Val Val Cys Leu Leu Asn Asn Phe Tyr Pro Arg Glu Ala Lys
   130 135 140
   Val Gln Trp Lys Val Asp Asn Ala Leu Gln Ser Gly Asn Ser Gln Glu
   145 150 155 160
   Ser Val Thr Glu Gln Asp Ser Lys Asp Ser Thr Tyr Ser Leu Ser Ser
   165 170 175
   Thr Leu Thr Leu Ser Lys Ala Asp Tyr Glu Lys His Lys Val Tyr Ala
   180 185 190
   Cys Glu Val Thr His Gln Gly Leu Ser Ser Pro Val Thr Lys Ser Phe
   195 200 205
   Asn Arg Gly Glu Cys
   210
<210> 86
   <211> 449
   <212> PRT
   <213> Artificial sequence
<220>
   <223> Heavy chain amino acid sequence of the anti-CD20 monoclonal antibody obinutuzumab.
<400> 86
   Gln Val Gln Leu Val Gln Ser Gly Ala Glu Val Lys Lys Pro Gly Ser
   1 5 10 15
   Ser Val Lys Val Ser Cys Lys Ala Ser Gly Tyr Ala Phe Ser Tyr Ser
   20 25 30
   Trp Ile Asn Trp Val Arg Gln Ala Pro Gly Gln Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser Ala Ser Thr Lys Gly Pro Ser Val Phe
   115 120 125
   Pro Leu Ala Pro Ser Ser Lys Ser Thr Ser Gly Gly Thr Ala Ala Leu
   130 135 140
   Gly Cys Leu Val Lys Asp Tyr Phe Pro Glu Pro Val Thr Val Ser Trp
   145 150 155 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Gln Thr Tyr Ile Cys Asn Val Asn His Lys Pro
           195                 200                 205
   Ser Asn Thr Lys Val Asp Lys Lys Val Glu Pro Lys Ser Cys Asp Lys
       210                 215                 220
   Thr His Thr Cys Pro Pro Cys Pro Ala Pro Glu Leu Leu Gly Gly Pro
   225                 230                 235                 240
   Ser Val Phe Leu Phe Pro Pro Lys Pro Lys Asp Thr Leu Met Ile Ser
                   245                 250                 255
   Arg Thr Pro Glu Val Thr Cys Val Val Val Asp Val Ser His Glu Asp
               260                 265                 270
   Pro Glu Val Lys Phe Asn Trp Tyr Val Asp Gly Val Glu Val His Asn
           275                 280                 285
   Ala Lys Thr Lys Pro Arg Glu Glu Gln Tyr Asn Ser Thr Tyr Arg Val
       290                 295                 300
   Val Ser Val Leu Thr Val Leu His Gln Asp Trp Leu Asn Gly Lys Glu
   305                 310                 315                 320
   Tyr Lys Cys Lys Val Ser Asn Lys Ala Leu Pro Ala Pro Ile Glu Lys
                   325                 330                 335
   Thr Ile Ser Lys Ala Lys Gly Gln Pro Arg Glu Pro Gln Val Tyr Thr
               340                 345                 350
   Leu Pro Pro Ser Arg Asp Glu Leu Thr Lys Asn Gln Val Ser Leu Thr
           355                 360                 365
   Cys Leu Val Lys Gly Phe Tyr Pro Ser Asp Ile Ala Val Glu Trp Glu
       370                 375                 380
   Ser Asn Gly Gln Pro Glu Asn Asn Tyr Lys Thr Thr Pro Pro Val Leu
   385                 390                 395                 400
   Asp Ser Asp Gly Ser Phe Phe Leu Tyr Ser Lys Leu Thr Val Asp Lys
                   405                 410                 415
   Ser Arg Trp Gln Gln Gly Asn Val Phe Ser Cys Ser Val Met His Glu
   420 425 430
   Ala Leu His Asn His Tyr Thr Gln Lys Ser Leu Ser Leu Ser Pro Gly
   435 440 445
   Lys
<210> 87
   <211> 219
   <212> PRT
   <213> Artificial sequence
<220>
   <223> Light chain amino acid sequence of the anti-CD20 monoclonal antibody obinutuzumab.
<400> 87
   Asp Ile Val Met Thr Gln Thr Pro Leu Ser Leu Pro Val Thr Pro Gly
   1 5 10 15
   Glu Pro Ala Ser Ile Ser Cys Arg Ser Ser Lys Ser Leu Leu His Ser
   20 25 30
   Asn Gly Ile Thr Tyr Leu Tyr Trp Tyr Leu Gln Lys Pro Gly Gln Ser
   35 40 45
   Pro Gln Leu Leu Ile Tyr Gln Met Ser Asn Leu Val Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Ala Gln Asn
   85 90 95
   Leu Glu Leu Pro Tyr Thr Phe Gly Gly Gly Thr Lys Val Glu Ile Lys
   100 105 110
   Arg Thr Val Ala Ala Pro Ser Val Phe Ile Phe Pro Pro Ser Asp Glu
   115 120 125
   Gln Leu Lys Ser Gly Thr Ala Ser Val Val Cys Leu Leu Asn Asn Phe
   130 135 140
   Tyr Pro Arg Glu Ala Lys Val Gln Trp Lys Val Asp Asn Ala Leu Gln
   145 150 155 160
   Ser Gly Asn Ser Gln Glu Ser Val Thr Glu Gln Asp Ser Lys Asp Ser
   165 170 175
   Thr Tyr Ser Leu Ser Ser Thr Leu Thr Leu Ser Lys Ala Asp Tyr Glu
   180 185 190
   Lys His Lys Val Tyr Ala Cys Glu Val Thr His Gln Gly Leu Ser Ser
   195 200 205
   Pro Val Thr Lys Ser Phe Asn Arg Gly Glu Cys
   210 215
<210> 88
   <211> 122
   <212> PRT
   <213> Artificial sequence
<220>
   <223> Variable heavy chain amino acid sequence of the anti-CD20 monoclonal antibody ofatumumab.
<400> 88
   Glu Val Gln Leu Val Glu Ser Gly Gly Gly Leu Val Gln Pro Gly Arg
   1 5 10 15
   Ser Leu Arg Leu Ser Cys Ala Ala Ser Gly Phe Thr Phe Asn Asp Tyr
   20 25 30
   Ala Met His Trp Val Arg Gln Ala Pro Gly Lys Gly Leu Glu Trp Val
   35 40 45
   Ser Thr Ile Ser Trp Asn Ser Gly Ser Ile Gly Tyr Ala Asp Ser Val
   50 55 60
   Lys Gly Arg Phe Thr Ile Ser Arg Asp Asn Ala Lys Lys Ser Leu Tyr
   65 70 75 80
   Leu Gln Met Asn Ser Leu Arg Ala Glu Asp Thr Ala Leu Tyr Tyr Cys
   85 90 95
   Ala Lys Asp Ile Gln Tyr Gly Asn Tyr Tyr Tyr Gly Met Asp Val Trp
   100 105 110
   Gly Gln Gly Thr Thr Val Thr Val Ser Ser
   115 120
<210> 89
   <211> 107
   <212> PRT
   <213> Artificial sequence
<220>
   <223> Variable light chain amino acid sequence of the anti-CD20 monoclonal antibody ofatumumab.
<400> 89
<210> 90
   <211> 222
   <212> PRT
   <213> Artificial Sequence
<220>
   <223> Fab fragment of heavy chain amino acid sequence of the anti-CD20 monoclonal antibody ofatumumab.
<400> 90
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asn Asp Tyr
               20                  25                  30
   Ala Met His Trp Val Arg Gln Ala Pro Gly Lys Gly Leu Glu Trp Val
           35                  40                  45
   Ser Thr Ile Ser Trp Asn Ser Gly Ser Ile Gly Tyr Ala Asp Ser Val
       50                  55                  60
   Lys Gly Arg Phe Thr Ile Ser Arg Asp Asn Ala Lys Lys Ser Leu Tyr
   65 70 75 80
   Leu Gln Met Asn Ser Leu Arg Ala Glu Asp Thr Ala Leu Tyr Tyr Cys
   85 90 95
   Ala Lys Asp Ile Gln Tyr Gly Asn Tyr Tyr Tyr Gly Met Asp Val Trp
   100 105 110
   Gly Gln Gly Thr Thr Val Thr Val Ser Ser Ala Ser Thr Lys Gly Pro
   115 120 125
   Ser Val Phe Pro Leu Ala Pro Gly Ser Ser Lys Ser Thr Ser Gly Thr
   130 135 140
   Ala Ala Leu Gly Cys Leu Val Lys Asp Tyr Phe Pro Glu Pro Val Thr
   145 150 155 160
   Val Ser Trp Asn Ser Gly Ala Leu Thr Ser Gly Val His Thr Phe Pro
   165 170 175
   Ala Val Leu Gln Ser Ser Gly Leu Tyr Ser Leu Ser Ser Val Val Thr
   180 185 190
   Val Pro Ser Ser Ser Leu Gly Thr Gln Thr Tyr Ile Cys Asn Val Asn
   195 200 205
   His Lys Pro Ser Asn Thr Lys Val Asp Lys Lys Val Glu Pro
   210 215 220
<210> 91
   <211> 211
   <212> PRT
   <213> Artificial Sequence
<220>
   <223> Fab fragment of light chain amino acid sequence of the anti-CD20 monoclonal antibody ofatumumab.
<400> 91
   Glu Ile Val Leu Thr Gln Ser Pro Ala Thr Leu Ser Leu Ser Pro Gly
   1 5 10 15
   Glu Arg Ala Thr Leu Ser Cys Arg Ala Ser Gln Ser Val Ser Ser Tyr
   20 25 30
   Leu Ala Trp Tyr Gln Gln Lys Pro Gly Gln Ala Pro Arg Leu Leu Ile
   35 40 45
   Tyr Asp Ala Ser Asn Arg Ala Thr Gly Ile Pro Ala Arg Phe Ser Gly
   50 55 60
   Ser Gly Ser Gly Thr Asp Phe Thr Leu Thr Ile Ser Ser Leu Glu Pro
   65 70 75 80
   Glu Asp Phe Ala Val Tyr Tyr Cys Gln Gln Arg Ser Asn Trp Pro Ile
   85 90 95
   Thr Phe Gly Gln Gly Thr Arg Leu Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg
   210
<210> 92
   <211> 451
   <212> PRT
   <213> Artificial Sequence
<220>
   <223> Heavy chain amino acid sequence of the anti-CD20 monoclonal antibody veltuzumab.
<400> 92
   Gln Val Gln Leu Gln Gln Ser Gly Ala Glu Val Lys Lys Pro Gly Ser
   1 5 10 15
   Ser Val Lys Val Ser Cys Lys Ala Ser Gly Tyr Thr Phe Thr Ser Tyr
   20 25 30
   Asn Met His Trp Val Lys Gln Ala Pro Gly Gln Gly Leu Glu Trp Ile
   35 40 45
   Gly Ala Ile Tyr Pro Gly Met Gly Asp Thr Ser Tyr Asn Gln Lys Phe
   50 55 60
   Lys Gly Lys Ala Thr Leu Thr Ala Asp Glu Ser Thr Asn Thr Ala Tyr
   65 70 75 80
   Met Glu Leu Ser Ser Leu Arg Ser Glu Asp Thr Ala Phe Tyr Tyr Cys
   85 90 95
   Ala Arg Ser Thr Tyr Tyr Gly Gly Asp Trp Tyr Phe Asp Val Trp Gly
   100 105 110
   Gln Gly Thr Thr Val Thr Val Ser Ser Ala Ser Thr Lys Gly Pro Ser
   115 120 125
   Val Phe Pro Leu Ala Pro Ser Ser Lys Ser Thr Ser Gly Gly Thr Ala
   130 135 140
   Ala Leu Gly Cys Leu Val Lys Asp Tyr Phe Pro Glu Pro Val Thr Val
   145 150 155 160
   Ser Trp Asn Ser Gly Ala Leu Thr Ser Gly Val His Thr Phe Pro Ala
   165 170 175
   Val Leu Gln Ser Ser Gly Leu Tyr Ser Leu Ser Ser Val Val Thr Val
   180 185 190
   Pro Ser Ser Ser Leu Gly Thr Gln Thr Tyr Ile Cys Asn Val Asn His
   195 200 205
   Lys Pro Ser Asn Thr Lys Val Asp Lys Arg Val Glu Pro Lys Ser Cys
   210 215 220
   Asp Lys Thr His Thr Cys Pro Pro Cys Pro Ala Pro Glu Leu Leu Gly
   225 230 235 240
   Gly Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser His
   260 265 270
   Glu Asp Pro Glu Val Lys Phe Asn Trp Tyr Val Asp Gly Val Glu Val
   275 280 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Glu Glu Met Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 93
   <211> 213
   <212> PRT
   <213> Artificial Sequence
<220>
   <223> Light chain amino acid sequence of the anti-CD20 monoclonal antibody veltuzumab.
<400> 93
   Asp Ile Gln Leu Thr Gln Ser Pro Ser Ser Leu Ser Ala Ser Val Gly
   1               5                   10                  15
   Asp Arg Val Thr Met Thr Cys Arg Ala Ser Ser Ser Val Ser Tyr Ile
               20                  25                  30
   His Trp Phe Gln Gln Lys Pro Gly Lys Ala Pro Lys Pro Trp Ile Tyr
   35 40 45
   Ala Thr Ser Asn Leu Ala Ser Gly Val Pro Val Arg Phe Ser Gly Ser
   50 55 60
   Gly Ser Gly Thr Asp Tyr Thr Phe Thr Ile Ser Ser Leu Gln Pro Glu
   65 70 75 80
   Asp Ile Ala Thr Tyr Tyr Cys Gln Gln Trp Thr Ser Asn Pro Pro Thr
   85 90 95
   Phe Gly Gly Gly Thr Lys Leu Glu Ile Lys Arg Thr Val Ala Ala Pro
   100 105 110
   Ser Val Phe Ile Phe Pro Pro Ser Asp Glu Gln Leu Lys Ser Gly Thr
   115 120 125
   Ala Ser Val Val Cys Leu Leu Asn Asn Phe Tyr Pro Arg Glu Ala Lys
   130 135 140
   Val Gln Trp Lys Val Asp Asn Ala Leu Gln Ser Gly Asn Ser Gln Glu
   145 150 155 160
   Ser Val Thr Glu Gln Asp Ser Lys Asp Ser Thr Tyr Ser Leu Ser Ser
   165 170 175
   Thr Leu Thr Leu Ser Lys Ala Asp Tyr Glu Lys His Lys Val Tyr Ala
   180 185 190
   Cys Glu Val Thr His Gln Gly Leu Ser Ser Pro Val Thr Lys Ser Phe
   195 200 205
   Asn Arg Gly Glu Cys
   210
<210> 94
   <211> 447
   <212> PRT
   <213> Artificial Sequence
<220>
   <223> Heavy chain amino acid sequence of the anti-CD20 monoclonal antibody tositumomab.
<400> 94
   Gln Ala Tyr Leu Gln Gln Ser Gly Ala Glu Leu Val Arg Pro Gly Ala
   1 5 10 15
   Ser Val Lys Met Ser Cys Lys Ala Ser Gly Tyr Thr Phe Thr Ser Tyr
               20                  25                  30
   Asn Met His Trp Val Lys Gln Thr Pro Arg Gln Gly Leu Glu Trp Ile
           35                  40                  45
   Gly Ala Ile Tyr Pro Gly Asn Gly Asp Thr Ser Tyr Asn Gln Lys Phe
       50                  55                  60
   Lys Gly Lys Ala Thr Leu Thr Val Asp Lys Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Arg Val Val Tyr Tyr Ser Asn Ser Tyr Trp Tyr Phe Asp Val Trp
               100                 105                 110
   Gly Thr Gly Thr Thr Val Thr Val Ser Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Lys Ala Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
   260 265 270
   Val Lys Phe Asn Trp Tyr Val Asp Gly Val Glu Val His Asn Ala Lys
   275 280 285
   Thr Lys Pro Arg Glu Glu Gln Tyr Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Ala Leu Pro Ala Pro Ile Glu Lys Thr Ile
   325 330 335
   Ser Lys Ala Lys Gly Gln Pro Arg Glu Pro Gln Val Tyr Thr Leu Pro
   340 345 350
   Pro Ser Arg Asp Glu Leu Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 95
   <211> 210
   <212> PRT
   <213> Artificial Sequence
<220>
   <223> Light chain amino acid sequence of the anti-CD20 monoclonal antibody tositumomab.
<400> 95
   Gln Ile Val Leu Ser Gln Ser Pro Ala Ile Leu Ser Ala Ser Pro Gly
   1               5                   10                  15
   Glu Lys Val Thr Met Thr Cys Arg Ala Ser Ser Ser Val Ser Tyr Met
               20                  25                  30
   His Trp Tyr Gln Gln Lys Pro Gly Ser Ser Pro Lys Pro Trp Ile Tyr
           35                  40                  45
   Ala Pro Ser Asn Leu Ala Ser Gly Val Pro Ala Arg Phe Ser Gly Ser
       50                  55                  60
   Gly Ser Gly Thr Ser Tyr Ser Leu Thr Ile Ser Arg Val Glu Ala Glu
   65                  70                  75                  80
   Asp Ala Ala Thr Tyr Tyr Cys Gln Gln Trp Ser Phe Asn Pro Pro Thr
                   85                  90                  95
   Phe Gly Ala Gly Thr Lys Leu Glu Leu Lys Arg Thr Val Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn Arg
       210
<210> 97
   <211> 209
   <212> PRT
   <213> Artificial Sequence
<220>
   <223> Light chain amino acid sequence of the anti-CD20 monoclonal antibody ibritumomab.
<400> 97 SEQUENCE LISTING
<110> Acerta Pharma B.V.
<120> THERAPEUTIC COMBINATIONS OF A BTK INHIBITOR, A PI3K INHIBITOR, A JAK-2 INHIBITOR, A PD-1 INHIBITOR, AND/OR A PD-L1 INHIBITOR
<130> 055112-5014-WO
<140> PCT/IB2015/000000 <141> 2015-08-11
<150> 62/035,812 <151> 2014-08-11
<150> 62/088,357 <151> 2014-12-05
<150> 62/115,489 <151> 2015-02-12
<150> 62/181,164 <151> 2015-06-17
<160> 97
<170> PatentIn version 3.5
<210> 1
   <211> 440
   <212> PRT
   <213> Artificial sequence
<220>
   <223> Heavy Chain Amino Acid Sequence, Anti-PD-1 mAb (nivolumab, BMS936558; also 5C4 in WO 2006/121168) (SEQ ID NO:17 IN WO 2014/055648A1)
<400> 1
   Gln Val Gln Leu Val Glu Ser Gly Gly Gly Val Val Gln Pro Gly Arg
   1               5                   10                  15
   Ser Leu Arg Leu Asp Cys Lys Ala Ser Gly Ile Thr Phe Ser Asn Ser
               20                  25                  30
   Gly Met His Trp Val Arg Gln Ala Pro Gly Lys Gly Leu Glu Trp Val
           35                  40                  45
   Ala Val Ile Trp Tyr Asp Gly Ser Lys Arg Tyr Tyr Ala Asp Ser Val
       50                  55                  60
   Lys Gly Arg Phe Thr Ile Ser Arg Asp Asn Ser Lys Asn Thr Leu Phe
   65                  70                  75                  80
   Leu Gln Met Asn Ser Leu Arg Ala Glu Asp Thr Ala Val Tyr Tyr Cys
                   85                  90                  95
   Ala Thr Asn Asp Asp Tyr Trp Gly Gln Gly Thr Leu Val Thr Val Ser
               100                 105                 110
   Ser Ala Ser Thr Lys Gly Pro Ser Val Phe Pro Leu Ala Pro Cys Ser
           115                 120                 125
   Arg Ser Thr Ser Glu Ser Thr Ala Ala Leu Gly Cys Leu Val Lys Asp
       130                 135                 140
   Tyr Phe Pro Glu Pro Val Thr Val Ser Trp Asn Ser Gly Ala Leu Thr
   145                 150                 155                 160
   Ser Gly Val His Thr Phe Pro Ala Val Leu Gln Ser Ser Gly Leu Tyr
                   165                 170                 175
   Ser Leu Ser Ser Val Val Thr Val Pro Ser Ser Ser Leu Gly Thr Lys
               180                 185                 190
   Thr Tyr Thr Cys Asn Val Asp His Lys Pro Ser Asn Thr Lys Val Asp
           195                 200                 205
   Lys Arg Val Glu Ser Lys Tyr Gly Pro Pro Cys Pro Pro Cys Pro Ala
       210                 215                 220
   Pro Glu Phe Leu Gly Gly Pro Ser Val Phe Leu Phe Pro Pro Lys Pro
   225                 230                 235                 240
   Lys Asp Thr Leu Met Ile Ser Arg Thr Pro Glu Val Thr Cys Val Val
                   245                 250                 255
   Val Asp Val Ser Gln Glu Asp Pro Glu Val Gln Phe Asn Trp Tyr Val
               260                 265                 270
   Asp Gly Val Glu Val His Asn Ala Lys Thr Lys Pro Arg Glu Glu Gln
           275                 280                 285
   Phe Asn Ser Thr Tyr Arg Val Val Ser Val Leu Thr Val Leu His Gln
       290                 295                 300
   Asp Trp Leu Asn Gly Lys Glu Tyr Lys Cys Lys Val Ser Asn Lys Gly
   305                 310                 315                 320
   Leu Pro Ser Ser Ile Glu Lys Thr Ile Ser Lys Ala Lys Gly Gln Pro
                   325                 330                 335
   Arg Glu Pro Gln Val Tyr Thr Leu Pro Pro Ser Gln Glu Glu Met Thr
               340                 345                 350
   Lys Asn Gln Val Ser Leu Thr Cys Leu Val Lys Gly Phe Tyr Pro Ser
           355                 360                 365
   Asp Ile Ala Val Glu Trp Glu Ser Asn Gly Gln Pro Glu Asn Asn Tyr
       370                 375                 380
   Lys Thr Thr Pro Pro Val Leu Asp Ser Asp Gly Ser Phe Phe Leu Tyr
   385                 390                 395                 400
   Ser Arg Leu Thr Val Asp Lys Ser Arg Trp Gln Glu Gly Asn Val Phe
                   405                 410                 415
   Ser Cys Ser Val Met His Glu Ala Leu His Asn His Tyr Thr Gln Lys
               420                 425                 430
   Ser Leu Ser Leu Ser Leu Gly Lys
           435                 440
<210> 2
   <211> 214
   <212> PRT
   <213> Artificial sequence
<220>
   <223> Light Chain Amino Acid Sequence, Anti-PD-1 mAb (nivolumab, BMS936558; 5C4 in WO 2006/121168) (SEQ ID NO:18 IN WO 2014/055648 A1)
<400> 2
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Val Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Ser Ser Asn Trp Pro Arg
                   85                  90                  95
   Thr Phe Gly Gln Gly Thr Lys Val Glu Ile Lys Arg Thr Val Ala Ala
                   100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 3
   <211> 113
   <212> PRT
   <213> Artificial sequence
<220>
   <223> Heavy Chain Variable Region (VH) Amino Acid Sequence Anti-PD-1 mAb (nivolumab, BMS936558; 5C4 in WO 2006/121168) (SEQ ID NO:4 from WO 2006/121168) (SEQ ID NO:19 IN WO 2014/055648 A1)
<400> 3
<210> 4
   <211> 107
   <212> PRT
   <213> Artificial sequence
<220>
   <223> Light Chain Variable Region (VL) Amino Acid Sequence Anti-PD-1 mAb (nivolumab, BMS936558; 5C4 in WO 2006/121168) (SEQ ID NO:11 from WO 2006/121168) (SEQ ID NO:21 IN WO 2014/055648 A1)
<400> 4
<210> 5
   <211> 5
   <212> PRT
   <213> Artificial sequence
<220>
   <223> Heavy Chain CDR1 Amino Acid Sequence Anti-PD-1 mAb (nivolumab, BMS936558; 5C4 in WO 2006/121168) (SEQ ID NO:18 from WO 2006/121168) (SEQ ID NO:23 from WO 2014/055648 A1))
<400> 5
   Asn Ser Gly Met His
   1 5
<210> 6
   <211> 17
   <212> PRT
   <213> Artificial sequence
<220>
   <223> Heavy Chain CDR2 Amino Acid Sequence Anti-PD-1 mAb (nivolumab, BMS936558; 5C4 in WO 2006/121168) (SEQ ID NO:25 from WO 2006/121168) (SEQ ID NO:24 from WO 2014/055648 A1)
<400> 6
   Val Ile Trp Tyr Asp Gly Ser Lys Arg Tyr Tyr Ala Asp Ser Val Lys
   1 5 10 15
   Gly
<210> 7
   <211> 4
   <212> PRT
   <213> Artificial sequence
<220>
   <223> Heavy Chain CDR3 Amino Acid Sequence Anti-PD-1 mAb (nivolumab, BMS936558; 5C4 in WO 2006/121168) (SEQ ID NO:32 from WO 2006/121168) (SEQ ID NO:25 from WO 2014/055648 A1)
<400> 7
   Asn Asp Asp Tyr
   1
<210> 8
   <211> 11
   <212> PRT
   <213> Artificial sequence
<220>
   <223> Light Chain CDR1 Amino Acid Sequence Anti-PD-1 mAb (nivolumab, BMS936558; 5C4 in WO 2006/121168) (SEQ ID NO:39 from WO 2006/121168) (SEQ ID NO:26 from WO 2014/055648 A1)
<400> 8
   Arg Ala Ser Gln Ser Val Ser Ser Tyr Leu Ala
   1 5 10
<210> 9
   <211> 7
   <212> PRT
   <213> Artificial sequence
<220>
   <223> Light Chain CDR2 Amino Acid Sequence Anti-PD-1 mAb (nivolumab, BMS936558; 5C4 in WO 2006/121168) (SEQ ID NO:46 from WO 2006/121168) (SEQ ID NO:27 from WO 2014/055648 A1)
<400> 9
   Asp Ala Ser Asn Arg Ala Thr
   1               5
<210> 10
   <211> 9
   <212> PRT
   <213> Artificial sequence
<220>
   <223> Light Chain CDR3 Amino Acid Sequence Anti-PD-1 mAb (nivolumab, BMS936558; 5C4 in WO 2006/121168) (SEQ ID NO:53 from WO 2006/121168) (SEQ ID NO:28 from WO 2014/055648 A1)
<400> 10
   Gln Gln Ser Ser Asn Trp Pro Arg Thr
   1               5
<210> 11
   <211> 466
   <212> PRT
   <213> Artificial sequence
<220>
   <223> pembrolizumab 409A-H heavy chain full length (SEQ ID NO:31 from US Patent No. 8,354,509 B2)
<400> 11
   Met Ala Val Leu Gly Leu Leu Phe Cys Leu Val Thr Phe Pro Ser Cys
   1               5                   10                  15
   Val Leu Ser Gln Val Gln Leu Val Gln Ser Gly Val Glu Val Lys Lys
               20                  25                  30
   Pro Gly Ala Ser Val Lys Val Ser Cys Lys Ala Ser Gly Tyr Thr Phe
           35                  40                  45
   Thr Asn Tyr Tyr Met Tyr Trp Val Arg Gln Ala Pro Gly Gln Gly Leu
       50                  55                  60
   Glu Trp Met Gly Gly Ile Asn Pro Ser Asn Gly Gly Thr Asn Phe Asn
   65                  70                  75                  80
   Glu Lys Phe Lys Asn Arg Val Thr Leu Thr Thr Asp Ser Ser Thr Thr
                   85                  90                  95
   Thr Ala Tyr Met Glu Leu Lys Ser Leu Gln Phe Asp Asp Thr Ala Val
               100                 105                 110
   Tyr Tyr Cys Ala Arg Arg Asp Tyr Arg Phe Asp Met Gly Phe Asp Tyr
   115 120 125
   Trp Gly Gln Gly Thr Thr Val Thr Val Ser Ser Ala Ser Thr Lys Gly
   130 135 140
   Pro Ser Val Phe Pro Leu Ala Pro Cys Ser Arg Ser Thr Ser Glu Ser
   145 150 155 160
   Thr Ala Ala Leu Gly Cys Leu Val Lys Asp Tyr Phe Pro Glu Pro Val
   165 170 175
   Thr Val Ser Trp Asn Ser Gly Ala Leu Thr Ser Gly Val His Thr Phe
   180 185 190
   Pro Ala Val Leu Gln Ser Ser Gly Leu Tyr Ser Leu Ser Ser Val Val
   195 200 205
   Thr Val Pro Ser Ser Ser Leu Gly Thr Lys Thr Tyr Thr Cys Asn Val
   210 215 220
   Asp His Lys Pro Ser Asn Thr Lys Val Asp Lys Arg Val Glu Ser Lys
   225 230 235 240
   Tyr Gly Pro Pro Cys Pro Pro Cys Pro Ala Pro Glu Phe Leu Gly Gly
   245 250 255
   Pro Ser Val Phe Leu Phe Pro Pro Lys Pro Lys Asp Thr Leu Met Ile
   260 265 270
   Ser Arg Thr Pro Glu Val Thr Cys Val Val Val Asp Val Ser Gln Glu
   275 280 285
   Asp Pro Glu Val Gln Phe Asn Trp Tyr Val Asp Gly Val Glu Val His
   290 295 300
   Asn Ala Lys Thr Lys Pro Arg Glu Glu Gln Phe Asn Ser Thr Tyr Arg
   305 310 315 320
   Val Val Ser Val Leu Thr Val Leu His Gln Asp Trp Leu Asn Gly Lys
   325 330 335
   Glu Tyr Lys Cys Lys Val Ser Asn Lys Gly Leu Pro Ser Ser Ile Glu
   340 345 350
   Lys Thr Ile Ser Lys Ala Lys Gly Gln Pro Arg Glu Pro Gln Val Tyr
   355 360 365
   Thr Leu Pro Pro Ser Gln Glu Glu Met Thr Lys Asn Gln Val Ser Leu
   370 375 380
   Thr Cys Leu Val Lys Gly Phe Tyr Pro Ser Asp Ile Ala Val Glu Trp
   385 390 395 400
   Glu Ser Asn Gly Gln Pro Glu Asn Asn Tyr Lys Thr Thr Pro Pro Val
   405 410 415
   Leu Asp Ser Asp Gly Ser Phe Phe Leu Tyr Ser Arg Leu Thr Val Asp
   420 425 430
   Lys Ser Arg Trp Gln Glu Gly Asn Val Phe Ser Cys Ser Val Met His
   435 440 445
   Glu Ala Leu His Asn His Tyr Thr Gln Lys Ser Leu Ser Leu Ser Leu
   450 455 460
   Gly Lys
   465
<210> 12
   <211> 447
   <212> PRT
   <213> Artificial sequence
<220>
   <223> pembrolizumab heavy chain
<400> 12
   Gln Val Gln Leu Val Gln Ser Gly Val Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Tyr Met Tyr Trp Val Arg Gln Ala Pro Gly Gln Gly Leu Glu Trp Met
           35                  40                  45
   Gly Gly Ile Asn Pro Ser Asn Gly Gly Thr Asn Phe Asn Glu Lys Phe
       50                  55                  60
   Lys Asn Arg Val Thr Leu Thr Thr Asp Ser Ser Thr Thr Thr Ala Tyr
   65                  70                  75                  80
   Met Glu Leu Lys Ser Leu Gln Phe Asp Asp Thr Ala Val Tyr Tyr Cys
                   85                  90                  95
   Ala Arg Arg Asp Tyr Arg Phe Asp Met Gly Phe Asp Tyr Trp Gly Gln
   100 105 110
   Gly Thr Thr Val Thr Val Ser Ser Ala Ser Thr Lys Gly Pro Ser Val
   115 120 125
   Phe Pro Leu Ala Pro Cys Ser Arg Ser Thr Ser Glu Ser Thr Ala Ala
   130 135 140
   Leu Gly Cys Leu Val Lys Asp Tyr Phe Pro Glu Pro Val Thr Val Ser
   145 150 155 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Lys Thr Tyr Thr Cys Asn Val Asp His Lys
   195 200 205
   Pro Ser Asn Thr Lys Val Asp Lys Arg Val Glu Ser Lys Tyr Gly Pro
   210 215 220
   Pro Cys Pro Pro Cys Pro Ala Pro Glu Phe Leu Gly Gly Pro Ser Val
   225 230 235 240
   Phe Leu Phe Pro Pro Lys Pro Lys Asp Thr Leu Met Ile Ser Arg Thr
   245 250 255
   Pro Glu Val Thr Cys Val Val Val Asp Val Ser Gln Glu Asp Pro Glu
   260 265 270
   Val Gln Phe Asn Trp Tyr Val Asp Gly Val Glu Val His Asn Ala Lys
   275 280 285
   Thr Lys Pro Arg Glu Glu Gln Phe Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Gly Leu Pro Ser Ser Ile Glu Lys Thr Ile
   325 330 335
   Ser Lys Ala Lys Gly Gln Pro Arg Glu Pro Gln Val Tyr Thr Leu Pro
   340 345 350
   Pro Ser Gln Glu Glu Met Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Arg Leu Thr Val Asp Lys Ser Arg
   405 410 415
   Trp Gln Glu Gly Asn Val Phe Ser Cys Ser Val Met His Glu Ala Leu
   420 425 430
   His Asn His Tyr Thr Gln Lys Ser Leu Ser Leu Ser Leu Gly Lys
   435 440 445
<210> 13
   <211> 130
   <212> PRT
   <213> Artificial sequence
<220>
   <223> pembrolizumab K09A-L-11 light chain variable region (SEQ ID NO:32 from US Patent No. 8,354,509 B2)
<400> 13
   Met Ala Pro Val Gln Leu Leu Gly Leu Leu Val Leu Phe Leu Pro Ala
   1               5                   10                  15
   Met Arg Cys Glu Ile Val Leu Thr Gln Ser Pro Ala Thr Leu Ser Leu
               20                  25                  30
   Ser Pro Gly Glu Arg Ala Thr Leu Ser Cys Arg Ala Ser Lys Gly Val
           35                  40                  45
   Ser Thr Ser Gly Tyr Ser Tyr Leu His Trp Tyr Gln Gln Lys Pro Gly
       50                  55                  60
   Gln Ala Pro Arg Leu Leu Ile Tyr Leu Ala Ser Tyr Leu Glu Ser Gly
   65                  70                  75                  80
   Val Pro Ala Arg Phe Ser Gly Ser Gly Ser Gly Thr Asp Phe Thr Leu
                   85                  90                  95
   Thr Ile Ser Ser Leu Glu Pro Glu Asp Phe Ala Val Tyr Tyr Cys Gln
               100                 105                 110
   His Ser Arg Asp Leu Pro Leu Thr Phe Gly Gly Gly Thr Lys Val Glu
           115                 120                 125
   Ile Lys
       130
<210> 14
   <211> 237
   <212> PRT
   <213> Artificial sequence
<220>
   <223> pembrolizumab K09A-L-11 light chain (SEQ ID NO:36 from US Patent No. 8,354,509 B2)
<400> 14
   Met Ala Pro Val Gln Leu Leu Gly Leu Leu Val Leu Phe Leu Pro Ala
   1               5                   10                  15
   Met Arg Cys Glu Ile Val Leu Thr Gln Ser Pro Ala Thr Leu Ser Leu
               20                  25                  30
   Ser Pro Gly Glu Arg Ala Thr Leu Ser Cys Arg Ala Ser Lys Gly Val
           35                  40                  45
   Ser Thr Ser Gly Tyr Ser Tyr Leu His Trp Tyr Gln Gln Lys Pro Gly
       50                  55                  60
   Gln Ala Pro Arg Leu Leu Ile Tyr Leu Ala Ser Tyr Leu Glu Ser Gly
   65                  70                  75                  80
   Val Pro Ala Arg Phe Ser Gly Ser Gly Ser Gly Thr Asp Phe Thr Leu
                   85                  90                  95
   Thr Ile Ser Ser Leu Glu Pro Glu Asp Phe Ala Val Tyr Tyr Cys Gln
               100                 105                 110
   His Ser Arg Asp Leu Pro Leu Thr Phe Gly Gly Gly Thr Lys Val Glu
           115                 120                 125
   Ile Lys Arg Thr Val Ala Ala Pro Ser Val Phe Ile Phe Pro Pro Ser
       130                 135                 140
   Asp Glu Gln Leu Lys Ser Gly Thr Ala Ser Val Val Cys Leu Leu Asn
   145                 150                 155                 160
   Asn Phe Tyr Pro Arg Glu Ala Lys Val Gln Trp Lys Val Asp Asn Ala
                   165                 170                 175
   Leu Gln Ser Gly Asn Ser Gln Glu Ser Val Thr Glu Gln Asp Ser Lys
               180                 185                 190
   Asp Ser Thr Tyr Ser Leu Ser Ser Thr Leu Thr Leu Ser Lys Ala Asp
           195                 200                 205
   Tyr Glu Lys His Lys Val Tyr Ala Cys Glu Val Thr His Gln Gly Leu
       210                 215                 220
   Ser Ser Pro Val Thr Lys Ser Phe Asn Arg Gly Glu Cys
   225                 230                 235
<210> 15
   <211> 15
   <212> PRT
   <213> Artificial sequence
<220>
   <223> pembrolizumab hPD-1.09A light chain CDR1 (SEQ ID NO:15 from US Patent No. 8,354,509 B2)
<400> 15
   Arg Ala Ser Lys Gly Val Ser Thr Ser Gly Tyr Ser Tyr Leu His
   1               5                   10                  15
<210> 16
   <211> 7
   <212> PRT
   <213> Artificial sequence
<220>
   <223> pembrolizumab hPD-1.09A light chain CDR2 (SEQ ID NO:16 from US Patent No. 8,354,509 B2)
<400> 16
   Leu Ala Ser Tyr Leu Glu Ser
   1               5
<210> 17
   <211> 9
   <212> PRT
   <213> Artificial sequence
<220>
   <223> pembrolizumab hPD-1.09A light chain CDR3 (SEQ ID NO:17 from US Patent No. 8,354,509 B2)
<400> 17
   Gln His Ser Arg Asp Leu Pro Leu Thr
   1               5
<210> 18
   <211> 5
   <212> PRT
   <213> Artificial sequence
<220>
   <223> pembrolizumab hPD-1.09A heavy chain CDR1 (SEQ ID NO:18 from US Patent No. 8,354,509 B2)
<400> 18
   Asn Tyr Tyr Met Tyr
   1               5
<210> 19
   <211> 16
   <212> PRT
   <213> Artificial sequence
<220>
   <223> pembrolizumab hPD-1.09A heavy chain CDR2 (SEQ ID NO:19 from US Patent No. 8,354,509 B2)
<400> 19
   Gly Ile Asn Pro Ser Asn Gly Gly Thr Asn Phe Asn Glu Lys Phe Lys
   1               5                   10                  15
<210> 20
   <211> 11
   <212> PRT
   <213> Artificial sequence
<220>
   <223> pembrolizumab hPD-1.09A heavy chain CDR3 (SEQ ID NO:20 from US Patent No. 8,354,509 B2)
<400> 20
   Arg Asp Tyr Arg Phe Asp Met Gly Phe Asp Tyr
   1               5                   10
<210> 21
   <211> 447
   <212> PRT
   <213> Artificial sequence
<220>
   <223> pidilizumab heavy chain
<400> 21
   Gln Val Gln Leu Val Gln Ser Gly Ser Glu Leu Lys Lys Pro Gly Ala
   1               5                   10                  15
   Ser Val Lys Ile Ser Cys Lys Ala Ser Gly Tyr Thr Phe Thr Asn Tyr
               20                  25                  30
   Gly Met Asn Trp Val Arg Gln Ala Pro Gly Gln Gly Leu Gln Trp Met
           35                  40                  45
   Gly Trp Ile Asn Thr Asp Ser Gly Glu Ser Thr Tyr Ala Glu Glu Phe
       50                  55                  60
   Lys Gly Arg Phe Val Phe Ser Leu Asp Thr Ser Val Asn Thr Ala Tyr
   65                  70                  75                  80
   Leu Gln Ile Thr Ser Leu Thr Ala Glu Asp Thr Gly Met Tyr Phe Cys
                   85                  90                  95
   Val Arg Val Gly Tyr Asp Ala Leu Asp Tyr Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Arg Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Ala Leu Pro Ala Pro Ile Glu Lys Thr Ile
   325 330 335
   Ser Lys Ala Lys Gly Gln Pro Arg Glu Pro Gln Val Tyr Thr Leu Pro
   340 345 350
   Pro Ser Arg Glu Glu Met Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 22
   <211> 213
   <212> PRT
   <213> Artificial sequence
<220>
   <223> pidilizumab light chain
<400> 22
   Glu Ile Val Leu Thr Gln Ser Pro Ser Ser Leu Ser Ala Ser Val Gly
   1 5 10 15
   Asp Arg Val Thr Ile Thr Cys Ser Ala Arg Ser Ser Val Ser Tyr Met
   20 25 30
   His Trp Phe Gln Gln Lys Pro Gly Lys Ala Pro Lys Leu Trp Ile Tyr
   35 40 45
   Arg Thr Ser Asn Leu Ala Ser Gly Val Pro Ser Arg Phe Ser Gly Ser
       50                  55                  60
   Gly Ser Gly Thr Ser Tyr Cys Leu Thr Ile Asn Ser Leu Gln Pro Glu
   65                  70                  75                  80
   Asp Phe Ala Thr Tyr Tyr Cys Gln Gln Arg Ser Ser Phe Pro Leu Thr
                   85                  90                  95
   Phe Gly Gly Gly Thr Lys Leu Glu Ile Lys Arg Thr Val Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn Arg Gly Glu Cys
       210
<210> 23
   <211> 117
   <212> PRT
   <213> Artificial sequence
<220>
   <223> variable heavy chain region of the PD-1 inhibitor pidilizumab (corresponding to SEQ ID NO:5 in U.S. Patent No. 8,686,119 B2)
<400> 23
   Gln Val Gln Leu Val Gln Ser Gly Ser Glu Leu Lys Lys Pro Gly Ala
   1               5                   10                  15
   Ser Val Lys Ile Ser Cys Lys Ala Ser Gly Tyr Thr Phe Thr Asn Tyr
               20                  25                  30
   Gly Met Asn Trp Val Arg Gln Ala Pro Gly Gln Gly Leu Gln Trp Met
   35 40 45
   Gly Trp Ile Asn Thr Asp Ser Gly Glu Ser Thr Tyr Ala Glu Glu Phe
   50 55 60
   Lys Gly Arg Phe Val Phe Ser Leu Asp Thr Ser Val Asn Thr Ala Tyr
   65 70 75 80
   Leu Gln Ile Thr Ser Leu Thr Ala Glu Asp Thr Gly Met Tyr Phe Cys
   85 90 95
   Val Arg Val Gly Tyr Asp Ala Leu Asp Tyr Trp Gly Gln Gly Thr Leu
   100 105 110
   Val Thr Val Ser Ser
   115
<210> 24
   <211> 106
   <212> PRT
   <213> Artificial sequence
<220>
   <223> variable light chain region of the PD-1 inhibitor pidilizumab (corresponding to SEQ ID NO:5 in U.S. Patent No. 8,686,119 B2)
<400> 24
   Glu Ile Val Leu Thr Gln Ser Pro Ser Ser Leu Ser Ala Ser Val Gly
   1 5 10 15
   Asp Arg Val Thr Ile Thr Cys Ser Ala Arg Ser Ser Val Ser Tyr Met
   20 25 30
   His Trp Phe Gln Gln Lys Pro Gly Lys Ala Pro Lys Leu Trp Ile Tyr
   35 40 45
   Arg Thr Ser Asn Leu Ala Ser Gly Val Pro Ser Arg Phe Ser Gly Ser
   50 55 60
   Gly Ser Gly Thr Ser Tyr Cys Leu Thr Ile Asn Ser Leu Gln Pro Glu
   65 70 75 80
   Asp Phe Ala Thr Tyr Tyr Cys Gln Gln Arg Ser Ser Phe Pro Leu Thr
   85 90 95
   Phe Gly Gly Gly Thr Lys Leu Glu Ile Lys
   100 105
<210> 25
   <211> 7
   <212> PRT
   <213> Homo sapiens
<220>
   <221> PEPTIDE
   <222> (1)..(7)
   <223> BC loop
<400> 25
   Ser Asn Thr Ser Glu Ser Phe
   1               5
<210> 26
   <211> 22
   <212> PRT
   <213> Artificial Sequence
<220>
   <223> Branched peptide at LYS8 with SEQ ID NO:25
<220>
   <221> SITE
   <222> (1)..(22)
   <223> Branched amino acid sequence, SEQ ID NO:25 attached to the Lys8 residue
<400> 26
   Ser Asn Thr Ser Glu Ser Phe Lys Phe Arg Val Thr Gln Leu Ala Pro
   1               5                   10                  15
   Lys Ala Gln Ile Lys Glu
               20
<210> 27
   <211> 22
   <212> PRT
   <213> Artificial Sequence
<220>
   <223> Branched peptide Sequence with NH2 modified C terminal and branching at Lys8 and a lipid attached to Lys 17
<220>
   <221> SITE
   <222> (1)..(22)
   <223> Branch amino acid SEQ ID NO:25 attached to the Lys8 residue
<220>
   <221> SITE
   <222> (1)..(22)
   <223> C16 lipid attached to Lys 17
<400> 27
   Ser Asn Thr Ser Glu Ser Phe Lys Phe Arg Val Thr Gln Leu Ala Pro
   1               5                   10                  15
   Lys Ala Gln Ile Lys Glu
               20
<210> 28
   <211> 22
   <212> PRT
   <213> Artificial Sequence
<220>
   <223> Branched peptide sequence with NH2 modified C terminal and with two branches
<220>
   <221> SITE
   <222> (1)..(22)
   <223> First branch amino acid SEQ ID NO:25 attached to the Lys8 residue
<220>
   <221> SITE
   <222> (1)..(22)
   <223> Second branch MPA-NH-CH2-CH2-O-CH2-CH2-O-CO attached to the Lys17 residue
<400> 28
   Ser Asn Thr Ser Glu Ser Phe Lys Phe Arg Val Thr Gln Leu Ala Pro
   1               5                   10                  15
   Lys Ala Gln Ile Lys Glu
               20
<210> 29
   <211> 22
   <212> PRT
   <213> Artificial Sequence
<220>
   <223> Branched peptide Sequence with NH2 modified C terminal and branching at Lys8 residue and a lipid attached to Lys 21
<220>
   <221> SITE
   <222> (1)..(22)
   <223> Branch amino acid sequence SEQ ID NO:25 attached to the Lys8 residue
<220>
   <221> SITE
   <222> (1)..(22)
   <223> C16 lipid attached to Lys 21
<400> 29
   Ser Asn Thr Ser Glu Ser Phe Lys Phe Arg Val Thr Gln Leu Ala Pro
   1 5 10 15
   Lys Ala Gln Ile Lys Glu
   20
<210> 30
   <211> 451
   <212> PRT
   <213> Artificial sequence
<220>
   <223> durvalumab (MEDI4736) heavy chain
<400> 30
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Arg Tyr
   20 25 30
   Trp Met Ser Trp Val Arg Gln Ala Pro Gly Lys Gly Leu Glu Trp Val
   35 40 45
   Ala Asn Ile Lys Gln Asp Gly Ser Glu Lys Tyr Tyr Val Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Arg Glu Gly Gly Trp Phe Gly Glu Leu Ala Phe Asp Tyr Trp Gly
   100 105 110
   Gln Gly Thr Leu Val Thr Val Ser Ser Ala Ser Thr Lys Gly Pro Ser
   115 120 125
   Val Phe Pro Leu Ala Pro Ser Ser Lys Ser Thr Ser Gly Gly Thr Ala
   130 135 140
   Ala Leu Gly Cys Leu Val Lys Asp Tyr Phe Pro Glu Pro Val Thr Val
   145 150 155 160
   Ser Trp Asn Ser Gly Ala Leu Thr Ser Gly Val His Thr Phe Pro Ala
   165 170 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Arg Val Glu Pro Lys Ser Cys
       210                 215                 220
   Asp Lys Thr His Thr Cys Pro Pro Cys Pro Ala Pro Glu Phe Glu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Ser Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Glu Glu Met Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
   435 440 445
   Pro Gly Lys
   450
<210> 31
   <211> 265
   <212> PRT
   <213> Artificial sequence
<220>
   <223> durvalumab (MEDI4736) light chain
<400> 31
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Arg Tyr
   20 25 30
   Trp Met Ser Trp Val Arg Gln Ala Pro Gly Lys Gly Leu Glu Trp Val
   35 40 45
   Ala Asn Glu Ile Val Leu Thr Gln Ser Pro Gly Thr Leu Ser Leu Ser
   50 55 60
   Pro Gly Glu Arg Ala Thr Leu Ser Cys Arg Ala Ser Gln Arg Val Ser
   65 70 75 80
   Ser Ser Tyr Leu Ala Trp Tyr Gln Gln Lys Pro Gly Gln Ala Pro Arg
   85 90 95
   Leu Leu Ile Tyr Asp Ala Ser Ser Arg Ala Thr Gly Ile Pro Asp Arg
   100 105 110
   Phe Ser Gly Ser Gly Ser Gly Thr Asp Phe Thr Leu Thr Ile Ser Arg
   115 120 125
   Leu Glu Pro Glu Asp Phe Ala Val Tyr Tyr Cys Gln Gln Tyr Gly Ser
   130 135 140
   Leu Pro Trp Thr Phe Gly Gln Gly Thr Lys Val Glu Ile Lys Arg Thr
   145 150 155 160
   Val Ala Ala Pro Ser Val Phe Ile Phe Pro Pro Ser Asp Glu Gln Leu
   165 170 175
   Lys Ser Gly Thr Ala Ser Val Val Cys Leu Leu Asn Asn Phe Tyr Pro
   180 185 190
   Arg Glu Ala Lys Val Gln Trp Lys Val Asp Asn Ala Leu Gln Ser Gly
   195 200 205
   Asn Ser Gln Glu Ser Val Thr Glu Gln Asp Ser Lys Asp Ser Thr Tyr
   210 215 220
   Ser Leu Ser Ser Thr Leu Thr Leu Ser Lys Ala Asp Tyr Glu Lys His
   225 230 235 240
   Lys Val Tyr Ala Cys Glu Val Thr His Gln Gly Leu Ser Ser Pro Val
   245 250 255
   Thr Lys Ser Phe Asn Arg Gly Glu Cys
   260 265
<210> 32
   <211> 121
   <212> PRT
   <213> Artificial sequence
<220>
   <223> durvalumab (MEDI4736) heavy chain variable region, SEQ ID NO:72 from US2013/0034559A1
<400> 32
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Arg Tyr
   20 25 30
   Trp Met Ser Trp Val Arg Gln Ala Pro Gly Lys Gly Leu Glu Trp Val
   35 40 45
   Ala Asn Ile Lys Gln Asp Gly Ser Glu Lys Tyr Tyr Val Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Arg Glu Gly Gly Trp Phe Gly Glu Leu Ala Phe Asp Tyr Trp Gly
   100 105 110
   Gln Gly Thr Leu Val Thr Val Ser Ser
   115 120
<210> 33
   <211> 108
   <212> PRT
   <213> Artificial sequence
<220>
   <223> durvalumab (MEDI4736) light chain variable region, SEQ ID NO:77 from US2013/0034559A1
<400> 33
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Arg Val Ser Ser Ser
   20 25 30
   Tyr Leu Ala Trp Tyr Gln Gln Lys Pro Gly Gln Ala Pro Arg Leu Leu
   35 40 45
   Ile Tyr Asp Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Tyr Gly Ser Leu Pro
   85 90 95
   Trp Thr Phe Gly Gln Gly Thr Lys Val Glu Ile Lys
   100 105
<210> 34
   <211> 5
   <212> PRT
   <213> Artificial sequence
<220>
   <223> durvalumab (MEDI4736) heavy chain variable region CDR1, SEQ ID NO:23 from US2013/0034559A1
<400> 34
   Arg Tyr Trp Met Ser
   1 5
<210> 35
   <211> 17
   <212> PRT
   <213> Artificial sequence
<220>
   <223> durvalumab (MEDI4736) heavy chain variable region CDR2, SEQ ID NO:24 from US2013/0034559A1
<400> 35
   Asn Ile Lys Gln Asp Gly Ser Glu Lys Tyr Tyr Val Asp Ser Val Lys
   1               5                   10                  15
   Gly
<210> 36
   <211> 12
   <212> PRT
   <213> Artificial sequence
<220>
   <223> durvalumab (MEDI4736) heavy chain variable region CDR3, SEQ ID NO:25 from US2013/0034559A1
<400> 36
   Glu Gly Gly Trp Phe Gly Glu Leu Ala Phe Asp Tyr
   1               5                   10
<210> 37
   <211> 12
   <212> PRT
   <213> Artificial sequence
<220>
   <223> durvalumab (MEDI4736) light chain variable region CDR1, SEQ ID NO:28 from US2013/0034559A1
<400> 37
   Arg Ala Ser Gln Arg Val Ser Ser Ser Tyr Leu Ala
   1               5                   10
<210> 38
   <211> 7
   <212> PRT
   <213> Artificial sequence
<220>
   <223> durvalumab (MEDI4736) light chain variable region CDR2, SEQ ID NO:29 from US2013/0034559A1
<400> 38
   Asp Ala Ser Ser Arg Ala Thr
   1               5
<210> 39
   <211> 9
   <212> PRT
   <213> Artificial sequence
<220>
   <223> durvalumab (MEDI4736) light chain variable region CDR3, SEQ ID NO:30 from US2013/0034559A1
<400> 39
   Gln Gln Tyr Gly Ser Leu Pro Trp Thr
   1               5
<210> 40
   <211> 5
   <212> PRT
   <213> Artificial sequence
<220>
   <223> durvalumab (MEDI4736) alternative heavy chain variable region CDR1, SEQ ID NO:3 from US2013/0034559A1
<400> 40
   Thr Tyr Ser Met Asn
   1               5
<210> 41
   <211> 17
   <212> PRT
   <213> Artificial sequence
<220>
   <223> durvalumab (MEDI4736) alternative heavy chain variable region CDR2, SEQ ID NO:4 from US2013/0034559A1
<400> 41
   Ser Ile Ser Ser Ser Gly Asp Tyr Ile Tyr Tyr Ala Asp Ser Val Lys
   1               5                   10                  15
   Gly
<210> 42
   <211> 11
   <212> PRT
   <213> Artificial sequence
<220>
   <223> durvalumab (MEDI4736) alternative heavy chain variable region CDR3, SEQ ID NO:5 from US2013/0034559A1
<400> 42
   Asp Leu Val Thr Ser Met Val Ala Phe Asp Tyr
   1               5                   10
<210> 43
   <211> 11
   <212> PRT
   <213> Artificial sequence
<220>
   <223> durvalumab (MEDI4736) alternative light chain variable region CDR1, SEQ ID NO:8 from US2013/0034559A1
<400> 43
   Ser Gly Asp Ala Leu Pro Gln Lys Tyr Val Phe
   1               5                   10
<210> 44
   <211> 7
   <212> PRT
   <213> Artificial sequence
<220>
   <223> durvalumab (MEDI4736) alternative light chain variable region CDR2, SEQ ID NO:9 from US2013/0034559A1
<400> 44
   Glu Asp Ser Lys Arg Pro Ser
   1               5
<210> 45
   <211> 11
   <212> PRT
   <213> Artificial sequence
<220>
   <223> durvalumab (MEDI4736) alternative light chain variable region CDR3, SEQ ID NO:10 from US2013/0034559A1
<400> 45
   Tyr Ser Thr Asp Arg Ser Gly Asn His Arg Val
   1               5                   10
<210> 46
   <211> 5
   <212> PRT
   <213> Artificial sequence
<220>
   <223> durvalumab (MEDI4736) alternative heavy chain variable region CDR1, SEQ ID NO:13 from US2013/0034559A1
<400> 46
   Ser Tyr Trp Met Ser
   1               5
<210> 47
   <211> 17
   <212> PRT
   <213> Artificial sequence
<220>
   <223> durvalumab (MEDI4736) alternative heavy chain variable region CDR2, SEQ ID NO:14 from US2013/0034559A1
<400> 47
   Asn Ile Lys Gln Asp Gly Gly Glu Gln Tyr Tyr Val Asp Ser Val Lys
   1 5 10 15
   Gly
<210> 48
   <211> 11
   <212> PRT
   <213> Artificial sequence
<220>
   <223> durvalumab (MEDI4736) alternative heavy chain variable region CDR3, SEQ ID NO:15 from US2013/0034559A1
<400> 48
   Asp Trp Asn Tyr Gly Tyr Tyr Asp Met Asp Val
   1 5 10
<210> 49
   <211> 12
   <212> PRT
   <213> Artificial sequence
<220>
   <223> durvalumab (MEDI4736) alternative light chain variable region CDR1, SEQ ID NO:18 from US2013/0034559A1
<400> 49
   Arg Ala Ser Gln Ser Val Ser Ser Asn Tyr Leu Ala
   1 5 10
<210> 50
   <211> 7
   <212> PRT
   <213> Artificial sequence
<220>
   <223> durvalumab (MEDI4736) alternative light chain variable region CDR2, SEQ ID NO:19 from US2013/0034559A1
<400> 50
   Gly Thr Ser Ser Arg Ala Thr
   1 5
<210> 51
   <211> 9
   <212> PRT
   <213> Artificial sequence
<220>
   <223> durvalumab (MEDI4736) alternative light chain variable region CDR3, SEQ ID NO:20 from US2013/0034559A1
<400> 51
   Gln Gln Tyr Gly Ser Ser Ile Phe Thr
   1 5
<210> 52
   <211> 5
   <212> PRT
   <213> Artificial sequence
<220>
   <223> durvalumab (MEDI4736) alternative heavy chain variable region CDR1, SEQ ID NO:63 from US2013/0034559A1
<400> 52
   Thr Tyr Ser Met Asn
   1 5
<210> 53
   <211> 17
   <212> PRT
   <213> Artificial sequence
<220>
   <223> durvalumab (MEDI4736) alternative heavy chain variable region CDR2, SEQ ID NO:64 from US2013/0034559A1
<400> 53
   Ser Ile Ser Ser Ser Gly Asp Tyr Ile Tyr Tyr Ala Asp Ser Val Lys
   1 5 10 15
   Gly
<210> 54
   <211> 11
   <212> PRT
   <213> Artificial sequence
<220>
   <223> durvalumab (MEDI4736) alternative heavy chain variable region CDR3, SEQ ID NO:65 from US2013/0034559A1
<400> 54
   Asp Leu Val Thr Ser Met Val Ala Phe Asp Tyr
   1 5 10
<210> 55
   <211> 11
   <212> PRT
   <213> Artificial sequence
<220>
   <223> durvalumab (MEDI4736) alternative light chain variable region CDR1, SEQ ID NO:68 from US2013/0034559A1
<400> 55
   Ser Gly Asp Ala Leu Pro Gln Lys Tyr Val Phe
   1 5 10
<210> 56
   <211> 7
   <212> PRT
   <213> Artificial sequence
<220>
   <223> durvalumab (MEDI4736) alternative light chain variable region CDR2, SEQ ID NO:69 from US2013/0034559A1
<400> 56
   Glu Asp Ser Lys Arg Pro Ser
   1 5
<210> 57
   <211> 11
   <212> PRT
   <213> Artificial sequence
<220>
   <223> durvalumab (MEDI4736) alternative light chain variable region CDR3, SEQ ID NO:70 from US2013/0034559A1
<400> 57
   Tyr Ser Thr Asp Arg Ser Gly Asn His Arg Val
   1 5 10
<210> 58
   <211> 5
   <212> PRT
   <213> Artificial sequence
<220>
   <223> durvalumab (MEDI4736) alternative heavy chain variable region CDR1, SEQ ID NO:73 from US2013/0034559A1
<400> 58
   Arg Tyr Trp Met Ser
   1 5
<210> 59
   <211> 17
   <212> PRT
   <213> Artificial sequence
<220>
   <223> durvalumab (MEDI4736) alternative heavy chain variable region CDR2, SEQ ID NO:74 from US2013/0034559A1
<400> 59
   Asn Ile Lys Gln Asp Gly Ser Glu Lys Tyr Tyr Val Asp Ser Val Lys
   1 5 10 15
   Gly
<210> 60
   <211> 12
   <212> PRT
   <213> Artificial sequence
<220>
   <223> durvalumab (MEDI4736) alternative heavy chain variable region CDR3, SEQ ID NO:75 from US2013/0034559A1
<400> 60
   Glu Gly Gly Trp Phe Gly Glu Leu Ala Phe Asp Tyr
   1 5 10
<210> 61
   <211> 12
   <212> PRT
   <213> Artificial sequence
<220>
   <223> durvalumab (MEDI4736) alternative light chain variable region CDR1, SEQ ID NO:78 from US2013/0034559A1
<400> 61
   Arg Ala Ser Gln Arg Val Ser Ser Ser Tyr Leu Ala
   1 5 10
<210> 62
   <211> 7
   <212> PRT
   <213> Artificial sequence
<220>
   <223> durvalumab (MEDI4736) alternative light chain variable region CDR2, SEQ ID NO:79 from US2013/0034559A1
<400> 62
   Asp Ala Ser Ser Arg Ala Thr
   1 5
<210> 63
   <211> 9
   <212> PRT
   <213> Artificial sequence
<220>
   <223> durvalumab (MEDI4736) alternative light chain variable region CDR3, SEQ ID NO:80 from US2013/0034559A1
<400> 63
   Gln Gln Tyr Gly Ser Leu Pro Trp Thr
   1 5
<210> 64
   <211> 448
   <212> PRT
   <213> Artificial sequence
<220>
   <223> atezolizumab (MPDL3280A) heavy chain
<400> 64
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Asp Ser
   20 25 30
   Trp Ile His Trp Val Arg Gln Ala Pro Gly Lys Gly Leu Glu Trp Val
   35 40 45
   Ala Trp Ile Ser Pro Tyr Gly Gly Ser Thr Tyr Tyr Ala Asp Ser Val
   50 55 60
   Lys Gly Arg Phe Thr Ile Ser Ala Asp Thr Ser Lys Asn Thr Ala Tyr
   65 70 75 80
   Leu Gln Met Asn Ser Leu Arg Ala Glu Asp Thr Ala Val Tyr Tyr Cys
   85 90 95
   Ala Arg Arg His Trp Pro Gly Gly Phe Asp Tyr Trp Gly Gln Gly Thr
   100 105 110
   Leu Val Thr Val Ser Ser Ala Ser Thr Lys Gly Pro Ser Val Phe Pro
   115 120 125
   Leu Ala Pro Ser Ser Lys Ser Thr Ser Gly Gly Thr Ala Ala Leu Gly
   130 135 140
   Cys Leu Val Lys Asp Tyr Phe Pro Glu Pro Val Thr Val Ser Trp Asn
   145 150 155 160
   Ser Gly Ala Leu Thr Ser Gly Val His Thr Phe Pro Ala Val Leu Gln
   165 170 175
   Ser Ser Gly Leu Tyr Ser Leu Ser Ser Val Val Thr Val Pro Ser Ser
   180 185 190
   Ser Leu Gly Thr Gln Thr Tyr Ile Cys Asn Val Asn His Lys Pro Ser
   195 200 205
   Asn Thr Lys Val Asp Lys Lys Val Glu Pro Lys Ser Cys Asp Lys Thr
   210 215 220
   His Thr Cys Pro Pro Cys Pro Ala Pro Glu Leu Leu Gly Gly Pro Ser
   225 230 235 240
   Val Phe Leu Phe Pro Pro Lys Pro Lys Asp Thr Leu Met Ile Ser Arg
   245 250 255
   Thr Pro Glu Val Thr Cys Val Val Val Asp Val Ser His Glu Asp Pro
   260 265 270
   Glu Val Lys Phe Asn Trp Tyr Val Asp Gly Val Glu Val His Asn Ala
   275 280 285
   Lys Thr Lys Pro Arg Glu Glu Gln Tyr Ala Ser Thr Tyr Arg Val Val
   290 295 300
   Ser Val Leu Thr Val Leu His Gln Asp Trp Leu Asn Gly Lys Glu Tyr
   305 310 315 320
   Lys Cys Lys Val Ser Asn Lys Ala Leu Pro Ala Pro Ile Glu Lys Thr
   325 330 335
   Ile Ser Lys Ala Lys Gly Gln Pro Arg Glu Pro Gln Val Tyr Thr Leu
   340 345 350
   Pro Pro Ser Arg Glu Glu Met Thr Lys Asn Gln Val Ser Leu Thr Cys
   355 360 365
   Leu Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Tyr Ser Lys Leu Thr Val Asp Lys Ser
   405 410 415
   Arg Trp Gln Gln Gly Asn Val Phe Ser Cys Ser Val Met His Glu Ala
   420 425 430
   Leu His Asn His Tyr Thr Gln Lys Ser Leu Ser Leu Ser Pro Gly Lys
   435 440 445
<210> 65
   <211> 214
   <212> PRT
   <213> Artificial sequence
<220>
   <223> atezolizumab (MPDL3280A) light chain
<400> 65
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Val Ser Thr Ala
   20 25 30
   Val Ala Trp Tyr Gln Gln Lys Pro Gly Lys Ala Pro Lys Leu Leu Ile
   35 40 45
   Tyr Ser Ala Ser Phe Leu Tyr Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Leu Tyr His Pro Ala
   85 90 95
   Thr Phe Gly Gln Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 66
   <211> 118
   <212> PRT
   <213> Artificial sequence
<220>
   <223> atezolizumab (MPDL3280A) VH region (corresponds to SEQ ID NO:20 in U.S. Patent No. 8,217,149)
<400> 66
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Asp Ser
   20 25 30
   Trp Ile His Trp Val Arg Gln Ala Pro Gly Lys Gly Leu Glu Trp Val
   35 40 45
   Ala Trp Ile Ser Pro Tyr Gly Gly Ser Thr Tyr Tyr Ala Asp Ser Val
   50 55 60
   Lys Gly Arg Phe Thr Ile Ser Ala Asp Thr Ser Lys Asn Thr Ala Tyr
   65 70 75 80
   Leu Gln Met Asn Ser Leu Arg Ala Glu Asp Thr Ala Val Tyr Tyr Cys
   85 90 95
   Ala Arg Arg His Trp Pro Gly Gly Phe Asp Tyr Trp Gly Gln Gly Thr
   100 105 110
   Leu Val Thr Val Ser Ala
   115
<210> 67
   <211> 108
   <212> PRT
   <213> Artificial sequence
<220>
   <223> atezolizumab (MPDL3280A) VL region (corresponds to SEQ ID NO:21 in U.S. Patent No. 8,217,149)
<400> 67
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Leu Tyr His Pro Ala
                   85                  90                  95
   Thr Phe Gly Gln Gly Thr Lys Val Glu Ile Lys Arg
               100                 105
<210> 68
   <211> 10
   <212> PRT
   <213> Artificial sequence
<220>
   <223> atezolizumab (MPDL3280A) HVR-H1 region (corresponds to SEQ ID NO:1 in U.S. Patent No. 8,217,149)
<220>
   <221> variant
   <222> (6).. (6)
   <223> residue is D or G
<400> 68
   Gly Phe Thr Phe Ser Xaa Ser Trp Ile His
   1               5                   10
<210> 69
   <211> 18
   <212> PRT
   <213> Artificial sequence
<220>
   <223> atezolizumab (MPDL3280A) HVR-H2 region (corresponds to SEQ ID NO:2 in U.S. Patent No. 8,217,149
<220>
   <221> variant
   <222> (4)..(4)
   <223> residue is S or L
<220>
   <221> variant
   <222> (10)..(10)
   <223> residue is T or S
<400> 69
   Ala Trp Ile Xaa Pro Tyr Gly Gly Ser Xaa Tyr Tyr Ala Asp Ser Val
   1               5                   10                  15
   Lys Gly
<210> 70
   <211> 9
   <212> PRT
   <213> Artificial sequence
<220>
   <223> atezolizumab (MPDL3280A) HVR-H3 region (corresponds to SEQ ID NO:3 in U.S. Patent No. 8,217,149
<400> 70
   Arg His Trp Pro Gly Gly Phe Asp Tyr
   1               5
<210> 71
   <211> 11
   <212> PRT
   <213> Artificial sequence
<220>
   <223> atezolizumab (MPDL3280A) HVR-L1 region (corresponds to SEQ ID NO:8 in U.S. Patent No. 8,217,149
<220>
   <221> variant
   <222> (5)..(5)
   <223> residue is D or V
<220>
   <221> variant
   <222> (6).. (6)
   <223> residue is V or I
<220>
   <221> variant
   <222> (7)..(7)
   <223> residue is S or N
<220>
   <221> variant
   <222> (9).. (9)
   <223> residue is A or F
<220>
   <221> variant
   <222> (10)..(10)
   <223> residue is V or L
<400> 71
   Arg Ala Ser Gln Xaa Xaa Xaa Thr Xaa Xaa Ala
   1 5 10
<210> 72
   <211> 7
   <212> PRT
   <213> Artificial sequence
<220>
   <223> atezolizumab (MPDL3280A) HVR-L2 region (corresponds to SEQ ID NO:9 in U.S. Patent No. 8,217,149
<220>
   <221> variant
   <222> (4).. (4)
   <223> residue is F or T
<220>
   <221> variant
   <222> (6).. (6)
   <223> residue is Y or A
<400> 72
   Ser Ala Ser Xaa Leu Xaa Ser
   1 5
<210> 73
   <211> 9
   <212> PRT
   <213> Artificial sequence
<220>
   <223> atezolizumab (MPDL3280A) HVR-L3 region (corresponds to SEQ ID NO:10 in U.S. Patent No. 8,217,149
<220>
   <221> variant
   <222> (3)..(3)
   <223> residue is Y, G, F or S
<220>
   <221> variant
   <222> (4)..(4)
   <223> residue is L, Y, F, or W
<220>
   <221> variant
   <222> (5)..(5)
   <223> residue is Y, N, A, T, G, F or I
<220>
   <221> variant
   <222> (6).. (6)
   <223> residue is H, V, P, T or I
<220>
   <221> variant
   <222> (8)..(8)
   <223> residue is A, W, R, P or T
<400> 73
   Gln Gln Xaa Xaa Xaa Xaa Pro Xaa Thr
   1               5
<210> 74
   <211> 450
   <212> PRT
   <213> Artificial Sequence
<220>
   <223> avelumab (MSB0010718C) heavy chain
<400> 74
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ile Met Met Trp Val Arg Gln Ala Pro Gly Lys Gly Leu Glu Trp Val
           35                  40                  45
   Ser Ser Ile Tyr Pro Ser Gly Gly Ile Thr Phe Tyr Ala Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Ile Lys Leu Gly Thr Val Thr Thr Val Asp Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Gln Thr Tyr Ile Cys Asn Val Asn His Lys
   195 200 205
   Pro Ser Asn Thr Lys Val Asp Lys Lys Val Glu Pro Lys Ser Cys Asp
   210 215 220
   Lys Thr His Thr Cys Pro Pro Cys Pro Ala Pro Glu Leu Leu Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser His Glu
   260 265 270
   Asp Pro Glu Val Lys Phe Asn Trp Tyr Val Asp Gly Val Glu Val His
   275 280 285
   Asn Ala Lys Thr Lys Pro Arg Glu Glu Gln Tyr Asn Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Pro Ala Pro Ile Glu
   325 330 335
   Lys Thr Ile Ser Lys Ala Lys Gly Gln Pro Arg Glu Pro Gln Val Tyr
   340 345 350
   Thr Leu Pro Pro Ser Arg Asp Glu Leu Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Lys
   450
<210> 75
   <211> 216
   <212> PRT
   <213> Artificial Sequence
<220>
   <223> avelumab (MSB0010718C) light chain
<400> 75
   Gln Ser Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Gly Tyr
               20                  25                  30
   Asn Tyr Val Ser Trp Tyr Gln Gln His Pro Gly Lys Ala Pro Lys Leu
           35                  40                  45
   Met Ile Tyr Asp Val Ser Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Ser Tyr Thr Ser Ser
                   85                  90                  95
   Ser Thr Arg Val Phe Gly Thr Gly Thr Lys Val Thr Val Leu Gly Gln
               100                 105                 110
   Pro Lys Ala Asn Pro Thr Val Thr Leu Phe Pro Pro Ser Ser Glu Glu
           115                 120                 125
   Leu Gln Ala Asn Lys Ala Thr Leu Val Cys Leu Ile Ser Asp Phe Tyr
       130                 135                 140
   Pro Gly Ala Val Thr Val Ala Trp Lys Ala Asp Gly Ser Pro Val Lys
   145                 150                 155                 160
   Ala Gly Val Glu Thr Thr Lys Pro Ser Lys Gln Ser Asn Asn Lys Tyr
                   165                 170                 175
   Ala Ala Ser Ser Tyr Leu Ser Leu Thr Pro Glu Gln Trp Lys Ser His
               180                 185                 190
   Arg Ser Tyr Ser Cys Gln Val Thr His Glu Gly Ser Thr Val Glu Lys
           195                 200                 205
   Thr Val Ala Pro Thr Glu Cys Ser
       210                 215
<210> 76
   <211> 120
   <212> PRT
   <213> Artificial Sequence
<220>
   <223> avelumab (MSB0010718C) VH region (corresponding to SEQ ID NO:24 in U.S. Patent Application Publication No. US 2014/0341917 A1).
<400> 76
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ile Met Met Trp Val Arg Gln Ala Pro Gly Lys Gly Leu Glu Trp Val
           35                  40                  45
   Ser Ser Ile Tyr Pro Ser Gly Gly Ile Thr Phe Tyr Ala Asp Thr Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Ile Lys Leu Gly Thr Val Thr Thr Val Asp Tyr Trp Gly Gln
               100                 105                 110
   Gly Thr Leu Val Thr Val Ser Ser
           115                 120
<210> 77
   <211> 110
   <212> PRT
   <213> Artificial Sequence
<220>
   <223> avelumab (MSB0010718C) VL region (corresponding to SEQ ID NO:25 in U.S. Patent Application Publication No. US 2014/0341917 A1).
<400> 77
   Gln Ser Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Gly Tyr
               20                  25                  30
   Asn Tyr Val Ser Trp Tyr Gln Gln His Pro Gly Lys Ala Pro Lys Leu
           35                  40                  45
   Met Ile Tyr Asp Val Ser Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Ser Tyr Thr Ser Ser
                   85                  90                  95
   Ser Thr Arg Val Phe Gly Thr Gly Thr Lys Val Thr Val Leu
               100                 105                 110
<210> 78
   <211> 5
   <212> PRT
   <213> Artificial Sequence
<220>
   <223> avelumab (MSB0010718C) HVR-H1 region (corresponding to SEQ ID NO:15 in U.S. Patent Application Publication No. US 2014/0341917 A1).
<400> 78
   Ser Tyr Ile Met Met
   1               5
<210> 79
   <211> 17
   <212> PRT
   <213> Artificial Sequence
<220>
   <223> avelumab (MSB0010718C) HVR-H2 region (corresponding to SEQ ID NO:16 in U.S. Patent Application Publication No. US 2014/0341917 A1).
<400> 79
   Ser Ile Tyr Pro Ser Gly Gly Ile Thr Phe Tyr Ala Asp Thr Val Lys
   1               5                   10                  15
   Gly
<210> 80
   <211> 11
   <212> PRT
   <213> Artificial Sequence
<220>
   <223> avelumab (MSB0010718C) HVR-H3 region (corresponding to SEQ ID NO:17 in U.S. Patent Application Publication No. US 2014/0341917 A1).
<400> 80
   Ile Lys Leu Gly Thr Val Thr Thr Val Asp Tyr
   1               5                   10
<210> 81
   <211> 14
   <212> PRT
   <213> Artificial Sequence
<220>
   <223> avelumab (MSB0010718C) HVR-L1 region (corresponding to SEQ ID NO:18 in U.S. Patent Application Publication No. US 2014/0341917 A1).
<400> 81
   Thr Gly Thr Ser Ser Asp Val Gly Gly Tyr Asn Tyr Val Ser
   1               5                   10
<210> 82
   <211> 7
   <212> PRT
   <213> Artificial Sequence
<220>
   <223> avelumab (MSB0010718C) HVR-L2 region (corresponding to SEQ ID NO:19 in U.S. Patent Application Publication No. US 2014/0341917 A1).
<400> 82
   Asp Val Ser Asn Arg Pro Ser
   1               5
<210> 83
   <211> 10
   <212> PRT
   <213> Artificial Sequence
<220>
   <223> avelumab (MSB0010718C) HVR-L3 region (corresponding to SEQ ID NO:20 in U.S. Patent Application Publication No. US 2014/0341917 A1).
<400> 83
   Ser Ser Tyr Thr Ser Ser Ser Thr Arg Val
   1               5                   10
<210> 84
   <211> 451
   <212> PRT
   <213> Artificial Sequence
<220>
   <223> Heavy chain amino acid sequence of the anti-CD20 monoclonal antibody rituximab.
<400> 84
   Gln Val Gln Leu Gln Gln Pro Gly Ala Glu Leu Val Lys Pro Gly Ala
   1               5                   10                  15
   Ser Val Lys Met Ser Cys Lys Ala Ser Gly Tyr Thr Phe Thr Ser Tyr
               20                  25                  30
   Asn Met His Trp Val Lys Gln Thr Pro Gly Arg Gly Leu Glu Trp Ile
           35                  40                  45
   Gly Ala Ile Tyr Pro Gly Asn Gly Asp Thr Ser Tyr Asn Gln Lys Phe
       50                  55                  60
   Lys Gly Lys Ala Thr Leu Thr Ala Asp Lys Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Ser Thr Tyr Tyr Gly Gly Asp Trp Tyr Phe Asn Val Trp Gly
               100                 105                 110
   Ala Gly Thr Thr Val Thr Val Ser Ala Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
   180 185 190
   Pro Ser Ser Ser Leu Gly Thr Gln Thr Tyr Ile Cys Asn Val Asn His
   195 200 205
   Lys Pro Ser Asn Thr Lys Val Asp Lys Lys Val Glu Pro Lys Ser Cys
   210 215 220
   Asp Lys Thr His Thr Cys Pro Pro Cys Pro Ala Pro Glu Leu Leu Gly
   225 230 235 240
   Gly Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser His
   260 265 270
   Glu Asp Pro Glu Val Lys Phe Asn Trp Tyr Val Asp Gly Val Glu Val
   275 280 285
   His Asn Ala Lys Thr Lys Pro Arg Glu Glu Gln Tyr Asn Ser Thr Tyr
   290 295 300
   Arg Val Val Ser Val Leu Thr Val Leu His Gln Asp Trp Leu Asn Gly
   305 310 315 320
   Lys Glu Tyr Lys Cys Lys Val Ser Asn Lys Ala Leu Pro Ala Pro Ile
   325 330 335
   Glu Lys Thr Ile Ser Lys Ala Lys Gly Gln Pro Arg Glu Pro Gln Val
   340 345 350
   Tyr Thr Leu Pro Pro Ser Arg Asp Glu Leu Thr Lys Asn Gln Val Ser
   355 360 365
   Leu Thr Cys Leu Val Lys Gly Phe Tyr Pro Ser Asp Ile Ala Val Glu
   370 375 380
   Trp Glu Ser Asn Gly Gln Pro Glu Asn Asn Tyr Lys Thr Thr Pro Pro
   385 390 395 400
   Val Leu Asp Ser Asp Gly Ser Phe Phe Leu Tyr Ser Lys Leu Thr Val
   405 410 415
   Asp Lys Ser Arg Trp Gln Gln Gly Asn Val Phe Ser Cys Ser Val Met
   420 425 430
   His Glu Ala Leu His Asn His Tyr Thr Gln Lys Ser Leu Ser Leu Ser
           435                 440                 445
   Pro Gly Lys
       450
<210> 85
   <211> 213
   <212> PRT
   <213> Artificial sequence
<220>
   <223> Light chain amino acid sequence of the anti-CD20 monoclonal antibody rituximab.
<400> 85
   Gln Ile Val Leu Ser Gln Ser Pro Ala Ile Leu Ser Ala Ser Pro Gly
   1               5                   10                  15
   Glu Lys Val Thr Met Thr Cys Arg Ala Ser Ser Ser Val Ser Tyr Ile
               20                  25                  30
   His Trp Phe Gln Gln Lys Pro Gly Ser Ser Pro Lys Pro Trp Ile Tyr
           35                  40                  45
   Ala Thr Ser Asn Leu Ala Ser Gly Val Pro Val Arg Phe Ser Gly Ser
       50                  55                  60
   Gly Ser Gly Thr Ser Tyr Ser Leu Thr Ile Ser Arg Val Glu Ala Glu
   65                  70                  75                  80
   Asp Ala Ala Thr Tyr Tyr Cys Gln Gln Trp Thr Ser Asn Pro Pro Thr
                   85                  90                  95
   Phe Gly Gly Gly Thr Lys Leu Glu Ile Lys Arg Thr Val Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
   180 185 190
   Cys Glu Val Thr His Gln Gly Leu Ser Ser Pro Val Thr Lys Ser Phe
   195 200 205
   Asn Arg Gly Glu Cys
   210
<210> 86
   <211> 449
   <212> PRT
   <213> Artificial sequence
<220>
   <223> Heavy chain amino acid sequence of the anti-CD20 monoclonal antibody obinutuzumab.
<400> 86
   Gln Val Gln Leu Val Gln Ser Gly Ala Glu Val Lys Lys Pro Gly Ser
   1 5 10 15
   Ser Val Lys Val Ser Cys Lys Ala Ser Gly Tyr Ala Phe Ser Tyr Ser
   20 25 30
   Trp Ile Asn Trp Val Arg Gln Ala Pro Gly Gln Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser Ala Ser Thr Lys Gly Pro Ser Val Phe
   115 120 125
   Pro Leu Ala Pro Ser Ser Lys Ser Thr Ser Gly Gly Thr Ala Ala Leu
   130 135 140
   Gly Cys Leu Val Lys Asp Tyr Phe Pro Glu Pro Val Thr Val Ser Trp
   145 150 155 160
   Asn Ser Gly Ala Leu Thr Ser Gly Val His Thr Phe Pro Ala Val Leu
   165 170 175
   Gln Ser Ser Gly Leu Tyr Ser Leu Ser Ser Val Val Thr Val Pro Ser
   180 185 190
   Ser Ser Leu Gly Thr Gln Thr Tyr Ile Cys Asn Val Asn His Lys Pro
   195 200 205
   Ser Asn Thr Lys Val Asp Lys Lys Val Glu Pro Lys Ser Cys Asp Lys
   210 215 220
   Thr His Thr Cys Pro Pro Cys Pro Ala Pro Glu Leu Leu Gly Gly Pro
   225 230 235 240
   Ser Val Phe Leu Phe Pro Pro Lys Pro Lys Asp Thr Leu Met Ile Ser
   245 250 255
   Arg Thr Pro Glu Val Thr Cys Val Val Val Asp Val Ser His Glu Asp
   260 265 270
   Pro Glu Val Lys Phe Asn Trp Tyr Val Asp Gly Val Glu Val His Asn
   275 280 285
   Ala Lys Thr Lys Pro Arg Glu Glu Gln Tyr Asn Ser Thr Tyr Arg Val
   290 295 300
   Val Ser Val Leu Thr Val Leu His Gln Asp Trp Leu Asn Gly Lys Glu
   305 310 315 320
   Tyr Lys Cys Lys Val Ser Asn Lys Ala Leu Pro Ala Pro Ile Glu Lys
   325 330 335
   Thr Ile Ser Lys Ala Lys Gly Gln Pro Arg Glu Pro Gln Val Tyr Thr
   340 345 350
   Leu Pro Pro Ser Arg Asp Glu Leu Thr Lys Asn Gln Val Ser Leu Thr
   355 360 365
   Cys Leu Val Lys Gly Phe Tyr Pro Ser Asp Ile Ala Val Glu Trp Glu
   370 375 380
   Ser Asn Gly Gln Pro Glu Asn Asn Tyr Lys Thr Thr Pro Pro Val Leu
   385 390 395 400
   Asp Ser Asp Gly Ser Phe Phe Leu Tyr Ser Lys Leu Thr Val Asp Lys
   405 410 415
   Ser Arg Trp Gln Gln Gly Asn Val Phe Ser Cys Ser Val Met His Glu
   420 425 430
   Ala Leu His Asn His Tyr Thr Gln Lys Ser Leu Ser Leu Ser Pro Gly
   435 440 445
   Lys
<210> 87
   <211> 219
   <212> PRT
   <213> Artificial sequence
<220>
   <223> Light chain amino acid sequence of the anti-CD20 monoclonal antibody obinutuzumab.
<400> 87
   Asp Ile Val Met Thr Gln Thr Pro Leu Ser Leu Pro Val Thr Pro Gly
   1 5 10 15
   Glu Pro Ala Ser Ile Ser Cys Arg Ser Ser Lys Ser Leu Leu His Ser
   20 25 30
   Asn Gly Ile Thr Tyr Leu Tyr Trp Tyr Leu Gln Lys Pro Gly Gln Ser
   35 40 45
   Pro Gln Leu Leu Ile Tyr Gln Met Ser Asn Leu Val Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Ala Gln Asn
   85 90 95
   Leu Glu Leu Pro Tyr Thr Phe Gly Gly Gly Thr Lys Val Glu Ile Lys
   100 105 110
   Arg Thr Val Ala Ala Pro Ser Val Phe Ile Phe Pro Pro Ser Asp Glu
   115 120 125
   Gln Leu Lys Ser Gly Thr Ala Ser Val Val Cys Leu Leu Asn Asn Phe
   130 135 140
   Tyr Pro Arg Glu Ala Lys Val Gln Trp Lys Val Asp Asn Ala Leu Gln
   145 150 155 160
   Ser Gly Asn Ser Gln Glu Ser Val Thr Glu Gln Asp Ser Lys Asp Ser
   165 170 175
   Thr Tyr Ser Leu Ser Ser Thr Leu Thr Leu Ser Lys Ala Asp Tyr Glu
   180 185 190
   Lys His Lys Val Tyr Ala Cys Glu Val Thr His Gln Gly Leu Ser Ser
   195 200 205
   Pro Val Thr Lys Ser Phe Asn Arg Gly Glu Cys
   210 215
<210> 88
   <211> 122
   <212> PRT
   <213> Artificial sequence
<220>
   <223> Variable heavy chain amino acid sequence of the anti-CD20 monoclonal antibody ofatumumab.
<400> 88
   Glu Val Gln Leu Val Glu Ser Gly Gly Gly Leu Val Gln Pro Gly Arg
   1 5 10 15
   Ser Leu Arg Leu Ser Cys Ala Ala Ser Gly Phe Thr Phe Asn Asp Tyr
   20 25 30
   Ala Met His Trp Val Arg Gln Ala Pro Gly Lys Gly Leu Glu Trp Val
   35 40 45
   Ser Thr Ile Ser Trp Asn Ser Gly Ser Ile Gly Tyr Ala Asp Ser Val
   50 55 60
   Lys Gly Arg Phe Thr Ile Ser Arg Asp Asn Ala Lys Lys Ser Leu Tyr
   65 70 75 80
   Leu Gln Met Asn Ser Leu Arg Ala Glu Asp Thr Ala Leu Tyr Tyr Cys
   85 90 95
   Ala Lys Asp Ile Gln Tyr Gly Asn Tyr Tyr Tyr Gly Met Asp Val Trp
   100 105 110
   Gly Gln Gly Thr Thr Val Thr Val Ser Ser
   115 120
<212> PRT
   <213> Artificial sequence
<220>
   <223> Variable light chain amino acid sequence of the anti-CD20 monoclonal antibody ofatumumab.
<400> 89
<210> 90
   <211> 222
   <212> PRT
   <213> Artificial Sequence
<220>
   <223> Fab fragment of heavy chain amino acid sequence of the anti-CD20 monoclonal antibody ofatumumab.
<400> 90
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asn Asp Tyr
               20                  25                  30
   Ala Met His Trp Val Arg Gln Ala Pro Gly Lys Gly Leu Glu Trp Val
           35                  40                  45
   Ser Thr Ile Ser Trp Asn Ser Gly Ser Ile Gly Tyr Ala Asp Ser Val
       50                  55                  60
   Lys Gly Arg Phe Thr Ile Ser Arg Asp Asn Ala Lys Lys Ser Leu Tyr
   65                  70                  75                  80
   Leu Gln Met Asn Ser Leu Arg Ala Glu Asp Thr Ala Leu Tyr Tyr Cys
                   85                  90                  95
   Ala Lys Asp Ile Gln Tyr Gly Asn Tyr Tyr Tyr Gly Met Asp Val Trp
               100                 105                 110
   Gly Gln Gly Thr Thr Val Thr Val Ser Ser Ala Ser Thr Lys Gly Pro
           115                 120                 125
   Ser Val Phe Pro Leu Ala Pro Gly Ser Ser Lys Ser Thr Ser Gly Thr
       130                 135                 140
   Ala Ala Leu Gly Cys Leu Val Lys Asp Tyr Phe Pro Glu Pro Val Thr
   145                 150                 155                 160
   Val Ser Trp Asn Ser Gly Ala Leu Thr Ser Gly Val His Thr Phe Pro
                   165                 170                 175
   Ala Val Leu Gln Ser Ser Gly Leu Tyr Ser Leu Ser Ser Val Val Thr
               180                 185                 190
   Val Pro Ser Ser Ser Leu Gly Thr Gln Thr Tyr Ile Cys Asn Val Asn
           195                 200                 205
   His Lys Pro Ser Asn Thr Lys Val Asp Lys Lys Val Glu Pro
       210                 215                 220
<210> 91
   <211> 211
   <212> PRT
   <213> Artificial Sequence
<220>
   <223> Fab fragment of light chain amino acid sequence of the anti-CD20 monoclonal antibody ofatumumab.
<400> 91
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Val Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Arg Ser Asn Trp Pro Ile
                   85                  90                  95
   Thr Phe Gly Gln Gly Thr Arg Leu Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg
       210
<210> 92
   <211> 451
   <212> PRT
   <213> Artificial Sequence
<220>
   <223> Heavy chain amino acid sequence of the anti-CD20 monoclonal antibody veltuzumab.
<400> 92
   Gln Val Gln Leu Gln Gln Ser Gly Ala Glu Val Lys Lys Pro Gly Ser
   1               5                   10                  15
   Ser Val Lys Val Ser Cys Lys Ala Ser Gly Tyr Thr Phe Thr Ser Tyr
               20                  25                  30
   Asn Met His Trp Val Lys Gln Ala Pro Gly Gln Gly Leu Glu Trp Ile
           35                  40                  45
   Gly Ala Ile Tyr Pro Gly Met Gly Asp Thr Ser Tyr Asn Gln Lys Phe
       50                  55                  60
   Lys Gly Lys Ala Thr Leu Thr Ala Asp Glu Ser Thr Asn Thr Ala Tyr
   65                  70                  75                  80
   Met Glu Leu Ser Ser Leu Arg Ser Glu Asp Thr Ala Phe Tyr Tyr Cys
                   85                  90                  95
   Ala Arg Ser Thr Tyr Tyr Gly Gly Asp Trp Tyr Phe Asp Val Trp Gly
               100                 105                 110
   Gln Gly Thr Thr Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Arg Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
   290 295 300
   Arg Val Val Ser Val Leu Thr Val Leu His Gln Asp Trp Leu Asn Gly
   305 310 315 320
   Lys Glu Tyr Lys Cys Lys Val Ser Asn Lys Ala Leu Pro Ala Pro Ile
   325 330 335
   Glu Lys Thr Ile Ser Lys Ala Lys Gly Gln Pro Arg Glu Pro Gln Val
   340 345 350
   Tyr Thr Leu Pro Pro Ser Arg Glu Glu Met Thr Lys Asn Gln Val Ser
   355 360 365
   Leu Thr Cys Leu Val Lys Gly Phe Tyr Pro Ser Asp Ile Ala Val Glu
   370 375 380
   Trp Glu Ser Asn Gly Gln Pro Glu Asn Asn Tyr Lys Thr Thr Pro Pro
   385 390 395 400
   Val Leu Asp Ser Asp Gly Ser Phe Phe Leu Tyr Ser Lys Leu Thr Val
   405 410 415
   Asp Lys Ser Arg Trp Gln Gln Gly Asn Val Phe Ser Cys Ser Val Met
   420 425 430
   His Glu Ala Leu His Asn His Tyr Thr Gln Lys Ser Leu Ser Leu Ser
   435 440 445
   Pro Gly Lys
   450
<210> 93
   <211> 213
   <212> PRT
   <213> Artificial Sequence
<220>
   <223> Light chain amino acid sequence of the anti-CD20 monoclonal antibody veltuzumab.
<400> 93
   Asp Ile Gln Leu Thr Gln Ser Pro Ser Ser Leu Ser Ala Ser Val Gly
   1 5 10 15
   Asp Arg Val Thr Met Thr Cys Arg Ala Ser Ser Ser Val Ser Tyr Ile
   20 25 30
   His Trp Phe Gln Gln Lys Pro Gly Lys Ala Pro Lys Pro Trp Ile Tyr
   35 40 45
   Ala Thr Ser Asn Leu Ala Ser Gly Val Pro Val Arg Phe Ser Gly Ser
   50 55 60
   Gly Ser Gly Thr Asp Tyr Thr Phe Thr Ile Ser Ser Leu Gln Pro Glu
   65 70 75 80
   Asp Ile Ala Thr Tyr Tyr Cys Gln Gln Trp Thr Ser Asn Pro Pro Thr
   85 90 95
   Phe Gly Gly Gly Thr Lys Leu Glu Ile Lys Arg Thr Val Ala Ala Pro
   100 105 110
   Ser Val Phe Ile Phe Pro Pro Ser Asp Glu Gln Leu Lys Ser Gly Thr
   115 120 125
   Ala Ser Val Val Cys Leu Leu Asn Asn Phe Tyr Pro Arg Glu Ala Lys
   130 135 140
   Val Gln Trp Lys Val Asp Asn Ala Leu Gln Ser Gly Asn Ser Gln Glu
   145 150 155 160
   Ser Val Thr Glu Gln Asp Ser Lys Asp Ser Thr Tyr Ser Leu Ser Ser
   165 170 175
   Thr Leu Thr Leu Ser Lys Ala Asp Tyr Glu Lys His Lys Val Tyr Ala
   180 185 190
   Cys Glu Val Thr His Gln Gly Leu Ser Ser Pro Val Thr Lys Ser Phe
   195 200 205
   Asn Arg Gly Glu Cys
   210
<210> 94
   <211> 447
   <212> PRT
   <213> Artificial Sequence
<220>
   <223> Heavy chain amino acid sequence of the anti-CD20 monoclonal antibody tositumomab.
<400> 94
   Gln Ala Tyr Leu Gln Gln Ser Gly Ala Glu Leu Val Arg Pro Gly Ala
   1 5 10 15
   Ser Val Lys Met Ser Cys Lys Ala Ser Gly Tyr Thr Phe Thr Ser Tyr
   20 25 30
   Asn Met His Trp Val Lys Gln Thr Pro Arg Gln Gly Leu Glu Trp Ile
   35 40 45
   Gly Ala Ile Tyr Pro Gly Asn Gly Asp Thr Ser Tyr Asn Gln Lys Phe
   50 55 60
   Lys Gly Lys Ala Thr Leu Thr Val Asp Lys Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Arg Val Val Tyr Tyr Ser Asn Ser Tyr Trp Tyr Phe Asp Val Trp
   100 105 110
   Gly Thr Gly Thr Thr Val Thr Val Ser Gly Pro Ser Val Phe Pro Leu
   115 120 125
   Ala Pro Ser Ser Lys Ser Thr Ser Gly Gly Thr Ala Ala Leu Gly Cys
   130 135 140
   Leu Val Lys Asp Tyr Phe Pro Glu Pro Val Thr Val Ser Trp Asn Ser
   145 150 155 160
   Gly Ala Leu Thr Ser Gly Val His Thr Phe Pro Ala Val Leu Gln Ser
   165 170 175
   Ser Gly Leu Tyr Ser Leu Ser Ser Val Val Thr Val Pro Ser Ser Ser
   180 185 190
   Leu Gly Thr Gln Thr Tyr Ile Cys Asn Val Asn His Lys Pro Ser Asn
   195 200 205
   Thr Lys Val Asp Lys Lys Ala Glu Pro Lys Ser Cys Asp Lys Thr His
   210 215 220
   Thr Cys Pro Pro Cys Pro Ala Pro Glu Leu Leu Gly Gly Pro Ser Val
   225 230 235 240
   Phe Leu Phe Pro Pro Lys Pro Lys Asp Thr Leu Met Ile Ser Arg Thr
   245 250 255
   Pro Glu Val Thr Cys Val Val Val Asp Val Ser His Glu Asp Pro Glu
   260 265 270
   Val Lys Phe Asn Trp Tyr Val Asp Gly Val Glu Val His Asn Ala Lys
   275 280 285
   Thr Lys Pro Arg Glu Glu Gln Tyr Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Ala Leu Pro Ala Pro Ile Glu Lys Thr Ile
   325 330 335
   Ser Lys Ala Lys Gly Gln Pro Arg Glu Pro Gln Val Tyr Thr Leu Pro
   340 345 350
   Pro Ser Arg Asp Glu Leu Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 95
   <211> 210
   <212> PRT
   <213> Artificial Sequence
<220>
   <223> Light chain amino acid sequence of the anti-CD20 monoclonal antibody tositumomab.
<400> 95
   Gln Ile Val Leu Ser Gln Ser Pro Ala Ile Leu Ser Ala Ser Pro Gly
   1               5                   10                  15
   Glu Lys Val Thr Met Thr Cys Arg Ala Ser Ser Ser Val Ser Tyr Met
               20                  25                  30
   His Trp Tyr Gln Gln Lys Pro Gly Ser Ser Pro Lys Pro Trp Ile Tyr
           35                  40                  45
   Ala Pro Ser Asn Leu Ala Ser Gly Val Pro Ala Arg Phe Ser Gly Ser
       50                  55                  60
   Gly Ser Gly Thr Ser Tyr Ser Leu Thr Ile Ser Arg Val Glu Ala Glu
   65                  70                  75                  80
   Asp Ala Ala Thr Tyr Tyr Cys Gln Gln Trp Ser Phe Asn Pro Pro Thr
                   85                  90                  95
   Phe Gly Ala Gly Thr Lys Leu Glu Leu Lys Arg Thr Val Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn Arg
       210
<210> 96
   <211> 443
   <212> PRT
   <213> Artificial Sequence
<220>
   <223> Heavy chain amino acid sequence of the anti-CD20 monoclonal antibody ibritumomab.
<400> 96
<210> 97
   <211> 209
   <212> PRT
   <213> Artificial Sequence
<220>
   <223> Light chain amino acid sequence of the anti-CD20 monoclonal antibody ibritumomab.
<400> 97
   Gln Ile Val Leu Ser Gln Ser Pro Ala Ile Leu Ser Ala Ser Pro Gly
   1 5 10 15
   Glu Lys Val Thr Met Thr Cys Arg Ala Ser Ser Ser Val Ser Tyr Met
               20                  25                  30
   His Trp Tyr Gln Gln Lys Pro Gly Ser Ser Pro Lys Pro Trp Ile Tyr
           35                  40                  45
   Ala Pro Ser Asn Leu Ala Ser Gly Val Pro Ala Arg Phe Ser Gly Ser
       50                  55                  60
   Gly Ser Gly Thr Ser Tyr Ser Leu Thr Ile Ser Arg Val Glu Ala Glu
   65                  70                  75                  80
   Asp Ala Ala Thr Tyr Tyr Cys Gln Gln Trp Ser Phe Asn Pro Pro Thr
                   85                  90                  95
   Phe Gly Ala Gly Thr Lys Leu Glu Leu Lys Arg Ala Asp Ala Ala Pro
               100                 105                 110
   Thr Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn

## Description

### CROSS-REFERENCE TO RELATED APPLICATIONS

This application claims the benefit of U.S. Provisional Application No. 62/035,812 filed on August 11, 2014; U.S. Provisional Application No. 62/088,357 filed on December 5, 2014; U.S. Provisional Application No. 62/115,489 filed on February 12, 2015; and U.S. Provisional Application No. 62/181,164 filed on June 17, 2015.

### FIELD OF THE INVENTION

In some embodiments, therapeutic combinations of a programmed death 1 (PD-1) inhibitor or PD-1 ligand (PD-L1) inhibitor, a Bruton's tyrosine kinase (BTK) inhibitor, and optionally a Janus kinase 2 (JAK-2) inhibitor, and/or a phosphoinositide 3-kinase (PI3K) inhibitor, and uses of the therapeutic combinations are disclosed herein.

### BACKGROUND OF THE INVENTION

Bruton's tyrosine kinase (BTK) is a Tec family non-receptor protein kinase expressed in B cells and myeloid cells. BTK is composed of the pleckstrin homology (PH), Tec homology (TH), Src homology 3 (SH3), Src homology 2 (SH2), and tyrosine kinase or Src homology 1 (TK or SH1) domains. The function of BTK in signaling pathways activated by the engagement of the B cell receptor (BCR) in mature B cells and FCER1 on mast cells is well established. Functional mutations in BTK in humans result in a primary immunodeficiency disease (X-linked agammaglobuinaemia) characterized by a defect in B cell development with a block between pro- and pre-B cell stages. The result is an almost complete absence of B lymphocytes, causing a pronounced reduction of serum immunoglobulin of all classes. These findings support a key role for BTK in the regulation of the production of auto-antibodies in autoimmune diseases.

BTK is expressed in numerous B cell lymphomas and leukemias. Other diseases with an important role for dysfunctional B cells are B cell malignancies, as described in Hendriks, et al., Nat. Rev. Cancer, 2014, 14, 219-231. The reported role for BTK in the regulation of proliferation and apoptosis of B cells indicates the potential for BTK inhibitors in the treatment of B cell lymphomas. BTK inhibitors have thus been developed as potential therapies for many of these malignancies, as described in D'Cruz, et al., OncoTargets and Therapy 2013, 6, 161-176.

Programmed death 1 (PD-1) is a 288-amino acid transmembrane immunocheckpoint receptor protein expressed by T cells, B cells, natural killer (NK) T cells, activated monocytes, and dendritic cells. PD-1, which is also known as CD279, is an immunoreceptor belonging to the CD28 family and in humans is encoded by the *Pdcd1* gene on chromosome 2. PD-1 consists of one immunoglobulin (Ig) superfamily domain, a transmembrane region, and an intracellular domain containing an immunoreceptor tyrosine-based inhibitory motif (ITIM) and an immunoreceptor tyrosine-based switch motif (ITSM). PD-1 and its ligands (PD-L1 and PD-L2) play a key role in immune tolerance, as described in Keir, et al., Annu. Rev. Immunol. 2008, 26, 677-704. PD-1 provides inhibitory signals that negatively regulate T cell immune responses. PD-L1 (also known as B7-H1 or CD274) and PD-L2 (also known as B7-DC or CD273) are expressed on tumor cells and stromal cells, which may be encountered by activated T cells expressing PD-1, leading to immunosuppression of the T cells. PD-L1 is a 290 amino acid transmembrane protein encoded by the *Cd274* gene on human chromosome 9. Blocking the interaction between PD-1 and its ligands PD-L1 and PD-L2 by use of a PD-1 inhibitor, a PD-L1 inhibitor, and/or a PD-L2 inhibitor can overcome immune resistance, as demonstrated in recent clinical studies, such as that described in Topalian, et al., N. Eng. J. Med. 2012, 366, 2443. PD-L1 is expressed on many tumor cell lines, while PD-L2 is expressed is expressed mostly on dendritic cells and a few tumor lines. In addition to T cells (which inducibly express PD-1 after activation), PD-1 is also expressed on B cells, natural killer cells, macrophages, activated monocytes, and dendritic cells.

In many solid tumors, the supportive microenvironment (which may make up the majority of the tumor mass) is a dynamic force that enables tumor survival. The tumor microenvironment is generally defined as a complex mixture of "cells, soluble factors, signaling molecules, extracellular matrices, and mechanical cues that promote neoplastic transformation, support tumor growth and invasion, protect the tumor from host immunity, foster therapeutic resistance, and provide niches for dominant metastases to thrive," as described in Swartz, et al., Cancer Res., 2012, 72, 2473. Although tumors express antigens that should be recognized by T cells, tumor clearance by the immune system is rare because of immune suppression by the microenvironment. Addressing the tumor cells themselves with e.g. chemotherapy has also proven to be insufficient to overcome the protective effects of the microenvironment. New approaches are thus urgently needed for more effective treatment of solid tumors that take into account the role of the microenvironment. The supportive microenvironment also plays a critical role in many B cell cancers such as acute leukemias, myelodysplastic syndromes, chronic lymphocytic leukemia (CLL) and small lymphocytic leukemia (SLL), mucosa-associated lymphoid tissue (MALT) lymphomas, and multiple myeloma (MM). Burger, et al., Blood, 2009, 114, 3367-75. For example, CLL and SLL cells rapidly accumulate and are resistant to apoptosis in vivo, but are known to die rapidly in vitro. Buchner, et al., Blood 2010, 115, 4497-506. One cause of this effect is from nonmalignant accessory cells in the tumor microenvironment, such as stromal cell contact mediated cell survival. Stromal cells in the bone marrow and lymph nodes are known to have an antiapoptotic and protective effect on CLL cells, protecting them from both chemotherapeutic and spontaneous apoptosis. Mudry, et al., Blood 2000, 96, 1926-32. The chemokine SDF1α (CXCL12) directs homing of CLL cells towards protective niches. Burger, et al., Blood 2005, 106, 1824-30. Existing drugs that target the BCR pathway in B cell malignancies can lead to some lymphocytosis (lymphocyte egress from nodal compartments), through disruption of CXCR4-SDF1α signaling and other adhesion factors in bone marrow and the resulting mobilization of cells. However, existing therapies may not eradicate residual malignant B cell populations in the microenvironment of the bone marrow and lymph nodes, where protective stromal cells prevent apoptosis. There is thus an urgent need for treatments that reduce or overcome the protective effect of the microenvironment on CLL cells to enable superior clinical responses in patients.

PI3K inhibitors are members of a unique and conserved family of intracellular lipid kinases that phosphorylate the 3'-OH group on phosphatidylinositols or phosphoinositides. PI3K inhibitors are key signaling enzymes that relay signals from cell surface receptors to downstream effectors. The PI3K family comprises 15 kinases with distinct substrate specificities, expression patterns, and modes of regulation. The class I PI3K inhibitors (p110α, p110β, p110δ, and p110γ) are typically activated by tyrosine kinases or G-protein coupled receptors to generate PIP3, which engages downstream effectors such as those in the Akt/PDK1 pathway, mTOR, the Tec family kinases, and the Rho family GTPases.

The PI3K signaling pathway is known to be one of the most highly mutated in human cancers. PI3K signaling is also a key factor in disease states including hematologic malignancies, non-Hodgkin's lymphoma (such as diffuse large B-cell lymphoma), allergic contact dermatitis, rheumatoid arthritis, osteoarthritis, inflammatory bowel diseases, chronic obstructive pulmonary disorder, psoriasis, multiple sclerosis, asthma, disorders related to diabetic complications, and inflammatory complications of the cardiovascular system such as acute coronary syndrome. The role of PI3K in cancer has been discussed, for example, in Engleman, Nat. Rev. Cancer 2009, 9, 550-562. The PI3K-δ and PI3K-γ isoforms are preferentially expressed in normal and malignant leukocytes.

The delta (δ) isoform of class I PI3K (PI3K-δ) is involved in mammalian immune system functions such as T-cell function, B-cell activation, mast cell activation, dendritic cell function, and neutrophil activity. Due to its role in immune system function, PI3K-δ is also involved in a number of diseases related to undesirable immune response such as allergic reactions, inflammatory diseases, inflammation mediated angiogenesis, rheumatoid arthritis, auto-immune diseases such as lupus, asthma, emphysema and other respiratory diseases. The gamma (γ) isoform of class I PI3K (PI3K-γ) is also involved in immune system functions and plays a role in leukocyte signaling and has been implicated in inflammation, rheumatoid arthritis, and autoimmune diseases such as lupus.

Downstream mediators of the PI3K signal transduction pathway include Akt and mammalian target of rapamycin (mTOR). One important function of Akt is to augment the activity of mTOR, through phosphorylation of TSC2 and other mechanisms. mTOR is a serine-threonine kinase related to the lipid kinases of the PI3K family and has been implicated in a wide range of biological processes including cell growth, cell proliferation, cell motility and survival. Disregulation of the mTOR pathway has been reported in various types of cancer.

In view of the above, PI3K inhibitors are prime targets for drug development, as described in Kurt, et al., Anticancer Res. 2012, 32, 2463-70. Several PI3K inhibitors are known, including those that are PI3K-δ inhibitors, PI3K-γ inhibitors and those that are PI3K-δ,γ inhibitors.

E Klyuchnikov et al. (Bone Marrow Transplantation., 2013, 49, 1-7) discloses the treatment of diffuse large B cell lymphoma (OLBCL) via hematopoietic stem cell transplantation (auto-HCT) and describes the attempts to improve the efficiency of auto-HCT such as incorporating novel agents including PD-1 antibodies as as maintenance therapy after auto-HCT. This document also discloses the use of ibrutinib, a BTK inhibitor, as efficient to treat OLBCL.

The present invention also includes the unexpected discovery that the combination of a PD-1 inhibitor and a BTK inhibitor is effective and synergistic in the treatment of any of several types of cancers such as solid tumor cancers. The present invention includes the unexpected discovery that combinations of a BTK inhibitor, a PD-1 inhibitor (such as an anti-PD-1 antibody) or a PD-L1 inhibitor (such as an anti-PD-Ll antibody), and optionally a PI3K inhibitor exhibit surprising synergy in the suppression of the supportive solid tumor microenvironment.

### SUMMARY OF THE INVENTION

The invention provides a pharmaceutical combination comprising (1) a programmed death 1 (PD-1) inhibitor or a programmed death ligand 1 (PD-L1) inhibitor and (2) a Bruton's tyrosine kinase (BTK) inhibitor or a pharmaceutically acceptable salt thereof for use in treatment of cancer in a human subject, wherein the BTK inhibitor is: 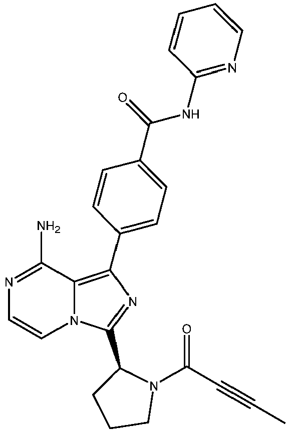 , the PD-1 inhibitor is pembrolizumab, and the PD-L1 inhibitor is durvalumab.

In an embodiment, the invention provides a pharmaceutical combination as defined above for use in treating leukemia, lymphoma or a solid tumor cancer in a human subject.

In an embodiment, the invention provides a pharmaceutical combination as defined above for use in treating leukemia, lymphoma or a solid tumor cancer in a human subject, wherein the combination comprises a therapeutically effective amount of a PI3K inhibitor, a PD-1 inhibitor or a PD-L1 inhibitor as above, and a BTK inhibitor as above.

In an embodiment, the invention provides a pharmaceutical combination for use in treating leukemia, lymphoma or a solid tumor cancer in a human subject, wherein the combination comprises a therapeutically effective amount of a PI3K-γ inhibitor, a PD-1 inhibitor or a PD-L1 inhibitor as defined above, and a BTK inhibitor as defined above.

In an embodiment, the invention provides a pharmaceutical combination for use in treating leukemia, lymphoma or a solid tumor cancer in a human subject, wherein the combination comprises a therapeutically effective amount of a PI3K-δ inhibitor, a PD-1 inhibitor or a PD-L1 inhibitor as defined above, and a BTK inhibitor as defined above.

In an embodiment, the invention provides a pharmaceutical combination for use in treating leukemia, lymphoma or a solid tumor cancer in a human subject, wherein the combination comprises a therapeutically effective amount of a PI3K-γ,δ inhibitor, a PD-1 inhibitor or a PD-L1 inhibitor as defined above, and a BTK inhibitor as defined above.

In an embodiment, the invention provides a pharmaceutical combination for use in treating leukemia, lymphoma, or a solid tumor cancer in a human subject, wherein the combination comprises a therapeutically effective amount of a PD-1 inhibitor or a PD-L1 inhibitor as defined above and a BTK inhibitor as defined above, and an anti-CD20 antibody selected from the group consisting of rituximab, obinutuzumab, ofatumumab, veltuzumab, tositumomab, ¹³¹I-tositumomab, ibritumomab, ⁹⁰Y-ibritumomab, ¹¹¹In-ibritumomab, and ibritumomab tiuxetan, and fragments, derivatives, conjugates, variants, radioisotope-labeled complexes, and biosimilars thereof.

In an embodiment, the invention provides a pharmaceutical combination for use intreating leukemia, lymphoma, or a solid tumor cancer in a human subject, wherein the combination comprises a therapeutically effective amount of a PI3K inhibitor, a PD-1 inhibitor or a PD-L1 inhibitor as defined above, and a BTK inhibitor as defined above, and an anti-CD20 antibody selected from the group consisting of rituximab, obinutuzumab, ofatumumab, veltuzumab, tositumomab, ¹³¹I-tositumomab, ibritumomab, ⁹⁰Y-ibritumomab, ¹¹¹In-ibritumomab, and ibritumomab tiuxetan, and fragments, derivatives, conjugates, variants, radioisotope-labeled complexes, and biosimilars thereof.

In an embodiment, the invention provides a composition comprising a combination comprising (1) a PD-1 inhibitor (pembrolizumab) or a PD-L1 inhibitor (durvalumab); and (2) a BTK inhibitor of formula (XVIII) or a pharmaceutically acceptable salt thereof, for use in the treatment of cancer. This composition is typically a pharmaceutical composition.

In an embodiment, the invention provides a composition comprising a combination comprising (1) a PD-1 inhibitor or a PD-L1 inhibitor; (2) a BTK inhibitor or a pharmaceutically acceptable salt,thereof; and (3) a PI3K inhibitor or a pharmaceutically acceptable salt, solvate, or hydrate thereof for use in the treatment of cancer in a human, wherein the BTK inhibitor is 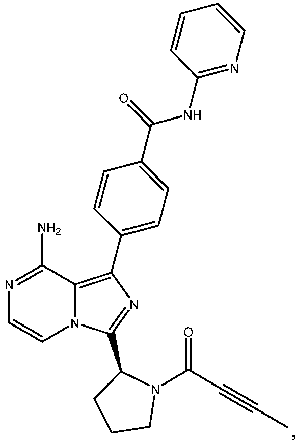 the PD-1 inhibitor is pembrolizumab, and the PD-L1 inhibitor is durvalumab. This composition is typically a pharmaceutical composition.

In an embodiment, the invention provides a composition comprising a combination comprising (1) a PD-1 inhibitor or a PD-L1 inhibitor as above; (2) a BTK inhibitor as above (3) a PI3K-δ inhibitor or a pharmaceutically acceptable salt, solvate, or hydrate thereof, for use in the treatment of cancer in a human. This composition is typically a pharmaceutical composition.

In an embodiment, the invention provides a composition comprising (1) a PD-1 inhibitor or a PD-L1 inhibitor as above; (2) a BTK inhibitor as above; and (3) an anti-coagulant or antiplatelet active pharmaceutical ingredient, for use in the treatment of cancer in a human. This composition is typically a pharmaceutical composition.

In an embodiment, the invention provides a composition comprising (1) a PD-1 inhibitor or a PD-L1 inhibitor as above; (2) a BTK inhibitor as above; and (3) a PI3K inhibitor or a pharmaceutically acceptable salt, solvate, or hydrate thereof; and (4) an anti-coagulant or antiplatelet active pharmaceutical ingredient for use in the treatment of cancer in a human. This composition is typically a pharmaceutical composition.

In an embodiment, the invention provides a composition comprising (1) a PD-1 inhibitor or a PD-L1 inhibitor as above; (2) a BTK inhibitor as above; and (3) a PI3K-δ inhibitor or a pharmaceutically acceptable salt, solvate, or hydrate thereof; and (4) an anti-coagulant or antiplatelet active pharmaceutical ingredient for use in the treatment of cancer in a human. This composition is typically a pharmaceutical composition.

The anti-coagulant or the anti-platelet active pharmaceutical ingredient in some specific embodiments is a compound selected from the group consisting of acenocoumarol, anagrelide, anagrelide hydrochloride, abciximab, aloxiprin, antithrombin, apixaban, argatroban, aspirin, aspirin with extended-release dipyridamole, beraprost, betrixaban, bivalirudin, carbasalate calcium, cilostazol, clopidogrel, clopidogrel bisulfate, cloricromen, dabigatran etexilate, darexaban, dalteparin, dalteparin sodium, defibrotide, dicumarol, diphenadione, dipyridamole, ditazole, desirudin, edoxaban, enoxaparin, enoxaparin sodium, eptifibatide, fondaparinux, fondaparinux sodium, heparin, heparin sodium, heparin calcium, idraparinux, idraparinux sodium, iloprost, indobufen, lepirudin, low molecular weight heparin, melagatran, nadroparin, otamixaban, parnaparin, phenindione, phenprocoumon, prasugrel, picotamide, prostacyclin, ramatroban, reviparin, rivaroxaban, sulodexide, terutroban, terutroban sodium, ticagrelor, ticlopidine, ticlopidine hydrochloride, tinzaparin, tinzaparin sodium, tirofiban, tirofiban hydrochloride, treprostinil, treprostinil sodium, triflusal, vorapaxar, warfarin, warfarin sodium, ximelagatran, salts thereof, solvates thereof, hydrates thereof, and combinations thereof.

In an embodiment, the invention provides a kit comprising a combination comprising (1) a PD-1 inhibitor or a PD-L1 inhibitor; and (2) a composition comprising a BTK inhibitor for use in the treatment of cancer in a human, wherein the BTK inhibitor is 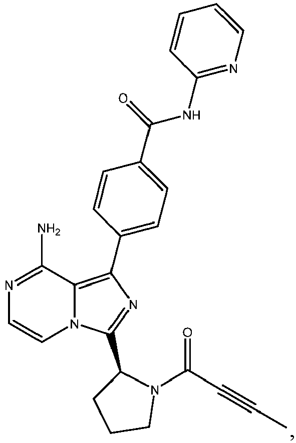 the PD-1 inhibitor is pembrolizumab, and the PD-L1 inhibitor is durvalumab. These compositions are typically pharmaceutical compositions. The kit is for co-administration of a PD-1 or PD-L1 inhibitor and a BTK inhibitor, either simultaneously or separately.

In an embodiment, the invention provides a composition comprising a combination comprising (1) a PD-1 inhibitor or a PD-L1 inhibitor as defined above; (2) a composition comprising a BTK inhibitor as defined above; and (3) a composition comprising a PI3K inhibitor or a pharmaceutically acceptable salt, solvate, or hydrate thereof for use in the treatment of cancer in a human. These compositions are typically pharmaceutical compositions. The kit is for co-administration of PD-1 or PD-L1 inhibitor, a BTK inhibitor, and a PI3K inhibitor, either simultaneously or separately.

In an embodiment, the invention provides a composition comprising a combination comprising (1) a PD-1 inhibitor or a PD-L1 inhibitor as defined above; (2) a composition comprising a BTK inhibitor as defined above; and (3) a composition comprising a PI3K-δ inhibitor or a pharmaceutically acceptable salt, solvate, or hydrate thereof for use in the treatment of cancer in a human. These compositions are typically pharmaceutical compositions. The kit is for co-administration of a PD-1 or a PD-L1 inhibitor, a BTK inhibitor, and an anti-coagulant or antiplatelet active pharmaceutical ingredient, either simultaneously or separately.

In an embodiment, the invention provides a composition comprising a combination comprising (1) a PD-1 inhibitor or a PD-L1 inhibitor as defined above; (2) a composition comprising a BTK inhibitor as defined above; and (3) an anti-coagulant or antiplatelet active pharmaceutical ingredient for use in the treatment of cancer in a human. These compositions are typically pharmaceutical compositions. The kit is for co-administration of a PD-1 or a PD-L1 inhibitor, a BTK inhibitor, and an anti-coagulant or antiplatelet active pharmaceutical ingredient, either simultaneously or separately.

In an embodiment, the invention provides a composition comprising a combination comprising (1) a composition comprising a PD-1 inhibitor or a PD-L1 inhibitor as defined above; (2) a composition comprising a BTK inhibitor as defined above; and (3) a composition comprising a PI3K inhibitor or a pharmaceutically acceptable salt, solvate, or hydratethereof; and (4) an anti-coagulant or antiplatelet active pharmaceutical ingredient for use in the treatment of cancer in a human subject. These compositions are typically pharmaceutical compositions. The kit is for co-administration of a PI3K-δ inhibitor and an anti-coagulant or antiplatelet active pharmaceutical ingredient, either simultaneously or separately.

In an embodiment, the invention provides a composition comprising (1) a composition comprising a PD-1 inhibitor or a PD-L1 inhibitor as defined above; (2) a composition comprising a BTK inhibitor as defined above; and (3) a composition comprising a PI3K-δ inhibitor or a pharmaceutically acceptable salt, solvate, or hydrate thereof; and (4) an anti-coagulant or antiplatelet active pharmaceutical ingredient for use in the treatment of cancer in a human subject. These compositions are typically pharmaceutical compositions. The kit is for co-administration of a PD-1 or a PD-L1 inhibitor, a BTK inhibitor, a PI3K-δ inhibitor, and an anti-coagulant or antiplatelet active pharmaceutical ingredient, either simultaneously or separately.

The combination, compositions and the kits disclosed herein are for use in treating cancer. In some specific embodiments, the compositions and the kits disclosed herein are for use in treating cancer selected from the group consisting of a B cell hematological malignancy selected from the hematological malignancy is selected from the group consisting of chronic lymphocytic leukemia (CLL), small lymphocytic leukemia (SLL), non-Hodgkin's lymphoma (NHL), diffuse large B cell lymphoma (DLBCL), follicular lymphoma (FL), mantle cell lymphoma (MCL), Hodgkin's lymphoma, B cell acute lymphoblastic leukemia (B-ALL), Burkitt's lymphoma, Waldenstrom's macroglobulinemia (WM), Burkitt's lymphoma, multiple myeloma (MM), or myelofibrosis.

In some embodiments, the combination, compositions and the kits disclosed herein are for use in treating a cancer selected from the group consisting of bladder cancer, squamous cell carcinoma including head and neck cancer, pancreatic ductal adenocarcinoma (PDA), pancreatic cancer, colon carcinoma, mammary carcinoma, breast cancer, fibrosarcoma, mesothelioma, renal cell carcinoma, lung carcinoma, thyoma, prostate cancer, colorectal cancer, ovarian cancer, acute myeloid leukemia, thymus cancer, brain cancer, squamous cell cancer, skin cancer, eye cancer, retinoblastoma, melanoma, intraocular melanoma, oral cavity and oropharyngeal cancers, gastric cancer, stomach cancer, cervical cancer, head, neck, renal cancer, kidney cancer, liver cancer, ovarian cancer, prostate cancer, colorectal cancer, esophageal cancer, testicular cancer, gynecological cancer, thyroid cancer, acquired immune deficiency syndrome (AIDS)-related cancers (e.g., lymphoma and Kaposi's sarcoma), viral-induced cancer, glioblastoma, glioma, esophageal tumors, hematological neoplasms, non-small-cell lung cancer, chronic myelocytic leukemia, diffuse large B-cell lymphoma, esophagus tumor, follicle center lymphoma, head and neck tumor, hepatitis C virus infection, hepatocellular carcinoma, Hodgkin's disease, metastatic colon cancer, multiple myeloma, non-Hodgkin's lymphoma, indolent non-Hodgkin's lymphoma, ovary tumor, pancreas tumor, renal cell carcinoma, small-cell lung cancer, stage IV melanoma, chronic lymphocytic leukemia, B-cell acute lymphoblastic leukemia (ALL), mature B-cell ALL, follicular lymphoma, mantle cell lymphoma, and Burkitt's lymphoma.

In other embodiments, the combination, compositions and the kits disclosed herein are for use in treating a solid tumor cancer selected from the group consisting of bladder cancer, non-small cell lung cancer, cervical cancer, anal cancer, pancreatic cancer, squamous cell carcinoma including head and neck cancer, renal cell carcinoma, melanoma, ovarian cancer, small cell lung cancer, glioblastoma, glioma, gastrointestinal stromal tumor, breast cancer, lung cancer, colorectal cancer, thyroid cancer, bone sarcoma, stomach cancer, oral cavity cancer, oropharyngeal cancer, gastric cancer, kidney cancer, liver cancer, prostate cancer, colorectal cancer, esophageal cancer, testicular cancer, gynecological cancer, thyroid cancer, colon cancer, and brain cancer.

In some embodiments, the combination, compositions and the kits disclosed herein are for use in treating a solid tumor cancer, wherein the compositions are in a dosage that is effective in inhibiting signaling between the cells of the solid tumor cancer and at least one tumor microenvironment selected from the group consisting of macrophages, monocytes, mast cells, helper T cells, cytotoxic T cells, regulatory T cells, natural killer cells, myeloid-derived suppressor cells, regulatory B cells, neutrophils, dendritic cells, and fibroblasts.

In some embodiments, the combination, compositions and the kits disclosed herein are for use in treating a solid tumor cancer, wherein the compositions are in a dosage that is effective in increasing immune system recognition and rejection of the solid tumor by the human body receiving the treatment.

In some embodiments, the combination, compositions and the kits disclosed herein are for use in treating cancer, wherein the PD-1 or PD-L1 inhibitor is administered before administration of the BTK inhibitor.

In some embodiments, the combination, compositions and the kits disclosed herein are for use in treating cancer, wherein the PD-1 or PD-L1 inhibitor is administered concurrently with the administration of the BTK inhibitor.

In some embodiments, the combination, compositions and the kits disclosed herein are for use in treating cancer, wherein the PD-1 or PD-L1 inhibitor is administered to the subject after administration of the BTK inhibitor.

### BRIEF DESCRIPTION OF THE DRAWINGS

The foregoing summary, as well as the following detailed description of the invention, will be better understood when read in conjunction with the appended drawings.
FIG. 95 illustrates the dosing schema used for the α-PD-L1 inhibitor (BioXcell InVivoMAb anti-m-PD-Ll, Clone 10F.9G2) in combination with the BTK inhibitor of Formula (XVIII) in a syngeneic CT26 colon cancer model in the Balb/c strain of mice.
FIG. 100 illustrates the effects of vehicle on flux at two timepoints, as a control for comparison with FIG. 101, in the ID8 syngeneic orthotropic ovarian cancer model.
FIG. 101 illustrates the effects of the BTK inhibitor of Formula (XVIII) on flux at two timepoints, for comparison with FIG. 100, in the ID8 syngeneic orthotropic ovarian cancer model.
FIG. 102 illustrates tumor response to treatment with the BTK inhibitor of Formula (XVIII) correlates with a significant reduction in immunosuppressive tumor associated lymphocytes in tumor-bearing mice, in comparison to a control (vehicle).
FIG. 103 illustrates that treatment with the BTK inhibitor of Formula (XVIII) impairs ID8 ovarian cancer growth in the syngeneic murine model in comparison to a control (vehicle).
FIG. 104 illustrates that treatment with the BTK inhibitor of Formula (XVIII) induces a tumor response that correlates with a significant reduction in total B cells in tumor-bearing mice.
FIG. 105 illustrates that treatment with the BTK inhibitor of Formula (XVIII) induces a tumor response that correlates with a significant reduction in B regulatory cells (Bregs) in tumor-bearing mice.
FIG. 106 illustrates that treatment with the BTK inhibitor of Formula (XVIII) induces a tumor response that correlates with a significant reduction in immunosuppressive tumor associated Tregs.
FIG. 107 illustrates that treatment with the BTK inhibitor of Formula (XVIII) induces a tumor response that correlates with an increase in CD8⁺ T cells.
FIG. 108 illustrates bioluminescence images from mice in the different treatment arms of the ID8 ovarian cancer model study.
FIG. 135 illustrates the treatment schema used for the α-PD-L1 inhibitor (BioXcell InVivoMAb anti-m-PD-Ll, Clone 10F.9G2) in combination with the BTK inhibitor of Formula (XVIII), the PI3K inhibitor of Formula (IX), and the BTK inhibitor ibrutinib in a 4T1 orthotopic breast cancer model.
FIG. 138 illustrates the treatment schema used for the α-PD-L1 inhibitor (BioXcell InVivoMAb anti-m-PD-Ll, Clone 10F.9G2) in combination with the BTK inhibitor of Formula (XVIII), the PI3K inhibitor of Formula (IX), and the BTK inhibitor ibrutinib in an A20 orthotopic lymphoma model.
FIG. 141 illustrates *in vivo* potency of Formula (XVIII) (labeled "BTK inhibitor") and ibrutinib. Mice were gavaged at increasing drug concentration and sacrificed at one time point (3 h post-dose). BCR is stimulated with IgM and the expression of activation markers CD69 and CD86 are monitored by flow cytometry to determine EC₅₀'s. The results show that Formula (XVIII) is more potent at inhibiting expression of activation makers than ibrutinib.
FIG. 142 illustrates the results of the clinical study of Formula (XVIII) (labeled "BTK inhibitor") in CLL, which are shown in comparison to the results reported for ibrutinib in Figure 1A of Byrd, et al., N. Engl. J. Med. 2013, 369, 32-42. The results show that the BTK inhibitor of Formula (XVIII) causes a much smaller relative increase and much faster decrease in absolute lymphocyte count (ALC) relative to the BTK inhibitor ibrutinib. The sum of the product of greatest diameters (SPD) also decreases more rapidly during treatment with the BTK inhibitor than with the BTK inhibitor ibrutinib.
FIG. 143 shows overall response data shown by SPD of enlarged lymph nodes in CLL patients as a function of dose of the BTK inhibitor of Formula (XVIII).
FIG. 144 shows a comparison of progression-free survival (PFS) in CLL patients treated with the BTK inhibitor ibrutinib or the BTK inhibitor of Formula (XVIII). The ibrutinib data is taken from Byrd, et al., N. Engl. J. Med. 2013, 369, 32-42. CLL patients treated with Formula (XVIII) for at least 8 days are included.
FIG. 145 shows a comparison of number of patients at risk in CLL patients treated with the BTK inhibitor ibrutinib or the BTK inhibitor of Formula (XVIII). CLL patients treated with Formula (XVIII) for at least 8 days are included.
FIG. 146 shows a comparison of progression-free survival (PFS) in CLL patients exhibiting the 17p deletion and treated with the BTK inhibitor ibrutinib or the BTK inhibitor of Formula (XVIII). The ibrutinib data is taken from Byrd, et al., N. Engl. J. Med. 2013, 369, 32-42.
FIG. 147 shows a comparison of number of patients at risk in CLL patients exhibiting the 17p deletion and treated with the BTK inhibitor ibrutinib or the BTK inhibitor of Formula (XVIII). The ibrutinib data is taken from Byrd, et al., N. Engl. J. Med. 2013, 369, 32-42. CLL patients treated with Formula (XVIII) for at least 8 days are included.
FIG. 148 shows improved BTK target occupancy of Formula (XVIII) at lower dosage versus ibrutinib in relapsed/refractory CLL patients.
FIG. 149 shows the % change in myeloid-derived suppressor cell (MDSC) (monocytic) level over 28 days versus % ALC change at Cycle 1, day 28 (C1D28) with trendlines.
FIG. 150 shows the % change in MDSC (monocytic) level over 28 days versus % ALC change at Cycle 2, day 28 (C2D28) with trendlines.
FIG. 151 shows the % change in natural killer (NK) cell level over 28 days versus % ALC change at Cycle 1, day 28 (C2D28) with trendlines.
FIG. 152 shows the % change in NK cell level over 28 days versus % ALC change at Cycle 2, day 28 (C2D28) with trendlines.
FIG. 153 compares the % change in MDSC (monocytic) level and % change in NK cell level over 28 days versus % ALC change with the % change in level of CD4⁺ T cells, CD8⁺ T cells, CD4⁺/CD8⁺ T cell ratio, NK-T cells, PD-1⁺CD4⁺ T cells, and PD-1⁺ CD8⁺ T cells, also versus % ALC change, at Cycle 1 day 28 (C1D28). Trendlines are shown for % change in MDSC (monocytic) level and % change in NK cell level.
FIG. 154 compares the % change in MDSC (monocytic) level and % change in NK cell level over 28 days versus % ALC change with the % change in level of CD4⁺ T cells, CD8⁺ T cells, CD4⁺/CD8⁺ T cell ratio, NK-T cells, PD-1⁺CD4⁺ T cells, and PD-1⁺ CD8⁺ T cells, also versus % ALC change, at Cycle 2 day 28 (C2D28). Trendlines are shown for % change in MDSC (monocytic) level and % change in NK cell level.
FIG. 155 shows additional clinical data related to that presented in FIG. 142.
FIG. 156 shows additional clinical data related to that presented in FIG. 148, and includes BID dosing results.
FIG. 157 illustrates PFS for patients with 17p deletion.
FIG. 158 illustrates PFS across relapsed/refractory patients with 17p deletion and with 11q deletion and no 17p deletion.
FIG. 159 illustrates PFS for patients with 11q deletion and no 17p deletion.
FIG. 160 illustrates shows additional clinical SPD results from the clinical study of Formula (XVIII) in relapsed/refractory CLL patients.
FIG. 161 illustrates that treatment of CLL patients with Formula (XVIII) resulted in increased apoptosis.
FIG. 162 illustrates a decrease in CXCL12 levels observed in patients treated with Formula (XVIII).
FIG. 163 illustrates a decrease in CCL2 levels observed in patients treated with Formula (XVIII).

### BRIEF DESCRIPTION OF THE SEQUENCE LISTING

SEQ ID NO:11 is the 409A-H heavy chain full length sequence of the PD-1 inhibitor pembrolizumab (corresponding to SEQ ID NO:31 in U.S. Patent No. 8,354,509 B2).
SEQ ID NO:12 is amino acids 20 to 466 of the heavy chain full length sequence of the PD-1 inhibitor pembrolizumab.
SEQ ID NO:13 is the K09A-L-11 light chain variable region sequence of the PD-1 inhibitor pembrolizumab (corresponding to SEQ ID NO:32 in U.S. Patent No. 8,354,509 B2).
SEQ ID NO:14 is the K09A-L-11 light chain full length sequence of the PD-1 inhibitor pembrolizumab (corresponding to SEQ ID NO:36 in U.S. Patent No. 8,354,509 B2).
SEQ ID NO:15 is the hPD-1.09A light chain CDR1 sequence of the PD-1 inhibitor pembrolizumab (corresponding to SEQ ID NO:15 in U.S. Patent No. 8,354,509 B2).
SEQ ID NO:16 is the hPD-1.09A light chain CDR2 sequence of the PD-1 inhibitor pembrolizumab (corresponding to SEQ ID NO: 16 in U.S. Patent No. 8,354,509 B2).
SEQ ID NO:17 is the hPD-1.09A light chain CDR3 sequence of the PD-1 inhibitor pembrolizumab (corresponding to SEQ ID NO: 17 in U.S. Patent No. 8,354,509 B2).
SEQ ID NO:18 is the hPD-1.09A heavy chain CDR1 sequence of the PD-1 inhibitor pembrolizumab (corresponding to SEQ ID NO:18 in U.S. Patent No. 8,354,509 B2).
SEQ ID NO: 19 is the hPD-1.09A heavy chain CDR2 sequence of the PD-1 inhibitor pembrolizumab (corresponding to SEQ ID NO: 19 in U.S. Patent No. 8,354,509 B2).
SEQ ID NO:20 is the hPD-1.09A heavy chain CDR3 sequence of the PD-1 inhibitor pembrolizumab (corresponding to SEQ ID NO:20 in U.S. Patent No. 8,354,509 B2).
SEQ ID NO:30 is the heavy chain of the anti-PD-Ll antibody durvalumab (MEDI4736).
SEQ ID NO:31 is the light chain of the anti-PD-Ll antibody durvalumab (MEDI4736).
SEQ ID NO:32 is the durvalumab (MEDI4736) anti-PD-Ll antibody heavy chain variable region (corresponding to SEQ ID NO:72 in U.S. Patent Application Publication No. US 2013/0034559 A1).
SEQ ID NO:33 is the durvalumab (MEDI4736) anti-PD-Ll antibody light chain variable region (corresponding to SEQ ID NO:77 in U.S. Patent Application Publication No. US 2013/0034559 A1).
SEQ ID NO:34 is the durvalumab (MEDI4736) anti-PD-Ll antibody heavy chain variable region CDR1 (corresponding to SEQ ID NO:3 in U.S. Patent Application Publication No. US 2013/0034559 A1).
SEQ ID NO:35 is the durvalumab (MEDI4736) anti-PD-Ll antibody heavy chain variable region CDR2 (corresponding to SEQ ID NO:4 in U.S. Patent Application Publication No. US 2013/0034559 A1).
SEQ ID NO:36 is the durvalumab (MEDI4736) anti-PD-Ll antibody heavy chain variable region CDR3 (corresponding to SEQ ID NO:5 in U.S. Patent Application Publication No. US 2013/0034559 A1).
SEQ ID NO:37 is the durvalumab (MEDI4736) anti-PD-Ll antibody light chain variable region CDR1 (corresponding to SEQ ID NO:8 in U.S. Patent Application Publication No. US 2013/0034559 A1).
SEQ ID NO:38 is the durvalumab (MEDI4736) anti-PD-Ll antibody light chain variable region CDR2 (corresponding to SEQ ID NO:9 in U.S. Patent Application Publication No. US 2013/0034559 A1).
SEQ ID NO:39 is the durvalumab (MEDI4736) anti-PD-Ll antibody light chain variable region CDR3 (corresponding to SEQ ID NO:10 in U.S. Patent Application Publication No. US 2013/0034559 A1).
SEQ ID NO:84 is the heavy chain amino acid sequence of the anti-CD20 monoclonal antibody rituximab.
SEQ ID NO:85 is the light chain amino acid sequence of the anti-CD20 monoclonal antibody rituximab.
SEQ ID NO:86 is the heavy chain amino acid sequence of the anti-CD20 monoclonal antibody obinutuzumab.
SEQ ID NO:87 is the light chain amino acid sequence of the anti-CD20 monoclonal antibody obinutuzumab.
SEQ ID NO:88 is the variable heavy chain amino acid sequence of the anti-CD20 monoclonal antibody ofatumumab.
SEQ ID NO:89 is the variable light chain amino acid sequence of the anti-CD20 monoclonal antibody ofatumumab.
SEQ ID NO:90 is the Fab fragment heavy chain amino acid sequence of the anti-CD20 monoclonal antibody ofatumumab.
SEQ ID NO:91 is the Fab fragment light chain amino acid sequence of the anti-CD20 monoclonal antibody ofatumumab.
SEQ ID NO:92 is the heavy chain amino acid sequence of the anti-CD20 monoclonal antibody veltuzumab.
SEQ ID NO:93 is the light chain amino acid sequence of the anti-CD20 monoclonal antibody veltuzumab.
SEQ ID NO:94 is the heavy chain amino acid sequence of the anti-CD20 monoclonal antibody tositumomab.
SEQ ID NO:95 is the light chain amino acid sequence of the anti-CD20 monoclonal antibody tositumomab.
SEQ ID NO:96 is the heavy chain amino acid sequence of the anti-CD20 monoclonal antibody ibritumomab.
SEQ ID NO:97 is the light chain amino acid sequence of the anti-CD20 monoclonal antibody ibritumomab.

### DETAILED DESCRIPTION OF THE INVENTION

Unless defined otherwise, all technical and scientific terms used herein have the same meaning as is commonly understood by one of skill in the art to which this invention belongs.

The terms "co-administration," "co-administering," "administered in combination with," and "administering in combination with" as used herein, encompass administration of two or more active pharmaceutical ingredients to a subject so that both active pharmaceutical ingredients and/or their metabolites are present in the subject at the same time. Co-administration includes simultaneous administration in separate compositions, administration at different times in separate compositions, or administration in a composition in which two or more active pharmaceutical ingredients are present. Simultaneous administration in separate compositions and administration in a composition in which two or more active pharmaceutical ingredients are present is preferred.

The term "effective amount" or "therapeutically effective amount" refers to that amount of a compound or combination of compounds as described herein that is sufficient to effect the intended application including disease treatment. A therapeutically effective amount may vary depending upon the intended application (*in vitro* or *in vivo*), or the subject and disease condition being treated (*e.g.,* the weight, age and gender of the subject), the severity of the disease condition, the manner of administration, *etc.* which can readily be determined by one of ordinary skill in the art. The term also applies to a dose that will induce a particular response in target cells, (*e.g.,* the reduction of platelet adhesion and/or cell migration). The specific dose will vary depending on the particular compounds chosen, the dosing regimen to be followed, whether the compound is administered in combination with other compounds, timing of administration, the tissue to which it is administered, and the physical delivery system in which the compound is carried.

A "therapeutic effect" as that term is used herein, encompasses a therapeutic benefit and/or a prophylactic benefit as described herein. A prophylactic effect includes delaying or eliminating the appearance of a disease or condition, delaying or eliminating the onset of symptoms of a disease or condition, slowing, halting, or reversing the progression of a disease or condition, or any combination thereof.

The term "pharmaceutically acceptable salt" refers to salts derived from a variety of organic and inorganic counter ions known in the art. Pharmaceutically acceptable acid addition salts can be formed with inorganic acids and organic acids. Inorganic acids from which salts can be derived include, for example, hydrochloric acid, hydrobromic acid, sulfuric acid, nitric acid and phosphoric acid. Organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid and salicylic acid. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese and aluminum. Organic bases from which salts can be derived include, for example, primary, secondary, and tertiary amines, substituted amines including naturally occurring substituted amines, cyclic amines and basic ion exchange resins. Specific examples include isopropylamine, trimethylamine, diethylamine, triethylamine, tripropylamine, and ethanolamine. In some embodiments, the pharmaceutically acceptable base addition salt is chosen from ammonium, potassium, sodium, calcium, and magnesium salts.

"Pharmaceutically acceptable carrier" or "pharmaceutically acceptable excipient" is intended to include any and all solvents, dispersion media, coatings, antibacterial and antifungal agents, isotonic and absorption delaying agents, and inert ingredients. The use of such pharmaceutically acceptable carriers or pharmaceutically acceptable excipients for active pharmaceutical ingredients is well known in the art. Except insofar as any conventional pharmaceutically acceptable carrier or pharmaceutically acceptable excipient is incompatible with the active pharmaceutical ingredient, its use in the therapeutic compositions of the invention is contemplated. Additional active pharmaceutical ingredients, such as other drugs, can also be incorporated into the described compositions and methods.

The terms "QD," "qd," or "q.d." mean *quaque die*, once a day, or once daily. The terms "BID," "bid," or "b.i.d." mean *bis in die*, twice a day, or twice daily. The terms "TID," "tid," or "t.i.d." mean *ter in die*, three times a day, or three times daily. The terms "QID," "qid," or "q.i.d." mean *quater in die*, four times a day, or four times daily.

The term *"in vivo"* refers to an event that takes place in a subject's body.

The term *"in vitro"* refers to an event that takes places outside of a subject's body. *In vitro* assays encompass cell-based assays in which cells alive or dead are employed and may also encompass a cell-free assay in which no intact cells are employed.

Unless otherwise stated, the chemical structures depicted herein are intended to include compounds which differ only in the presence of one or more isotopically enriched atoms. For example, compounds where one or more hydrogen atoms is replaced by deuterium or tritium, or wherein one or more carbon atoms is replaced by ¹³C- or ¹⁴C-enriched carbons, are within the scope of this invention.

When ranges are used herein to describe, for example, physical or chemical properties such as molecular weight or chemical formulae, all combinations and subcombinations of ranges and specific embodiments therein are intended to be included. Use of the term "about" when referring to a number or a numerical range means that the number or numerical range referred to is an approximation within experimental variability (or within statistical experimental error), and thus the number or numerical range may vary. The variation is typically from 0% to 15%, preferably from 0% to 10%, more preferably from 0% to 5% of the stated number or numerical range. The term "comprising" (and related terms such as "comprise" or "comprises" or "having" or "including") includes those embodiments such as, for example, an embodiment of any composition of matter, method or process that "consist of' or "consist essentially of' the described features.

"Alkyl" refers to a straight or branched hydrocarbon chain radical consisting solely of carbon and hydrogen atoms, containing no unsaturation, having from one to ten carbon atoms (e.g., (Ci-io)alkyl or C₁-₁₀ alkyl). Whenever it appears herein, a numerical range such as "1 to 10" refers to each integer in the given range - *e.g.,* "1 to 10 carbon atoms" means that the alkyl group may consist of 1 carbon atom, 2 carbon atoms, 3 carbon atoms, *etc.,* up to and including 10 carbon atoms, although the definition is also intended to cover the occurrence of the term "alkyl" where no numerical range is specifically designated. Typical alkyl groups include, but are in no way limited to, methyl, ethyl, propyl, isopropyl, *n*-butyl, *iso*-butyl, sec-butyl isobutyl, *tert-butyl,* pentyl, isopentyl, neopentyl, hexyl, septyl, octyl, nonyl and decyl. The alkyl moiety may be attached to the rest of the molecule by a single bond, such as for example, methyl (Me), ethyl (Et), *n*-propyl (Pr), 1-methylethyl (*iso*-propyl), *n*-butyl, *n*-pentyl, 1,1-dimethylethyl (*tert*-butyl) and 3-methylhexyl. Unless stated otherwise specifically in the specification, an alkyl group is optionally substituted by one or more of substituents which are independently heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, -SR^{a}, -OC(O)-R^{a}, -N(R^{a})₂, -C(O)R^{a}, -C(O)OR^{a}, - OC(O)N(R^{a})₂, -C(O)N(R^{a})₂, -N(R^{a})C(O)OR^{a}, -N(R^{a})C(O)R^{a}, -N(R^{a})C(O)N(R^{a})₂, N(R^{a})C(NR^{a})N(R^{a})₂, -N(R^{a})S(O)ₜR^{a} (where t is 1 or 2), -S(O)ₜOR^{a} (where t is 1 or 2), -S(O)ₜN(R^{a})₂ (where t is 1 or 2), or PO₃(R^{a})₂ where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

"Alkylaryl" refers to an -(alkyl)aryl radical where aryl and alkyl are as disclosed herein, having from one to ten carbon atoms, and which are optionally substituted by one or more of the substituents described as suitable substituents for aryl and alkyl respectively.

"Alkylhetaryl" refers to an -(alkyl)hetaryl radical where hetaryl and alkyl are as disclosed herein, having from one to ten carbon atoms, and which are optionally substituted by one or more of the substituents described as suitable substituents for aryl and alkyl respectively.

"Alkylheterocycloalkyl" refers to an -(alkyl) heterocycyl radical where alkyl and heterocycloalkyl are as disclosed herein, having from one to ten carbon atoms, and which are optionally substituted by one or more of the substituents described as suitable substituents for heterocycloalkyl and alkyl respectively.

An "alkene" moiety refers to a group consisting of at least two carbon atoms and at least one carbon-carbon double bond, and an "alkyne" moiety refers to a group consisting of at least two carbon atoms and at least one carbon-carbon triple bond. The alkyl moiety, whether saturated or unsaturated, may be branched, straight chain, or cyclic.

"Alkenyl" refers to a straight or branched hydrocarbon chain radical group consisting solely of carbon and hydrogen atoms, containing at least one double bond, and having from two to ten carbon atoms (*i*.*e*., (C₂-₁₀)alkenyl or C₂-₁₀ alkenyl). Whenever it appears herein, a numerical range such as "2 to 10" refers to each integer in the given range - *e.g.,* "2 to 10 carbon atoms" means that the alkenyl group may consist of 2 carbon atoms, 3 carbon atoms, *etc.,* up to and including 10 carbon atoms. The alkenyl moiety may be attached to the rest of the molecule by a single bond, such as for example, ethenyl (*i*.*e*., vinyl), prop-1-enyl (*i*.*e*., allyl), but-1-enyl, pent-1-enyl and penta-1,4-dienyl. Unless stated otherwise specifically in the specification, an alkenyl group is optionally substituted by one or more substituents which are independently alkyl, heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, - SR^{a}, -OC(O)-R^{a}, -N(R^{a})₂, -C(O)R^{a}, -C(O)OR^{a}, -OC(O)N(R^{a})₂, -C(O)N(R^{a})₂, - N(R^{a})C(O)OR^{a}, -N(R^{a})C(O)R^{a}, -N(R^{a})C(O)N(R^{a})₂, N(R^{a})C(NR^{a})N(R^{a})₂, -N(R^{a})S(O)ₜR^{a} (where t is 1 or 2), -S(O)ₜOR^{a} (where t is 1 or 2), -S(O)ₜN(R^{a})₂ (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

"Alkenyl-cycloalkyl" refers to an -(alkenyl)cycloalkyl radical where alkenyl and cyclo alkyl are as disclosed herein, having from two to ten carbon atoms, and which are optionally substituted by one or more of the substituents described as suitable substituents for alkenyl and cycloalkyl respectively.

"Alkynyl" refers to a straight or branched hydrocarbon chain radical group consisting solely of carbon and hydrogen atoms, containing at least one triple bond, having from two to ten carbon atoms (i.e., (C₂-₁₀)alkynyl or C₂-₁₀ alkynyl). Whenever it appears herein, a numerical range such as "2 to 10" refers to each integer in the given range - *e.g.,* "2 to 10 carbon atoms" means that the alkynyl group may consist of 2 carbon atoms, 3 carbon atoms, *etc.,* up to and including 10 carbon atoms. The alkynyl may be attached to the rest of the molecule by a single bond, for example, ethynyl, propynyl, butynyl, pentynyl and hexynyl. Unless stated otherwise specifically in the specification, an alkynyl group is optionally substituted by one or more substituents which independently are: alkyl, heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, -SR^{a}, -OC(O)-R^{a}, -N(R^{a})₂, -C(O)R^{a}, -C(O)OR^{a}, -OC(O)N(R^{a})₂, -C(O)N(R^{a})₂, -N(R^{a})C(O)OR^{a}, -N(R^{a})C(O)R^{a}, -N(R^{a})C(O)N(R^{a})₂, N(R^{a})C(NR^{a})N(R^{a})₂, -N(R^{a})S(O)ₜR^{a} (where t is 1 or 2), -S(O)ₜOR^{a} (where t is 1 or 2), -S(O)ₜN(R^{a})₂ (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

"Alkynyl-cycloalkyl" refers to an -(alkynyl)cycloalkyl radical where alkynyl and cycloalkyl are as disclosed herein, having from two to ten carbon atoms, and which are optionally substituted by one or more of the substituents described as suitable substituents for alkynyl and cycloalkyl respectively.

"Carboxaldehyde" refers to a -(C=O)H radical.

"Carboxyl" refers to a -(C=O)OH radical.

"Cyano" refers to a -CN radical.

"Cycloalkyl" refers to a monocyclic or polycyclic radical that contains only carbon and hydrogen, and may be saturated, or partially unsaturated. Cycloalkyl groups include groups having from 3 to 10 ring atoms (i.e. (C₃-₁₀)cycloalkyl or C₃-₁₀ cycloalkyl). Whenever it appears herein, a numerical range such as "3 to 10" refers to each integer in the given range - *e.g.,* "3 to 10 carbon atoms" means that the cycloalkyl group may consist of 3 carbon atoms, *etc.,* up to and including 10 carbon atoms. Illustrative examples of cycloalkyl groups include, but are not limited to the following moieties: cyclopropyl, cyclobutyl, cyclopentyl, cyclopentenyl, cyclohexyl, cyclohexenyl, cycloheptyl, cyclooctyl, cyclononyl, cyclodecyl, and norbornyl. Unless stated otherwise specifically in the specification, a cycloalkyl group is optionally substituted by one or more substituents which independently are: alkyl, heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, -SR^{a}, -OC(O)-R^{a}, -N(R^{a})₂, -C(O)R^{a}, -C(O)OR^{a}, -OC(O)N(R^{a})₂, -C(O)N(R^{a})₂, -N(R^{a})C(O)OR^{a}, - N(R^{a})C(O)R^{a}, -N(R^{a})C(O)N(R^{a})₂, N(R^{a})C(NR^{a})N(R^{a})₂, -N(R^{a})S(O)ₜR^{a} (where t is 1 or 2), -S(O)ₜOR^{a} (where t is 1 or 2), -S(O)ₜN(R^{a})₂ (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

"Cycloalkyl-alkenyl" refers to a -(cycloalkyl)alkenyl radical where cycloalkyl and alkenyl are as disclosed herein, having from three to ten carbon atoms, and which are optionally substituted by one or more of the substituents described as suitable substituents for cycloalkyl and alkenyl, respectively.

"Cycloalkyl-heterocycloalkyl" refers to a -(cycloalkyl)heterocycloalkyl radical where cycloalkyl and heterocycloalkyl are as disclosed herein, having from three to ten carbon atoms, and which are optionally substituted by one or more of the substituents described as suitable substituents for cycloalkyl and heterocycloalkyl, respectively.

"Cycloalkyl-heteroaryl" refers to a -(cycloalkyl)heteroaryl radical where cycloalkyl and heteroaryl are as disclosed herein, having from three to ten carbon atoms, and which are optionally substituted by one or more of the substituents described as suitable substituents for cycloalkyl and heteroaryl, respectively.

The term "alkoxy" refers to the group -O-alkyl, including from 1 to 8 carbon atoms of a straight, branched, cyclic configuration and combinations thereof attached to the parent structure through an oxygen. Examples include, but are not limited to, methoxy, ethoxy, propoxy, isopropoxy, cyclopropyloxy and cyclohexyloxy. "Lower alkoxy" refers to alkoxy groups containing one to six carbons.

The term "substituted alkoxy" refers to alkoxy wherein the alkyl constituent is substituted (*i*.*e*., -O-(substituted alkyl)). Unless stated otherwise specifically in the specification, the alkyl moiety of an alkoxy group is optionally substituted by one or more substituents which independently are: alkyl, heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, -SR^{a}, -OC(O)-R^{a}, -N(R^{a})₂, -C(O)R^{a}, -C(O)OR^{a}, -OC(O)N(R^{a})₂, -C(O)N(R^{a})₂, -N(R^{a})C(O)OR^{a}, - N(R^{a})C(O)R^{a}, -N(R^{a})C(O)N(R^{a})₂, N(R^{a})C(NR^{a})N(R^{a})₂, -N(R^{a})S(O)ₜR^{a} (where t is 1 or 2), -S(O)ₜOR^{a} (where t is 1 or 2), -S(O)ₜN(R^{a})₂ (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

The term "alkoxycarbonyl" refers to a group of the formula (alkoxy)(C=O)-attached through the carbonyl carbon wherein the alkoxy group has the indicated number of carbon atoms. Thus a (C₁-₆)alkoxycarbonyl group is an alkoxy group having from 1 to 6 carbon atoms attached through its oxygen to a carbonyl linker. "Lower alkoxycarbonyl" refers to an alkoxycarbonyl group wherein the alkoxy group is a lower alkoxy group.

The term "substituted alkoxycarbonyl" refers to the group (substituted alkyl)-O-C(O)- wherein the group is attached to the parent structure through the carbonyl functionality, and wherein the alkoxy group has the indicated number of carbon atoms. Unless stated otherwise specifically in the specification, the alkyl moiety of an alkoxycarbonyl group is optionally substituted by one or more substituents which independently are: alkyl, heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, -SR^{a}, -OC(O)-R^{a}, -N(R^{a})₂, -C(O)R^{a}, -C(O)OR^{a}, -OC(O)N(R^{a})₂, -C(O)N(R^{a})₂, -N(R^{a})C(O)OR^{a}, -N(R^{a})C(O)R^{a}, -N(R^{a})C(O)N(R^{a})₂, N(R^{a})C(NR^{a})N(R^{a})₂, -N(R^{a})S(O)ₜR^{a} (where t is 1 or 2), -S(O)ₜOR^{a} (where t is 1 or 2), -S(O)ₜN(R^{a})₂ (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

"Acyl" refers to the groups (alkyl)-C(O)-, (aryl)-C(O)-, (heteroaryl)-C(O)-, (heteroalkyl)-C(O)- and (heterocycloalkyl)-C(O)-, having from one to ten carbon atoms, wherein the group is attached to the parent structure through the carbonyl functionality. If the R radical is heteroaryl or heterocycloalkyl, the hetero ring or chain atoms contribute to the total number of chain or ring atoms. Unless stated otherwise specifically in the specification, the alkyl, aryl or heteroaryl moiety of the acyl group is optionally substituted by one or more substituents which are independently alkyl, heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, - SR^{a}, -OC(O)-R^{a}, -N(R^{a})₂, -C(O)R^{a}, -C(O)OR^{a}, -OC(O)N(R^{a})₂, -C(O)N(R^{a})₂, - N(R^{a})C(O)OR^{a}, -N(R^{a})C(O)R^{a}, -N(R^{a})C(O)N(R^{a})₂, N(R^{a})C(NR^{a})N(R^{a})₂, -N(R^{a})S(O)ₜR^{a} (where t is 1 or 2), -S(O)ₜOR^{a} (where t is 1 or 2), -S(O)ₜN(R^{a})₂ (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

"Acyloxy" refers to a R(C=O)O- radical wherein "R" is alkyl, aryl, heteroaryl, heteroalkyl or heterocycloalkyl, which are as described herein. If the R radical is heteroaryl or heterocycloalkyl, the hetero ring or chain atoms contribute to the total number of chain or ring atoms. Unless stated otherwise specifically in the specification, the "R" of an acyloxy group is optionally substituted by one or more substituents which independently are: alkyl, heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, -SR^{a}, -OC(O)-R^{a}, -N(R^{a})₂, -C(O)R^{a}, -C(O)OR^{a}, -OC(O)N(R^{a})₂, -C(O)N(R^{a})₂, -N(R^{a})C(O)OR^{a}, -N(R^{a})C(O)R^{a}, -N(R^{a})C(O)N(R^{a})₂, N(R^{a})C(NR^{a})N(R^{a})₂, -N(R^{a})S(O)ₜR^{a} (where t is 1 or 2), -S(O)ₜOR^{a} (where t is 1 or 2), -S(O)ₜN(R^{a})₂ (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

"Amino" or "amine" refers to a -N(R^{a})₂ radical group, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl, unless stated otherwise specifically in the specification. When a -N(R^{a})₂ group has two R^{a} substituents other than hydrogen, they can be combined with the nitrogen atom to form a 4-, 5-, 6- or 7-membered ring. For example, -N(R^{a})₂ is intended to include, but is not limited to, 1-pyrrolidinyl and 4-morpholinyl. Unless stated otherwise specifically in the specification, an amino group is optionally substituted by one or more substituents which independently are: alkyl, heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, -SR^{a}, -OC(O)-R^{a}, -N(R^{a})₂, -C(O)R^{a}, -C(O)OR^{a}, - OC(O)N(R^{a})₂, -C(O)N(R^{a})₂, -N(R^{a})C(O)OR^{a}, -N(R^{a})C(O)R^{a}, -N(R^{a})C(O)N(R^{a})₂, N(R^{a})C(NR^{a})N(R^{a})₂, -N(R^{a})S(O)ₜR^{a} (where t is 1 or 2), -S(O)ₜOR^{a} (where t is 1 or 2), -S(O)ₜN(R^{a})₂ (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

The term "substituted amino" also refers to N-oxides of the groups -NHR^{d}, and NR^{d}R^{d} each as described above. N-oxides can be prepared by treatment of the corresponding amino group with, for example, hydrogen peroxide or m-chloroperoxybenzoic acid.

"Amide" or "amido" refers to a chemical moiety with formula -C(O)N(R)₂ or -NHC(O)R, where R is selected from the group consisting of hydrogen, alkyl, cycloalkyl, aryl, heteroaryl (bonded through a ring carbon) and heteroalicyclic (bonded through a ring carbon), each of which moiety may itself be optionally substituted. The R₂ of -N(R)₂ of the amide may optionally be taken together with the nitrogen to which it is attached to form a 4-, 5-, 6- or 7-membered ring. Unless stated otherwise specifically in the specification, an amido group is optionally substituted independently by one or more of the substituents as described herein for alkyl, cycloalkyl, aryl, heteroaryl, or heterocycloalkyl. An amide may be an amino acid or a peptide molecule attached to a compound disclosed herein, thereby forming a prodrug. The procedures and specific groups to make such amides are known to those of skill in the art and can readily be found in seminal sources such as Greene and Wuts, Protective Groups in Organic Synthesis, 3rd Ed., John Wiley & Sons, New York, N.Y., 1999, which is incorporated herein by reference in its entirety.

"Aromatic" or "aryl" or "Ar" refers to an aromatic radical with six to ten ring atoms (e.g., C₆-C₁₀ aromatic or C₆-C₁₀ aryl) which has at least one ring having a conjugated pi electron system which is carbocyclic (e.g., phenyl, fluorenyl, and naphthyl). Bivalent radicals formed from substituted benzene derivatives and having the free valences at ring atoms are named as substituted phenylene radicals. Bivalent radicals derived from univalent polycyclic hydrocarbon radicals whose names end in "-yl" by removal of one hydrogen atom from the carbon atom with the free valence are named by adding "-idene" to the name of the corresponding univalent radical, *e.g.,* a naphthyl group with two points of attachment is termed naphthylidene. Whenever it appears herein, a numerical range such as "6 to 10" refers to each integer in the given range; *e.g.,* "6 to 10 ring atoms" means that the aryl group may consist of 6 ring atoms, 7 ring atoms, *etc.,* up to and including 10 ring atoms. The term includes monocyclic or fused-ring polycyclic (*i.e.,* rings which share adjacent pairs of ring atoms) groups. Unless stated otherwise specifically in the specification, an aryl moiety is optionally substituted by one or more substituents which are independently alkyl, heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, -SR^{a}, -OC(O)-R^{a}, - N(R^{a})₂, -C(O)R^{a}, -C(O)OR^{a}, -OC(O)N(R^{a})₂, -C(O)N(R^{a})₂, -N(R^{a})C(O)OR^{a}, - N(R^{a})C(O)R^{a}, -N(R^{a})C(O)N(R^{a})₂, N(R^{a})C(NR^{a})N(R^{a})₂, -N(R^{a})S(O)ₜR^{a} (where t is 1 or 2), -S(O)ₜOR^{a} (where t is 1 or 2), -S(O)ₜN(R^{a})₂ (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

"Aralkyl" or "arylalkyl" refers to an (aryl)alkyl-radical where aryl and alkyl are as disclosed herein and which are optionally substituted by one or more of the substituents described as suitable substituents for aryl and alkyl respectively.

"Ester" refers to a chemical radical of formula -COOR, where R is selected from the group consisting of alkyl, cycloalkyl, aryl, heteroaryl (bonded through a ring carbon) and heteroalicyclic (bonded through a ring carbon). The procedures and specific groups to make esters are known to those of skill in the art and can readily be found in seminal sources such as Greene and Wuts, Protective Groups in Organic Synthesis, 3rd Ed., John Wiley & Sons, New York, N.Y., 1999, which is incorporated herein by reference in its entirety. Unless stated otherwise specifically in the specification, an ester group is optionally substituted by one or more substituents which independently are: alkyl, heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, -SR^{a}, -OC(O)-R^{a}, -N(R^{a})₂, -C(O)R^{a}, -C(O)OR^{a}, - OC(O)N(R^{a})₂, -C(O)N(R^{a})₂, -N(R^{a})C(O)OR^{a}, -N(R^{a})C(O)R^{a}, -N(R^{a})C(O)N(R^{a})₂, N(R^{a})C(NR^{a})N(R^{a})₂, -N(R^{a})S(O)ₜR^{a} (where t is 1 or 2), -S(O)ₜOR^{a} (where t is 1 or 2), -S(O)ₜN(R^{a})₂ (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

"Fluoroalkyl" refers to an alkyl radical, as defined above, that is substituted by one or more fluoro radicals, as defined above, for example, trifluoromethyl, difluoromethyl, 2,2,2-trifluoroethyl, and 1-fluoromethyl-2-fluoroethyl. The alkyl part of the fluoroalkyl radical may be optionally substituted as defined above for an alkyl group.

"Halo", "halide", or, alternatively, "halogen" is intended to mean fluoro, chloro, bromo or iodo. The terms "haloalkyl," "haloalkenyl," "haloalkynyl" and "haloalkoxy" include alkyl, alkenyl, alkynyl and alkoxy structures that are substituted with one or more halo groups or with combinations thereof. For example, the terms "fluoroalkyl" and "fluoroalkoxy" include haloalkyl and haloalkoxy groups, respectively, in which the halo is fluorine.

"Heteroalkyl", "heteroalkenyl" and "heteroalkynyl" include optionally substituted alkyl, alkenyl and alkynyl radicals and which have one or more skeletal chain atoms selected from an atom other than carbon, e.g., oxygen, nitrogen, sulfur, phosphorus or combinations thereof. A numerical range may be given - *e.g.,* C₁-C₄ heteroalkyl which refers to the chain length in total, which in this example is 4 atoms long. A heteroalkyl group may be substituted with one or more substituents which independently are: alkyl, heteroalkyl, alkenyl, alkynyl, cycloalkyl, heterocycloalkyl, aryl, arylalkyl, heteroaryl, heteroarylalkyl, hydroxy, halo, cyano, nitro, oxo, thioxo, trimethylsilanyl, -OR^{a}, -SR^{a}, - OC(O)-R^{a}, -N(R^{a})₂, -C(O)R^{a}, -C(O)OR^{a}, -OC(O)N(R^{a})₂, -C(O)N(R^{a})₂, -N(R^{a})C(O)OR^{a}, - N(R^{a})C(O)R^{a}, -N(R^{a})C(O)N(R^{a})₂, N(R^{a})C(NR^{a})N(R^{a})₂, -N(R^{a})S(O)ₜR^{a} (where t is 1 or 2), -S(O)ₜOR^{a} (where t is 1 or 2), -S(O)ₜN(R^{a})₂ (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

"Heteroalkylaryl" refers to an -(heteroalkyl)aryl radical where heteroalkyl and aryl are as disclosed herein and which are optionally substituted by one or more of the substituents described as suitable substituents for heteroalkyl and aryl, respectively.

"Heteroalkylheteroaryl" refers to an -(heteroalkyl)heteroaryl radical where heteroalkyl and heteroaryl are as disclosed herein and which are optionally substituted by one or more of the substituents described as suitable substituents for heteroalkyl and heteroaryl, respectively.

"Heteroalkylheterocycloalkyl" refers to an -(heteroalkyl)heterocycloalkyl radical where heteroalkyl and heterocycloalkyl are as disclosed herein and which are optionally substituted by one or more of the substituents described as suitable substituents for heteroalkyl and heterocycloalkyl, respectively.

"Heteroalkylcycloalkyl" refers to an -(heteroalkyl)cycloalkyl radical where heteroalkyl and cycloalkyl are as disclosed herein and which are optionally substituted by one or more of the substituents described as suitable substituents for heteroalkyl and cycloalkyl, respectively.

"Heteroaryl" or "heteroaromatic" or "HetAr" refers to a 5- to 18-membered aromatic radical (*e.g.,* C₅-C₁₃ heteroaryl) that includes one or more ring heteroatoms selected from nitrogen, oxygen and sulfur, and which may be a monocyclic, bicyclic, tricyclic or tetracyclic ring system. Whenever it appears herein, a numerical range such as "5 to 18" refers to each integer in the given range - *e.g.,* "5 to 18 ring atoms" means that the heteroaryl group may consist of 5 ring atoms, 6 ring atoms, etc., up to and including 18 ring atoms. Bivalent radicals derived from univalent heteroaryl radicals whose names end in "-yl" by removal of one hydrogen atom from the atom with the free valence are named by adding "-idene" to the name of the corresponding univalent radical - *e.g.,* a pyridyl group with two points of attachment is a pyridylidene. A N-containing "heteroaromatic" or "heteroaryl" moiety refers to an aromatic group in which at least one of the skeletal atoms of the ring is a nitrogen atom. The polycyclic heteroaryl group may be fused or non-fused. The heteroatom(s) in the heteroaryl radical are optionally oxidized. One or more nitrogen atoms, if present, are optionally quaternized. The heteroaryl may be attached to the rest of the molecule through any atom of the ring(s). Examples of heteroaryls include, but are not limited to, azepinyl, acridinyl, benzimidazolyl, benzindolyl, 1,3-benzodioxolyl, benzofuranyl, benzooxazolyl, benzo[*d*]thiazolyl, benzothiadiazolyl, benzo[*b*][1,4]dioxepinyl, benzo[*b*][1,4]oxazinyl, 1,4-benzodioxanyl, benzonaphthofuranyl, benzoxazolyl, benzodioxolyl, benzodioxinyl, benzoxazolyl, benzopyranyl, benzopyranonyl, benzofuranyl, benzofuranonyl, benzofurazanyl, benzothiazolyl, benzothienyl(benzothiophenyl), benzothieno[3,2-*d*]pyrimidinyl, benzotriazolyl, benzo[4,6]imidazo[1,2-*a*]pyridinyl, carbazolyl, cinnolinyl, cyclopenta[*d*]pyrimidinyl, 6,7-dihydro-5*H*-cyclopenta[4,5]thieno[2,3-*d*]pyrimidinyl, 5,6-dihydrobenzo[*h*]quinazolinyl, 5,6-dihydrobenzo[*h*]cinnolinyl, 6,7-dihydro-5*H-*benzo[6,7]cyclohepta[1,2-*c*]pyridazinyl, dibenzofuranyl, dibenzothiophenyl, furanyl, furazanyl, furanonyl, furo[3,2-c]pyridinyl, 5,6,7,8,9,10-hexahydrocycloocta[*d*]pyrimidinyl, 5,6,7,8,9,10-hexahydrocycloocta[*d*]pyridazinyl, 5,6,7,8,9,10-hexahydrocycloocta[*d*]pyridinyl, isothiazolyl, imidazolyl, indazolyl, indolyl, indazolyl, isoindolyl, indolinyl, isoindolinyl, isoquinolyl, indolizinyl, isoxazolyl, 5,8-methano-5,6,7,8-tetrahydroquinazolinyl, naphthyridinyl, 1,6-naphthyridinonyl, oxadiazolyl, 2-oxoazepinyl, oxazolyl, oxiranyl, 5,6,6a,7,8,9,10,10a-octahydrobenzo[*h*]quinazolinyl, 1-phenyl-1*H*-pyrrolyl, phenazinyl, phenothiazinyl, phenoxazinyl, phthalazinyl, pteridinyl, purinyl, pyranyl, pyrrolyl, pyrazolyl, pyrazolo[3,4-*d*]pyrimidinyl, pyridinyl, pyrido[3,2-*d*]pyrimidinyl, pyrido[3,4-*d*]pyrimidinyl, pyrazinyl, pyrimidinyl, pyridazinyl, pyrrolyl, quinazolinyl, quinoxalinyl, quinolinyl, isoquinolinyl, tetrahydroquinolinyl, 5,6,7,8-tetrahydroquinazolinyl, 5,6,7,8-tetrahydrobenzo[4,5]thieno[2,3-*d*]pyrimidinyl, 6,7,8,9-tetrahydro-5*H-*cyclohepta[4,5]thieno[2,3-*d*]pyrimidinyl, 5,6,7,8-tetrahydropyrido[4,5-c]pyridazinyl, thiazolyl, thiadiazolyl, thiapyranyl, triazolyl, tetrazolyl, triazinyl, thieno[2,3-*d*]pyrimidinyl, thieno[3,2-*d*]pyrimidinyl, thieno[2,3-c]pyridinyl, and thiophenyl (*i.e.* thienyl). Unless stated otherwise specifically in the specification, a heteroaryl moiety is optionally substituted by one or more substituents which are independently: alkyl, heteroalkyl, alkenyl, alkynyl, cycloalkyl, heterocycloalkyl, aryl, arylalkyl, heteroaryl, heteroarylalkyl, hydroxy, halo, cyano, nitro, oxo, thioxo, trimethylsilanyl, -OR^{a}, -SR^{a}, - OC(O)-R^{a}, -N(R^{a})₂, -C(O)R^{a}, -C(O)OR^{a}, -OC(O)N(R^{a})₂, -C(O)N(R^{a})₂, -N(R^{a})C(O)OR^{a}, - N(R^{a})C(O)R^{a}, -N(R^{a})C(O)N(R^{a})₂, N(R^{a})C(NR^{a})N(R^{a})₂, -N(R^{a})S(O)ₜR^{a} (where t is 1 or 2), -S(O)ₜOR^{a} (where t is 1 or 2), -S(O)ₜN(R^{a})₂ (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

Substituted heteroaryl also includes ring systems substituted with one or more oxide (-O-) substituents, such as, for example, pyridinyl N-oxides.

"Heteroarylalkyl" refers to a moiety having an aryl moiety, as described herein, connected to an alkylene moiety, as described herein, wherein the connection to the remainder of the molecule is through the alkylene group.

"Heterocycloalkyl" refers to a stable 3- to 18-membered non-aromatic ring radical that comprises two to twelve carbon atoms and from one to six heteroatoms selected from nitrogen, oxygen and sulfur. Whenever it appears herein, a numerical range such as "3 to 18" refers to each integer in the given range - *e.g.,* "3 to 18 ring atoms" means that the heterocycloalkyl group may consist of 3 ring atoms, 4 ring atoms, *etc.,* up to and including 18 ring atoms. Unless stated otherwise specifically in the specification, the heterocycloalkyl radical is a monocyclic, bicyclic, tricyclic or tetracyclic ring system, which may include fused or bridged ring systems. The heteroatoms in the heterocycloalkyl radical may be optionally oxidized. One or more nitrogen atoms, if present, are optionally quaternized. The heterocycloalkyl radical is partially or fully saturated. The heterocycloalkyl may be attached to the rest of the molecule through any atom of the ring(s). Examples of such heterocycloalkyl radicals include, but are not limited to, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl. Unless stated otherwise specifically in the specification, a heterocycloalkyl moiety is optionally substituted by one or more substituents which independently are: alkyl, heteroalkyl, alkenyl, alkynyl, cycloalkyl, heterocycloalkyl, aryl, arylalkyl, heteroaryl, heteroarylalkyl, hydroxy, halo, cyano, nitro, oxo, thioxo, trimethylsilanyl, -OR^{a}, -SR^{a}, -OC(O)-R^{a}, -N(R^{a})₂, -C(O)R^{a}, -C(O)OR^{a}, -OC(O)N(R^{a})₂, - C(O)N(R^{a})₂, -N(R^{a})C(O)OR^{a}, -N(R^{a})C(O)R^{a}, -N(R^{a})C(O)N(R^{a})₂, N(R^{a})C(NR^{a})N(R^{a})₂, - N(R^{a})S(O)ₜR^{a} (where t is 1 or 2), -S(O)ₜOR^{a} (where t is 1 or 2), -S(O)ₜN(R^{a})₂ (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

"Heterocycloalkyl" also includes bicyclic ring systems wherein one non-aromatic ring, usually with 3 to 7 ring atoms, contains at least 2 carbon atoms in addition to 1-3 heteroatoms independently selected from oxygen, sulfur, and nitrogen, as well as combinations comprising at least one of the foregoing heteroatoms; and the other ring, usually with 3 to 7 ring atoms, optionally contains 1-3 heteroatoms independently selected from oxygen, sulfur, and nitrogen and is not aromatic.

"Nitro" refers to the -NO₂ radical.

"Oxa" refers to the -O- radical.

"Oxo" refers to the =O radical.

"Sulfanyl" refers to groups that include -S-(optionally substituted alkyl), -S-(optionally substituted aryl), -S-(optionally substituted heteroaryl) and -S-(optionally substituted heterocycloalkyl).

"Sulfinyl" refers to groups that include -S(O)-H, -S(O)-(optionally substituted alkyl), -S(O)-(optionally substituted amino), -S(O)-(optionally substituted aryl), -S(O)-(optionally substituted heteroaryl) and -S(O)-(optionally substituted heterocycloalkyl).

"Sulfonyl" refers to groups that include -S(O₂)-H, -S(O₂)-(optionally substituted alkyl), -S(O₂)-(optionally substituted amino), -S(O₂)-(optionally substituted aryl), -S(O₂)-(optionally substituted heteroaryl), and -S(O₂)-(optionally substituted heterocycloalkyl).

"Sulfonamidyl" or "sulfonamido" refers to a -S(=O)₂-NRR radical, where each R is selected independently from the group consisting of hydrogen, alkyl, cycloalkyl, aryl, heteroaryl (bonded through a ring carbon) and heteroalicyclic (bonded through a ring carbon). The R groups in -NRR of the -S(=O)₂-NRR radical may be taken together with the nitrogen to which it is attached to form a 4-, 5-, 6- or 7-membered ring. A sulfonamido group is optionally substituted by one or more of the substituents described for alkyl, cycloalkyl, aryl, heteroaryl, respectively.

"Sulfoxyl" refers to a -S(=O)₂OH radical.

"Sulfonate" refers to a -S(=O)₂-OR radical, where R is selected from the group consisting of alkyl, cycloalkyl, aryl, heteroaryl (bonded through a ring carbon) and heteroalicyclic (bonded through a ring carbon). A sulfonate group is optionally substituted on R by one or more of the substituents described for alkyl, cycloalkyl, aryl, heteroaryl, respectively. "Isomers" are different compounds that have the same molecular formula. "Stereoisomers" are isomers that differ only in the way the atoms are arranged in space - *i.e.,* having a different stereochemical configuration. "Enantiomers" are a pair of stereoisomers that are non-superimposable mirror images of each other. A 1:1 mixture of a pair of enantiomers is a "racemic" mixture. The term "(±)" is used to designate a racemic mixture where appropriate. "Diastereoisomers" are stereoisomers that have at least two asymmetric atoms, but which are not mirror-images of each other. The absolute stereochemistry is specified according to the Cahn-Ingold-Prelog R-S system. When a compound is a pure enantiomer the stereochemistry at each chiral carbon can be specified by either (R) or (*S*)*.* Resolved compounds whose absolute configuration is unknown can be designated (+) or (-) depending on the direction (dextro- or levorotatory) which they rotate plane polarized light at the wavelength of the sodium D line. Certain of the compounds described herein contain one or more asymmetric centers and can thus give rise to enantiomers, diastereomers, and other stereoisomeric forms that can be defined, in terms of absolute stereochemistry, as (R) or (*S*)*.* The present chemical entities, pharmaceutical compositions and methods are meant to include all such possible isomers, including racemic mixtures, optically pure forms and intermediate mixtures. Optically active (R)- and (*S*)-isomers can be prepared using chiral synthons or chiral reagents, or resolved using conventional techniques. When the compounds described herein contain olefinic double bonds or other centers of geometric asymmetry, and unless specified otherwise, it is intended that the compounds include both E and Z geometric isomers.

"Enantiomeric purity" as used herein refers to the relative amounts, expressed as a percentage, of the presence of a specific enantiomer relative to the other enantiomer. For example, if a compound, which may potentially have an (R)- or an (*S*)-isomeric configuration, is present as a racemic mixture, the enantiomeric purity is about 50% with respect to either the (R)- or (*S*)-isomer. If that compound has one isomeric form predominant over the other, for example, 80% (*S*)-isomer and 20% (*R*)-isomer, the enantiomeric purity of the compound with respect to the (S)-isomeric form is 80%. The enantiomeric purity of a compound can be determined in a number of ways known in the art, including chromatography using a chiral support, polarimetric measurement of the rotation of polarized light, nuclear magnetic resonance spectroscopy using chiral shift reagents which include but are not limited to lanthanide containing chiral complexes or Pirkle's reagents, or derivatization of a compounds using a chiral compound such as Mosher's acid followed by chromatography or nuclear magnetic resonance spectroscopy.

In preferred embodiments, the enantiomerically enriched composition has a higher potency with respect to therapeutic utility per unit mass than does the racemic mixture of that composition. Enantiomers can be isolated from mixtures by methods known to those skilled in the art, including chiral high pressure liquid chromatography (HPLC) and the formation and crystallization of chiral salts; or preferred enantiomers can be prepared by asymmetric syntheses. See, for example, Jacques, et al., Enantiomers, Racemates and Resolutions (Wiley Interscience, New York, 1981); Eliel, Stereochemistry of Carbon Compounds (McGraw-Hill, NY, 1962); and Eliel and Wilen, Stereochemistry of Organic Compounds (Wiley-Interscience, New York, 1994).

The terms "enantiomerically enriched" and "non-racemic," as used herein, refer to compositions in which the percent by weight of one enantiomer is greater than the amount of that one enantiomer in a control mixture of the racemic composition (e.g., greater than 1:1 by weight). For example, an enantiomerically enriched preparation of the (*S*)-enantiomer, means a preparation of the compound having greater than 50% by weight of the (*S*)-enantiomer relative to the (*R*)-enantiomer, such as at least 75% by weight, or such as at least 80% by weight. In some embodiments, the enrichment can be significantly greater than 80% by weight, providing a "substantially enantiomerically enriched" or a "substantially non-racemic" preparation, which refers to preparations of compositions which have at least 85% by weight of one enantiomer relative to other enantiomer, such as at least 90% by weight, or such as at least 95% by weight. The terms "enantiomerically pure" or "substantially enantiomerically pure" refers to a composition that comprises at least 98% of a single enantiomer and less than 2% of the opposite enantiomer.

"Moiety" refers to a specific segment or functional group of a molecule. Chemical moieties are often recognized chemical entities embedded in or appended to a molecule.

"Tautomers" are structurally distinct isomers that interconvert by tautomerization. "Tautomerization" is a form of isomerization and includes prototropic or proton-shift tautomerization, which is considered a subset of acid-base chemistry. "Prototropic tautomerization" or "proton-shift tautomerization" involves the migration of a proton accompanied by changes in bond order, often the interchange of a single bond with an adjacent double bond. Where tautomerization is possible (e.g. in solution), a chemical equilibrium of tautomers can be reached. An example of tautomerization is keto-enol tautomerization. A specific example of keto-enol tautomerization is the interconversion of pentane-2,4-dione and 4-hydroxypent-3-en-2-one tautomers. Another example of tautomerization is phenol-keto tautomerization. A specific example of phenol-keto tautomerization is the interconversion of pyridin-4-ol and pyridin-4(1*H*)-one tautomers.

A "leaving group or atom" is any group or atom that will, under selected reaction conditions, cleave from the starting material, thus promoting reaction at a specified site. Examples of such groups, unless otherwise specified, include halogen atoms and mesyloxy, p-nitrobenzensulphonyloxy and tosyloxy groups.

"Protecting group" is intended to mean a group that selectively blocks one or more reactive sites in a multifunctional compound such that a chemical reaction can be carried out selectively on another unprotected reactive site and the group can then be readily removed after the selective reaction is complete. A variety of protecting groups are disclosed, for example, in T. H. Greene and P. G. M. Wuts, Protective Groups in Organic Synthesis, Third Edition, John Wiley & Sons, New York (1999).

"Solvate" refers to a compound in physical association with one or more molecules of a pharmaceutically acceptable solvent.

"Substituted" means that the referenced group may have attached one or more additional groups, radicals or moieties individually and independently selected from, for example, acyl, alkyl, alkylaryl, cycloalkyl, aralkyl, aryl, carbohydrate, carbonate, heteroaryl, heterocycloalkyl, hydroxy, alkoxy, aryloxy, mercapto, alkylthio, arylthio, cyano, halo, carbonyl, ester, thiocarbonyl, isocyanato, thiocyanato, isothiocyanato, nitro, oxo, perhaloalkyl, perfluoroalkyl, phosphate, silyl, sulfinyl, sulfonyl, sulfonamidyl, sulfoxyl, sulfonate, urea, and amino, including mono- and di-substituted amino groups, and protected derivatives thereof. The substituents themselves may be substituted, for example, a cycloalkyl substituent may itself have a halide substituent at one or more of its ring carbons. The term "optionally substituted" means optional substitution with the specified groups, radicals or moieties.

As used herein, the term "warhead" or "warhead group" refers to a functional group present on a compound disclosed herein wherein that functional group is capable of covalently binding to an amino acid residue (such as cysteine, lysine, histidine, or other residues capable of being covalently modified) present in the binding pocket of the target protein, thereby irreversibly inhibiting the protein.

Compounds disclosed herein also include crystalline and amorphous forms of those compounds, including, for example, polymorphs, pseudopolymorphs, solvates, hydrates, unsolvated polymorphs (including anhydrates), conformational polymorphs, and amorphous forms of the compounds, as well as mixtures thereof. "Crystalline form" and "polymorph" are intended to include all crystalline and amorphous forms of the compound, including, for example, polymorphs, pseudopolymorphs, solvates, hydrates, unsolvated polymorphs (including anhydrates), conformational polymorphs, and amorphous forms, as well as mixtures thereof, unless a particular crystalline or amorphous form is referred to.

Compounds disclosed herein also include antibodies. The terms "antibody" and its plural form "antibodies" refer to whole immunoglobulins and any antigen-binding fragment ("antigen-binding portion") or single chains thereof. An "antibody" further refers to a glycoprotein comprising at least two heavy (H) chains and two light (L) chains inter-connected by disulfide bonds, or an antigen-binding portion thereof. Each heavy chain is comprised of a heavy chain variable region (abbreviated herein as V_{H}) and a heavy chain constant region. The heavy chain constant region is comprised of three domains, CHI, CH2 and CH3. Each light chain is comprised of a light chain variable region (abbreviated herein as V_{L}) and a light chain constant region. The light chain constant region is comprised of one domain, C_{L}. The V_{H} and V_{L} regions of an antibody may be further subdivided into regions of hypervariability, which are referred to as complementarity determining regions (CDR) or hypervariable regions (HVR), and which can be interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen epitope or epitopes. The constant regions of the antibodies may mediate the binding of the immunoglobulin to host tissues or factors, including various cells of the immune system (e.g., effector cells) and the first component (Clq) of the classical complement system.

The terms "monoclonal antibody," "mAb," "monoclonal antibody composition," or their plural forms refer to a preparation of antibody molecules of single molecular composition. A monoclonal antibody composition displays a single binding specificity and affinity for a particular epitope. Monoclonal antibodies specific to e.g. PD-1, PD-L1, or PD-L2 can be made using knowledge and skill in the art of injecting test subjects with PD-1, PD-L1, or PD-L2 antigen and then isolating hybridomas expressing antibodies having the desired sequence or functional characteristics. DNA encoding the monoclonal antibodies is readily isolated and sequenced using conventional procedures (e.g., by using oligonucleotide probes that are capable of binding specifically to genes encoding the heavy and light chains of the monoclonal antibodies). The hybridoma cells serve as a preferred source of such DNA. Once isolated, the DNA may be placed into expression vectors, which are then transfected into host cells such as E. coli cells, simian COS cells, Chinese hamster ovary (CHO) cells, or myeloma cells that do not otherwise produce immunoglobulin protein, to obtain the synthesis of monoclonal antibodies in the recombinant host cells. Recombinant production of antibodies will be described in more detail below.

The terms "antigen-binding portion" or "antigen-binding fragment" of an antibody (or simply "antibody portion"), as used herein, refers to one or more fragments of an antibody that retain the ability to specifically bind to an antigen (e.g., PD-1, PD-L1, or PD-L2). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the V_{L}, V_{H}, C_{L} and CHI domains; (ii) a F(ab')2 fragment, a bivalent fragment comprising two Fab fragments linked by a disulfide bridge at the hinge region; (iii) a Fd fragment consisting of the V_{H} and CHI domains; (iv) a Fv fragment consisting of the V_{L} and V_{H} domains of a single arm of an antibody, (v) a domain antibody (dAb) fragment (Ward et al., Nature, 1989, 341, 544-546), which may consist of a V_{H} or a V_{L} domain; and (vi) an isolated complementarity determining region (CDR). Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules known as single chain Fv (scFv); see e.g., Bird et al., Science 1988, 242, 423-426; and Huston et al., Proc. Natl. Acad. Sci. USA 1988, 85, 5879-5883). Such scFv chain antibodies are also intended to be encompassed within the terms "antigen-binding portion" or "antigen-binding fragment" of an antibody. These antibody fragments are obtained using conventional techniques known to those with skill in the art, and the fragments are screened for utility in the same manner as are intact antibodies.

The term "human antibody," as used herein, is intended to include antibodies having variable regions in which both the framework and CDR regions are derived from human germline immunoglobulin sequences. Furthermore, if the antibody contains a constant region, the constant region also is derived from human germline immunoglobulin sequences. The human antibodies disclosed herein may include amino acid residues not encoded by human germline immunoglobulin sequences (e.g., mutations introduced by random or site-specific mutagenesis *in vitro* or by somatic mutation *in vivo*). The term "human antibody", as used herein, is not intended to include antibodies in which CDR sequences derived from the germline of another mammalian species, such as a mouse, have been grafted onto human framework sequences.

The term "human monoclonal antibody" refers to antibodies displaying a single binding specificity which have variable regions in which both the framework and CDR regions are derived from human germline immunoglobulin sequences. In one embodiment, the human monoclonal antibodies are produced by a hybridoma which includes a B cell obtained from a transgenic nonhuman animal, e.g., a transgenic mouse, having a genome comprising a human heavy chain transgene and a light chain transgene fused to an immortalized cell.

The term "recombinant human antibody", as used herein, includes all human antibodies that are prepared, expressed, created or isolated by recombinant means, such as (a) antibodies isolated from an animal (e.g., a mouse) that is transgenic or transchromosomal for human immunoglobulin genes or a hybridoma prepared therefrom (described further below), (b) antibodies isolated from a host cell transformed to express the human antibody, e.g., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable regions in which the framework and CDR regions are derived from human germline immunoglobulin sequences. In certain embodiments, however, such recombinant human antibodies can be subjected to *in vitro* mutagenesis (or, when an animal transgenic for human Ig sequences is used, *in vivo* somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

As used herein, "isotype" refers to the antibody class (e.g., IgM or IgG1) that is encoded by the heavy chain constant region genes.

The phrases "an antibody recognizing an antigen" and "an antibody specific for an antigen" are used interchangeably herein with the term "an antibody which binds specifically to an antigen."

The term "human antibody derivatives" refers to any modified form of the human antibody, e.g., a conjugate of the antibody and another active pharmaceutical ingredient or antibody. The term "conjugate" or "immunoconjugate" refers to an antibody, or a fragment thereof, conjugated to a therapeutic moiety, such as a bacterial toxin, a cytotoxic drug or a radionuclide-containing toxin. Toxic moieties can be conjugated to antibodies disclosed herein using methods available in the art.

The terms "humanized antibody," "humanized antibodies," and "humanized" are intended to refer to antibodies in which CDR sequences derived from the germline of another mammalian species, such as a mouse, have been grafted onto human framework sequences. Additional framework region modifications may be made within the human framework sequences. Humanized forms of non-human (for example, murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a 15 hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin. For further details, see Jones et al., Nature 1986, 321, 522-525; Riechmann et al., Nature 1988, 332, 323-329; and Presta, Curr. Op. Struct. Biol. 1992, 2, 593-596.

The term "chimeric antibody" is intended to refer to antibodies in which the variable region sequences are derived from one species and the constant region sequences are derived from another species, such as an antibody in which the variable region sequences are derived from a mouse antibody and the constant region sequences are derived from a human antibody.

A "diabody" is a small antibody fragment with two antigen-binding sites. The fragments comprises a heavy chain variable domain (V_{H}) connected to a light chain variable domain (V_{L}) in the same polypeptide chain (V_{H}-V_{L} or V_{L}-V_{H}). By using a linker that is too short to allow pairing between the two domains on the same chain, the domains are forced to pair with the complementary domains of another chain and create two antigen-binding sites. Diabodies are described more fully in, *e*.*g*., European Patent No. EP 404,097, International Patent Publication No. WO 93/11161; and Bolliger et al., Proc. Natl. Acad. Sci. USA 1993, 90, 6444-6448.

The term "glycosylation" refers to a modified derivative of an antibody. An aglycoslated antibody lacks glycosylation. Glycosylation can be altered to, for example, increase the affinity of the antibody for antigen. Such carbohydrate modifications can be accomplished by, for example, altering one or more sites of glycosylation within the antibody sequence. For example, one or more amino acid substitutions can be made that result in elimination of one or more variable region framework glycosylation sites to thereby eliminate glycosylation at that site. A glycosylation may increase the affinity of the antibody for antigen, as described in U.S. Patent Nos. 5,714,350 and 6,350,861. Additionally or alternatively, an antibody can be made that has an altered type of glycosylation, such as a hypofucosylated antibody having reduced amounts of fucosyl residues or an antibody having increased bisecting GlcNac structures. Such altered glycosylation patterns have been demonstrated to increase the ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies disclosed herein to thereby produce an antibody with altered glycosylation. For example, the cell lines Ms704, Ms705, and Ms709 lack the fucosyltransferase gene, FUT8 (alpha (1,6) fucosyltransferase), such that antibodies expressed in the Ms704, Ms705, and Ms709 cell lines lack fucose on their carbohydrates. The Ms704, Ms705, and Ms709 FUT8-/- cell lines were created by the targeted disruption of the FUT8 gene in CHO/DG44 cells using two replacement vectors (see e.g. U.S. Patent Publication No. 2004/0110704 or Yamane-Ohnuki, et al. Biotechnol. Bioeng., 2004, 87, 614-622). As another example, European Patent No. EP 1,176,195 describes a cell line with a functionally disrupted FUT8 gene, which encodes a fucosyl transferase, such that antibodies expressed in such a cell line exhibit hypofucosylation by reducing or eliminating the alpha 1,6 bond-related enzyme, and also describes cell lines which have a low enzyme activity for adding fucose to the N-acetylglucosamine that binds to the Fc region of the antibody or does not have the enzyme activity, for example the rat myeloma cell line YB2/0 (ATCC CRL 1662). International Patent Publication WO 03/035835 describes a variant CHO cell line, Lec 13 cells, with reduced ability to attach fucose to Asn(297)-linked carbohydrates, also resulting in hypofucosylation of antibodies expressed in that host cell (see also Shields, et al., J. Biol. Chem. 2002,277, 26733-26740. International Patent Publication WO 99/54342 describes cell lines engineered to express glycoprotein-modifying glycosyl transferases (e.g., beta(1,4)-N-acetylglucosaminyltransferase III (GnTIII)) such that antibodies expressed in the engineered cell lines exhibit increased bisecting GlcNac structures which results in increased ADCC activity of the antibodies (see also Umana, et al., Nat. Biotech. 1999, 17, 176-180). Alternatively, the fucose residues of the antibody may be cleaved off using a fucosidase enzyme. For example, the fucosidase alpha-L-fucosidase removes fucosyl residues from antibodies as described in Tarentino, et al., Biochem. 1975, 14, 5516-5523.

"Pegylation" refers to a modified antibody, or a fragment thereof, that typically is reacted with polyethylene glycol (PEG), such as a reactive ester or aldehyde derivative of PEG, under conditions in which one or more PEG groups become attached to the antibody or antibody fragment. Pegylation may, for example, increase the biological (e.g., serum) half life of the antibody. Preferably, the pegylation is carried out via an acylation reaction or an alkylation reaction with a reactive PEG molecule (or an analogous reactive water-soluble polymer). As used herein, the term "polyethylene glycol" is intended to encompass any of the forms of PEG that have been used to derivatize other proteins, such as mono (Ci-Cio) alkoxy- or aryloxy-polyethylene glycol or polyethylene glycol-maleimide. The antibody to be pegylated may be an aglycosylated antibody. Methods for pegylation are known in the art and can be applied to the antibodies disclosed herein, as described for example in European Patent Nos. EP 0154316 and EP 0401384.

As used herein, an antibody that "specifically binds to human PD-1" is intended to refer to an antibody that binds to human PD-1 with a K_{D} of 1×10⁻⁷ M or less, more preferably 5×10⁻⁸ M or less, more preferably 1×10⁻⁸ M or less, more preferably 5×10⁻⁹ M or less.

As used herein, an antibody that "specifically binds to human PD-L1" is intended to refer to an antibody that binds to human PD-L1 with a K_{D} of 1×10⁻⁷ M or less, more preferably 5×10⁻⁸ M or less, more preferably 1×10⁻⁸ M or less, more preferably 5×10⁻⁹ M or less.

As used herein, an antibody that "specifically binds to human PD-L2" is intended to refer to an antibody that binds to human PD-L2 with a K_{D} of 1×10⁻⁷ M or less, more preferably 5×10⁻⁸ M or less, more preferably 1×10⁻⁸ M or less, more preferably 5×10⁻⁹ M or less.

As used herein, an antibody that "specifically binds to human CD20" is intended to refer to an antibody that binds to human CD20 with a K_{D} of 1×10⁻⁷ M or less, more preferably 5×10⁻⁸ M or less, more preferably 1×10⁻⁸ M or less, more preferably 5×10⁻⁹ M or less.

The term "radioisotope-labeled complex" refers to both non-covalent and covalent attachment of a radioactive isotope, such as ⁹⁰Y, ¹¹¹In, or ¹³¹I, to an antibody, including conjugates.

The term "biosimilar" means a biological product that is highly similar to a U.S. licensed reference biological product notwithstanding minor differences in clinically inactive components, and for which there are no clinically meaningful differences between the biological product and the reference product in terms of the safety, purity, and potency of the product. Furthermore, a similar biological or "biosimilar" medicine is a biological medicine that is similar to another biological medicine that has already been authorized for use by the European Medicines Agency. The term "biosimilar" is also used synonymously by other national and regional regulatory agencies. Biological products or biological medicines are medicines that are made by or derived from a biological source, such as a bacterium or yeast. They can consist of relatively small molecules such as human insulin or erythropoietin, or complex molecules such as monoclonal antibodies. For example, if the reference anti-CD20 monoclonal antibody is rituximab, an anti-CD20 biosimilar monoclonal antibody approved by drug regulatory authorities with reference to rituximab is a "biosimilar to" rituximab or is a "biosimilar thereof" rituximab.

The term "hematological malignancy" refers to mammalian cancers and tumors of the hematopoietic and lymphoid tissues, including tissues of the blood, bone marrow, lymph nodes, and lymphatic system. Hematological malignancies are also referred to as "liquid tumors." Hematological malignancies include, but are not limited to, ALL, CLL, SLL, acute myelogenous leukemia (AML), chronic myelogenous leukemia (CML), acute monocytic leukemia (AMoL), Hodgkin's lymphoma, and non-Hodgkin's lymphomas. The term "B cell hematological malignancy" refers to hematological malignancies that affect B cells.

The term "solid tumor" refers to an abnormal mass of tissue that usually does not contain cysts or liquid areas. Solid tumors may be benign or malignant. The term "solid tumor cancer" refers to malignant, neoplastic, or cancerous solid tumors. Solid tumor cancers include, but are not limited to, sarcomas, carcinomas, and lymphomas, such as cancers of the lung, breast, prostate, colon, rectum, and bladder. The tissue structure of solid tumors includes interdependent tissue compartments including the parenchyma (cancer cells) and the supporting stromal cells in which the cancer cells are dispersed and which may provide a supporting microenvironment.

The term "microenvironment," as used herein, may refer to the tumor microenvironment as a whole or to an individual subset of cells within the microenvironment.

For the avoidance of doubt, it is intended herein that particular features (for example integers, characteristics, values, uses, diseases, formulae, compounds or groups) described in conjunction with a particular aspect, embodiment or example of the invention are to be understood as applicable to any other aspect, embodiment or example described herein unless incompatible therewith. Thus such features may be used where appropriate in conjunction with any of the definition, claims or embodiments defined herein. All of the features disclosed in this specification (including any accompanying claims, abstract and drawings), and/or all of the steps of any method or process so disclosed, may be combined in any combination, except combinations where at least some of the features and/or steps are mutually exclusive.

### Co-administration of compounds

An aspect disclosed herein is a composition, such as a pharmaceutical composition, comprising a combination of a a BTK inhibitor and a PD-1 inhibitor, or a PD-L1 inhibitor, and optionally a PI3K inhibitor, a JAK-2 inhibitor or PD-L2 inhibitor for use in the treatment of cancer in a human subject, wherein the BTK inhibitor is 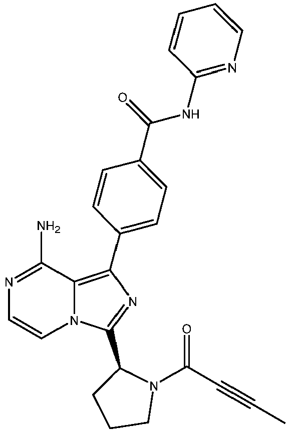 or a pharmaceutically acceptable salt thereof, the PD-1 inhibitor is pembrolizumab, and the PD-L1 inhibitor is durvalumab.

In an embodiment, the PI3K inhibitor is a PI3K-γ inhibitor.

In an embodiment, the PI3K inhibitor is a PI3K-δ inhibitor.

In an embodiment, the PI3K inhibitor is a PI3K-γ,δ inhibitor.

In an embodiment, the PI3K inhibitor is a selective PI3K inhibitor.

In an embodiment, the PI3K inhibitor, PI3K-γ inhibitor, PI3K-δ inhibitor, or PI3K-γ,δ inhibitor is in the form of a pharmaceutically acceptable salt, solvate, or hydrate, .

In an embodiment, the BTK inhibitor is in the form of a pharmaceutically acceptable salt. In an embodiment, the PI3K inhibitor, PI3K-γ inhibitor, PI3K-δ inhibitor, or PI3K-γ,δ inhibitor is administered to the subject before administration of the BTK inhibitor.

In an embodiment, the PI3K inhibitor, PI3K-γ inhibitor, PI3K-δ inhibitor, or PI3K-γ,δ inhibitor is administered concurrently with the administration of the BTK inhibitor.

In an embodiment, the PI3K inhibitor, PI3K-γ inhibitor, PI3K-δ inhibitor, PI3K-γ,δ inhibitor is administered to the subject after administration of the BTK inhibitor.

In an embodiment, the PD-1 inhibitor is administered to the subject before administration of the BTK inhibitor.

In an embodiment, the PD-1 inhibitor is administered concurrently with the administration of the BTK inhibitor.

In an embodiment, the PD-1 inhibitor is administered to the subject after administration of the BTK inhibitor.

In an embodiment, the BTK inhibitor, PD-1 inhibitor, JAK-2 inhibitor, and PI3K inhibitor are administered concurrently.

In an embodiment, the subject is a human.

### PI3K Inhibitors

The PI3K inhibitor may be any PI3K inhibitor known in the art. In particular, it is one of the PI3K inhibitors described in more detail in the following paragraphs. Preferably, it is a PI3K inhibitor selected from the group consisting of a PI3K-γ inhibitor, a PI3K-δ inhibitor, and a PI3K-γ,δ inhibitor. In one specific embodiment, it is a PI3K-δ inhibitor. For avoidance of doubt, references herein to a PI3K inhibitor may refer to a compound or a pharmaceutically acceptable salt, ester, solvate, or hydrate thereof.

In an embodiment, the PI3K inhibitor, which is preferably selected from the group consisting of a PI3K-γ inhibitor, a PI3K-δ inhibitor, and a PI3K-γ,δ inhibitor, is a compound selected from the structures disclosed in U.S. Patent Nos. 8,193,182 and 8,569,323, and U.S. Patent Application Publication Nos. 2012/0184568 A1, 2013/0344061 A1, and 2013/0267521 A1. In an embodiment, the PI3K inhibitor (which may be a PI3K-γ inhibitor, PI3K-δ inhibitor, or PI3K-γ,δ inhibitor) is a compound of Formula (I): 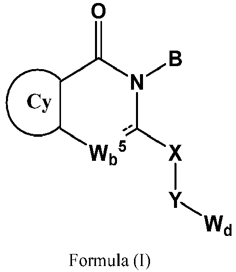 or a pharmaceutically acceptable salt, solvate, or hydrate thereof, wherein:
Cy is aryl or heteroaryl substituted by 0 or 1 occurrences of R³ and 0, 1, 2, or 3 occurrences of R⁵;
W_{b}⁵ is CR⁸, CHR⁸, or N;
R⁸ is hydrogen, alkyl, alkenyl, alkynyl, cycloalkyl, heteroalkyl, alkoxy, amido, amino, acyl, acyloxy, sulfonamido, halo, cyano, hydroxyl or nitro;
B is hydrogen, alkyl, amino, heteroalkyl, cycloalkyl, heterocyclyl, aryl, or heteroaryl, each of which is substituted with 0, 1, 2, 3, or 4 occurrences of R²;
each R² is independently alkyl, heteroalkyl, alkenyl, alkynyl, cycloalkyl, heterocyclyl, aryl, arylalkyl, heteroaryl, heteroarylalkyl, alkoxy, amido, amino, acyl, acyloxy, alkoxycarbonyl, sulfonamido, halo, cyano, hydroxyl, nitro, phosphate, urea, or carbonate;
X is -(CH(R⁹))_{z}-;
Y is -N(R⁹)-C(=O)-, -C(=O)-N(R⁹)-, -C(=O)-N(R⁹)-(CHR⁹)-, -N(R⁹)-S(=O)-, -S(=O)-N(R⁹)-, S(=O)₂-N(R⁹)-, -N(R⁹)-C(=O)-N(R⁹) or -N(R⁹)S(=O)₂-;
z is an integer of 1, 2, 3, or 4;
R³ is alkyl, alkenyl, alkynyl, cycloalkyl, heterocyclyl, fluoroalkyl, heteroalkyl, alkoxy, amido, amino, acyl, acyloxy, sulfinyl, sulfonyl, sulfoxide, sulfone, sulfonamido, halo, cyano, aryl, heteroaryl, hydroxyl, or nitro;
each R⁵ is independently alkyl, alkenyl, alkynyl, cycloalkyl, heteroalkyl, alkoxy, amido, amino, acyl, acyloxy, sulfonamido, halo, cyano, hydroxyl, or nitro;
each R⁹ is independently hydrogen, alkyl, cycloalkyl, heterocyclyl, or heteroalkyl; or two adjacent occurrences of R⁹ together with the atoms to which they are attached form a 4- to 7-membered ring;
W_{d} is heterocyclyl, aryl, cycloalkyl, or heteroaryl, each of which is substituted with one or more R¹⁰, R¹¹, R¹² or R¹³, and
R¹⁰, R¹¹, R¹² and R¹³ are each independently hydrogen, alkyl, heteroalkyl, alkenyl, alkynyl, cycloalkyl, heterocyclyl, aryl, arylalkyl, heteroaryl, heteroarylalkyl, alkoxy, heterocyclyloxy, amido, amino, acyl, acyloxy, alkoxycarbonyl, sulfonamido, halo, cyano, hydroxyl, nitro, phosphate, urea, carbonate or NR'R" wherein R' and R" are taken together with nitrogen to form a cyclic moiety.

In an embodiment, the PI3K inhibitor (which may be a PI3K-γ inhibitor, PI3K-δ inhibitor, or PI3K-γ,δ inhibitor) is a compound of Formula (I-1): 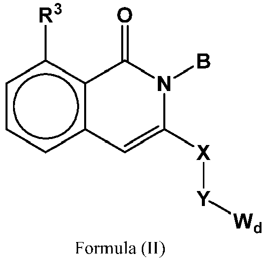 or a pharmaceutically acceptable salt, solvate, or hydrate thereof, wherein:
B is a moiety of Formula (II-A): 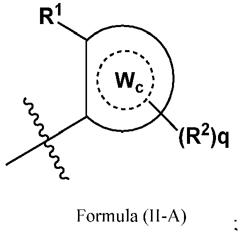
W_{c} is aryl, heteroaryl, heterocycloalkyl, or cycloalkyl;
q is an integer of 0, 1, 2, 3, or 4;
X is a bond or -(CH(R⁹))_{z}-, and z is an integer of 1, 2, 3 or 4;
Y is a bond, -N(R⁹)-, -O-, -S-, -S(=O)-, -S(=O)₂, -C(=O)-, -C(=O)(CHR⁹)_{z}-, -N(R⁹)-C(=O)-, -N(R⁹)-C(=O)NH- or -N(R⁹)C(R⁹)₂-;
z is an integer of 1, 2, 3, or 4;
W_{d} is: 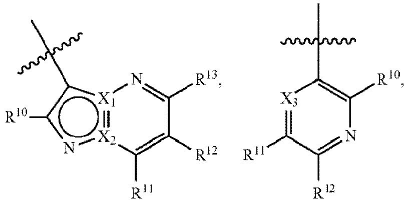 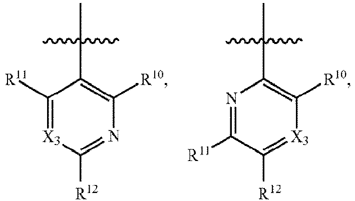 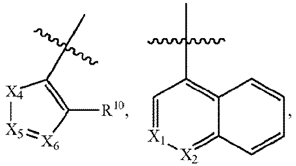 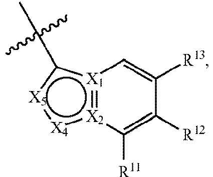 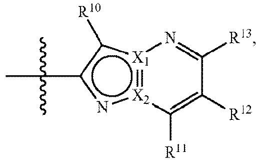 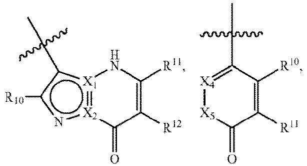 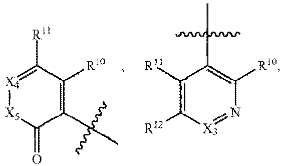 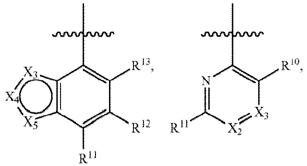 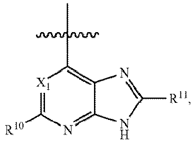 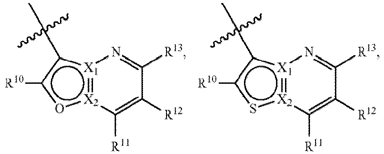 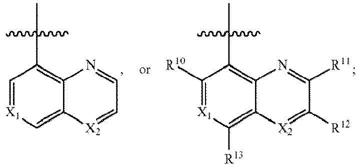
X₁, X₂ and X₃ are each independently C, CR¹³ or N; and X₄, X₅ and X₆ are each independently N, NH, CR¹³, S or O;
R¹ is hydrogen, alkyl, alkenyl, alkynyl, alkoxy, amido, alkoxycarbonyl, sulfonamido, halo, cyano, or nitro;
R² is alkyl, alkenyl, alkynyl, cycloalkyl, heterocycloalkyl, aryl, heteroaryl, heteroarylalkyl, alkoxy, amino, halo, cyano, hydroxy or nitro;
R³ is hydrogen, alkyl, alkenyl, alkynyl, cycloalkyl, heterocycloalkyl, alkoxy, amido, amino, alkoxycarbonyl sulfonamido, halo, cyano, hydroxy or nitro; and
each instance of R⁹ is independently hydrogen, alkyl, or heterocycloalkyl.

In a preferred embodiment, the PI3K inhibitor (which may be a PI3K-γ inhibitor, PI3K-δ inhibitor, or PI3K-γ,δ inhibitor) is a compound of Formula (III): 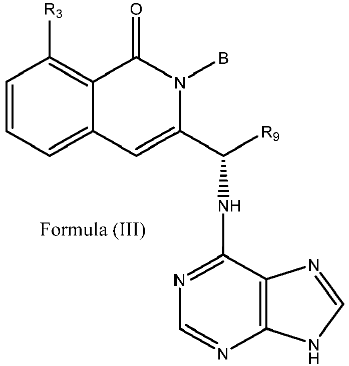
or a pharmaceutically acceptable salt, solvate, orhydrate thereof,
where B is a moiety of Formula (II-A),
R² is alkyl, alkenyl, alkynyl, cycloalkyl, heterocycloalkyl, aryl, heteroaryl, heteroarylalkyl, alkoxy, amino, halo, cyano, hydroxy or nitro; and
R⁹ is hydrogen, alkyl, or heterocycloalkyl.

In a preferred embodiment, the PI3K-γ,δ inhibitor is a compound of Formula (III-A): 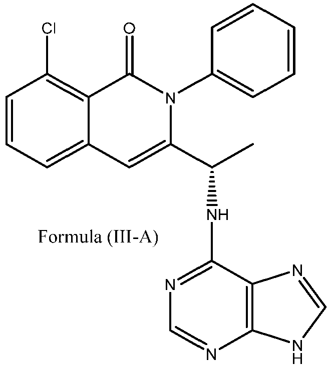 or a pharmaceutically acceptable salt, solvate, or hydrate thereof. Formula (III-A) is also known as IPI-145 or duvelisib (Infinity Pharmaceuticals) and has been studied at doses of 5 mg and 25 mg in clinical trials, including those described in Flinn, et al., Blood, 2014, 124, 802, and O'Brien, et al., Blood, 2014, 124, 3334.

In a preferred embodiment, the PI3K inhibitor is a compound of Formula (IV): 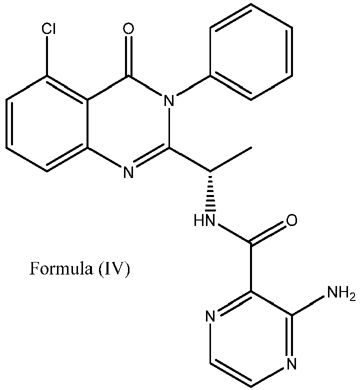 or a pharmaceutically acceptable salt, solvate, or hydrate thereof.

In a preferred embodiment, the PI3K inhibitor is (*S*)-3-(1-((9*H*-purin-6-yl)amino)ethyl)-8-chloro-2-phenylisoquinolin-1(2*H*)-one or a pharmaceutically acceptable salt, solvate, or hydrate thereof.

In a preferred embodiment, the PI3K inhibitor is (*S*)-3-amino-N-(1-(5-chloro-4-oxo-3-phenyl-3,4-dihydroquinazolin-2-yl)ethyl)pyrazine-2-carboxamide or a pharmaceutically acceptable salt, solvate, or hydrate thereof.

In an embodiment, the PI3K inhibitor (which may be a PI3K-γ inhibitor, PI3K-δ inhibitor, or PI3K-γ,δ inhibitor) is a compound selected from the structures disclosed in U.S. Patent Nos. 8,193,199, 8,586,739, and 8,901,135. In an embodiment, the PI3K inhibitor or PI3K-δ inhibitor is a compound of Formula (V): 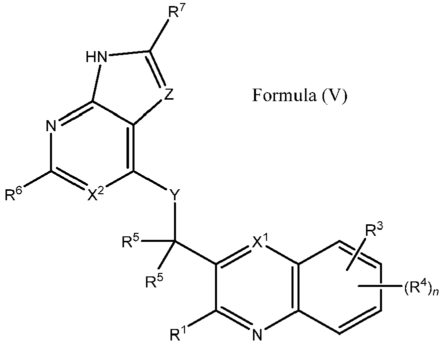 or a pharmaceutically acceptable salt, solvate, or hydrate thereof, wherein:
X¹ is C(R⁹) or N;
X² is C(R₁₀) or N;
Y is N(R¹¹), O or S;
Z is CR⁸ or N;
n is 0, 1, 2 or 3;
R¹ is a direct-bonded or oxygen -linked saturated, partially saturated or unsaturated 5-, 6-or 7-membered monocyclic ring containing 0, 1, 2, 3 or 4 atoms selected from N, O and S, but containing no more than one 0 or S, wherein the available carbon atoms of the ring are substituted by 0, 1 or 2 oxo or thioxo groups, wherein the ring is substituted by 0 or 1 R² substituents, and the ring is additionally substituted by 0, 1, 2 or 3 substituents independently selected from halo, nitro, cyano, (C₁₋₄)alkyl, O(C₁₋₄)alkyl, O(C₁₋₄)haloalkyl, NHC₁₋₄, N((C₁₋₄)alkyl)(C₁₋₄)alkyl and (C₁₋₄)haloalkyl;
R² is selected from halo, (C₁₋₄)haloalkyl, cyano, nitro, -C(=O)R^{a}, -C(=O)OR^{a}, - C(=O)NR^{a}R^{a}, -C(=NR^{a})NR^{a}R^{a}, -OR^{a}, -OC(=O)R^{a}, -OC(=O)NR^{a}R^{a}. - OC(=O)N(R^{a})S(=O)₂R^{a}, -O(C₂₋₆)alkylNR^{a}R^{a}, -O(C₂₋₆)alkylOR^{a}, -SR^{a}, OS(=O)R^{a}, -S(=O)₂R^{a}, -S(=O)₂NR^{a}R^{a}, -S(=O)₂N(R^{a})C(=O)R^{a}, - S(=O)₂N(R^{a})C(=O)OR^{a}, -S(=O)₂N(R^{a})C(=O)NR^{a}R^{a}, -NR^{a}R^{a}, -N(R^{a})C(=O)R^{a}, -N(R^{a})C(=O)OR^{a}, -N(R^{a})C(=O)NR^{a}R^{a}, -N(R^{a})C(=NR^{a})NR^{a}R^{a}, - N(R^{a})S(=O)₂R^{a}, -N(R^{a})S(=O)₂NR^{a}R^{a}, -NR^{a}(C₂₋₆alkylNR^{a}R^{a} and -NR^{a}(C₂₋₆)alkylOR^{a}; or R² is selected from (C₁₋₆)alkyl, phenyl, benzyl, heteroaryl, heterocycle, -((C₁₋₃)alkyl)heteroaryl, -((C₁₋₃)alkyl)heterocycle, -O((C₁₋₃)alkyl)heteroaryl, - O((C₁₋₃)alkyl)heterocycle, -NR^{a}((C₁₋₃)alkyl)heteroaryl, -NR^{a}((C₁₋₃)alkyl)heterocycle, -(C₁₋₃)alkyl)phenyl, -O((C₁₋₃)alkyl)phenyl and -NR^{a}((C₁₋₃)alkyl)phenyl all of which are substituted by 0, 1, 2 or 3 substituents selected from (C₁₋₄)haloalkyl, O(C₁₋₄)alkyl, Br, Cl, F, I and (C₁₋₄)alkyl;
R³ is selected from H, halo, (C₁₋₄)haloalkyl, cyano, nitro, -C(=O)R^{a}, -C(=O)R^{a}, - C(=O)NR^{a}R^{a}, -C(=NR^{a})NR^{a}R^{a}, -OR^{a}, -OC(=O)R^{a}, -OC(=O)NR^{a}R^{a}, - OC(=O)N(R^{a})S(=O)₂R², -O(C₂₋₆)alkylNR^{a}R^{a}, -O(C₂₋₆)alkylOR^{a}, -SR^{a}, - S(=O)R^{a}, -S(=O)₂R^{a}, -S(=O)₂NR^{a}R^{a}, -S(=O)₂N(R^{a})C(=O)R^{a}, - S(=O)₂N(R^{a})C(=O)OR^{a}, -S(=O)₂N(R^{a})C(=O)NR^{a}R^{a}, -NR^{a}R^{a}, -N(R^{a})C(=O)R^{a}, -N(R^{a})C(=O)OR^{a}, -N(R^{a})C(=O)NR^{a}R^{a}, -N(R^{a})C(=NR^{a})NR^{a}R^{a}, - N(R^{a})S(=O)₂R^{a}, -N(R^{a})S(=O)₂NR^{a}NR^{a}R^{a}, -NR^{a}(C₂₋₆)alkylOR^{a}, (C₁₋₆)alkyl, phenyl, benzyl, heteroaryl and heterocycle, wherein the (C₁₋₆)alkyl, phenyl, benzyl, heteroaryl and heterocycle are additionally substituted by 0, 1, 2 or 3 substituents selected from (C₁₋₆)haloalkyl, O(C₁₋₆)alkyl, Br, Cl, F, I and (C₁₋₆)alkyl;
R⁴ is, independently, in each instance, halo, nitro, cyano, (C₁₋₄)alkyl, O(C₁₋₄)alkyl, O(C₁₋₄)haloalkyl, NH(C₁₋₄)alkyl, N((C₁₋₄)alkyl)(C₁₋₄)alkyl or (C₁₋₄)haloalkyl;
R⁵ is, independently, in each instance, H, halo, (C₁₋₆)alkyl, (C₁₋₄)haloalkyl, or (C₁₋₆)alkyl substituted by 1, 2 or 3 substituents selected from halo, cyano, OH, O(C₁₋₄)alkyl, (C₁₋₄)alkyl, (C₁₋₃)haloalkyl, O(C₁₋₄)alkyl, NH₂, NHC₁₋₄)alkyl, N(C₁₋₄)alkyl(C₁₋₄)alkyl; or both R⁵ groups together form a (C₃₋₆)spiroalkyl substituted by 0, 1, 2 or 3 substituents selected from halo, cyano, OH, O(C₁₋₄)alkyl, (C₁₋₄)alkyl, (C₁₋₃)haloalkyl, O(C₁₋₄)alkyl, NH₂, NH(C₁₋₄)alkyl, N((C₁₋₄)alkyl)(C₁₋₄)alkyl;
R⁶ is selected from H, halo, (C₁₋₆)alkyl, (C₁₋₄)haloalkyl, cyano, nitro, -C(=O)R^{a}, - C(=O)OR^{a}, -C(=O)NR^{a}R^{a}, -C(=NR^{a})NR^{a}R^{a}, -S(=O)R^{a}, -S(=O)₂R^{a}, - S(=O)₂NR^{a}R^{a}, -S(=O)₂N(R^{a})C(=O)R^{a}, -S(=O)₂N(R^{a})C(=O)OR^{a}, - S(=O)₂N(R^{a})C(=O)NR^{a}R^{a};
R⁷ is selected from H, halo, (C₁₋₆)alkyl, (C₁₋₄)haloalkyl, cyano, nitro, -C(=O)R^{a}, - C(=O)OR^{a}, -C(=O)NR^{a}R^{a}, -C(=NR^{a})NR^{a}R^{a}, -S(=O)R^{a}, -S(=O)₂R^{a}, - S(=O)₂NR^{a}R^{a}, -S(=O)₂N(R^{a})C(=O)R^{a}, -S(=O)₂N(R^{a})C(=O)OR^{a}, - S(=O)₂N(R^{a})C(=O)NR^{a}R^{a};
R⁸ is selected from H, (C₁₋₆)haloalkyl, Br, Cl, F, I, OR^{a}, NR^{a}R^{a}, (C₁₋₆)alkyl, phenyl, benzyl, heteroaryl and heterocycle, wherein the (C₁₋₆)alkyl, phenyl, benzyl, heteroaryl and heterocycle are additionally substituted by 0, 1, 2 or 3 substituents selected from (C₁₋₆)haloalkyl, O(C₁₋₆)alkyl, Br, Cl, F, I and (C₁₋₆)alkyl;
R⁹ is selected from H, halo, (C₁₋₄)haloalkyl, cyano, nitro, -C(=O)R^{a}, -C(=O)OR^{a}, - C(=O)NR^{a}R^{a}C(=NR^{a})NR^{a}R^{a}, -OR^{a}, -OC(=O)R^{a}, -OC(=O)NR^{a}R^{a}, - OC(=O)N(R^{a})S(=O)₂R^{a}, -O(C₂₋₆)alkylOR^{a}, -SR^{a}, -S(=O)R^{a}, -S(=O)₂R^{a}, - S(=O)₂NR^{a}R^{a}, -S(=O)₂N(R^{a})C(=O)R^{a}, -S(=O)₂N(R^{a})C(=O)OR^{a}, - S(=O)₂N(R^{a})C(=O)NR^{a}R^{a}, NR^{a}R^{a}, -N(R^{a})C(=O)R^{a},-N(R^{a})C(=O)OR^{a}, - N(R^{a})C(O)NR^{a}R^{a}N(R^{a}C(=NR^{a})NR^{a}R^{a}, -N(R^{a})S(=O)₂R^{a}, -N(R^{a})S(=O)₂NR^{a}R^{a}, - NR^{a}(C₂₋₆alkylNR^{a}R^{a}, -NR(C₁₋₆)alkyl, phenyl, benzyl, heteroaryl and heterocycle, wherein the (C₁₋₆ alkyl, phenyl, benzyl, heteroaryl and heterocycle are additionally substituted by 0, 1, 2 or 3 substituents selected from halo, (C₁₋₄)haloalkyl, cyano, nitro, -C(=O)R^{a}, -C(=O)OR^{a}, -C(=O)NR^{a}R^{a}, -C(=NR^{a})NR^{a}R^{a}, -OR^{a}, - OC(=O)R^{a}, OC(=O)NR^{a}R^{a}, -OC(=O)N(R^{a})S(=O)₂R^{a}, -O(C₂₋₆)alkylNR^{a}R^{a}, - O(C₂₋₆)alkylOR^{a}, -SR^{a}, -S(=O)R^{a}, -S(=O)₂R^{a}, -S(=O)₂NR^{a}R^{a}, - S(=O)₂N(R^{a})C(=O)R^{a}, -S(=O)₂N(R^{a})C(=O)OR^{a}, -S(=O)₂N(R^{a})C(=O)NR^{a}R^{a}, NR^{a}R^{a}, -N(R^{a})C(=O)R^{a}, -N(R^{a})C(=O)OR^{a}, -N(R^{a})C(=O)NR^{a}R^{a}, - N(R^{a})C(=NR^{a})NR^{a}R^{a}, -N(R^{a})S(=O)₂R^{a}, -N(R^{a})S(=O)₂NR^{a}R^{a}, -NR^{a}(C₂₋₆)alkylOR^{a}, -NR^{a}(C₂₋₆)alkylOR^{a}; or R⁹ is a saturated, partially-saturated or unsaturated 5-, 6- or 7-membered monocyclic ring containing 0, 1, 2, 3 or 4 atoms selected from N, O and S, but containing no more than one O or S, wherein the available carbon atoms of the ring are substituted by 0, 1 or 2 oxo or thioxo groups, wherein the ring is substituted by 0, 1, 2, 3 or 4 substituents selected from halo, (C₁₋₄)haloalkyl, cyano, nitro, -C(=O)R^{a}, -C(=O)OR^{a}, -C(=O)NR^{a}R^{a}, - C(=NR^{a})NR^{a}R^{a}, -OR^{a}, -OC(=O)R^{a}, -OC(=O)NR^{a}R^{a}, - OC(=O)N(R^{a})S(=O)₂R^{a}, -O(C₂₋₆)alkylNR^{a}R^{a}, -O(C₂₋₆)alkylOR^{a}, -SR^{a}, - S(=O)R^{a}, -S(=O)₂R^{a}, -S(=O)₂NR^{a}R^{a}, -S(=O)₂N(R^{a})C(=O)R^{a}, - S(=O)₂N(R^{a})C(=O)OR^{a}, -S(=O)₂N(R^{a})C(=O)NR^{a}R^{a}, -NR^{a}R^{a}, -N(R^{a})C(=O)R^{a}, -N(R^{a})C(=O)OR^{a}, -N(R^{a})C(=O)NR^{a}R^{a}, -N(R^{a})C(=NR^{a})NR^{a}R^{a}, - N(R^{a})S(=O)₂R^{a}, -N(R^{a})S(=O)₂NR^{a}R^{a}, -NR^{a}(C₂₋₆)alkylNR^{a}R^{a} and -NR^{a}(C₂₋₆)alkylOR^{a};
R¹⁰ is H, (C₁₋₃)alkyl, (C₁₋₃)haloalkyl, cyano, nitro, CO₂R^{a}, C(=O)NR^{a}R^{a}, - C(=NR^{a})NR^{a}R^{a}, -S(=O)₂N(R^{a})C(=O)R^{a}, -S(=O)₂N(R^{a})C(=O)OR^{a}, - S(=O)₂N(R^{a})C(=O)NR^{a}R^{a}, -S(=O)R^{b}, S(=O)₂R^{b} or S(=O)₂NR^{a}R^{a};
R¹¹ is H or (C₁₋₄)alkyl;
R^{a} is independently, at each instance, H or R^{b}; and
R^{b} is independently, at each instance, phenyl, benzyl or (C₁₋₆)alkyl, the phenyl, benzyl and (C₁₋₆)alkyl being substituted by 0, 1, 2 or 3 substituents selected from halo, (C₁₋₄)alkyl, (C₁₋₃)haloalkyl, -O(C₁₋₄)alkyl, -NH₂, -NHC₁₋₄)alkyl, -N((C₁₋₄)alkyl)(C₁₋₄ )alkyl.

In another embodiment, the PI3K inhibitor (which may be a PI3K-γ inhibitor, PI3K-δ inhibitor, or PI3K-γ,δ inhibitor) is a compound of Formula (VI): 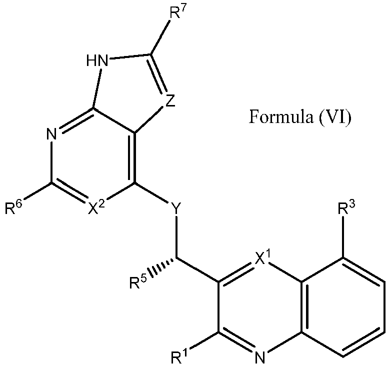 or a pharmaceutically acceptable salt, solvate, or hydrate thereof, wherein:
X¹ is C(R⁹) or N;
X² is C(R¹⁰) or N;
Y is N(R¹¹), O or S;
Z is CR⁸ or N;
R¹ is a direct-bonded or oxygen-linked saturated, partially-saturated or unsaturated 5-, 6-or 7-membered monocyclic ring containing 0, 1, 2, 3 or 4 atoms selected from N, O and S, but containing no more than one O or S, wherein the available carbon atoms of the ring are substituted by 0, 1 or 2 oxo or thioxo groups, wherein the ring is substituted by 0 or 1 R² substituents, and the ring is additionally substituted by 0, 1, 2 or 3 substituents independently selected from halo, nitro, cyano, (C₁₋₄)alkyl, O(C₁₋₄)alkyl, O(C₁₋₄)haloalkyl, (NHC₁₋₄)alkyl, N(C₁₋₄alkyl)(C₁₋₄)alkyl and (C₁₋₄)haloalkyl;
R² is selected from halo, (C₁₋₄)haloalkyl, cyano, nitro, -C(=O)R^{a}, -C(=O)OR^{a}, - C(=O)NR^{a}R^{a}, -C(=NR^{a})NR^{a}R^{a}, -OR^{a}, -OC(=O)R^{a}, -OC(=O)NR^{a}R^{a}, - OC(=O)N(R^{a})S(=O)₂R^{a}, -O(C₂₋₆)alkylNR^{a}R^{a}, -O(C₂₋₆)alkylOR^{a}, -S(=O)R^{a}, - S(=O)₂R^{a}, -S(=O)₂NR^{a}R^{a}, -S(=O)₂N(R^{a})C(=O)R^{a}, -S(=O)₂N(R^{a})C(=O)OR^{a}, - S(=O)₂N(R^{a})C(=O)NR^{a}R^{a}, -NR^{a}R^{a}, -N(R^{a})C(=O)R^{a},-N(R^{a})C(=O)OR^{a}, - N(R^{a})C(=O)NR^{a}R^{a}, -N(R^{a})C(=NR^{a})NR^{a}R^{a}, -N(R^{a})S(=O)₂R^{a}, - N(R^{a})S(=O)₂NR^{a}R^{a}, -NR^{a}(C₂₋₆alkylNR^{a}R^{a} and -NR(C₂₋₆)alkylOR; or R² is selected from (C₁₋₆)alkyl, phenyl, benzyl, heteroaryl, heterocycle, -((C₁₋₃)alkyl)heteroaryl, -((C₁₋₃)alkyl)heterocycle, -O((C₁₋₃)alkyl)heteroaryl, -O((C₁₋₃)alkyl)heterocycle, -NR^{a}((C₁₋₃)alkyl)heteroaryl, -NR^{a}((C₁₋₃)alkyl)heterocycle, - ((C₁₋₃)alkyl)phenyl, -O((C₁₋₃)alkyl)phenyl and -NR^{a}(C₁₋₃ alkyl)phenyl all of which are substituted by 0, 1, 2 or 3 substituents selected from (C₁₋₄)haloalkyl, O(C₁₋₄)alkyl, Br, Cl, F, I and (C₁₋₄)alkyl;
R³ is selected from H, halo, (C₁₋₄)haloalkyl, cyano, nitro, -C(=O)R^{a}, -C(=O)OR^{a}, C(=O)NR^{a}R^{a}C(=NR^{a})NR^{a}R^{a}, -OR^{a}, -OC(=O)R^{a}, -OC(=O)NR^{a}R^{a}, - OC(=O)N(R^{a})S(=O)₂R^{a}, -O(C₂₋₆)alkylNR^{a}R^{a}, -O(C₂₋₆)alkylOR^{a}, -SR^{a}, - S(=O)R^{a}, -S(=O)₂R^{a}, -S(=O)₂NR^{a}R^{a}, -S(=O)₂N(R^{a})C(=O)R^{a}, - S(=O)₂N(R^{a})C(=O)OR^{a}, -S(=O)₂N(R^{a})C(=O)NR^{a}R^{a}, NR^{a}R^{a}, -N(R^{a})C(=O)R^{a}, - N(R^{a})C(=O)OR^{a}, -N(R^{a})C(=O)NR^{a}R^{a}, -N(R^{a})C(=NR^{a})NR^{a}R^{a}, -N(R^{a})S(=O)₂R^{a}, -N(R^{a})S(=O)₂NR^{a}R^{a}, -NR^{a}(C₂₋₆)alkylOR^{a}, (C₁₋₆)alkyl, phenyl, benzyl, heteroaryl and heterocycle, wherein the (C₁₋₆)alkyl, phenyl, benzyl, heteroaryl and heterocycle are additionally substituted by 0, 1, 2 or 3 substituents selected from (C₁₋₆)haloalkyl, O(C₁₋₆)alkyl, Br, Cl, F, I and (C₁₋₆)alkyl;
R⁵ is, independently, in each instance, H, halo, (C₁₋₆)alkyl, (C₁₋₄)haloalkyl, or (C₁₋₆)alkyl substituted by 1, 2 or 3 substituents selected from halo, cyano, OH, O(C₁₋₄)alkyl, (C₁₋₄)alkyl, (C₁₋₃)haloalkyl, O(C₁₋₄)alkyl, NH₂, (NHC₁₋₄)alkyl, N(C₁₋₄)alkyl(C₁₋₄)alkyl; or both R⁵ groups together form a C₃₋₆-spiroalkyl substituted by 0, 1, 2 or 3 substituents selected from halo, cyano, OH, O(C₁₋₄)alkyl, (C₁₋₄)alkyl, (C₁₋₃)haloalkyl, O(C₁₋₄)alkyl, NH₂, (NHC₁₋₄)alkyl, N((C₁₋₄)alkyl)(C₁₋₄)alkyl;
R⁶ is selected from H, halo, (C₁₋₆)alkyl, (C₁₋₄)haloalkyl, cyano, nitro, -C(=O)R^{a}, - C(=O)OR^{a}, -C(=O)NR^{a}R^{a}, -C(=NR^{a})NR^{a}R^{a}, -S(=O)R^{a}, -S(=O)₂R^{a}, - S(=O)₂NR^{a}R^{a}, -S(=O)₂N(R^{a})C(=O)R^{a}, -S(=O)₂N(R^{a})C(=O)OR^{a}, - S(=O)₂N(R^{a})C(=O)NR^{a}R^{a};
R⁷ is selected from H, halo, (C₁₋₆)alkyl, (C₁₋₄)haloalkyl, cyano, nitro, -C(=O)R^{a}, - C(=O)OR^{a}, -C(=O)NR^{a}R^{a}, -C(=NR^{a})NR^{a}R^{a}, -S(=O)R^{a}S(=O)₂R^{a}, - S(=O)₂NR^{a}R^{a}, -S(=O)₂N(R^{a})C(=O)R^{a}, -S(=O)₂N(R^{a})C(=O)OR^{a}, - S(=O)₂N(R^{a})C(=O)NR^{a}R^{a};
R⁸ is selected from H, (C₁₋₆)haloalkyl, Br, Cl, F, I, OR^{a}, NR^{a}R^{a}, (C₁₋₆)alkyl, phenyl, benzyl, heteroaryl and heterocycle, wherein the (C₁₋₆)alkyl, phenyl, benzyl, heteroaryl and heterocycle are additionally substituted by 0, 1, 2 or 3 substituents selected from (C₁₋₆)haloalkyl, O(C₁₋₆alkyl, Br, Cl, F, I and (C₁₋₆)alkyl;
R⁹ is selected from H, halo, (C₁₋₄)haloalkyl, cyano, nitro, -C(=O)R^{a}, -C(=O)OR^{a}, - C(=O)NR^{a}R^{a}, -C(=NR^{a})NR^{a}R^{a}, -OR^{a}, -OC(=O)R^{a}, OC(=O)NR^{a}R^{a}, - OC(=O)N(R^{a})S(=O)₂R^{a}, -O(C₂₋₆)alkylNR^{a}R^{a}, -O(C₂₋₆)alkylOR^{a}, -SR^{a}, - S(=O)R^{a}, -S(=O)₂R^{a}, -S(=O)₂NR^{a}R^{a}, -S(=O)₂N(R^{a})C(=O)R^{a}, - S(=O)₂N(R^{a})C(=O)OR^{a}, -S(=O)₂N(R^{a})C(=O)NR^{a}R^{a}, -NR^{a}R^{a}, -N(R^{a})C(=O)R^{a}, -N(R^{a})C(=O)OR^{a}, -N(R^{a})C(=O)NR^{a}R^{a}, -N(R^{a})C(=NR^{a})NR^{a}R^{a}, - N(R^{a})S(=O)₂R^{a}, -N(R^{a})S(=O)₂NR^{a}R^{a}, -NR^{a}(C₂₋₆)alkylNR^{a}R^{a}, -NR^{a}(C₂₋₆)alkylOR^{a}, (C₁₋₆)alkyl, phenyl, benzyl, heteroaryl and heterocycle, wherein the (C₁₋₆)alkyl, phenyl, benzyl, heteroaryl and heterocycle are additionally substituted by 0, 1, 2 or 3 substituents selected from halo, (C₁₋₄)haloalkyl, cyano, nitro, -C(=O)R^{a}, - C(=O)OR^{a}, -C(=O)NR^{a}R^{a}, -C(=NR^{a})NR^{a}R^{a}, -OR^{a}, -OC(=O)R^{a}, - OC(=O)NR^{a}R^{a}, -OC(=O)N(R^{a})S(=O)₂R^{a}, -O(C₂₋₆)alkylOR^{a}, -SR^{a}, -S(=O)R^{a}, -S(=O)₂R^{a}, -S(=O)₂NR^{a}R^{a}, -S(=O)₂N(R^{a})C(=O)R^{a}, -S(=O)₂N(R^{a})C(=O)OR^{a}, -S(=O)₂N(R^{a})C(=O)NR^{a}R^{a}, NR^{a}R^{a}, -N(R^{a})C(=O)R^{a},-N(R^{a})C(=O)OR^{a}, - N(R^{a})C(=O)NR^{a}R^{a}, -N(R^{a})C(=NR^{a})NR^{a}R^{a}, -N(R^{a})S(=O)₂R^{a}, - N(R^{a})S(=O)₂NR^{a}R^{a}, -NR^{a}(C₂₋₆)alkylNR^{a}, -NR^{a}(C₂₋₆)alkylOR^{a}; or R⁹ is a saturated, partially-saturated or unsaturated 5-, 6- or 7-membered monocyclic ring containing 0, 1, 2, 3 or 4 atoms selected from N, O and S, but containing no more than one O or S, wherein the available carbon atoms of the ring are substituted by 0, 1 or 2 oxo or thioxo groups, wherein the ring is substituted by 0, 1, 2, 3 or 4 substituents selected from halo, (C₁₋₄)haloalkyl, cyano, nitro, -C(=O)R^{a}, -C(=O)OR^{a}, -C(=O)NR^{a}R^{a}, -C(=NR^{a})NR^{a}R^{a}, -OR^{a}, -OC(=O)R^{a}, -OC(=O)NR^{a}R^{a}, - OC(=O)N(R^{a})S(=O)₂R^{a}, -O(C₂₋₆)alkylOR^{a}, -SR^{a}, -S(=O)R^{a}, -S(=O)₂R^{a}, - S(=O)₂NR^{a}R^{a}, -S(=O)₂N(R^{a})C(=O)R^{a}, -S(=O)₂N(R^{a})C(=O)OR^{a}, - S(=O)₂N(R^{a})C(=O)NR^{a}R^{a}, -NR^{a}R^{a}, -N(R^{a})C(=O)R^{a},-N(R^{a})C(=O)OR^{a}, - N(R^{a})C(=O)NR^{a}R^{a}, -N(R^{a})C(=NR^{a})NR^{a}R^{a}, -N(R^{a})S(=O)₂R^{a}, - N(R^{a})S(=O)₂NR^{a}R^{a}, -NR^{a}(C₂₋₆ alkylNR^{a}R^{a} and -NR(C₂₋₆)alkylOR^{a};
R¹⁰ is H, (C₁₋₃)alkyl, (C₁₋₃)haloalkyl, cyano, nitro, CO₂R^{a}, C(=O)NR^{a}R^{a}, - C(=NR^{a})NR^{a}R^{a}, -S(=O)₂N(R^{a})C(=O)R^{a}, -S(=O)₂N(R^{a})C(=O)OR^{a}, - S(=O)₂N(R^{a})C(=O)NR^{a}R^{a}, -S(=O)R^{b}, S(=O)₂R^{b} or S(=O)₂NR^{a}R^{a}; -R¹¹ is H or (C₁₋₄)alkyl;
R^{a} is independently, at each instance, H or R^{b}; and
R^{b} is independently, at each instance, phenyl, benzyl or (C₁₋₆)alkyl, the phenyl, benzyl and (C₁₋₆)alkyl being substituted by 0, 1, 2 or 3 substituents selected from halo, (C₁₋₄)alkyl, (C₁₋₃) haloalkyl, -O(C₁₋₄)alkyl, -NH₂, -NH(C₁₋₄)alkyl, -N(C₁₋₄)alkyl(C₁₋₄)alkyl.

In another embodiment, the PI3K inhibitor (which may be a PI3K-γ inhibitor, PI3K-δ inhibitor, or PI3K-γ,δ inhibitor) is a compound of Formula (VII): 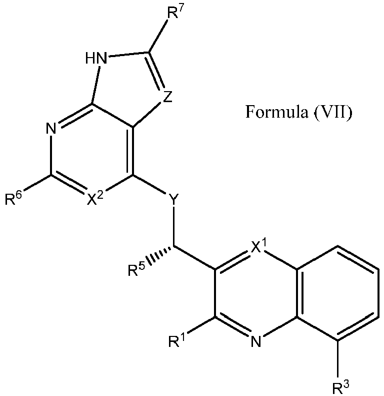 or a pharmaceutically acceptable salt, solvate, or hydrate thereof, wherein:
X¹ is C(R⁹) or N;
X² is C(R¹⁰) or N;
Y is N(R¹¹), O or S;
Z is CR⁸ or N;
R¹ is a direct-bonded or oxygen-linked saturated, partially-saturated or unsaturated 5-, 6-or 7-membered monocyclic ring containing 0, 1, 2, 3 or 4 atoms selected from N, O and S, but containing no more than one O or S, wherein the available carbon atoms of the ring are substituted by 0, 1 or 2 oxo or thioxo groups, wherein the ring is substituted by 0 or 1 R² substituents, and the ring is additionally substituted by 0, 1, 2 or 3 substituents independently selected from halo, nitro, cyano, (C₁₋₄)alkyl, O(C₁₋₄)alkyl, O(C₁₋₄)haloalkyl, NH(C₁₋₄)alkyl, N(C₁₋₄)alkyl(C₁₋₄)alkyl and (C₁₋₄)haloalkyl;
R² is selected from halo, (C₁₋₄)haloalkyl, cyano, nitro, -C(=O)R^{a}, -C(=O)OR^{a}, - C(=O)NR^{a}R^{a}, -C(=NR^{a})NR^{a}R^{a}, -OR^{a}, -OC(=O)R^{a}, -OC(=O)NR^{a}R^{a}, - OC(=O)N(R^{a})S(=O)₂R^{a}, -OC₂₋₆)alkylNR^{a}R^{a}, -OC₂₋₆)alkylOR^{a}, -SR^{a}, - S(=O)R^{a}, -S(=O)₂R^{a}, -S(=O)₂NR^{a}R^{a}, -S(=O)₂N(R)C(=O)R^{a}, - S(=O)₂N(R^{a})C(=O)OR^{a}, -S(=O)₂N(R^{a})C(=O)NR^{a}R^{a}, NR^{a}R^{a}, -N(R^{a})C(=O)R^{a}, - N(R^{a})C(=O)OR^{a}, -N(R^{a})C(=O)NR^{a}R^{a}, -N(R^{a})C(=NR^{a})NR^{a}R^{a}, -N(R^{a})S(=O)₂R^{a}, -N(R^{a})S(=O)₂NR^{a}R^{a}, -NR^{a}(C₂₋₆ alkylNR^{a}R^{a} and -NR^{a}(C₂₋₆)alkylOR^{a}; or R² is selected from (C₁₋₆)alkyl, phenyl, benzyl, heteroaryl, heterocycle, -(C₁₋₃ alkyl)heteroaryl, -(C₁₋₃ alkyl)heterocycle, -O(C₁₋₃ alkyl)heteroaryl, -O((C₁₋₃)alkyl)heterocycle, -NR^{a}(C₁₋₃ alkyl)heteroaryl, -NR^{a}(C₁₋₃ alkyl)heterocycle, -(C₁₋₃ alkyl)phenyl, -O(C₁₋₃ alkyl)phenyl and -NR^{a}(C₁₋₃ alkyl)phenyl all of which are substituted by 0, 1, 2 or 3 substituents selected from (C₁₋₄)haloalkyl, O(C₁₋₄)alkyl, Br, Cl, F, I and (C₁₋₄)alkyl;
R³ is selected from H, halo, (C₁₋₄)haloalkyl, cyano, nitro, -C(=O)R^{a}, -C(=O)OR^{a}, - C(=O)NR^{a}R^{a}, -C(=NR^{a})NR^{a}R^{a}, -OR^{a}, -OC(=O)R^{a}, -OC(=O)NR^{a}R^{a}, - OC(=O)N(R^{a})S(=O)₂R^{a}, -OC₂₋₆)alkylNR^{a}R^{a}, -OC₂₋₆)alkylOR¹, -SR^{a}, - S(=O)R^{a}, -S(=O)₂R^{a}, -S(=O)₂NR^{a}R^{a}, -S(=O)₂N(R^{a})C(=O)R^{a}, - S(=O)₂N(R^{a})C(=O)OR^{a}, -S(=O)₂N(R^{a})C(=O)NR^{a}R^{a}, -NR^{a}R^{a}, -N(R^{a})C(=O)R^{a}, -N(R^{a})C(=O)OR^{a}, -N(R^{a})C(=O)NR^{a}R^{a}, -N(R^{a})C(=NR^{a})NR^{a}R^{a}, - N(R^{a})S(=O)₂R^{a}, -N(R^{a})S(=O)₂NR^{a}R^{a}, -NR^{a}(C₂₋₆)alkylNR^{a}R^{a}, -NR^{a}(C₂₋₆)alkylOR^{a}, (C₁₋₆)alkyl, phenyl, benzyl, heteroaryl and heterocycle, wherein the (C₁₋₆)alkyl, phenyl, benzyl, heteroaryl and heterocycle are additionally substituted by 0, 1, 2 or 3 substituents selected from (C₁₋₆)haloalkyl, O(C₁₋₆)alkyl, Br, Cl, F, I and (C₁₋₆)alkyl;
R⁵ is, independently, in each instance, H, halo, (C₁₋₆)alkyl, (C₁₋₄)haloalkyl, or (C₁₋₆)alkyl substituted by 1, 2 or 3 substituents selected from halo, cyano, OH, O(C₁₋₄)alkyl, (C₁₋₄)alkyl, (C₁₋₃)haloalkyl, O(C₁₋₄)alkyl, NH₂, NHC₁₋₄)alkyl, N(C₁₋₄)alkyl(C₁₋₄)alkyl; or both R⁵ groups together form a C₃₋₆-spiroalkyl substituted by 0, 1, 2 or 3 substituents selected from halo, cyano, OH, O(C₁₋₄)alkyl, (C₁₋₄)alkyl, (C₁₋₃)haloalkyl, O(C₁₋₄)alkyl, NH₂, NHC₁₋₄)alkyl, N(C₁₋₄)alkyl)C₁₋₄)alkyl;
R⁶ is selected from H, halo, (C₁₋₆)alkyl, (C₁₋₄haloalkyl, cyano, nitro, -C(=O)R^{a}, - C(=O)OR^{a}, -C(=O)NR^{a}R^{a}, -C(=NR^{a})NR^{a}R^{a}, -S(=O)R^{a}S(=O)₂R^{a}, - S(=O)₂NR^{a}R^{a}, -S(=O)₂N(R^{a})C(=O)R^{a}, -S(=O)₂N(R^{a})C(=O)OR^{a}, - S(=O)₂N(R^{a})C(=O)NR^{a}R^{a};
R⁷ is selected from H, halo, (C₁₋₆)alkyl, (C₁₋₄haloalkyl, cyano, nitro, -C(=O)R^{a}, - C(=O)OR^{a}, -C(=O)NR^{a}R^{a}, -C(=NR^{a})NR^{a}R^{a}, -S(=O)R^{a}S(=O)₂R^{a}, - S(=O)₂NR^{a}R^{a}, -S(=O)₂N(R^{a})C(=O)R^{a}, -S(=O)₂N(R^{a})C(=O)OR^{a}, - S(=O)₂N(R^{a})C(=O)NR^{a}R^{a};
R⁸ is selected from H, (C₁₋₆)haloalkyl, Br, Cl, F, I, OR^{a}, NR^{a}R^{a}, (C₁₋₆)alkyl, phenyl, benzyl, heteroaryl and heterocycle, wherein the (C₁₋₆)alkyl, phenyl, benzyl, heteroaryl and heterocycle are additionally substituted by 0, 1, 2 or 3 substituents selected from (C₁₋₆)haloalkyl, O(C₁₋₆)alkyl, Br, Cl, F, I and (C₁₋₆)alkyl;
R⁹ is selected from H, halo, (C₁₋₄)haloalkyl, cyano, nitro, -C(=O)R^{a}, -C(=O)OR^{a}, - C(=O)NR^{a}R^{a}, -C(=NR^{a})NR^{a}R^{a}, -OR^{a}, -OC(=O)R^{a}, -OC(=O)NR^{a}R^{a}, - OC(=O)N(R^{a})S(=O)₂R^{a}, -OC₂₋₆)alkylNR^{a}R^{a}, -OC₂₋₆)alkylOR^{a}, -SR^{a}, - S(=O)R^{a}, -S(=O)₂R^{a}, -S(=O)₂NR^{a}R^{a}, -S(=O)₂N(R^{a})C(=O)R^{a}, - S(=O)₂N(R^{a})C(=O)OR^{a}, -S(=O)₂N(R^{a})C(=O)NR^{a}R^{a}, -NR^{a}R^{a}, -N(R^{a})C(=O)R^{a}, -N(R^{a})C(=O)OR^{a}, -N(R^{a})C(=O)NR^{a}R^{a}, -N(R^{a})C(=NR^{a})NR^{a}R^{a}, - N(R^{a})S(=O)₂R^{a}, -N(R^{a})S(=O)₂NR^{a}R^{a}, -NR^{a}(C₂₋₆)alkylOR^{a}, (C₁₋₆)alkyl, phenyl, benzyl, heteroaryl and heterocycle, wherein the (C₁₋₆)alkyl, phenyl, benzyl, heteroaryl and heterocycle are additionally substituted by 0, 1, 2 or 3 substituents selected from halo, (C₁₋₄)haloalkyl, cyano, nitro, -C(=O)R^{a}, -C(=O)OR^{a}, -C(=O)NR^{a}R^{a}, - C(=NR^{a})NR^{a}R^{a}, -OR⁸, -OC(=O)R⁸, -OC(=O)NR²R⁸, - OC(=O)N(R^{a})S(=O)₂R^{a}, -OC₂₋₆)alkylNR^{a}R^{a}, -OC₂₋₆)alkylOR^{a}, -SR^{a}, - S(=O)R^{a}, -S(=O)₂R⁸, -S(=O)₂NR^{a}R^{a}, -S(=O)₂N(R^{a})C(=O)R^{a}, - S(=O)₂N(R^{a})C(=O)OR^{a}, -S(=O)₂N(R^{a})C(=O)NR^{a}R^{a}, -NR^{a}R^{a}, -N(R^{a})C(=O)R^{a}, -N(R^{a})C(=O)OR^{a}, -N(R^{a})C(=O)NR^{a}R^{a}, -N(R^{a})C(=NR^{a})NR^{a}R^{a}, - N(R^{a})S(=O)₂R^{a}, -N(R^{a})S(=O)₂NR^{a}R^{a}, -NR^{a}(C₂₋₆)alkylNR^{a}R^{a}, -NR^{a}(C₂₋₆)alkylOR^{a}; or R⁹ is a saturated, partially-saturated or unsaturated 5-, 6- or 7-membered monocyclic ring containing 0, 1, 2, 3 or 4 atoms selected from N, O and S, but containing no more than one O or S, wherein the available carbon atoms of the ring are substituted by 0, 1 or 2 oxo or thioxo groups, wherein the ring is substituted by 0, 1, 2, 3 or 4 substituents selected from halo, (C₁₋₄)haloalkyl, cyano, nitro, - C(=O)R^{a}, -C(=O)OR^{a}, -C(=O)NR^{a}R^{a}, -C(=NR^{a})NR^{a}R^{a}, -OR^{a}, -OC(=O)R^{a}, -OC(=O)NR^{a}R^{a}, -OC(=O)N(R^{a})S(=O)₂R^{a}, -OC₂₋₆)alkylNR^{a}R^{a}, -OC₂₋₆)alkylOR^{a}, -SR^{a}, -S(=O)R^{a}, -S(=O)₂R^{a}, -S(=O)₂NR^{a}R^{a}, - S(=O)₂N(R^{a})C(=O)R^{a}, -S(=O)₂N(R^{a})C(=O)OR^{a}, -S(=O)₂N(R^{a})C(=O)NR^{a}R^{a}, - NR^{a}R^{a}, -N(R^{a})C(=O)R^{a}, -N(R^{a})C(=O)OR^{a}, -N(R^{a})C(=O)NR^{a}R^{a}, - N(R^{a})C(=NR^{a})NR^{a}R^{a}, -N(R^{a})S(=O)₂R^{a}, -N(R^{a})S(=O)₂NR^{a}R^{a}, -NR^{a}(C₂₋₆)alkylNR^{a}R^{a} and -NR^{a}(C₂₋₆)alkylOR^{a};
R¹⁰ is H, (C₁₋₃ alkyl, (C₁₋₃)haloalkyl, cyano, nitro, CO₂R^{a}, C(=O)NR^{a}R^{a}, - C(=NR^{a})NR^{a}R^{a}, -S(=O)₂N(R^{a})C(=O)R^{a}, -S(=O)₂N(R^{a})C(=O)OR^{a}, - S(=O)₂N(R^{a})C(=O)NR^{a}R^{a}, -S(=O)R^{b}, S(=O)₂R^{b} or S(=O)₂NR^{a}R^{a};
R¹¹ is H or (C₁₋₄)alkyl;
R^{a} is independently, at each instance, H or R^{b}; and
R^{b} is independently, at each instance, phenyl, benzyl or (C₁₋₆)alkyl, the phenyl, benzyl and (C₁₋₆)alkyl being substituted by 0, 1, 2 or 3 substituents selected from halo, (C₁₋₄)alkyl, (C₁₋₃)haloalkyl, -O(C₁₋₄)alkyl, -NH₂, -NHC₁₋₄)alkyl, -N(C₁₋₄)alkyl(C₁₋₄)alkyl.

In another embodiment, the PI3K inhibitor (which may be a PI3K-γ inhibitor, PI3K-δ inhibitor, or PI3K-γ,δ inhibitor) is a compound of Formula (VIII): 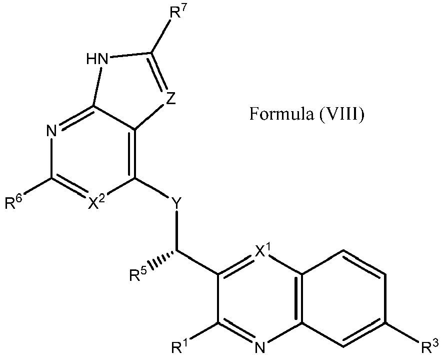 or a pharmaceutically acceptable salt, solvate, or hydrate thereof, wherein:
X¹ is C(R⁹) or N;
X² is C(R¹⁰) or N;
Y is N(R¹¹), O or S;
Z is CR⁸ or N;
R¹ is a direct-bonded or oxygen-linked saturated, partially-saturated or unsaturated 5-, 6-or 7-membered monocyclic ring containing 0, 1, 2, 3 or 4 atoms selected from N, O and S, but containing no more than one O or S, wherein the available carbon atoms of the ring are substituted by 0, 1 or 2 oxo or thioxo groups, wherein the ring is substituted by 0 or 1 R² substituents, and the ring is additionally substituted by 0, 1, 2 or 3 substituents independently selected from halo, nitro, cyano, (C₁₋₄)alkyl, O(C₁₋₄)alkyl, O(C₁₋₄)haloalkyl, NH(C₁₋₄)alkyl, N(C₁₋₄ alkyl)C₁₋₄)alkyl and (C₁₋₄)haloalkyl;
R² is selected from halo, (C₁₋₄)haloalkyl, cyano, nitro, -C(=O)R^{a}, -C(=O)OR^{a}, - C(=O)NR^{a}R^{a}-C(=NR^{a})NR^{a}R^{a}, -OR^{a}, -OC(=O)R^{a}, -OC(=O)NR^{a}R^{a}, - OC(=O)N(R^{a})S(=O)₂R^{a}, -OC₂₋₆alkylOR, -SR^{a}, -S(=O)R^{a}, -S(=O)₂R^{a}, - S(=O)₂NR^{a}R^{a}, -S(=O)₂N(R^{a})C(=O)R^{a}, -S(=O)₂N(R^{a})C(=O)OR^{a}, - S(=O)₂N(R^{a})C(=O)NR^{a}R^{a}, -NR^{a}R^{a}, -N(R^{a})C(=O)R^{a}, -N(R^{a})C(=O)OR^{a}, - N(R^{a})C(=O)NR^{a}R^{a}, -N(R^{a})C(=NR^{a})NR^{a}R^{a}, -N(R^{a})S(=O)₂R^{a}, - N(R^{a})S(=O)₂NR^{a}R^{a}, -NR^{a}(C₂₋₆ alkylNR^{a}R^{a} and -NR(C₂₋₆alkylOR^{a}; or R² is selected from (C₁₋₆alkyl, phenyl, benzyl, heteroaryl, heterocycle, -(C₁₋₃ alkyl)heteroaryl, -(C₁₋₃ alkyl)heterocycle, -O(C₁₋₃ alkyl)heteroaryl, -O(C₁₋₃ alkyl)heterocycle, -NR^{a}(C₁₋₃ alkyl)heteroaryl, -NR^{a}(C₁₋₃ alkyl)heterocycle, -(C₁₋₃ alkyl)phenyl, -O(C₁₋₃ alkyl)phenyl and -NR^{a}(C₁₋₃ alkyl)phenyl all of which are substituted by 0, 1, 2 or 3 substituents selected from (C₁₋₄ haloalkyl, O(C₁₋₄)alkyl, Br, Cl, F, I and (C₁₋₄)alkyl;
R³ is selected from H, halo, (C₁₋₄)haloalkyl, cyano, nitro, -C(=O)R^{a}, -C(=O)OR^{a}, - C(=O)NR^{a}R^{a}-C(=NR^{a})NR^{a}R^{a}, -OR^{a}, -OC(=O)R^{a}, -OC(=O)NR^{a}R^{a}, - OC(=O)N(R^{a})S(=O)₂R^{a}, -OC₂₋₆alkylOR, -SR^{a}, -S(=O)R^{a}, -S(=O)₂R^{a}, - S(=O)₂NR^{a}R^{a}, -S(=O)₂N(R^{a})C(=O)R^{a}, -S(=O)₂N(R^{a})C(=O)OR^{a}, - S(=O)₂N(R^{a})C(=O)NR^{a}R^{a}, -NR^{a}R^{a}, -N(R^{a})C(=O)R^{a}, -N(R^{a})C(=O)OR^{a}, - N(R^{a})C(=O)NR^{a}R^{a}, -N(R^{a})C(=NR^{a})NR^{a}R^{a}, -N(R^{a})S(=O)₂R^{a}, - N(R^{a})S(=O)₂NR^{a}NR^{a}, -NR^{a}, -NR^{a}(C₂₋₆)alkylOR^{a}, (C₁₋₆)alkyl, phenyl, benzyl, heteroaryl and heterocycle, wherein the (C₁₋₆)alkyl, phenyl, benzyl, heteroaryl and heterocycle are additionally substituted by 0, 1, 2 or 3 substituents selected from (C₁₋₆)haloalkyl, O(C₁₋₆)alkyl, Br, Cl, F, I and (C₁₋₆)alkyl;
R⁵ is, independently, in each instance, H, halo, (C₁₋₆)alkyl, (C₁₋₄)haloalkyl, or (C₁₋₆)alkyl substituted by 1, 2 or 3 substituents selected from halo, cyano, OH, O(C₁₋₄)alkyl, (C₁₋₄)alkyl, (C₁₋₃)haloalkyl, O(C₁₋₄)alkyl, NH₂, NH(C₁₋₄)alkyl, N(C₁₋₄)alkyl(C₁₋₄)alkyl; or both R⁵ groups together form a C₃₋₆-spiroalkyl substituted by 0, 1, 2 or 3 substituents selected from halo, cyano, OH, O(C₁₋₄)alkyl, (C₁₋₄)alkyl, (C₁₋₃)haloalkyl, O(C₁₋₄)alkyl, NH₂, NH(C₁₋₄)alkyl, N(C₁₋₄)alkyl(C₁₋₄)alkyl;
R⁶ is selected from H, halo, (C₁₋₆)alkyl, (C₁₋₄)haloalkyl, cyano, nitro, -C(=O)R^{a}, - C(=O)OR^{a}, -C(=O)NR^{a}R^{a}, -C(=NR^{a})NR^{a}R^{a}, -S(=O)R^{a}, -S(=O)₂R^{a},-S(=O)₂NR^{a}R^{a}, -S(=O)₂N(R^{a})C(=O)R^{a}, -S(=O)₂N(R^{a})C(=O)OR^{a},-S(=O)₂N(R^{a})C(=O)NR^{a}R^{a};
R⁷ is selected from H, halo, (C₁₋₆)alkyl, (C₁₋₄)haloalkyl, cyano, nitro, -C(=O)R^{a}, - C(=O)OR^{a}, -C(=O)NR^{a}R^{a}, -C(=NR^{a})NR^{a}R^{a}, -S(=O)R^{a}, -S(=O)₂R^{a},-S(=O)₂NR^{a}R^{a}, -S(=O)₂N(R^{a})C(=O)R^{a}, -S(=O)₂N(R^{a})C(=O)OR^{a},-S(=O)₂N(R^{a})C(=O)NR^{a}R^{a};
R⁸ is selected from H, (C₁₋₆)haloalkyl, Br, Cl, F, I, OR^{a}, NR^{a}R^{a}, (C₁₋₆alkyl, phenyl, benzyl, heteroaryl and heterocycle, wherein the (C₁₋₆)alkyl, phenyl, benzyl, heteroaryl and heterocycle are additionally substituted by 0, 1, 2 or 3 substituents selected from (C₁₋₆)haloalkyl, OC₁₋₆ alkyl, Br, Cl, F, I and (C₁₋₆)alkyl;
R⁹ is selected from H, halo, (C₁₋₄)haloalkyl, cyano, nitro, -C(=O)R^{a}, -C(=O)OR^{a},-C(=O)NR^{a}R^{a}, -C(NR^{a})NR^{a}R^{a}, -OR^{a}, -OC(=O)R^{a}, -OC(=O)NR^{a}R^{a},-OC(=O)N(R^{a})S(=O)2R^{a}, -OC₂₋₆alkylNR^{a}R^{a}, -OC₂₋₆alkylOR^{a}, -SR^{a}, -S(=O)R^{a}, -S(=O)₂R^{a}, -S(=O)₂NR^{a}R^{a}, -S(=O)₂N(R^{a})C(=O)R^{a}, -S(=O)₂N(R^{a})C(=O)OR^{a}, -S(=O)₂N(R^{a})C(=O)NR^{a}R^{a}, -NR^{a}R^{a}, -N(R^{a})C(=O)R^{a}, -N(R^{a})C(=O)OR^{a},-N(R^{a})C(=O)NR^{a}R^{a}, —N(R^{a})C(=NR^{a})NR^{a}R^{a}, -N(R^{a})S(=O)₂R^{a},-N(R^{a})S(=O)₂NR^{a}R^{a}, -NR^{a}(C₂₋₆alkylNR^{a}R^{a}, -NR^{a}(C₂₋₆alkylOR^{a}, (C₁₋₆)alkyl, phenyl, benzyl, heteroaryl and heterocycle, wherein the (C₁₋₆)alkyl, phenyl, benzyl, heteroaryl and heterocycle are additionally substituted by 0, 1, 2 or 3 substituents selected from halo, (C₁₋₄)haloalkyl, cyano, nitro, -C(=O)R^{a}, -C(=O)OR^{a},-C(=O)NR^{a}R^{a}, -C(NR^{a})NR^{a}R^{a}, -OR^{a}, -OC(=O)R^{a}, OC(=O)NR^{a}R^{a},-OC(=O)N(R^{a})S(=O)₂R^{a}, -OC₂₋₆alkylOR^{a}, -SR^{a}, -S(=O)R^{a}, -S(=O)₂R^{a},-S(=O)₂NR^{a}R^{a}, -S(=O)₂N(R^{a})C(=O)R^{a}, -S(=O)₂N(R^{a})C(=O)OR^{a},-S(=O)₂N(R^{a})C(=O)NR^{a}R^{a}, -NR^{a}R^{a}, -N(R^{a})C(=O)R^{a}, -N(R^{a})C(=O)OR^{a},-N(R^{a})C(=O)NR^{a}R^{a}, —N(R^{a})C(=NR^{a})NR^{a}R^{a}, -N(R^{a})S(=O)₂R^{a},-N(R^{a})S(=O)₂NR^{a}R^{a}, -NR^{a}(C₂₋₆alkylNR^{a}R^{a}, -NR^{a}(C₂₋₆alkylOR^{a}; or R⁹ is a saturated, partially-saturated or unsaturated 5-, 6- or 7-membered monocyclic ring containing 0, 1, 2, 3 or 4 atoms selected from N, O and S, but containing no more than one O or S, wherein the available carbon atoms of the ring are substituted by 0, 1 or 2 oxo or thioxo groups, wherein the ring is substituted by 0, 1, 2, 3 or 4 substituents selected from halo, (C₁₋₄)haloalkyl, cyano, nitro, -C(O)R^{a},-C(=O)OR^{a}, -C(=O)NR^{a}R^{a}, -C(=NR^{a})NR^{a}R^{a}, -OR^{a}, -OC(=O)R^{a},-OC(=O)NR^{a}R^{a}, -OC(=O)N(R^{a})S(=O)₂R^{a}, -OC₂₋₆alkylNR^{a}R^{a}, -OC₂₋₆alkylOR^{a}, -SR^{a}, -S(=O)R^{a}, -S(=O)₂R^{a}, -S(=O)₂NR^{a}R^{a}, -S(=O)₂N(R^{a})C(=O)R^{a},-S(=O)₂N(R^{a})C(=O)OR^{a}, -S(=O)₂N(R^{a})C(=O)NR^{a}R^{a}, -NR^{a}R^{a}, -N(R^{a})C(=O)R^{a}, -N(R^{a})C(=O)OR^{a}, -N(R^{a})C(=O)NR^{a}R^{a}, -N(R^{a})C(=NR^{a})NR^{a}R^{a},-N(R^{a})S(=O)₂R^{a}, -N(R^{a})S(=O)₂NR^{a}R^{a}, -NR^{a}(C₂₋₆ alkylNR^{a}R^{a} and -NR^{a}(C₂₋₆alkylOR^{a};
R¹⁰ is H, (C₁₋₃ alkyl, (C₁₋₃)haloalkyl, cyano, nitro, CO₂R^{a}, C(=O)NR^{a}R^{a},-C(=NR^{a})NR^{a}R^{a}, -S(=O)₂N(R^{a})C(=O)R^{a}, -S(=O)₂N(R^{a})C(=O)OR^{a},-S(=O)₂N(R^{a})C(=O)NR^{a}R^{a}, -S(=O)R^{b}, -S(=O)₂R^{b} or S(=O)₂NR^{a}R^{a};
R¹¹ is H or (C₁₋₄)alkyl;
R^{a} is independently, at each instance, H or R^{b}; and
R^{b} is independently, at each instance, phenyl, benzyl or (C₁₋₆)alkyl, the phenyl, benzyl and (C₁₋₆) alkyl being substituted by 0, 1, 2 or 3 substituents selected from halo, (C₁₋₄)alkyl, (C₁₋₃ haloalkyl, -O(C₁₋₄)alkyl, -NH₂, -NH(C₁₋₄)alkyl, -N(C₁₋₄)alkyl(C₁₋₄)alkyl.

In a preferred embodiment, the PI3K inhibitor (which may be a PI3K-γ inhibitor, PI3K-δ inhibitor, or PI3K-γ,δ inhibitor) is a compound of Formula (VIII) wherein X¹ is C(R⁹) and X² is N.

In a preferred embodiment, the PI3K inhibitor (which may be a PI3K-γ inhibitor, PI3K-δ inhibitor, or PI3K-γ,δ inhibitor) is a compound of Formula (VIII) wherein X¹ is C(R⁹) and X² is C(R¹⁰).

In a preferred embodiment, the PI3K inhibitor (which may be a PI3K-γ inhibitor, PI3K-δ inhibitor, or PI3K-γ,δ inhibitor) is a compound of Formula (VIII) wherein R¹ is phenyl substituted by 0, 1, 2, or 3 independently selected R² substituents.

In a preferred embodiment, the PI3K inhibitor (which may be a PI3K-γ inhibitor, PI3K-δ inhibitor, or PI3K-γ,δ inhibitor) is a compound of Formula (VIII) wherein R¹ is phenyl.

In a preferred embodiment, the PI3K inhibitor (which may be a PI3K-γ inhibitor, PI3K-δ inhibitor, or PI3K-γ,δ inhibitor) is a compound of Formula (VIII) wherein R¹ is selected from 2-methylphenyl, 2-chlorophenyl, 2-trifluoromethylphenyl, 2-fluorophenyl and 2-methoxyphenyl.

In a preferred embodiment, the PI3K inhibitor (which may be a PI3K-γ inhibitor, PI3K-δ inhibitor, or PI3K-γ,δ inhibitor) is a compound of Formula (VIII) wherein R¹ is phenoxy.

In a preferred embodiment, the PI3K inhibitor (which may be a PI3K-γ inhibitor, PI3K-δ inhibitor, or PI3K-γ,δ inhibitor) is a compound of Formula (VIII) wherein R¹ is a direct-bonded or oxygen-linked saturated, partially-saturated or unsaturated 5-, 6- or 7-membered monocyclic ring containing 1, 2, 3 or 4 atoms selected from N, O and S, but containing no more than one O or S, wherein the available carbon atoms of the ring are substituted by 0, 1 or 2 oxo or thioxo groups, wherein the ring is substituted by 0 or 1 R² substituents, and the ring is additionally substituted by 0, 1, 2 or 3 substituents independently selected from halo, nitro, cyano, C₁₋₄alkyl, OC₁₋₄alkyl, OC₁₋₄haloalkyl, NHC₁₋₄alkyl, N(C₁₋₄alkyl)C₁₋₄alkyl and C₁₋₄haloalkyl.

In a preferred embodiment, the PI3K inhibitor (which may be a PI3K-γ inhibitor, PI3K-δ inhibitor, or PI3K-γ,δ inhibitor) is a compound of Formula (VIII) wherein R¹ is an unsaturated 5- or 6-membered monocyclic ring containing 1, 2, 3 or 4 atoms selected from N, O and S, but containing no more than one O or S, wherein the ring is substituted by 0 or 1 R² substituents, and the ring is additionally substituted by 0, 1, 2 or 3 substituents independently selected from halo, nitro, cyano, C₁₋₄alkyl, OC₁₋₄alkyl, OC₁₋₄haloalkyl, NHC₁₋₄alkyl, N(C₁₋₄alkyl)C₁₋₄alkyl and C₁₋₄haloalkyl.

In a preferred embodiment, the PI3K inhibitor (which may be a PI3K-γ inhibitor, PI3K-δ inhibitor, or PI3K-γ,δ inhibitor) is a compound of Formula (VIII) wherein R¹ is an unsaturated 5- or 6-membered monocyclic ring containing 1, 2, 3 or 4 atoms selected from N, O and S, but containing no more than one O or S, wherein the ring is substituted by 0 or 1 R² substituents, and the ring is additionally substituted by 1, 2 or 3 substituents independently selected from halo, nitro, cyano, C₁₋₄alkyl, OC₁₋₄alkyl, OC₁₋₄haloalkyl, NHC₁₋₄alkyl, N(C₁₋₄alkyl)C₁₋₄alkyl and C₁₋₄haloalkyl.

In a preferred embodiment, the PI3K inhibitor (which may be a PI3K-γ inhibitor, PI3K-δ inhibitor, or PI3K-γ,δ inhibitor) is a compound of Formula (VIII) wherein R¹ is an unsaturated 5- or 6-membered monocyclic ring containing 1, 2, 3 or 4 atoms selected from N, O and S.

In a preferred embodiment, the PI3K inhibitor (which may be a PI3K-γ inhibitor, PI3K-δ inhibitor, or PI3K-γ,δ inhibitor) is a compound of Formula (VIII) wherein R¹ is selected from pyridyl and pyrimidinyl.

In a preferred embodiment, the PI3K inhibitor (which may be a PI3K-γ inhibitor, PI3K-δ inhibitor, or PI3K-γ,δ inhibitor) is a compound of Formula (VIII) wherein R³ is selected from halo, C₁₋₄haloalkyl, cyano, nitro, -C(O)R^{a}, -C(=O)OR^{a},-C(=O)NR^{a}R^{a}, -C(NR^{a})NR^{a}R^{a}, -OR^{a}, -OC(=O)R^{a}, -OC(=O)NR^{a}R^{a}, - OC(=O)N(R^{a})S(=O)₂R^{a}, -OC₂₋₆alkylNR^{a}R^{a}, -OC₂₋₆alkylOR^{a}, -SR^{a}, -S(=O)R^{a},-S(=O)₂R^{a}, -S(=O)NR^{a}R^{a}, -S(=O)₂N(R^{a})C(=O)R^{a}, -S(=O)₂N(R^{a})C(O)OR^{a},-S(=O)₂N(R^{a})C(=O)NR^{a}R^{a}, -NR^{a}R^{a}, -N(R^{a})C(=O)R^{a}, -N(R^{a})C(=O)OR^{a},-N(R^{a})C(=O)NR^{a}R^{a}, —N(R^{a})C(=NR^{a})NR^{a}R^{a}, -N(R^{a})S(=O)₂R^{a}, -N(R^{a})S(=O)₂NR^{a}R^{a}, -NR^{a}(C₂₋₆alkylNR^{a}R^{a}, -NR^{a}, C₁₋₆alkyl, phenyl, benzyl, heteroaryl and heterocycle, wherein the C₁₋₆alkyl, phenyl, benzyl, heteroaryl and heterocycle are additionally substituted by 0, 1, 2 or 3 substituents selected from C₁₋₆)haloalkyl, OC₁₋₆alkyl, Br, Cl, F, I and C₁₋₆alkyl.

In a preferred embodiment, the PI3K inhibitor (which may be a PI3K-γ inhibitor, PI3K-δ inhibitor, or PI3K-γ,δ inhibitor) is a compound of Formula (VIII) wherein R³ is H.

In a preferred embodiment, the PI3K inhibitor (which may be a PI3K-γ inhibitor, PI3K-δ inhibitor, or PI3K-γ,δ inhibitor) is a compound of Formula (VIII) wherein R³ is selected from F, Cl, C₁₋₆alkyl, phenyl, benzyl, heteroaryl and heterocycle, wherein the C₁₋₆alkyl, phenyl, benzyl, heteroaryl and heterocycle are additionally substituted by 0, 1, 2 or 3 substituents selected from C₁₋₆)haloalkyl, OC₁₋₆alkyl, Br, Cl, F, I and C₁₋₆alkyl.

In a preferred embodiment, the PI3K inhibitor (which may be a PI3K-γ inhibitor, PI3K-δ inhibitor, or PI3K-γ,δ inhibitor) is a compound of Formula (VIII) wherein R⁵ is, independently, in each instance, H, halo, C₁₋₆alkyl, C₁₋₄haloalkyl, or C₁₋₆alkyl substituted by 1, 2 or 3 substituents selected from halo, cyano, OH, OC₁₋₄)alkyl, C₁₋₄)alkyl, C₁₋₃)haloalkyl, OC₁₋₄)alkyl, NH₂, NHC₁₋₄)alkyl, N(C₁₋₄)alkyl)C₁₋₄)alkyl; or both R⁵ groups together form a C₃₋₆spiroalkyl substituted by 0, 1, 2 or 3 substituents selected from halo, cyano, OH, OC₁₋₄)alkyl,C₁₋₄)alkyl, C₁₋₃)haloalkyl, OC₁₋₄)alkyl, NH₂, NHC₁₋₄)alkyl, N(C₁₋₄)alkyl)C₁₋₄)alkyl.

In a preferred embodiment, the PI3K inhibitor (which may be a PI3K-γ inhibitor, PI3K-δ inhibitor, or PI3K-γ,δ inhibitor) is a compound of Formula (VIII) wherein R⁵ is H.

In a preferred embodiment, the PI3K inhibitor (which may be a PI3K-γ inhibitor, PI3K-δ inhibitor, or PI3K-γ,δ inhibitor) is a compound of Formula (VIII) wherein one R⁵ is S-methyl, the other is H.

In a preferred embodiment, the PI3K inhibitor (which may be a PI3K-γ inhibitor, PI3K-δ inhibitor, or PI3K-γ,δ inhibitor) is a compound of Formula (VIII) wherein at least one R⁵ is halo, C₁₋₆alkyl, C₁₋₄haloalkyl, or C₁₋₆alkyl substituted by 1, 2 or 3 substituents selected from halo, cyano, OH, OC₁₋₄)alkyl,C₁₋₄)alkyl, C₁₋₃)haloalkyl, OC₁₋₄)alkyl, NH₂, NHC₁₋₄)alkyl, N(C₁₋₄)alkyl)C₁₋₄)alkyl.

In a preferred embodiment, the PI3K inhibitor (which may be a PI3K-γ inhibitor, PI3K-δ inhibitor, or PI3K-γ,δ inhibitor) is a compound of Formula (VIII) wherein R⁶ is H.

In a preferred embodiment, the PI3K inhibitor (which may be a PI3K-γ inhibitor, PI3K-δ inhibitor, or PI3K-γ,δ inhibitor) is a compound of Formula (VIII) wherein R⁶ is F, Cl, cyano or nitro.

In a preferred embodiment, the PI3K inhibitor (which may be a PI3K-γ inhibitor, PI3K-δ inhibitor, or PI3K-γ,δ inhibitor) is a compound of Formula (VIII) wherein R⁷ is H.

In a preferred embodiment, the PI3K inhibitor (which may be a PI3K-γ inhibitor, PI3K-δ inhibitor, or PI3K-γ,δ inhibitor) is a compound of Formula (VIII) wherein R⁷ is F, Cl, cyano or nitro.

In a preferred embodiment, the PI3K inhibitor (which may be a PI3K-γ inhibitor, PI3K-δ inhibitor, or PI3K-γ,δ inhibitor) is a compound of Formula (VIII) wherein R⁸ is selected from H, CF₃, C₁₋₃ alkyl, Br, Cl and F.

In a preferred embodiment, the PI3K inhibitor (which may be a PI3K-γ inhibitor, PI3K-δ inhibitor, or PI3K-γ,δ inhibitor) is a compound of Formula (VIII) wherein R⁸ is selected from H.

In a preferred embodiment, the PI3K inhibitor (which may be a PI3K-γ inhibitor, PI3K-δ inhibitor, or PI3K-γ,δ inhibitor) is a compound of Formula (VIII) wherein R⁸ is selected from CF₃, C₁₋₃ alkyl, Br, Cl and F.

In a preferred embodiment, the PI3K inhibitor (which may be a PI3K-γ inhibitor, PI3K-δ inhibitor, or PI3K-γ,δ inhibitor) is a compound of Formula (VIII) wherein R⁹ is H.

In a preferred embodiment, the PI3K inhibitor (which may be a PI3K-γ inhibitor, PI3K-δ inhibitor, or PI3K-γ,δ inhibitor) is a compound of Formula (VIII) wherein R⁹ is selected from halo, C₁₋₄haloalkyl, cyano, nitro, -C(=O)R^{a}, -C(=O)OR^{a}, -C(=O)NR^{a}R^{a}, -C(=NR^{a})NR^{a}R^{a}, -OR^{a}, -OC(=O)R^{a}, -OC(=O)NR^{a}R^{a},-OC(=O)N(R^{a})S(=O)₂R^{a}, -OC₂₋₆alkylNR^{a}R^{a}, -OC₂₋₆alkylOR^{a},-SR^{a}, -S(=O)R^{a},-S(=O)₂R^{a}, -S(=O)₂NR^{a}R^{a}, -S(=O)₂N(R^{a})C(=O)R^{a}, -S(=O)₂N(R^{a})C(=O)OR^{a},-S(=O)₂N(R^{a})C(=O)NR^{a}R^{a}, -NR^{a}R^{a}, -N(R^{a})C(=O)R^{a}, -N(R^{a})C(=O)OR^{a},-N(R^{a})C(=O)NR^{a}R^{a}, -N(R^{a})C(=NR^{a})NR^{a}R^{a}, -N(R^{a})S(=O)₂R^{a}, -N(R^{a})S(=O)2NR^{a}R^{a}, -NR^{a}(C₂₋₆alkylNR^{a}R^{a}, -NR^{a}(C₂₋₆alkylOR^{a}, C₁₋₆alkyl, phenyl, benzyl, heteroaryl and heterocycle, wherein the C₁₋₆alkyl, phenyl, benzyl, heteroaryl and heterocycle are additionally substituted by 0, 1, 2 or 3 substituents selected from halo, C₁₋₄haloalkyl, cyano, nitro, -C(=O)R^{a}, -C(=O)OR^{a}, -C(=O)NR^{a}R^{a}, -C(=NR^{a})NR^{a}R^{a}, -OR^{a},-OC(=O)R^{a}, -OC(=O)NR^{a}R^{a}, -OC(=O)N(R^{a})S(=O)₂R^{a}, -OC₂₋₆alkylOR^{a}, -SR^{a},-S(=O)R^{a}, -S(=O)₂R^{a}, -S(=O)₂NR^{a}R^{a}, -S(=O)₂N(R^{a})C(=O)R^{a},-S(=O)₂N(R^{a})C(=O)OR^{a}, -S(=O)₂N(R^{a})C(=O)NR^{a}R^{a}, -NR^{a}R^{a}, -N(R^{a})C(=O)R^{a},-N(R^{a})C(=O)OR^{a}, -N(R^{a})C(=O)NR^{a}R^{a}, -N(R^{a})C(=NR^{a})NR^{a}R^{a}, -N(R^{a})S(=O)₂R^{a},-N(R^{a})S(=O)₂NR^{a}R^{a}, -NR^{a}(C₂₋₆alkylNR^{a}R^{a}, -NR^{a}(C₂₋₆alkylOR^{a}.

In a preferred embodiment, the PI3K inhibitor (which may be a PI3K-γ inhibitor, PI3K-δ inhibitor, or PI3K-γ,δ inhibitor) is a compound of Formula (VIII) wherein R⁹ is a saturated, partially-saturated or unsaturated 5-, 6- or 7-membered monocyclic ring containing 0, 1, 2, 3 or 4 atoms selected from N, O and S, but containing no more than one O or S, wherein the available carbon atoms of the ring are substituted by 0, 1 or 2 oxo or thioxo groups, wherein the ring is substituted by 0, 1, 2, 3 or 4 substituents selected from halo, C₁₋₄haloalkyl, cyano, nitro, -C(=O)R^{a}, -C(=O)OR^{a}, -C(=O)NR^{a}R^{a}, -C(=NR^{a})NR^{a}R^{a}, -OR^{a}, -OC(=O)R^{a}, -OC(=O)NR^{a}R^{a}, - OC(=O)N(R^{a})S(=O)₂R^{a}, -OC₂₋₆alkylNR^{a}R^{a}, -OC₂₋₆alkylOR^{a},-SR^{a}, -S(=O)R^{a},-S(=O)₂R^{a}, -S(=O)₂NR^{a}R^{a}, -S(=O)₂N(R^{a})C(=O)R^{a}, -S(=O)₂N(R^{a})C(=O)OR^{a},-S(=O)₂N(R^{a})C(=O)NR^{a}R^{a}, -NR^{a}R^{a}, -N(R^{a})C(=O)R^{a}, -N(R^{a})C(=O)OR^{a},-N(R^{a})C(=O)NR^{a}R^{a}, -N(R^{a})C(=NR^{a})NR^{a}R^{a}, -N(R^{a})S(=O)₂R^{a}, -N(R^{a})S(=O)₂NR^{a}R^{a}, -NR^{a}(C₂₋₆alkylNR^{a}R^{a} and -NR^{a}(C₂₋₆alkylOR^{a}.

In a preferred embodiment, the PI3K inhibitor (which may be a PI3K-γ inhibitor, PI3K-δ inhibitor, or PI3K-γ,δ inhibitor) is a compound of Formula (VIII) wherein R¹⁰ is H.

In a preferred embodiment, the PI3K inhibitor (which may be a PI3K-γ inhibitor, PI3K-δ inhibitor, or PI3K-γ,δ inhibitor) is a compound of Formula (VIII) wherein R¹⁰ is cyano, nitro, CO₂R^{a}, C(=O)NR^{a}R^{a}, -C(=NR^{a})NR^{a}R^{a},-S(=O)₂N(R^{a})C(=O)R^{a}, -S(=O)₂N(R^{a})C(=O)OR^{a}, -S(=O)₂N(R^{a})C(=O)NR^{a}R^{a}, S(=O)R^{b}, S(=O)₂R^{b} or S(=O)₂NR^{a}R^{a}.

In a preferred embodiment, the PI3K inhibitor (which may be a PI3K-γ inhibitor, PI3K-δ inhibitor, or PI3K-γ,δ inhibitor) is a compound of Formula (VIII) wherein R¹¹ is H.

In a preferred embodiment, the PI3K-δ inhibitor is a compound of Formula (IX): 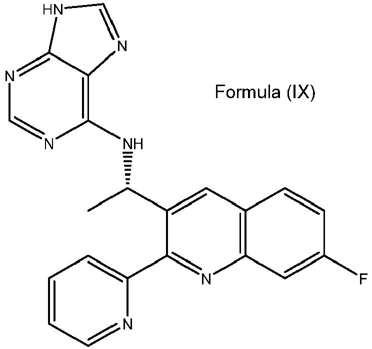 or a pharmaceutically acceptable salt, solvate, or hydratethereof.

In a preferred embodiment, the PI3K inhibitor or PI3K-δ inhibitor is *(S)-N-*(1-(7-fluoro-2-(pyridin-2-yl)quinolin-3-yl)ethyl)-9*H*-purin-6-amine or a pharmaceutically acceptable salt, solvate, or hydrate thereof.

In a preferred embodiment, the PI3K-δ inhibitor is a compound of Formula (X): 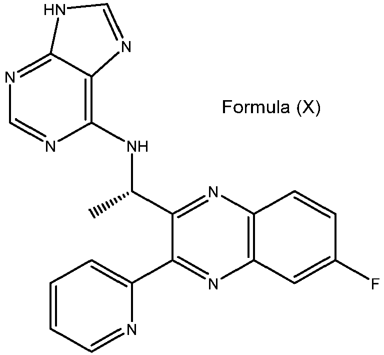 or a pharmaceutically acceptable salt, solvate, or hydrate thereof.

In a preferred embodiment, the PI3K inhibitor or PI3K-δ inhibitor is *(S)-N-*(1-(6-fluoro-3-(pyridin-2-yl)quinoxalin-2-yl)ethyl)-9*H*-purin-6-amine or a pharmaceutically acceptable salt, solvate, or hydrate thereof.

In a preferred embodiment, the PI3K-δ inhibitor is a compound of Formula (XI): 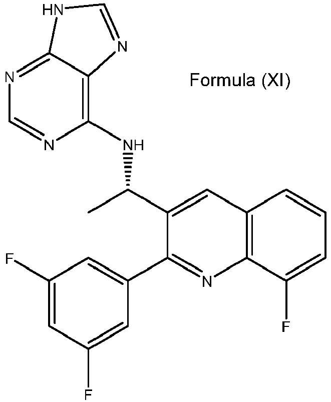 or a pharmaceutically acceptable salt, solvate, or hydrate thereof.

In a preferred embodiment, the PI3K-δ inhibitor is (*S*)-*N*-(1-(2-(3,5-difluorophenyl)-8-fluoroquinolin-3-yl)ethyl)-9*H*-purin-6-amine or a pharmaceutically-acceptable salt, solvate, or hydrate thereof.

In a preferred embodiment, the PI3K-δ inhibitor is a compound of Formula (XII): 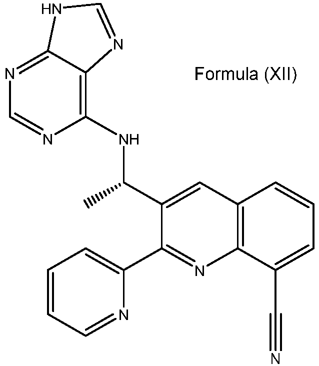 or a pharmaceutically acceptable salt, solvate, or hydrate thereof.

In a preferred embodiment, the PI3K-δ inhibitor is (*S*)-3-(1-((9*H*-purin-6-yl)amino)ethyl)-2-(pyridin-2-yl)quinoline-8-carbonitrile or a pharmaceutically acceptable salt, solvate, or hydrate thereof.

In a preferred embodiment, the PI3K-δ inhibitor is a compound of Formula (XIII): 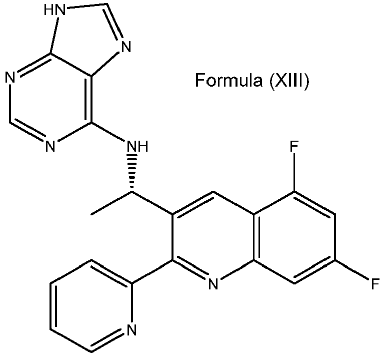 or a pharmaceutically acceptable salt, solvate, or hydrate thereof

In a preferred embodiment, the PI3K-δ inhibitor is (*S*)-*N*-(1-(5,7-difluoro-2-(pyridin-2-yl)quinolin-3-yl)ethyl)-9*H*-purin-6-amine or a pharmaceutically acceptable salt, solvate, or hydrate thereof.

In an embodiment, the PI3K inhibitor or PI3K-δ inhibitor is a compound selected from the structures disclosed in U.S. Patent Nos. 7,932,260 and 8,207,153. In an embodiment, the PI3K inhibitor or PI3K-δ inhibitor is a compound of Formula (XIV): 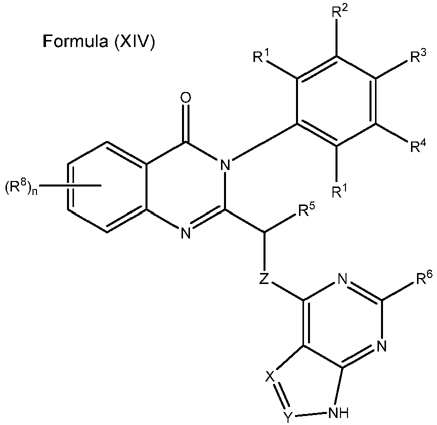 wherein
X and Y, independently, are N or CH;
Z is N-R⁷ or O;
R¹ are the same and are hydrogen, halo, or C₁₋₃ alkyl;
R² and R³, independently, are hydrogen, halo, or C₁₋₃ alkyl;
R⁴ is hydrogen, halo, OR^{a}, CN, C₂₋₆alkynyl, C(=O)R^{a}, C(=O)NR^{a}R^{b}, C₃-₆heterocycloalkyl, C₁₋₃ alkyleneC₃₋₆heterocycloalkyl, O(C₁₋₃)alkyleneOR^{a}, O(C₁₋₃)alkyleneNR^{a}R^{b}, O(C₁₋₃)alkyleneC₃₋₆ cycloalkyl, OC₃₋₆heterocycloalkyl, O(C₁₋₃)alkyleneC≡CH, or O(C₁₋₃)alkyleneC(=O)NR^{a}R^{b};
R⁵ is (C₁₋₃)alkyl, CH₂CF₃, phenyl, CH₂C≡CH, (C₁₋₃)alkyleneOR^{e}, (C₁₋₄)alkyleneNR^{a}R^{b}, or C₁₋₄ alkyleneNHC(=O)OR^{a},
R⁶ is hydrogen, halo, or NR^{a}R^{b};
R⁷ is hydrogen or R⁵ and R⁷ are taken together with the atoms to which they are attached to form a five- or six-membered saturated ring;
R⁸ is C₁₋₃ alkyl, halo, CF₃, or CH₂C₃₋₆heterocycloalkyl;
n is 0, 1, or 2;
R^{a} is hydrogen, (C₁₋₄)alkyl, or CH2C6H5;
R^{b} is hydrogen or C₁₋₃ alkyl; and
R^{c} is hydrogen, C₁₋₃ alkyl, or halo,
wherein when the R¹ groups are different from hydrogen, R² and R⁴ are the same; or a pharmaceutically acceptable salt, solvate, or hydrate thereof.

In a preferred embodiment, the PI3K inhibitor or PI3K-δ inhibitor is an enantiomer of Formula (XIV), as shown in Formula (XV): 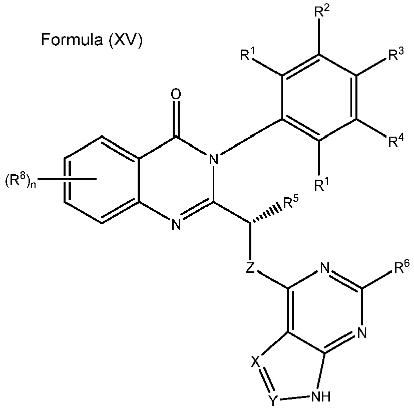 wherein X, Y, Z, R¹ through R⁸, R^{a}, R^{b}, R^{c}, and n are as defined above for Formula (XIV).

In various embodiments exhibiting increased potency relative to other compounds, n = 1, 2, or 3 and R⁸ is C₁₋₃alkyl, F, Cl, or CF₃. Alternatively, in such embodiments, n is 0 (such that there is no R⁸ substituent).

In further embodiments exhibiting increased potency, X is N and Y is CH. Alternatively, X and Y may also both be CH.

In further embodiments exhibiting increased potency, R⁶ is hydrogen, halo, or NH₂. Preferably, R⁶ is hydrogen.

In preferred embodiments exhibiting increased potency, n is 0 or 1; R⁸ (if n is 1) is C₁₋₃alkyl, F, Cl, or CF₃; R⁶ is hydrogen; X is N and Y is CH or X and Y are both CH; Z is NH; R¹ are the same and are hydrogen, halo, or C₁₋₃alkyl; and R² and R³, independently, are hydrogen, halo, or C₁₋₃alkyl. Preferably, R¹, R², and R³ are hydrogen.

Unexpectedly, potency against PI3K-δ is conserved when R¹ is the same. In structural formulae (I) and (II), R² and R⁴ may differ provided that R¹ is H. When R¹ is H, free rotation is unexpectedly permitted about the bond connecting the phenyl ring substituent to the quinazoline ring, and the compounds advantageously do not exhibit atropisomerism (i.e., multiple diasteromer formation is avoided). Alternatively, R² and R⁴ can be the same such that the compounds advantageously do not exhibit atropisomerism.

In preferred embodiments, Z is N-R⁷, and the bicyclic ring system containing X and Y is:

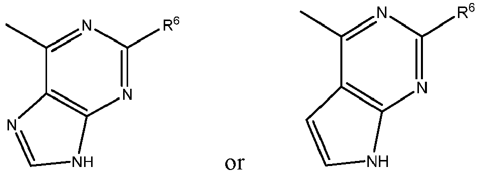

In other preferred embodiments of Formula (XIV) or Formula (XV), X, Y, Z, R^{a}, R^{b}, and R^{c} are as defined above for Formula (XIV), and R¹ is hydrogen, fluoro, chloro, methyl, or 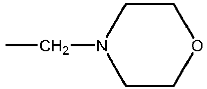 and R² is hydrogen, methyl, chloro, or fluoro; R³ is hydrogen or fluoro; R⁶ is NH₂, hydrogen, or fluoro; R⁷ is hydrogen or R⁵ and R⁷ are taken together to form 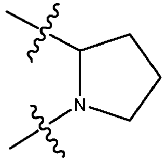 R⁸ is methyl, trifluoromethyl, chloro, or fluoro; R⁴ is hydrogen, fluoro, chloro, OH, OCH₃, OCH₂C=CH, O(CH₂)₂N(CH₃)₂, C(=O)CH₃, C=CH, CN, C(=O)NH₂, OCH₂C(=O)NH₂, O(CH₂)₂OCH₃, O(CH₂)₂N(CH₃)₂, 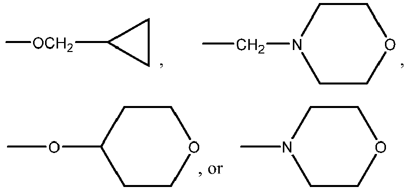 and R⁵ is methyl, ethyl, propyl, phenyl, CH₂OH, CH₂OCH₂C₆H₅, CH₂CF₃, CH₂OC(CH₃)₃, CH₂C≡CH, (CH₂)₃N(C₂H₅)₂, (CH2)3NH2, (CH2)4NH2, (CH₂)₃NHC(=O)OCH₂C₆H₅, or (CH₂)₄NHC(=O)OCH₂C₆H₅; R^{c} is hydrogen, methyl, fluoro, or bromo; and n is 0 or 1.

As used with respect to Formula (XIV) and Formula (XV), the term "alkyl" is defined as straight chained and branched hydrocarbon groups containing the indicated number of carbon atoms, e.g., methyl, ethyl, and straight chain and branched propyl and butyl groups. The terms "(C₁₋₃)alkylene" and "(C₁₋₄)alkylene" are defined as hydrocarbon groups containing the indicated number of carbon atoms and one less hydrogen than the corresponding alkyl group. The term "(C₂₋₆)alkynyl" is defined as a hydrocarbon group containing the indicated number of carbon atoms and a carbon-carbon triple bond. The term "(C₃₋₆)cycloalkyl" is defined as a cyclic hydrocarbon group containing the indicated number of carbon atoms. The term "(C₂₋₆)heterocycloalkyl" is defined similarly as cycloalkyl except the ring contains one or two heteroatoms selected from the group consisting of O, NR^{a}, and S. The term "halo" is defined as fluoro, bromo, chloro, and iodo.

In a preferred embodiment, the PI3K inhibitor (which may be a PI3K-γ inhibitor, PI3K-δ inhibitor, or PI3K-γ,δ inhibitor) is idelalisib. In a preferred embodiment, the PI3K inhibitor (which may be a PI3K-γ inhibitor, PI3K-δ inhibitor, or PI3K-γ,δ inhibitor) is the compound of Formula (XVI): 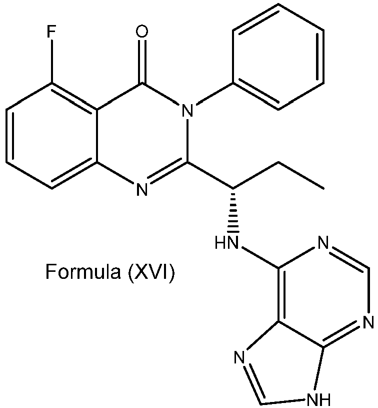 or a pharmaceutically acceptable salt, solvate, or hydrate thereof.

In a preferred embodiment, the PI3K inhibitor (which may be a PI3K-γ inhibitor, PI3K-δ inhibitor, or PI3K-γ,δ inhibitor) is (*S*)-2-(1-((9*H*-purin-6-yl)amino)propyl)-5-fluoro-3-phenylquinazolin-4(3*H*)-one or a pharmaceutically acceptable salt, solvate, or hydrate thereof.

In an embodiment, the PI3K inhibitor (which may be a PI3K-γinhibitor, PI3K-δ inhibitor, or PI3K-y,δ inhibitor) is 4(3*H*)-quinazolinone, 5-fluoro-3-phenyl-2-[(1*S*)-1-(9*H*-purin-6-ylamino)propyl]-5-fluoro-3-phenyl-2-{(1*S*)-1-[(7*H*-purin-6-yl)amino]propyl}quinazolin-4(3*H*)-one or a pharmaceutically acceptable salt, solvate, or hydrate thereof

In an embodiment, the PI3K inhibitor (which may be a PI3K-γ inhibitor, PI3K-δ inhibitor, or PI3K-γ,δ inhibitor) is GS-9901. Other PI3K inhibitors suitable for use in the described combination with a BTK inhibitor also include, but are not limited to, those described in, for example, U.S. Patent No. 8,193,182 and U.S. Published Application Nos. 2013/0267521; 2013/0053362; 2013/0029984; 2013/0029982; 2012/0184568; and 2012/0059000.

### BTK Inhibitors

The BTK inhibitor in the combination for use in the present invention is a compound of Formula (XVIII): 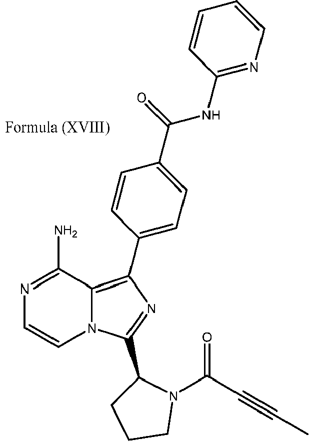 or a pharmaceutically acceptable salt thereof. The preparation of this compound is described in International Patent Application Publication No. WO 2013/010868 and U.S. Patent Application Publication No. US 2014/0155385 A1. In brief, Formula (XVIII), may be prepared as follows.

(*S*)-4-(8-amino-3-(1-(but-2-ynoyl)pyrrolidin-2-yl)imidazo[1,5-*a*]pyrazin-1-yl)-*N*-(pyridin-2-yl)benzamide was made from (*S*)-4-(8-Amino-3-(pyrrolidin-2-yl)imidazo[1 ,5-*a*]pyrazin-1-yl)-N-(pyridin-2-yl)benzamide and 2-butynoic acid as follows. To a solution of (*S*)-4-(8-Amino-3-(pyrrolidin-2-yl)imidazo[1,5-*a*]pyrazin-1-yl)-N-(pyridin-2-yl)benzamide (19.7 mg, 0.049 mmol), triethylamine (20 mg, 0.197 mmol, 0.027 mL) 2-butynoic acid (4.12 mg, 0.049 mmol) in dichloromethane (2 mL) was added HATU (18.75 mg, 0.049 mmol). The mixture was stirred for 30 min at room temperature. The mixture was washed with water dried over magnesium sulfate and concentrated in vacuo. The residue was purified by preparative HPLC. Fractions containing product were collected and reduced to dryness to afford the title compound (10.5 mg, 18.0%).

(*S*)-4-(8-Amino-3-(pyrrolidin-2-yl)imidazo[1,5-*a*]pyrazin-1-yl)-*N*-(pyridin-2-yl)benzamide was prepared from the following intermediary compounds.
(a). (3-Chloropyrazin-2-yl)methanamine hydrochloride was prepared as follows. To a solution of 3-chloropyrazine-2-carbonitrile (160 g, 1 .147 mol) in acetic acid (1.5 L) was added Raney Nickel (50% slurry in water, 70 g, 409 mmol). The resulting mixture was stirred under 4 bar hydrogen at room temperature overnight. Raney Nickel was removed by filtration over decalite and the filtrate was concentrated under reduced pressure and co-evaporated with toluene. The remaining brown solid was dissolved in ethyl acetate at 50°C and cooled on an ice-bath. 2M hydrogen chloride solution in diethyl ether (1 .14 L) was added in 30 min. The mixture was allowed to stir at room temperature over weekend. The crystals were collected by filtration, washed with diethyl ether and dried under reduced pressure at 40°C. The product brown solid obtained was dissolved in methanol at 60°C. The mixture was filtered and partially concentrated, cooled to room temperature and diethyl ether (1000 ml) was added. The mixture was allowed to stir at room temperature overnight. The solids formed were collected by filtration, washed with diethyl ether and dried under reduced pressure at 40°C to give 153.5 g of (3-chloropyrazin-2-yl)methanamine.hydrochloride as a brown solid (74.4 %, content 77 %).
(b). (*S*)-benzyl 2-((3-chloropyrazin-2-yl)methylcarbamoyl)pyrrolidine-1-carboxylate was prepared as follows. To a solution of (3-chloropyrazin-2-yl)methanamine HCI (9.57 g, 21.26 mmol, 40% wt) and Z-Pro-OH (5.3 g, 21.26 mmol) in dichloromethane (250 mL) was added triethylamine (11.85 mL, 85 mmol) and the reaction mixture was cooled to 0°C. After 15 min stirring at 0°C, HATU (8.49 g, 22.33 mmol) was added. The mixture was stirred for 1 hour at 0°C and then overnight at room temperature. The mixture was washed with 0.1 M HCI-solution, 5% NaHC03, water and brine, dried over sodium sulfate and concentrated in vacuo. The product was purified using silica gel chromatography (heptane/ethyl acetate = 1/4 v/v%) to give 5 g of (S)-benzyl 2-((3-chloropyrazin-2-yl)methylcarbamoyl)pyrrolidine-1-carboxylate (62.7%).
(c). (*S*)-Benzyl 2-(8-chloroimidazo[1,5-*a*]pyrazin-3-yl)pyrrolidine-1-carboxylate was prepared as follows. (*S*)-Benzyl 2-((3-chloropyrazin-2-yl)metbylcarbamoyl)pyrrolidine-1-carboxylate (20.94 mmol, 7.85 g) was dissolved in acetonitrile (75 ml), 1 ,3-dimethyl-2-imidazolidinone (62.8 mmol, 6.9 ml, 7.17 g) was added and the reaction mixture was cooled to 0°C before POCI3 (84 mmol, 7.81 ml, 12.84 g) was added drop wise while the temperature remained around 5°C. The reaction mixture was refluxed at 60-65°C overnight. The reaction mixture was poured carefully in ammonium hydroxide 25% in water (250 ml)/crushed ice (500 ml) to give a yellow suspension (pH -8-9) which was stirred for 15 min until no ice was present in the suspension. Ethyl acetate was added, layers were separated and the aqueous layer was extracted with ethyl acetate (3x). The organic layers were combined and washed with brine, dried over sodium sulfate, filtered and evaporated to give 7.5 g crude product. The crude product was purified using silica gel chromatography (heptane/ethyl acetate = 1/4 v/v%) to give 6.6 g of (S)-benzyl 2-(8- chloroimidazo[1 ,5-a]pyrazin-3-yl)pyrrolidine-1-carboxylate (88%).
(d). (S)-Benzyl 2-(1-bromo-8-chloroimidazo[1 ,5-a]pyrazin-3-yl)pyrrolidine-1-carboxylate was prepared as follows. N-Bromosuccinimide (24.69 mmol, 4.4 g) was added to a stirred solution of (S)-benzyl 2-(8- chloroimidazo[1,5-a]pyrazin-3-yl)pyrrolidine-1-carboxylate (24.94 mmol, 8.9 g) in DMF (145 mL). The reaction was stirred 3 h at rt. The mixture was poored (slowly) in a stirred mixture of water (145 mL), ethyl acetate (145 mL) and brine (145 mL). The mixture was then transferred into a separating funnel and extracted. The water layer was extracted with 2x145 mL ethyl acetate. The combined organic layers were washed with 3x300 mL water, 300 mL brine, dried over sodium sulfate, filtered and evaporated. The product was purified using silica gel chromatography (ethyl acetate/heptane = 3/1 v/v%) to give 8.95 g of (S)-benzyl 2-(1-bromo-8-chloroimidazo[1,5-a]pyrazin-3-yl)pyrrolidine-1-carboxylate (82.3%).
(e). (*S*)-Benzyl 2-(8-amino-1-bromoimidazo[1,5-*a*]pyrazin-3-yl)pyrrolidine-1-carboxylate was prepared as follows. (S)-Benzyl 2-(8-amino-1-bromoimidazo[1,5-a]pyrazin-3-yl)pyrrolidine-1-carboxylate (20.54 mmol, 8.95 g) was suspended in 2-propanol (113 ml) in a pressure vessel. 2-propanol (50 ml) was cooled to -78°C in a preweighed flask (with stopper and stirring bar) and ammonia gas (646 mmol, 11 g) was lead through for 15 minutes. The resulting solution was added to the suspension in the pressure vessel. The vessel was closed and stirred at room temperature and a slight increase in pressure was observed. Then the suspension was heated to 110 °C which resulted in an increased pressure to 4.5 bar. The clear solution was stirred at 1 10 °C, 4.5 bar overnight. After 18h the pressure remained 4 bar. The reaction mixture was concentrated in vacuum, the residue was suspended in ethyl acetate and subsequent washed with water. The layers were separated and the aqueous layer was extracted with ethyl acetate. The combined organic layers were washed with water, saturated sodium chloride solution, dried over sodium sulfate and concentrated to give 7.35 g of (S)-benzyl 2-(8-amino-1-bromoimidazo[1,5-a]pyrazin-3-yl)pyrrolidine-1-carboxylate (86%).

(S)-4-(8-Amino-3-(pyrrolidin-2-yl)imidazo[1,5-a]pyrazin-1-yl)-N-(pyridin-2-yl)benzamide was prepared as follows.
(a). (S)-benzyl 2-(8-amino-1-(4-(pyridin-2-ylcarbamoyl)phenyl)imidazo[1,5-a]pyrazin-3-yl)pyrrolidine-1-carboxylate was prepared as follows. (S)-benzyl 2-(8-amino-1-bromoimidazo[1,5-a]pyrazin-3-yl)pyrrolidine-1-carboxylate (0.237 mmol, 98.5 mg) and 4-(pyridin-2-yl-aminocarbonyl)benzeneboronic acid (0.260 mmol, 63.0 mg) were suspended in a mixture of 2N aqueous potassium carbonate solution (2.37 mmol, 1.18 mL) and dioxane (2.96 mL). Nitrogen was bubbled through the mixture, followed by the addition of 1,1'-bis(diphenylphosphino)ferrocene palladium (ii) chloride (0.059 mmol, 47.8 mg). The reaction mixture was heated for 20 minutes at 140°C in the microwave. Water was added to the reaction mixture, followed by an extraction with ethyl acetate (2x). The combined organic layer was washed with brine, dried over magnesium sulfate and evaporated. The product was purified using silicagel and dichloromethane/methanol = 9/1 v/v% as eluent to afford 97.1 mg of (S)-benzyl 2-(8-amino-1-(4-(pyridin-2-ylcarbamoyl)phenyl)imidazo[1,5-a]pyrazin-3-yl)pyrrolidine-1-carboxylate (77%).
(b). (S)-4-(8-Amino-3-(pyrrolidin-2-yl)imidazo[1,5-alpyrazin-1-yl)-N-(pyridin-2-yl)benzamide was prepared as follows. To (S)-benzyl 2-(8-amino-1-(4-(pyridin-2-ylcarbamoyl)phenyl)imidazo[1,5-a]pyrazin-3-yl)pyrrolidine-1- carboxylate (0.146 mmol, 78 mg) was added a 33% hydrobromic acid/acetic acid solution (1 1.26 mmol, 2 ml) and the mixture was left at room temperature for 1 hour. The mixture was diluted with water and extracted with dichloromethane. The aqueous phase was neutralized using 2N sodium hydroxide solution, and then extracted with dichloromethane. the organic layer was dried over magnesium sulfate, filtered and evaporated to give 34 mg of (S)-4-(8-Amino-3-(pyrrolidin-2-yl)imidazo[1,5-a]pyrazin-1-yl)-N-(pyridin-2-yl)benzamide (58%).

In a preferred embodiment, the BTK inhibitor is (*S*)-4-(8-amino-3-(1-(but-2-ynoyl)pyrrolidin-2-yl)imidazo[1,5-*a*]pyrazin-1-yl)-*N*-(pyridin-2-yl)benzamideor a pharmaceutically acceptable salt, thereof.

### JAK-2 Inhibitors

The JAK-2 inhibitor may be any JAK-2 inhibitor known in the art. In particular, it is one of the JAK-2 inhibitors described in more detail in the following paragraphs. For avoidance of doubt, references herein to a JAK-2 inhibitor may refer to a compound or a pharmaceutically acceptable salt, ester, solvate, or hydrate thereof.

In an embodiment, the JAK-2 inhibitor is a compound of Formula (XXIX): 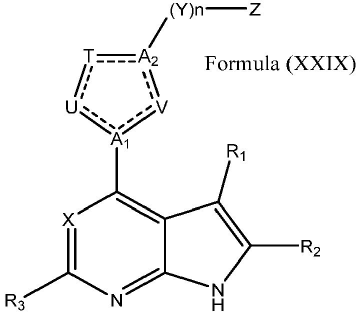 or a pharmaceutically acceptable salt, solvate, or hydrate, thereof, wherein:
A¹ and A² are independently selected from C and N;
T, U, and V are independently selected from O, S, N, CR⁵, and NR⁶;
wherein the 5-membered ring formed by A¹, A², U, T, and V is aromatic;
X is N or CR⁴;
Y is C₁₋₈ alkylene, C₂₋₈ alkenylene, C₂₋₈ alkynylene, (CR¹¹R¹²)ₚ-(C₃₋₁₀ cycloalkylene)-(CR¹¹R¹²)_{q}, (CR¹¹R¹²)ₚ-(arylene)-(CR¹¹R¹²)_{q}, (CR¹¹R¹²)ₚ₋(C₁₋₁₀ heterocycloalkylene)-(CR¹¹R¹²)_{q}, (CR¹¹R¹²)ₚ-(heteroarylene)-(CR¹¹R¹²)_{q}, (CR¹¹R¹²)ₚO(CR¹¹R¹²)_{q}, (CR¹¹R¹²)ₚS(CR¹¹R¹²)_{q}, (CR¹¹R¹²)ₚC(O)(CR¹¹R¹²)_{q}, (CR¹¹R¹²)ₚC(O)NR^{c}(CR¹¹R¹²)q, (CR¹¹R¹¹)ₚC(O)O(CR¹¹R¹²)_{q}, (CR¹¹R¹²)ₚOC(O)(CR¹¹R¹²)_{q}, (CR¹¹R¹²)ₚOC(O)NR^{c}(CR¹¹R¹²)_{q}, (CR¹¹R¹²)ₚNR^{c}(CR¹¹R¹²)_{q}, (CR¹¹R¹²)ₚNR^{c}C(O)NR^{d}(CR¹¹R¹²)_{q}, (CR¹¹R¹²)ₚS(O)(CR¹¹R¹²)_{q}, (CR¹¹R¹²)ₚS(O)NR^{c}(CR¹¹R¹²)_{q}, (CR¹¹R¹²)ₚS(O)₂(CR¹¹R¹²)_{q}, or (CR¹¹R¹²)ₚS(O)₂NR^{C}(CR¹¹R¹²)_{q}, wherein said C₁₋₈ alkylene, C₂₋₈ alkenylene, C₂₋₈ alkynylene, cycloalkylene, arylene, heterocycloalkylene, or heteroarylene, is optionally substituted with 1, 2, or 3 substituents independently selected from -D¹-D²-D³-D⁴;
Z is H, halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, =C-Rⁱ, =N-Rⁱ, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{C}S(O)₂R^{b}, C(=NOH)R^{b}, C(=NO(C₁₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d};
wherein when Z is H, n is 1;
or the -(Y)ₙ-Z moiety is taken together with i) A² to which the moiety is attached, ii) R⁵ or R⁶ of either T or V, and iii) the C or N atom to which the R⁵ or R⁶ of either T or V is attached to form a 4- to 20-membered aryl, cycloalkyl, heteroaryl, or heterocycloalkyl ring fused to the 5-membered ring formed by A¹, A², U, T, and V, wherein said 4- to 20-membered aryl, cycloalkyl, heteroaryl, or heterocycloalkyl ring is optionally substituted by 1, 2, 3, 4, or 5 substituents independently selected from - (W)ₘ-Q;
W is C₁₋₈ alkylenyl, C₂₋₈ alkenylenyl, C₂₋₈ alkynylenyl, O, S, C(O), C(O)NR^{c'}, C(O)O, OC(O), OC(O)NR^{c'}, NR^{c'}, NR^{c'}C(O)NR^{d'}, S(O), S(O)NR^{c'}, S(O)₂, or S(O)₂NR^{c'};
Q is H, halo, CN, NO₂, C₁₋₈ alkyl, C₂₋₈ alkenyl, C₂₋₈ alkynyl, C₁₋₈ haloalkyl, halosulfanyl, aryl, cycloalkyl, heteroaryl, or heterocycloalkyl, wherein said C₁₋₈ alkyl, C₂₋₈ alkenyl, C₂₋₈ alkynyl, C₁₋₈ haloalkyl, aryl, cycloalkyl, heteroaryl, or heterocycloalkyl is optionally substituted with 1, 2, 3 or 4 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy², CN, NO₂, OR^{a'}, SR^{a'}, C(O)R^{b'}, C(O)NR^{c'}R^{d'}, C(O)OR^{a'}, OC(O)R^{b'}, OC(O)NR^{c'}R^{d'}, NR^{c'}R^{d'}, NR^{c'}C(O)R^{b'}, NR^{c'}C(O)NR^{c'}R^{d'}, NR^{c'}C(O)OR^{a'}, S(O)R^{b'}, S(O)NR^{c'}R^{d'}, S(O)₂R^{b'}, NR^{c'}S(O)₂R^{b'}, and S(O)₂NR^{c'}R^{d'};
Cy¹ and Cy² are independently selected from aryl, heteroaryl, cycloalkyl, and heterocycloalkyl, each optionally substituted by 1, 2, 3, 4 or 5 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, CN, NO₂, OR^{a"}, SR^{a"}, C(O)R^{b"}, C(O)NR^{c"}R^{d"}, C(O)OR^{a"}, OC(O)R^{b"}OC(O)NR^{c"}R^{d"}, NR^{c"}R^{d"}, NR^{c"}C(O)R^{b"}, NR^{c"}C(O)OR^{a"}, NR^{c"}S(O)R^{b"}, NR^{c"}S(O)₂R^{b"}, S(O)R^{b"}, S(O)NR^{c"}R^{d"}, S(O)₂R^{b"}, and S(O)₂NR^{c"}R^{d"};
R¹, R², R³, and R⁴ are independently selected from H, halo, C₁₋₄ alkyl, C₂₋₄ alkenyl, C₂₋₄ alkynyl, C₁₋₄ haloalkyl, halosulfanyl, aryl, cycloalkyl, heteroaryl, heterocycloalkyl, CN, NO₂, OR⁷, SR⁷, C(O)R⁸, C(O)NR⁹R¹⁰, C(O)OR⁷OC(O)R⁸, OC(O)NR⁹R¹⁰, NR⁹R¹⁰, NR⁹C(O)R⁸, NR^{c}C(O)OR⁷, S(O)R⁸, S(O)NR⁹R¹⁰, S(O)₂R⁸, NR⁹S(O)₂R⁸, and S(O)₂NR⁹R¹⁰;
R⁵ is H, halo, C₁₋₄ alkyl, C₂₋₄ alkenyl, C₂₋₄ alkynyl, C₁₋₄ haloalkyl, halosulfanyl, CN, NO₂, OR⁷, SR⁷, C(O)R⁸, C(O)NR⁹R¹⁰, C(O)OR⁷, OC(O)R⁸, OC(O)NR⁹R¹⁰, NR⁹R¹⁰, NR⁹C(O)R⁸, NR⁹C(O)OR⁷, S(O)R⁸, S(O)NR⁹R¹⁰, S(O)₂R⁸, NR⁹S(O)₂R⁸, or S(O)₂NR⁹R¹⁰;
R⁶ is H, C₁₋₄ alkyl, C₂₋₄ alkenyl, C₂₋₄ alkynyl, C₁₋₄ haloalkyl, OR⁷, C(O)R⁸, C(O)NR⁹R¹⁰, C(O)OR⁷, S(O)R⁸, S(O)NR⁹R¹⁰, S(O)₂R⁸, or S(O)₂NR⁹R¹⁰;
R⁷ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
R⁸ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
R⁹ and R¹⁰ are independently selected from H, C₁₋₁₀ alkyl, C₁₋₆ haloalkyl, C₂₋₆ alkenyl, C₂₋₆ alkynyl, C₁₋₆ alkylcarbonyl, arylcarbonyl, C₁₋₆ alkylsulfonyl, arylsulfonyl, aryl, heteroaryl, cycloalkyl, heterocycloalkyl, arylalkyl, heteroarylalkyl, cycloalkylalkyl and heterocycloalkylalkyl;
or R⁹ and R¹⁰ together with the N atom to which they are attached form a 4-, 5-, 6- or 7-membered heterocycloalkyl group;
R¹¹ and R¹² are independently selected from H and -E¹-E²-E³-E⁴;
D¹ and E¹ are independently absent or independently selected from C₁₋₆ alkylene, C₂₋₆ alkenylene, C₂₋₆ alkynylene, arylene, cycloalkylene, heteroarylene, and heterocycloalkylene, wherein each of the C₁₋₆ alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆ alkyl, C₁₋₆ haloalkyl, C₂₋₈ alkoxyalkyl, C₁₋₆ alkoxy, C₁₋₆ haloalkoxy, amino, C₁₋₆ alkylamino, and C₂₋₈ dialkylamino;
D² and E² are independently absent or independently selected from C₁₋₆ alkylene, C₂₋₆ alkenylene, C₂₋₆ alkynylene, (Ci-6 alkylene)ᵣ-O-(C₁₋₆ alkylene)s, (C₁₋₆ alkylene)ᵣ-S-(Ci-6 alkylene)s, (C₁₋₆ alkylene)s, -NR^{e}-(C₁₋₆ alkylene)s, (C₁₋₆ alkylene)ᵣ-CO-(C₁₋₆ alkylene)s, (C₁₋₆ alkylene)ᵣ-COO-(C₁₋₆ alkylene)s, (C₁₋₆ alkylene)ᵣ-CONR^{e}-(C₁₋₆ alkylene)s, (C₁₋₆ alkylene)ᵣ-SO--(C₁₋₆ alkylene)s, (C₁₋₆ alkylene)ᵣ-SO₂--(C₁₋₆ alkylene)s, (Ci-6 alkylene)ᵣ-SONR^{e}-(C₁₋₆ alkylene)s, and (Ci-6 alkylene)r-NR^{e}CONR^{f}-(C₁₋₆alkylene)ₛ, wherein each of the C₁₋₆ alkylene, C₂₋₆ alkenylene, and C₂₋₆ alkynylene is optionally substituted by 1, 2 or 3 substituents independently selected from halo, CN, NO₂, N₃, SCN, OH, C₁₋₆ alkyl, C₁₋₆ haloalkyl, C₂₋₈ alkoxyalkyl, C₁₋₆ alkoxy, C₁₋₆ haloalkoxy, amino, C₁₋₆ alkylamino, and C₂₋₈ dialkylamino;
D³ and E³ are independently absent or independently selected from C₁₋₆ alkylene, C₂₋₆ alkenylene, C₂₋₆ alkynylene, arylene, cycloalkylene, heteroarylene, and heterocycloalkylene, wherein each of the C₁₋₆ alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆ alkyl, C₁₋₆ haloalkyl, C₂₋₈ alkoxyalkyl, C₁₋₆ alkoxy, C₁₋₆ haloalkoxy, amino, C₁₋₆ alkylamino, and C₂₋₈ dialkylamino;
D⁴ and E⁴ are independently selected from H, halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl))R^{b}, and S(O)₂NR^{c}R^{d};
R^{a} is H, Cy¹, --(C₁₋₆ alkyl)-Cy¹, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆ alkyl, C₁₋₆ haloalkyl, halosulfanyl, aryl, arylalkyl, heteroaryl, heteroarylalkyl, cycloalkyl and heterocycloalkyl;
R^{b} is H, Cy¹, --(C₁₋₆ alkyl)-Cy¹, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
R^{a'} and R^{a"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, halosulfanyl, aryl, arylalkyl, heteroaryl, heteroarylalkyl, cycloalkyl and heterocycloalkyl;
R^{b'} and R^{b"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
R^{c} and R^{d} are independently selected from H, Cy¹, --(C₁₋₆ alkyl)-Cy¹, C₁₋₁₀ alkyl, C₁₋₆ haloalkyl, C₂₋₆ alkenyl, C₂₋₆ alkynyl, wherein said C₁₋₁₀ alkyl, C₁₋₆ haloalkyl, C₂₋₆ alkenyl, or C₂₋₆ alkynyl, is optionally substituted with 1, 2, or 3 substituents independently selected from Cy¹, -(C₁₋₆ alkyl)-Cy¹, OH, CN, amino, halo, C₁₋₆ alkyl, C₁₋₆ haloalkyl, C₁₋₆ haloalkyl, and halosulfanyl;
or R^{c} and R^{d} together with the N atom to which they are attached form a 4-, 5-, 6- or 7-membered heterocycloalkyl group optionally substituted with 1, 2, or 3 substituents independently selected from Cy¹, -(C₁₋₆ alkyl)-Cy¹, OH, CN, amino, halo, C₁₋₆ alkyl, C₁₋₆ haloalkyl, C₁₋₆ haloalkyl, and halosulfanyl;
R^{c'} and R^{d'} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
or R^{c'} and R^{d'} together with the N atom to which they are attached form a 4-, 5-, 6- or 7-membered heterocycloalkyl group optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
R^{c"} and R^{d"} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, halosulfanyl, C₁₋₆ haloalkyl, aryl, arylalkyl, heteroaryl, heteroarylalkyl, cycloalkyl and heterocycloalkyl;
or R^{c"} and R^{d"} together with the N atom to which they are attached form a 4-, 5-, 6- or 7-membered heterocycloalkyl group optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
Rⁱ is H, CN, NO₂, or C₁₋₆ alkyl;
R^{e} and R^{f} are independently selected from H and C₁₋₆ alkyl;
Rⁱ is H, CN, or NO₂;
m is 0 or 1;
n is 0 or 1;
p is 0, 1, 2, 3, 4, 5, or 6;
q is 0, 1,2, 3, 4, 5 or 6;
r is 0 or 1; and
s is 0 or 1.

In some embodiments, when X is N, n is 1, and the moiety formed by A¹, A², U, T, V, and -(Y)ₙ-Z has the formula: 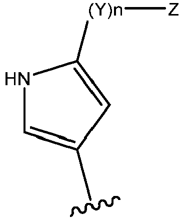 then Y is other than (CR¹¹R¹²)ₚC(O)NR^{c}(CR¹¹R¹²)_{q}.

In some embodiments, when X is N, the 5-membered ring formed by A¹, A², U, T, and V is other than pyrrolyl.

In some embodiments, when X is CH, n is 1, and the moiety formed by A¹, A², U, T, V, and -(Y)ₙ-Z has the formula: 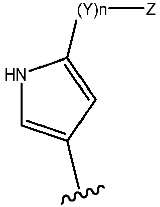 then -(Y)ₙ-Z is other than COOH.

In some embodiments, when X is CH or C-halo, R¹, R², and R³ are each H, n is 1, and the moiety formed by A¹, A², U, T, V, and -(Y)ₙ-Z has the formula: 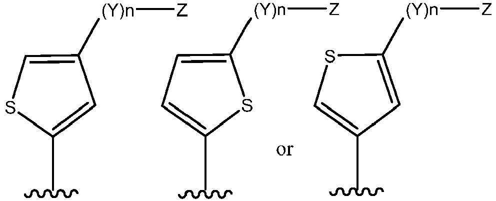 then Y is other than (CR¹¹R¹²)ₚC(O)NR^{c}(CR¹¹R¹²)_{q} or (CR¹¹R¹²)ₚC(O)(CR¹¹R¹²)_{q}.

In some embodiments, when X is CH or C-halo, R¹, R², and R³ are each H, n is 0, and the moiety formed by A¹, A², U, T, V, and -(Y)ₙ-Z has the formula: 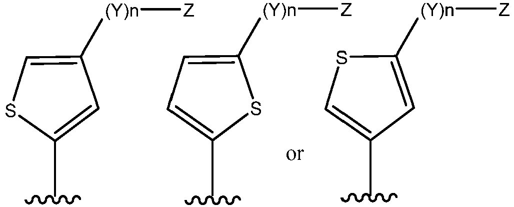 then Z is other than CN, halo, or C₁₋₄ alkyl.

In some embodiments, when X is CH or C-halo, R¹, R², and R³ are each H, n is 1, and the moiety formed by A¹, A², U, T, V, and -(Y)ₙ-Z has the formula: 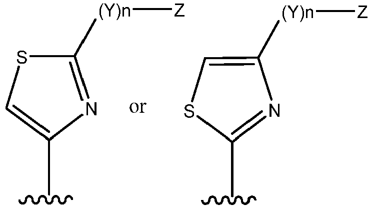 then Y is other than (CR¹¹R¹²)ₚC(O)NR^{c}(CR¹¹R¹²)_{q} or (CR¹¹R¹²)ₚC(O)(CR¹¹R¹²)_{q}.

In some embodiments, when X is CH or C-halo, R¹, R², and R³ are each H, n is 1, and the moiety formed by A¹, A², U, T, V, and -(Y)ₙ-Z has the formula: 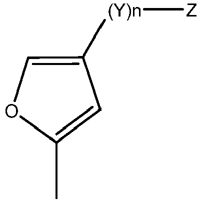 then Y is other than (CR¹¹R¹²)ₚNR^{c}(CR¹¹R¹²)_{q}.

In some embodiments, when X is CH or C-halo and R¹, R², and R³ are each H, then the moiety formed by A¹, A², U, T, V, and -(Y)ₙ-Z has a formula other than:

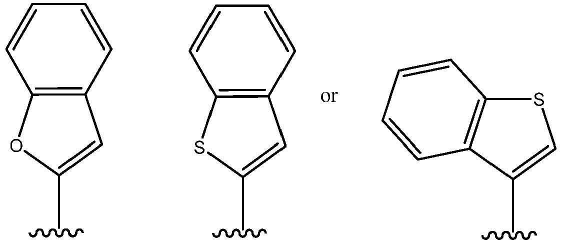

In some embodiments:
Z is H, halo, CN, NO₂, C₁₋₈ alkyl, C₂₋₈ alkenyl, C₂₋₈ alkynyl, C₁₋₈ haloalkyl, aryl, cycloalkyl, heteroaryl, or heterocycloalkyl, wherein said C₁₋₈ alkyl, C₂₋₈ alkenyl, C₂₋₈ alkynyl, C₁₋₈ haloalkyl, aryl, cycloalkyl, heteroaryl, or heterocycloalkyl is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO2, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d};
Q is H, halo, CN, NO₂, C₁₋₈ alkyl, C₂₋₈ alkenyl, C₂₋₈ alkynyl, C₁₋₈ haloalkyl, aryl, cycloalkyl, heteroaryl, or heterocycloalkyl, wherein said C₁₋₈ alkyl, C₂₋₈ alkenyl, C₂₋₈ alkynyl, C₁₋₈ haloalkyl, aryl, cycloalkyl, heteroaryl, or heterocycloalkyl is optionally substituted with 1, 2, 3 or 4 substituents independently selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy², CN, NO₂, OR^{a'}, SR^{a'}, C(O)R^{b'}, C(O)NR^{c'}R^{d'}, C(O)OR^{a'}, OC(O)R^{b'}, OC(O)NR^{c'}R^{d'}, NR^{c'}R^{d'}, NR^{c'}C(O)R^{b'}, NR^{c'}C(O)NR^{c'}R^{d'}, NR^{c'}C(O)OR^{a'}, S(O)R^{b'}, S(O)NR^{c'}R^{d'}, S(O)₂R^{b'}, NR^{c'}S(O)₂R^{b'}, and S(O)₂NR^{c'}R^{d'};
Cy¹ and Cy¹ are independently selected from aryl, heteroaryl, cycloalkyl, and heterocycloalkyl, each optionally substituted by 1, 2, 3, 4 or 5 substituents independently selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, CN, NO₂, OR^{a"}, SR^{a"}, C(O)R^{b"}, C(O)NR^{c"}R^{d"}, C(O)OR^{a"}, OC(O)R^{b"}, OC(O)NR^{c"}R^{d"}, NR^{c"}R^{d"}, NR^{c"}C(O)R^{b"}, NR^{c"}C(O)OR^{a"}, NR^{c"}S(O)R^{b"}, NR^{c"}S(O)₂R^{b"}, S(O)R^{b"}, S(O)NR^{c"}R^{d"}, S(O)₂R^{b"}, and S(O)₂NR^{c"}R^{d"};
R¹, R², R³, and R⁴ are independently selected from H, halo, C₁₋₄ alkyl, C₂₋₄ alkenyl, C₂₋₄ alkynyl, C₁₋₄ haloalkyl, aryl, cycloalkyl, heteroaryl, heterocycloalkyl, CN, NO₂, OR⁷, SR⁷, C(O)R⁸, C(O)NR⁹R¹⁰, C(O)OR⁷OC(O)R⁸, OC(O)NR⁹R¹⁰, NR⁹R¹⁰, NR⁹C(O)R⁸, NR^{c}C(O)OR⁷, S(O)R⁸, S(O)NR⁹R¹⁰, S(O)₂R⁸, NR⁹S(O)₂R⁸, and S(O)₂NR⁹R¹⁰;
R⁵ is H, halo, C₁₋₄ alkyl, C₂₋₄ alkenyl, C₂₋₄ alkynyl, C₁₋₄ haloalkyl, CN, NO₂, OR⁷, SR⁷, C(O)R⁸, C(O)NR⁹R¹⁰, C(O)OR⁷, OC(O)R⁸, OC(O)NR⁹R¹⁰, NR⁹R¹⁰, NR⁹C(O)R⁸, NR⁹C(O)OR⁷, S(O)R⁸, S(O)NR⁹R¹⁰, S(O)₂R⁸, NR⁹S(O)₂R⁸, or S(O)₂NR⁹R¹⁰;
R⁶ is H, C₁₋₄ alkyl, C₂₋₄ alkenyl, C₂₋₄ alkynyl, C₁₋₄ haloalkyl, OR⁷, C(O)R⁸, C(O)NR⁹R¹⁰, C(O)OR⁷, S(O)R⁸, S(O)NR⁹R¹⁰, S(O)₂R⁸, or S(O)₂NR⁹R¹⁰;
R⁷ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
R⁸ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
R⁹ and R¹⁰ are independently selected from H, C₁₋₁₀ alkyl, C₁₋₆ haloalkyl, C₂₋₆ alkenyl, C₂₋₆ alkynyl, C₁₋₆ alkylcarbonyl, arylcarbonyl, C₁₋₆ alkylsulfonyl, arylsulfonyl, aryl, heteroaryl, cycloalkyl, heterocycloalkyl, arylalkyl, heteroarylalkyl, cycloalkylalkyl and heterocycloalkylalkyl;
or R⁹ and R¹⁰ together with the N atom to which they are attached form a 4-, 5-, 6- or 7-membered heterocycloalkyl group;
R¹¹ and R¹² are independently selected from H, halo, OH, CN, C₁₋₄ alkyl, C₁₋₄ haloalkyl, C₂₋₄ alkenyl, C₂₋₄ alkynyl, C₁₋₄ hydroxyalkyl, C₁₋₄ cyanoalkyl, aryl, heteroaryl, cycloalkyl, and heterocycloalkyl;
R^{a}, R^{a'}, and R^{a"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, aryl, arylalkyl, heteroaryl, heteroarylalkyl, cycloalkyl and heterocycloalkyl;
R^{b}, R^{b'} and R^{b"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, aryl, arylalkyl, heteroaryl, heteroarylalkyl, cycloalkyl and heterocycloalkyl;
R^{c} and R^{d} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, aryl, arylalkyl, heteroaryl, heteroarylalkyl, cycloalkyl or heterocycloalkyl;
or R^{c} and R^{d} together with the N atom to which they are attached form a 4-, 5-, 6- or 7-membered heterocycloalkyl group optionally substituted with 1, 2, or 3 substituents independently selected from OH, CN, amino, halo, C₁₋₆ alkyl, C₁₋₆ haloalkyl, C₁₋₆ haloalkyl, aryl, arylalkyl, heteroaryl, heteroarylalkyl, cycloalkyl and heterocycloalkyl;
R^{c'} and R^{d'} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, aryl, arylalkyl, heteroaryl, heteroarylalkyl, cycloalkyl and heterocycloalkyl;
or R^{c'} and R^{d'} together with the N atom to which they are attached form a 4-, 5-, 6- or 7-membered heterocycloalkyl group optionally substituted with 1, 2, or 3 substituents independently selected from OH, CN, amino, halo, C₁₋₆ alkyl, C₁₋₆ haloalkyl, C₁₋₆ haloalkyl, aryl, arylalkyl, heteroaryl, heteroarylalkyl, cycloalkyl and heterocycloalkyl;
R^{c"} and R^{d"} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, aryl, arylalkyl, heteroaryl, heteroarylalkyl, cycloalkyl and heterocycloalkyl;
or R^{c"} and R^{d"} together with the N atom to which they are attached form a 4-, 5-, 6- or 7-membered heterocycloalkyl group optionally substituted with 1, 2, or 3 substituents independently selected from OH, CN, amino, halo, C₁₋₆ alkyl, C₁₋₆ haloalkyl, C₁₋₆ haloalkyl, aryl, arylalkyl, heteroaryl, heteroarylalkyl, cycloalkyl and heterocycloalkyl.

In some embodiments, X is N.

In some embodiments, X is CR⁴.

In some embodiments, A¹ is C.

In some embodiments, A¹ is N.

In some embodiments, A² is C.

In some embodiments, A² is N.

In some embodiments, at least one of A¹, A², U, T, and V is N.

In some embodiments, the 5-membered ring formed by A¹, A², U, T, and V is pyrrolyl, pyrazolyl, imidazolyl, oxazolyl, thiazolyl, or oxadiazolyl.

In some embodiments, the 5-membered ring formed by A¹, A², U, T, and V is selected from: 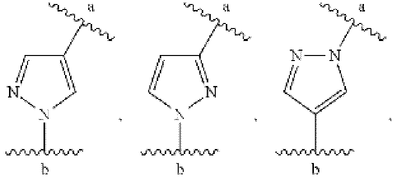 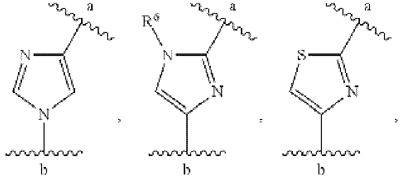 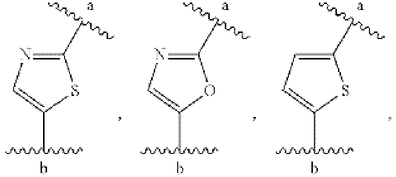 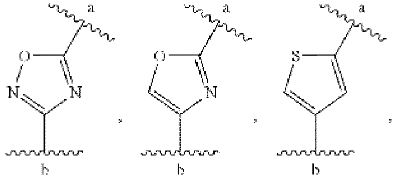 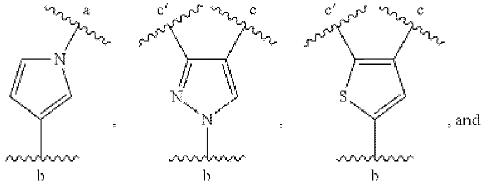 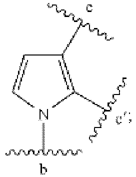 wherein:
a designates the site of attachment of moiety -(Y)ₙ-Z;
b designates the site of attachment to the core moiety: 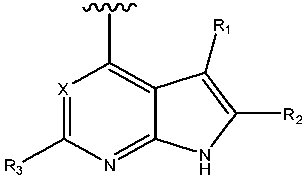
and c and c' designate the two sites of attachment of the fused 4- to 20-membered aryl, cycloalkyl, heteroaryl, or heterocycloalkyl ring.

In some embodiments, the 5-membered ring formed by A¹, A², U, T, and V is: 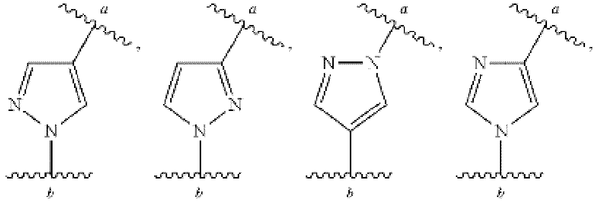 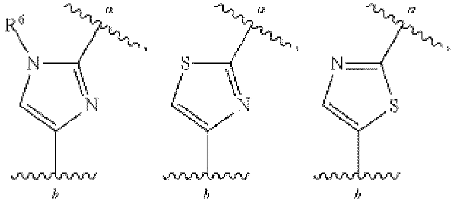 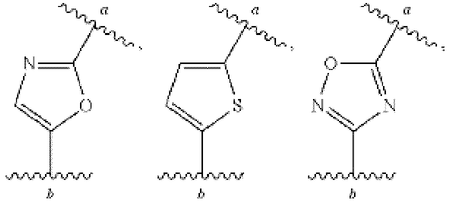 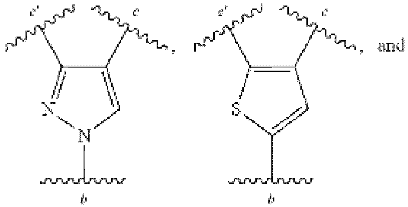 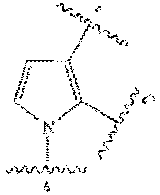 wherein:
a designates the site of attachment of moiety -(Y)ₙ-Z;
b designates the site of attachment to the core moiety. 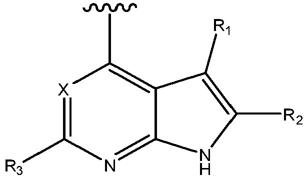
and c and c' designate the two sites of attachment of the fused 4- to 20-membered aryl, cycloalkyl, heteroaryl, or heterocycloalkyl ring.

In some embodiments, the 5-membered ring formed by A¹, A², U, T, and V is selected from: 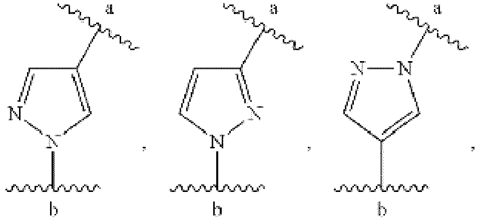 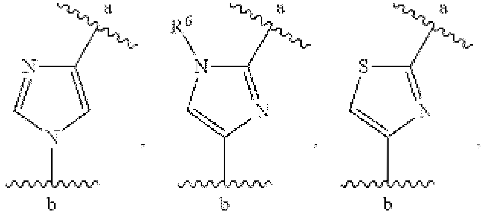 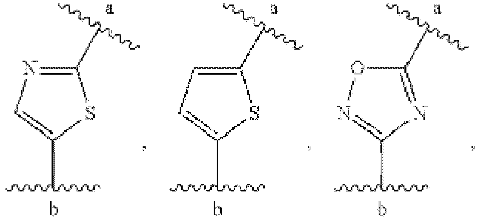 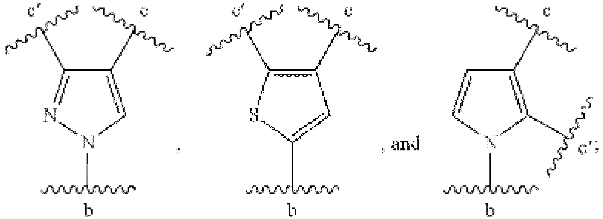 wherein:
a designates the site of attachment of moiety -(Y)ₙ-Z;
b designates the site of attachment to the core moiety: 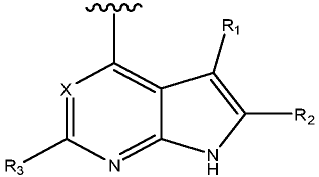
and c and c' designate the two sites of attachment of the fused 4- to 20-membered aryl, cycloalkyl, heteroaryl, or heterocycloalkyl ring.

In some embodiments, the 5-membered ring formed by A¹, A², U, T, and V is selected from: 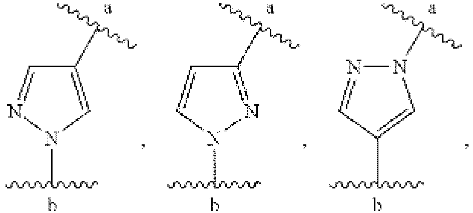 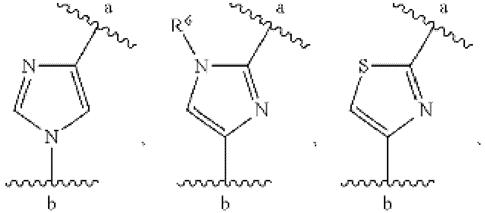 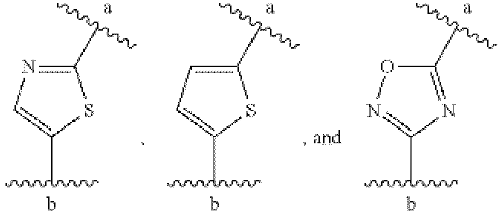 wherein:
a designates the site of attachment of moiety -(Y)ₙ-Z;
b designates the site of attachment to the core moiety:

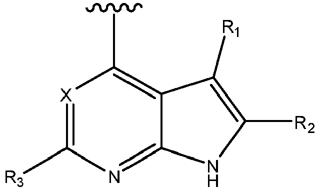

In some embodiments, the 5-membered ring formed by A¹, A², U, T, and V is selected from: 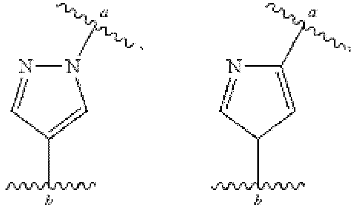 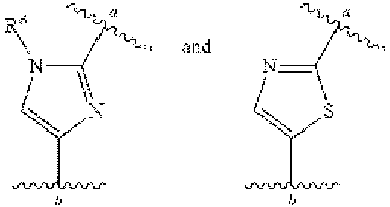 wherein:
a designates the site of attachment of moiety -(Y)ₙ-Z;
b designates the site of attachment to the core moiety:

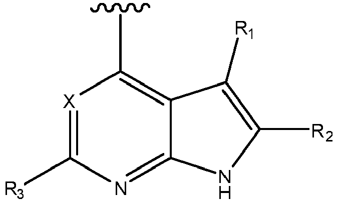

In some embodiments, the 5-membered ring formed by A¹, A², U, T, and V is selected from: 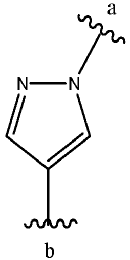 wherein:
a designates the site of attachment of moiety -(Y)ₙ-Z;
b designates the site of attachment to the core moiety:

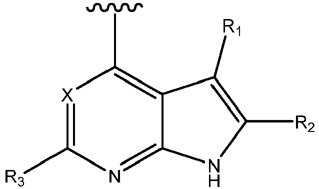

In some embodiments, n is 0.

In some embodiments, n is 1.

In some embodiments, n is 1 and Y is C₁₋₈ alkylene, C₂₋₈ alkenylene, (CR¹¹R¹²)ₚC(O)(CR¹¹R¹²)_{q}, (CR¹¹R¹²)ₚC(O)NR^{c}(CR¹¹R¹²)_{q}, (CR¹¹R¹²)ₚC(O)O(CR¹¹R¹²)_{q}, (CR¹¹R¹²)ₚOC(O)(CR¹¹R¹²)_{q}, wherein said C₁₋₈ alkylene or C₂₋₈ alkenylene, is optionally substituted with 1, 2, or 3 halo, OH, CN, amino, C₁₋₄ alkylamino, or C₂₋₈ dialkylamino.

In some embodiments, n is 1 and Y is C₁₋₈ alkylene, (CR¹¹R¹²)ₚC(O)(CR¹¹R¹²)_{q}, (CR¹¹R¹²)ₚC(O)NR^{c}(CR¹¹R¹²)_{q}, (CR¹¹R¹²)ₚC(O)O(CR¹¹R¹²)_{q}, wherein said C₁₋₈ alkylene is optionally substituted with 1, 2, or 3 halo, OH, CN, amino, C₁₋₄ alkylamino, or C₂₋₈ dialkylamino.

In some embodiments, n is 1 and Y is C₁₋₈ alkylene optionally substituted with 1, 2, or 3 halo, OH, CN, amino, C₁₋₄ alkylamino, or C₂₋₈ dialkylamino.

In some embodiments, n is 1 and Y is ethylene optionally substituted with 1, 2, or 3 halo, OH, CN, amino, C₁₋₄ alkylamino, or C₂₋₈ dialkylamino.

In some embodiments, n is 1 and Y is (CR¹¹R¹²)ₚC(O)(CR¹¹R¹²)_{q} (CR¹¹R¹²)ₚC(O)NR^{c}(CR¹¹R¹²)_{q}, or (CR¹¹R¹²)ₚ C(O)O(CR¹¹R¹²)_{q}.

In some embodiments, Y is C₁₋₈ alkylene, C₂₋₈ alkenylene, C₂₋₈ alkynylene, (CR¹¹R¹²)ₚ-(C₃₋₁₀ cycloalkylene)-(CR¹¹R¹²)_{q}, (CR¹¹R¹²)ₚ-(arylene)-(CR¹¹R¹²)_{q}, (CR¹¹R¹²)ₚ-(C₁₋₁₀ heterocycloalkylene)-(CR¹¹R¹²)_{q}, (CR¹¹R¹²)ₚ-(heteroarylene)-(CR¹¹R¹²)_{q}, (CR¹¹R¹²)ₚO(CR¹¹R¹²)_{q}, or (CR¹¹R¹²)ₚS(CR¹¹R¹²)_{q}, wherein said C₁₋₈ alkylene, C₂₋₈ alkenylene, C₂₋₈ alkynylene, cycloalkylene, arylene, heterocycloalkylene, or heteroarylene, is optionally substituted with 1, 2, or 3 substituents independently selected from -D¹-D²-D³-D⁴.

In some embodiments, Y is C₁₋₈ alkylene, C₂₋₈ alkenylene, C₂₋₈ alkynylene, (CR¹¹R¹²)ₚ-(C₃₋₁₀ cycloalkylene)-(CR¹¹R¹²)_{q}, (CR¹¹R¹²)ₚ-(arylene)-(CR¹¹R¹²)_{q}, (CR¹¹R¹²)ₚ-(C₁₋₁₀ heterocycloalkylene)-(CR¹¹R¹²)_{q}, (CR¹¹R¹²)ₚ-(heteroarylene)-(CR¹¹R¹²)_{q}, (CR¹¹R¹²)ₚO(CR¹¹R¹²)_{q}, or (CR¹¹R¹²)ₚS(CR¹¹R¹²)_{q}, wherein said C₁₋₈ alkylene, C₂₋₈ alkenylene, C₂₋₈ alkynylene, cycloalkylene, arylene, heterocycloalkylene, or heteroarylene, is optionally substituted with 1, 2, or 3 substituents independently selected from D⁴.

In some embodiments, Y is C₁₋₈ alkylene, C₂₋₈ alkenylene, C₂₋₈ alkynylene, or (CR¹¹R¹²)ₚ-(C₃₋₁₀ cycloalkylene)-(CR¹¹R¹²)_{q}, wherein said C₁₋₈ alkylene, C₂₋₈ alkenylene, C₂₋₈ alkynylene, or cycloalkylene, is optionally substituted with 1, 2, or 3 substituents independently selected from -D¹-D²-D³-D⁴.

In some embodiments, Y is C₁₋₈ alkylene, C₂₋₈ alkenylene, C₂₋₈ alkynylene, or (CR¹¹R¹²)ₚ-(C₃₋₁₀ cycloalkylene)-(CR¹¹R¹²)_{q}, wherein said C₁₋₈ alkylene, C₂₋₈ alkenylene, C₂₋₈ alkynylene, or cycloalkylene, is optionally substituted with 1, 2, or 3 substituents independently selected from D⁴.

In some embodiments, Y is C₁₋₈ alkylene, C₂₋₈ alkenylene, or C₂₋₈ alkynylene, each optionally substituted with 1, 2, or 3 substituents independently selected from -D¹-D²-D³-D⁴.

In some embodiments, Y is C₁₋₈ alkylene optionally substituted with 1, 2, or 3 substituents independently selected from -D¹-D²-D³-D⁴.

In some embodiments, Y is C₁₋₈ alkylene optionally substituted with 1, 2, or 3 substituents independently selected from D⁴.

In some embodiments, Y is C₁₋₈ alkylene, C₂₋₈ alkenylene, C₂₋₈ alkynylene, (CR¹¹R¹²)ₚO-(CR¹¹R¹²)_{q}, (CR¹¹R¹²)ₚS(CR¹¹R¹²)_{q}, (CR¹¹R¹²)C(O)(CR¹¹R¹²)_{q}, (CR¹¹R¹²)ₚC(O)NR^{c}(CR¹¹R¹²)_{q}, (CR¹¹R¹²)ₚC(O)O(CR¹¹R¹²)_{q}, (CR¹¹R¹²)ₚOC(O)(CR¹¹R¹²)_{q}, (CR¹¹R¹²)ₚOC(O)NR^{c}(CR¹¹R¹²)_{q}, (CR¹¹R¹²)ₚNR^{c}(CR¹¹R¹²)_{q}, (CR¹¹R¹²)ₚNR^{c}C(O)NR^{d}(CR¹¹R¹²)_{q}, (CR¹¹R¹²)ₚS(O)(CR¹¹R¹²)_{q}, (CR¹¹R¹²)ₚS(O)NR^{c}(CR¹¹R¹²)_{q}, (CR¹¹R¹²)ₚS(O)₂(CR¹¹R¹²)_{q}, or (CR¹¹R¹²)ₚS(O)₂NR^{c}(CR¹¹R¹²)_{q}, wherein said C₁₋₈ alkylene, C₂₋₈ alkenylene, C₂₋₈ alkynylene is optionally substituted with 1, 2, or 3 substituents independently selected from halo, OH, CN, amino, C₁₋₄ alkylamino, and C₂₋₈ dialkylamino.

In some embodiments, Y is C₁₋₈ alkylene, C₂₋₈ alkenylene, C₂₋₈ alkynylene, (CR¹¹R¹²)ₚ-(C₃₋₁₀ cycloalkylene)-(CR¹¹R¹²)_{q}, (CR¹¹R¹²)ₚ-(arylene)-(CR¹¹R¹²)_{q}, (CR¹¹R¹²)ₚ-(C₁₋₁₀ heterocycloalkylene)-(CR¹¹R¹²)_{q}, (CR¹¹R¹²)ₚ-(heteroarylene)-(CR¹¹R¹²)_{q}, (CR¹¹R¹²)ₚO(CR¹¹R¹²)_{q}, (CR¹¹R¹²)ₚS(CR¹¹R¹²)_{q}, (CR¹¹R¹²)ₚC(O)(CR¹¹R¹²)_{q}, (CR¹¹R¹²)ₚC(O)NR^{c}(CR¹¹R¹²)_{q}, (CR¹¹R¹²)ₚC(O)O(CR¹¹R¹²)_{q}, (CR¹¹R¹²)ₚOC(O)(CR¹¹R¹²)_{q}, (CR¹¹R¹²)ₚOC(O)NR^{c}(CR¹¹R¹²)_{q}, (CR¹¹R¹²)ₚNR^{c}(CR¹¹R¹²)_{q} (CR¹¹R¹²)ₚNR^{c}C(O)NR^{d}(CR¹¹R¹²)_{q}, (CR¹¹R¹²)S(O)(CR¹¹R¹²)_{q}, (CR¹¹R¹²)ₚS(O)NR^{c}(CR¹¹R¹²)_{q}, (CR¹¹R¹²)ₚS(O)₂(CR¹¹R¹²)_{q}, or (CR¹¹R¹²)ₚS(O)₂NR^{c}(CR¹¹R¹²)_{q}, wherein said C₁₋₈ alkylene, C₂₋₈ alkenylene, C₂₋₈ alkynylene, cycloalkylene, arylene, heterocycloalkylene, or heteroarylene, is optionally substituted with 1, 2, or 3 substituents independently selected from halo, OH, CN, amino, C₁₋₄ alkylamino, and C₂₋₈ dialkylamino.

In some embodiments, p is 0.

In some embodiments, p is 1.

In some embodiments, p is 2.

In some embodiments, q is 0.

In some embodiments, q is 1.

In some embodiments, q is 2.

In some embodiments, one of p and q is 0 and the other of p and q is 1, 2, or 3.

In some embodiments, Z is H, halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl))R^{b}, and S(O)₂NR^{c}R^{d}.

In some embodiments, Z is aryl, cycloalkyl, heteroaryl, or heterocycloalkyl, each optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is aryl, cycloalkyl, heteroaryl, or heterocycloalkyl, each optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is aryl or heteroaryl, each optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is aryl or heteroaryl, each optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is phenyl or 5- or 6-membered heteroaryl, each optionally substituted with 1, 2, 3, 4, 5, or 6 substituents selected from halo, C₁₋₄alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is phenyl or 5- or 6-membered heteroaryl, each optionally substituted with 1, 2, 3, 4, 5, or 6 substituents selected from halo, C₁₋₄alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is phenyl optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO2, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is phenyl optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO2, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{c}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is cycloalkyl or heterocycloalkyl, each optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is cycloalkyl or heterocycloalkyl, each optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO2, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NR¹)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is cyclopropyl, cyclobutyl, cyclopentyl, cyclohexyl, or cycloheptyl, each optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is C₁₋₈ alkyl, C₂₋₈ alkenyl, or C₂₋₈ alkynyl, each optionally substituted with 1, 2, 3, 4, 5, or 6 substituents selected from halo, C₁₋₄alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.In some embodiments, Z is C₁₋₈ alkyl, C₂₋₈ alkenyl, or C₂₋₈ alkynyl, each optionally substituted with 1, 2, 3, 4, 5, or 6 substituents selected from halo, C₁₋₄ alkyl, C₂₋₄ alkenyl, C2-4 alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is aryl, cycloalkyl, heteroaryl, or heterocycloalkyl, each optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is aryl, cycloalkyl, heteroaryl, or heterocycloalkyl, each optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is aryl or heteroaryl, each optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is aryl or heteroaryl, each optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is phenyl or 5- or 6-membered heteroaryl, each optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is phenyl or 5- or 6-membered heteroaryl, each optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is phenyl optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is phenyl optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO2, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is cycloalkyl or heterocycloalkyl, each optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is cycloalkyl or heterocycloalkyl, each optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is C₁₋₈ alkyl, C₂₋₈ alkenyl, or C₂₋₈ alkynyl, each optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is C₁₋₈ alkyl, C₂₋₈ alkenyl, or C₂₋₈ alkynyl, each optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, C₁₋₄, hydroxyalkyl, C₁₋₄ cyanoalkyl, Cy¹, CN, NO2, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is C₁₋₈ alkyl, C₂₋₈ alkenyl, C₂₋₈ alkynyl, aryl, cycloalkyl, heteroaryl, or heterocycloalkyl, each optionally substituted with 1, 2, 3, 4, 5, or 6 substituents independently selected from halo, C₁₋₄ alkyl, C₁₋₄ haloalkyl, halosulfanyl, C₁₋₄ hydroxyalkyl, C₁₋₄ cyanoalkyl, Cy¹, CN, NO2, OR^{a}, C(O)NR^{c}R^{d}, C(O)OR^{a}, NR^{c}R^{d}, NR^{c}C(O)R^{b}, and S(O)₂R^{b}.

In some embodiments, Z is C₁₋₈ alkyl, C₂₋₈ alkenyl, C₂₋₈ alkynyl, aryl, cycloalkyl, heteroaryl, or heterocycloalkyl, each optionally substituted with 1, 2, 3, 4, 5, or 6 substituents independently selected from halo, C₁₋₄ alkyl, C₁₋₄ haloalkyl, C₁₋₄ hydroxyalkyl, C₁₋₄ cyanoalkyl, Cy¹, CN, NO2, OR^{a}, C(O)NR^{c}R^{d}, C(O)OR^{a}, NR^{c}R^{d}, NR^{c}C(O)R^{b}, and S(O)₂R^{b}.

In some embodiments, Z is C₁₋₈ alkyl, C₂₋₈ alkenyl, C₂₋₈ alkynyl, aryl, cycloalkyl, heteroaryl, or heterocycloalkyl, each optionally substituted with 1, 2, or 3 substituents independently selected from halo, C₁₋₄ alkyl, C₁₋₄ haloalkyl, halosulfanyl, C₁₋₄ hydroxyalkyl, C₁₋₄ cyanoalkyl, Cy¹, CN, NO2, OR^{a}, C(O)NR^{c}R^{d}, C(O)OR^{a}, NR^{c}R^{d}, NR^{c}C(O)R^{b}, and S(O)₂R^{b}.

In some embodiments, Z is C₁₋₈ alkyl, C₂₋₈ alkenyl, C₂₋₈ alkynyl, aryl, cycloalkyl, heteroaryl, or heterocycloalkyl, each optionally substituted with 1, 2, or 3 substituents independently selected from halo, C₁₋₄ alkyl, C₁₋₄ haloalkyl, C₁₋₄ hydroxyalkyl, C₁₋₄ cyanoalkyl, Cy¹, CN, NO2, OR^{a}, C(O)NR^{c}R^{d}, C(O)OR^{a}, NR^{c}R^{d}, NR^{c}C(O)R^{b}, and S(O)₂R^{b}.

In some embodiments, Z is substituted with at least one substituent comprising at least one CN group.

In some embodiments, Z is C₁₋₈ alkyl, C₂₋₈ alkenyl, C₂₋₈ alkynyl, aryl, cycloalkyl, heteroaryl, or heterocycloalkyl, each substituted with at least one CN or C₁₋₄ cyanoalkyl and optionally substituted with 1, 2, 3, 4, or 5 further substituents selected from halo, C₁₋₄ alkyl, C₂₋₈ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO2, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is C₁₋₈ alkyl, C₂₋₈ alkenyl, C₂₋₈ alkynyl, aryl, cycloalkyl, heteroaryl, or heterocycloalkyl, each substituted with at least one CN or C₁₋₄ cyanoalkyl and optionally substituted with 1, 2, 3, 4, or 5 further substituents selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, wherein the -(Y)ₙ-Z moiety is taken together with i) A² to which said moiety is attached, ii) R⁵ or R⁶ of either T or V, and iii) the C or N atom to which said R⁵ or R⁶ of either T or V is attached to form a 4- to 20-membered aryl, cycloalkyl, heteroaryl, or heterocycloalkyl ring fused to the 5-membered ring formed by A¹, A², U, T, and V, wherein said 4- to 20-membered aryl, cycloalkyl, heteroaryl, or heterocycloalkyl ring is optionally substituted by 1, 2, 3, 4, or 5 substituents independently selected from -(W)ₘ-Q.

In some embodiments, wherein the -(Y)ₙ-Z moiety is taken together with i) A² to which said moiety is attached, ii) R⁵ or R⁶ of either T or V, and iii) the C or N atom to which said R⁵ or R⁶ of either T or V is attached to form a 4- to 8-membered aryl, cycloalkyl, heteroaryl, or heterocycloalkyl ring fused to the 5-membered ring formed by A¹, A², U, T, and V, wherein said 4- to 8-membered aryl, cycloalkyl, heteroaryl, or heterocycloalkyl ring is optionally substituted by 1, 2, 3, 4, or 5 substituents independently selected from -(W)ₘ-Q.

In some embodiments, the -(Y)ₙ-Z moiety is taken together with i) A² to which said moiety is attached, ii) R⁵ or R⁶ of either T or V, and iii) the C or N atom to which said R⁵ or R⁶ of either T or V is attached to form a 6-membered aryl, cycloalkyl, heteroaryl, or heterocycloalkyl ring fused to the 5-membered ring formed by A¹, A², U, T, and V, wherein said 6-membered aryl, cycloalkyl, heteroaryl, or heterocycloalkyl ring is optionally substituted by 1, 2, or 3 substituents independently selected from halo, CN, NO₂, C₁₋₈ alkyl, C₂₋₈ alkenyl, C₂₋₈ alkynyl, C₁₋₈ haloalkyl, aryl, cycloalkyl, heteroaryl, or heterocycloalkyl wherein said C₁₋₈ alkyl, C₂₋₈ alkenyl, C₂₋₈ alkynyl, C₁₋₈ haloalkyl, aryl, cycloalkyl, heteroaryl, or heterocycloalkyl is optionally substituted by 1, 2 or 3 CN.

In some embodiments, Cy¹ and Cy² are independently selected from aryl, heteroaryl, cycloalkyl, and heterocycloalkyl, each optionally substituted by 1, 2, 3, 4 or 5 substituents independently selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, CN, NO2, OR^{a"}, SR^{a"}, C(O)R^{b"}, C(O)NR^{c"}R^{d"}, C(O)OR^{a"}, OC(O)R^{b"}, OC(O)NR^{c"}R^{d"}, NR^{c"}R^{d"}, NR^{c"}C(O)R^{b"}, NR^{c"}C(O)OR^{a"}, S(O)R^{b"}, S(O)NR^{c"}R^{d"}, S(O)₂R^{b"}, and S(O)₂NR^{c"}R^{d"}.

In some embodiments, Cy¹ and Cy² are independently selected from aryl, heteroaryl, cycloalkyl, and heterocycloalkyl, each optionally substituted by 1, 2, 3, 4 or 5 substituents independently selected from halo, C₁₋₄ alkyl, C₂₋₄ alkenyl, C₂₋₄ alkynyl, C₁₋₄ haloalkyl, CN, NO2, OR^{a"}, SR^{a"}, C(O)R^{b"}, C(O)NR^{c"}R^{d"}, C(O)OR^{a"}, OC(O)R^{b"}, OC(O)NR^{c"}R^{d"}, NR^{c"}R^{d"}, NR^{c"}C(O)R^{b"}, NR^{c"}C(O)OR^{a"}S(O)R^{b"}, S(O)NR^{c"}R^{d"}, S(O)₂R^{b"}, and S(O)₂NR^{c"}R^{d"}.

In some embodiments, Cy¹ and Cy² are independently selected from cycloalkyl and heterocycloalkyl, each optionally substituted by 1, 2, 3, 4 or 5 substituents independently selected from halo, C₁₋₄ alkyl, C₂₋₄ alkenyl, C₂₋₄ alkynyl, C₁₋₄ haloalkyl, CN, NO2, OR^{a"}, SR^{a"}, C(O)R^{b"}, C(O)NR^{c"}R^{d"}, C(O)OR^{a"}, OC(O)R^{b"}OC(O)NR^{c"}R^{d"}, NR^{C"}R^{d"}, NR^{c"}C(O)R^{b", NR c"}C(O)OR^{a"}, S(O)R^{b"}, S(O)NR^{c"}R^{d"}, S(O)₂R^{b"}, and S(O)₂NR^{c"}R^{d"}.

In some embodiments, Cy¹ and Cy² are independently selected from cycloalkyl optionally substituted by 1, 2, 3, 4 or 5 substituents independently selected from halo, C₁₋₄ alkyl, C2-4 alkenyl, C2-4 alkynyl, C₁₋₄ haloalkyl, CN, NO2, OR^{a"}, SR^{a"}, C(O)R^{b"}, C(O)NR^{c"}R^{d"}, C(O)OR^{a"}, OC(O)R^{b"}, OC(O)NR^{c"}R^{d"}, NR^{c"}R^{d"}, NR^{c"}C(O)R^{b"}, NR^{c"}C(O)OR^{a"}S(O)R^{b"}, S(O)NR^{c"}R^{d"}, S(O)₂R^{b"}, and S(O)₂NR^{c"}R^{d"}.

In some embodiments, R¹, R², R³, and R⁴ are independently selected from H, halo, C₁₋₄ alkyl, C₂₋₄ alkenyl, C₂₋₄ alkynyl, C₁₋₄ haloalkyl, aryl, cycloalkyl, heteroaryl, heterocycloalkyl, CN, NO₂, OR⁷, SR⁷, C(O)R⁸, C(O)NR⁹R¹⁰, C(O)OR⁷OC(O)R⁸, OC(O)NR⁹R¹⁰, NR⁹R¹⁰, NR⁹C(O)R⁸, NR^{c}C(O)OR⁷, S(O)R⁸, S(O)NR⁹R¹⁰, S(O)₂R⁸, NR⁹S(O)₂R⁸, and S(O)₂NR⁹R¹⁰.

In some embodiments, R¹, R², R³, and R⁴ are independently selected from H, halo, and C₁₋₄ alkyl.

In some embodiments, R¹, R², R³, and R⁴ are each H.

In some embodiments, R¹ is H, halo, or C₁₋₄ alkyl.

In some embodiments, R⁵ is H, halo, C₁₋₄ alkyl, C₂₋₄ alkenyl, C₂₋₄ alkynyl, C₁₋₄ haloalkyl, CN, NO₂, OR⁷, SR⁷, C(O)R⁸, C(O)NR⁹R¹⁰, C(O)OR⁷, OC(O)R⁸, OC(O)NR⁹R¹⁰, NR⁹R¹⁰, NR⁹C(O)R⁸, NR⁹C(O)OR⁷, S(O)R⁸, S(O)NR⁹R¹⁰, S(O)₂R⁸, NR⁹S(O)₂R⁸, or S(O)₂NR⁹R¹⁰.

In some embodiments, R⁵ is H, halo, C₁₋₄ alkyl, C₁₋₄ haloalkyl, halosulfanyl, CN, or NR⁹R¹⁰.

In some embodiments, R⁵ is H, halo, C₁₋₄ alkyl, C₁₋₄ haloalkyl, CN, or NR⁹R¹⁰.

In some embodiments, R⁵ is H.

In some embodiments, R⁶ is H or C₁₋₄ alkyl.

In some embodiments, R⁶ is H.

In some embodiments, R¹¹ and R¹² are independently selected from H, halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d}, wherein said Ci-g alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl))R^{b}, and S(O)₂NR^{c}R^{d}.

In some embodiments, R¹¹ and R¹² are independently selected from H, halo, OH, CN, (C₁₋₄)alkyl, (C₁₋₄)haloalkyl, halosulfanyl, SCN, (C₂₋₄)alkenyl, (C₂₋₄)alkynyl, (C₁₋₄)hydroxyalkyl, (C₁₋₄)cyanoalkyl, aryl, heteroaryl, cycloalkyl, and heterocycloalkyl.

In some embodiments, R¹¹ and R¹² are independently selected from H, halo, OH, CN, (C₁₋₄)alkyl, (C₁₋₄)haloalkyl, (C₂₋₄)alkenyl, (C₂₋₄)alkynyl, (C₁₋₄)hydroxyalkyl, (C₁₋₄)cyanoalkyl, aryl, heteroaryl, cycloalkyl, and heterocycloalkyl.

In a preferred embodiment, the JAK-2 inhibitor is ruxolitinib (available from Incyte Corp. and Novartis AG). In a preferred embodiment, the JAK-2 inhibitor is ruxolitinib phosphate (available from Incyte Corp. and Novartis AG). In a preferred embodiment, the JAK-2 inhibitor is (*R*)-3-(4-(7*H*-pyrrolo[2,3-*d*]pyrimidin-4-yl)-1*H-*pyrazol-1-yl)-3-cyclopentylpropanenitrile. In a preferred embodiment, the JAK-2 inhibitor is the phosphate salt of (*R*)-3-(4-(7*H*-pyrrolo[2,3-*d*]pyrimidin-4-yl)-1*H*-pyrazol-1-yl)-3-cyclopentylpropanenitrile. In a preferred embodiment, the JAK-2 inhibitor is (3*R*)-3-cyclopentyl-3-[4-(7*H*-pyrrolo[2,3-d]pyrimidin-4-yl)-1*H*-pyrazol-1-yl]propanenitrile. In a preferred embodiment, the JAK-2 inhibitor is a compound of Formula (XXX): 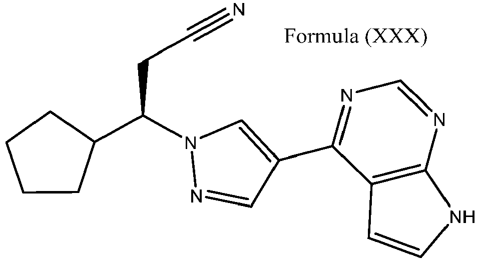 or a pharmaceutically acceptable salt, solvate, or hydrate thereof. The preparation of this compound is described in U.S. Patent Nos. 8,604,043, 7,834,022, 8,486,902, 8,530,485, 7,598,257, 8,541,425, and 8,410,265 and U.S. Patent Application Publication Nos. 2010/0298355 A1, 2008/0312258 A1, 2011/0082159 A1, 2011/0086810 A1, 2013/0345157 A1, 2014/0018374 A1, 2014/0005210 A1, 2011/0223210 A1, 2011/0224157 A1, 2007/0135461 A1, 2010/0022522 A1, 2013/0253193 A1, 2013/0253191 A1, 2013/0253190 A1, 2010/0190981 A1, 2013/0338134 A1, 2008/0312259 A1, 2014/0094477 A1, and 2014/0094476 A1. In an embodiment, the JAK-2 inhibitor is a compound selected from the structures disclosed in U.S. Patent Nos. 8,604,043, 7,834,022, 8,486,902, 8,530,485, 7,598,257, 8,541,425, and 8,410,265 and U.S. Patent Application Publication Nos. 2010/0298355 A1, 2008/0312258 A1, 2011/0082159 A1, 2011/0086810 A1, 2013/0345157 A1, 2014/0018374 A1, 2014/0005210 A1, 2011/0223210 A1, 2011/0224157 A1, 2007/0135461 A1, 2010/0022522 A1, 2013/0253193 A1, 2013/0253191 A1, 2013/0253190 A1, 2010/0190981 A1, 2013/0338134 A1, 2008/0312259 A1, 2014/0094477 A1, and 2014/0094476 A1.

Ruxolitinib may be prepared according to the procedures given in the references above, or by the procedure of Example 67 of U.S. Patent No. 7598257. Briefly, the preparation is as follows:
Step 1. (2E)- and (2Z)-3-Cyclopentylacrylonitrile. To a solution of 1.0 M potassium tert-butoxide in THF (235 mL) at 0° C. was added dropwise a solution of diethyl cyanomethylphosphonate (39.9 mL, 0.246 mol) in TBF (300 mL). The cold bath was removed and the reaction was warmed to room temperature followed by recooling to 0° C., at which time a solution of cyclopentanecarbaldehyde (22.0 g, 0.224 mol) in THF (60 mL) was added dropwise. The bath was removed and the reaction warmed to ambient temperature and stirred for 64 hours. The mixture was partitioned between diethyl ether and water, the aqueous was extracted with three portions of ether, followed by two portions of ethyl acetate. The combined extracts were washed with brine, then dried over sodium sulfate, filtered and concentrated in vacuo to afford a mixture containing 24.4 g of olefin isomers which was used without further purification (89%). ¹H NMR (400 MHz, CDCl3): δ 6.69 (dd, 1H, trans olefin), 6.37 (t, 1H, cis olefin), 5.29 (dd, 1H, trans olefin), 5.20 (d, 1H, cis olefin), 3.07-2.95 (m, 1H, cis product), 2.64-2.52 (m, 1H, trans product), 1.98-1.26 (m, 16H).
Step 2. (3R)- and (3*S*)-3-Cyclopentyl-3-[4-(7-[2-(trimethylsilyl)ethoxy]methyl-7*H*-pyrrolo[2,3-*d*]-pyrimidin-4-yl)-1*H*-pyrazol-1-yl]propanenitrile. To a solution of 4-(1*H*-pyrazol-4-yl)-7-[2-(trimethylsilyl)ethoxy]methyl-7*H*-pyrrolo[2,3-d]-pyrimidine (15.0 g, 0.0476 mol) in ACN (300 mL) was added 3-cyclopentylacrylonitrile (15 g, 0.12 mol) (as a mixture of cis and trans isomers), followed by DBU (15 mL, 0.10 mol). The resulting mixture was stirred at room temperature overnight. The ACN was evaporated. The mixture was diluted with ethyl acetate, and the solution was washed with 1.0 N HCl. The aqueous layer was back-extracted with three portions of ethyl acetate. The combined organic extracts were washed with brine, dried over sodium sulfate, filtered and concentrated. The crude product was purified by silica gel chromatography (gradient of ethyl acetate/hexanes) to yield a viscous clear syrup, which was dissolved in ethanol and evaporated several times to remove ethyl acetate, to afford 19.4 g of racemic adduct (93%). The enantiomers were separated by preparative-HPLC, (OD-H column, 15% ethanol/hexanes) and used separately in the next step to generate their corresponding final product. The final products (see Step 3) stemming from each of the separated enantiomers were found to be active JAK inhibitors; however, the final product stemming from the second peak to elute from the preparative-HPLC was more active than its enantiomer. The products may be isolated by preparative HPLC or other means known to those of skill in the art for use in Step 3 below. ¹H NMR (300 MHz, CDCl3): δ 8.85 (s, 1H), 8.32 (s, 2H), 7.39 (d, 1H), 6.80 (d, 1H), 5.68 (s, 2H), 4.26 (dt, 1H), 3.54 (t, 2H), 3.14 (dd, 1H), 2.95 (dd, 1H), 2.67-2.50 (m, 1H), 2.03-1.88 (m, 1H), 1.80-1.15 (m, 7H), 0.92 (t, 2H), -0.06 (s, 9H); MS(ES): 437 (M+1).
Step 3. To a solution of 3-cyclopentyl-3-[4-(7-[2-(trimethylsilyl)ethoxy]methyl-7H-pyrrolo[2,3-d]-pyrimidin-4-yl)-1*H*-pyrazol-1-yl]propanenitrile (6.5 g, 0.015 mol, R or S enantiomer as isolated above) in DCM (40 mL) was added TFA (16 mL) and this was stirred for 6 hours. The solvent and TFA were removed in vacuo. The residue was dissolved in DCM and concentrated using a rotary evaporator two further times to remove as much as possible of the TFA. Following this, the residue was stirred with ethylenediamine (4 mL, 0.06 mol) in methanol (30 mL) overnight. The solvent was removed in vacuo, water was added and the product was extracted into three portions of ethyl acetate. The combined extracts were washed with brine, dried over sodium sulfate, decanted and concentrated to afford the crude product which was purified by flash column chromatography (eluting with a gradient of methanol/DCM). The resulting mixture was further purified by preparative-HPLC/MS (C18 eluting with a gradient of ACN/H2O containing 0.15% NH4OH) to afford product (2.68 g, 58%). ¹H NMR (400 MHz, D6-dmso): δ 12.11 (br s, 1H), 8.80 (s, 1H), 8.67 (s, 1H), 8.37 (s, 1H), 7.60 (d, 1H), 6.98 (d, 1H), 4.53 (dt, 1H), 3.27 (dd, 1H), 3.19 (dd, 1H), 2.48-2.36 (m, 1H), 1.86-1.76 (m, 1H), 1.68-1.13 (m, 7H); MS(ES): 307 (M+1).

Ruxolitinib prepared according to the steps above, or any other procedure, may be used as its free base for the compositions and methods described heren. Ruxolitinib may also be used in a salt form. For example, a crystalline phosphoric acid salt of (*R*)-3-(4-(7*H*-pyrrolo[2,3-*d*]pyrimidin-4-yl)-1*H*-pyrazol-1-yl)-3-cyclopentylpropanenitrile may be prepared from the free base as follows according to the procedure given in Example 2 of U.S. Patent No. 8,722,693. To a test tube was added (*R*)-3-(4-(7*H*-pyrrolo[2,3-*d*]pyrimidin-4-yl)-1*H*-pyrazol-1-yl)-3-cyclopentylpropanenitrile (153.5 mg) and phosphoric acid (56.6 mg) followed by isopropyl alcohol (IPA) (5.75 mL). The resulting mixture was heated to clear, cooled to room temperature, and then stirred for another 2 hours. The precipitate was collected by filtration and the cake was washed with 0.6 mL of cold IPA. The cake was dried under vacuum to constant weight to provide the final salt product (171.7 mg). The phosphroic acid salt is a 1:1 salt by ¹H NMR and crystallinity is confirmed by X-ray powder diffraction (XRPD). Differential scanning calorimetry (DSC) of the produce yields a sharp melting peak at about 198.7° C.

In an embodiment, the JAK-2 inhibitor is a compound of Formula (XXXI): 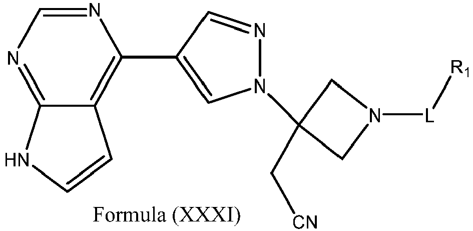 or a pharmaceutically acceptable salt, solvate, or hydrate thereof, wherein:
L is SO₂ or CO;
R¹ is C₁₋₆ alkyl, C₃₋₇ cycloalkyl, phenyl, 5- or 6-membered heteroaryl, indolyl, NR²R³, or
OR⁴, wherein said alkyl, cycloalkyl, phenyl, or heteroaryl is optionally substituted with 1, 2, or 3 substituents independently selected from F, CN, and C₁₋₄ alkyl;
R² and R³ are independently selected from H, C₁₋₄ alkyl, and phenyl; and
R⁴ is C₁₋₆ alkyl, phenyl, or benzyl.

In some embodiments, when L is SO₂, then R¹ is other than OR⁴.

In some embodiments, when L is SO₂, then R¹ is C₁₋₆ alkyl, C₃₋₇ cycloalkyl, phenyl, 5- or 6-membered heteroaryl, or NR²R³, wherein said alkyl, cycloalkyl, phenyl, or heteroaryl is optionally substituted with 1, 2, or 3 substituents independently selected from F and C₁₋₄ alkyl.

In some embodiments, when L is CO, then R¹ is C₃₋₇ cycloalkyl, phenyl, 5- or 6-membered heteroaryl, indolyl, NR²R³, or OR⁴, wherein said cycloalkyl, phenyl, or heteroaryl is optionally substituted with 1, 2, or 3 substituents independently selected from CN and C₁₋₄ alkyl.

In some embodiments, L is SO₂.

In some embodiments, L is CO.

In some embodiments, R¹ is methyl, ethyl, n-propyl, isopropyl, n-butyl, t-butyl, 2-methylprop-1-yl, 1-methylprop-1-yl, each optionally substituted with 1, 2, or 3 F.

In some embodiments, R¹ is C₁₋₄ alkyl.

In some embodiments, R¹ is ethyl.

In some embodiments, R¹ is C₃₋₇ cycloalkyl optionally substituted by C₁₋₄ alkyl.

In some embodiments, R¹ is phenyl optionally substituted with F, methyl, or CN.

In some embodiments, R¹ is 5-membered heteroaryl selected from thienyl, pyrazolyl, pyrrolyl, 1,2,4-oxadiazolyl, and isoxazolyl, each optionally substituted with C₁₋₄ alkyl.

In some embodiments, R¹ is pyridinyl.

In some embodiments, R¹ is NR²R³ or OR⁴.

In some embodiments, L is SO₂ and R¹ is C₁₋₆ alkyl.

In an embodiment, the JAK-2 inhibitor is baricitinib (available from Incyte Corp. and Eli Lilly & Co.). In an embodiment, the JAK-2 inhibitor is 2-(3-(4-(7*H-*pyrrolo[2,3-d]pyrimidin-4-yl)-1*H*-pyrazol-1-yl)-1 -(ethylsulfonyl)azetidin-3-yl)acetonitrile. In an embodiment, the JAK-2 inhibitor is a compound of Formula (XXXII): 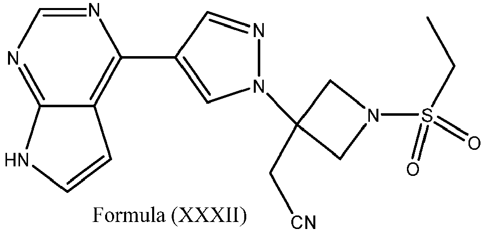 or a pharmaceutically acceptable salt, solvate, or hydrate thereof. The preparation of this compound is described in U.S. Patent Nos. 8,158,616 and 8,420,629, U.S. Patent Application Publication Nos. 2009/0233903 A1; 2013/0225556 A1; and, 2012/0077798 A1, and International Patent Application Publication No. WO 2014/0028756. In an embodiment, the JAK-2 inhibitor is a compound described in U.S. Patent Nos. 8,158,616 and 8,420,629, U.S. Patent Application Publication Nos. 2009/0233903 A1; 2013/0225556 A1; and, 2012/0077798 A1, and International Patent Application Publication No. WO 2014/0028756.

In an embodiment, the JAK-2 inhibitor is a compound of Formula (XXXIII): 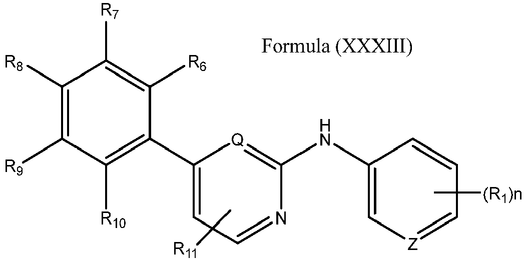 or a pharmaceutically acceptable salt, solvate, or hydratethereof, wherein:
Q and Z are independently selected from N and CR¹; n is 1, 2 or 3;
R¹ is independently selected from hydrogen, halogen, R², OR², OH, R⁴, OR⁴, CN, CF₃, (CH₂)ₙN(R²)₂, NO₂, R²R⁴, SO₂R⁴, NR²SO₂R³, COR⁴, NR²COR³, CO₂H, CO₂R², NR²COR⁴, R²CN, R²CN, R²OH, R²OR³ and OR⁵R⁴; or two R¹ substituents together with the carbons which they are attached to form an unsaturated 5 or 6 membered heterocyclyl;
R² is substituted or unsubstituted C₁₋₄alkyl or substituted or unsubstituted C₁₋₄ alkylene where up to 2 carbon atoms can be optionally replaced with CO, NR^{Y}, C0NR^{Y}, S, SO₂ or O;
R³ is R², C₂₋₄ alkenyl or substituted or unsubstituted aryl;
R⁴ is NH₂, NHR², N(R')₂, substituted or unsubstituted morpholino, substituted or unsubstituted thiomorpholino, substituted or unsubstituted thiomorpholino-1-oxide, substituted or unsubstituted thiomorpholino-1, 1-dioxide, substituted or unsubstituted piperazinyl, substituted or unsubstituted piperidinyl, substituted or unsubstituted pyridinyl, substituted or unsubstituted pyrrolidinyl, substituted or unsubstituted pyrrolyl, substituted or unsubstituted oxazolyl, substituted or unsubstituted imidazolyl, substituted or unsubstituted tetrahydrofuranyl and substituted or unsubstituted tetrahydropyranyl;
R⁵ is substituted or unsubstituted C₁₋₄alkylene;
R⁶-R¹⁰ are independently selected from H, R^{X}CN, halogen, substituted or unsubstituted C_{M}alkyl, OR¹, CO₂R¹, N(R')₂, NO₂, CON(R')_{2J} SO₂N(R^{Y})₂, N(SO₂R^₂, substituted or unsubstituted piperazinyl, N(R^{Y})SO₂R² and CF_{3;} R^{x} is absent or substituted or unsubstituted C₁₋₆alkylene wherein up to 2 carbon atoms can be optionally replaced with CO, NSO₂R¹, NR^{Y}, C0NR^{Y}, S, SO₂ or O; R^{γ} is H or substituted or unsubstituted C₁₋₄ alkyl; and
R¹¹ is selected from H, halogen, substituted or unsubstituted C₁₋₄ alkyl, OR², CO₂R², CN, CON(R')₂ and CF₃, or an enantiomer thereof.

In a preferred embodiment, the JAK-2 inhibitor is momelotinib (Gilead Sciences). Momelotinib is also known as CYT-387. In a preferred embodiment, the JAK-2 inhibitor is *N*-(cyanomethyl)-4-(2-((4-morpholinophenyl)amino)pyrimidin-4-yl)benzamide. In a preferred embodiment, the JAK-2 inhibitor is a compound of Formula (XXXIV): 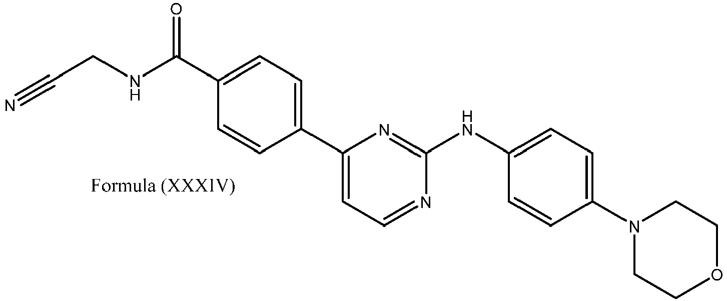 or a pharmaceutically acceptable salt, solvate, or hydrate thereof. The preparation of this compound is described in U.S. Patent No. 8,486,941 and U.S. Patent Application Publication Nos. 2010/0197671 A1; 2014/0005180 A1; 2014/0011803 A1; and, 2014/0073643 A1. In an embodiment, the JAK-2 inhibitor is a compound described in U.S. Patent No. 8,486,941 and U.S. Patent Application Publication Nos. 2010/0197671 A1; 2014/0005180 A1; 2014/0011803 A1; and, 2014/0073643 A1.

In an embodiment, the JAK-2 inhibitor is a compound of Formula (XXXV): 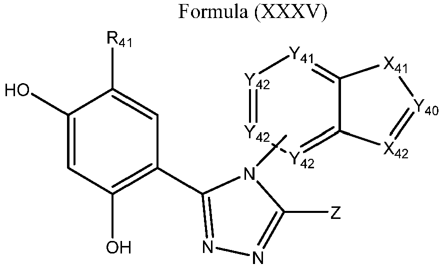 or a tautomer thereof, or a clathrate thereof, or a pharmaceutically acceptable salt, solvate, or hydrate thereof, wherein:
X₄₁ is O, S, or NR₄₂;
X₄₂ is CR₄₄ or N;
Y₄₀ is N or CR₄₃;
Y₄₁ is N or CR₄₅;
Y₄₂, for each occurrence, is independently N, C or CR₄₆;
Z is OH SH, or NHR₇;
R₄₁ is -H, -OH, -SH, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, halo, cyano, nitro, guanadino, a haloalkyl, a heteroalkyl, an alkoxy or cycloalkoxy, a haloalkoxy, - NR₁₀R₁₁, -OR₇, -C(O)R₇, -C(O)OR₇, -C(S)R₇, -C(O)SR₇, -C(S)SR₇, - C(S)OR₇, -C(S)NR₁₀R₁₁, -C(NR₈)OR₇, -C(NR₈)R₇, -C(NR₈)NR₁₀R₁₁, - C(NR₈)SR₇, -OC(O)R₇, -OC(O)OR₇, -OC(S)OR₇, -OC(₈)OR₇, -SC(O)R₇, - SC(O)OR₇, -SC(NR₈)OR₇, -OC(S)R₇, -SC(S)R₇, -SC(S)OR₇, - OC(O)NR₁₀R₁₁, -OC(S)NR₁₀R₁₁, -OC(NR₈)NR₁₀R₁₁, -SC(O)NR₁₀R₁₁, - SC(NR₈)NR₁₀R₁₁, -SC(S)NR₁₀R₁₁, -OC(NR₈)R₇, -SC(NR₈)R₇, -C(O)NR₁₀R₁₁, -NR₈C(O)R₇, -NR₇C(S)R₇, -NR₇C(S)OR₇, -NR₇C(NR₈)R₇, -NR₇C(O)OR₇, -NR₇C(NR₈)OR₇, -NR₇C(O)NR₁₀R₁₁, -NR₇C(S)NR₁₀R₁₁, - NR₇C(NR₈)NR₁₀R₁₁, -SR₇, -S(O)ₚR₇, -OS(O)ₚR₇, -OS(O)ₚOR₇, - OS(O)ₚNR₁₀R₁₁, -S(O)ₚOR₇, -NR₈S(O)ₚR₇, -NR₇S(O)ₚNR₁₀R₁₁, - NR₇S(O)ₚOR₇, -S(O)ₚNR₁₀R₁₁, -SS(O)ₚR₇, -SS(O)ₚOR₇, -SS(O)ₚNR₁₀R₁₁, - OP(O)(OR₇)₂, or -SP(O)(OR₇)₂;
R₄₂ is -H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, hydroxyalkyl, alkoxyalkyl, a haloalkyl, a heteroalkyl, - C(O)R₇, -(CH₂)ₘC(O)OR₇, -C(O)OR₇, -OC(O)R₇, -C(O)NR₁₀R₁₁, -S(O)ₚR₇, -S(O)ₚOR₇, or -S(O)ₚNR₁₀R₁₁;
R₄₃ and R₄₄ are, independently, -H, -OH, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, hydroxyalkyl, alkoxyalkyl, halo, cyano, nitro, guanadino, a haloalkyl, a heteroalkyl, -C(O)R₇, - C(O)OR₇, -OC(O)R₇, -C(O)NR₁₀R₁₁, -NR₈C(O)R₇, -SR₇, -S(O)ₚR₇, - OS(O)ₚR₇, -S(O)ₚOR₇, -NR₈S(O)ₚR₇, -S(O)ₚNR₁₀R₁₁, or R₄₃ and R₄₄ taken together with the carbon atoms to which they are attached form an optionally substituted cycloalkenyl, an optionally substituted aryl, an optionally substituted heterocyclyl, or an optionally substituted heteroaryl;
R45 is -H, -OH, -SH, -NR₇H, -OR₂₆, -SR₂₆, -NHR₂₆, -O(CH₂)ₘOH, - O(CH₂)ₘSH, -O(CH₂)ₙNR₇H, -S(CH₂)ₘOH, -S(CH₂)ₘSH, -S(CH₂)ₘNR₇H, - OC(O)NR₁₀R₁₁, -SC(O)NR₁₀R₁₁, -NR₇C(O)NR₁₀R₁₁, -OC(O)R₇, -SC(O)R₇, - NR₇C(O)R₇, -OC(O)OR₇, -SC(O)OR₇, -NR₇C(O)OR₇, -OCH₂C(O)R₇, - SCH₂C(O)R₇, -NR₇CH₂C(O)R₇, -OCH₂C(O)OR₇, -SCR₂C(O)OR₇, - NR₇CH₂C(O)OR₇, -OCH₂C(O)NR₁₀R₁₁, -SCH₂C(O)NR₁₀R₁₁, - NR₇CH₂C(O)NR₁₀R₁₁, -OS(O)ₚR₇, -SS(O)ₚR₇, -NR₇S(O)ₚR₇, -OS(O)ₚNR₁₀R₁₁, -SS(O)ₚNR₁₀R₁₁, -NR₇S(O)ₚNR₁₀R₁₁, -OS(O)ₚOR₇, -SS(O)ₚOR₇, - NR₇S(O)ₚOR₇, -OC(S)R₇, -SC(S)R₇, -NR₇C(S)R₇, -OC(S)OR₇, -SC(S)OR₇, -NR₇C(S)OR₇, -OC(S)NR₁₀R₁₁, -SC(S)NR₁₀R₁₁, -NR₇C(S)NR₁₀R₁₁, - OC(NR₈)R₇, -SC(NR₈)R₇, -NR₇C(N₈)R₇, -OC(NR₈)OR₇, -SC(NR₈)OR₇, - NR₇C(NR₈)OR₇, -OC(NR₈)NR₁₀R₁₁, -SC(NR₈)NR₁₀R₁₁, or -NR₇C(N₈)NR₁₀R₁₁;
R₄₆, for each occurrence, is independently, selected from the group consisting of H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, halo, cyano, nitro, guanadino, a haloalkyl, a heteroalkyl, - NR₁₀R₁₁, -OR₇, -C(O)R₇, -C(O)OR₇, -OC(O)R₇, -C(O)NR₁₀R₁₁, - NR₈C(O)R₇, -SR₇, -S(O)ₚR₇, -OS(O)ₚR₇, -S(O)ₚOR₇, -NR₈S(O)ₚR₇, or - S(O)ₚNR₁₀R₁₁;
R₇ and R₈, for each occurrence, are, independently, -H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
R₁₀ and R₁₁, for each occurrence, are independently -H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₁₀ and R₁₁, taken together with the nitrogen to which they are attached, form an optionally substituted heterocyclyl or an optionally substituted heteroaryl;
R₂₆, for each occurrence is, is independently, a lower alkyl;
p, for each occurrence, is, independently, 1 or 2; and
m, for each occurrence, is independently, 1, 2, 3, or 4.

In an embodiment, the JAK-2 inhibitor is a compound of Formula (XXXVI): 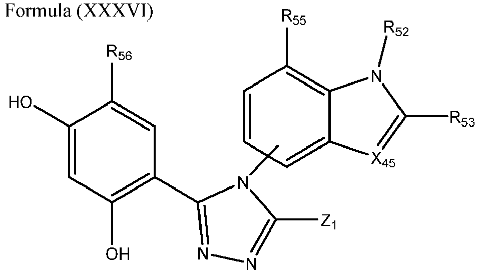 or a tautomer thereof, or a clathrate thereof, or a pharmaceutically acceptable salt, solvate, or hydrate thereof, wherein:
X₄₅ is CR₅₄ or N;
Z1 is -OH or -SH;
R₅₆ is selected from the group consisting of -H, methyl, ethyl, isopropyl, and cyclopropyl;
R₅₂ is selected from the group consisting of -H, methyl, ethyl, n-propyl, isopropyl, n-butyl, n-pentyl, n-hexyl, -(CH₂)₂OCH₃, -CH₂C(O)OH, and -C(O)N(CH₃)₂;
R₅₃ and R₅₄ are each, independently, -H, methyl, ethyl, or isopropyl; or R₅₃ and R₅₄ taken together with the carbon atoms to which they are attached form a phenyl, cyclohexenyl, or cyclooctenyl ring; and
R₅₅ is selected from the group consisting of -H, -OH, -OCH₃, and -OCH₂CH₃.

In a preferred embodiment, the JAK-2 inhibitor is ganetespib. In a preferred embodiment, the JAK-2 inhibitor is 5-(2,4-dihydroxy-5-isopropylphenyl)-4-(1-methyl-1*H*-indol-5-yl)-2,4-dihydro-3*H*-1,2,4-triazol-3-one. In a preferred embodiment, the JAK-2 inhibitor is a compound of Formula (XXXVII): 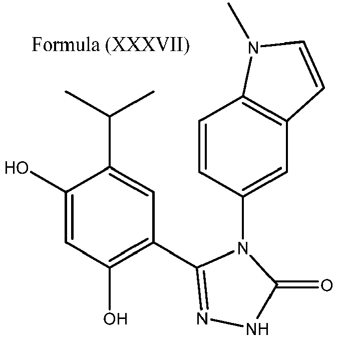 or a pharmaceutically acceptable salt, solvate, or hydrate thereof. The preparation of this compound is described in U.S. Patent Nos. 7,825,148 and 8,628,752, U.S. Patent Application Publication Nos. 2006/0167070 A1; 2014/0024030 A1; 2014/0051665 A1; 2014/0045908 A1; 2012/0128665 A1; 2013/0109045 A1, and 2014/0079636 A1, and, International Patent Application Publication No. WO 2013/170182; WO 2013/028505; WO 2013/067162; WO 2013/173436; WO 2013/006864; WO 2012/162584; WO 2013/170159; WO 2013/067165; WO 2013/074594; WO 2012/162372; WO 2012/162293; and WO 2012/155063. In an embodiment, the JAK-2 inhibitor is a compound described in U.S. Patent Nos. 7,825,148 and 8,628,752, U.S. Patent Application Publication Nos. 2006/0167070 A1; 2014/0024030 A1; 2014/0051665 A1; 2014/0045908 A1; 2012/0128665 A1; 2013/0109045 A1, and 2014/0079636 A1, and, International Patent Application Publication No. WO 2013/170182; WO 2013/028505; WO 2013/067162; WO 2013/173436; WO 2013/006864; WO 2012/162584; WO 2013/170159; WO 2013/067165; WO 2013/074594; WO 2012/162372; WO 2012/162293; and WO 2012/155063.

In an embodiment, the JAK-2 inhibitor is a compound of Formula (XXXVIII): 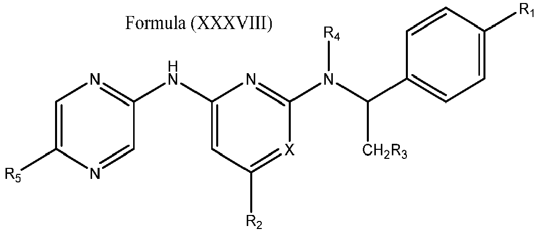 or a pharmaceutically acceptable salt, solvate, or hydrate thereof, wherein the compound is defined by the following (I) or (II).
(I): X represents CH or N; R₁ represents a halogen;
   R₂ represents: (1) H, (2) a halogen, (3) cyano,(4) a group represented by the following general formula [2]: 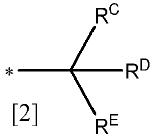 (wherein ^{∗} indicates the binding position; and R^{C}, R^{D} and R^{E} are the same or different and each represents (a) H, or (b) alkyl optionally substituted by hydroxy or alkoxy, or alternatively two of R^{C}, R^{D} and R^{E} are taken together with the adjacent C to represent a N-containing saturated heterocyclic group and the other one is H, the saturated heterocyclic group optionally substituted by alkylsulfonyl),
   (5) a group represented by the following general formula [3]: 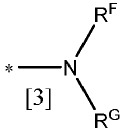 (wherein ^{∗} has the same meaning as described above; and R^{F} and R^{G} are the same or different and each represents (a) H, (b) alkyl optionally substituted by one or two groups selected from the group consisting of hydroxy, amino, dialkylamino, a saturated cyclic amino group, alkylcarbonylamino, alkylsulfonylamino, aryl, heteroaryl optionally substituted by alkyl, tetrahydrofuranyl, and carbamoyl, (c) alkylcarbonyl, (d) alkylsulfonyl, (e) carbamoyl, or (f) heteroaryl optionally substituted by alkyl, or alternatively R^{F} and R^{G} are taken together with the adjacent N to represent a saturated cyclic amino group, which may optionally be substituted by one or two groups selected from the group consisting of (a) halogen, (b) cyano, (c) hydroxy, (d) alkyl optionally substituted by one or two groups selected from the group consisting of hydroxy, alkoxy, amino, alkoxycarbonylamino, alkylsulfonylamino, and alkylcarbonylamino, (e) cycloalkyl, (f) haloalkyl, (g) alkoxy, (h) oxo, (i) a group represented by the following general formula [4]: 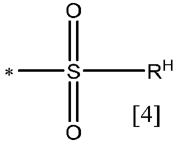 (wherein ^{∗} has the same meaning as described above; and R^{H} represents alkyl or aryl), (j) a group represented by the following general formula [5]: 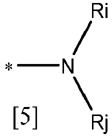 (wherein ^{∗} has the same meaning as described above; and R^{I} and R^{J} are the same or different and each represents H, alkyl, carbamoyl, alkylcarbonyl, or alkylsulfonyl), (k) a group represented by the following general formula [6]: 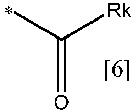 (wherein ^{∗} has the same meaning as described above; and R^{K} represents alkyl, hydroxy, amino, alkylamino, dialkylamino, cycloalkylamino, (cycloalkyl)alkylamino, (hydroxyalkyl)amino, (alkoxyalkyl)amino, alkoxy, alkylsulfonylamino, or a saturated cyclic amino group), and (1) a saturated cyclic amino group optionally substituted by hydroxy; and the saturated cyclic amino group, which is formed by combining R^{F}, R^{G} and the adjacent N, may form a spiro-linkage with a group represented by the following general formula [7A] or [7B]: 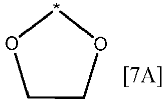 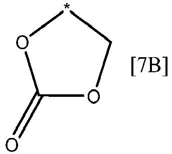 (wherein has the same meaning as described above)),
   (6) a group represented by the following general formula [8]: 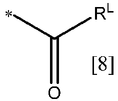 (wherein ^{∗} has the same meaning as described above; and R^{L} represents (a) alkyl, (b) hydroxy, (c) alkoxy, (d) saturated cyclic amino group optionally substituted by alkyl or alkylsulfonyl, or (e) an amino optionally substituted by one or two groups selected from the group consisting of alkyl, cycloalkyl, (cycloalkyl)alkyl, aralkyl; haloalkyl, dialkylaminoalkyl, alkoxyalkyl, and hydroxyalkyl),
   (7) a group represented by the following general formula [9]: 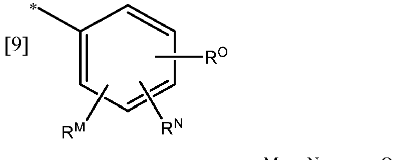 (wherein ^{∗} has the same meaning as described above; and R^{M}, R^{N} and R^{O} are the same or different and each represents H, halogen, cyano, alkoxy, carbamoyl, sulfamoyl, monoalkylaminosulfonyl, or alkylsulfonyl, or alternatively two of R^{M}, R^{N} and R^{O} are taken together to represent methylenedioxy),
   (8) -OR^{P}(R^{P} represents an alkyl optionally substituted by a group selected from the group consisting of hydroxy, dialkylamino, alkoxy, tetrahydrofuranyl, and cycloalkyl, or an optionally O-containing saturated cyclic group optionally substituted by hydroxy), or
   (9) a heteroaryl optionally substituted by one or two groups selected from the group consisting of cyano, halogen, hydroxy, alkoxy, alkylcarbonyl, carbamoyl, alkyl, cycloalkyl, (cycloalkyl)alkyl, aralkyl, hydroxycarbonyl and alkoxyalkyl;
   R₃ represents H or hydroxy;
   R₂ represents H or alkyl; and
   R₅ represents H or alkyl;
(II): X represents -CR^{A};
   R^{A} represents a group represented by the following general formula [10]: 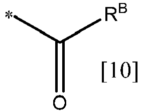 (wherein ^{∗} has the same meaning as described above; and R^{B} represents (a) amino optionally substituted by one or two groups selected from the group consisting of alkyl, cycloalkyl, (cycloalkyl)alkyl, and alkoxyalkyl, (b) alkoxy, (c) hydroxy, or (d) a saturated cyclic amino group);
   R₁ represents a halogen;
   R₂ represents H;
   R₃ represents E or hydroxy;
   R₄ represents H or alkyl; and
   R₅ represents H or alkyl.

In a preferred embodiment, the JAK-2 inhibitor is NS-018. In an embodiment, the JAK-2 inhibitor is (*S*)-*N*²-(1-(4-fluorophenyl)ethyl)-6-(1-methyl-1*H*-pyrazol-4-yl)-*N*⁴-(pyrazin-2-yl)pyrimidine-2,4-diamine. NS-018 has been described in Nakaya, et al., Blood Cancer J. 2014, 4, e174. In an embodiment, the JAK-2 inhibitor has the chemical structure shown in Formula (XXXIX): 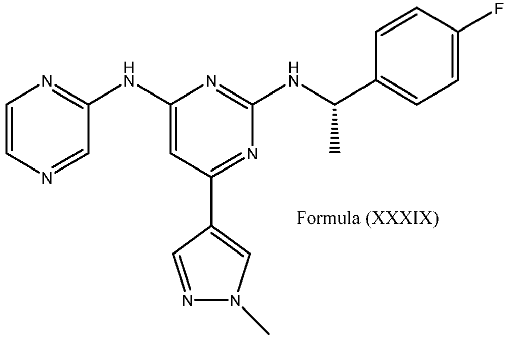 or a pharmaceutically acceptable salt, solvate, or hydrate thereof. The preparation of this compound is described in U.S. Patent Nos. 8,673,891 and 8,586,591, U.S. Patent Application Publication Nos. 2011/0288065 A1 and 2013/0131082 A1, and International Patent Application Publication No. WO 2012/020787 and WO 2012/020786. In an embodiment, the JAK-2 inhibitor is a compound described in U.S. Patent Nos. 8,673,891 and 8,586,591, U.S. Patent Application Publication Nos. 2011/0288065 A1 and 2013/0131082 A1, and International Patent Application Publication No. WO 2012/020787 and WO 2012/020786.

In an embodiment, the JAK-2 inhibitor is a compound of Formula (XL): 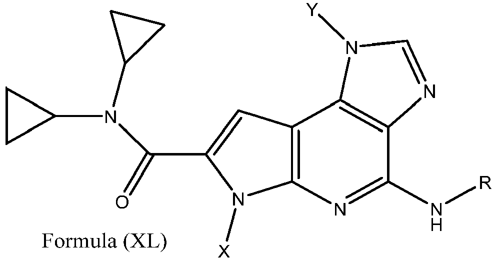 or a stereoisomer, tautomer, or pharmaceutically acceptable salt, solvate, or hydratethereof, wherein:
Y is C₁₋₄ alkyl;
X is C₁₋₄ alkyl;
R is 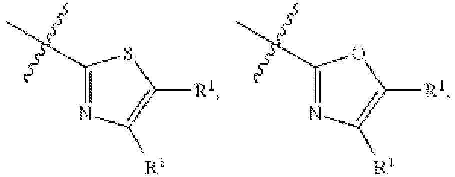 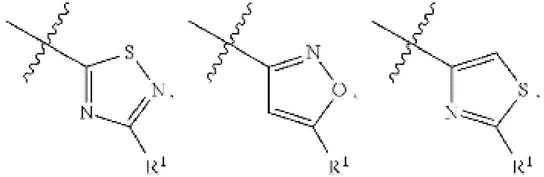 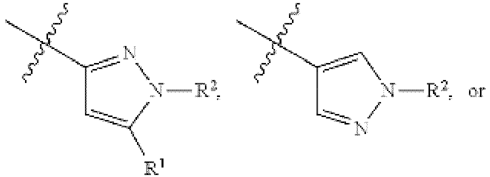 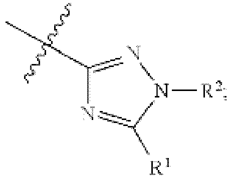 any of which are optionally fused with a 5 or 6 membered carbocycle or heterocycle having one heteroatom selected from NR³ or S, said fused carbocycle or heterocycle being optionally substituted with 0-3 R¹.
R¹ is H, halo, CN, C₁₋₆ alkyl substituted with 0-3 R^{c}, CF3, CONR^{a}R^{a}, NR^{a}R^{a}, COOR^{b}, SO₂-(C₁₋₄)alkyl, C(O)R^{d}, cycloalkyl substituted with 0-3 R^{e}, furanyl, tetrahydropyranyl, or pyridinyl;
R² is absent, H, C₁₋₆ alkyl substituted with 0-3 R^{c}, C(O)O-(C₁₋₄)alkyl, SO₂-(C₁₋₄)alkyl, cycloalkyl substituted with 0-3 R^{e}, or tetrahydropyranyl;
R³ is absent, H, or C(O)O-(C₁₋₄)alkyl;
R^{a} is H, C₁₋₆ alkyl substituted with 0-3 R^{e}, C₃₋₆ cycloalkyl substituted with 0-3 R^{e}, tetrahydropyranyl, or dioxotetrahydrothiophenyl;
R^{b} is H or C₁₋₆ alkyl;
R^{c} is H, halo, CN, OH, O-(C₁₋₄)alkyl, O--(C₁₋₄)alkyl-O-(C₁₋₄)alkyl, NH₂, N(C₁₋₄ alkyl)₂, C(O)N(C₁₋₄ alkyl)₂, SO₂-(C₁₋₄)alkyl, or morpholinyl or piperazinyl, either of which are optionally substituted with 0-1 C₁₋₄ alkyl;
R^{d} is C₁₋₆ alkyl, or azeridinyl, azetidinyl, pyrrolidinyl, piperidinyl, morpholinyl, piperazinyl, dioxidothiomorpholinyl or tetrahydropyranyl, any of which are substituted with 0-2 R^{e}; and
R^{e} is H, halo, CN, C₁₋₄ alkyl, OH, O-(C₁₋₄)alkyl, SO₂-(C₁₋₄)alkyl, NHC(O)-(C₁-₄)alkyl, morpholinyl, OC(O)-(C₁₋₄)alkyl, C(O)N(C₁₋₄ alkyl)₂, or O-(C₁₋₄)alkyl-O-(C₁₋₄)alkyl.

In an embodiment, the JAK-2 inhibitor is a compound of Formula (XL), wherein:
R is: 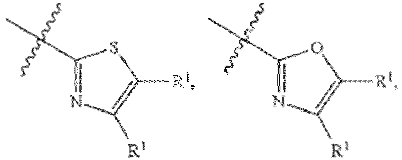 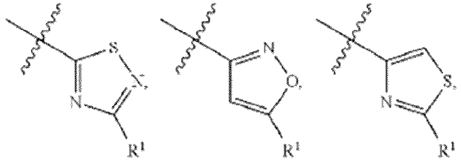 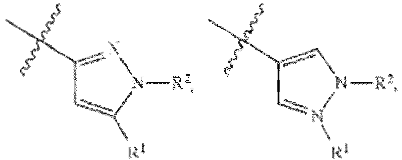 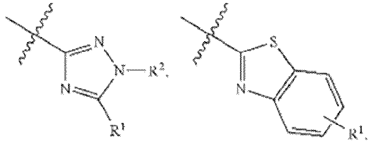 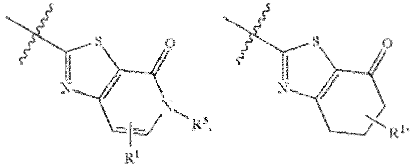 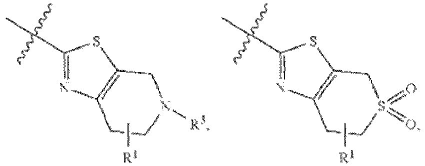 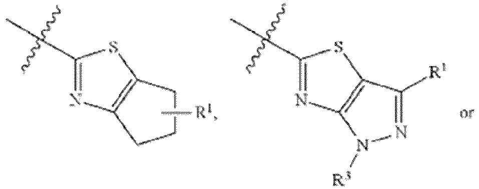 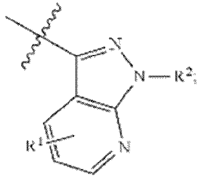 any of which are optionally substituted with 0-3 R¹.

In an embodiment, the JAK-2 inhibitor is a compound of Formula (XL), wherein Y is methyl and X is ethyl.

In another embodiment, the JAK-2 inhibitor is a compound of Formula (XL), wherein:
R is:

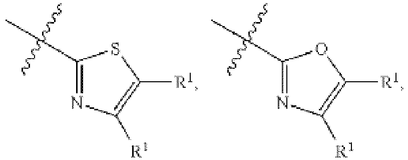

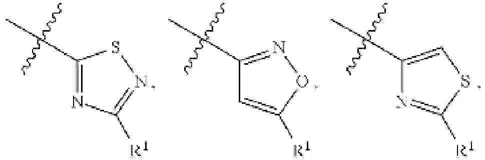

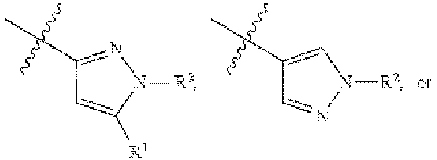

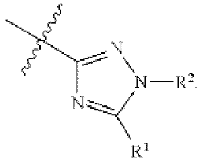

In an embodiment, the JAK-2 inhibitor is a compound of Formula (XL), wherein:
R is: 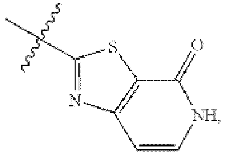 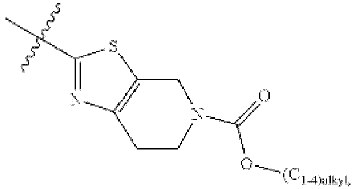 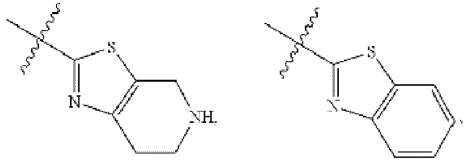 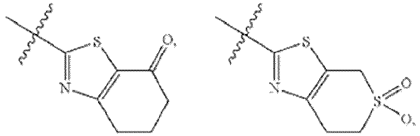 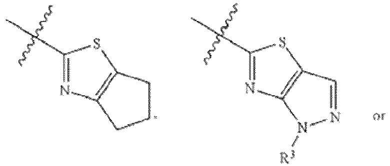 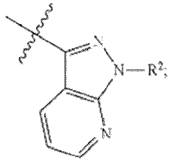 any of which are optionally substituted with 0-2 R¹.

In an embodiment, the JAK-2 inhibitor is a compound of Formula (XL), wherein
R is: 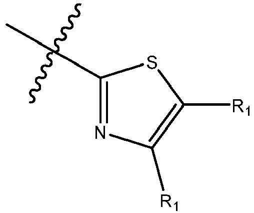
R¹ is H, halo, CN, C₁₋₆ alkyl substituted with 0-3 R^{c}, CF3, CONR^{a}R^{a}, COOR^{b}, SO₂₋(C₁₋₄)alkyl, C(O)R^{d}, cycloalkyl substituted with 0-3 R^{e}, or pyridinyl;
R^{a} is H, Ci-6 alkyl substituted with 0-3 R^{e}, C₃₋₆ cycloalkyl substituted with 0-3 R^{e}, tetrahydropyranyl or dioxotetrahydrothiophenyl;
R^{b} is H or C₁₋₆ alkyl;
R^{c} is H, halo, OH, O-(C₁₋₄)alkyl, SO₂-(C₁₋₄)alkyl or morpholinyl;
R^{d} is C₁₋₆ alkyl, or azetidinyl, pyrrolidinyl, morpholinyl, piperazinyl or dioxidothiomorpholinyl, any of which are substituted with 0-2 R^{e};
R^{e} is H, halo, CN, OH, O-(C₁₋₄)alkyl, SO₂-(C₁₋₄)alkyl, NHC(O)-(C₁₋₄)alkyl or morpholinyl.

In an embodiment, the JAK-2 inhibitor is a compound of Formula (XL), wherein:
R is: 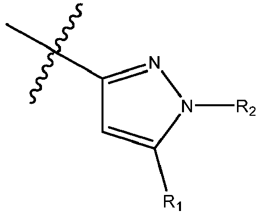
R¹ is H, halo, C₁₋₆ alkyl substituted with 0-3 R^{c}, CF3, CONR^{a}R^{a}, COOR^{b}, C(O)R^{d}, cycloalkyl substituted with 0-3 R^{e} or furanyl;
R² is H, C₁₋₆ alkyl substituted with 0-3 R^{c}, SO₂-(C₁₋₄)alkyl, cycloalkyl substituted with 0-3 R^{e}, or tetrahydropyranyl;
R^{a} is H, or C₁₋₆ alkyl substituted with 0-3 R^{e};
R^{b} is H or C₁₋₆ alkyl;
R^{c} is H, halo, CN, OH, O-(C₁₋₄)alkyl, O-(C₁₋₄)alkyl-O-(C₁₋₄)alkyl, NH₂, N(C₁₋₄ alkyl)₂, C(O)N(C₁₋₄ alkyl)₂, SO₂-(C₁₋₄)alkyl, or morpholinyl or piperazinyl, either of which are optionally substituted with 0-1 C₁₋₄ alkyl;
R^{d} is C₁₋₆ alkyl, or morpholinyl, piperazinyl or dioxidothiomorpholinyl, any of which are substituted with 0-2 R^{e}; and
R^{e} is H, C₁₋₄ alkyl, CN, OH, NHC(O)-(C₁₋₄)alkyl or morpholinyl.

In an embodiment, the JAK-2 inhibitor is a compound of Formula (XL), wherein:
R is: 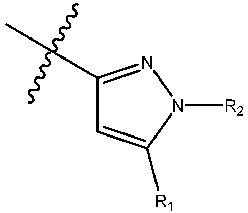
R¹ is C₁₋₆ alkyl substituted with 0-3 R^{c}; and
R² is C₁₋₆ alkyl.

In a preferred embodiment, the JAK-2 inhibitor is BMS-911543. In a preferred embodiment, the JAK-2 inhibitor is *N*,*N*-dicyclopropyl-4-((1,5-dimethyl-1*H*-pyrazol-3-yl)amino)-6-ethyl-1-methyl-1,6-dihydroimidazo[4,5-*d*]pyrrolo[2,3-*b*]pyridine-7-carboxamide. In a preferred embodiment, the JAK-2 inhibitor is a compound of Formula (XLI): 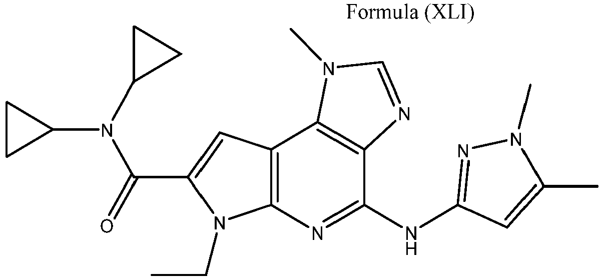 or a pharmaceutically acceptable salt, solvate, or hydrate thereof. The preparation of this compound is described in U.S. Patent Nos. 8,673,933 and 8,202,881 and U.S. Patent Application Publication Nos. 2013/0225551 A1 and 2011/0059943 A1. In an embodiment, the JAK-2 inhibitor is a compound described in U.S. Patent Nos. 8,673,933 and 8,202,881 and U.S. Patent Application Publication Nos. 2013/0225551 A1 and 2011/0059943 A1.

In a preferred embodiment, the JAK-2 inhibitor is gandotinib. In a preferred embodiment, the JAK-2 inhibitor is 3-(4-chloro-2-fluorobenzyl)-2-methyl-*N*-(5-methyl-1*H*-pyrazol-3-yl)-8-(morpholinomethyl)imidazo[1,2-*b*]pyridazin-6-amine. In a preferred embodiment, the JAK-2 inhibitor is a compound of Formula (XLII): 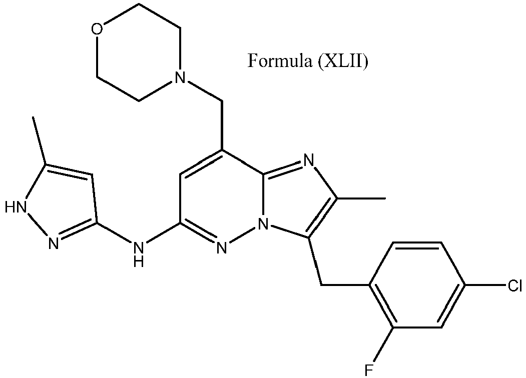 or a pharmaceutically acceptable salt, solvate, or hydrate thereof. The preparation of this compound is described in U.S. Patent No. 7,897,600 and U.S. Patent Application Publication Nos. 2010/0152181 A1 and 2010/0286139 A1. In an embodiment, the JAK-2 inhibitor is a compound described in U.S. Patent No. 7,897,600 and U.S. Patent Application Publication Nos. 2010/0152181 A1 and 2010/0286139 A1.

In an embodiment, the JAK-2 inhibitor is a compound of Formula (XLIII): 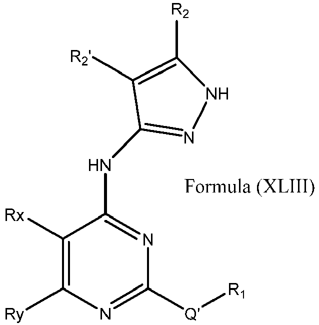 or a pharmaceutically acceptable salt, solvate, or hydrate thereof, wherein:
R^{x} and R^{y} are independently selected from the group consisting of -T-R³ and -L-Z-R³;
Q' is selected from the group consisting of -CR6"=CR6"- and wherein said - CR^{6"}=CR^{6"}- may be a cis or trans double bond or a mixture thereof,
R¹ is -T-(Ring D);
Ring D is a 5-7 membered monocyclic ring or 8-10 membered bicyclic ring selected from the group consisting of aryl, heteroaryl, heterocyclyl, and carbocyclyl, said heteroaryl or heterocyclyl ring having 1-4 ring heteroatoms selected from the group consisting of nitrogen, oxygen, and sulfur, wherein each substitutable ring carbon of Ring D is independently substituted by oxo, -T-R⁵ or -V-Z-R⁵, and each substitutable ring nitrogen of Ring D is independently substituted by -R⁴;
T is a valence bond or -(C(R^{6'})₂)-A-;
A is a valence bond or a C₁-C₃ alkylidene chain wherein a methylene unit of said C₁₋₃ alkylidene chain is optionally replaced by --O--, -S-, -N(R⁴)-, -CO-, - CONH-, -NHCO-, -SO₂-, -SO₂NH-, -NHSO₂-, -CO₂-, -OC(O)-, -OC(O)NH-, or -NHCO₂-;
Z is a C₁₋₄ alkylidene chain;
L is selected from the group consisting of -O-, -S-, -SO-, -SO₂-, - N(R⁶)SO₂-SO₂N(R⁶)-, -N(R⁶)-, -CO-, -CO₂-, -N(R⁶)CO-, - N(R⁶)C(O)O-, -N(R⁶)CON(R⁶)-, -N(R⁶)SO₂N(R⁶)-, -N(R⁶)N(R⁶)-, - C(O)N(R⁶)-, -OC(O)N(R⁶)-, -C(R⁶)₂-O-, -C(R⁶)₂-, -C(R⁶)₂SO-, - C(R⁶)₂SO₂-, -C(R⁶)₂SO₂N(R⁶)-, -C(R⁶)₂N(R⁶)-, -C(R⁶)₂N(R⁶)C(O)-, - C(R⁶)₂N(R⁶)C(O)O-, -C(R⁶)=NN(R⁶)-, -C(R⁶)=N-O-, - C(R⁶)₂N(R⁶)N(R⁶)-, -C(R⁶)₂N(R⁶)SO₂N(R⁶)-, and -C(R⁶)₂N(R⁶)CON(R⁶)-;
R² and R^{2'} are independently selected from the group consisting of -R and -T-W-R⁶, or R² and R^{2'} taken together with their intervening atoms form a fused, 5-8 membered, unsaturated or partially unsaturated ring having 0-3 ring heteroatoms selected from the group consisting of nitrogen, oxygen, and sulfur, wherein each substitutable ring carbon of said fused ring formed by R² and R^{2'} is independently substituted by halo, oxo, -CN, -NO₂, R⁷, or -V-R⁶, and each substitutable ring nitrogen of said ring formed by R² and R^{2'} is independently substituted by -R⁴;
R³ is selected from the group consisting of -R, -halo, -OR, -C(=O)R, -CO₂R, - COCOR, -COCH₂COF, -NO₂, -CN, -S(O)R, -S(O)₂R, -SR, -N(R⁴)₂, - CON(R⁷)₂, -SO₂N(R⁷)₂, --OC(=O)R, -N(R⁷)COR, -N(R⁷)CO₂(C₁₋₆ aliphatic), - N(R⁴)N(R⁴)₂, -C=NN(R⁴)₂, -C=N-OR, -N(R⁷)CON(R⁷)₂, -N(R⁷)SO₂N(R⁷)₂, -N(R⁴)SO₂R, and -OC(=O)N(R)₂;
each R is independently hydrogen or an optionally substituted group selected from the group consisting of C₁₋₆ aliphatic, C₆₋₁₀ aryl, a heteroaryl ring having 5-10 ring atoms, and a heterocyclyl ring having 5-10 ring atoms;
each R⁴ is independently selected from the group consisting of -R⁷, -COR⁷, - CO₂(optionally substituted C₁₋₆ aliphatic), -CON(R⁷)₂, and -SO₂R⁷;
each R⁵ is independently selected from the group consisting of -R, halo, -OR, - C(=O)R, -CO₂R, -COCOR, -NO₂, -CN, -S(O)R, -SO₂R, -SR, -N(R⁴)₂, -CON(R⁴)₂, -SO₂N(R⁴)₂, -OC(=O)R, -N(R⁴)COR, -N(R⁴)CO₂ (optionally substituted C₁₋₆ aliphatic), -N(R⁴)N(R⁴)₂, -C=NN(R⁴)₂, -C=N-OR, - N(R⁴)CON(R⁴)₂, -N(R⁴)SO₂N(R⁴)₂, -N(R⁴)SO₂R, and -OC(=O)N(R⁴)₂;
V is selected from the group consisting of -O-, -S-, -SO-, -SO₂-, - N(R⁶)SO₂-, -SO₂N(R⁶)-, -N(R⁶)-, -CO-, -CO₂-, -N(R⁶)CO-, - N(R⁶)C(O)O-, -N(R⁶)CON(R⁶)-, -N(R⁶)SO₂N(R⁶)-, -N(R⁶)N(R⁶)-, - C(O)N(R⁶)-, -OC(O)N(R⁶)-, -C(R⁶)₂O-, -C(R⁶)₂S-, -C(R⁶)₂SO-, - C(R⁶)₂SO₂-, -C(R⁶)₂SO₂N(R⁶)-, -C(R⁶)₂N(R⁶)-, -C(R⁶)₂N(R⁶)C(O)-, - C(R⁶)₂N(R⁶)C(O)O-, -C(R⁶)=NN(R⁶)-, -C(R⁶)=N-O-, - C(R⁶)₂N(R⁶)N(R⁶)-, -C(R⁶)₂N(R⁶)SO₂N(R⁶)-, and -C(R⁶)₂N(R⁶)CON(R⁶)-;
W is selected from the group consisting of -C(R⁶)₂O-, -C(R⁶)₂S-, -C(R⁶)₂SO-, -C(R⁶)₂SO₂- -C(R⁶)₂SO₂N(R⁶)-, -C(R⁶)₂N(R⁶)-, -CO-, -CO₂-, - C(R⁶)OC(O)-, -C(R⁶)OC(O)N(R⁶)-, -C(R⁶)₂N(R⁶)CO-, - C(R⁶)₂N(R⁶)C(O)O-, -C(R⁶)=NN(R⁶)-, -C(R⁶)=N-O-, - C(R⁶)₂N(R⁶)N(R⁶)-, -C(R⁶)₂N(R⁶)SO₂N(R⁶)-, -C(R⁶)₂N(R⁶)CON(R⁶)-, and -CON(R⁶)-;
each R⁶ is independently selected from the group consisting of hydrogen and an optionally substituted C₁₋₄ aliphatic group, or two R⁶ groups on the same nitrogen atom may be taken together with the nitrogen atom to form a 3-6 membered heterocyclyl or heteroaryl ring;
each R^{6'} is independently selected from the group consisting of hydrogen and a C₁₋₄ aliphatic group, or two R^{6'} on the same carbon atom are taken together to form a 3-8 membered carbocyclic ring;
each R^{6"} is independently selected from the group consisting of hydrogen, a C₁₋₄ aliphatic group, halogen, optionally substituted aryl, and optionally substituted heteroaryl, or two R⁶ on adjacent carbon atoms are taken together to form a 5-7 membered carbocyclic ring; and
each R⁷ is independently selected from the group consisting of hydrogen and an optionally substituted C₁₋₆ aliphatic group, or two R⁷ on the same nitrogen are taken together with the nitrogen to form a 5-8 membered heterocyclyl or heteroaryl ring.

In a preferred embodiment, the JAK-2 inhibitor is ENMD-2076. In a preferred embodiment, the JAK-2 inhibitor is (*E*)-*N*-(5-methyl-1*H*-pyrazol-3-yl)-6-(4-methylpiperazin-1-yl)-2-styrylpyrimidin-4-amine. In a preferred embodiment, the JAK-2 inhibitor is a compound of Formula (XLIV): 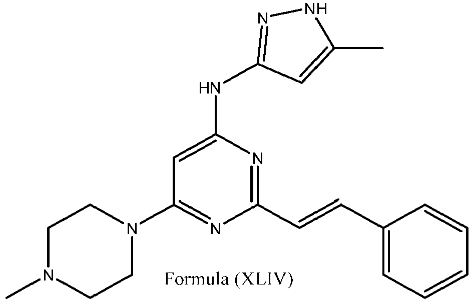 or a pharmaceutically acceptable salt, solvate, or hydrate thereof. The preparation of this compound is described in U.S. Patent Nos. 8,153,630; 7,563,787; and, 8,114,870 and U.S. Patent Application Publication Nos. 2008/0200485 A1; 2007/0142368 A1; 2009/0264422 A1; 2011/0318393 A1; and, 2009/0029992 A1. In an embodiment, the JAK-2 inhibitor is a compound described in U.S. Patent Nos. 8,153,630; 7,563,787; and, 8,114,870 and U.S. Patent Application Publication Nos. 2008/0200485 A1; 2007/0142368 A1; 2009/0264422 A1; 2011/0318393 A1; and, 2009/0029992 A1.

In an embodiment, the JAK-2 inhibitor is a compound of Formula (XLV): 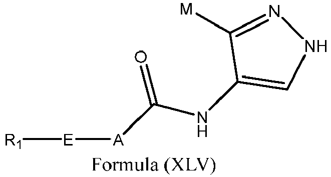 or a pharmaceutically acceptable salt, solvate, hydrate, tautomer or N-oxide thereof, wherein M is selected from a group D1 and a group D2: 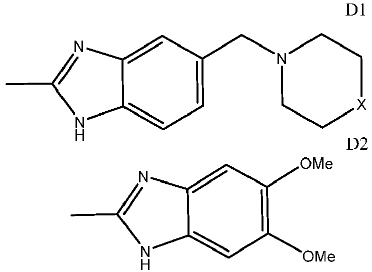 and wherein:
(A) when M is a group D1:
   X is selected from O, NH and NCH₃;
   A is selected from a bond and a group NR₂ where R² is hydrogen or methyl;
   E is selected from a bond, CH₂, CH(CN) and C(CH₃)₂;
   R₁ is selected from:
      (i) a cycloalkyl group of 3 to 5 ring members optionally substituted by hydroxy, fluorine, amino, methylamino, methyl or ethyl;
      (ii) a saturated heterocyclic group of 4 to 6 ring members containing 1 or 2 heteroatom ring members selected from O, N, S and SO₂, the heterocyclic group being optionally substituted by (C₁₋₄)alkyl, amino or hydroxy; but excluding unsubstituted 4-morpholinyl, unsubstituted tetrahydropyran-4-yl, unsubstituted 2-pyrrolidinyl, and unsubstituted and 1-substituted piperidine-4-yl;
      (iii) a 2,5-substituted phenyl group of the formula: 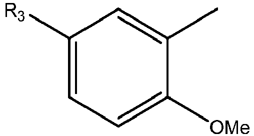 wherein (a) when X is NH or N-CH₃, R₃ is selected from chlorine and cyano; and (b) when X is O, R₃ is CN;
      (iv) a group CR₆R₇R₈ wherein R₆ and R₇ are each selected from hydrogen and methyl, and R₈ is selected from hydrogen, methyl, (C₁₋₄)alkylsulphonylmethyl, hydroxymethyl and cyano;
      (v) a pyridazin-4-yl group optionally substituted by one or two substituents selected from methyl, ethyl, methoxy and ethoxy;
      (vi) a substituted imidazothiazole group wherein the substituents are selected from methyl, ethyl, amino, fluorine, chlorine, amino and methylamino; and
      (vii) an optionally substituted 1,3-dihydro-isoindol-2-yl or optionally substituted 2,3-dihydro-indol-1-yl group wherein the optional substituents in each case are selected from halogen, cyano, amino, C₁₋₄ mono- and dialkylamino, CONH₂ or CONH-(C₁₋₄)alkyl, C₁₋₄ alkyl and C₁₋₄ alkoxy wherein the C₁₋₄ alkyl and C₁₋₄ alkoxy groups are optionally substituted by hydroxy, methoxy, or amino;
      (viii) 3-pyridyl optionally substituted by one or two substituents selected from hydroxy, halogen, cyano, amino, C₁₋₄ mono- and dialkylamino, CONH₂ or CONH--C₁₋₄ alkyl, C₁₋₄ alkyl and C₁₋₄ alkoxy wherein the C₁₋₄ alkyl and C₁₋₄ alkoxy groups are optionally substituted by hydroxy, methoxy, or amino, but excluding the compounds 2-oxo-1,2-dihydro-pyridine-3-carboxylic acid [3-(5-morpholin-4-ylmethyl-*1H*-benzoimidazol-2-yl)-*1H*-pyrazol-4-yl]-amide and 2,6-dimethoxy-N-[3-(5-morpholin-4-ylmethyl-*1H-*benzoimidazol-2-yl)-*1H*-pyrazol-4-yl]-nicotinamide;
      (ix) thiomorpholine or an S-oxide or S,S-dioxide thereof optionally substituted by one or two substituents selected from halogen, cyano, amino, C₁₋₄ mono- and dialkylamino, CONH₂ or CONH-C₁₋₄ alkyl, C₁₋₄ alkyl and C₁₋₄ alkoxy wherein the C₁₋₄ alkyl and C₁₋₄ alkoxy groups are optionally substituted by hydroxy, methoxy, or amino; and
      when E-A is NR₂, R₁ is additionally selected from:
      (x) 2-fluorophenyl, 3-fluorophenyl, 4-fluorophenyl, 2,4-difluorophenyl, 3,4-difluorophenyl, 2,5-difluorophenyl, 3,5-difluorophenyl, 2,4,6-trifluorophenyl, 2-methoxyphenyl, 5-chloro-2-methoxyphenyl, cyclohexyl, unsubstituted 4-tetrahydropyranyl and tert-butyl;
      (xi) a group NR₁₀R₁₁ where R₁₀ and R₁₁ are each C₁₋₄ alkyl or R₁₀ and R₁₁ are linked so that NR₁₀R₁₁ forms a saturated heterocyclic group of 4 to 6 ring members optionally containing a second heteroatom ring member selected from O, N, S and SO₂, the heterocyclic group being optionally substituted by CI-4 alkyl, amino or hydroxy;
      (xii) pyridone optionally substituted by one or two substituents selected from hydroxy, halogen, cyano, amino, C₁₋₄ mono- and dialkylamino, CONH2, CONH--C₁₋₄ alkyl, C₁₋₄ alkyl and C₁₋₄ alkoxy wherein the C₁₋₄ alkyl and C₁₋₄ alkoxy groups are optionally substituted by hydroxy, methoxy, or amino;
      when E-A is C(CH₃)₂NR₂ or CH₂-NR₂, R₁ is additionally selected from:
      (xiii) unsubstituted 2-furyl and 2,6-difluorophenyl; and
      when E-A is C(CH3)₂NR₂, R₁ is additionally selected from:
      (xiv) unsubstituted phenyl; and
      when E is CH₂, R₁ is additionally selected from:
      (xv) unsubstituted tetrahydropyran-4-yl; and
(B) when M is a group D2:
   A is selected from a bond and a group NR₂ where R₂ is hydrogen or methyl;
   E is selected from a bond, CH₂, CH(CN) and C(CH₃)₂;
   R₁ is selected from:
      (xvi) a 2-substituted 3-furyl group of the formula: 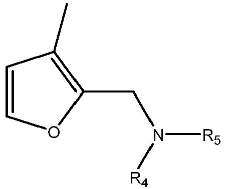 wherein R₄ and R₅ are the same or different and are selected from hydrogen and C₁₋₄ alkyl, or R₄ and R₅ are linked so that NR₄R₅ forms a 5- or 6-membered saturated heterocyclic group optionally containing a second heteroatom or group selected from O, NH, NMe, S or SO₂, the 5- or 6-membered saturated ring being optionally substituted by hydroxy, fluorine, amino, methylamino, methyl or ethyl; (xvii) a 5-substituted 2-furyl group of the formula: 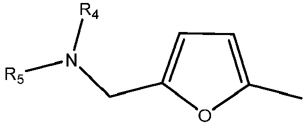 wherein R₄ and R₅ are the same or different and are selected from hydrogen and C₁₋₄ alkyl, or R₄ and R₅ are linked so that NR₄R₅ forms a 5- or 6-membered saturated heterocyclic group optionally containing a second heteroatom or group selected from O, NH, NMe, S or SO₂, the 5- or 6-membered saturated heterocyclic group being optionally substituted by hydroxy, fluorine, amino, methylamino, methyl or ethyl;
         with the proviso that the compound is not 5-piperidin-1-ylmethyl-furan-2-carboxylic acid [3-(5,6-dimethoxy-1*H*-benzoimidazol-2-yl)-1*H*-pyrazol-4-yl]-amide;
      (xviii) a group of the formula: 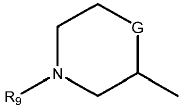 wherein R₉ is hydrogen, methyl, ethyl or isopropyl; G is CH, O, S, SO, SO₂ or NH and the group is optionally substituted by one, two or three substituents selected from C₁₋₄ hydrocarbyl, hydroxy, C₁₋₄ hydrocarbyloxy, fluorine, amino, mono- and di-C₁₋₄ alkylamino and wherein the C₁₋₄ hydrocarbyl and C₁₋₄ hydrocarbyloxy groups are each optionally substituted by hydroxy, fluorine, amino, mono- or di-C₁₋₄ alkylamino; and (xix) a 3,5-disubstituted phenyl group of the formula: 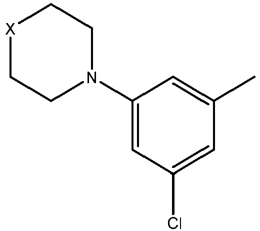 wherein X is selected from O, NH and NCH₃; and
(C) when M is a group D1:
   and X is O; A is a group NR₂ where R² is hydrogen; E is a bond; and R₁ is 2,6-difluorophenyl; then the compound of the Formula (XLV) is an acid addition salt selected from salts formed with an acid selected from the group consisting of acetic, adipic, alginic, ascorbic (e.g. L-ascorbic), aspartic (e.g. L-aspartic), benzenesulphonic, benzoic, camphoric (e.g. (+) camphoric), capric, caprylic, carbonic, citric, cyclamic, dodecanoate, dodecylsulphuric, ethane-1,2-disulphonic, ethanesulphonic, fumaric, galactaric, gentisic, glucoheptonic, D-gluconic, glucuronic (e.g. D-glucuronic), glutamic (e.g. L-glutamic), α-oxoglutaric, glycolic, hippuric, hydrochloric, isethionic, isobutyric, lactic (e.g. (+)-L-lactic and (±)-DL-lactic), lactobionic, laurylsulphonic, maleic, malic, (-)-L-malic, malonic, methanesulphonic, mucic, naphthalenesulphonic (e.g. naphthalene-2-sulphonic), naphthalene-1,5-disulphonic, nicotinic, oleic, orotic, oxalic, palmitic, pamoic, phosphoric, propionic, sebacic, stearic, succinic, sulphuric, tartaric (e.g. (+)-L-tartaric), thiocyanic, toluenesulphonic (e.g. p-toluenesulphonic), valeric and xinafoic acids.

In a preferred embodiment, the JAK-2 inhibitor is AT-9283. In a preferred embodiment, the JAK-2 inhibitor is 1-cyclopropyl-3-(3-(5-(morpholinomethyl)-1*H-*benzo[*d*]imidazol-2-yl)-1*H*-pyrazol-4-yl)urea. In a preferred embodiment, the JAK-2 inhibitor is a compound of Formula (XLVI): 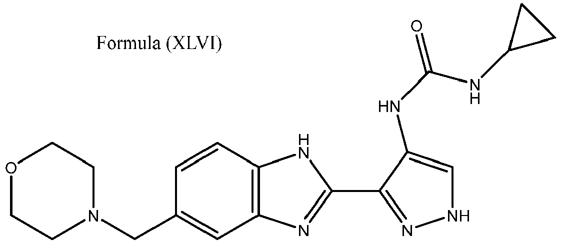 or a pharmaceutically acceptable salt, solvate, or hydrate thereof. The preparation of this compound is described in U.S. Patent Nos. 8,399,442 and 7,977,477 and U.S. Patent Application Publication Nos. 2010/0004232 A1; 2014/0010892 A1; 2011/0224203 A1; and, 2007/0135477. In an embodiment, the JAK-2 inhibitor is a compound described in U.S. Patent Nos. 8,399,442 and 7,977,477 and U.S. Patent Application Publication Nos. 2010/0004232 A1; 2014/0010892 A1; 2011/0224203 A1; and, 2007/0135477.

In an embodiment, the JAK-2 inhibitor is a compound of Formula (XLVII): 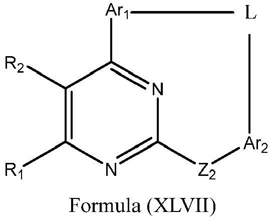 or a pharmaceutically acceptable salt, solvate, or hydrate thereof, wherein:
R¹ and R² are each independently selected from the group consisting of: H, halogen, alkyl, alkenyl, alkynyl, haloalkyl, haloalkenyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroarylheteroalkyl, arylheteroalkyl, hydroxy, hydroxyalkyl, alkoxy, alkoxyalkyl, alkoxyaryl, alkenyloxy, alkynyloxy, cycloalkylkoxy, heterocycloalkyloxy, aryloxy, arylalkyloxy, phenoxy, benzyloxy, heteroaryloxy, amino, alkylamino, aminoalkyl, acylamino, arylamino, sulfonylamino, sulfinylamino, -COOH, -COR³, -COOR³, -CONHR³, -NHCOR³, - NHCOOR³, -NHCONHR³, alkoxycarbonyl, alkylaminocarbonyl, sulfonyl, alkylsulfonyl, alkylsulfinyl, arylsulfonyl, arylsulfinyl, aminosulfonyl, -SR³, R⁴S(O)R⁶-, R⁴S(O)₂R⁶-, R⁴C(O)N(R⁵)R⁶-, R⁴SO₂N(R⁵)R⁶-, R⁴N(R⁵)C(O)R⁶-, R⁴N(R⁵)SO₂R⁶-, R⁴N(R⁵)C(O)N(R⁵)R⁶- and acyl, each of which may be optionally substituted;
each R³, R⁴, and R⁵ is independently selected from the group consisting of H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;
each R⁶ is independently selected from the group consisting of a bond, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;
Z² is independently selected from the group consisting of a bond, O, S, -N(R⁷)-, - N(R⁷)C₁₋₂alkyl-, and -C₁₋₂alkylN(R⁷)-;
each R⁷ is independently selected from the group consisting of H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;
Ar¹ and Ar² are each independently selected from the group consisting of aryl and heteroaryl, each of which may be optionally substituted;
L is a group of formula:

   -X¹-Y-X²-
wherein X¹ is attached to Ar¹ and X² is attached to Ar², and wherein X¹, X² and Y are selected such that the group L has between 5 and 15 atoms in the normal chain,
X¹ and X² are each independently a heteroalkyl group containing at least one oxygen atom in the normal chain,
Y is a group of formula -CR^{a}=CR^{b}- or an optionally substituted cycloalkyl group,
wherein R^{a} and R^{b} are each independently selected from the group consisting of H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted, or
R^{a} and R^{b} may be joined such that when taken together with the carbon atoms to which they are attached they form a cycloalkenyl or cycloheteroalkenyl group;
or a pharmaceutically acceptable salt, solvate, or hydrate thereof, or an N-oxide thereof.

In certain embodiments Z² is selected from the group consisting of a bond, -N(R⁷)-, and -S-. In one specific embodiment Z² is -N(R⁷)-. In an even more specific embodiment Z² is -N(H)-.
Ar¹ and Ar² are each independently selected from the group consisting of aryl and heteroaryl and may be monocyclic, bicyclic or polycyclic moieties. In certain embodiments each of Ar¹ and Ar² is a monocyclic or bicyclic moiety. In certain embodiments each of Ar¹ and Ar² are a monocyclic moiety.

In certain embodiments Ar¹ is selected from the group consisting of: 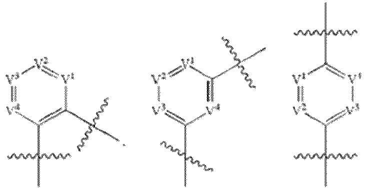 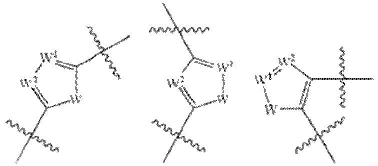 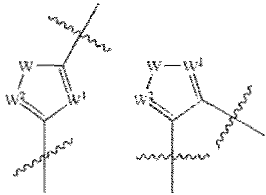 and 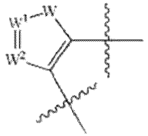 wherein V¹, V², V³ and V⁴ are each independently selected from the group consisting of N, and C(R¹⁰);
W is selected from the group consisting of O, S and NR¹⁰;
W¹ and W² are each independently selected from the group consisting of N and CR¹⁰;
wherein each R¹⁰ is independently selected from the group consisting of: H, halogen, alkyl, alkenyl, alkynyl, haloalkyl, haloalkenyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroarylheteroalkyl, arylheteroalkyl, hydroxy, hydroxyalkyl, alkoxy, alkoxyalkyl, alkoxyaryl, alkenyloxy, alkynyloxy, cycloalkylkoxy, heterocycloalkyloxy, aryloxy, arylalkyloxy, phenoxy, benzyloxy, heteroaryloxy, amino, alkylamino, aminoalkyl, acylamino, arylamino, sulfonylamino, sulfinylamino, -COOH, -COR³, -COOR³, -CONHR³, -NHCOR³, - NHCOOR³, -NHCONHR³, alkoxycarbonyl, alkylaminocarbonyl, sulfonyl, alkylsulfonyl, alkylsulfinyl, arylsulfonyl, arylsulfinyl, aminosulfonyl, -SR³, R⁴S(O)R⁶-, R⁴S(O)₂R⁶-, R⁴C(O)N(R⁵)R⁶-, R⁴SO₂N(R⁵)R⁶-, R⁴N(R⁵)C(O)R⁶-, R⁴N(R⁵)SO₂R⁶-, R⁴N(R⁵)C(O)N(R⁵)R⁶- and acyl, each of which may be optionally substituted,
wherein R³, R⁴, R⁵ and R⁶ are as defined above.
In certain embodiments Ar¹ is selected from the group consisting of: 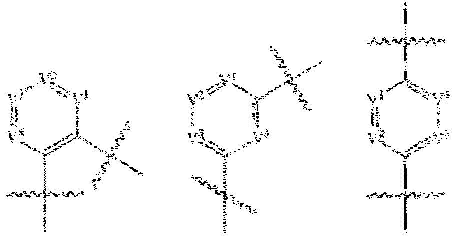 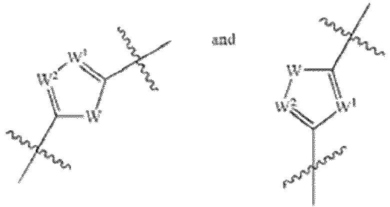 wherein V¹, V², V³, V⁴, W, W¹, W², R³, R⁴, R⁵ and R⁶ are as defined above.

In certain embodiments Ar¹ is selected from the group consisting of: 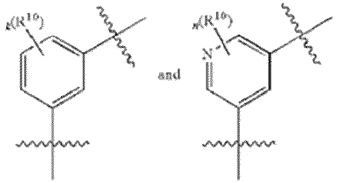
wherein each R¹⁰ is independently as defined above,
k is an integer selected from the group consisting of 0, 1, 2, 3, and 4; and
n is an integer selected from the group consisting of 0, 1, and 2.

In yet an even further embodiment Ar¹ is selected from the group consisting of: 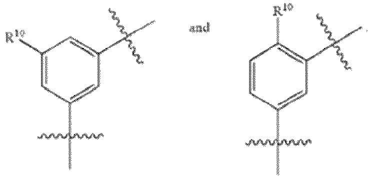 wherein R¹⁰ is as defined above.

In certain embodiments Ar¹ is selected from the group consisting of: 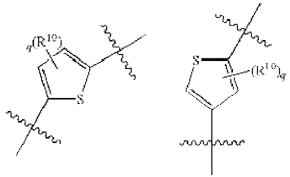 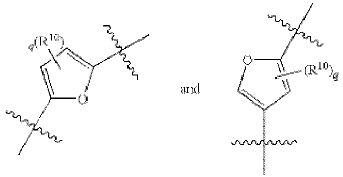 wherein each R¹⁰ is independently as defined above, and
q is an integer selected from the group consisting of 0, 1 and 2.

In certain embodiments Ar¹ is selected from the group consisting of:

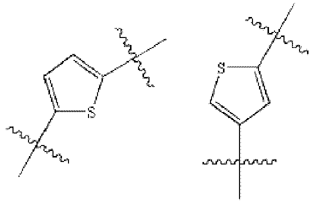

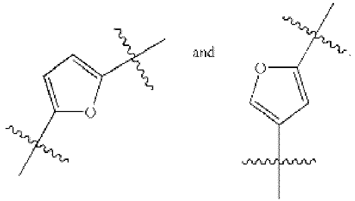

In certain embodiments Ar¹ is selected from the group consisting of:

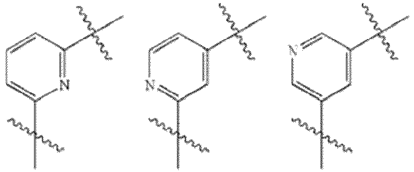

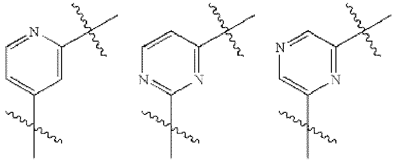

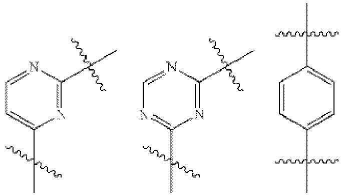

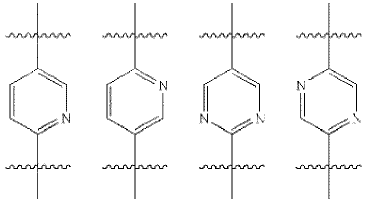

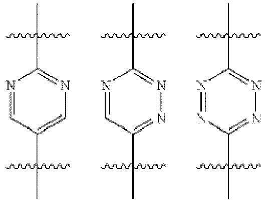

In certain embodiments Ar² is selected from the group consisting of: 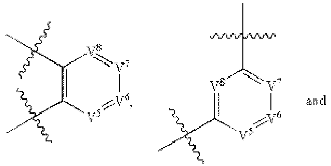 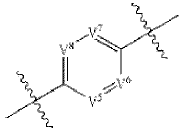 wherein V⁵, V⁶, V⁷ and V⁸ are independently selected from the group consisting of N, and C(R¹¹);
wherein each R¹¹ is independently selected from the group consisting of: H, halogen, alkyl, alkenyl, alkynyl, haloalkyl, haloalkenyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroarylheteroalkyl, arylheteroalkyl, hydroxy, hydroxyalkyl, alkoxy, alkoxyalkyl, alkoxyaryl, alkenyloxy, alkynyloxy, cycloalkylkoxy, heterocycloalkyloxy, aryloxy, arylalkyloxy, phenoxy, benzyloxy, heteroaryloxy, amino, alkylamino, aminoalkyl, acylamino, arylamino, sulfonylamino, sulfinylamino, -COOH, -COR³, -COOR³, -CONHR³, -NHCOR³, -NHCOOR³, -NHCONHR³, alkoxycarbonyl, alkylaminocarbonyl, sulfonyl, alkylsulfonyl, alkylsulfinyl, arylsulfonyl, arylsulfinyl, aminosulfonyl, -SR³, R⁴S(O)R⁶-, R⁴S(O)₂R⁶-, R⁴C(O)N(R⁵)R⁶-, R⁴SO₂N(R⁵)R⁶-, R⁴N(R⁵)C(O)R⁶-, R⁴N(R⁵)SO₂R⁶-, R⁴N(R⁵)C(O)N(R⁵)R⁶- and acyl, each of which may be optionally substituted.

In certain embodiments Ar² is selected from the group consisting of: 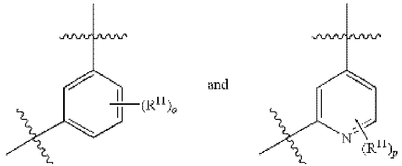 wherein each R¹¹ is independently as defined above
o is an integer selected from the group consisting of 0, 1, 2, 3, and 4; and
p is an integer selected from the group consisting of 0, 1, 2, and 3.

In certain embodiments Ar² is selected from the group consisting of: 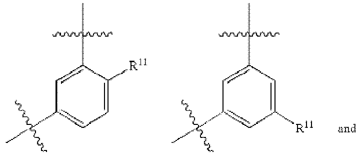 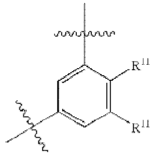 wherein each R¹¹ is as defined above.

In a further embodiment Ar² is selected from the group consisting of:

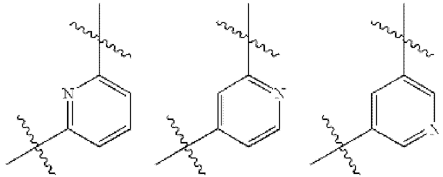

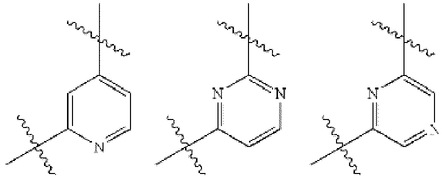

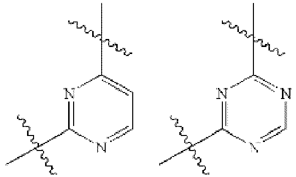

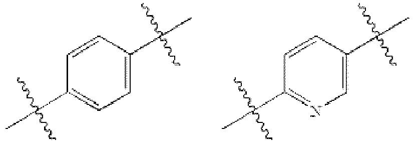

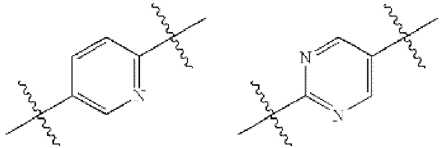

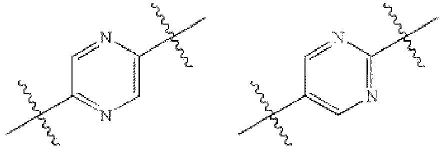

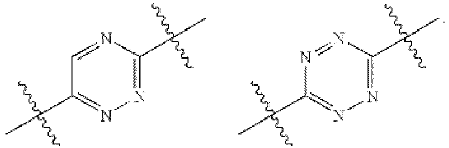

In an embodiment, the JAK-2 inhibitor is a compound of Formula (XLVIII): 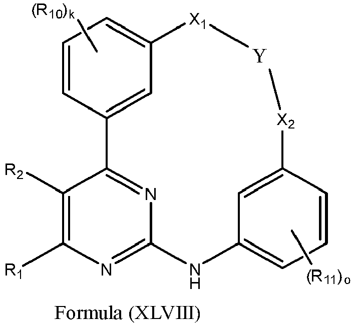 or a pharmaceutically acceptable salt, solvate, or hydrate thereof, wherein R¹, R², R¹⁰, R¹¹, X¹, X², Y, k and o are as defined above.

In an embodiment, the JAK-2 inhibitor is a compound of Formula (XLIX): 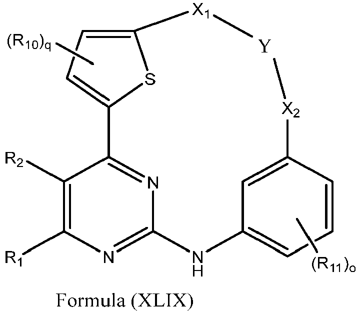 or a pharmaceutically acceptable salt, solvate, or hydrate thereof
wherein R¹, R², R¹⁰, R¹¹, X¹, X², Y, q and o are as defined above.

In an embodiment, the JAK-2 inhibitor is a compound of Formula (L): 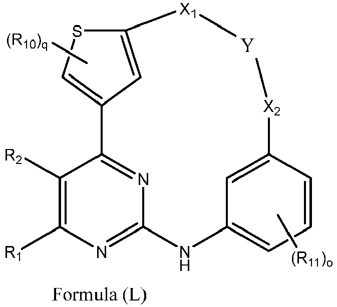 or a pharmaceutically acceptable salt, solvate, or hydrate thereof, wherein R¹, R², R¹⁰, R¹¹, X¹, X², Y, q and o are as defined above.

In an embodiment, the JAK-2 inhibitor is a compound of Formula (LI): 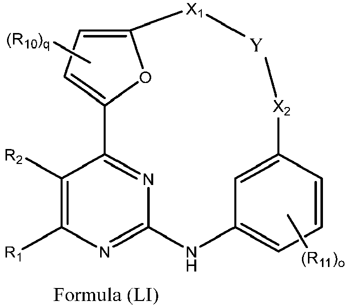 or a pharmaceutically acceptable salt, solvate, or hydrate thereof, wherein R¹, R², R¹⁰, R¹¹, X¹, X², Y, q and o are as defined above.

In an embodiment, the JAK-2 inhibitor is a compound of Formula (LII): 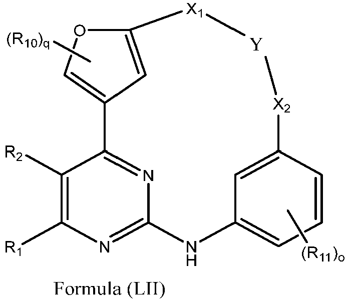 or a pharmaceutically acceptable salt, solvate, or hydrate thereof, wherein R¹, R², R¹⁰, R¹¹, X¹, X², Y, q and o are as defined above.

In an embodiment, the JAK-2 inhibitor is a compound of Formula (LIII): 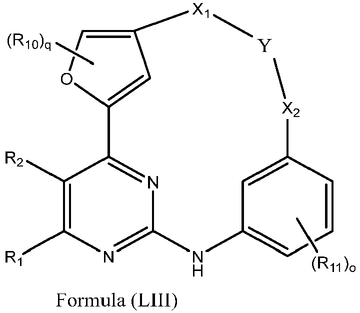 or a pharmaceutically acceptable salt, solvate, or hydrate thereof, wherein R¹, R², R¹⁰, R¹¹, X¹, X², Y, q and o are as defined above.

In embodiments where the JAK-2 inhibitor is a compound of Formulas (XLVII)-(LIII), X¹, X² and Y are chosen such that there are between 5 and 15 atoms in the normal chain. In one embodiment, X¹, X² and Y are chosen such that there are between 6 and 15 atoms in the normal chain. In one specific embodiment, X¹, X² and Y are chosen such that there are 7 atoms in the normal chain. In another specific embodiment, X¹, X² and Y are chosen such that there are 8 atoms in the normal chain.

In embodiments where the JAK-2 inhibitor is a compound of Formulas (XLVII)-(LIII), X¹ and X² are each independently a heteroalkyl group containing at least one oxygen atom in the normal chain. In certain embodiments X¹ is selected from the group consisting of: (a) -O(C₁₋₅)alkyl-, (b) -(C₁₋₅)alkylO-, and (c) -(C₁₋₅)alkylO(C₁₋₅)alkyl. In certain embodiments X¹ is selected from the group consisting of: (a) - OCH₂- (b) -CH₂O-, (c) -OCH₂CH₂-, (d) -CH₂CH₂O-, (e) -CH₂OCH₂-, and (f) -CH₂CH₂OCH₂-. In one specific embodiment X¹ is -OCH₂-. In another specific embodiment X¹ is -CH₂O-. In another specific embodiment X¹ is -OCH₂CH₂-. In another specific embodiment X¹ is -CH₂CH₂O-. In another specific embodiment X¹ is -CH₂OCH₂-. In another specific embodiment X¹ is -CH₂CH₂OCH₂-. In certain embodiments X² is selected from the group consisting of: (a) -O(C₁₋₅)alkyl-, (b) -(C₁₋₅)alkylO-, and (c) -(C₁₋₅)alkylO(C₁₋₅)alkyl. In certain embodiments X² is selected from the group consisting of: (a) -OCH₂- (b) -CH₂O-, (c) -OCH₂CH₂-, (d) - CH₂CH₂O-, (e) -CH₂OCH₂-, and (f) -CH₂CH₂OCH₂-. In one specific embodiment X² is -OCH₂-. In another specific embodiment X¹ is -CH₂O-. In another specific embodiment X² is -OCH₂CH₂-. In another specific embodiment X² is -CH₂CH₂O-. In another specific embodiment X² is -CH₂OCH₂-. In another specific embodiment X² is -CH₂CH₂OCH₂-.

In a preferred embodiment, the JAK-2 inhibitor is pacritinib. Pacritinib is also known as SB1518. In a preferred embodiment, the JAK-2 inhibitor is (*E*)-4⁴-(2-(pyrrolidin-1-yl)ethoxy)-6,11-dioxa-3-aza-2(4,2)-pyrimidina-1,4(1,3)-dibenzenacyclododecaphan-8-ene. In a preferred embodiment, the JAK-2 inhibitor is 14,19-dioxa-5,7,27-triazatetracyclo[19.3.1.1^{2,6}.1^{8,12}]heptacosa-1(25),2,4,6(27),8,10,12(26),16,21,23-decaene, 11-[2-(1-pyrrolidinyl)ethoxy]-, (16E)-. In a preferred embodiment, the JAK-2 inhibitor is (16E)-11-[2-(pyrrolidin-1-yl)ethoxy]-14,19-dioxa-5,7,27-triazatetracyclo[19.3.1.1^{2,6}.1^{8,12}]heptacosa-1(24),2,4,6,8,10,12(26),16,21(25),22-decaene. In an embodiment, the JAK-2 inhibitor is a compound of Formula (LIV): 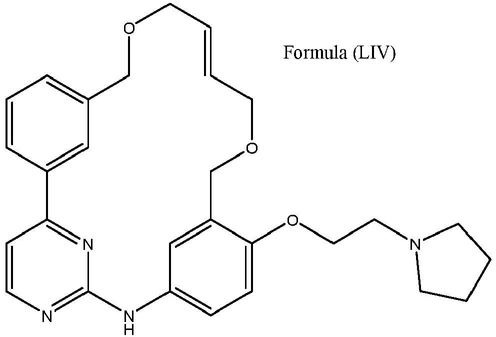 or a pharmaceutically acceptable salt, solvate, or hydrate thereof. In an embodiment, the structure of Formula (LIV) may be a tautomeric form. The preparation of Formula (LIV) is described in U.S. Patent Nos. 8,143,255; 8,153,632; and, 8,415,338 and U.S. Patent Application Publication Nos. 2009/0258886 A1; 2012/0142680 A1; 2012/0196855 A1; and 2013/0172338 A1. The preparation and properties of this JAK-2 inhibitor are known to those of ordinary skill in the art, and for example are described in: Hart, et al., SB1518, a novel macrocyclic pyrimidine-based JAK2 inhibitor for the treatment of myeloid and lymphoid malignancies, Leukemia 2011, 25, 1751-1759; Hart, et al., Pacritinib (SB1518), a JAK2/FLT3 inhibitor for the treatment of acute myeloid leukemia, Blood Cancer J., 2011, 1(11), e44; William, et al., Discovery of the macrocycle 11-(2-pyrrolidin-1-yl-ethoxy)-14,19-dioxa-5,7,26-triaza-tetracyclo[19.3.1.1(2,6).1(8,12)]heptacosa-1(25),2(26),3,5,8,10,12(27),16,21,23-decaene (SB1518), a potent Janus kinase 2/fms-like tyrosine kinase-3 (JAK2/FLT3) inhibitor for the treatment of myelofibrosis and lymphoma. J. Med. Chem. 2011, 54, 4638-4658; Poulsen, et al. Structure-based design of oxygen-linked macrocyclic kinase inhibitors: discovery of SB1518 and SB1578, potent inhibitors of Janus kinase 2 (JAK2) and Fms-like tyrosine kinase-3 (FLT3). J. Comput. AidedMol. Des. 2012, 26, 437-450.

In an embodiment, the JAK-2 inhibitor is selected from the structures disclosed in U.S. Patent Nos. 8,143,255; 8,153,632; and 8,415,338 and U.S. Patent Application Publication Nos. 2009/0258886 A1; 2012/0142680 A1; 2012/0196855 A1; and 2013/0172338 A1.

In a preferred embodiment, the JAK-2 inhibitor is (*E*)-4⁴-(2-(pyrrolidin-1-yl)ethoxy)-6,11-dioxa-3-aza-2(4,2)-pyrimidina-1(2,5)-furana-4(1,3)-benzenacyclododecaphan-8-ene. In a preferred embodiment, the JAK-2 inhibitor is (9E)-15 -(2-(pyrrolidin-1 -yl)ethoxy)-7,12,25-trioxa-19,21,24-triaza-tetracyclo[18.3.1.1(2,5).1(14,18)]hexacosa-1(24),2,4,9,14(26),15,17,20,22-nonaene. In a preferred embodiment, the JAK-2 inhibitor is a compound of Formula (LIV-A): 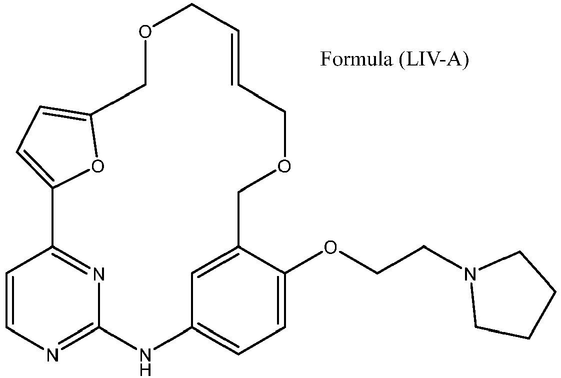 or a pharmaceutically acceptable salt, solvate, or hydrate thereof. The preparation and properties of this JAK-2 inhibitor are known to those of ordinary skill in the art, and for example are described in: Madan, et α/., SB1578, a novel inhibitor of JAK2, FLT3, and c-Fms for the treatment of rheumatoid arthritis, J. Immunol. 2012,189, 4123-4134 and William et al., Discovery of the macrocycle (9E)-15-(2-(pyrrolidin-1-yl)ethoxy)-7,12,25-trioxa-19,21,24-triaza-tetracyclo[18.3.1.1(2,5).1(14,18)]hexacosa-1(24),2,4,9,14(26),15,17,20,22-nonaene (SB1578), a potent inhibitor of janus kinase 2/fms-like tyrosine kinase-3 (JAK2/FLT3) for the treatment of rheumatoid arthritis. J. Med. Chem. 2012, 55, 2623-2640.

In an embodiment, the JAK-2 inhibitor is a compound selected from the structures disclosed in U.S. Patent No. 8,349,851 and U.S. Patent Application Publication Nos. 2010/0317659 A1, 2013/0245014, 2013/0296363 A1. In an embodiment, the JAK-2 inhibitor is a compound of Formula (LV): 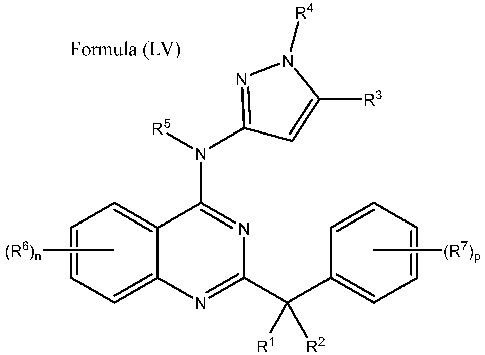 or a pharmaceutically acceptable salt, solvate, or hydrate thereof, wherein R¹ and R² are selected from (i), (ii), (iii), (iv), and (v) as follows:
(i) R¹ and R² together form =O, =S, NR⁹ or =CR¹⁰R¹¹;
(ii) R¹ and R² are both -OR⁸, or R¹ and R², together with the carbon atom to which they are attached, form dioxacycloalkyl;
(iii) R¹ is hydrogen or halo; and R² is halo; and
(iv) R¹ is alkyl, alkenyl, alkynyl, cycloalkyl or aryl, wherein the alkyl, alkenyl, alkynyl, cycloalkyl and aryl is optionally substituted with one or more substituents selected from halo, cyano, alkyl, -R^{x}OR^{w}, -R^{x}S(O)_{q}R^{v}, -R^{x}NR^{y}R^{z}and -C(O)OR^{w}; and R² is halo or -OR⁸; and
(v) R¹ is halo, deutero, -OR¹², -NR¹³R¹⁴, or -S(O)_{q}R¹⁵; and R² is hydrogen, deutero, alkyl, alkenyl, alkynyl, cycloalkyl or aryl, wherein the alkyl, alkenyl, alkynyl, cycloalkyl and aryl, is optionally substituted with one or more substitutents selected from halo, cyano, alkyl, -R^{x}OR^{w}, -R^{x}S(O)_{q}R^{v} and -R^{x}NR^{y}R^{z};
   R³ is hydrogen, halo, alkyl, cyano, haloalkyl, cycloalkyl, cycloalkylalkyl, hydroxy or alkoxy;
   R⁴ and R⁵ are each independently hydrogen or alkyl;
   each R⁶ is independently selected from halo, alkyl, alkenyl, alkynyl, haloalkyl, cycloalkyl, -R^{x}OR¹⁸, -R^{x}NR¹⁹R²⁰, and -R^{x}S(O)_{q}R^{v};
   each R⁷ is independently halo, alkyl, haloalkyl or -R^{x}OR^{w};
   R⁸ is alkyl, alkenyl or alkynyl;
   R⁹ is hydrogen, alkyl, haloalkyl, hydroxy, alkoxy or amino;
   R¹⁰ is hydrogen or alkyl;
   R¹¹ is hydrogen, alkyl, haloalkyl or -C(O)OR⁸;
   R¹² is selected from hydrogen, alkyl, alkenyl, alkynyl, cycloalkyl, cycloalkylalkyl, heterocyclyl, heterocyclylalkyl, aryl, aralkyl, heteroaryl, heteroaralkyl, -C(O)R^{v}, - C(O)OR^{w} and -C(O)NR^{y}R^{z}, wherein the alkyl, alkenyl, alkynyl, cycloalkyl, cycloalkylalkyl, heterocyclyl, heterocyclylalkyl, aryl, aralkyl, heteroaryl and heteroaralkyl are each optionally substituted with one or more substituents independently selected from halo, oxo, alkyl, hydroxy, alkoxy, amino and alkylthio;
   R¹³ and R¹⁴ are selected as follows:
      (i) R¹³ is hydrogen or alkyl; and R¹⁴ is selected from hydrogen, alkyl, alkenyl, alkynyl, cycloalkyl, cycloalkylalkyl, heterocyclyl, heterocyclylalkyl, aryl, aralkyl, heteroaryl, heteroaralkyl, alkoxy, -C(O)R^{v}, -C(O)OR^{w}, -C(O)NR^{y}R^{z} and -S(O)_{q}R^{v}, wherein the alkyl, alkenyl, alkynyl, cycloalkyl, cycloalkylalkyl, heterocyclyl, heterocyclylalkyl, aryl, aralkyl, heteroaryl and heteroaralkyl are each optionally substituted with one or more substituents independently selected from halo, oxo, alkyl, hydroxy, alkoxy, amino and alkylthio; or
      (ii) R¹³ and R¹⁴, together with the nitrogen atom to which they are attached, form heterocyclyl or heteroaryl wherein the heterocyclyl or heteroaryl is optionally substituted with one or more substituents independently selected from halo, alkyl, hydroxy, alkoxy, amino and alkylthio and wherein the heterocyclyl is also optionally substituted with oxo;
   R¹⁵ is alkyl, alkenyl, alkynyl, cycloalkyl, cycloalkylalkyl, heterocyclyl, heterocyclylalkyl, aryl, aralkyl, heteroaryl, heteroaralkyl, -C(O)NR^{y}R^{z} or -NR^{y}R^{z}, wherein the alkyl, alkenyl, alkynyl, cycloalkyl, cycloalkylalkyl, heterocyclyl, heterocyclylalkyl, aryl, aralkyl, heteroaryl and heteroaralkyl are each optionally substituted with one or more substituents independently selected from halo, oxo, alkyl, hydroxy, alkoxy, amino and alkylthio;
   R¹⁸ is hydrogen, alkyl, haloalkyl, hydroxy(C₂₋₆)alkyl, alkenyl, alkynyl, cycloalkyl, cycloalkylalkyl, heterocyclyl, heterocyclylalkyl, aryl, aralkyl, heteroaryl or heteroarylalkyl; wherein R¹⁸ is optionally substituted with 1 to 3 groups Q¹, each Q¹ independently selected from alkyl, hydroxyl, halo, haloalkyl, alkoxy, aryloxy, alkoxyalkyl, alkoxycarbonyl, alkoxysulfonyl, hydroxycarbonyl, cycloalkyl, heterocyclyl, aryl, heteroaryl, haloaryl and amino;
   R¹⁹ and R²⁰ are selected as follows:
      (i) R¹⁹ and R²⁰ are each independently hydrogen or alkyl; or
      (ii) R¹⁹ and R²⁰, together with the nitrogen atom to which they are attached, form a heterocyclyl or heteroaryl which is optionally substituted with 1 to 2 groups each independently selected from halo, alkyl, haloalkyl, hydroxyl and alkoxy;
   each R^{x} is independently alkylene or a direct bond;
   R^{v} is hydrogen, alkyl, alkenyl or alkynyl;
   R^{w} is independently hydrogen, alkyl, alkenyl, alkynyl or haloalkyl;
   R^{y} and R^{z} are selected as follows:
      (i) R^{y} and R^{z} are each independently hydrogen, alkyl, alkenyl, alkynyl, cycloalkyl or haloalkyl;
      (ii) R^{y} and R^{z}, together with the nitrogen atom to which they are attached, form a heterocyclyl or heteroaryl which is optionally substituted with 1 to 2 groups each independently selected from halo, alkyl, haloalkyl, hydroxyl and alkoxy;
   n is 0-4;
   p is 0-5; and
   each q is independently 0, 1 or 2.

In a preferred embodiment, the JAK-2 inhibitor is AC-410 (available from Ambit Biosciences). In a preferred embodiment, the JAK-2 inhibitor is (*S*)-(4-fluorophenyl)(4-((5-methyl-1*H-*pyrazol-3-yl)amino)quinazolin-2-yl)methanol. In a preferred embodiment, the JAK-2 inhibitor is a compound of Formula (LVI): 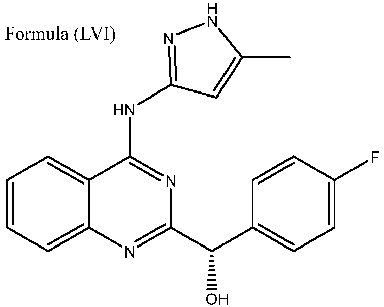 or a pharmaceutically acceptable salt, solvate, or hydrate thereof. The preparation of racemic (4-fluorophenyl)(4-((5-methyl-1*H*-pyrazol-3-yl)amino)quinazolin-2-yl)methanol hydrochloride is described in Examples 3 and 12 of U.S. Patent No. 8,349,851. Other preparation methods known to one of skill in the art also may be used. The preparation of the compound of Formula (LVI) is also described in the following paragraphs.

The preparation of (4-fluorophenyl)(4-(5-methyl-1*H-*pyrazol-3-ylamino)quinazolin-2-yl)methanone is accomplished by the following two steps (A and B). Step A: To a solution of ethyl 4-chloroquinazoline-2-carboxylate (0.6 g, 2.53 mmol) in THF (6 mL) at -40 °C, was added dropwise a 1 M solution of 4-fluorophenylmagnesium bromide in THF (3 mL, 3.0 mmol, 1.2 eq). The mixture was stirred at -40 °C for 4 h. The reaction was quenched by adding 0.5 N HCl solution (5 mL) and the mixture was extracted with EtOAc (2 × 10 mL). The combined organic layers were washed with brine and dried over MgSO₄. The crude product was purified on a silica gel column using a mixture of EtOAc-hexanes as eluent. (4-chloroquinazoline-2-yl)(4-fluorophenyl)methanone was obtained as a light yellow solid (440 mg, 60%). ¹H NMR (300 MHz, DMSO-d6) δ 7.45-740 (m, 2H), 8.07-8.03 (m, 1H), 8.17-8.13 (m, 2H), 8.23 (m, 2H), 8.42 (d, 1H); LC-MS (ESI) m/z 287 (M+H)⁺. Step B: To a solution of (4-chloroquinazolin-2-yl)(4-fluorophenyl)methanone (84 mg, 0.30 mmol) in DMF (3mL) were added DIEA (0.103 mL, 0.6 mmol) and 5-methyl-1*H*-pyrazol-3-amine (88 mg, 0.9 mmol at rt. The reaction mixture was heated at 40 °C overnight. The reaction was quenched by adding water and the yellow precipitate was collected by filtration and washed with water. The crude product was purified by silica gel chromatography eluting with DCM/MeOH to give (4-fluorophenyl)(4-(5-methyl-1*H*-pyrazol-3-ylamino)quinazolin-2-yl)methanone (30 mg, 29%). ¹H NMR (300 MHz, DMSO-d6) δ 2.19 (s, 3H), 6.54 (s, 1H), 7.40 (m, 2H), 7.68 (t, 1H), 7.9-7.7 (m, 2H), 8.08 (m, 2H), 8.74 (d, 1H), 10.66 (s, 1H), 12.20 (s, 1H);LC-MS (ESI) m/z 348 (M+H)⁺.

To a solution of 4-fluorophenyl)(4-(5-methyl-1*H*-pyrazol-3-ylamino)quinazolin-2-yl)methanone (60 mg, 0.172 mmol) in 1:1 MeOH/THF (10 mL) at 0 °C, was added NaBH₄ (64 mg, 1.69 mmol). The reaction mixture was stirred at 0° C. for 1.5 h. The reaction mixture was quenched by adding a few drops of acetone and concentrated to dryness. The crude solid was purified on HPLC to afford (4-fluorophenyl)(4-(5-methyl-1*H*-pyrazol-3-ylamino)quinazolin-2-yl)methanol (18 mg, 30%); ¹H NMR (300 MHz, DMSO-d6) δ 2.25 (s, 3H), 5.67 (s, 1H), 5.83 (bs, 1H), 6.40 (bs, 1H), 7.13 (m, 2H), 7.55-7.53 (m, 3H), 7.79 (s, 2H), 8.57 (bs, 1H), 10.43 (s, 1H), 12.12 (bs, 1H); LC-MS (ESI) m/z 350 (M+H)⁻.

To a suspension of (4-fluorophenyl)(4-(5-methyl-1*H*-pyrazol-3-ylamino)quinazolin-2-yl)methanone (2.3 g) in 30% MeOH/DCM (60 mL) at 0 °C was added dropwise 4M HCl/1,4-dioxane (10 mL). After all solid material had dissolved, the mixture was concentrated under reduced pressure, and to the residue was added 30% CH₃CN/H₂O (80 mL) and the mixture was sonicated until all solid material had dissolved. The mixture was frozen and lyophilized overnight to afford (4-fluoropheny1)(4-(5-methyl-1*H*-pyrazol-3-ylamino)quinazolin-2-yl)methanol hydrochloride (100%). ¹H NMR (300 MHz, DMSO-d6) δ 2.25 (s, 3H), 6.02 (s, 1H), 6.20 (s, 1H), 7.27 (t, 2H), 7.60 (qt, 2H), 7.80 (t, 1H), 8.08 (t, 1H), 8.23 (d, 1H), 8.83 (d, 1H), 12.16 (s, 1H), 14.51 (b, 1H); LC-MS (ESI) m/z 350 (M+H)⁺. The compound of Formula (LVI), (*S*)-(4-fluorophenyl)(4-((5-methyl-1*H*-pyrazol-3-yl)amino)quinazolin-2-yl)methanol, may be obtained from this preparation by chiral liquid chromatographic separation of the enantiomers, or by other well known techniques for resolution of enantiomers, such as those described in: Eliel et al., Stereochemistry of Organic Compounds, Wiley-Interscience, New York, 1994.

In a preferred embodiment, the JAK-2 inhibitor is (*R*)-(4-fluorophenyl)(4-((5-methyl-1*H*-pyrazol-3-yl)amino)quinazolin-2-yl)methanol, which is also known in the art to be active as a JAK-2 inhibitor. In a preferred embodiment, the JAK-2 inhibitor is racemic (4-fluorophenyl)(4-((5-methyl-1*H*-pyrazol-3-yl)amino)quinazolin-2-yl)methanol, which is also known in the art to be active as a JAK-2 inhibitor.

In some preferred embodiments, JAK-2 inhibitors having Formula (LV) or Formula (LVI) can be prepared, isolated, or obtained by any method known to one of skill in the art, including synthesis from a suitable optically pure precursor, asymmetric synthesis from an achiral starting material, or resolution of a racemic or enantiomeric mixture, for example, chiral chromatography, recrystallization, resolution, diastereomeric salt formation, or derivatization into diastereomeric adducts followed by separation.

A method for preparation of the compound of Formula (LVI)comprises resolving racemic (4-fluorophenyl)(4-(5-methyl-1*H*-pyrazol-3-ylamino)quinazolin-2-yl)methanol with chiral chromatography. In certain embodiments, the two individual enantiomers are separated using a chiral column, wherein the stationary phase is silica gel coated with a chiral selector such as tris-(3,5-dimethylphenyl)carbamoyl cellulose.

A method for preparation of the compound of Formula (LVI) comprises the step of reducing the achiral ketone (4-fluorophenyl)(4-(5-methyl-1*H*-pyrazol-3-ylamino)quinazolin-2-yl)methanone, prepared as described above or by other methods known to one of skill in the art, with hydrogen in the present of a chiral catalyst. The achiral ketone (4-fluorophenyl)(4-(5-methyl-1*H*-pyrazol-3-ylamino)quinazolin-2-yl)methanone may be reduced to predominantly a single enantiomeric product with a chiral reducing system of "type A" or "type B," wherein type A and type B differ from each other solely by having chiral auxiliaries of opposite chiralities. In certain embodiments, the chiral catalyst is [(S)-P-Phos RuCl₂(S)-DAIPEN].

The reduction of the achiral ketone (4-fluorophenyl)(4-(5-methyl-1*H*-pyrazol-3-ylamino)quinazolin-2-yl)methanone in presence of a chiral catalyst may be carried out in isopropyl alcohol as a solvent. The reduction of achiral ketone (4-fluorophenyl)(4-(5-methyl-1*H*-pyrazol-3-ylamino)quinazolin-2-yl)methanone in the presence of a chiral catalyst is carried out in isopropyl alcohol and water mixture as a solvent. Isopropyl alcohol and water are used in a ratio of 1:1, 8:1 or 9:1. DMSO is used as a cosolvent in the reaction. Alternatively, DMSO is used in amounts of 10, 20 or 30% based on the total amount of isopropyl alcohol and water mixture. Alternatively, isopropyl alcohol, DMSO and water are used in a ratio of 1:1:1, 4:4:0.5, 8:1:1, 47:47:6, 41:58:1, 44:50:6, or 18:79:3. Alternatively, isopropyl alcohol, DMSO and water are used in a ratio of 41:58:1. Alternatively, isopropyl alcohol, and DMSO are used in a ratio of 1:1. Alternatively, the reduction is carried out in presence of a base, such as potassium hydroxide, potassium tert butoxide and others. Alternatively, the base is used in 2-15 mol %, in one embodiment, 2 mol %, 5 mol %, 10 mol %, 12.5 mol % or 15 mol %. Alternatively, the reduction is carried out at a temperature of 40-80 °C, in one embodiment, 40 °C, 50 °C, 60 °C, 70 °C or 80 °C. Alternatively, the reduction is carried out at a temperature of 70 °C. Alternatively, the reduction is carried out at a pressure of 4 bar to 30 bar, in one embodiment, 4, 5, 10, 15, 20, 25 or 30 bar. Alternatively, the reduction is carried out at a pressure of 4 bar. Alternatively, the catalyst loading in the reaction is 100/1, 250/1, 500/1, 1000/1, 2000/1, 3000/1, 4000/1, 5000/1, 7000/1, 10,0000/1 or 20,000/1. In certain embodiments, the catalyst loading in the reaction is 2000/1 or 4000/1.

A method for preparation of the compound of Formula (LVI)comprises the step of reducing the achiral ketone (4-fluorophenyl)(4-(5-methyl-1*H*-pyrazol-3-ylamino)quinazolin-2-yl)methanone with a ketoreductase (e.g., alcohol dehydrogenase). *See* Moore, et al., Acc. Chem. Res. 2007, 40, 1412-1419; Daussmann, et al., Engineering in Life Sciences 2006, 6, 125-129; Schlummer, et al., Specialty Chemicals Magazine 2008, 28, 48-49; Osswald, et al., Chimica Oggi 2007, 25(Suppl.), 16-18; and Kambourakis, et al., PharmaChem 2006, 5(9), 2-5.

An alternative method for preparation of the compound of Formula (LVI) comprises the step of reducing the achiral ketone (4-fluorophenyl)(4-(5-methyl-1*H-*pyrazol-3-ylamino)quinazolin-2-yl)methanone with a reducing reagent (e.g., borane or borohydride reagents) in the presence of a chiral catalyst. In certain embodiments, the reducing agent is borane or a borohydride reagent. In certain embodiments, the chiral catalyst is a chiral oxazaborolidine. Cory, et al., Tetrahedron Letters 1996, 37, 5675; Cho, Chem. Soc. Rev. 2009, 38, 443.

Another method for preparation of the compound of Formula (LVI) comprises the step of reducing the achiral ketone (4-fluorophenyl)(4-(5-methy1-1*H*-pyrazol-3-ylamino)quinazolin-2-yl)methanone via asymmetric hydrosilylation, as described in U.S. Patent Application Publication No. 2008/0269490.

Another method for preparation of the compound of Formula (LVI) comprises the step of reducing the achiral ketone (4-fluorophenyl)(4-(5-methy1-1*H*-pyrazol-3-ylamino)quinazolin-2-yl)methanone via transfer hydrogenation catalyzed by an iridium complex, as described in Malacea, et al., Coord. Chem. Rev. 2010, 254, 729-752.

The starting materials used in the synthesis of the compound of Formula (LVI) disclosed herein are either commercially available or can be prepared by a method known to one of skill in the art. For example, the achiral ketone (4-fluorophenyl)(4-(5-methyl-1*H*-pyrazol-3-ylamino)quinazolin-2-yl)methanone can be prepared according to the methods described in U.S. Patent Nos. 8,349,851, issued January 8, 2013, and 8,703,943, issued April 22, 2014.

In an embodiment, the JAK-2 inhibitor is a JAK-2 inhibitor described in U.S. Patent Application Publication No. US 2013/0225614 A1. In an embodiment, the JAK-2 inhibitor is a compound of Formula (LV-A): 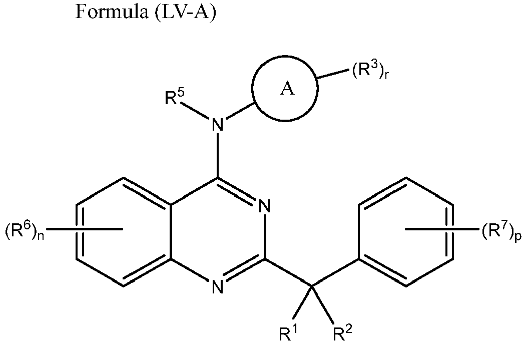 or a pharmaceutically acceptable salt, solvate, or hydrate thereof, wherein A is azolyl other than pyrazolyl;
R¹ and R² are selected from (i), (ii), (iii), (iv) and (v) as follows:
   (i) R¹ and R² together form =0, =S, =NR⁹ or =CR¹⁰Rⁿ;
   (ii) R¹ and R² are both -OR⁸, or R¹ and R², together with the carbon atom to which they are attached, form cycloalkyl or heterocyclyl wherein the cycloalkyl is substituted with one to four substituents selected from halo, deutero, alkyl, haloalkyl, -OR, -N(R )₂, and -S(O)_{q}R and wherein the heterocyclyl contains one to two heteroatoms wherein each heteroatom is independently selected from O, NR²⁴, S, S(O) and S(O)₂;
   (iii) R¹ is hydrogen or halo; and R² is halo;
   (iv) R¹ is alkyl, alkenyl, alkynyl, cycloalkyl or aryl, wherein the alkyl, alkenyl, alkynyl, cycloalkyl and aryl are each optionally substituted with one to four substitutents selected from halo, deutero, alkyl, cycloalkyl, heterocyclyl, aryl, heteroaryl, cyano, =0, =N-OR²¹, -R^{x}OR²¹, -R^{X}N(R²²)₂, -R^{x}S(O)_{q}R²³, -C(O)R²¹, - C(O)OR²¹ and - C(O)N(R²²)₂ ; and
   (v) R¹ is halo, deutero, -OR¹², -NR¹³R¹⁴, or -S(O)_{q}R¹⁵; and R² is hydrogen, deutero, alkyl, alkenyl, alkynyl, cycloalkyl or aryl, wherein the alkyl, alkenyl, alkynyl, cycloalkyl and aryl are each optionally substituted with one to four substitutents selected from halo, cyano, alkyl, -R^{x}OR^{w}, -R^{x}S(O)_{q}R^{v} and -R^{x}NR^{y}R^{z};
R³ is hydrogen, deutero,halo, alkyl, cyano, haloalkyl, deuteroalkyl, cycloalkyl, cycloalkylalkyl, hydroxy or alkoxy;
R⁵ is hydrogen or alkyl; each R⁶ is independently selected from halo, alkyl, alkenyl, alkynyl, haloalkyl, cycloalkyl, -R^{x}OR¹⁸, -R^{x}NR¹⁹R²⁰, and -R^{x}S(O)_{q}R^{v};
each R⁷ is independently halo, alkyl, haloalkyl or -R^{x}OR^{w};
R is alkyl, alkenyl or alkynyl;
R⁹ is hydrogen, alkyl, haloalkyl, hydroxy, alkoxy or amino;
R¹⁰ is hydrogen or alkyl;
R¹¹ is hydrogen, alkyl, haloalkyl or -C(O)OR⁸;
R¹² is selected from hydrogen, alkyl, alkenyl, alkynyl, cycloalkyl, cycloalkylalkyl, heterocyclyl, heterocyclylalkyl, aryl, aralkyl, heteroaryl, heteroaralkyl, -C(O)R^{v}, - C(O)OR^{w} and -C(O)NR^{y}R^{z}, wherein the alkyl, alkenyl, alkynyl, cycloalkyl, cycloalkylalkyl, heterocyclyl, heterocyclylalkyl, aryl, aralkyl, heteroaryl and heteroaralkyl are each optionally substituted with one or more, in one embodiment, one to four, in one embodiment, one to three, in one embodiment, one, two or three, substituents independently selected from halo, oxo, alkyl, hydroxy, alkoxy, amino and alkylthio;
R¹³ and R¹⁴ are selected as follows:
   (i) R¹³ is hydrogen or alkyl; and R¹⁴ is selected from hydrogen, alkyl, alkenyl, alkynyl, cycloalkyl, cycloalkylalkyl, heterocyclyl, heterocyclylalkyl, aryl, aralkyl, heteroaryl, heteroaralkyl, alkoxy, -C(O)R^{v}, -C(O)OR^{w}, -C(O)NR^{y}R^{z} and -S(O)_{q}R^{v}, wherein the alkyl, alkenyl, alkynyl, cycloalkyl, cycloalkylalkyl, heterocyclyl, heterocyclylalkyl, aryl, aralkyl, heteroaryl and heteroaralkyl are each optionally substituted with one or more, in one embodiment, one to four, in one embodiment, one to three, in one embodiment, one, two or three, substituents independently selected from halo, oxo, alkyl, hydroxy, alkoxy, amino and alkylthio; or
   (ii) R¹³ and R¹⁴, together with the nitrogen atom to which they are attached, form heterocyclyl or heteroaryl wherein the heterocyclyl or heteroaryl are substituted with one or more, in one embodiment, one to four, in one embodiment, one to three, in one embodiment, one, two or three, substituents independently selected from halo, alkyl, hydroxy, alkoxy, amino and alkylthio and wherein the heterocyclyl is optionally substituted with oxo; R¹⁵ is alkyl, alkenyl, alkynyl, cycloalkyl, cycloalkylalkyl, heterocyclyl, heterocyclylalkyl, aryl, aralkyl, heteroaryl, heteroaralkyl, -C(O)NR^{y}R^{z} or -NR^{y}R^{z}, wherein the alkyl, alkenyl, alkynyl, cycloalkyl, cycloalkylalkyl, heterocyclyl, heterocyclylalkyl, aryl, aralkyl, heteroaryl and heteroaralkyl are each optionally substituted with one or more, in one embodiment, one to four, in one embodiment, one to three, in one embodiment, one, two or three, substituents independently selected from halo, oxo, alkyl, hydroxy, alkoxy, amino and alkylthio;
R¹⁸ is hydrogen, alkyl, haloalkyl, hydroxyalkyl, alkenyl, alkynyl, cycloalkyl, cycloalkylalkyl, heterocyclyl, heterocyclylalkyl, aryl, aralkyl, heteroaryl or heteroarylalkyl; wherein R¹⁸ is optionally substituted with 1 to 3 groups Q¹, each Q¹ independently selected from alkyl, hydroxyl, halo, oxo, haloalkyl, alkoxy, aryloxy, alkoxyalkyl, alkoxycarbonyl, alkoxysulfonyl, carboxyl, cycloalkyl, heterocyclyl, aryl, heteroaryl, haloaryl and amino;
R¹⁹ and R²⁰ are selected as follows:
   (i) R¹⁹ and R²⁰ are each independently hydrogen or alkyl; or
   (ii) R¹⁹ and R²⁰, together with the nitrogen atom to which they are attached, form a heterocyclyl or heteroaryl which are each optionally substituted with 1 to 2 groups each independently selected from halo, oxo, alkyl, haloalkyl, hydroxyl and alkoxy;
R²¹ is hydrogen, alkyl, alkenyl, alkynyl, haloalkyl or cycloalkyl;
each R²² is independently hydrogen, alkyl, alkenyl, alkynyl, haloalkyl or cycloalkyl; or both R²², together with the nitrogen atom to which they are attached, form a heterocyclyl optionally substituted with oxo;
R²³ is alkyl, alkenyl, alkynyl or haloalkyl;
R²⁴ is hydrogen or alkyl;
each R^{x} is independently alkylene or a direct bond;
R^{v} is hydrogen, alkyl, alkenyl or alkynyl;
R^{w} is independently hydrogen, alkyl, alkenyl, alkynyl or haloalkyl;
R^{y} and R^{Z} are selected as follows:
   (i) R^{y} and R^{z} are each independently hydrogen, alkyl, alkenyl, alkynyl, cycloalkyl or haloalkyl; or
   (ii) R^{y} and R^{z}, together with the nitrogen atom to which they are attached, form a heterocyclyl or heteroaryl which are optionally substituted with 1 to 2 groups each independently selected from halo, alkyl, haloalkyl, hydroxyl and alkoxy;
n is 0-4;
p is 0-5;
each q is independently 0, 1 or 2; and
r is 1-3.

In an embodiment, the JAK-2 inhibitor of Formula (LV-A) is a compound of Formula (LV-B): 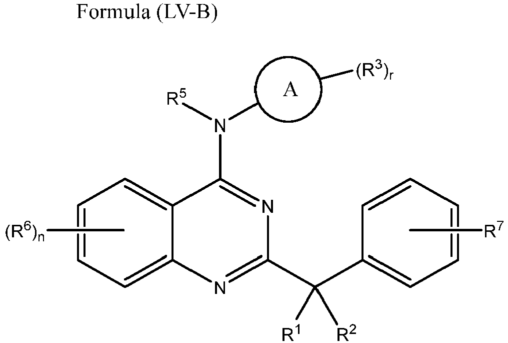 or a pharmaceutically acceptable salt, solvate, or hydrate thereof, wherein
A is imidazolyl, oxazolyl, thiazolyl, thiadiazolyl, or triazolyl;
R³ is hydrogen, alkyl, haloalkyl or cycloalkyl;
each R⁶ is independently selected from halo, alkyl, alkenyl, alkynyl, haloalkyl, cycloalkyl, -R^{x}OR¹⁸, -R^{x}NR¹⁹R²⁰, and -R^{x}S(O)_{q}R^{v};
R⁷ is halo;
R¹⁸ is hydrogen, alkyl, haloalkyl, hydroxyalkyl, alkenyl, alkynyl, cycloalkyl, cycloalkylalkyl, heterocyclyl, heterocyclylalkyl, aryl, aralkyl, heteroaryl or heteroarylalkyl; wherein R¹⁸ is optionally substituted with 1 to 3 groups Q¹, each Q¹ independently selected from alkyl, hydroxyl, halo, oxo, haloalkyl, alkoxy, aryloxy, alkoxyalkyl, alkoxycarbonyl, alkoxysulfonyl, carboxyl, cycloalkyl, heterocyclyl, aryl, heteroaryl, haloaryl and amino;
R¹⁹ and R²⁰ are selected as follows:
   (i) R¹⁹ and R²⁰ are each independently hydrogen or alkyl; or
   (ii) R¹⁹ and R²⁰, together with the nitrogen atom to which they are attached, form a heterocyclyl or heteroaryl which are each optionally substituted with 1 to 2 groups each independently selected from halo, oxo, alkyl, haloalkyl, hydroxyl and alkoxy;
each R^{x} is independently alkylene or a direct bond;
R^{v} is hydrogen, alkyl, alkenyl or alkynyl;
R^{y} and R^{z} are selected as follows:
   (i) R^{y} and R^{z} are each independently hydrogen, alkyl, alkenyl, alkynyl, cycloalkyl or haloalkyl; or
   (ii) R^{y} and R^{z}, together with the nitrogen atom to which they are attached, form a heterocyclyl or heteroaryl which are optionally substituted with 1 to 2 groups each independently selected from halo, alkyl, haloalkyl, hydroxyl and alkoxy;
n is 0-3;
each q is independently 0, 1 or 2; and
r is 1-3.

In a preferred embodiment of the JAK-2 inhibitor of Formula (LV-A) or (LV-B), R³ is hydrogen or alkyl.

In a preferred embodiment of the JAK-2 inhibitor of Formula (LV-A) or (LV-B), A is imidazolyl, oxazolyl, thiazolyl, thiadiazolyl, or triazolyl.

In a preferred embodiment of the JAK-2 inhibitor of Formula (LV-A) or (LV-B), R⁷ is fluro.

In a preferred embodiment, the JAK-2 inhibitor of Formula (LV-A) is a compound of Formula (LV-C): 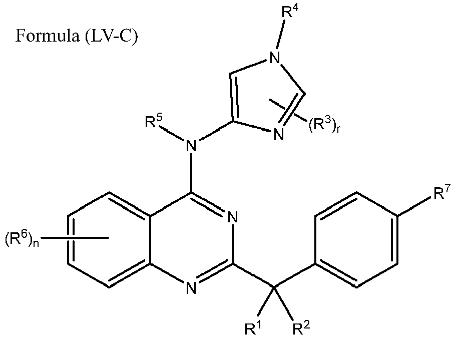 or a pharmaceutically acceptable salt, solvate, or hydrate thereof, where
R¹ and R² are selected as follows:
   (i) R¹ and R² together form =0;
   (ii) R¹ and R², together with the carbon atom to which they are attached, form dioxacycloalkyl or cycloalkyl wherein the cycloalkyl is substituted with one to four substituents selected from halo, deutero, alkyl, cycloalkyl, heterocyclyl, aryl, heteroaryl, cyano, =0, and hydroxy;
   (iii) R¹ is hydrogen or halo; and R² is halo;
   (iv) R¹ is alkyl, and R² is hydrogen, alkyl, halo, hydroxy or alkoxy; or
   (v) R¹ is halo, hydroxy or alkoxy; and R² is hydrogen or alkyl;
R³ is hydrogen, alkyl or cycloalkyl,
R⁴ is hydrogen or alkyl;
R⁵ is hydrogen or alkyl;
R⁷ is halo; and
n is 0-3.

In a preferred embodiment of the JAK-2 inhibitor of Formula (LV-C), n is 0.

In an embodiment, JAK-2 inhibitor of Formula (LV-A) has the structure of Formula (LV-D): 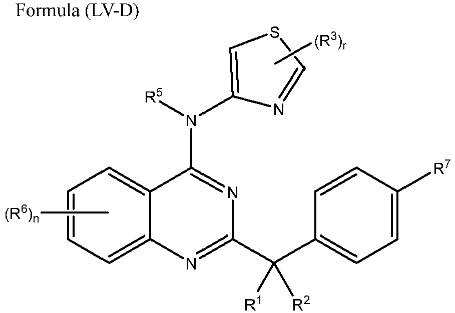 or a pharmaceutically acceptable salt, solvate, or hydrate thereof, where
R¹ and R² are selected as follows:
   (i) R¹ and R² together form =0;
   (ii) R¹ and R², together with the carbon atom to which they are attached, form dioxacycloalkyl or cycloalkyl wherein the cycloalkyl is substituted with one to four substituents selected from halo, deutero, alkyl, cycloalkyl, heterocyclyl, aryl, heteroaryl, cyano, =0, and hydroxy;
   (iii) R¹ is hydrogen or halo; and R² is halo;
   (iv) R¹ is alkyl, and R² is hydrogen, alkyl, halo, hydroxy or alkoxy; or
   (v) R¹ is halo, hydroxy or alkoxy; and R² is hydrogen or alkyl; R³ is hydrogen, alkyl or cycloalkyl,
R⁵ is hydrogen or alkyl;
R⁷ is halo; and
n is 0-3.

In a preferred embodiment of the JAK-2 inhibitor of Formula (LV-D), n is 0.

In a preferred embodiment, JAK-2 inhibitor of Formula (LV-D) is selected from the group consisting of:
(4-fluorophenyl)(4-((1-methyl-1*H*-imidazol-4-yl)amino)quinazolin-2-yl)methanol;
(4-((1*H*-imidazol-4-yl)amino)quinazolin-2-yl)(4-fluorophenyl)methanol;
(4-fluorophenyl)(4-(thiazol-4-ylamino)quinazolin-2-yl)methanol;
(4-fluorophenyl)(4-((5-methylthiazol-2-yl)amino)quinazolin-2-yl)methanol; and 2-(difluoro(4-fluorophenyl)methyl)-N-(1-methyl-1*H*-imidazol-4-yl)quinazolin-4-amine,
or a pharmaceutically acceptable salt, solvate, or hydrate thereof.

In an embodiment, the JAK-2 inhibitor is a compound of Formula (LVII): 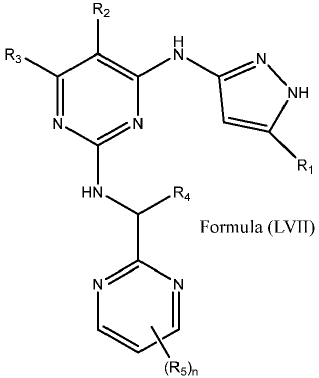 or a pharmaceutically acceptable salt, solvate, or hydrate thereof, wherein:
R¹ is selected from hydrogen, hydroxy, amino, mercapto, C₁₋₆alkyl, C₂₋₆alkenyl, C₂₋₆alkynyl, C₁₋₆alkoxy, C₁₋₆alkanoyloxy, N-(C₁₋₆alkyl)amino, N,N-(C₁₋₆alkyl)₂amino, C₁₋₆alkanoylamino, C₁₋₆alkylsulphonylamino, 3-5-membered carbocyclyl or 3-5-membered heterocyclyl; wherein R¹ may be optionally substituted on carbon by one or more R⁶; and wherein if said heterocyclyl contains an -NH-moiety that nitrogen may be optionally substituted by a group selected from R⁷;
R² and R³ are independently selected from hydrogen, halo, nitro, cyano, hydroxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)₂₋amino, C₁₋₆alkanoylamino, N-(C₁₋₆alkyl)carbamoyl, N,N-(C₁₋₆alkyl)₂carbamoyl, C₁₋₆alkylS(O)a wherein a is 0 to 2, C₁₋₆alkoxycarbonyl, N-(C₁₋₆alkyl)sulphamoyl, N,N-(C₁₋₆alkyl)₂sulphamoyl, (C₁₋₆alkyl)₂N-S(O)₂-NH-, (C₁₋₆alkyl)NH-S(O)₂-NH-, NH₂-S(O)₂-NH-, (C₁₋₆alkyl)₂N-S(O)₂-N(C₁₋₆alkyl)-, (C₁₋₆alkyl)NH--S(O)₂-⁻N(C₁₋₆alkyl)-, NH₂--S(O)₂--N(C₁₋₆alkyl)-, N-(C₁₋₆alkyl)-N-(C₁₋₆alkylsulphonyl)amino, C₁₋₆alkylsulphonylamino, carbocyclyl-R¹⁹- or heterocyclyl-R²¹; wherein R² and R³ independently of each other may be optionally substituted on carbon by one or more R⁸; and wherein if said heterocyclyl contains an -NH- moiety that nitrogen may be optionally substituted by a group selected from R⁹;
R⁴ is selected from cyano, carboxy, carbamoyl, C₁₋₆alkyl, C₂₋₆alkenyl, C₂₋₆alkynyl, C₁₋₆alkanoyl, N-(C₁₋₆alkyl)carbamoyl, N,N-(C₁₋₆alkyl)₂carbamoyl, C₁₋₆alkoxycarbonyl, carbocyclyl or heterocyclyl; wherein R⁴ may be optionally substituted on carbon by one or more R¹⁰; and wherein if said heterocyclyl contains an -NH- moiety that nitrogen may be optionally substituted by a group selected from R¹¹;
R⁵ is selected from halo, nitro, cyano, hydroxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)₂amino, C₁₋₆alkanoylamino, N-(C₁₋₆alkyl)carbamoyl, N,N-(C₁₋₆alkyl)₂carbamoyl, C₁₋₆alkylS(O)a wherein a is 0 to 2, C₁₋₆alkoxycarbonyl, N-(C₁₋₆alkyl)sulphamoyl, N,N-(C₁₋₆alkyl)₂sulphamoyl, C₁₋₆alkylsulphonylamino, carbocyclyl or heterocyclyl; wherein R⁵ may be optionally substituted on carbon by one or more R¹²; and wherein if said heterocyclyl contains an -NH- moiety that nitrogen may be optionally substituted by a group selected from R¹³;
n=0, 1, 2 or 3; wherein the values of R⁵ may be the same or different;
R⁶, R⁸, R¹⁰ and R¹² are independently selected from halo, nitro, cyano, hydroxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)₂amino, C₁₋₆alkanoylamino, N-(C₁₋₆alkyl)carbamoyl, N,N-(C₁₋₆alkyl)₂carbamoyl, C₁₋₆alkylS(O)ₐ wherein a is 0 to 2, C₁₋₆alkoxycarbonyl, N-(C₁₋₆alkyl)sulphamoyl, N,N-(C₁₋₆alkyl)₂sulphamoyl, C₁₋₆alkylsulphonylamino, carbocyclyl or heterocyclyl; wherein R⁶, R⁸, R¹⁰ and R¹² independently of each other may be optionally substituted on carbon by one or more R¹⁴; and wherein if said heterocyclyl contains an -NH- moiety that nitrogen may be optionally substituted by a group selected from R¹⁵;
R⁷, R⁹, R¹¹, R¹³ and R¹⁵ are independently selected from C₁₋₆alkyl, C₁₋₆alkanoyl, C₁₋₆alkylsulphonyl, C₁₋₆alkoxycarbonyl, carbamoyl, N-(C₁₋₆alkyl)carbamoyl, N,N-(C₁₋₆alkyl)carbamoyl, benzyl, benzyloxycarbonyl, benzoyl and phenylsulphonyl; wherein R⁷, R⁹, R¹¹, R¹³ and R¹⁵ independently of each other may be optionally substituted on carbon by on or more R¹⁶;
R¹⁴ and R¹⁶ are independently selected from halo, nitro, cyano, hydroxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)₂amino, C₁₋₆alkanoylamino, N-(C₁₋₆alkyl)carbamoyl, N,N-(C₁₋₆alkyl)₂carbamoyl, C₁₋₆alkylS(O)ₐ wherein a is 0 to 2, C₁₋₆alkoxycarbonyl, N-(C₁₋₆alkyl)sulphamoyl, N,N-(C₁₋₆alkyl)₂sulphamoyl, C₁₋₆alkylsulphonylamino, carbocyclyl or heterocyclyl; wherein R¹⁴ and R¹⁶ independently of each other may be optionally substituted on carbon by one or more R¹⁷; and wherein if said heterocyclyl contains an -NH-moiety that nitrogen may be optionally substituted by a group selected from R¹⁸;
R¹⁷ is selected from halo, nitro, cyano, hydroxy, trifluoromethoxy, trifluoromethyl, amino, carboxy, carbamoyl, mercapto, sulphamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulphinyl, ethylsulphinyl, mesyl, ethylsulphonyl, methoxycarbonyl, ethoxycarbonyl, N-methylsulphamoyl, N-ethylsulphamoyl, N,N-dimethylsulphamoyl, N,N-diethylsulphamoyl or N-methyl-N-ethylsulphamoyl; and
R¹⁹ and R²¹ are independently selected from a direct bond, --O--, -N(R²²)-, -C(O)-, -N(R²³)C(O)- -C(O)N(R²⁴)-, -S(O)ₛ-, -SO₂N(R²⁵)- or -N(R²⁶)SO₂-; wherein R²², R²³, R²⁴, R²⁵ and R²⁶ are independently selected from hydrogen or C₁₋₆alkyl and s is 0-2;
R¹⁸ is selected from C₁₋₆alkyl, C₁₋₆alkanoyl, C₁₋₆alkylsulphonyl, C₁₋₆alkoxycarbonyl, carbamoyl, N-(C₁₋₆alkyl)carbamoyl, N,N-(C₁₋₆alkyl)carbamoyl, benzyl, benzyloxycarbonyl, benzoyl and phenylsulphonyl;
or a pharmaceutically acceptable salt, solvate, or hydrate thereof.

In an embodiment, the JAK-2 inhibitor is a compound of Formula (LVII), wherein:
R¹ is selected from hydrogen, hydroxy, amino, mercapto, C₁₋₆alkyl, C₂₋₆alkenyl, C₂₋₆alkynyl, C₁₋₆alkoxy, C₁₋₆alkanoyloxy, N-(C₁₋₆alkyl)amino, N,N-(C₁₋₆alkyl)₂amino, C₁₋₆alkanoylamino, C₁₋₆alkylsulphonylamino, 3-5-membered carbocyclyl or 3-5-membered heterocyclyl; wherein R¹ may be optionally substituted on carbon by one or more R⁶; and wherein if said heterocyclyl contains an -NH-moiety that nitrogen may be optionally substituted by a group selected from R⁷;
R² and R³ are independently selected from hydrogen, halo, nitro, cyano, hydroxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)₂amino, C₁₋₆alkanoylamino, N-(C₁₋₆alkyl)carbamoyl, N,N-(C₁₋₆alkyl)₂carbamoyl, C₁₋₆alkylS(O)a wherein a is 0 to 2, C₁₋₆alkoxycarbonyl, N-(C₁₋₆alkyl)sulphamoyl, N,N-(C₁₋₆alkyl)₂sulphamoyl, C₁₋₆alkylsulphonylamino, carbocyclyl-R¹⁹- or heterocyclyl-R²¹-; wherein R² and R³ independently of each other may be optionally substituted on carbon by one or more R⁸; and wherein if said heterocyclyl contains an -NH- moiety that nitrogen may be optionally substituted by a group selected from R⁹;
R⁴ is selected from cyano, carboxy, carbamoyl, C₁₋₆alkyl, C₂₋₆alkenyl, C₂₋₆alkynyl, C₁₋₆alkanoyl, N-(C₁₋₆alkyl)carbamoyl, N,N-(C₁₋₆alkyl)₂carbamoyl, C₁₋₆alkoxycarbonyl, carbocyclyl or heterocyclyl; wherein R⁴ may be optionally substituted on carbon by one or more R¹⁰; and wherein if said heterocyclyl contains an -NH- moiety that nitrogen may be optionally substituted by a group selected from R¹¹;
R⁵ is selected from halo, nitro, cyano, hydroxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)₂amino, C₁₋₆alkanoylamino, N-(C₁₋₆alkyl)carbamoyl, N,N-(C₁₋₆alkyl)₂carbamoyl, C₁₋₆alkylS(O)a wherein a is 0 to 2, C₁₋₆alkoxycarbonyl, N-(C₁₋₆alkyl)sulphamoyl, N,N-(C₁₋₆alkyl)₂sulphamoyl, C₁₋₆alkylsulphonylamino, carbocyclyl or heterocyclyl; wherein R⁵ may be optionally substituted on carbon by one or more R²; and wherein if said heterocyclyl contains an -NH- moiety that nitrogen may be optionally substituted by a group selected from R¹³;
n=0, 1, 2 or 3; wherein the values of R⁵ may be the same or different;
R⁶, R⁸, R¹⁰ and R¹² are independently selected from halo, nitro, cyano, hydroxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)₂amino, C₁₋₆alkanoylamino, N-(C₁₋₆alkyl)carbamoyl, N,N-(C₁₋₆alkyl)₂carbamoyl, C₁₋₆alkylS(O)a wherein a is 0 to 2, C₁₋₆alkoxycarbonyl, N-(C₁₋₆alkyl)sulphamoyl, N,N-(C₁₋₆alkyl)₂sulphamoyl, C₁₋₆alkylsulphonylamino, carbocyclyl or heterocyclyl; wherein R⁶, R⁸, R¹⁰ and R¹² independently of each other may be optionally substituted on carbon by one or more R¹⁴; and wherein if said heterocyclyl contains an -NH- moiety that nitrogen may be optionally substituted by a group selected from R¹⁵;
R⁷, R⁹, R¹¹, R¹³ and R¹⁵ are independently selected from C₁₋₆alkyl, C₁₋₆alkanoyl, C₁₋₆alkylsulphonyl, C₁₋₆alkoxycarbonyl, carbamoyl, N-(C₁₋₆alkyl)carbamoyl, N,N-(C₁₋₆alkyl)carbamoyl, benzyl, benzyloxycarbonyl, benzoyl and phenylsulphonyl; wherein R⁷, R⁹, R¹¹, R¹³ and R¹⁵ independently of each other may be optionally substituted on carbon by on or more R¹⁶;
R¹⁴ and R¹⁶ are independently selected from halo, nitro, cyano, hydroxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)₂amino, C₁₋₆alkanoylamino, N-(C₁₋₆alkyl)carbamoyl, N,N-(C₁₋₆alkyl)₂carbamoyl, C₁₋₆alkylS(O)ₐ wherein a is 0 to 2, C₁₋₆alkoxycarbonyl, N-(C₁₋₆alkyl)sulphamoyl, N,N-(C₁₋₆alkyl)₂sulphamoyl, C₁₋₆alkylsulphonylamino, carbocyclyl or heterocyclyl; wherein R¹⁴ and R¹⁶ independently of each other may be optionally substituted on carbon by one or more R¹⁷; and wherein if said heterocyclyl contains an -NH-moiety that nitrogen may be optionally substituted by a group selected from R¹⁸;
R¹⁷ is selected from halo, nitro, cyano, hydroxy, trifluoromethoxy, trifluoromethyl, amino, carboxy, carbamoyl, mercapto, sulphamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulphinyl, ethylsulphinyl, mesyl, ethylsulphonyl, methoxycarbonyl, ethoxycarbonyl, N-methylsulphamoyl, N-ethylsulphamoyl, N,N-dimethylsulphamoyl, N,N-diethylsulphamoyl or N-methyl-N-ethylsulphamoyl; and
R¹⁹ and R²¹ are independently selected from -O-, -N(R²²)-, -C(O)-, - N(R²³)C(O)-, -C(O)N(R²⁴)-, -S(O)ₛ-, -SO₂N(R²⁵)- or -N(R²⁶)SO₂-; wherein R²², R²³, R²⁴, R²⁵ and R²⁶ are independently selected from hydrogen or C₁₋₆alkyl and s is 0-2;
R¹⁸ is selected from C₁₋₆alkyl, C₁₋₆alkanoyl, C₁₋₆alkylsulphonyl, C₁₋₆alkoxycarbonyl, carbamoyl, N-(C₁₋₆alkyl)carbamoyl, N,N-(C₁₋₆alkyl)carbamoyl, benzyl, benzyloxycarbonyl, benzoyl and phenylsulphonyl;
or a pharmaceutically acceptable salt, solvate, or hydrate thereof.

In an embodiment, the JAK-2 inhibitor is a compound of Formula (LVII), wherein:
R¹ is selected from hydrogen, hydroxy, amino, mercapto, C₁₋₆alkyl, C₂₋₆alkenyl, C₂₋₆alkynyl, C₁₋₆alkoxy, C₁₋₆alkanoyloxy, N-(C₁₋₆alkyl)amino, N,N-(C₁₋₆alkyl)₂amino, C₁₋₆alkanoylamino, C₁₋₆alkylsulphonylamino, 3-5-membered carbocyclyl or 3-5-membered heterocyclyl; wherein R¹ may be optionally substituted on carbon by one or more R⁶; and wherein if said heterocyclyl contains an -NH-moiety that nitrogen may be optionally substituted by a group selected from R⁷;
R² and R³ are independently selected from hydrogen, halo, nitro, cyano, hydroxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)₂amino, C₁₋₆alkanoylamino, N-(C₁₋₆alkyl)carbamoyl, N,N-(C₁₋₆alkyl)₂carbamoyl, C₁₋₆alkylS(O)a wherein a is 0 to 2, C₁₋₆alkoxycarbonyl, N-(C₁₋₆alkyl)sulphamoyl, N,N-(C₁₋₆alkyl)₂sulphamoyl, N-(C₁₋₆alkyl)-N-(C₁₋₆alkylsulphonyl)amino, C₁₋₆alkylsulphonylamino, carbocyclyl-R¹⁹- or heterocyclyl-R²¹-; wherein R² and R³ independently of each other may be optionally substituted on carbon by one or more R⁸; and wherein if said heterocyclyl contains an -NH-moiety that nitrogen may be optionally substituted by a group selected from R⁹;
R⁴ is selected from cyano, carboxy, carbamoyl, C₁₋₆alkyl, C₂₋₆alkenyl, C₂₋₆alkynyl, C₁₋₆alkanoyl, N-(C₁₋₆alkyl)carbamoyl, N,N-(C₁₋₆alkyl)₂carbamoyl, C₁₋₆alkoxycarbonyl, carbocyclyl or heterocyclyl; wherein R⁴ may be optionally substituted on carbon by one or more R¹⁰; and wherein if said heterocyclyl contains an -NH- moiety that nitrogen may be optionally substituted by a group selected from R¹¹;
R⁵ is selected from halo, nitro, cyano, hydroxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)₂amino, C₁₋₆alkanoylamino, N-(C₁₋₆alkyl)carbamoyl, N,N-(C₁₋₆alkyl)₂carbamoyl, C₁₋₆alkylS(O)ₐ wherein a is 0 to 2, C₁₋₆alkoxycarbonyl, N-(C₁₋₆alkyl)sulphamoyl, N,N-(C₁₋₆alkyl)₂sulphamoyl, C₁₋₆alkylsulphonylamino, carbocyclyl or heterocyclyl; wherein R⁵ may be optionally substituted on carbon by one or more R¹²; and wherein if said heterocyclyl contains an -NH- moiety that nitrogen may be optionally substituted by a group selected from R¹³;
n=0, 1, 2 or 3; wherein the values of R⁵ may be the same or different;
R⁶, R⁸, R¹⁰ and R¹² are independently selected from halo, nitro, cyano, hydroxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, N-(C₁₋₆alkyl)amino, N,N-(C₁₋₆alkyl)₂amino, C₁₋₆alkanoylamino, N-(C₁₋₆alkyl)carbamoyl, N,N-(C₁₋₆alkyl)₂carbamoyl, C₁₋₆alkylS(O)a wherein a is 0 to 2, C₁₋₆alkoxycarbonyl, N-(C₁₋₆alkyl)sulphamoyl, N,N-(C₁₋₆alkyl)₂sulphamoyl, C₁₋₆alkylsulphonylamino, carbocyclyl or heterocyclyl; wherein R⁶, R⁸, R¹⁰ and R¹² independently of each other may be optionally substituted on carbon by one or more R¹⁴; and wherein if said heterocyclyl contains an -NH- moiety that nitrogen may be optionally substituted by a group selected from R¹⁵;
R⁷, R⁹, R¹¹, R¹³ and R¹⁵ are independently selected from (C₁₋₆)alkyl, (C₁₋₆)alkanoyl, (C₁₋₆)alkylsulphonyl, (C₁₋₆)alkoxycarbonyl, carbamoyl, N-((C₁₋₆)alkyl)carbamoyl, N,N-((C₁₋₆)alkyl)carbamoyl, benzyl, benzyloxycarbonyl, benzoyl and phenylsulphonyl; wherein R⁷, R⁹, R¹¹, R¹³ and R¹⁵ independently of each other may be optionally substituted on carbon by on or more R¹⁶;
R¹⁴ and R¹⁶ are independently selected from halo, nitro, cyano, hydroxy, amino, carboxy, carbamoyl, mercapto, sulphamoyl, (C₁₋₆)alkyl, (C₂₋₆)alkenyl, (C₂₋₆)alkynyl, (C₁₋₆)alkoxy, (C₁₋₆)alkanoyl, (C₁₋₆)alkanoyloxy, N-((C₁₋₆)alkyl)amino, N,N-((C₁₋₆)alkyl)₂amino, (C₁₋₆)alkanoylamino, N-((C₁₋₆)alkyl)carbamoyl, N,N-((C₁₋₆)alkyl)₂carbamoyl, (C₁₋₆)alkylS(O)ₐ wherein a is 0 to 2, (C₁₋₆)alkoxycarbonyl, N-((C₁₋₆)alkyl)sulphamoyl, N,N-((C₁₋₆)alkyl)₂sulphamoyl, (C₁₋₆)alkylsulphonylamino, carbocyclyl or heterocyclyl; wherein R¹⁴ and R¹⁶ independently of each other may be optionally substituted on carbon by one or more R¹⁷; and wherein if said heterocyclyl contains an -NH- moiety that nitrogen may be optionally substituted by a group selected from R¹⁸;
R¹⁷ is selected from halo, nitro, cyano, hydroxy, trifluoromethoxy, trifluoromethyl, amino, carboxy, carbamoyl, mercapto, sulphamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulphinyl, ethylsulphinyl, mesyl, ethylsulphonyl, methoxycarbonyl, ethoxycarbonyl, N-methylsulphamoyl, N-ethylsulphamoyl, N,N-dimethylsulphamoyl, N,N-diethylsulphamoyl or N-methyl-N-ethylsulphamoyl; and
R¹⁹ and R²¹ are independently selected from a direct bond, -O-, -N(R²²)-, -C(O)-, -N(R²³)C(O)-, -C(O)N(R²⁴)-, -S(O)ₛ-, -SO₂N(R²⁵)- or -N(R²⁶)SO₂-; wherein R²², R²³, R²⁴, R²⁵ and R²⁶ are independently selected from hydrogen or (C₁₋₆)alkyl and s is 0-2;
R¹⁸ is selected from (C₁₋₆)alkyl, (C₁₋₆)alkanoyl, (C₁₋₆)alkylsulphonyl, (C₁₋₆)alkoxycarbonyl, carbamoyl, N((C₁₋₆)alkyl)carbamoyl, N,N-((C₁₋₆)alkyl)carbamoyl, benzyl, benzyloxycarbonyl, benzoyl and phenylsulphonyl;
or a pharmaceutically acceptable salt, solvate, or hydrate thereof.

Particular values of the variable groups contained in Formula (LVII) are as follows. Such values may be used, where appropriate, with any of the definitions, claims or embodiments defined hereinbefore or hereinafter in relation to compounds of Formula (LVII).
R¹ is selected from (C₁₋₆)alkyl, (C₁₋₆)alkoxy, 3-5-membered carbocyclyl, and N,N-((C₁₋₆)alkyl)₂amino, wherein R¹ may be optionally substituted on carbon by one or more R⁶; and wherein R⁶ is halo,
R¹ is (C₁₋₆)alkoxy or 3-5-membered carbocyclyl.
R¹ is selected from (C₁₋₆)alkyl, (C₁₋₆)alkoxy or 3-5-membered carbocyclyl.
R¹ is (C₁₋₆)alkyl or (C₁₋₆)alkoxy.
R¹ is 3-5 membered carbocyclyl.
R¹ is N,N((C₁₋₆)alkyl)₂amino.
R¹ is (C₁₋₆)alkyl.
R¹ is (C₁₋₄)alkyl.
R¹ is (C₁₋₆)alkoxy.
R¹ is selected from methyl, methoxy, trifluoroethoxy, isopropoxy, cyclopropyl, and N,N-dimethylamino;
R¹ is isopropoxy or cyclopropyl.
R¹ is methyl, methoxy, isopropoxy or cyclopropyl.
R¹ is selected from methyl, methoxy, isopropoxy, N,N-dimethylamino, and cyclopropyl. R¹ is isopropoxy.
R¹ is methyl.
R¹ is ethyl.
R¹ is selected from methyl, ethyl, propyl, and butyl.
R¹ is selected from (C₁₋₄)alkyl, (C₁₋₄)alkoxy, and cyclopropyl.
R¹ is methoxy.
R¹ is cyclopropyl. R¹ is N,N-dimethylamino.
R² is selected from hydrogen, halo, nitro, and (C₁₋₆)alkyl, wherein R² may be optionally substituted on carbon by one or more R⁸; and wherein R⁸ is halo.
R² is selected from hydrogen, chloro, fluoro, bromo, nitro, and trifluoromethyl.
R² is halo.
R² is (C₁₋₆)alkyl, wherein R² may be optionally substituted on carbon by one or more R⁸; and wherein R⁸ is halo.
R² and R³ are independently selected from hydrogen, halo, nitro, cyano, hydroxy, amino, carboxy, carbamoyl, mercapto, sulphamoyl, (C₁₋₆)alkyl, (C₂₋₆)alkenyl, (C₂₋₆)alkynyl, (C₁₋₆)alkoxy, (C₁₋₆)alkanoyl, (C₁₋₆)alkanoyloxy, N-((C₁₋₆)alkyl)amino, N,N-((C₁₋₆)alkyl)₂amino, (C₁₋₆)alkanoylamino, N((C₁₋₆)alkyl)carbamoyl, N,N-((C₁₋₆)alkyl)₂carbamoyl, (C₁₋₆)alkylS(O)a wherein a is 0 to 2, (C₁₋₆)alkoxycarbonyl, N-((C₁₋₆)alkyl)sulphamoyl, N,N--((C₁₋₆)alkyl)₂sulphamoyl, (C₁₋₆)alkylsulphonylamino, carbocyclyl-R¹⁹- or heterocyclyl-R²¹-; wherein R² and R³ independently of each other may be optionally substituted on carbon by one or more R⁸; and wherein if said heterocyclyl contains an -NH- moiety that nitrogen may be optionally substituted by a group selected from R⁹.
R² and R³ are independently selected from hydrogen, halo, nitro, cyano, hydroxy, amino, carboxy, carbamoyl, mercapto, sulphamoyl, (C₁₋₆)alkyl, (C₂₋₆)alkenyl, (C₂₋₆)alkynyl, (C₁₋₆)alkoxy, (C₁₋₆)alkanoyl, (C₁₋₆)alkanoyloxy, N-((C₁₋₆)alkyl)amino, N,N-((C₁₋₆)alkyl)₂amino, (C₁₋₆)alkanoylamino, N((C₁₋₆)alkyl)carbamoyl, N,N-((C₁₋₆)alkyl)₂carbamoyl, (C₁₋₆)alkylS(O)a wherein a is 0 to 2, (C₁₋₆)alkoxycarbonyl, N-()C₁₋₆₎alkyl)sulphamoyl, N,N-((C₁₋₆)alkyl)₂sulphamoyl, N-((C₁₋₆)alkyl)-N-((C₁₋₆)alkylsulphonyl)amino, (C₁₋₆)alkylsulphonylamino, carbocyclyl-R¹⁹- or heterocyclyl-R²¹-; wherein R² and R³ independently of each other may be optionally substituted on carbon by one or more R⁸; and wherein if said heterocyclyl contains an -NH- moiety that nitrogen may be optionally substituted by a group selected from R⁹.
R² and R³ are independently selected from hydrogen, halo, N-((C₁₋₆)alkyl)-N-((C₁₋₆)alkylsulphonyl)amino, or heterocyclyl-R²¹-; wherein R²¹ is a direct bond.
R² and R³ are independently selected from hydrogen and halo.
R² and R³ are independently selected from hydrogen and chloro.
R² and R³ are independently selected from hydrogen, fluoro, chloro, bromo, N-methyl-N-mesylamino and morpholino.
R² is halo and R³ is hydrogen.
R² is chloro and R³ is hydrogen.
R² is chloro or fluoro and R³ is hydrogen. R³ is selected from hydrogen, halo, cyano, N-((C₁₋₆)alkyl)-N--((C₁₋₆)alkylsulphonyl)amino, (C₁₋₆)alkyl, ((C₁₋₆)alkyl)₂N--S(O)₂-N((C₁₋₆)alkyl)-, and heterocyclyl-R²¹-, wherein R³ may be optionally substituted on carbon by one or more R⁸; wherein R⁸ is halo; and wherein R²¹ is a bond.
R³ is hydrogen.
R³ is halo.
R³ is selected from N--((C₁₋₆)alkyl)-N-((C₁₋₆)alkylsulphonyl)amino and ((C₁₋₆)alkyl)₂N-S(O)₂-N((C₁₋₆)alkyl)-.
R³ is selected from heterocyclyl-R²¹-, wherein R³ may be optionally substituted on carbon by one or more R⁵; wherein R⁵ is halo; and wherein R²¹ is a bond.
R³ is selected from hydrogen, chloro, cyano, trifluoromethyl, (CH₃)₂N-S(O)₂-N(CH₃)-, N-methyl-N-mesylamino, and morpholino.
R³ is (CH₃)₂N-S(O)₂-N(CH₃)-.
R³ is N-methyl-N-mesylamino,
R³ is morpholino,
R⁴ is (C₁₋₆)alkyl.
R⁴ is methyl.
R⁵ is halo.
R⁵ is fluoro.
n=1.
R¹⁹ and R²¹ are independently selected from -O-, -N(R²²)-, -C(O)-, - N(l²³)C(O)-, -C(O)N(R²⁴)-, -S(O)ₛ-, -SO₂N(R²⁵)- or -N(R²⁶)SO₂-; wherein R²², R²³, R²⁴, R²⁵ and R²⁶ are independently selected from hydrogen or (C₁₋₆)alkyl and s is 0-2.

Therefore in a further aspect there is provided a compound of Formula (LVII) (as depicted herein above) wherein:
R¹ is selected from (C₁₋₆)alkyl, (C₁₋₆)alkoxy or 3-5-membered carbocyclyl;
R¹ and R³ are independently selected from hydrogen, halo, N-((C₁₋₆)alkyl)-N-((C₁₋₆)alkylsulphonyl)amino, or heterocyclyl-R²¹-;
R⁴ is (C₁₋₆)alkyl;
R⁵ is halo;
n=1;
R²¹ is a direct bond;
or a pharmaceutically acceptable salt, solvate, or hydrate thereof.

Therefore, in an embodiment of the invention, the JAK-2 inhibitor is a compound of Formula (LVII) wherein:
R¹ is (C₁₋₆)alkoxy;
R² and R³ are independently selected from hydrogen and halo;
R⁴ is (C₁₋₆)alkyl;
R⁵ is halo;
n=1;
or a pharmaceutically acceptable salt, solvate, or hydrate thereof.

In an embodiment of the invention, the JAK-2 inhibitor is a compound of Formula (LVII) wherein:
R¹ is methyl, methoxy, isopropoxy or cyclopropyl;
R² and R³ are independently selected from hydrogen, fluoro, chloro, bromo, N-methyl-N-mesylamino and morpholino;
R⁴ is methyl;
R⁵ is fluoro; and
n=1;
or a pharmaceutically acceptable salt, solvate, or hydrate thereof.

In an embodiment of the invention, the JAK-2 inhibitor is a compound of Formula (LVII) wherein:
R¹ is selected from (C₁₋₆)alkyl, (C₁₋₆)alkoxy, 3-5-membered carbocyclyl, and N,N-((C₁₋₆)alkyl)₂amino, wherein R¹ may be optionally substituted on carbon by one or more R⁶;
R² is selected from hydrogen, halo, nitro, and (C₁₋₆)alkyl, wherein R² may be optionally substituted on carbon by one or more R⁸;
R³ is selected from hydrogen, halo, cyano, N-((C₁₋₆)alkyl)-N-((C₁₋₆)alkylsulphonyl)amino, (C₁₋₆)alkyl, ((C₁₋₆)alkyl)₂N-S(O)₂-N((C₁₋₆)alkyl)-, and heterocyclyl-R²¹-, wherein R³ may be optionally substituted on carbon by one or more R⁸;
R⁴ is (C₁₋₆)alkyl;
R⁵ is halo;
R⁶ is halo;
R⁸ is halo;
R²¹ is a bond; and
n=1;
or a pharmaceutically acceptable salt, solvate, or hydrate thereof.

In an embodiment of the invention, the JAK-2 inhibitor is a compound of Formula (LVII) wherein:
R¹ is selected from methyl, methoxy, trifluoroethoxy, isopropoxy, cyclopropyl, and N,N-dimethylamino;
R² is selected from hydrogen, chloro, fluoro, bromo, nitro, and trifluoromethyl;
R³ is selected from hydrogen, chloro, cyano, trifluoromethyl, (CH₃)₂N-S(O)₂-N(CH₃)-, N-methyl-N-mesylamino, and morpholino;
R⁴ is methyl;
R⁵ is fluoro; and
n is 1;
or a pharmaceutically acceptable salt, solvate, or hydrate thereof.

In an embodiment of the invention, the JAK-2 inhibitor is a compound of Formula (LVII) wherein:
R¹ is selected from (C₁₋₆)alkoxy, wherein R¹ may be optionally substituted on carbon by one or more R⁶;
R² is selected from hydrogen and halo;
R³ is selected from hydrogen, halo, and heterocyclyl-R²¹-;
R⁴ is (C₁₋₆)alkyl;
R⁵ is halo;
R⁶ is halo;
R²¹ is a bond;
n is 1;
or a pharmaceutically acceptable salt, solvate, or hydrate thereof.

In an embodiment of the invention, the JAK-2 inhibitor is a compound of Formula (LVII) wherein:
R¹ is selected from (C₁₋₄)alkyl, (C₁₋₄)alkoxy, and cyclopropyl;
R² is selected from hydrogen, halo, nitro, and (C₁₋₆)alkyl, wherein R² may be optionally substituted on carbon by one or more R⁸;
R³ is selected from hydrogen, halo, cyano, N-((C₁₋₆)alkyl)-N-((C₁₋₆)alkylsulphonyl)amino, (C₁₋₆)alkyl, ((C₁₋₆)alkyl)₂N-S(O)₂-N((C₁₋₆)alkyl)-, and heterocyclyl-R²¹-, wherein R³ may be optionally substituted on carbon by one or more R⁸;
R⁴ is (C₁₋₆)alkyl;
R⁵ is halo;
R⁶ is halo;
R⁸ is halo;
R²¹ is a bond; and
n=1;
or a pharmaceutically acceptable salt, solvate, or hydrate thereof.

In a preferred embodiment, the JAK-2 inhibitor is AZD-1480. In a preferred embodiment, the JAK-2 inhibitor is (*S*)-5-chloro-N²-(1-(5-fluoropyrimidin-2-yl)ethyl)-N⁴-(5-methyl-1*H*-pyrazol-3-yl)pyrimidine-2,4-diamine. In a preferred embodiment, the JAK-2 inhibitor has the chemical structure shown in Formula (LVIII): 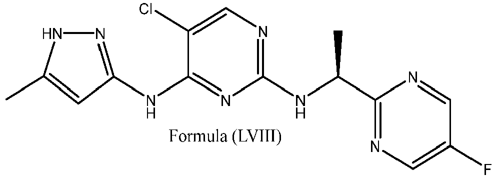 or a pharmaceutically acceptable salt, solvate, or hydrate thereof. The preparation of this compound is described in U.S. Patent No. 8,088,784 and U.S. Patent Application Publication Nos. 2008/0287475 A1; 2010/0160325 A1; and, 2012/0071480 A1. In an embodiment, the JAK-2 inhibitor is selected from the compounds described in U.S. Patent No. 8,088,784 and U.S. Patent Application Publication Nos. 2008/0287475 A1; 2010/0160325 A1; and, 2012/0071480 A1.

In an embodiment, the JAK-2 inhibitor is a compound of Formula (LIX): 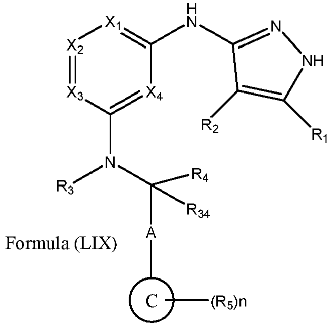 or a pharmaceutically acceptable salt, solvate, or hydrate thereof, wherein:
R¹ and R² are independently selected from hydrogen, halo, nitro, cyano, hydroxy, trifluoromethoxy, amino, carboxy, carbamoyl, mercapto, sulphamoyl, C₁₋₆ alkyl, C₂₋₆ alkenyl, C₂₋₆ alkynyl, C₁₋₆ alkoxy, C₁₋₆ alkanoyl, C₁₋₆ alkanoyloxy, N-(C₁₋₆alkyl)amino, N,N-(C₁₋₆ alkyl)₂-amino, C₁₋₆ alkanoylamino, N-(C₁₋₆ alkyl)carbamoyl, N,N-(C₁₋₆ alkyl)₂-carbamoyl, C₁₋₆ alkylS(O)a wherein a is 0 to 2, C₁₋₆ alkoxycarbonyl, N-(C₁₋₆ alkyl)sulphamoyl, N,N-(C₁₋₆ alkyl)₂sulphamoyl, C₁₋₆ alkylsulphonylamino, carbocyclyl or heterocyclyl; wherein R¹ and R² independently of each other may be optionally substituted on carbon by one or more R⁶; and wherein if said heterocyclyl contains an -NH- moiety that nitrogen may be optionally substituted by a group selected from R⁷;
one of X¹, X², X³ and X⁴ is =N-, the other three are independently selected from =CR⁸-, =CR⁹- and =CR¹⁰-;
R³ is hydrogen or optionally substituted C₁₋₆ alkyl; wherein said optional substituents are selected from one or more R¹¹;
R⁴ and R³⁴ are independently selected from hydrogen, halo, nitro, cyano, hydroxy, trifluoromethoxy, amino, carboxy, carbamoyl, mercapto, sulphamoyl, C₁₋₆ alkyl, C₂₋₆ alkenyl, C₂₋₆ alkynyl, C₁₋₆ alkoxy, C₁₋₆ alkanoyl, C₁₋₆ alkanoyloxy, N-(C₁₋₆ alkyl)amino, N,N-(C₁₋₆ alkyl)₂amino, C₁₋₆ alkanoylamino, N-(C₁₋₆ alkyl)carbamoyl, N,N-(C₁₋₆ alkyl)₂carbamoyl, C₁₋₆ alkylS(O)a wherein a is 0 to 2, C₁₋₆ alkoxycarbonyl, N-(C₁₋₆alkyl)sulphamoyl, N,N-(C₁₋₆ alkyl)₂sulphamoyl, C₁₋₆ alkylsulphonylamino, carbocyclyl or heterocyclyl; wherein R⁴ and R³⁴ may be independently optionally substituted on carbon by one or more R¹²; and wherein if said heterocyclyl contains an -NH- moiety that nitrogen may be optionally substituted by a group selected from R¹³;
A is a direct bond or C₁₋₂alkylene; wherein said C₁₋₂alkylene may be optionally substituted by one or more R¹⁴;
Ring C is carbocyclyl or heterocyclyl; wherein if said heterocyclyl contains an -NH-moiety that nitrogen may be optionally substituted by a group selected from R¹⁵;
R⁵ is selected from halo, nitro, cyano, hydroxy, trifluoromethoxy, amino, carboxy, carbamoyl, mercapto, sulphamoyl, C₁₋₆ alkyl, C₂₋₆ alkenyl, C₂₋₆ alkynyl, C₁₋₆ alkoxy, C₁₋₆ alkanoyl, C₁₋₆ alkanoyloxy, N-(C₁₋₆alkyl)amino, N,N-(C₁₋₆ alkyl)₂amino, C₁₋₆ alkanoylamino, N-(C₁₋₆ alkyl)carbamoyl, N,N-(C₁₋₆ alkyl)₂carbamoyl, C₁₋₆ alkylS(O)a wherein a is 0 to 2, C₁₋₆ alkoxycarbonyl, N-(C₁₋₆ alkyl)sulphamoyl, N,N-(C₁₋₆ alkyl)₂sulphamoyl, C₁₋₆ alkylsulphonylamino, carbocyclyl-R³⁷- or heterocyclyl-R³⁸-; wherein R⁵ may be optionally substituted on carbon by one or more R¹⁶; and wherein if said heterocyclyl contains an -NH- moiety that nitrogen may be optionally substituted by a group selected from R¹⁷;
n is 0, 1, 2 or 3; wherein the values of R⁵ may be the same or different;
R⁸, R⁹ and R¹⁰ are independently selected from hydrogen, halo, nitro, cyano, hydroxy, trifluoromethoxy, amino, carboxy, carbamoyl, mercapto, sulphamoyl, C₁₋₆ alkyl, C₂₋₆ alkenyl, C₂₋₆ alkynyl, C₁₋₆ alkoxy, C₁₋₆ alkanoyl, C₁₋₆ alkanoyloxy, N-(C₁₋₆ alkyl)amino, N,N-(C₁₋₆ alkyl)₂amino, C₁₋₆ alkanoylamino, N-(C₁₋₆ alkyl)carbamoyl, N,N-(C₁₋₆ alkyl)₂carbamoyl, C₁₋₆ alkylS(O)a wherein a is 0 to 2, C₁₋₆ alkoxycarbonyl, N-(C₁₋₆ alkyl)sulphamoyl, N,N-(C₁₋₆ alkyl)₂sulphamoyl, C₁₋₆ alkylsulphonylamino, carbocyclyl-R²⁵- or heterocyclyl-R²⁶-; wherein R⁸, R⁹ and R¹⁰ independently of each other may be optionally substituted on carbon by one or more R¹⁸; and wherein if said heterocyclyl contains an -NH- moiety that nitrogen may be optionally substituted by a group selected from R¹⁹;
R⁶, R¹¹, R¹², R¹⁴, R¹⁶ and R¹⁸ are independently selected from halo, nitro, cyano, hydroxy, trifluoromethoxy, amino, carboxy, carbamoyl, mercapto, sulphamoyl, C₁₋₆ alkyl, C₂₋₆ alkenyl, C₂₋₆ alkynyl, C₁₋₆ alkoxy, C₁₋₆ alkanoyl, C₁₋₆ alkanoyloxy, N-(C₁₋₆ alkyl)amino, N,N-(C₁₋₆ alkyl)₂amino, C₁₋₆ alkanoylamino, N-(C₁₋₆ alkyl)carbamoyl, N,N-(C₁₋₆ alkyl)₂carbamoyl, C₁₋₆ alkylS(O)a wherein a is 0 to 2, C₁₋₆ alkoxycarbonyl, N-(C₁₋₆ alkyl)sulphamoyl, N,N-(C₁₋₆ alkyl)₂sulphamoyl, C₁₋₆ alkylsulphonylamino, carbocyclyl-R²⁷- or heterocyclyl-R²⁸-; wherein R⁶, R¹¹, R¹², R¹⁴, R¹⁶ and R¹⁸ independently of each other may be optionally substituted on carbon by one or more R²⁰; and wherein if said heterocyclyl contains an -NH- moiety that nitrogen may be optionally substituted by a group selected from R²¹;
R⁷, R¹³, R¹⁵, R¹⁷, R¹⁹ and R²¹ are independently selected from C₁₋₆ alkyl, C₁₋₆ alkanoyl, C₁₋₆ alkylsulphonyl, C₁₋₆ alkoxycarbonyl, carbamoyl, N-(C₁₋₆ alkyl)carbamoyl, N,N-(C₁₋₆ alkyl)carbamoyl, benzyl, benzyloxycarbonyl, benzoyl and phenylsulphonyl; wherein R⁷, R¹³, R¹⁵, R¹⁷, R¹⁹ and R²¹ independently of each other may be optionally substituted on carbon by on or more R²²;
R²⁰ and R²² are independently selected from halo, nitro, cyano, hydroxy, trifluoromethoxy, amino, carboxy, carbamoyl, mercapto, sulphamoyl, C₁₋₆ alkyl, C₂₋₆ alkenyl, C₂₋₆ alkynyl, C₁₋₆ alkoxy, C₁₋₆ alkanoyl, C₁₋₆ alkanoyloxy, N-(C₁₋₆ alkyl)amino, N,N-(C₁₋₆ alkyl)₂amino, C₁₋₆ alkanoylamino, N-(C₁₋₆ alkyl)carbamoyl, N,N-(C₁₋₆ alkyl)₂carbamoyl, C₁₋₆ alkylS(O)a wherein a is 0 to 2, C₁₋₆ alkoxycarbonyl, N-(C₁₋₆ alkyl)sulphamoyl, N,N-(C₁₋₆ alkyl)₂sulphamoyl, C₁₋₆ alkylsulphonylamino, C₁₋₆ alkylsulphonyl-N--(C₁₋₆ alkyl)amino, carbocyclyl-R³⁵- or heterocyclyl-R³⁶-; wherein R²⁰ and R²² independently of each other may be optionally substituted on carbon by one or more R²³; and wherein if said heterocyclyl contains an -NH-moiety that nitrogen may be optionally substituted by a group selected from R²⁴;
R²⁵, R²⁶, R²⁷, R²⁸, R³⁵, R³⁶, R³⁷ and R³⁸ are independently selected from a direct bond,-O--, -N(R²⁹)-, -C(O)-, -N(R³⁰)C(O)-, -C(O)N(R³¹)-, -S(O)ₛ-,-NH=CH-, -SO₂N(R³²)- or -N(R³³)SO₂-; wherein R²⁹, R³⁰, R³¹, R³² and R³³ are independently selected from hydrogen or C₁₋₆ alkyl and s is 0-2;
R²³ is selected from halo, nitro, cyano, hydroxy, trifluoromethoxy, trifluoromethyl, amino, carboxy, carbamoyl, mercapto, sulphamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl, N-ethylcarbamoyl, N,N-dimethylcarbamoyl, N,N-diethylcarbamoyl, N-methyl-N-ethylcarbamoyl, methylthio, ethylthio, methylsulphinyl, ethylsulphinyl, mesyl, ethylsulphonyl, methoxycarbonyl, ethoxycarbonyl, N-methylsulphamoyl, N-ethylsulphamoyl, N,N-dimethylsulphamoyl, N,N-diethylsulphamoyl, N-methyl-N-ethylsulphamoyl or phenyl; and
R²⁴ is selected from C₁₋₆ alkyl, C₁₋₆ alkanoyl, C₁₋₆ alkylsulphonyl, C₁₋₆ alkoxycarbonyl, carbamoyl, N-(C₁₋₆ alkyl)carbamoyl, N,N-(C₁₋₆ alkyl)carbamoyl, benzyl, benzyloxycarbonyl, benzoyl and phenylsulphonyl;
or a pharmaceutically acceptable salt, solvate, or hydrate thereof.

In a preferred embodiment, the JAK-2 inhibitor is (*S*)-5-fluoro-2-((1-(4-fluorophenyl)ethyl)amino)-6-((5-methyl-1*H*-pyrazol-3-yl)amino)nicotinonitrile. In a preferred embodiment, the JAK-2 inhibitor is a compound of Formula (LX): 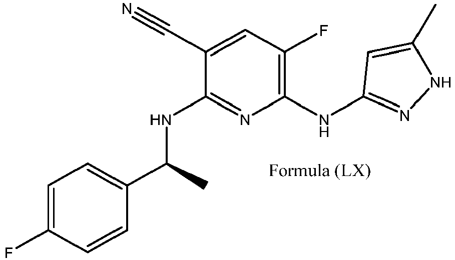 or a pharmaceutically acceptable salt, solvate, or hydrate thereof. The preparation of this compound is described in U.S. Patent No. 8,324,252 and U.S. Patent Application Publication Nos. 2008/0139561 A1 and 2013/0090358 A1. In an embodiment, the JAK-2 inhibitor is selected from the compounds described in U.S. Patent No. 8,324,252 and U.S. Patent Application Publication Nos. 2008/0139561 A1 and 2013/0090358 A1.

In an embodiment, the JAK-2 inhibitor is a compound of Formula (LXII): 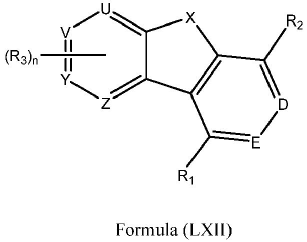 or a pharmaceutically acceptable salt, solvate, or hydrate thereof, or a stereoisomer thereof, wherein:
D is CH or N;
E is CH or N;
X is CH₂, NR₄, O or S;
U is CH or N;
V is CH or N;
Y is CH or N;
Z is CH or N;
R¹ is NR₅R₆, CR5R₆R₇, SR₅ or OR₅;
R² is (C=O)OH, (C=O)NH₂, (C=O)NHR₄ or heterocyclyl;
R³ is
   (a) hydrogen;
   (b) C₁₋₆ alkyl, which is optionally substituted with halo, hydroxyl, amino, phenyl, heterocyclyl, C₁₋₆ alkyl or R₁₀;
   (c) C₂₋₆ alkenyl, which is optionally substituted with halo, hydroxyl, amino, phenyl, heterocyclyl, C₁₋₆ alkyl or R₄;
   (d) C₃₋₁₀ cycloalkyl, which is optionally substituted with C₁₋₆ alkyl, OR₄, NR₈R₄, phenyl (which is optionally substituted with C₁₋₆ alkyl, OR₄ or NR₈R₄), halo, R₁₀ or heterocyclyl;
   (e) -(CO)R₈;
   (f) -(CO)-NR₈R₉;
   (g) C₄₋₁₀ heterocyclyl, which is optionally substituted on either the carbon or the heteroatom with C1-6 alkyl, halo, R₁₀, OR₄, NR₈R₄, phenyl (which is optionally substituted with C1-6 alkyl, OR₄ or NR₈R₄), -(CO)R₈ or -(CO)-NR₈R₉;
   (h) OR₄;
   (i) NR₈R₄;
   (j) halo;
   (k) Aryl, which is optionally substituted with one or more groups selected from C₁₋₆ alkyl (which is optionally substituted with one to three halo), halo or R₁₀;
   (l) Heteroaryl, which is optionally substituted with one or more groups selected from C₁₋₆ alkyl (which is optionally substituted with one to three halo), halo or R₁₀;
   (m) O-aryl, which is optionally substituted with one or more groups selected from C₁₋₆ alkyl, halo or R₁₀;
   (n) O--C₁₋₆ alkyl, which is optionally substituted with C₁₋₆ alky, halo or R₁₀; or
   (o) L-A-R₁₀;
R4 is
   (a) hydrogen;
   (b) C₁₋₆ alkyl, which is optionally substituted with halo, hydroxyl, amino, aryl or heterocyclyl;
   (c) C₃₋₁₀ cycloalkyl, which is optionally substituted with C₁₋₆ alkyl, OR₁₁, NR₈R₁₁, phenyl (which is optionally substituted with C₁₋₆ alkyl, OR₁₁ or NR₈R₁₁), heterocyclyl, aryl or heteroaryl;
   (d) -(CO)R₈;
   (e) -(CO)-NR₈R₉;
   (f) C₄₋₁₀ heterocyclyl, which is optionally substituted on either the carbon or the heteroatom with C₁₋₆ alkyl, OR₁₁, NR₈R₁₁, phenyl (which is optionally substituted with C₁₋₆ alkyl, OR₁₁ or NR₈R₁₁), heterocyclyl, -(CO)R₈ or -(CO)-NR₈R₉;
   (g) OR₁₁;
   (h) NR₈R₁₁;
   (i) Aryl, which is optionally substituted with one to five halo or R₁₀;
   (j) Heteroaryl (wherein the heteroaryl has 5 or 6 members in which 1, 2, 3, or 4 of the atoms is a heteroatom selected from N, S and O), which is optionally substituted with one to five halo or R₁₀;
R₅ is
   (a) hydrogen;
   (b) C₁₋₈ alkyl, which is optionally substituted with halo, hydroxyl, amino, aryl, cycloalkyl or heterocyclyl;
   (c) C₃₋₁₀ cycloalkyl, which is optionally substituted with C₁₋₆alkyl, (C₁₋₆ alkyl)aryl, (C₁₋₆ alkyl)OR₉, OR₄, NR₈R₄, phenyl (which is optionally substituted with C₁₋₆ alkyl, OR₄, NR₈R₄, heterocyclyl, -(CO)R₈ or -(CO)-NR₈R₉);
   (d) -(CO)R₈;
   (e) -(CO)-NR₈R₉;
   (f) C₁₋₆ alkyl(C=O)NR₈CR₉(C=O)NR₈R₉;
   (g) C₄₋₁₀ heterocyclyl which is optionally substituted on either the carbon or the heteroatom with one to three substituents selected from C₁₋₆ alkyl, halo, OR₄, NR₈R₄, -(CO)R₈, (CO)-NR₈R₉ or phenyl (which is optionally substituted with C₁₋₆ alkyl, OR₄, NR₈R₄, heterocyclyl, -(CO)R₈ or -(CO)-NR₈R₉);
R6 is
   (a) hydrogen;
   (b) C₁₋₈ alkyl, which is optionally substituted with halo, hydroxyl, amino, aryl, cycloalkyl or heterocyclyl;
   (c) C₃₋₁₀ cycloalkyl, which is optionally substituted with C₁₋₆ alkyl, (C₁₋₆ alkyl)aryl, (C₁₋₆ alkyl)OR₉, OR₄, NR₈R₄, phenyl (which is optionally substituted with C₁₋₆ alkyl, OR₄, NR₈R₄, heterocyclyl, -(CO)R₈ or -(CO)-NR₈R₉;
   (d) -(CO)R₈;
   (e) -(CO)-NR₈R₉;
   (f) C₁₋₆ alkyl(C=O)NR₈CR₉(C=O)NR₈R₉;
   (g) C₄₋₁₀ heterocyclyl which is optionally substituted on either the carbon or the heteroatom with one to three substituents selected from C₁₋₆ alkyl, halo, OR₄, NR₈R₄, -(CO)R₈, (CO)-NR₈R₉ or phenyl (which is optionally substituted with C₁₋₆ alkyl, OR₄, NR₈R₄, heterocyclyl, -(CO)R₈ or -(CO)-NR₈R₉);
R₇ is
   (a) hydrogen;
   (b) C₁₋₆ alkyl, which is optionally substituted with halo, hydroxyl, amino, phenyl or heterocyclyl;
   (c) C₃₋₁₀ cycloalkyl, which is optionally substituted with C₁₋₆ alkyl, OR₄, NR₈R₄, phenyl (which is optionally substituted with C₁₋₆ alkyl, OR₄, NR₈R₄, heterocyclyl, -(CO)R₈ or -(CO)-NR₈R₉);
   (d) C₄₋₁₀ heterocyclyl which is optionally substituted on either the carbon or the heteroatom with C₁₋₆ alkyl, OR₄, NR₈R₄, phenyl (which is optionally substituted with C₁₋₆ alkyl, OR₄, NR₈R₄, heterocyclyl, -(CO)R₈ or -(CO)-NR₈R₉);
Or R₅ and R₆, together with the atoms between them, can form a three to ten membered heterocyclic or heteroaryl ring which is optionally substituted with C₁₋₆ alkyl, (C₁₋₆ alkyl)aryl, (C₁₋₆ alkenyl)aryl, (C₁₋₆ alkyl)OR₉, OR₄, NR₈R₄, phenyl (which is optionally substituted with C₁₋₆ alkyl, OR₄, NR₈R₄, heterocyclyl, -(CO)R₈ or -(CO)-NR₈R₉),-(CO)R₈; -(CO)-NR8R9, or heterocyclyl;
R₈ is hydrogen or C₁₋₆ alkyl, -(CO)R₁₁, -(CO)N(R₁₁)₂;
R₉ is hydrogen or C₁₋₆ alkyl;
R₁₀ is:
   (a) hydrogen;
   (b) CO₂R₁₁;
   (c) C(O)R₁₁;
   (d) NHR₁₁;
   (e) NR₁₁R₁₂;
   (f) NHS(O)₂R₁₁;
   (g) NHC(O)R₁₁;
   (h) NHC(O)OR₁₁;
   (i) NH-C=(NH)NH₂;
   (j) NHC(O)NH₂;
   (k) NHC(O)NHR₁₁;
   (l) NHC(O)NR₁₁R₁₂;
   (m) NC3-6cycloalkyl;
   (n) C(O)NHR₁₁;
   (o) C(O)NR₁₁R₁₂;
   (p) SO₂NHR₁₁;
   (q) SO₂NHC(O)R₁₂; or
   (r) SO₂R₁₁;
R₁₁ is selected from the group consisting of:
   (a) hydrogen,
   (b) C₃₋₆cycloalkyl, which is optionally substituted with aryl, heteroaryl or one to five halo;
   (c) C₁₋₆ alkyl, which is optionally substituted with aryl, heteroaryl, or one to five halo;
   (d) Aryl, which is optionally substituted with one to five halo;
   (e) Heteroaryl (wherein the heteroaryl has 5 or 6 members in which 1, 2, 3, or 4 of the atoms is a heteroatom selected from N, S and O), which is optionally substituted with one to five halo;
R₁₂ is selected from the group consisting of:
   (a) hydrogen,
   (b) C₁₋₆alkyl, which is optionally substituted with aryl, heteroaryl or one to five halo;
   (c) C₃₋₆cycloalkyl, which is optionally substituted with aryl, heteroaryl or one to five halo;
   (d) Aryl, which is optionally substituted with one to five halo;
   (e) Heteroaryl (wherein the heteroaryl has 5 or 6 members in which 1, 2, 3, or 4 of the atoms is a heteroatom selected from N, S and O), which is optionally substituted with one to five halo;
A is absent or is selected from the group consisting of: aryl or heteroaryl (wherein the heteroaryl is a monocyclic ring of 5 or 6 atoms or a bicyclic ring of 9 or 10 atoms in which 1, 2, 3, or 4 of the atoms is a heteroatom selected from N, S and O), wherein said aryl or heteroaryl is optionally substituted with one or more substituents selected from halo, (C₁₋₃)alkyl, -C(O)OH, CF₃, -SO₂(C₁₋₃)alkyl, SO₂N(C₁₋₃)alkyl, SO₂NHC(O)-(C₁₋₃)alkyl or N(CH₃)₂;
L is absent or is selected from the group consisting of: -(CH₂)k-W-, -Z-(CH₂)k-, -C≡C-, -C₁₋₆alkyl-, -C₃₋₆cycloalkyl- and -C₂₋₅alkene-, wherein the alkene is optionally substituted with one or more groups selected from C₁₋₆alkyl or C₁₋₆cycloalkyl; W is selected from the group consisting of: O, NH, NC₁₋₆alkyl and S(O)m, with the proviso that when W is O, S(O)m, NH or NC₁₋₆alkyl and simultaneously A is absent then R₁₀ is CO₂R₁₁, COR₁₁, CONHR₁₁ or CONR₁₁R₁₂;
k=0, 1, 2, 3, 4, or 5;
m=0, 1, or 2; and
n=0, 1, 2, or 3.

In a preferred embodiment, the JAK-2 inhibitor is ((*R*)-7-(2-aminopyrimidin-5-yl)-1-((1-cyclopropyl-2,2,2-trifluoroethyl)amino)-5*H*-pyrido[4,3-*b*]indole-4-carboxamide, which is also named 7-(2-aminopyrimidin-5-yl)-1-{[(1*R*)-1-cyclopropyl-2,2,2-trifluoroethyl]amino}-5*H*-pyrido[4,3-*b*]indole-4-carboxamide. In a preferred embodiment, the JAK-2 inhibitor is a compound of Formula (LXII): 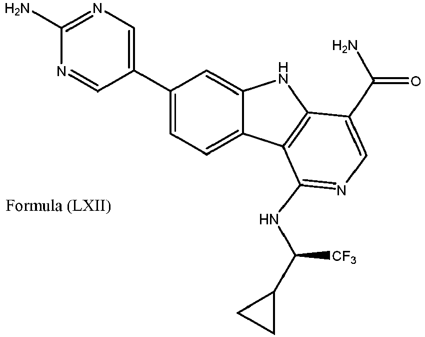 or a pharmaceutically acceptable salt, solvate, or hydrate thereof. The preparation of this compound is known to those of ordinary skill in the art, and is described in Lim, et al., Discovery of 1-amino-5H-pyrido[4,3-b]indol-4-carboxamide inhibitors of Janus kinase-2 (JAK2) for the treatment of myeloproliferative disorders, J. Med. Chem. 2011, 54, 7334-7349.

In an embodiment, the JAK-2 inhibitor is a compound selected from the JAK-2 inhibitors disclosed in U.S. Patent No. U.S. 8,518,964 or U.S. Patent Application Publication Nos. 2010/0048551 A1.

### PD-1 Inhibitors

The terms "inhibitor" and "blocker" are used interchangeably herein in reference to PD-1 inhibitors.

The anti-PD-1 antibody (PD-1 inhibitor) employed in the present invention is pembrolizumab, which is commercially available from Merck. Pembrolizumab is referred to as h409Al l in International Patent Publication No. WO 2008/156712 A1, U.S. Patent No. 8,354,509 and U.S. Patent Application Publication Nos. US 2010/0266617 A1, US 2013/0108651 A1, and US 2013/0109843 A2. Pembrolizumab has an immunoglobulin G4, anti-(human protein PDCD1 (programmed cell death 1)) (human-Mus musculus monoclonal heavy chain), disulfide with human-Mus musculus monoclonal light chain, dimer structure. The structure of pembrolizumab may also be described as immunoglobulin G4, anti-(human programmed cell death 1); humanized mouse monoclonal [228-L-proline(H10-S>P)]γ4 heavy chain (134-218')-disulfide with humanized mouse monoclonal κ light chain dimer (226-226":229-229")-bisdisulfide. Pembrolizumab is assigned CAS registry number 1374853-91-4 and is also known as lambrolizumab, MK-3475, and SCH-900475. The clinical safety and efficacy of pembrolizumab in various forms of cancer is described in Fuerst, Oncology Times, 2014, 36, 35-36; Robert, et al., Lancet, 2014, 384, 1109-17; and Thomas et al., Exp. Opin. Biol. Ther., 2014, 14, 1061-1064. In an embodiment, the pembrolizumab monoclonal antibody includes a heavy chain given by SEQ ID NO:12 and a light chain given by SEQ ID NO:14, and also shown below with disulfide and glycosylation information:
Heavy chain
Light chain
Disulfide bridges

| | | | |
|---|---|---|---|
| 22-96 | 22"-96" | 23'-92' | 23‴-92‴ |
| 134-218' | 134ʺ-218‴ | 138'-198' | 138‴-198‴ |
| 147-203 | 147"-203" | 226-226" | 229-229" |
| 261-321 | 261"-321" | 367-425 | 367"-425" |

Glycosylation sites (N)

| | |
|---|---|
| Asn-297 | Asn-297" |

An anti-PD-1 antibody comprises heavy and light chains having the sequences shown in SEQ ID NO:12 and SEQ ID NO:14, respectively

The anti-PD-1 antibody comprises the heavy and light chain CDRs or VRs of pembrolizumab. The antibody V_{H} region comprises the sequence of residues 20 to 446 of SEQ ID NO: 11, and the antibody V_{L} region comprises the sequence shown in SEQ ID NO:14.

The anti-PD-1 antibody comprises a heavy chain comprising amino acid residues 20 to 446 of SEQ ID NO:11 and a light chain comprising amino acid residues of 20-237 of SEQ ID NO:13.

The anti-PD-1 antibody sequences discussed and referenced in the foregoing embodiments are summarized in Table 1.

**TABLE 1. Anti-PD-1 antibody amino acid sequences.**

| **Identifier** | **Sequence (One-Letter Amino Acid Symbols)** | |
|---|---|---|
| SEQ ID NO:11 pembrolizumab heavy chain | | |
| SEQ ID NO:12 pembrolizumab heavy chain | | |
| SEQ ID NO:13 pembrolizumab variable light chainamino acid | | |
| SEQ ID NO:14 pembrolizumab light chain | | |
| SEQ ID NO:15 pembrolizumab light chain CDR1 | RASKGVSTSG YSYLH | 15 |
| SEQ ID NO:16 pembrolizumab light chain CDR2 | LASYLES | 7 |
| SEQ ID NO:17 pembrolizumab light chain CDR3 | QHSRDLPLT | 9 |
| SEQ ID NO:18 pembrolizumab heavy chain CDR1 | NYYMY | 5 |
| SEQ ID NO:19 pembrolizumab heavy chain CDR2 | GINPSNGGTN FNEKFK | 16 |
| SEQ ID NO:20 pembrolizumab heavy chain CDR3 | RDYRFDMGFD Y | 11 |

### PD-L1 Inhibitors

The PD-L1 inhibitor employed in the present invention is an anti-PD-L1 antibody and is durvalumab, which is also known as MEDI4736, produced by Medimmune, LLC, Gaithersburg, Maryland, a subsidiary of AstraZeneca plc.. In an embodiment, the anti-PD-L1 antibody is an antibody disclosed in U.S. Patent No. 8,779,108 or U.S. Patent Application Publication No. 2013/0034559. The clinical efficacy of durvalumab (MEDI4736, SEQ ID NO:30 and SEQ ID NO:31) has been described in: Page et al., Ann. Rev. Med., 2014, 65, 185-202; Brahmer, et al., J. Clin. Oncol. 2014, 32, 5s (supplement, abstract 8021); and McDermott, et al., Cancer Treatment Rev., 2014, 40, 1056-64. The durvalumab (MEDI4736) monoclonal antibody includes a V_{H} region given by SEQ ID NO:32 (corresponding to SEQ ID NO:72 in U.S. Patent No. 8,779,108) and a V_{L} region given by SEQ ID NO:33 (corresponding to SEQ ID NO:77 in U.S. Patent No. 8,779,108). The durvalumab monoclonal antibody includes disulfide linkages at 22-96, 22"-96", 23'-89', 23‴-89‴, 135'-195', 135‴-195‴, 148-204, 148"-204", 215'-224, 215‴-224ʺ, 230-230", 233-233", 265-325, 265"-325", 371-429, and 371ʺ-429'; and N-glycosylation sites at Asn-301 and Asn-301".

The anti-PD-Ll antibody comprises the heavy and light chains of durvalumab (MEDI4736). The anti-PD-Ll antibody comprises heavy and light chains having the sequences shown in SEQ ID NO:30 and SEQ ID NO:31, respectively. Monoclonal antibodies that inhibit PD-L1 can be prepared by procedures known to those of ordinary knowledge and skill in the art, e.g. by injecting test subjects with PD-L1 antigen and then isolating hybridomas expressing antibodies having the desired sequence or functional characteristics. DNA encoding the monoclonal antibodies is readily isolated and sequenced using conventional procedures (*e.g*., by using oligonucleotide probes that are capable of binding specifically to genes encoding the heavy and light chains of the monoclonal antibodies). The hybridoma cells serve as a preferred source of such DNA. Once isolated, the DNA may be placed into expression vectors, which are then transfected into host cells such as *E*. *coli* cells, simian COS cells, Chinese hamster ovary (CHO) cells, myeloma cells, or other suitable cells that do not otherwise produce immunoglobulin protein, to obtain the synthesis of monoclonal antibodies in the recombinant host cells. The details of recombinant production of specific antibodies may be found in the references cited in the foregoing, the disclosures of which are incorporated by reference herein. Monoclonal antibodies that inhibit PD-1 can be prepared by standard molecular biology methods using the sequences provided herein by reverse translation and insertion into appropriate DNA or RNA vectors.

The anti-PD-Ll antibody sequences referenced in the foregoing embodiments are summarized in Table 2.

**TABLE 2. Anti-PD-Ll antibody amino acid sequences.**

| **Identifier** | **Sequence (One-Letter Amino Acid Symbols)** | |
|---|---|---|
| SEQ ID NO:30 durvalumab (MEDI4736) heavy chain | | |
| SEQ ID NO:31 durvalumab (MEDI4736) light chain | | |
| SEQ ID NO:32 durvalumab (MEDI4736) variable heavy chain | | |
| SEQ ID NO:33 durvalumab (MEDI4736) variable light chain | | |
| SEQ ID NO:34 durvalumab (MEDI4736) heavy chain CDR1 | RYWMS | 5 |
| SEQ ID NO:35 durvalumab (MEDI4736) heavy chain CDR2 | NIKQDGSEKY YVDSVKG | 17 |
| SEQ ID NO:36 durvalumab (MEDI4736) heavy chain CDR3 | EGGWFGELAF DY | 12 |
| SEQ ID NO:37 durvalumab (MEDI4736) light chain CDR1 | RASQRVSSSY LA | 12 |
| SEQ ID NO:38 durvalumab (MEDI4736) light chain CDR2 | DASSRAT | 7 |
| SEQ ID NO:39 durvalumab (MEDI4736) light chain CDR3 | QQYGSLPWT | 9 |

### Pharmaceutical Compositions

In some embodiments, the invention provides pharmaceutical compositions for use in treating solid tumor cancers, lymphomas and leukemia.

The pharmaceutical compositions are typically formulated to provide a therapeutically effective amount of a combination of a BTK inhibitor of formula (XVIII) or a pharmaceutically acceptable salt thereof, a PD-1 inhibitor or a PD-L1 inhibitor, and optionally a PI3K inhibitor, including a PI3K-γ or PI3K-δ inhibitor, a JAK-2 inhibitor, as the active ingredients, wherein the PD-1 inhibitor is pembrolizumab and the PD-L1 inhibitor is durvalumab. Where desired, the pharmaceutical compositions contain one or more pharmaceutically acceptable excipients, carriers, including inert solid diluents and fillers, diluents, including sterile aqueous solution and various organic solvents, permeation enhancers, solubilizers and adjuvants.

The pharmaceutical compositions are administered as a combination of a BTK inhibitor of formula (XVIII) or a pharmaceutically acceptable salt thereof, a PD-1 inhibitor or a PD-L1 inhibitor, and optionally a PI3K inhibitor, including a PI3K-γ or PI3K-δ inhibitor, and a JAK-2 inhibitor,, wherein the PD-1 inhibitor is pembrolizumab and the PD-L1 inhibitor is durvalumab. Where desired, other active pharmaceutical ingredient(s) may be mixed into a preparation or both components may be formulated into separate preparations for use in combination separately or at the same time.

In some embodiments, the concentration of each of the PI3K, PD-1, and BTK inhibitors provided in the pharmaceutical compositions for use in the invention is independently less than, for example, 100%, 90%, 80%, 70%, 60%, 50%, 40%, 30%, 20%, 19%, 18%, 17%, 16%, 15%, 14%, 13%, 12%, 11%, 10%, 9%, 8%, 7%, 6%, 5%, 4%, 3%, 2%, 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002% or 0.0001% w/w, w/v or v/v, relative to the total mass or volume of the pharmaceutical composition.

In some embodiments, the concentration of each of the PI3K, JAK-2, PD-1, PD-L1, and BTK inhibitors provided in the pharmaceutical compositions for use in the invention is independently greater than 90%, 80%, 70%, 60%, 50%, 40%, 30%, 20%, 19.75%, 19.50%, 19.25% 19%, 18.75%, 18.50%, 18.25% 18%, 17.75%, 17.50%, 17.25% 17%, 16.75%, 16.50%, 16.25% 16%, 15.75%, 15.50%, 15.25% 15%, 14.75%, 14.50%, 14.25% 14%, 13.75%, 13.50%, 13.25% 13%, 12.75%, 12.50%, 12.25% 12%, 11.75%, 11.50%, 11.25% 11%, 10.75%, 10.50%, 10.25% 10%, 9.75%, 9.50%, 9.25% 9%, 8.75%, 8.50%, 8.25% 8%, 7.75%, 7.50%, 7.25% 7%, 6.75%, 6.50%, 6.25% 6%, 5.75%, 5.50%, 5.25% 5%, 4.75%, 4.50%, 4.25%, 4%, 3.75%, 3.50%, 3.25%, 3%, 2.75%, 2.50%, 2.25%, 2%, 1.75%, 1.50%, 125%, 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002% or 0.0001% w/w, w/v, or v/v, relative to the total mass or volume of the pharmaceutical composition.

In some embodiments, the concentration of each of the PI3K, JAK-2, PD-1, PD-L1, and BTK inhibitors in the combination for use in the invention is independently in the range from approximately 0.0001% to approximately 50%, approximately 0.001% to approximately 40%, approximately 0.01% to approximately 30%, approximately 0.02% to approximately 29%, approximately 0.03% to approximately 28%, approximately 0.04% to approximately 27%, approximately 0.05% to approximately 26%, approximately 0.06% to approximately 25%, approximately 0.07% to approximately 24%, approximately 0.08% to approximately 23%, approximately 0.09% to approximately 22%, approximately 0.1% to approximately 21%, approximately 0.2% to approximately 20%, approximately 0.3% to approximately 19%, approximately 0.4% to approximately 18%, approximately 0.5% to approximately 17%, approximately 0.6% to approximately 16%, approximately 0.7% to approximately 15%, approximately 0.8% to approximately 14%, approximately 0.9% to approximately 12% or approximately 1% to approximately 10% w/w, w/v or v/v, relative to the total mass or volume of the pharmaceutical composition.

In some embodiments, the concentration of each of the PI3K, JAK-2, PD-1, PD-L1, and BTK inhibitors in the combination for use in the invention is independently in the range from approximately 0.001% to approximately 10%, approximately 0.01% to approximately 5%, approximately 0.02% to approximately 4.5%, approximately 0.03% to approximately 4%, approximately 0.04% to approximately 3.5%, approximately 0.05% to approximately 3%, approximately 0.06% to approximately 2.5%, approximately 0.07% to approximately 2%, approximately 0.08% to approximately 1.5%, approximately 0.09% to approximately 1%, approximately 0.1% to approximately 0.9% w/w, w/v or v/v, relative to the total mass or volume of the pharmaceutical composition.

In some embodiments, the amount of each of the PI3K, JAK-2, PD-1, PD-L1, and BTK inhibitors in the combination for use in the invention is independently equal to or less than 3.0 g, 2.5 g, 2.0 g, 1.5 g, 1.0 g, 0.95 g, 0.9 g, 0.85 g, 0.8 g, 0.75 g, 0.7 g, 0.65 g, 0.6 g, 0.55 g, 0.5 g, 0.45 g, 0.4 g, 0.35 g, 0.3 g, 0.25 g, 0.2 g, 0.15 g, 0.1 g, 0.09 g, 0.08 g, 0.07 g, 0.06 g, 0.05 g, 0.04 g, 0.03 g, 0.02 g, 0.01 g, 0.009 g, 0.008 g, 0.007 g, 0.006 g, 0.005 g, 0.004 g, 0.003 g, 0.002 g, 0.001 g, 0.0009 g, 0.0008 g, 0.0007 g, 0.0006 g, 0.0005 g, 0.0004 g, 0.0003 g, 0.0002 g or 0.0001 g.

In some embodiments, the amount of each of the PI3K, JAK-2, PD-1, PD-L1, and BTK inhibitors in the combination for use in the invention is independently more than 0.0001 g, 0.0002 g, 0.0003 g, 0.0004 g, 0.0005 g, 0.0006 g, 0.0007 g, 0.0008 g, 0.0009 g, 0.001 g, 0.0015 g, 0.002 g, 0.0025 g, 0.003 g, 0.0035 g, 0.004 g, 0.0045 g, 0.005 g, 0.0055 g, 0.006 g, 0.0065 g, 0.007 g, 0.0075 g, 0.008 g, 0.0085 g, 0.009 g, 0.0095 g, 0.01 g, 0.015 g, 0.02 g, 0.025 g, 0.03 g, 0.035 g, 0.04 g, 0.045 g, 0.05 g, 0.055 g, 0.06 g, 0.065 g, 0.07 g, 0.075 g, 0.08 g, 0.085 g, 0.09 g, 0.095 g, 0.1 g, 0.15 g, 0.2 g, 0.25 g, 0.3 g, 0.35 g, 0.4 g, 0.45 g, 0.5 g, 0.55 g, 0.6 g, 0.65 g, 0.7 g, 0.75 g, 0.8 g, 0.85 g, 0.9 g, 0.95 g, 1 g, 1.5 g, 2 g, 2.5, or 3 g.

Each of the PI3K, JAK-2, PD-1, PD-L1, and BTK inhibitors in the combination for use inthe invention is effective over a wide dosage range. For example, in the treatment of adult humans, dosages independently range from 0.01 to 1000 mg, from 0.5 to 100 mg, from 1 to 50 mg per day, and from 5 to 40 mg per day are examples of dosages that may be used. The exact dosage will depend upon the route of administration, the form in which the compound is administered, the gender and age of the subject to be treated, the body weight of the subject to be treated, and the preference and experience of the attending physician.

Described below are non-limiting pharmaceutical compositions and methods for preparing the same.

### Pharmaceutical Compositions for Oral Administration

In some embodiments, the invention provides a pharmaceutical composition for oral administration and for use in the treatment of cancer in a human subject containing the combination of a BTK inhibitor of formula (XVIII) or a pharmaceutically acceptable salt thereof and a PD-1 inhibitor or a PD-L1 inhibitor and optionally a PI3K, and /or a JAK-2, inhibitor, and a pharmaceutical excipient suitable for oral administration, wherein the PD-1 inhibitor is pembrolizumab and the PD-L1 inhibitor is durvalumab.

In some embodiments, the invention provides a solid pharmaceutical composition for oral administration and for use in the treatment of cancer in a human containing: (i) an effective amount of each of a BTK inhibitor of formula (XVIII) or a pharmaceutically acceptable salt thereof and a PD-1 inhibitor or a PD-L1 inhibitor and optionally a a PI3K, and/or a JAK-2, inhibitor in combination and (ii) a pharmaceutical excipient suitable for oral administration, wherein the PD-1 inhibitor is pembrolizumab and the PD-L1 inhibitor is durvalumab. In some embodiments, the composition further contains (iii) an effective amount of a fourth compound.

In some embodiments, the pharmaceutical composition may be a liquid pharmaceutical composition suitable for oral consumption. Pharmaceutical compositions for use in the invention suitable for oral administration can be presented as discrete dosage forms, such as capsules, sachets, or tablets, or liquids or aerosol sprays each containing a predetermined amount of an active ingredient as a powder or in granules, a solution, or a suspension in an aqueous or non-aqueous liquid, an oil-in-water emulsion, a water-in-oil liquid emulsion, powders for reconstitution, powders for oral consumptions, bottles (including powders or liquids in a bottle), orally dissolving films, lozenges, pastes, tubes, gums, and packs. Such dosage forms can be prepared by any of the methods of pharmacy, but all methods include the step of bringing the active ingredient(s) into association with the carrier, which constitutes one or more necessary ingredients. In general, the compositions are prepared by uniformly and intimately admixing the active ingredient(s) with liquid carriers or finely divided solid carriers or both, and then, if necessary, shaping the product into the desired presentation. For example, a tablet can be prepared by compression or molding, optionally with one or more accessory ingredients. Compressed tablets can be prepared by compressing in a suitable machine the active ingredient in a free-flowing form such as powder or granules, optionally mixed with an excipient such as a binder, a lubricant, an inert diluent, and/or a surface active or dispersing agent. Molded tablets can be made by molding in a suitable machine a mixture of the powdered compound moistened with an inert liquid diluent.

The invention further encompasses the use of anhydrous pharmaceutical compositions and dosage forms since water can facilitate the degradation of some compounds. For example, water may be added (*e.g.,* 5%) in the pharmaceutical arts as a means of simulating long-term storage in order to determine characteristics such as shelf-life or the stability of formulations over time. Anhydrous pharmaceutical compositions and dosage forms for use in the invention can be prepared using anhydrous or low moisture containing ingredients and low moisture or low humidity conditions. Pharmaceutical compositions and dosage forms for use in the invention which contain lactose can be made anhydrous if substantial contact with moisture and/or humidity during manufacturing, packaging, and/or storage is expected. An anhydrous pharmaceutical composition may be prepared and stored such that its anhydrous nature is maintained. Accordingly, anhydrous compositions may be packaged using materials known to prevent exposure to water such that they can be included in suitable formulary kits. Examples of suitable packaging include, but are not limited to, hermetically sealed foils, plastic or the like, unit dose containers, blister packs, and strip packs.

Each of the PI3K, JAK-2, PD-1, PD-L1, and/or BTK inhibitors as active ingredients can be combined in an intimate admixture with a pharmaceutical carrier according to conventional pharmaceutical compounding techniques. The carrier can take a wide variety of forms depending on the form of preparation desired for administration. In preparing the compositions for an oral dosage form, any of the usual pharmaceutical media can be employed as carriers, such as, for example, water, glycols, oils, alcohols, flavoring agents, preservatives, and coloring agents in the case of oral liquid preparations (such as suspensions, solutions, and elixirs) or aerosols; or carriers such as starches, sugars, micro-crystalline cellulose, diluents, granulating agents, lubricants, binders, and disintegrating agents can be used in the case of oral solid preparations, in some embodiments without employing the use of lactose. For example, suitable carriers include powders, capsules, and tablets, with the solid oral preparations. If desired, tablets can be coated by standard aqueous or nonaqueous techniques.

Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (e.g., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, microcrystalline cellulose, and mixtures thereof.

Examples of suitable fillers for use in the pharmaceutical compositions and dosage forms disclosed herein include, but are not limited to, talc, calcium carbonate (e.g., granules or powder), microcrystalline cellulose, powdered cellulose, dextrates, kaolin, mannitol, silicic acid, sorbitol, starch, pre-gelatinized starch, and mixtures thereof.

Disintegrants may be used in the compositions for use in the invention to provide tablets that disintegrate when exposed to an aqueous environment. Too much of a disintegrant may produce tablets which disintegrate in the bottle. Too little may be insufficient for disintegration to occur, thus altering the rate and extent of release of the active ingredients from the dosage form. Thus, a sufficient amount of disintegrant that is neither too little nor too much to detrimentally alter the release of the active ingredient(s) may be used to form the dosage forms of the compounds disclosed herein. The amount of disintegrant used may vary based upon the type of formulation and mode of administration, and may be readily discernible to those of ordinary skill in the art. About 0.5 to about 15 weight percent of disintegrant, or about 1 to about 5 weight percent of disintegrant, may be used in the pharmaceutical composition. Disintegrants that can be used to form pharmaceutical compositions and dosage forms for use in the invention include, but are not limited to, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums or mixtures thereof.

Lubricants which can be used to form pharmaceutical compositions and dosage forms for use in the invention include, but are not limited to, calcium stearate, magnesium stearate, sodium stearyl fumarate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethylaureate, agar, or mixtures thereof. Additional lubricants include, for example, a syloid silica gel, a coagulated aerosol of synthetic silica, silicified microcrystalline cellulose, or mixtures thereof. A lubricant can optionally be added in an amount of less than about 0.5% or less than about 1% (by weight) of the pharmaceutical composition.

When aqueous suspensions and/or elixirs are desired for oral administration, the essential active ingredient therein may be combined with various sweetening or flavoring agents, coloring matter or dyes and, if so desired, emulsifying and/or suspending agents, together with such diluents as water, ethanol, propylene glycol, glycerin and various combinations thereof.

The tablets can be uncoated or coated by known techniques to delay disintegration and absorption in the gastrointestinal tract and thereby provide a sustained action over a longer period. For example, a time delay material such as glyceryl monostearate or glyceryl distearate can be employed. Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin or olive oil.

Surfactants which can be used to form pharmaceutical compositions and dosage forms for use in the invention include, but are not limited to, hydrophilic surfactants, lipophilic surfactants, and mixtures thereof. That is, a mixture of hydrophilic surfactants may be employed, a mixture of lipophilic surfactants may be employed, or a mixture of at least one hydrophilic surfactant and at least one lipophilic surfactant may be employed.

A suitable hydrophilic surfactant may generally have an HLB value of at least 10, while suitable lipophilic surfactants may generally have an HLB value of or less than about 10. An empirical parameter used to characterize the relative hydrophilicity and hydrophobicity of non-ionic amphiphilic compounds is the hydrophilic-lipophilic balance ("HLB" value). Surfactants with lower HLB values are more lipophilic or hydrophobic, and have greater solubility in oils, while surfactants with higher HLB values are more hydrophilic, and have greater solubility in aqueous solutions. Hydrophilic surfactants are generally considered to be those compounds having an HLB value greater than about 10, as well as anionic, cationic, or zwitterionic compounds for which the HLB scale is not generally applicable. Similarly, lipophilic (*i*.*e*., hydrophobic) surfactants are compounds having an HLB value equal to or less than about 10. However, HLB value of a surfactant is merely a rough guide generally used to enable formulation of industrial, pharmaceutical and cosmetic emulsions.

Hydrophilic surfactants may be either ionic or non-ionic. Suitable ionic surfactants include, but are not limited to, alkylammonium salts; fusidic acid salts; fatty acid derivatives of amino acids, oligopeptides, and polypeptides; glyceride derivatives of amino acids, oligopeptides, and polypeptides; lecithins and hydrogenated lecithins; lysolecithins and hydrogenated lysolecithins; phospholipids and derivatives thereof; lysophospholipids and derivatives thereof; carnitine fatty acid ester salts; salts of alkylsulfates; fatty acid salts; sodium docusate; acylactylates; mono- and di-acetylated tartaric acid esters of mono- and di-glycerides; succinylated mono- and di-glycerides; citric acid esters of mono- and di-glycerides; and mixtures thereof.

Within the aforementioned group, ionic surfactants include, by way of example: lecithins, lysolecithin, phospholipids, lysophospholipids and derivatives thereof; carnitine fatty acid ester salts; salts of alkylsulfates; fatty acid salts; sodium docusate; acylactylates; mono- and di-acetylated tartaric acid esters of mono- and di-glycerides; succinylated mono- and di-glycerides; citric acid esters of mono- and di-glycerides; and mixtures thereof.

Ionic surfactants may be the ionized forms of lecithin, lysolecithin, phosphatidylcholine, phosphatidylethanolamine, phosphatidylglycerol, phosphatidic acid, phosphatidylserine, lysophosphatidylcholine, lysophosphatidylethanolamine, lysophosphatidylglycerol, lysophosphatidic acid, lysophosphatidylserine, PEG-phosphatidylethanolamine, PVP-phosphatidylethanolamine, lactylic esters of fatty acids, stearoyl-2-lactylate, stearoyl lactylate, succinylated monoglycerides, mono/diacetylated tartaric acid esters of mono/diglycerides, citric acid esters of mono/diglycerides, cholylsarcosine, caproate, caprylate, caprate, laurate, myristate, palmitate, oleate, ricinoleate, linoleate, linolenate, stearate, lauryl sulfate, teracecyl sulfate, docusate, lauroyl carnitines, palmitoyl carnitines, myristoyl carnitines, and salts and mixtures thereof.

Hydrophilic non-ionic surfactants may include alkylglucosides; alkylmaltosides; alkylthioglucosides; lauryl macrogolglycerides; polyoxyalkylene alkyl ethers such as polyethylene glycol alkyl ethers; polyoxyalkylene alkylphenols such as polyethylene glycol alkyl phenols; polyoxyalkylene alkyl phenol fatty acid esters such as polyethylene glycol fatty acids monoesters and polyethylene glycol fatty acids diesters; polyethylene glycol glycerol fatty acid esters; polyglycerol fatty acid esters; polyoxyalkylene sorbitan fatty acid esters such as polyethylene glycol sorbitan fatty acid esters; hydrophilic transesterification products of a polyol with at least one member of the group consisting of glycerides, vegetable oils, hydrogenated vegetable oils, fatty acids, and sterols; polyoxyethylene sterols, derivatives, and analogues thereof; polyoxyethylated vitamins and derivatives thereof; polyoxyethylene-polyoxypropylene block copolymers; and mixtures thereof; polyethylene glycol sorbitan fatty acid esters and hydrophilic transesterification products of a polyol with at least one member of the group consisting of triglycerides, vegetable oils, and hydrogenated vegetable oils. The polyol may be glycerol, ethylene glycol, polyethylene glycol, sorbitol, propylene glycol, pentaerythritol, or a saccharide.

Other hydrophilic-non-ionic surfactants include, without limitation, PEG-10 laurate, PEG-12 laurate, PEG-20 laurate, PEG-32 laurate, PEG-32 dilaurate, PEG-12 oleate, PEG-15 oleate, PEG-20 oleate, PEG-20 dioleate, PEG-32 oleate, PEG-200 oleate, PEG-400 oleate, PEG-15 stearate, PEG-32 distearate, PEG-40 stearate, PEG-100 stearate, PEG-20 dilaurate, PEG-25 glyceryl trioleate, PEG-32 dioleate, PEG-20 glyceryl laurate, PEG-30 glyceryl laurate, PEG-20 glyceryl stearate, PEG-20 glyceryl oleate, PEG-30 glyceryl oleate, PEG-30 glyceryl laurate, PEG-40 glyceryl laurate, PEG-40 palm kernel oil, PEG-50 hydrogenated castor oil, PEG-40 castor oil, PEG-35 castor oil, PEG-60 castor oil, PEG-40 hydrogenated castor oil, PEG-60 hydrogenated castor oil, PEG-60 corn oil, PEG-6 caprate/caprylate glycerides, PEG-8 caprate/caprylate glycerides, polyglyceryl-10 laurate, PEG-30 cholesterol, PEG-25 phyto sterol, PEG-30 soya sterol, PEG-20 trioleate, PEG-40 sorbitan oleate, PEG-80 sorbitan laurate, polysorbate 20, polysorbate 80, POE-9 lauryl ether, POE-23 lauryl ether, POE-10 oleyl ether, POE-20 oleyl ether, POE-20 stearyl ether, tocopheryl PEG-100 succinate, PEG-24 cholesterol, polyglyceryl-10oleate, Tween 40, Tween 60, sucrose monostearate, sucrose monolaurate, sucrose monopalmitate, PEG 10-100 nonyl phenol series, PEG 15-100 octyl phenol series, and poloxamers.

Suitable lipophilic surfactants include, by way of example only: fatty alcohols; glycerol fatty acid esters; acetylated glycerol fatty acid esters; lower alcohol fatty acids esters; propylene glycol fatty acid esters; sorbitan fatty acid esters; polyethylene glycol sorbitan fatty acid esters; sterols and sterol derivatives; polyoxyethylated sterols and sterol derivatives; polyethylene glycol alkyl ethers; sugar esters; sugar ethers; lactic acid derivatives of mono- and di-glycerides; hydrophobic transesterification products of a polyol with at least one member of the group consisting of glycerides, vegetable oils, hydrogenated vegetable oils, fatty acids and sterols; oil-soluble vitamins/vitamin derivatives; and mixtures thereof. Within this group, preferred lipophilic surfactants include glycerol fatty acid esters, propylene glycol fatty acid esters, and mixtures thereof, or are hydrophobic transesterification products of a polyol with at least one member of the group consisting of vegetable oils, hydrogenated vegetable oils, and triglycerides.

In an embodiment, the composition may include a solubilizer to ensure good solubilization and/or dissolution of the compounds disclosed herein and to minimize precipitation of the compounds. This can be especially important for compositions for non-oral use - *e.g.*, compositions for injection. A solubilizer may also be added to increase the solubility of the hydrophilic drug and/or other components, such as surfactants, or to maintain the composition as a stable or homogeneous solution or dispersion.

Examples of suitable solubilizers include, but are not limited to, the following: alcohols and polyols, such as ethanol, isopropanol, butanol, benzyl alcohol, ethylene glycol, propylene glycol, butanediols and isomers thereof, glycerol, pentaerythritol, sorbitol, mannitol, transcutol, dimethyl isosorbide, polyethylene glycol, polypropylene glycol, polyvinylalcohol, hydroxypropyl methylcellulose and other cellulose derivatives, cyclodextrins and cyclodextrin derivatives; ethers of polyethylene glycols having an average molecular weight of about 200 to about 6000, such as tetrahydrofurfuryl alcohol PEG ether (glycofurol) or methoxy PEG; amides and other nitrogen-containing compounds such as 2-pyrrolidone, 2-piperidone, ε-caprolactam, N-alkylpyrrolidone, N-hydroxyalkylpyrrolidone, N-alkylpiperidone, N-alkylcaprolactam, dimethylacetamide and polyvinylpyrrolidone; esters such as ethyl propionate, tributylcitrate, acetyl triethylcitrate, acetyl tributyl citrate, triethylcitrate, ethyl oleate, ethyl caprylate, ethyl butyrate, triacetin, propylene glycol monoacetate, propylene glycol diacetate, .epsilon.-caprolactone and isomers thereof, δ-valerolactone and isomers thereof, β-butyrolactone and isomers thereof; and other solubilizers known in the art, such as dimethyl acetamide, dimethyl isosorbide, N-methyl pyrrolidones, monooctanoin, diethylene glycol monoethyl ether, and water.

Mixtures of solubilizers may also be used. Examples include triacetin, triethylcitrate, ethyl oleate, ethyl caprylate, dimethylacetamide, N-methylpyrrolidone, N-hydroxyethylpyrrolidone, polyvinylpyrrolidone, hydroxypropyl methylcellulose, hydroxypropyl cyclodextrins, ethanol, polyethylene glycol 200-100, glycofurol, transcutol, propylene glycol, and dimethyl isosorbide. Particularly preferred solubilizers include sorbitol, glycerol, triacetin, ethyl alcohol, PEG-400, glycofurol and propylene glycol.

The amount of solubilizer that can be included is not particularly limited. The amount of a given solubilizer may be limited to a bioacceptable amount, which may be readily determined by one of skill in the art. In some circumstances, it may be advantageous to include amounts of solubilizers far in excess of bioacceptable amounts, for example to maximize the concentration of the drug, with excess solubilizer removed prior to providing the composition to a patient using conventional techniques, such as distillation or evaporation. Thus, if present, the solubilizer can be in a weight ratio of 10%, 25%, 50%, 100%, or up to about 200% by weight, based on the combined weight of the drug, and other excipients. If desired, very small amounts of solubilizer may also be used, such as 5%, 2%, 1% or even less. Typically, the solubilizer may be present in an amount of about 1% to about 100%, more typically about 5% to about 25% by weight.

The composition can further include one or more pharmaceutically acceptable additives and excipients. Such additives and excipients include, without limitation, detackifiers, anti-foaming agents, buffering agents, polymers, antioxidants, preservatives, chelating agents, viscomodulators, tonicifiers, flavorants, colorants, odorants, opacifiers, suspending agents, binders, fillers, plasticizers, lubricants, and mixtures thereof.

In addition, an acid or a base may be incorporated into the composition to facilitate processing, to enhance stability, or for other reasons. Examples of pharmaceutically acceptable bases include amino acids, amino acid esters, ammonium hydroxide, potassium hydroxide, sodium hydroxide, sodium hydrogen carbonate, aluminum hydroxide, calcium carbonate, magnesium hydroxide, magnesium aluminum silicate, synthetic aluminum silicate, synthetic hydrocalcite, magnesium aluminum hydroxide, diisopropylethylamine, ethanolamine, ethylenediamine, triethanolamine, triethylamine, triisopropanolamine, trimethylamine, and tris(hydroxymethyl)aminomethane (TRIS). Also suitable are bases that are salts of a pharmaceutically acceptable acid, such as acetic acid, acrylic acid, adipic acid, alginic acid, alkanesulfonic acid, amino acids, ascorbic acid, benzoic acid, boric acid, butyric acid, carbonic acid, citric acid, fatty acids, formic acid, fumaric acid, gluconic acid, hydroquinosulfonic acid, isoascorbic acid, lactic acid, maleic acid, oxalic acid, para-bromophenylsulfonic acid, propionic acid, p-toluenesulfonic acid, salicylic acid, stearic acid, succinic acid, tannic acid, tartaric acid, thioglycolic acid, toluenesulfonic acid, and uric acid,. Salts of polyprotic acids, such as sodium phosphate, disodium hydrogen phosphate, and sodium dihydrogen phosphate can also be used. When the base is a salt, the cation can be any convenient and pharmaceutically acceptable cation, such as ammonium, alkali metals and alkaline earth metals. Example may include sodium, potassium, lithium, magnesium, calcium and ammonium.

Suitable acids are pharmaceutically acceptable organic or inorganic acids. Examples of suitable inorganic acids include hydrochloric acid, hydrobromic acid, hydriodic acid, sulfuric acid, nitric acid, boric acid, and phosphoric acid. Examples of suitable organic acids include acetic acid, acrylic acid, adipic acid, alginic acid, alkanesulfonic acids, amino acids, ascorbic acid, benzoic acid, boric acid, butyric acid, carbonic acid, citric acid, fatty acids, formic acid, fumaric acid, gluconic acid, hydroquinosulfonic acid, isoascorbic acid, lactic acid, maleic acid, methanesulfonic acid, oxalic acid, para-bromophenylsulfonic acid, propionic acid, p-toluenesulfonic acid, salicylic acid, stearic acid, succinic acid, tannic acid, tartaric acid, thioglycolic acid, toluenesulfonic acid and uric acid.

### Pharmaceutical Compositions for Injection

In some embodiments, the invention provides a pharmaceutical composition for use in the treatment of cancer and for injection containing the combination of the a BTK inhibitor of formula (XVIII) or a pharmaceutically acceptable salt thereof, and a PD-1 inhibitor or a PD-L1 inhibitor and optionally a PI3K, and /or JAK-2, inhibitor and a pharmaceutical excipient suitable for injection, wherein the PD-1 inhibitor is pembrolizumab and the PD-L1 inhibitor is durvalumab. Components and amounts of active pharmaceutical ingredients in the compositions are as described herein.

The forms in which the compositions for use in the present invention may be incorporated for administration by injection include aqueous or oil suspensions, or emulsions, with sesame oil, corn oil, cottonseed oil, or peanut oil, as well as elixirs, mannitol, dextrose, or a sterile aqueous solution, and similar pharmaceutical vehicles.

Aqueous solutions in saline are also conventionally used for injection. Ethanol, glycerol, propylene glycol and liquid polyethylene glycol (and suitable mixtures thereof), cyclodextrin derivatives, and vegetable oils may also be employed. The proper fluidity can be maintained, for example, by the use of a coating, such as lecithin, for the maintenance of the required particle size in the case of dispersion and by the use of surfactants. The prevention of the action of microorganisms can be brought about by various antibacterial and antifungal agents, for example, parabens, chlorobutanol, phenol, sorbic acid and thimerosal.

Sterile injectable solutions are prepared by incorporating the combination of the BTK inhibitor, and PD-1 or PD-L1 inhibitor and optionally, the PI3K inhibitors in the required amounts in the appropriate solvent with various other ingredients as enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, certain desirable methods of preparation are spray-drying, vacuum-drying and freeze-drying (lyophilization) techniques which yield a powder of the active ingredient plus any additional desired ingredient from a previously sterile-filtered solution thereof. Other lyophilized or spray-dried antibody formulations known to those of skill in the art may also be employed for use in the present invention. Such formulations include those disclosed in U.S. Patent Nos. 5,908,826, 6,267,958, 7,682,609, 7,592,004, and 8,298,530, and U.S. Patent Application Publication No. 2010/0158925.

### Pharmaceutical Compositions for Topical Delivery

In some embodiments, the invention provides a pharmaceutical composition for use in the treatment of cancer and for transdermal delivery containing the combination of a BTK inhibitor of formula (XVIII) or a pharmaceutically acceptable salt thereof, and a PD-1 inhibitor or a PD-L1 inhibitor and optionally a PI3K, and/or JAK-2, inhibitor and a pharmaceutical excipient suitable for transdermal delivery, wherein the PD-1 inhibitor is pembrolizumab and the PD-L1 inhibitor is durvalumab.

Compositions for use in the present invention can be formulated into preparations in solid, semi-solid, or liquid forms suitable for local or topical administration, such as gels, water soluble jellies, creams, lotions, suspensions, foams, powders, slurries, ointments, solutions, oils, pastes, suppositories, sprays, emulsions, saline solutions, dimethylsulfoxide (DMSO)-based solutions. In general, carriers with higher densities are capable of providing an area with a prolonged exposure to the active ingredients. In contrast, a solution formulation may provide more immediate exposure of the active ingredient to the chosen area.

The pharmaceutical compositions also may comprise suitable solid or gel phase carriers or excipients, which are compounds that allow increased penetration of, or assist in the delivery of, therapeutic molecules across the stratum corneum permeability barrier of the skin. There are many of these penetration-enhancing molecules known to those trained in the art of topical formulation. Examples of such carriers and excipients include, but are not limited to, humectants (*e.g.,* urea), glycols (*e.g.,* propylene glycol), alcohols (*e.g.,* ethanol), fatty acids (*e.g.,* oleic acid), surfactants (*e.g.,* isopropyl myristate and sodium lauryl sulfate), pyrrolidones, glycerol monolaurate, sulfoxides, terpenes (*e.g*., menthol), amines, amides, alkanes, alkanols, water, calcium carbonate, calcium phosphate, various sugars, starches, cellulose derivatives, gelatin, and polymers such as polyethylene glycols.

Another formulation for use in the the present invention employs transdermal delivery devices ("patches"). Such transdermal patches may be used to provide continuous or discontinuous infusion of the combination of a BTK inhibitor of formula (XVIII) or a pharmaceutically acceptable salt thereof, and a PD-1 inhibitor or a PD-L1 inhibitor and optionally a PI3K inhibitor in controlled amounts, either with or without another active pharmaceutical ingredient, wherein the PD-1 inhibitor is pembrolizumab and the PD-L1 inhibitor is durvalumab.

The construction and use of transdermal patches for the delivery of active pharmaceutical ingredients is well known in the art. See, *e.g.,* U.S. Patent Nos. 5,023,252; 4,992,445 and 5,001,139. Such patches may be constructed for continuous, pulsatile, or on demand delivery of active pharmaceutical ingredients.

### Other Pharmaceutical Compositions

Pharmaceutical compositions may also be prepared from compositions described herein and one or more pharmaceutically acceptable excipients suitable for sublingual, buccal, rectal, intraosseous, intraocular, intranasal, epidural, or intraspinal administration. Preparations for such pharmaceutical compositions are well-known in the art. See, *e.g.,* Anderson, et al., Handbook of Clinical Drug Data, Tenth Edition, McGraw-Hill, 2002; and Pratt and Taylor, eds., Principles of Drug Action, Third Edition, Churchill Livingston, N.Y., 1990.

Administration of the combination of a BTK inhibitor of formula (XVIII) or a pharmaceutically acceptable salt thereof, and a PD-1 inhibitor or a PD-L1 inhibitor and optionally a PI3K, and/or a JAK-2 inhibitor or pharmaceutical composition of these compounds, wherein the PD-1 inhibitor is pembrolizumab and the PD-L1 inhibitor is durvalumab, can be effected by any method that enables delivery of the compounds to the site of action. These methods include oral routes, intraduodenal routes, parenteral injection (including intravenous, intraarterial, subcutaneous, intramuscular, intravascular, intraperitoneal or infusion), topical (*e.g.,* transdermal application), rectal administration, via local delivery by catheter or stent or through inhalation. The combination of compounds can also be administered intraadiposally or intrathecally.

Parenteral administration forms include solutions or suspensions of active compound in sterile aqueous solutions, for example, aqueous propylene glycol or dextrose solutions. Such dosage forms can be suitably buffered, if desired.

The invention also provides the use of kits. The kits include a BTK inhibitor of formula (XVIII) or a pharmaceutically acceptable salt thereof, and a PD-1 inhibitor or a PD-L1 inhibitor and optionally a PI3K inhibitor, either alone or in combination in suitable packaging, and written material that can include instructions for use, discussion of clinical studies and listing of side effects, wherein the PD-1 inhibitor is pembrolizumab and the PD-L1 inhibitor is durvalumab. Such kits may also include information, such as scientific literature references, package insert materials, clinical trial results, and/or summaries of these, which indicate or establish the activities and/or advantages of the composition, and/or which describe dosing, administration, side effects, drug interactions, or other information useful to the health care provider. Such information may be based on the results of various studies, for example, studies using experimental animals involving in vivo models and studies based on human clinical trials. The kit may further contain another active pharmaceutical ingredient. In some embodiments, the BTK inhibitor of formula (XVIII) or a pharmaceutically acceptable salt thereof, and the PD-1 inhibitor or PD-L1 inhibitor and the active pharmaceutical ingredient are provided as separate compositions in separate containers within the kit. In some embodiments, the BTK inhibitor of formula (XVIII) or a pharmaceutically acceptable salt thereof, and the PD-1 inhibitor or PD-L1 inhibitor and the active pharmaceutical ingredient are provided as a single composition within a container in the kit. Suitable packaging and additional articles for use (e.g., measuring cup for liquid preparations, and foil wrapping to minimize exposure to air) are known in the art and may be included in the kit. Kits described herein can be provided, marketed and/or promoted to health providers, including physicians, nurses, pharmacists, and formulary officials. Kits may also, in some embodiments, be marketed directly to the consumer.

### Dosages and Dosing Regimens

The amounts of the combination of the BTK inhibitor of formula (XVIII) or a pharmaceutically acceptable salt thereof, and the PD-1 inhibitor (pembrolizumab) or PD-L1 inhibitor (durvalumab), wherein the PD-1 inhibitor is pembrolizumab and the PD-L1 inhibitor is durvalumab, and optionally a PI3K, and/or JAK-2 inhibitor administered will be dependent on the human being treated, the severity of the disorder or condition, the rate of administration, the disposition of the compounds and the discretion of the prescribing physician. However, an effective dosage is in the range of about 0.001 to about 100 mg per kg body weight per day, such as about 1 to about 35 mg/kg/day, in single or divided doses. For a 70 kg human, this would amount to about 0.05 to 7 g/day, such as about 0.05 to about 2.5 g/day. In some instances, dosage levels below the lower limit of the aforesaid range may be more than adequate, while in other cases still larger doses may be employed without causing any harmful side effect, for example by dividing such larger doses into several small doses for administration throughout the day.

In some embodiments, the combination of a BTK inhibitor of formula (XVIII) or a pharmaceutically acceptable salt thereof, and the PD-1 inhibitor (pembrolizumab) or PD-L1 (durvalumab) inhibitor and optionally a PI3K, and/or JAK-2 inhibitor is administered in a single dose. Typically, such administration will be by injection, for example by intravenous injection, in order to introduce the active pharmaceutical ingredients quickly. However, other routes may be used as appropriate. A single dose of the combination of a BTK inhibitor of formula (XVIII) or a pharmaceutically acceptable salt thereof, and the PD-1 inhibitor (pembrolizumab) or PD-L1 inhibitor (durvalumab) and optionally a PI3K, and/or JAK-2 inhibitor may also be used for treatment of an acute condition.

In some embodiments, the combination of a BTK inhibitor of formula (XVIII) or a pharmaceutically acceptable salt thereof, and the PD-1 inhibitor (pembrolizumab) or PD-L1 inhibitor (durvalumab) and optionally a PI3K, and/or JAK-2 inhibitor is administered in multiple doses. Dosing may be about once, twice, three times, four times, five times, six times, or more than six times per day. Dosing may be about once a month, once every two weeks, once a week, or once every other day. In other embodiments, the combination of a BTK inhibitor of formula (XVIII) or a pharmaceutically acceptable salt thereof, and the PD-1 inhibitor (pembrolizumab) or PD-L1 inhibitor (durvalumab) and optionally a PI3K, and/or JAK-2 inhibitor is administered about once per day to about 6 times per day. In another embodiment the administration of the combination of a BTK inhibitor of formula (XVIII) or a pharmaceutically acceptable salt thereof, and the PD-1 inhibitor (pembrolizumab) or PD-L1 inhibitor (durvalumab) and optionally a the PI3K, and/or JAK-2 inhibitor continues for less than about 7 days. In yet another embodiment the administration continues for more than about 6, 10, 14, 28 days, two months, six months, or one year. In some cases, continuous dosing is achieved and maintained as long as necessary.

Administration of the active pharmaceutical ingredients for use in the invention may continue as long as necessary. In some embodiments, the combination of a BTK inhibitor of formula (XVIII) or a pharmaceutically acceptable salt thereof, and the PD-1 inhibitor (pembrolizumab) or PD-L1 inhibitor (durvalumab) and optionally a PI3K, and/or JAK-2 inhibitor is administered for more than 1, 2, 3, 4, 5, 6, 7, 14, or 28 days. In some embodiments, the combination of a BTK inhibitor of formula (XVIII) or a pharmaceutically acceptable salt thereof, and the PD-1 inhibitor (pembrolizumab) or PD-L1 inhibitor (durvalumab) and optionally a PI3K, inhibitor is administered for less than 28, 14, 7, 6, 5, 4, 3, 2, or 1 day. In some embodiments, the combination of a BTK inhibitor of formula (XVIII) or a pharmaceutically acceptable salt thereof, and the PD-1 inhibitor (pembrolizumab) or PD-L1 inhibitor (durvalumab) and optionally a PI3K, and/or JAK-2 inhibitors is administered chronically on an ongoing basis - *e.g.,* for the treatment of chronic effects.

In some embodiments, an effective dosage of each of the BTK, PI3K, PD-1, PD-L1, or JAK-2 inhibitors is in the range of about 1 mg to about 500 mg, about 10 mg to about 300 mg, about 20 mg to about 250 mg, about 25 mg to about 200 mg, about 10 mg to about 200 mg, about 20 mg to about 150 mg, about 30 mg to about 120 mg, about 10 mg to about 90 mg, about 20 mg to about 80 mg, about 30 mg to about 70 mg, about 40 mg to about 60 mg, about 45 mg to about 55 mg, about 48 mg to about 52 mg, about 50 mg to about 150 mg, about 60 mg to about 140 mg, about 70 mg to about 130 mg, about 80 mg to about 120 mg, about 90 mg to about 110 mg, about 95 mg to about 105 mg, about 150 mg to about 250 mg, about 160 mg to about 240 mg, about 170 mg to about 230 mg, about 180 mg to about 220 mg, about 190 mg to about 210 mg, about 195 mg to about 205 mg, or about 198 to about 202 mg. In some embodiments, an effective dosage of each of the BTK, PI3K, PD-1, PD-L1, or JAK-2 inhibitors is about 25 mg, about 50 mg, about 75 mg, about 100 mg, about 125 mg, about 150 mg, about 175 mg, about 200 mg, about 225 mg, about 250 mg, about 275 mg, about 300 mg, about 325 mg, about 350 mg, about 375 mg, about 400 mg, about 425 mg, about 450 mg, about 475 mg, or about 500 mg. In some embodiments, an effective dosage of each of the BTK, PI3K, PD-1, PD-L1, or JAK-2 inhibitors is 25 mg, 50 mg, 75 mg, 100 mg, 125 mg, 150 mg, 175 mg, 200 mg, 225 mg, 250 mg, 275 mg, 300 mg, 325 mg, 350 mg, 375 mg, 400 mg, 425 mg, 450 mg, 475 mg, or 500 mg.

In some embodiments, an effective dosage of each of the BTK, PI3K, PD-1, PD-L1, or JAK-2 inhibitors is in the range of about 0.01 mg/kg to about 4.3 mg/kg, about 0.15 mg/kg to about 3.6 mg/kg, about 0.3 mg/kg to about 3.2 mg/kg, about 0.35 mg/kg to about 2.85 mg/kg, about 0.15 mg/kg to about 2.85 mg/kg, about 0.3 mg to about 2.15 mg/kg, about 0.45 mg/kg to about 1.7 mg/kg, about 0.15 mg/kg to about 1.3 mg/kg, about 0.3 mg/kg to about 1.15 mg/kg, about 0.45 mg/kg to about 1 mg/kg, about 0.55 mg/kg to about 0.85 mg/kg, about 0.65 mg/kg to about 0.8 mg/kg, about 0.7 mg/kg to about 0.75 mg/kg, about 0.7 mg/kg to about 2.15 mg/kg, about 0.85 mg/kg to about 2 mg/kg, about 1 mg/kg to about 1.85 mg/kg, about 1.15 mg/kg to about 1.7 mg/kg, about 1.3 mg/kg mg to about 1.6 mg/kg, about 1.35 mg/kg to about 1.5 mg/kg, about 2.15 mg/kg to about 3.6 mg/kg, about 2.3 mg/kg to about 3.4 mg/kg, about 2.4 mg/kg to about 3.3 mg/kg, about 2.6 mg/kg to about 3.15 mg/kg, about 2.7 mg/kg to about 3 mg/kg, about 2.8 mg/kg to about 3 mg/kg, or about 2.85 mg/kg to about 2.95 mg/kg. In some embodiments, an effective dosage of each of the BTK, PI3K, PD-1, PD-L1, or JAK-2 inhibitors is about 0.35 mg/kg, about 0.7 mg/kg, about 1 mg/kg, about 1.4 mg/kg, about 1.8 mg/kg, about 2.1 mg/kg, about 2.5 mg/kg, about 2.85 mg/kg, about 3.2 mg/kg, or about 3.6 mg/kg.

In some embodiments, an inhibitor of each of the BTK, PI3K, PD-1, PD-L1, or JAK-2 inhibitors is adminstered at a dosage of 10 to 400 mg BID, including a dosage of 5 mg, 10 mg, 12.5 mg, 25 mg, 40 mg, 50 mg, 75 mg, 100 mg, 150 mg, 175 mg, 200 mg, 225 mg, 250 mg, 275 mg, 300 mg, 325 mg, 350 mg, 375 mg, 400 mg, 425 mg, 450 mg, 475 mg, and 500 mg BID.

In some embodiments, a dose of the PD-1 or PD-L1 inhibitors is administered at a concentration of 10 mg/mL, 25 mg/mL, 40 mg/mL, 50 mg/mL, 60 mg/mL, 75 mg/mL, 100 mg/mL, and 200 mg/mL.

An effective amount of the combination of a BTK inhibitor of formula (XVIII) or a pharmaceutically acceptable salt thereof, and the PD-1 inhibitor (pembrolizumab) or PD-L1 inhibitor (durvalumab) and optionally a PI3K, and JAK-2 inhibitors may be administered in either single or multiple doses by any of the accepted modes of administration of active pharmaceutical ingredients having similar utilities, including rectal, buccal, intranasal and transdermal routes, by intra-arterial injection, intravenously, intraperitoneally, parenterally, intramuscularly, subcutaneously, orally, topically, or as an inhalant.

### Use in Treating Solid Tumor Cancers

In some embodiments, the invention relates to a combination as described above for use in treating a hyperproliferative disorder in a mammal, wherein the hyperproliferative disorder is a solid tumor cancer.

In some embodiments, the invention relates to a combination of a BTK inhibitor of formula (XVIII) or a pharmaceutically acceptable salt thereof and a PD-1 (pembrolizumab) or PD-L1 inhibitor (durvalumab) and optionally a PI3K inhibitor, including a PI3K-γ or PI3K-δ inhibitor, and/or a JAK-2 inhibitor, for use in treating a solid tumor cancer in a human selected from the group consisting of bladder cancer, squamous cell carcinoma, head and neck cancer, pancreatic ductal adenocarcinoma (PDA), pancreatic cancer, colon carcinoma, mammary carcinoma, breast cancer, fibrosarcoma, mesothelioma, renal cell carcinoma, lung carcinoma, thyoma, prostate cancer, colorectal cancer, ovarian cancer, acute myeloid leukemia, thymus cancer, brain cancer, squamous cell cancer, skin cancer, eye cancer, retinoblastoma, melanoma, intraocular melanoma, oral cavity cancer, oropharyngeal cancer, gastric cancer, stomach cancer, cervical cancer, renal cancer, kidney cancer, liver cancer, ovarian cancer, prostate cancer, colorectal cancer, esophageal cancer, testicular cancer, gynecological cancer, thyroid cancer, acquired immune deficiency syndrome (AIDS)-related cancers (e.g., lymphoma and Kaposi's sarcoma), viral-induced cancers such as cervical carcinoma (human papillomavirus), B-cell lymphoproliferative disease, nasopharyngeal carcinoma (Epstein-Barr virus), Kaposi's sarcoma and primary effusion lymphomas (Kaposi's sarcoma herpesvirus), hepatocellular carcinoma (hepatitis B and hepatitis C viruses), and T-cell leukemias (Human T-cell leukemia virus-1), glioblastoma, glioma, esophogeal tumors, head and neck tumor, metastatic colon cancer, head and neck squamous cell carcinoma, ovary tumor, pancreas tumor, renal cell carcinoma, hematological neoplasms, small-cell lung cancer, non-small-cell lung cancer, stage IV melanoma, and glioma.

In some embodiments, the invention relates to a combination as described above for use in treating a solid tumor cancer, wherein the dose is effective to inhibit signaling between the solid tumor cells and at least one microenvironment selected from the group consisting of macrophages, monocytes, mast cells, helper T cells, cytotoxic T cells, regulatory T cells, natural killer cells, myeloid-derived suppressor cells, regulatory B cells, neutrophils, dendritic cells, and fibroblasts. In some embodiments, the invention relates to a combination as described above for use in treating pancreatic cancer, breast cancer, ovarian cancer, melanoma, lung cancer, squamous cell carcinoma including head and neck cancer, and colorectal cancer, wherein the dose is effective to inhibit signaling between the solid tumor cells and at least one microenvironment selected from the group consisting of macrophages, monocytes, mast cells, helper T cells, cytotoxic T cells, regulatory T cells, natural killer cells, myeloid-derived suppressor cells, regulatory B cells, neutrophils, dendritic cells, and fibroblasts. In some embodiments, the invention provides a combination as described above for use in treating a glioma, wherein the glioma is selected from the group consisting of fibrillary astrocytoma, anaplastic astrocytoma, pilocytic astrocytoma, astrocytoma, pleomorphic xanthoastrocytoma, subependymal giant cell astrocytoma, glioblastoma multiforme, oligodendroglioma, ependymoma, subependymoma, choroid plexus tumor, choroid plexus papilloma, choroid plexus carcinoma, oligoastrocytoma, gliomatosis cerebri, and gliosarcoma. In some embodiments, the invention provides a combination for use in treating a cancer, wherein the cancer is selected from primary central nervous system lymphoma, reticulum cell sarcoma, diffuse histiocytic lymphoma, and microglioma.

Efficacy of the compounds and combinations of compounds described herein in treating, preventing, ameliorating, and/or managing the indicated diseases or disorders can be tested using various models known in the art. For example, models for determining efficacy of treatments for pancreatic cancer are described in Herreros-Villanueva, et al., World J. Gastroenterol. 2012, 18, 1286-1294. Models for determining efficacy of treatments for breast cancer are described, *e.g.,* in Fantozzi, Breast Cancer Res. 2006, 8, 212. Models for determining efficacy of treatments for ovarian cancer are described, for example, in Mullany, et al., Endocrinology 2012,153, 1585-92; and Fong, et al., J. Ovarian Res. 2009, 2, 12. Models for determining efficacy of treatments for melanoma are described, for example, in Damsky, et al., Pigment Cell & Melanoma Res. 2010, 23, 853-859. Models for determining efficacy of treatments for lung cancer are described, for example, in Meuwissen, et al., Genes & Development, 2005,19, 643-664. Models for determining efficacy of treatments for lung cancer are described, for example, in Kim, Clin. Exp. Otorhinolaryngol. 2009, 2, 55-60; and Sano, Head Neck Oncol. 2009, 1, 32. Models for determining efficacy of treatments for colorectal cancer, including the CT26 model, are described below in the examples.

### Use in Treating Hematological Malignancies

In an embodiment, the invention relates to a combination as described above for use in treating a cancer in a human, wherein the cancer is a B cell hematological malignancy selected from the group consisting of chronic lymphocytic leukemia (CLL), small lymphocytic leukemia (SLL), non-Hodgkin's lymphoma (NHL), diffuse large B cell lymphoma (DLBCL), follicular lymphoma (FL), mantle cell lymphoma (MCL), Hodgkin's lymphoma, B cell acute lymphoblastic leukemia (B-ALL), Burkitt's lymphoma, Waldenström's macroglobulinemia (WM), Burkitt's lymphoma, multiple myeloma, myelodysplatic syndromes, or myelofibrosis. In an embodiment, the invention relates to a combination as described herein for use in treating a cancer in a human, wherein the cancer is chronic myelocytic leukemia, acute myeloid leukemia, DLBCL (including activated B-cell (ABC) and germinal center B-cell (GCB) subtypes), follicle center lymphoma, Hodgkin's disease, multiple myeloma, indolent non-Hodgkin's lymphoma, and mature B-cell ALL.

In an embodiment, the invention relates to a combination as described herein for use in treating CLL in a human. In some embodiments for treatment of CLL, a therapeutically effective amount of a PD-1 inhibitor (pembrolizumab), or a PD-L1 (durvalumab) inhibitor, is administered before, after or concurrently with a BTK inhibitor of formula (XVIII) or a pharmaceutically acceptable salt thereof. In some embodiments for treatment of CLL, a therapeutically effective amount of a PD-L1 inhibitor (pembrolizumab), is co-administered with a BTK inhibitor of formula (XVIII) or a pharmaceutically acceptable salt thereof. In some embodiments for treatment of SLL, a therapeutically effective amount of a PD-1 inhibitor (pembrolizumab), or a PD-L1 inhibitor (durvalumab), is also administered before, after or concurrently with a BTK inhibitor of formula (XVIII) or a pharmaceutically acceptable salt thereof. In some embodiments for treatment of SLL, a therapeutically effective amount of a PD-L1 inhibitor (pembrolizumab) is co-administered with a BTK inhibitor of formula (XVIII) or a pharmaceutically acceptable salt thereof. In an embodiment, the invention relates to a combination for use in treating CLL in a human wherein the treatment comprises the step of administering to said human a BTK inhibitor of Formula (XVIII), or a pharmaceutically acceptable salt thereof, in a dosing regimen selected from the group consisting of 100 mg QD, 175 mg QD, 250 mg QD, 400 mg QD, and 100 mg BID. In an embodiment, the invention relates to a combination for use in treating CLL in a human wherein the treatment comprises the step of administering to said human a BTK inhibitor of Formula (XVIII), or a pharmaceutically acceptable salt thereof, in a dosing regimen selected from the group consisting of 100 mg QD, 175 mg QD, 250 mg QD, 400 mg QD, and 100 mg BID.

In an embodiment, the invention relates to a combination as described herein for use in treating CLL in a human. In an embodiment, the invention relates to a combination as described herein for use in treating SLL in a . In some embodiments, the combination for use further comprises a therapeutically effective amount of a PI3K inhibitor, including a PI3K inhibitor of Formula (IX) or a pharmaceutically acceptable salt, solvate or hydrate thereof that is adminstered before, after, or concurrently with a therapeutically effective amount of a BTK inhibitor of formula (XVIII) or a pharmaceutically acceptable salt thereof.

In an embodiment, the invention relates to a combination as described herein for use in treating a subtype of CLL in a human. A number of subtypes of CLL have been characterized. CLL is often classified for immunoglobulin heavy-chain variable-region (IgV_{H}) mutational status in leukemic cells. R. N. Damle, et al., Blood 1999, 94, 1840-47; T. J. Hamblin, et al., Blood 1999, 94, 1848-54. Patients with IgV_{H} mutations generally survive longer than patients without IgV_{H} mutations. ZAP70 expression (positive or negative) is also used to characterize CLL. L. Z. Rassenti, et al., N. Engl. J. Med. 2004, 351, 893-901. The methylation of ZAP-70 at CpG3 is also used to characterize CLL, for example by pyrosequencing. R. Claus, et al., J. Clin. Oncol. 2012, 30, 2483-91; J. A. Woyach, et al., Blood 2014,123, 1810-17. CLL is also classified by stage of disease under the Binet or Rai criteria. J. L. Binet, et al., Cancer 1977, 40, 855-64; K. R. Rai, T. Han, Hematol. Oncol. Clin. North Am. 1990, 4, 447-56. Other common mutations, such as 11p deletion, 13q deletion, and 17p deletion can be assessed using well-known techniques such as fluorescence *in situ* hybridization (FISH). In an embodiment, the invention relates to a combination as described herein for use in treating a CLL in a human, wherein the CLL is selected from the group consisting of IgV_{H} mutation negative CLL, ZAP-70 positive CLL, ZAP-70 methylated at CpG3 CLL, CD38 positive CLL, chronic lymphocytic leukemia characterized by a 17p13.1 (17p) deletion, and CLL characterized by a 11q22.3 (11q) deletion.

In an embodiment, the invention relates to a combination as described herein for use in treating a CLL in a human, wherein the CLL has undergone a Richter's transformation. Methods of assessing Richter's transformation, which is also known as Richter's syndrome, are described in P. Jain and S. O'Brien, Oncology, 2012, 26, 1146-52. Richter's transformation is a subtype of CLL that is observed in 5-10% of patients. It involves the development of aggressive lymphoma from CLL and has a generally poor prognosis.

In an embodiment, the invention relates to a combination as described herein for use in treating a subtype of CLL in a human, wherein the subtype of CLL is a subtype of CLL that increases monocytes and NK cells in peripheral blood when measured after a period of treatment with the BTK inhibitor selected from the group consisting of about 14 days, about 28 days, about 56 days, about 1 month, about 2 months, about 3 months, about 6 months, and about 1 year, and wherein the term "about" refers to a measurement interval of +/- 2 days. In some embodiments for treatment of a subtype of CLL, a therapeutically effective amount of a PD-1 inhibitor (pembrolizumab) or a PD-L1 inhibitor (durvalumab) is administered before, after or concurrently with a BTK inhibitor of formula (XVIII) or a pharmaceutically acceptable salt thereof. In some embodiments for treatment of a subtype of CLL, a therapeutically effective amount of a PD-L1 inhibitor (durvalumab) is co-administered with a BTK inhibitor of formula (XVIII) or a pharmaceutically acceptable salt thereof.

In an embodiment, the invention relates to a combination as described herein for use in treating CLL or SLL in a patient, wherein the patient is sensitive to lymphocytosis. In an embodiment, the invention relates to a combination as described herein for use in treating CLL or SLL in a patient, wherein the patient exhibits lymphocytosis caused by a disorder selected from the group consisting of a viral infection, a bacterial infection, a protozoal infection, or a post-splenectomy state. In an embodiment, the viral infection in any of the foregoing embodiments is selected from the group consisting of infectious mononucleosis, hepatitis, and cytomegalovirus. In an embodiment, the bacterial infection in any of the foregoing embodiments is selected from the group consisting of pertussis, tuberculosis, and brucellosis.

The uses described above may be used as first-line cancer therapy, or after treatment with conventional chemotherapic active pharmaceutical ingredients, including cyclophosphamide, fludarabine, cyclophosphamide and fludarabine (FC chemotherapy), and chlorambucil. The uses described above may also be supplemented with immunotherapeutic monoclonal antibodies such as the anti-CD52 monoclonal antibody alemtuzumab. In an embodiment, the invention relates to a combination as described herein for use in treating CLL in a human wherein the combination further comprises an active pharmaceutical ingredient selected from the group consisting of cyclophosphamide, fludarabine, cyclophosphamide, chlorambucil, salts, solvates, or hydrates thereof, and combinations thereof, and alemtuzumab, antigen-binding fragments, derivatives, conjugates, variants, and radioisotope-labeled complexes thereof.

In an embodiment, the invention relates to a combination as described herein for use in treating hematological malignancies in a human. Hematological malignancies include CLL and SLL, as well as other cancers of the blood, including B cell malignancies. In an embodiment, the invention relates to a combination for use in treating a hematological malignancy selected from the group consisting of non-Hodgkin's lymphoma (NHL), diffuse large B cell lymphoma (DLBCL), follicular lymphoma (FL), mantle cell lymphoma (MCL), Hodgkin's lymphoma, B cell acute lymphoblastic leukemia (B-ALL), Waldenström's macroglobulinemia (WM), Burkitt's lymphoma, multiple myeloma, or myelofibrosis in a human. In some embodiments for treatment of hematological malignancies, a therapeutically effective amount of a PD-1 inhibitor (pembrolizumab) or a PD-L1 inhibitor (durvalumab) is administered before, after or concurrently with a BTK inhibitor of formula (XVIII) or a pharmaceutically acceptable salt thereof. In some embodiments for treatment of hematological malignancies, a therapeutically effective amount of a PD-L1 inhibitor (durvalumab), is co-administered with a BTK inhibitor of formula (XVIII).

In an embodiment, the invention relates to a combination as described herein for use in treating a NHL selected from the group consisting of indolent NHL and aggressive. In some embodiments for treatment of a NHL, a therapeutically effective amount of a PD-1 inhibitor (pembrolizumab), or a PD-L1 inhibitor (durvalumab) is administered before, after or concurrently with a BTK inhibitor of formula (XVIII) or a pharmaceutically acceptable salt thereof. In some embodiments for treatment of a NHL, a therapeutically effective amount of a PD-L1 inhibitor (durvalumab) is co-administered with a BTK inhibitor of formula (XVIII) or a pharmaceutically acceptable salt thereof.

In an embodiment, the invention relates to a combination as described herein for use in treating a DLBCL selected from the group consisting of activated B-cell like diffuse large B-cell lymphoma (DLBCL-ABC) and germinal center B-cell like diffuse large B-cell lymphoma (DLBCL-GCB). In some embodiments for treatment of a DLBCL, a therapeutically effective amount of a PD-1 inhibitor (pembrolizumab), or a PD-L1 inhibitor (durvalumab) is administered before, after or concurrently with a BTK inhibitor of formula (XVIII) or a pharmaceutically acceptable salt thereof. In some embodiments for treatment of a DLBCL, a therapeutically effective amount of a PD-L1 inhibitor (durvalumab) is co-administered with a BTK inhibitor of formula (XVIII) or a pharmaceutically acceptable salt thereof.

In an embodiment, the invention relates to a combination as described herein for use in treating an MCL selected from the group consisting of mantle zone MCL, nodular MCL, diffuse MCL, and blastoid MCL (also known as blastic variant MCL). In some embodiments for treatment of an MCL, a therapeutically effective amount of a PD-1 inhibitor (pembrolizumab), or a PD-L1 inhibitor (durvalumab), is administered before, after or concurrently with a BTK inhibitor of formula (XVIII) or a pharmaceutically acceptable salt thereof. In some embodiments for treatment of an MCL, a therapeutically effective amount of a PD-L1 inhibitor (durvalumab) is co-administered with a BTK inhibitor of formula (XVIII) or a pharmaceutically acceptable salt thereof. In an embodiment, the invention relates to a combination as described herein for use in treating a B-ALL selected from the group consisting of early pre-B cell B-ALL, pre-B cell B-ALL, and mature B cell B-ALL (also known as Burkitt's leukemia),. In some embodiments for treatment of a B-ALL, a therapeutically effective amount of a PD-1 inhibitor (pembrolizumab), or a PD-L1 inhibitor (durvalumab), is administered before, after or concurrently with a BTK inhibitor of formula (XVIII) or a pharmaceutically acceptable salt thereof. In some embodiments for treatment of a B-ALL, a therapeutically effective amount of a PD-L1 inhibitor (durvalumab) is co-administered with a BTK inhibitor of formula (XVIII) or a pharmaceutically acceptable salt thereof.

In an embodiment, the invention relates to a combination as described herein for use in treating a Burkitt's lymphoma selected from the group consisting of sporadic Burkitt's lymphoma, endemic Burkitt's lymphoma, and human immunodeficiency virusassociated Burkitt's lymphoma. In some embodiments for treatment of a Burkitt's lymphoma, a therapeutically effective amount of a PD-1 inhibitor (pembrolizumab), a PD-L1 inhibitor (durvalumab), is administered before, after or concurrently with a BTK inhibitor of formula (XVIII) or a pharmaceutically acceptable salt thereof. In some embodiments for treatment of a Burkitt's lymphoma, a therapeutically effective amount of a PD-L1 inhibitor (durvalumab) is co-administered with a BTK inhibitor of formula (XVIII) or a pharmaceutically acceptable salt thereof.

In an embodiment, the invention relates to a combination as described herein for use in treating a multiple myeloma selected from the group consisting of hyperdiploid multiple myeloma and non-hyperdiploid multiple myeloma. In some embodiments for treatment of a multiple myeloma, a therapeutically effective amount of a PD-1 inhibitor (pembrolizumab), or a PD-L1 inhibitor (durvalumab) is administered before, after or concurrently with a BTK inhibitor of formula (XVIII) or a pharmaceutically acceptable salt thereof. In some embodiments for treatment of a multiple myeloma, a therapeutically effective amount of a PD-L1 inhibitor (durvalumab) is co-administered with a BTK inhibitor of formula (XVIII) or a pharmaceutically acceptable salt thereof.

In an embodiment, the invention relates to a combination as described herein for use in treating a myelofibrosis selected from the group consisting of primary myelofibrosis (also known as chronic idiopathic myelofibrosis) and myelofibrosis secondary to polycythemia vera or essential thrombocythaemia. In some embodiments, the invention provides a combination as described herein for use in treating disorders such as myeloproliferative disorders (MPDs), myeloproliferative neoplasms, polycythemia vera (PV), essential thrombocythemia (ET), primary myelofibrosis (PMF), myelodysplastic syndrome, chronic myelogenous leukemia (BCR-ABL1-positive), chronic neutrophilic leukemia, chronic eosinophilic leukemia, or mastocytosis. In some embodiments for treatment of a myelofibrosis, a therapeutically effective amount of a PD-1 inhibitor (pembrolizumab), or a PD-L1 inhibitor (durvalumab) is administered before, after or concurrently with a BTK inhibitor of formula (XVIII) or a pharmaceutically acceptable salt thereof. In some embodiments for treatment of a myelofibrosis, a therapeutically effective amount of a PD-L1 inhibitor (durvalumab) is co-administered with a BTK inhibitor of formula (XVIII) or a pharmaceutically acceptable salt thereof. In an embodiment, the invention relates to a combination as described herein for use in treating a subtype of a hematological malignancy in a human, wherein the subtype of a hematological malignancy is a subtype of a hematological malignancy that increases monocytes and NK cells in peripheral blood when measured after a period of treatment with the BTK inhibitor selected from the group consisting of about 14 days, about 28 days, about 56 days, about 1 month, about 2 months, about 3 months, about 6 months, and about 1 year, wherein the term "about" refers to a measurement interval of +/- 2 days, and wherein the hematological malignancy is selected from the group consisting of non-Hodgkin's lymphoma (NHL), diffuse large B cell lymphoma (DLBCL), follicular lymphoma (FL), mantle cell lymphoma (MCL), Hodgkin's lymphoma, B cell acute lymphoblastic leukemia (B-ALL), Waldenström's macroglobulinemia (WM), Burkitt's lymphoma, multiple myeloma, or myelofibrosis. In some embodiments for treatment of a subtype of a hematological malignancy, a therapeutically effective amount of a PD-1 inhibitor (pembrolizumab), or a PD-L1 inhibitor (durvalumab), is administered before, after or concurrently with a BTK inhibitor of formula (XVIII) or a pharmaceutically acceptable salt thereof. In some embodiments for treatment of a subtype of a hematological malignancy, a therapeutically effective amount of a PD-L1 inhibitor (durvalumab) is co-administered with a BTK inhibitor of formula (XVIII) or a pharmaceutically acceptable salt thereof.

### Combinations for use in Treating Patients Sensitive to Bleeding Events

In some embodiments, the invention provides a combination as described herein for use in treating a cancer in a human sensitive to bleeding events. In some embodiments for treatment of a cancer in a human sensitive to bleeding, a therapeutically effective amount of a PD-1 inhibitor (pembrolizumab), a PD-L1 inhibitor (durvalumab) is administered before, after or concurrently with a BTK inhibitor of formula (XVIII) or a pharmaceutically acceptable salt thereof. In some embodiments for treatment of a cancer in a human sensitive to bleeding, a therapeutically effective amount of a PD-L1 inhibitor (durvalumab) is co-administered with a BTK inhibitor of formula (XVIII) or a pharmaceutically acceptable salt thereof. In some embodiments, the invention provides a combination as described herein for use in of treating a cancer in a human intolerant to ibrutinib.

In some embodiments, the invention provides a combination as described herein for use in treating a cancer in a human sensitive to bleeding events wherein ther combination further comprises a therapeutically effective dose of an anticoagulent or antiplatelet active pharmaceutical ingredient.

In some embodiments, the invention provides a combination as described herein for use in treating a cancer in a human sensitive to bleeding events wherein the cancer is selected from the group consisting of bladder cancer, squamous cell carcinoma including head and neck cancer, pancreatic ductal adenocarcinoma (PDA), pancreatic cancer, colon carcinoma, mammary carcinoma, breast cancer, fibrosarcoma, mesothelioma, renal cell carcinoma, lung carcinoma, thyoma, prostate cancer, colorectal cancer, ovarian cancer, acute myeloid leukemia, thymus cancer, brain cancer, squamous cell cancer, skin cancer, eye cancer, retinoblastoma, melanoma, intraocular melanoma, oral cavity and oropharyngeal cancers, gastric cancer, stomach cancer, cervical cancer, head, neck, renal cancer, kidney cancer, liver cancer, ovarian cancer, prostate cancer, colorectal cancer, esophageal cancer, testicular cancer, gynecological cancer, thyroid cancer, acquired immune deficiency syndrome (AIDS)-related cancers (e.g., lymphoma and Kaposi's sarcoma), viral-induced cancer, glioblastoma, esophogeal tumors, hematological neoplasms, non-small-cell lung cancer, chronic myelocytic leukemia, diffuse large B-cell lymphoma, esophagus tumor, follicle center lymphoma, head and neck tumor, hepatitis C virus infection, hepatocellular carcinoma, Hodgkin's disease, metastatic colon cancer, multiple myeloma, non-Hodgkin's lymphoma, indolent non-Hodgkin's lymphoma, ovary tumor, pancreas tumor, renal cell carcinoma, small-cell lung cancer, stage IV melanoma, chronic lymphocytic leukemia, B-cell acute lymphoblastic leukemia (ALL), mature B-cell ALL, follicular lymphoma, mantle cell lymphoma, and Burkitt's lymphoma.

In some embodiments, the invention provides a combination as described herein for use in treating a cancer in a human sensitive to or intolerant to ibrutinib.

In some embodiments, the BTK inhibitor of formula (XVIII) or a pharmaceutically acceptable salt thereof and the anticoagulent or the antiplatelet active pharmaceutical ingredient are administered sequentially. In some embodiments, the BTK inhibitor of formula (XVIII) or a pharmaceutically acceptable salt thereof and the anticoagulent or the antiplatelet active pharmaceutical ingredient are administered concomittently. In some embodiments, the BTK inhibitor of formula (XVIII) or a pharmaceutically acceptable salt thereof is administered before the anticoagulent or the antiplatelet active pharmaceutical ingredient. In some embodiments, the BTK inhibitor of formula (XVIII) or a pharmaceutically acceptable salt thereof is administered after the anticoagulent or the antiplatelet active pharmaceutical ingredient. In some embodiments, a PD-1 (pembrolizumab) or PD-L1 (durvalumab) inhibitor is co-administered with the BTK inhibitor of formula (XVIII) or a pharmaceutically acceptable salt thereof and the anticoagulent or the antiplatelet active pharmaceutical ingredient at the same time or at different times.

Selected anti-platelet and anticoagulent active pharmaceutical ingredients for use in the combinations for use in the present invention include, but are not limited to, cyclooxygenase inhibitors (e.g., aspirin), adenosine diphosphate (ADP) receptor inhibitors (e.g., clopidogrel and ticlopidine), phosphodiesterase inhibitors (e.g., cilostazol), glycoprotein IIb/IIIa inhibitors (e.g., abciximab, eptifibatide, and tirofiban), adenosine reuptake inhibitors (e.g., dipyridamole), and acetylsalicylic acid (aspirin). In other embodiments, examples of anti-platelet active pharmaceutical ingredients for use in the present invention include anagrelide, aspirin/extended-release dipyridamole, cilostazol, clopidogrel, dipyridamole, prasugrel, ticagrelor, ticlopidine, vorapaxar, tirofiban HCl, eptifibatide, abciximab, argatroban, bivalirudin, dalteparin, desirudin, enoxaparin, fondaparinux, heparin, lepirudin, apixaban, dabigatran etexilate mesylate, rivaroxaban, and warfarin.

In an embodiment, the invention includes a combination as described herein for use in treating a cancer, comprising the step of orally administering, to a human in need thereof, a Bruton's tyrosine kinase (BTK) inhibitor, wherein the BTK inhibitor is (*S*)-4-(8-amino-3-(1-(but-2-ynoyl)pyrrolidin-2-yl)imidazo[1,5-*a*]pyrazin-1-yl)-*N*-(pyridin-2-yl)benzamideor a pharmaceutically acceptable saltthereof, and a PD-1 inhibitor or a PD-L1 inhibitor, wherein the PD-1 inhibitor is pembrolizumab and the PD-L1 inhibitor is durvalumab, wherein the combination further comprises a therapeutically effective dose of an anticoagulant or antiplatelet active pharmaceutical ingredient, wherein the anticoagulant or antiplatelet active pharmaceutical ingredient is selected from the group consisting of acenocoumarol, anagrelide, anagrelide hydrochloride, abciximab, aloxiprin, antithrombin, apixaban, argatroban, aspirin, aspirin with extended-release dipyridamole, beraprost, betrixaban, bivalirudin, carbasalate calcium, cilostazol, clopidogrel, clopidogrel bisulfate, cloricromen, dabigatran etexilate, darexaban, dalteparin, dalteparin sodium, defibrotide, dicumarol, diphenadione, dipyridamole, ditazole, desirudin, edoxaban, enoxaparin, enoxaparin sodium, eptifibatide, fondaparinux, fondaparinux sodium, heparin, heparin sodium, heparin calcium, idraparinux, idraparinux sodium, iloprost, indobufen, lepirudin, low molecular weight heparin, melagatran, nadroparin, otamixaban, parnaparin, phenindione, phenprocoumon, prasugrel, picotamide, prostacyclin, ramatroban, reviparin, rivaroxaban, sulodexide, terutroban, terutroban sodium, ticagrelor, ticlopidine, ticlopidine hydrochloride, tinzaparin, tinzaparin sodium, tirofiban, tirofiban hydrochloride, treprostinil, treprostinil sodium, triflusal, vorapaxar, warfarin, warfarin sodium, ximelagatran, salts thereof, solvates thereof, hydrates thereof, and combinations thereof.

### Combinations of BTK Inhibitors, PI3K Inhibitors, JAK-2 Inhibitors, PD-1 Inhibitors, and/or PD-L1 and PD-L2 Inhibitors with Anti-CD20 Antibodies

The combinations of the BTK inhibitor of formula (XVIII) or a pharmaceutically acceptable salt thereof and PD-1 inhibitors (pembrolizumab), or PD-L1 inhibitors (durvalumab) and optionally PI3K inhibitors, or JAK-2 inhibitors, may also be safely co-administered with immunotherapeutic antibodies such as the anti-CD20 antibodies rituximab, obinutuzumab, ofatumumab, veltuzumab, tositumomab, and ibritumomab, and or antigen-binding fragments, derivatives, conjugates, variants, and radioisotope-labeled complexes thereof, which may be given alone or with conventional chemotherapeutic active pharmaceutical ingredients such as those described herein. The CD20 antigen also called human B-lymphocyte-restricted differentiation antigen, Bp35, or B1) is found on the surface of normal "pre-B" and mature B lymphocytes, including malignant B lymphocytes. Nadler, et al., J. Clin. Invest. 1981, 67, 134-40; Stashenko, et al., J. Immunol. 1980, 139, 3260-85. The CD20 antigen is a glycosylated integral membrane protein with a molecular weight of approximately 35 kD. Tedder, et al., Proc. Natl. Acad. Sci. USA, 1988, 85, 208-12. CD20 is also expressed on most B cell non-Hodgkin's lymphoma cells, but is not found on hematopoietic stem cells, pro-B cells, normal plasma cells, or other normal tissues. Anti-CD20 antibodies are currently used as therapies for many hematological malignancies, including indolent NHL, aggressive NHL, and CLL/SLL. Lim, et. al., Haematologica 2010, 95, 135-43; Beers, et. al., Sem. Hematol. 2010, 47, 107-14; and Klein, et al., mAbs 2013, 5, 22-33. In an embodiment, the foregoing combinations exhibit synergistic effects that result in greater efficacy, less side effects, the use of less active pharmaceutical ingredient to achieve a given clinical result, and/or other synergistic effects.

In an embodiment, the invention relates to a combination as disclosed herein for use in treating a hematological malignancy, or solid tumor cancer, in a human wherein the combination further comprises an anti-CD20 antibody, wherein the anti-CD20 antibody is a monoclonal antibody or an antigen-binding fragment, derivative, conjugate, variant, or radioisotope-labeled complex thereof. In an embodiment, the invention relates to a combination as described herein for use in treating a hematological malignancy, or solid tumor cancer in a human wherein the combination further comprises an anti-CD20 antibody, wherein the anti-CD20 antibody is an anti-CD20 monoclonal antibody or an antigen-binding fragment, derivative, conjugate, variant, or radioisotope-labeled complex thereof, and wherein the anti-CD20 antibody specifically binds to human CD20 with a K_{D} selected from the group consisting of 1 ×10⁻⁷ M or less, 5 ×10⁻⁸ M or less, 1×10⁻⁸ M or less, and 5×10⁻⁹ M or less. Anti-CD20 monoclonal antibodies are classified as Type I or Type II, as described in Klein, et al., mAbs 2013, 5, 22-33. Type I anti-CD20 monoclonal antibodies are characterized by binding to the Class I epitope, localization of CD20 to lipid rafts, high complement-dependent cytotoxicity, full binding capacity, weak homotypic aggregation, and moderate cell death induction. Type II anti-CD20 monoclonal antibodies are characterized by binding to the Class I epitope, a lack of localization of CD20 to lipid rafts, low complement-dependent cytotoxicity, half binding capacity, homotypic aggregation, and strong cell death induction. Both Type I and Type II anti-CD20 monoclonal antibodies exhibit antibody-dependent cytotoxitcty (ADCC) and are thus useful with BTK inhibitors described herein. Type I anti-CD20 monoclonal antibodies include but are not limited to rituximab, ocrelizumab, and ofatumumab. Type II anti-CD20 monoclonal antibodies include but are not limited to obinutuzumab and tositumomab. In some embodiments for treatment of a hematological malignancy, or solid tumor cancer a therapeutically effective amount of a PD-1 inhibitor (pembrolizumab), or a PD-L1 inhibitor (durvalumab), is administered before, after or concurrently with a BTK inhibitor of formula (XVIII) or a pharmaceutically acceptable salt thereof. In some embodiments for treatment of a hematological malignancy, or solid tumor cancer a therapeutically effective amount of a PD-L1 inhibitor (durvalumab) is co-administered with a BTK inhibitor of formula (XVIII) or a pharmaceutically acceptable salt thereof. In an embodiment, the invention relates to a combination as described herein for use in treating a hematological malignancy, or solid tumor cancerwherein the combination further comprises an anti-CD20 antibody, wherein the anti-CD20 antibody is a monoclonal antibody or an antigen-binding fragment, derivative, conjugate, variant, or radioisotope-labeled complex thereof. In an embodiment, the invention relates to a combination as described herein for use intreating a hematological malignancy, or solid tumor cancer in a human wherein the combination further comprises an anti-CD20 antibody, wherein the anti-CD20 antibody is an anti-CD20 monoclonal antibody or an antigen-binding fragment, derivative, conjugate, variant, or radioisotope-labeled complex thereof, and wherein the anti-CD20 antibody specifically binds to human CD20 with a K_{D} selected from the group consisting of 1 ×10⁻⁷ M or less, 5 ×10⁻⁸ M or less, 1×10⁻⁸ M or less, and 5×10⁻⁹ M or less.

In an embodiment, the invention relates to a combination for use in treating a hematological malignancy or solid tumor cancer a human wherein the combination comprises a BTK inhibitor of Formula (XVIII), or a pharmaceutically acceptable salt thereof, and a PD-1 (pembrolizumab) or PD-L1 inhibitor (durvalumab), and further comprising a Type I anti-CD20 antibody, or an antigen-binding fragment, derivative, conjugate, variant, or radioisotope-labeled complex thereof. In an embodiment, the invention relates to a combination for use in treating a hematological malignancy or solid tumor cancer in a human wherein the combination comprises a BTK inhibitor of Formula (XVIII), or a pharmaceutically acceptable salt thereof, and a PD-1 (pembrolizumab) or PD-L1 inhibitor (durvalumab), , and further comprising a Type II anti-CD20 antibody, or an antigen-binding fragment, derivative, conjugate, variant, or radioisotope-labeled complex thereof.

In some embodiments, the combinations of the BTK inhibitors of formula (XVIII) or a pharmaceutically acceptable salt thereof with a PD-1 inhibitor (pembrolizumab) or a PD-L1 inhibnitor (durvalumab) and optionally with PI3K inhibitors, JAK-2 inhibitors, and the anti-CD20 monoclonal antibody are administered sequentially. In some embodiments, the combinations as described above are administered concomitantly. In some embodiments, the combinations of the BTK inhibitor of formula (XVIII) or a pharmaceutically acceptable salt thereof with the above PD-1 and PD-L1 inhibitors, and optionally the PI3K inhibitors, and JAK-2 inhibitors, is administered before the anti-CD20 monoclonal antibody. In some embodiments, the combinations of the BTK inhibitors of formula (XVIII) or a pharmaceutically acceptable salt thereof with the above PD-1 and PD-L1 inhibitors, and optionally the PI3K inhibitors or JAK-2 inhibitors is administered after the anticoagulant or the antiplatelet active pharmaceutical ingredient. In some embodiments, the combinations of the BTK inhibitors of formula (XVIII) or a pharmaceutically acceptable salt thereof with the above PD-1 and PD-L1 inhibitors, and optionally the PI3K inhibitors, JAK-2 inhibitors, and the anti-CD20 monoclonal antibody are administered over the same time period, and the BTK inhibitor administration continues after the anti-CD20 monoclonal antibody administration is completed.

In an embodiment, the anti-CD20 monoclonal antibody is rituximab, or an antigen-binding fragment, derivative, conjugate, variant, or radioisotope-labeled complex thereof. Rituximab is a chimeric murine-human monoclonal antibody directed against CD20, and its structure comprises an IgG1 kappa immunoglobulin containing murine light- and heavy-chain variable region sequences and human constant region sequences. Rituximab is composed of two heavy chains of 451 amino acids and two light chains of 213 amino acids. The amino acid sequence for the heavy chains of rituximab is set forth in SEQ ID NO: 84. The amino acid sequence for the light chains of rituximab is set forth in SEQ ID NO:85. Rituximab is commercially available, and its properties and use in cancer and other diseases is described in more detail in Rastetter, et al., Ann. Rev. Med. 2004, 55, 477-503, and in Plosker and Figgett, Drugs, 2003, 63, 803-43. In an embodiment, the anti-CD20 monoclonal antibody is an anti-CD20 biosimilar monoclonal antibody approved by drug regulatory authorities with reference to rituximab. In an embodiment, the anti-CD20 monoclonal antibody has a heavy chain sequence identity of greater than 90% to SEQ ID NO:84. In an embodiment, the anti-CD20 monoclonal antibody has a light chain sequence identity of greater than 90% to SEQ ID NO:85. In an embodiment, the anti-CD20 monoclonal antibody has a heavy chain sequence identity of greater than 95% to SEQ ID NO:84. In an embodiment, the anti-CD20 monoclonal antibody has a light chain sequence identity of greater than 95% to SEQ ID NO:85. In an embodiment, the anti-CD20 monoclonal antibody has a heavy chain sequence identity of greater than 98% to SEQ ID NO:84. In an embodiment, the anti-CD20 monoclonal antibody has a light chain sequence identity of greater than 98% to SEQ ID NO:85. In an embodiment, the anti-CD20 monoclonal antibody has a heavy chain sequence identity of greater than 99% to SEQ ID NO:84. In an embodiment, the anti-CD20 monoclonal antibody has a light chain sequence identity of greater than 99% to SEQ ID NO:85.

In an embodiment, the anti-CD20 monoclonal antibody is obinutuzumab, or an antigen-binding fragment, derivative, conjugate, variant, or radioisotope-labeled complex thereof. Obinutuzumab is also known as afutuzumab or GA-101. Obinutuzumab is a humanized monoclonal antibody directed against CD20. The amino acid sequence for the heavy chains of obinutuzumab is set forth in SEQ ID NO:86. The amino acid sequence for the light chains of obinutuzumab is set forth in SEQ ID NO:87. Obinutuzumab is commercially available, and its properties and use in cancer and other diseases is described in more detail in Robak, Curr. Opin. Investig. Drugs 2009, 10, 588-96. In an embodiment, the anti-CD20 monoclonal antibody is an anti-CD20 biosimilar monoclonal antibody approved by drug regulatory authorities with reference to obinutuzumab. In an embodiment, the anti-CD20 monoclonal antibody has a heavy chain sequence identity of greater than 90% to SEQ ID NO:86. In an embodiment, the anti-CD20 monoclonal antibody has a light chain sequence identity of greater than 90% to SEQ ID NO: 87. In an embodiment, the anti-CD20 monoclonal antibody has a heavy chain sequence identity of greater than 95% to SEQ ID NO:86. In an embodiment, the anti-CD20 monoclonal antibody has a light chain sequence identity of greater than 95% to SEQ ID NO:87. In an embodiment, the anti-CD20 monoclonal antibody has a heavy chain sequence identity of greater than 98% to SEQ ID NO:86. In an embodiment, the anti-CD20 monoclonal antibody has a light chain sequence identity of greater than 98% to SEQ ID NO:87. In an embodiment, the anti-CD20 monoclonal antibody has a heavy chain sequence identity of greater than 99% to SEQ ID NO:86. In an embodiment, the anti-CD20 monoclonal antibody has a light chain sequence identity of greater than 99% to SEQ ID NO:87. In an embodiment, the anti-CD20 monoclonal antibody obinutuzumab is an immunoglobulin G1, anti-(human B-lymphocyte antigen CD20 (membrane-spanning 4-domains subfamily A member 1, B-lymphocyte surface antigen B1, Leu-16 or Bp35)), humanized mouse monoclonal obinutuzumab des-CH3107-K-γ1 heavy chain (222-219')-disulfide with humanized mouse monoclonal obinutuzumab κ light chain dimer (228-228":231- 231")-bisdisulfide antibody.

In an embodiment, the anti-CD20 monoclonal antibody is ofatumumab, or an antigen-binding fragment, derivative, conjugate, variant, or radioisotope-labeled complex thereof. Ofatumumab is described in Cheson, J. Clin. Oncol. 2010, 28, 3525-30. The crystal structure of the Fab fragment of ofatumumab has been reported in Protein Data Bank reference 3GIZ and in Du, et al., Mol. Immunol. 2009, 46, 2419-2423. Ofatumumab is commercially available, and its preparation, properties, and use in cancer and other diseases are described in more detail in U.S. Patent No. 8,529,202 B2. In an embodiment, the anti-CD20 monoclonal antibody is an anti-CD20 biosimilar monoclonal antibody approved by drug regulatory authorities with reference to ofatumumab. In an embodiment, the anti-CD20 monoclonal antibody has a variable heavy chain sequence identity of greater than 90% to SEQ ID NO:88. In an embodiment, the anti-CD20 monoclonal antibody has a variable light chain sequence identity of greater than 90% to SEQ ID NO: 89. In an embodiment, the anti-CD20 monoclonal antibody has a variable heavy chain sequence identity of greater than 95% to SEQ ID NO:88. In an embodiment, the anti-CD20 monoclonal antibody has a variable light chain sequence identity of greater than 95% to SEQ ID NO:89. In an embodiment, the anti-CD20 monoclonal antibody has a variable heavy chain sequence identity of greater than 98% to SEQ ID NO:88. In an embodiment, the anti-CD20 monoclonal antibody has a variable light chain sequence identity of greater than 98% to SEQ ID NO:89. In an embodiment, the anti-CD20 monoclonal antibody has a variable heavy chain sequence identity of greater than 99% to SEQ ID NO: 88. In an embodiment, the anti-CD20 monoclonal antibody has a variable light chain sequence identity of greater than 99% to SEQ ID NO:89. In an embodiment, the anti-CD20 monoclonal antibody has a Fab fragment heavy chain sequence identity of greater than 90% to SEQ ID NO:90. In an embodiment, the anti-CD20 monoclonal antibody has a Fab fragment light chain sequence identity of greater than 90% to SEQ ID NO:91. In an embodiment, the anti-CD20 monoclonal antibody has a Fab fragment heavy chain sequence identity of greater than 95% to SEQ ID NO:90. In an embodiment, the anti-CD20 monoclonal antibody has a Fab fragment light chain sequence identity of greater than 95% to SEQ ID NO:91. In an embodiment, the anti-CD20 monoclonal antibody has a Fab fragment heavy chain sequence identity of greater than 98% to SEQ ID NO:90. In an embodiment, the anti-CD20 monoclonal antibody has a Fab fragment light chain sequence identity of greater than 98% to SEQ ID NO:91. In an embodiment, the anti-CD20 monoclonal antibody has a Fab fragment heavy chain sequence identity of greater than 99% to SEQ ID NO:90. In an embodiment, the anti-CD20 monoclonal antibody has a Fab fragment light chain sequence identity of greater than 99% to SEQ ID NO:91. In an embodiment, the anti-CD20 monoclonal antibody ofatumumab is an immunoglobulin G1, anti-(human B-lymphocyte antigen CD20 (membrane-spanning 4-domains subfamily A member 1, B-lymphocyte surface antigen B1, Leu-16 or Bp35)); human monoclonal ofatumumab-CD20 γ1 heavy chain (225-214')-disulfide with human monoclonal ofatumumab-CD20 κ light chain, dimer (231-231":234-234")-bisdisulfide antibody.

In an embodiment, the anti-CD20 monoclonal antibody is veltuzumab, or an antigen-binding fragment, derivative, conjugate, variant, or radioisotope-labeled complex thereof. Veltuzumab is also known as hA20. Veltuzumab is described in Goldenberg, et al., Leuk. Lymphoma 2010, 51, 747-55. In an embodiment, the anti-CD20 monoclonal antibody is an anti-CD20 biosimilar monoclonal antibody approved by drug regulatory authorities with reference to veltuzumab. In an embodiment, the anti-CD20 monoclonal antibody has a heavy chain sequence identity of greater than 90% to SEQ ID NO:92. In an embodiment, the anti-CD20 monoclonal antibody has a light chain sequence identity of greater than 90% to SEQ ID NO:93. In an embodiment, the anti-CD20 monoclonal antibody has a heavy chain sequence identity of greater than 95% to SEQ ID NO:92. In an embodiment, the anti-CD20 monoclonal antibody has a light chain sequence identity of greater than 95% to SEQ ID NO:93. In an embodiment, the anti-CD20 monoclonal antibody has a heavy chain sequence identity of greater than 98% to SEQ ID NO:92. In an embodiment, the anti-CD20 monoclonal antibody has a light chain sequence identity of greater than 98% to SEQ ID NO:93. In an embodiment, the anti-CD20 monoclonal antibody has a heavy chain sequence identity of greater than 99% to SEQ ID NO:92. In an embodiment, the anti-CD20 monoclonal antibody has a light chain sequence identity of greater than 99% to SEQ ID NO:93. In an embodiment, the anti-CD20 monoclonal antibody ofatumumab is an immunoglobulin G1, anti-(human B-lymphocyte antigen CD20 (membrane-spanning 4-domains subfamily A member 1, Leu-16, Bp35)); [218-arginine,360-glutamic acid,362-methionine]humanized mouse monoclonal hA20 γ1 heavy chain (224-213')-disulfide with humanized mouse monoclonal hA20 κ light chain (230-230":233-233")-bisdisulfide dimer

In an embodiment, the anti-CD20 monoclonal antibody is tositumomab, or an antigen-binding fragment, derivative, conjugate, variant, or radioisotope-labeled complex thereof. In an embodiment, the anti-CD20 monoclonal antibody is ¹³¹I-labeled tositumomab. In an embodiment, the anti-CD20 monoclonal antibody is an anti-CD20 biosimilar monoclonal antibody approved by drug regulatory authorities with reference to tositumomab. In an embodiment, the anti-CD20 monoclonal antibody has a heavy chain sequence identity of greater than 90% to SEQ ID NO:94. In an embodiment, the anti-CD20 monoclonal antibody has a light chain sequence identity of greater than 90% to SEQ ID NO:95. In an embodiment, the anti-CD20 monoclonal antibody has a heavy chain sequence identity of greater than 95% to SEQ ID NO:94. In an embodiment, the anti-CD20 monoclonal antibody has a light chain sequence identity of greater than 95% to SEQ ID NO:95. In an embodiment, the anti-CD20 monoclonal antibody has a heavy chain sequence identity of greater than 98% to SEQ ID NO:94. In an embodiment, the anti-CD20 monoclonal antibody has a light chain sequence identity of greater than 98% to SEQ ID NO:95. In an embodiment, the anti-CD20 monoclonal antibody has a heavy chain sequence identity of greater than 99% to SEQ ID NO:94. In an embodiment, the anti-CD20 monoclonal antibody has a light chain sequence identity of greater than 99% to SEQ ID NO:95.

In an embodiment, the anti-CD20 monoclonal antibody is ibritumomab, or an antigen-binding fragment, derivative, conjugate, variant, or radioisotope-labeled complex thereof. The active form of ibritumomab used in therapy is ibritumomab tiuxetan. When used with ibritumomab, the chelator tiuxetan (diethylene triamine pentaacetic acid) is complexed with a radioactive isotope such as ⁹⁰Y or ¹¹¹In. In an embodiment, the anti-CD20 monoclonal antibody is ibritumomab tiuxetan, or radioisotope-labeled complex thereof. In an embodiment, the anti-CD20 monoclonal antibody is an anti-CD20 biosimilar monoclonal antibody approved by drug regulatory authorities with reference to tositumomab. In an embodiment, the anti-CD20 monoclonal antibody has a heavy chain sequence identity of greater than 90% to SEQ ID NO:96. In an embodiment, the anti-CD20 monoclonal antibody has a light chain sequence identity of greater than 90% to SEQ ID NO:97. In an embodiment, the anti-CD20 monoclonal antibody has a heavy chain sequence identity of greater than 95% to SEQ ID NO:96. In an embodiment, the anti-CD20 monoclonal antibody has a light chain sequence identity of greater than 95% to SEQ ID NO:97. In an embodiment, the anti-CD20 monoclonal antibody has a heavy chain sequence identity of greater than 98% to SEQ ID NO:96. In an embodiment, the anti-CD20 monoclonal antibody has a light chain sequence identity of greater than 98% to SEQ ID NO:97. In an embodiment, the anti-CD20 monoclonal antibody has a heavy chain sequence identity of greater than 99% to SEQ ID NO:96. In an embodiment, the anti-CD20 monoclonal antibody has a light chain sequence identity of greater than 99% to SEQ ID NO:97.

In an embodiment, an anti-CD20 antibody selected from the group consisting of obinutuzumab, ofatumumab, veltuzumab, tositumomab, and ibritumomab, and or antigen-binding fragments, derivatives, conjugates, variants, and radioisotope-labeled complexes thereof, is administered to a subject by infusion in a dose selected from the group consisting of about 10 mg, about 20 mg, about 25 mg, about 50 mg, about 75 mg, 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, about 1800 mg, about 1900 mg, and about 2000 mg. In an embodiment, the anti-CD20 antibody is administered weekly. In an embodiment, the anti-CD20 antibody is admininstered every two weeks. In an embodiment, the anti-CD20 antibody is admininstered every three weeks. In an embodiment, the anti-CD20 antibody is admininstered monthly. In an embodiment, the anti-CD20 antibody is administered at a lower initial dose, which is escalated when administered at subsequent intervals admininstered monthly. For example, the first infusion can deliver 300 mg of anti-CD20 antibody, and subsequent weekly doses could deliver 2,000 mg of anti-CD20 antibody for eight weeks, followed by monthly doses of 2,000 mg of anti-CD20 antibody. During any of the foregoing embodiments, the combinations of the BTK inhibitors of formula (XVIII) or a pharmaceutically acceptable salt thereof with the above PD-1 or PD-L1 inhibitors, and optionally the PI3K inhibitors, and JAK-2 inhibitors, may be administered daily, twice daily, or at different intervals as described above, at the dosages described above.

In an embodiment, the invention provides a kit comprising a composition comprising a combination of the BTK inhibitors of formula (XVIII) or a pharmaceutically acceptable salt thereof with the above PD-1 or PD-L1 inhibitors, and optionally the with PI3K inhibitors, and JAK-2 inhibitors, wherein the kit further comprises a composition comprising an anti-CD20 antibody selected from the group consisting of rituximab, obinutuzumab, ofatumumab, veltuzumab, tositumomab, and ibritumomab, or an antigen-binding fragment, derivative, conjugate, variant, or radioisotope-labeled complex thereof, for use in the treatment of CLL or SLL, hematological malignancies, B cell malignancies, or, or any of the other diseases described herein. The compositions are typically both pharmaceutical compositions. The kit is for use in co-administration of the anti-CD20 antibody and the combination described herein, either simultaneously or separately, in the treatment of CLL or SLL, hematological malignancies, B cell malignancies, or any of the other diseases described herein.

The anti-CD20 antibody amino acid sequences referenced in the foregoing are summarized in Table 1.

**TABLE 1. Anti-CD20 antibody amino acid sequences.**

| **Identifier** | **Sequence (One-Letter Amino Acid Symbols)** |
|---|---|
| SEQ ID NO:84 rituximab heavy chain | |
| SEQ ID NO:85 rituximab light chain | |
| SEQ ID NO:86 obinutuzumab heavy chain | |
| SEQ ID NO:87 obinutuzumab light chain | |
| SEQ ID NO:88 ofatumumab variable heavy chain | |
| SEQ ID NO:89 ofatumumab variable light chain | |
| SEQ ID NO:90 ofatumumab Fab fragment heavy chain | |
| SEQ ID NO:91 ofatumumab Fab fragment light chain | |
| SEQ ID NO:92 veltuzumab heavy chain | |
| | |
| SEQ ID NO:93 veltuzumab light chain | |
| SEQ ID NO:94 tositumomab heavy chain | |
| SEQ ID NO:95 tositumomab light chain | |
| SEQ ID NO:96 ibritumomab heavy chain | |
| SEQ ID NO:97 ibritumomab light chain | |

### Combinations of BTK Inhibitors, PI3K Inhibitors, JAK-2 Inhibitors, PD-1 Inhibitors, and/or PD-L1 and PD-L2 Inhibitors with Chemotherapeutic Active Pharmaceutical Ingredients

The combinations of the BTK inhibitors of formula (XVIII) or a pharmaceutically acceptable salt thereof with a PD-1 (pembrolizumab) or PD-L1 (durvalumab) inhibitors, and optionally the with PI3K inhibitors, and JAK-2 inhibitors may also be safely co-administered with chemotherapeutic active pharmaceutical ingredients such as gemcitabine and albumin-bound paclitaxel (nab-paclitaxel). In an embodiment, the invention relates to a combination as described herein for use in treating a hematological malignancy, or solid tumor cancer in a human wherein the combination further comprises a therapeutically-effective amount of gemcitabine, or a pharmaceutically acceptable salt, solvate or hydrate thereof. In an embodiment, the solid tumor cancer in any of the foregoing embodiments is pancreatic cancer.

In an embodiment, the invention relates to a combination as described above for use in treating a hematological malignancy, or solid tumor cancerwherein the combination further comprises a therapeutically-effective amount of nab-paclitaxel. In an embodiment, the solid tumor cancer in any of the foregoing embodiments is pancreatic cancer.

In an embodiment, the invention provides a synergistic combination of a BTK inhibitor of Formula (XVIII) or a pharmaceutically acceptable salt thereof, a PD-1 inhibitor (pembrolizumab) and/or a PD-L1 inhibitor (durvalumab), and gemcitabine for use in the treatment of a cancer.

In an embodiment, the invention provides a synergistic combination of a BTK inhibitor of Formula (XVIII) or a pharmaceutically acceptable salt thereof, a PD-1 inhibitor (pembrolizumab) and/or a PD-L1 inhibitor (durvalumab), and gemcitabine for use in the treatment of a cancer, wherein the cancer is selected from the group consisting of ovarian cancer, breast cancer, non-small cell lung cancer, and pancreatic cancer. In an embodiment, the invention provides a synergistic combination of a BTK inhibitor of Formula (XVIII) or a pharmaceutically acceptable salt thereof, a PD-1 inhibitor (pembrolizumab) and/or a PD-L1 inhibitor (durvalumab), nab-paclitaxel, and gemcitabine for use in the treatment of a cancer, wherein the cancer is selected from the group consisting of ovarian cancer, breast cancer, non-small cell lung cancer, and pancreatic cancer.

In an embodiment, the invention relates to a combination as described herein for use in treating a hematological malignancy, or solid tumor cancer, in a human wherein the combination further comprises a therapeutically-effective amount of a chemotherapeutic active pharmaceutical ingredient selected from the group consisting of thalidomide, pomalidomide, lenalidomide, bortezomib, and combinations thereof.

In an embodiment, the invention provides a combination for use in treating a hematological malignancy or a solid tumor cancer in a human wherein the combination comprises a BTK inhibitor of Formula (XVIII), or a pharmaceutically acceptable saltthereof, and a PD-1 (pembrolizumab) or a PD-L1 (durvalumab) inhibitor, and further comprises a therapeutically-effective amount of bendamustine hydrochloride.

In an embodiment, the invention provides a combination as described herein for use in treating a hematological malignancy or a solid tumor cancer in a human wherein the combination further comprises a therapeutically-effective amount of a combination of fludarabine, cyclophosphamide, and rituximab (which collectively may be referred to as "FCR" or "FCR chemotherapy"). FCR chemotherapy has been shown to improve survival in patients with cancer, as described in Hallek, et al., Lancet. 2010, 376, 1164-1174.

In an embodiment, the invention provides a combination as described herein for use in treating a hematological malignancy or a solid tumor cancer in a human , wherein the combination further comprises a therapeutically-effective amount of a combination of rituximab, cyclophosphamide, doxorubicin hydrochloride (also referred to as hydroxydaunomycin), vincristine sulfate (also referred to as oncovin), and prednisone (which collectively may be referred to as "R-CHOP" or "R-CHOP chemotherapy"). R-CHOP chemotherapy has been shown to improve the 10-year progression-free and overall survival rates for patients with cancer, as described in Sehn, Blood, 2010, 116, 2000-2001.

In any of the foregoing embodiments, the chemotherapeutic active pharmaceutical ingredient or combinations thereof may be administered before, concurrently, or after administration of the PD-1 (pembrolizumab) and/or PD-L1 (durvlumab) inhibitors and the BTK inhibitors of formula (XVIII) or a pharmaceutically acceptable salt thereof.

While preferred embodiments of the invention are shown and described herein, such embodiments are provided by way of example only and are not intended to otherwise limit the scope of the invention as defined by the claims. Various alternatives to the described embodiments of the invention may be employed in practicing the invention.

### EXAMPLES

The embodiments encompassed herein are now described with reference to the following examples. These examples are provided for the purpose of illustration only and the disclosure encompassed herein should in no way be construed as being limited to these examples, but rather should be construed to encompass any and all variations which become evident as a result of the teachings provided herein.

### Reference Example 4 - Synergistic Combination of a BTK Inhibitor and the JAK-2 Inhibitor Pacritinib

Combination experiments were performed to determine the synergistic, additive, or antagonistic behavior of drug combinations using the methods described above in Example 2. The study was performed using the BTK inhibitor of Formula (XVIII) and the JAK-2 inhibitor of Formula LIV (pacritinib).

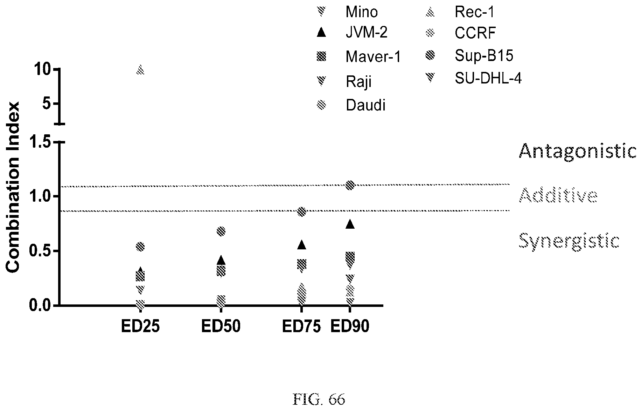

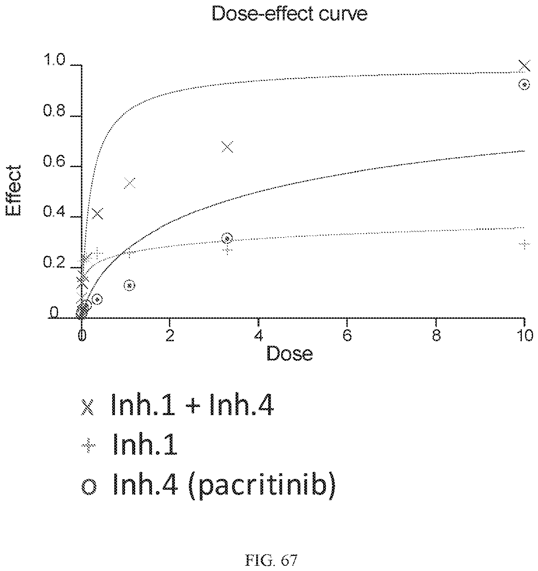

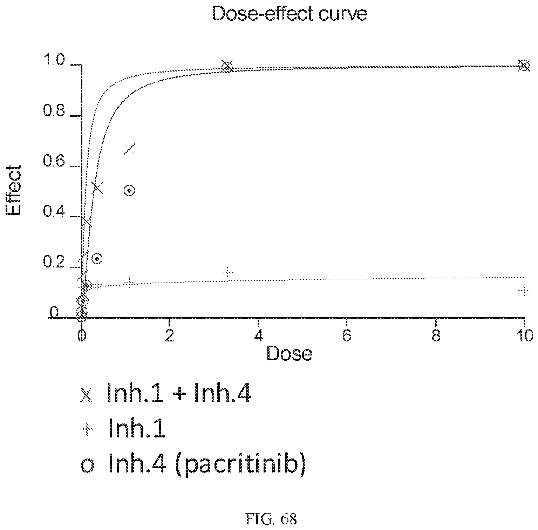

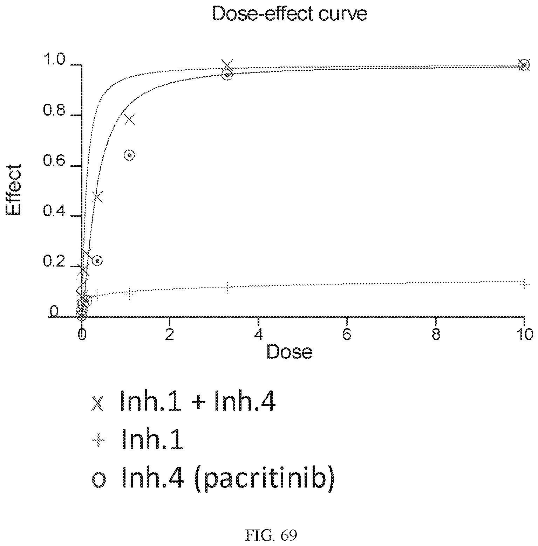

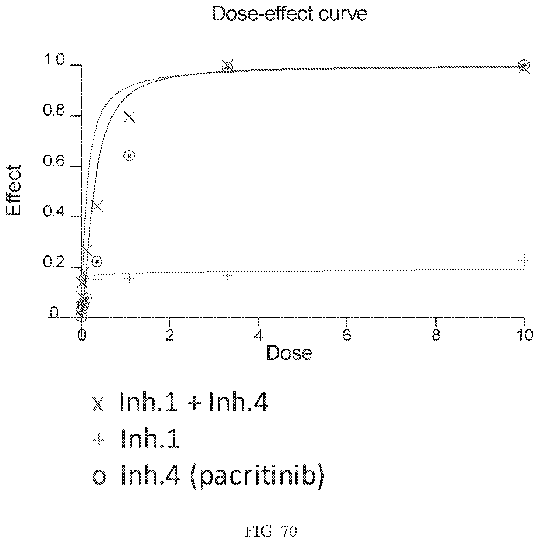

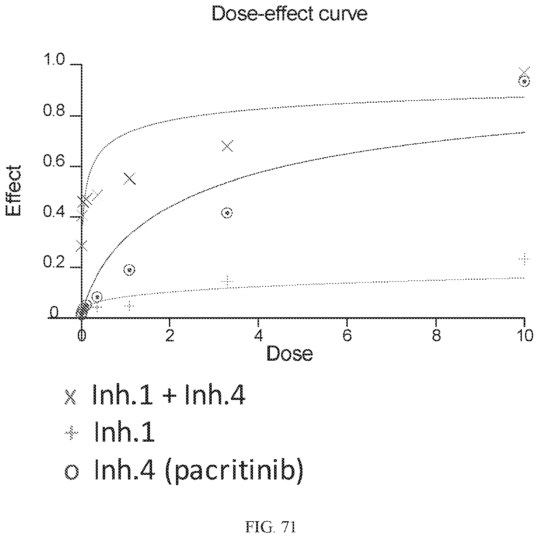

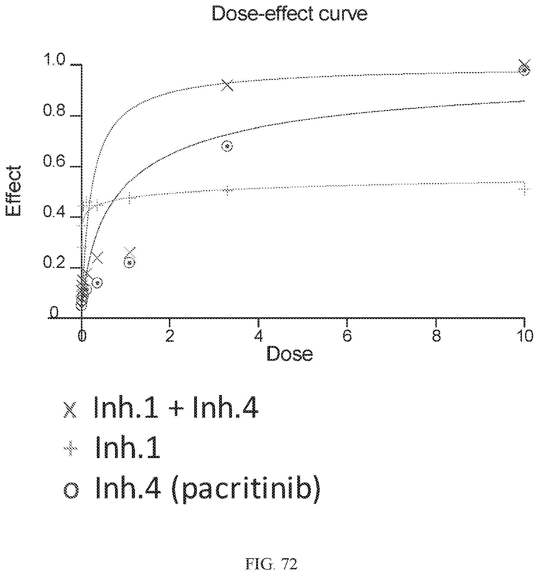

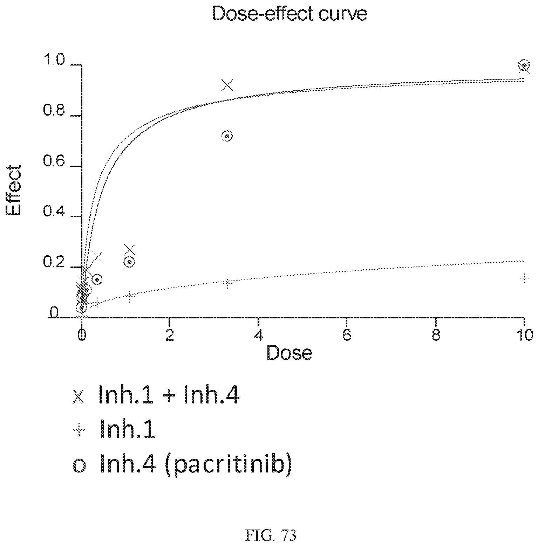

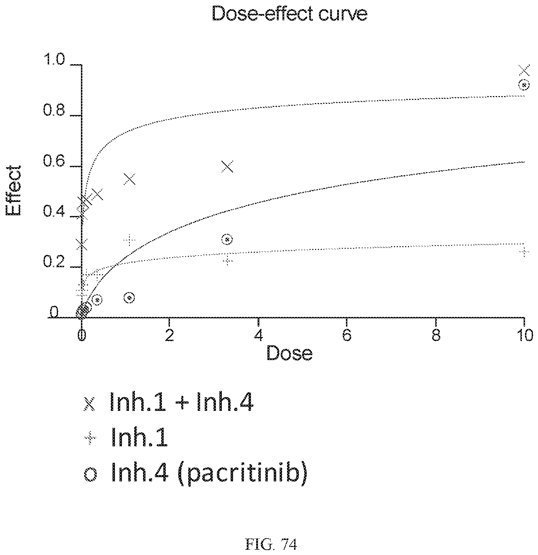

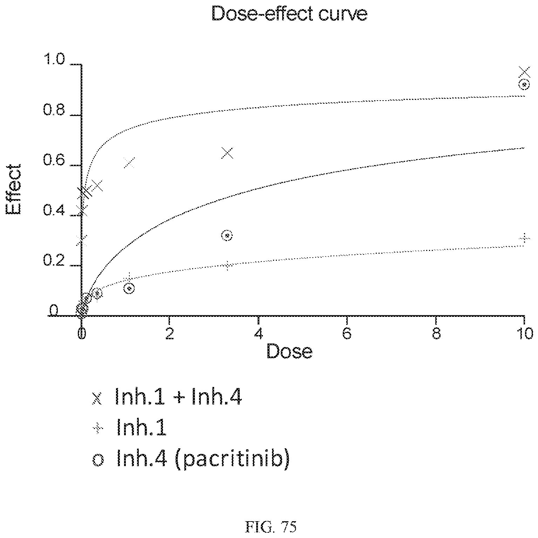

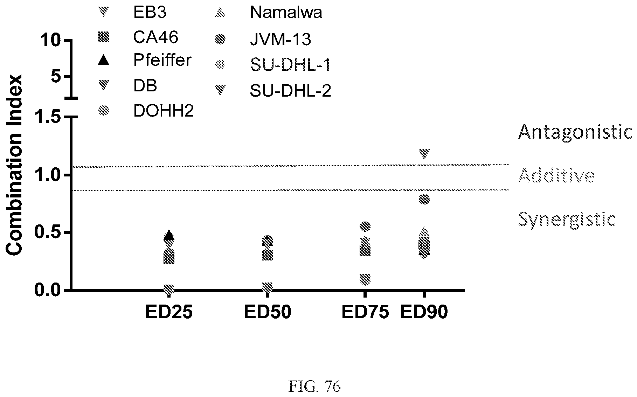

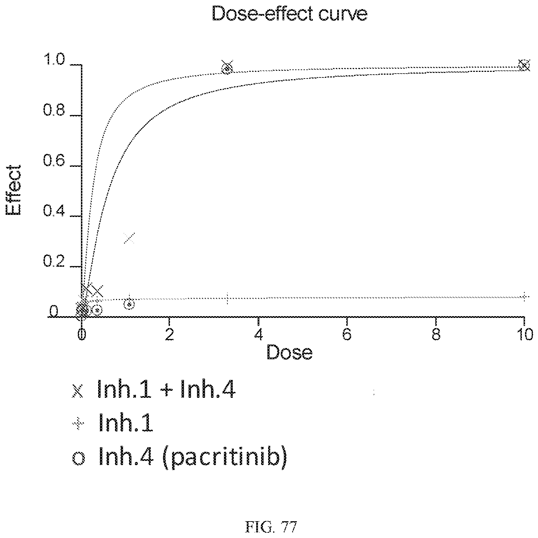

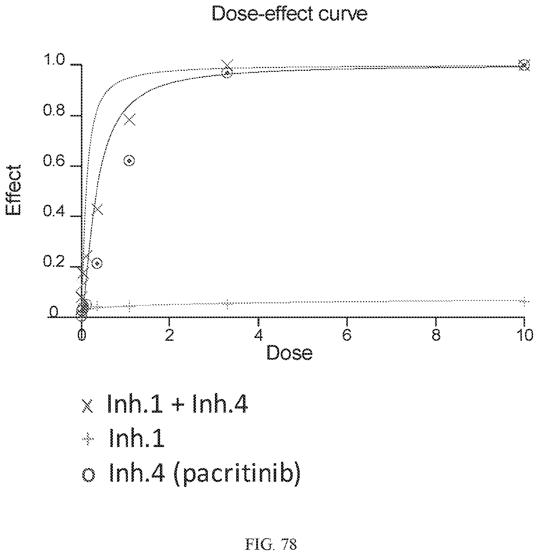

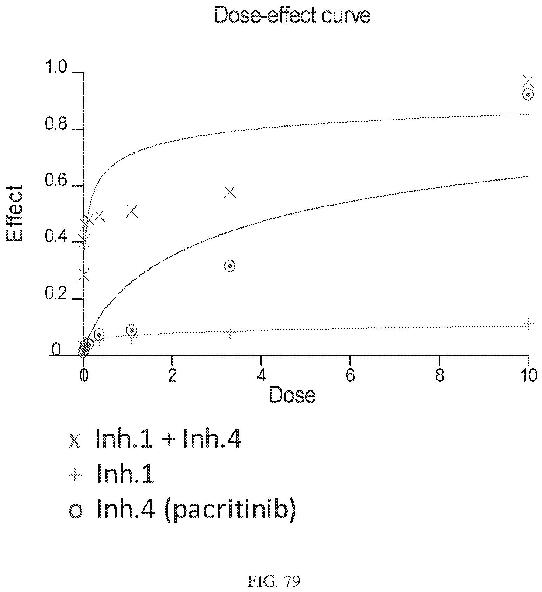

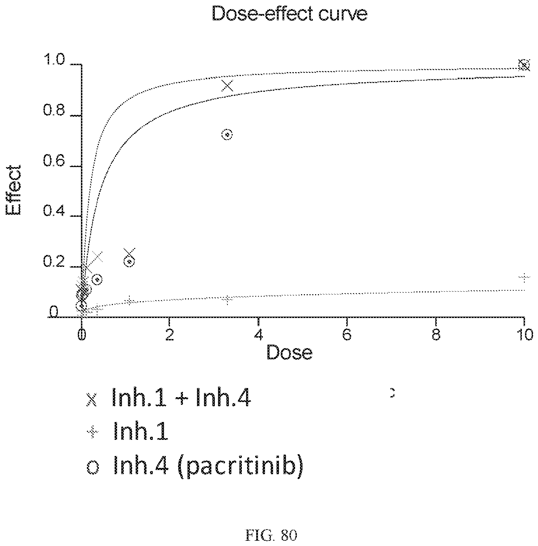

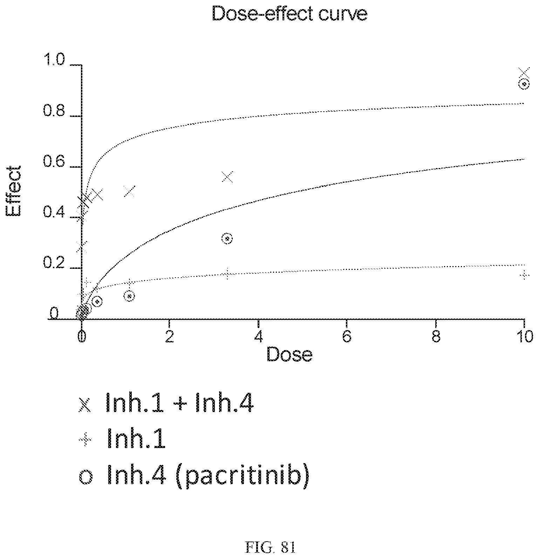

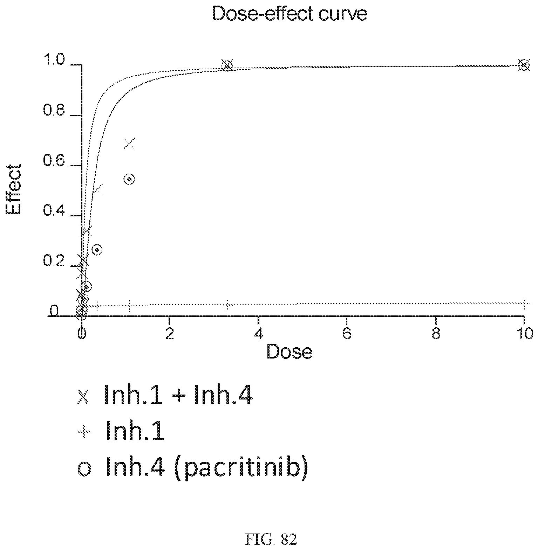

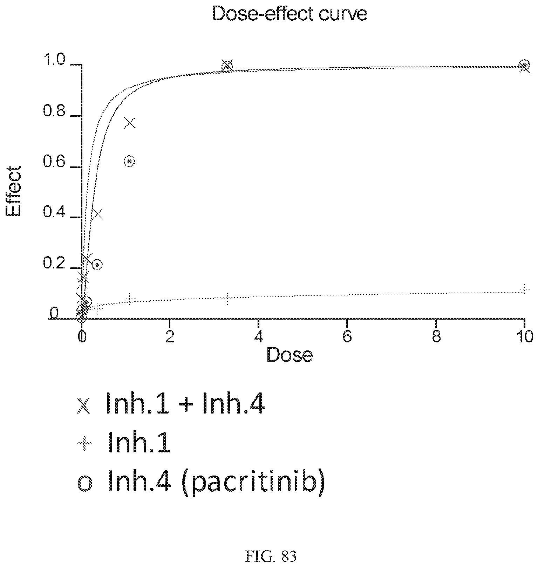

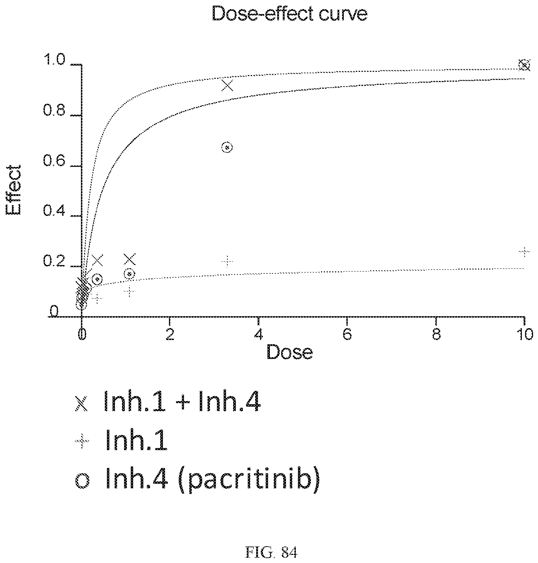

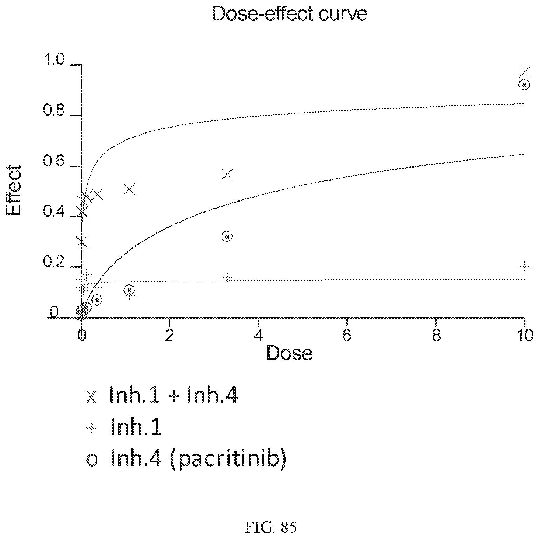

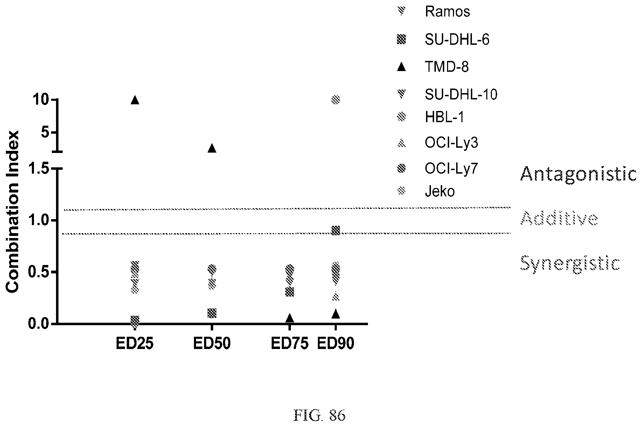

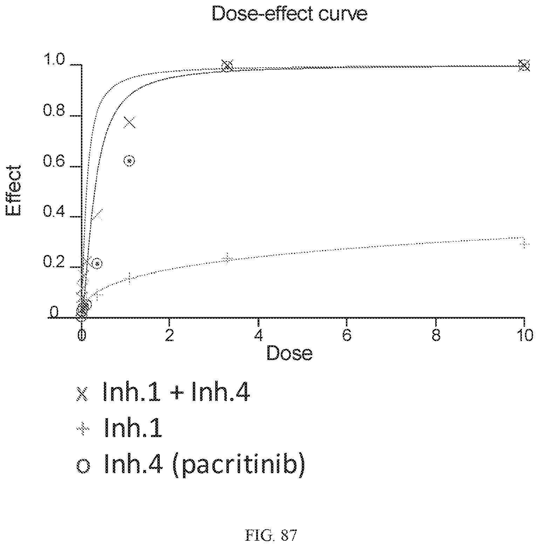

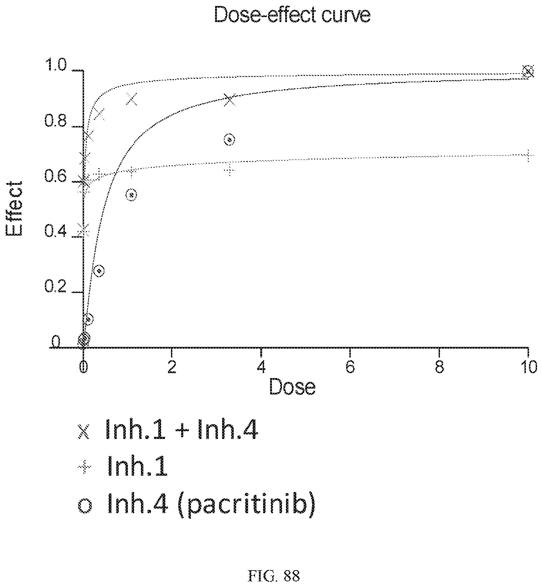

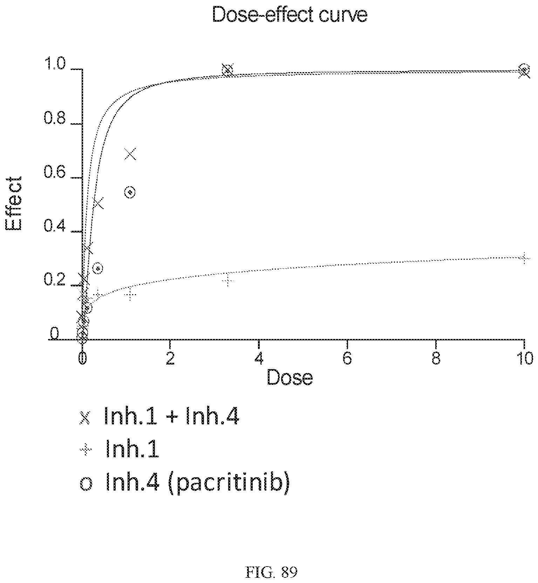

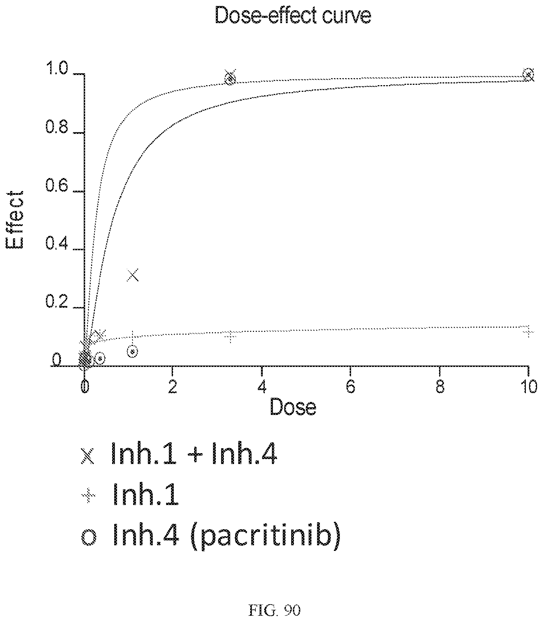

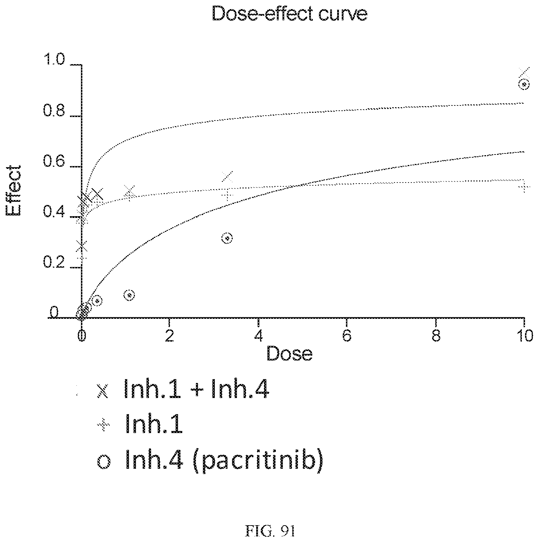

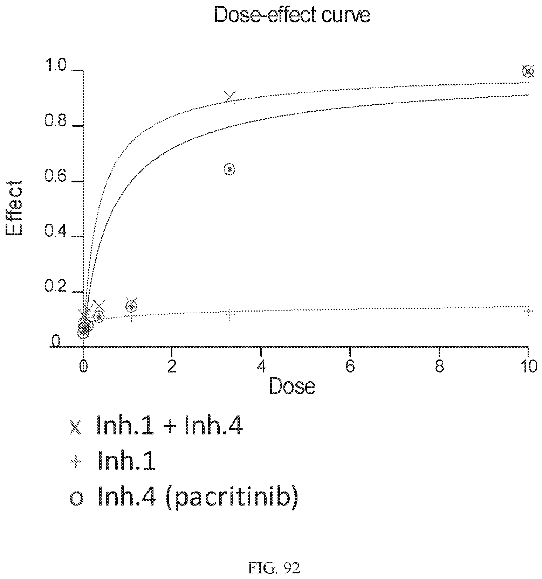

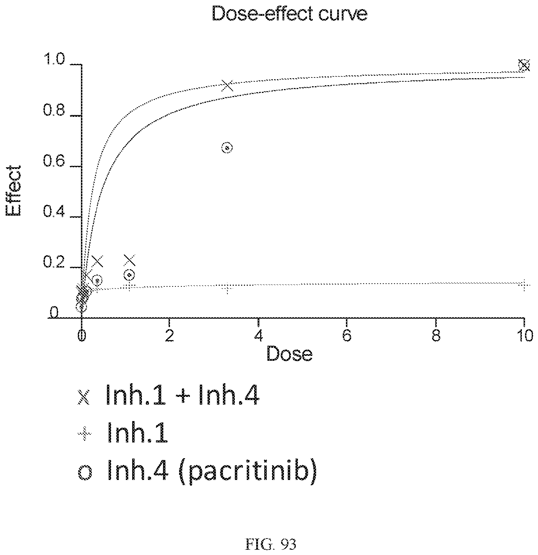

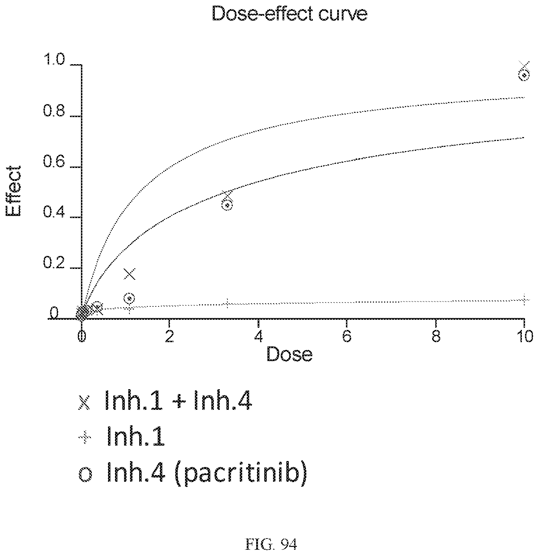

The detailed results of the cell line studies for the BTK inhibitor of Formula (XVIII) and the JAK-2 inhibitor of Formula LIV (pacritinib) are given in FIG. 66 to FIG. 94. The results of the cell line studies are summarized in Table 7.

**TABLE 7. Summary of results of the combination of a BTK inhibitor with the JAK-2 inhibitor of Formula LIV (pacritinib) (S = synergistic, A = additive, X = no effect).**

| **Cell Line** | **Indication** | **ED25** | **ED50** | **ED75** | **ED90** |
|---|---|---|---|---|---|
| Mino | MCL | S | S | S | S |
| JVM-2 | prolymphocytic leukemia | S | S | S | S |
| Maver-1 | B-ALL, MCL | S | S | S | S |
| Raji | B-ALL, Burkitt's | S | S | S | S |
| Daudi | Burkitt's | S | S | S | S |
| Rec-1 | FL | X | S | S | S |
| CCRF | B-ALL | S | S | S | S |
| Sup-B15 | B-ALL | S | S | A | A |
| SU-DHL-4 | DLBCL-ABC | S | S | S | S |
| EB3 | B-ALL, Burkitt's | S | S | S | S |
| CA46 | B-ALL, Burkitt's | S | S | S | S |
| Pfeiffer | FL | S | S | S | S |
| DB | B-ALL, MCL | S | S | S | S |
| DOHH2 | FL | S | S | S | S |
| Namalwa | B-ALL, Burkitt's | S | S | S | S |
| JVM-13 | B-ALL, MCL | S | S | S | S |
| SU-DHL-1 | DLBCL-ABC | S | S | S | S |
| SU-DHL-2 | DLBCL-ABC | S | S | S | X |
| Ramos | Burkitt's | S | S | S | S |
| SU-DHL-6 | DLBCL-GCB | S | S | S | A |
| TMD-8 | DLBCL-ABC | X | X | S | S |
| SU-DHL-10 | DLBCL-GCB | S | S | S | S |
| HBL-1 | DLBCL-ABC | S | S | S | X |
| OCI-Ly3 | DLBCL-ABC | S | S | S | S |
| OCI-Ly7 | DLBCL-ABC | S | S | S | S |
| Jeko | B-ALL, MCL | S | S | S | S |

### Reference Example 5 - Synergistic Combination of a BTK Inhibitor and a α-PD-L1 Inhibitor in the CT26 Colorectal Cancer Model

The CT26 (colon tumor #26) syngeneic mouse colorectal model was used to investigate the therapeutic efficacy of the combination of the BTK inhibitor of Formula (XVIII) and an anti-PD-L1 monoclonal antibody (BioXcell InVivoMAb anti-m-PD-L1, clone 10F.9G2). A syngeneic mouse colorectal model bears a tumor derived from the species of origin and allows for study of potential treatments in a human surrogate model with a functional immune system. CT cells were developed in 1975 by exposing Balb/c mice to *N*-nitroso-*N*-methylurethane, which lead to a rapid-growing grade IV carcinoma that may be readily implanted and easily metastasizes, as described in: Griswold et al., Cancer 1975, 36, 2441-2444. Further details of the CT26 syngeneic mouse colorectal model may be found in: Castle, et al., BMC Genomics, 2013, 15, 190; Endo, et al., Cancer Gene Therapy, 2002, 9, 142-148; Roth et al., Adv. Immunol. 1994, 57, 281-351; Fearon, et al., Cancer Res. 1988, 48, 2975-2980. CT26 cells share many molecular features with human cells and thus represent a model for aggressive, undifferentiated, refractory human colorectal carcinoma cells.

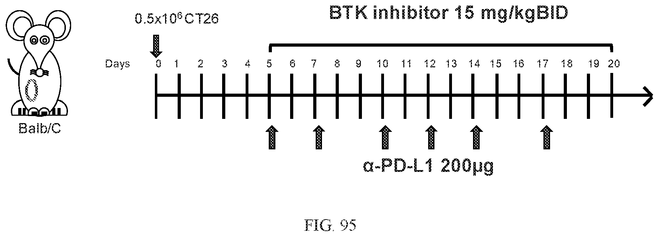

Tumor cells were cultured in complete Roswell Park Memorial Institute 1640 medium (cRPMI; Invitrogen) containing 10% fetal bovine serum (FBS; Thermo Scientific), 100 U/mL penicillin (Invitrogen), 100 µg/mL streptomycin (Invitrogen), and 50 µM 2-mercaptoethanol (2-ME) (Sigma-Aldrich). Tumor cells were implanted into mice while in the exponential growth phase (below 1.5 × 10⁶ cells/mL). Six to eight week old female Babl/c mice were inoculated with 5 × 10⁶ CT26 cells by subcutaneous injection into the right and left sides of their abdomen. Tumor growth was monitored with a digital caliper every 2 to 3 days and expressed as volume (length × width × height). The dosing scheme used with the Balb/c mice in the CT26 syngeneic mouse colorectal model is shown in FIG. 95.

### Reference Example 6 - BTK Inhibitory Effects on Solid Tumor Microenvironment in the ID8 Ovarian Cancer Model

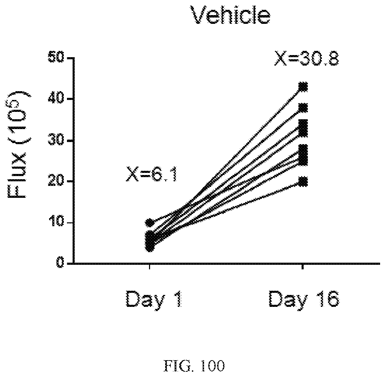

The ID8 syngeneic orthotropic ovarian cancer murine model was used to investigate the therapeutic efficacy of the BTK inhibitor of Formula (XVIII) through treatment of the solid tumor microenvironment. Human ovarian cancer models, including the ID8 syngeneic orthotropic ovarian cancer model and other animal models, are described in Fong and Kakar, J. Ovarian Res. 2009, 2, 12; Greenaway, et al., Gynecol. Oncol. 2008,108, 385-94; Urzua, et al., Tumour Biol. 2005, 26, 236-44; Janat-Amsbury, et al., Anticancer Res. 2006, 26, 3223-28; Janat-Amsbury, et al., Anticancer Res. 2006, 26, 2785-89. Animals were treated with vehicle or Formula (XVIII), 15 mg/kg/BID given orally. The results of the study are shown in FIG. 100, FIG. 101, FIG. 102, FIG. 103, FIG. 104, FIG. 105, FIG. 106, and FIG. 107.

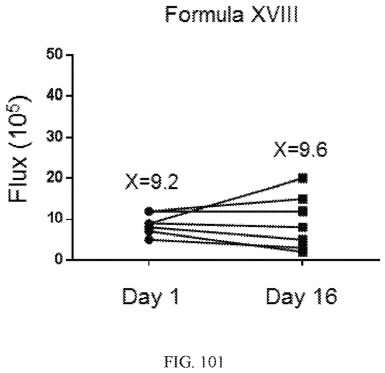

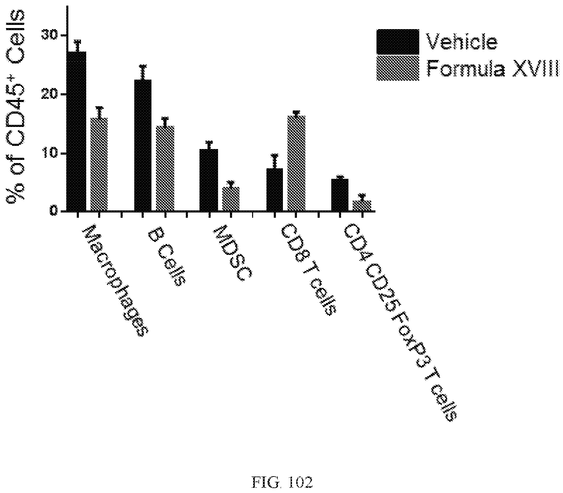

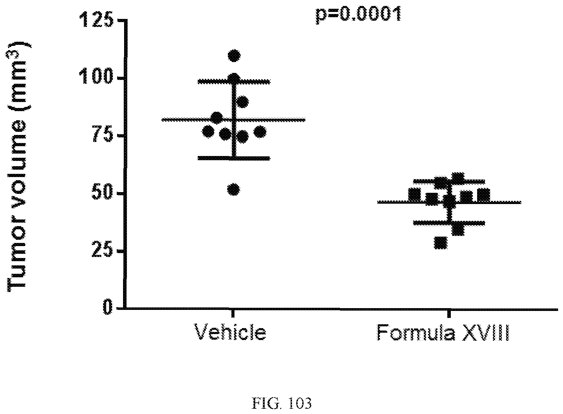

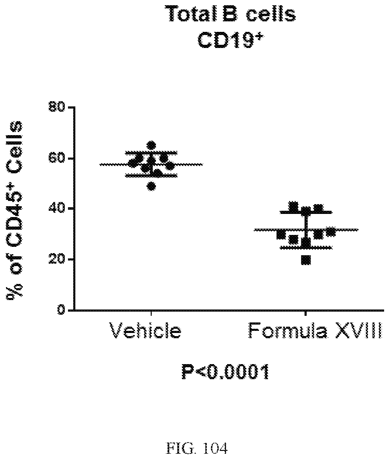

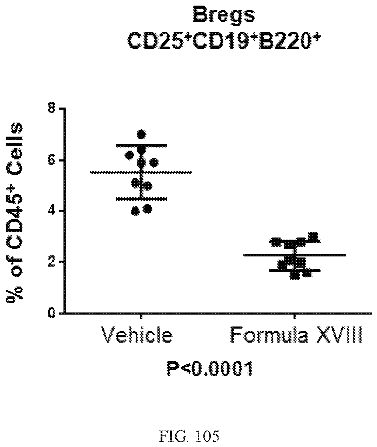

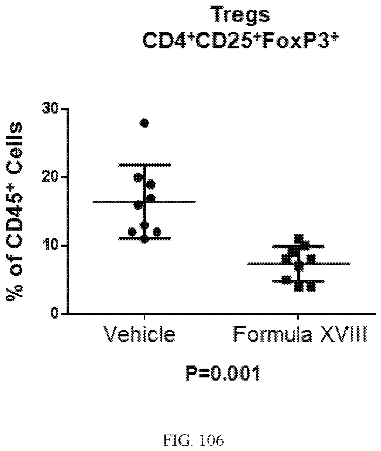

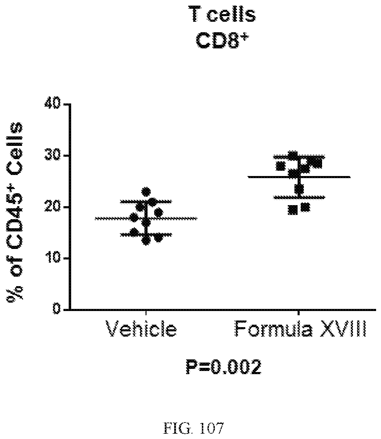

FIG. 100 and FIG. 101 demonstrate that the BTK inhibitor of Formula (XVIII) impairs ID8 ovarian cancer growth in the ID8 syngeneic murine model. FIG. 102 shows that tumor response to treatment with the BTK inhibitor of Formula (XVIII) correlates with a significant reduction in immunosuppressive tumor-associated lymphocytes in tumor-bearing mice. FIG. 103 shows treatment with the BTK inhibitor of Formula (XVIII) impairs ID8 ovarian cancer growth (through reduction in tumor volume) in the syngeneic murine model. FIG. 104 and FIG. 105 show that the tumor response induced by treatment with the BTK inhibitor of Formula (XVIII) correlates with a significant reduction in immunosuppressive B cells in tumor-bearing mice. FIG. 106 and FIG. 107 show that the tumor response induced by treatment with the BTK inhibitor of Formula (XVIII) correlates with a significant reduction in immunosuppressive tumor associated Tregs and an increase in cytolytic CD8⁺ T cells.

The results shown in FIG. 100 to FIG. 107 illustrate the surprising efficacy of the BTK inhibitor of Formula (XVIII) in modulating tumor microenvironment in a model predictive of efficacy as a treatment for ovarian cancer in humans.

### Reference Example 7 - Synergistic Combination of a BTK Inhibitor and an α-PD-L1 Inhibitor in the ID8 Ovarian Cancer Model

The ID8 ovarian cancer model of Reference Example 6 was also used to investigate potential synergistic effects between the BTK inhibitor of Formula (XVIII) and an α-PD-L1 inhibitor. Doses of 15 mg/kg BID of Formula (XVIII) and 150 µg of α-PD-L1 antibody (BioXcell InVivoMAb anti-m-PD-L1, clone 10F.9G2) were used. Each cohort consisted of 12 mice. Every two weeks mice are injected with luciferin, and tumor growth was monitored using the Xenogen system (Caliper Life Sciences, PerkinElmer, Hopkinton, MA, USA). Tumor size was measured by calculated flux.

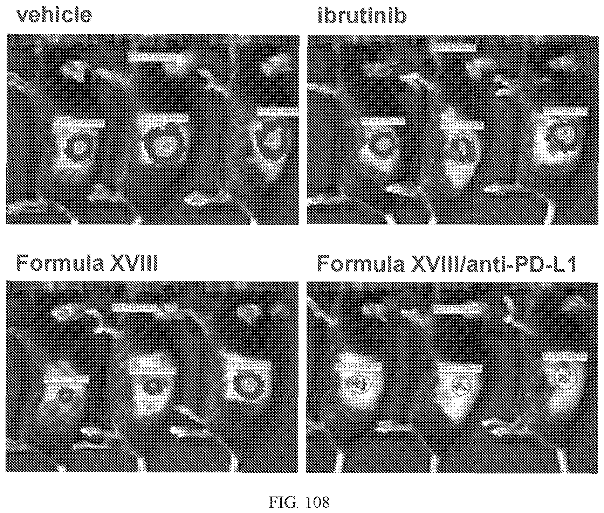

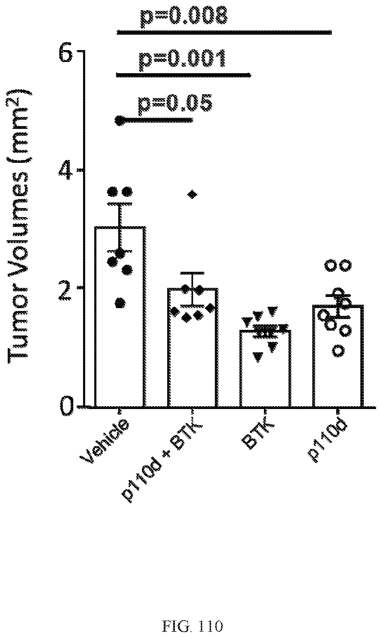

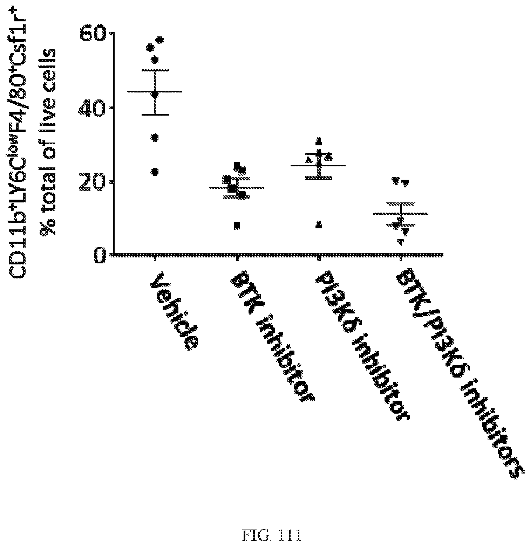

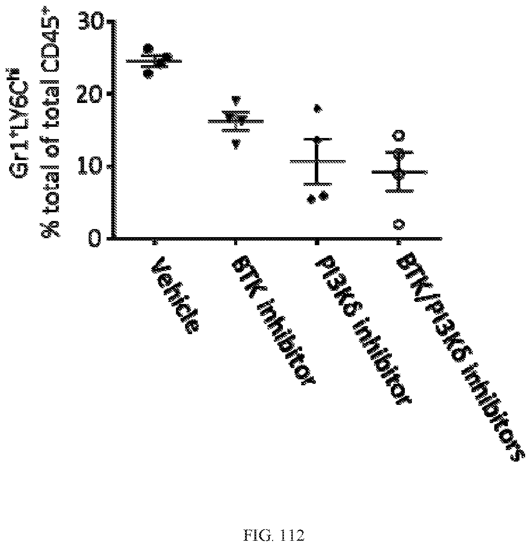

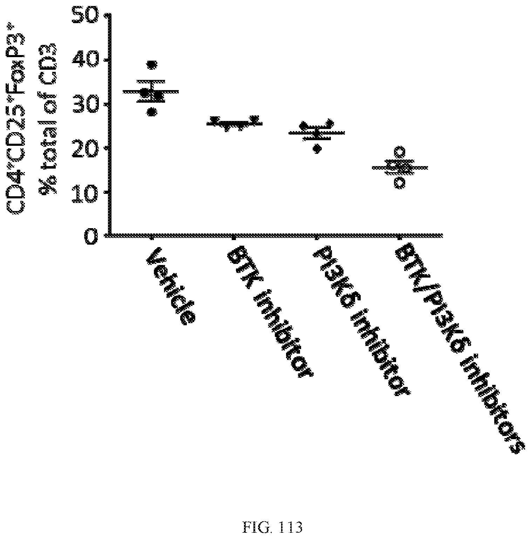

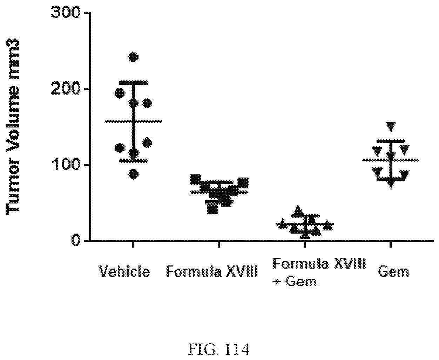

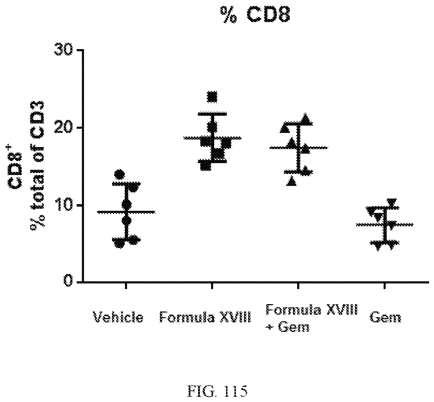

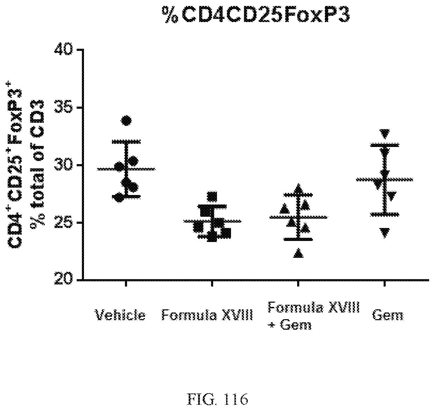

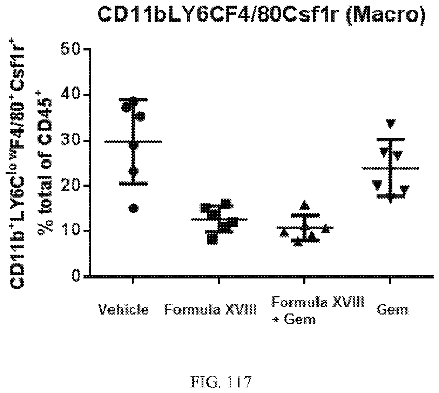

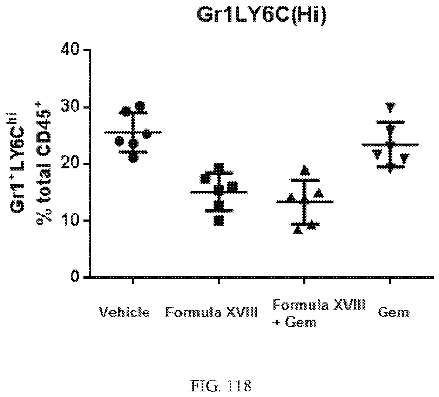

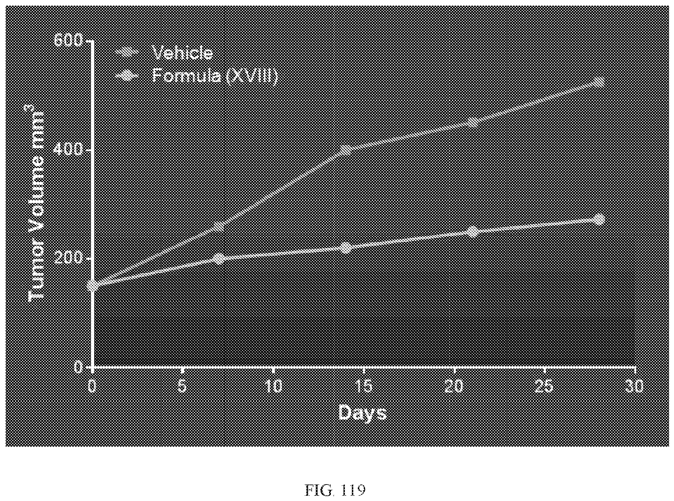

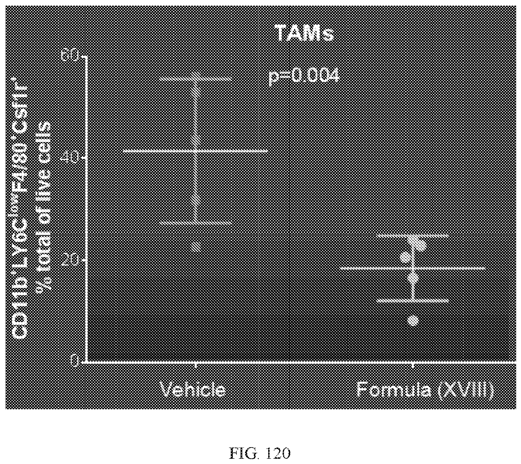

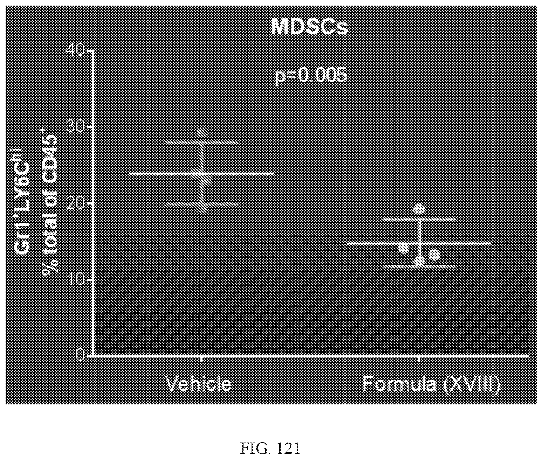

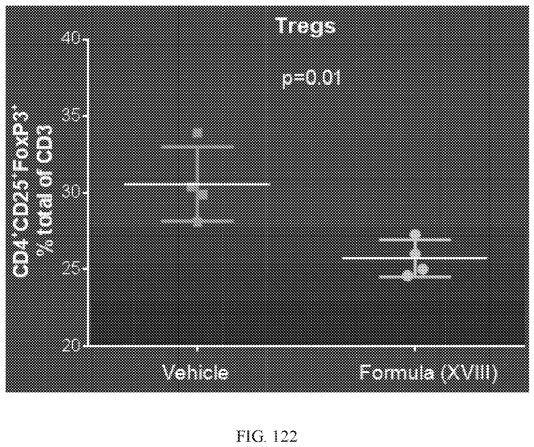

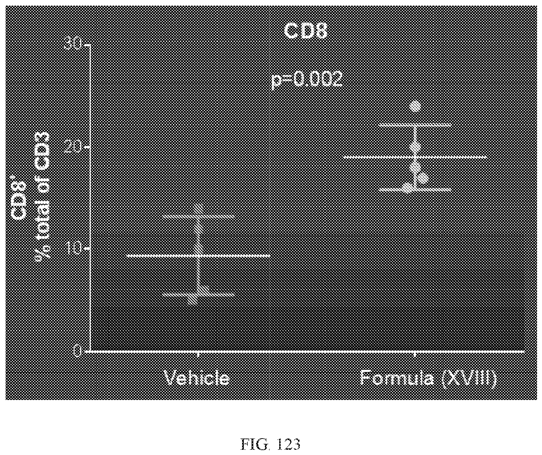

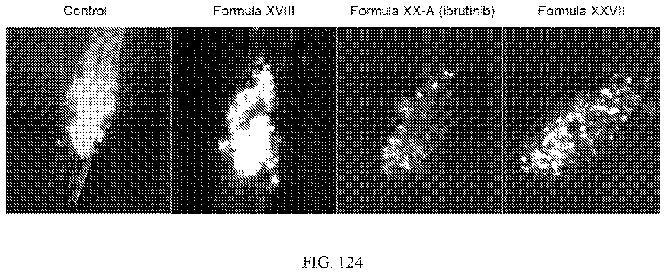

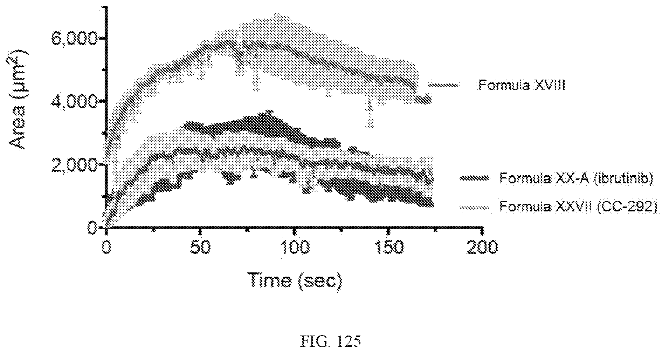

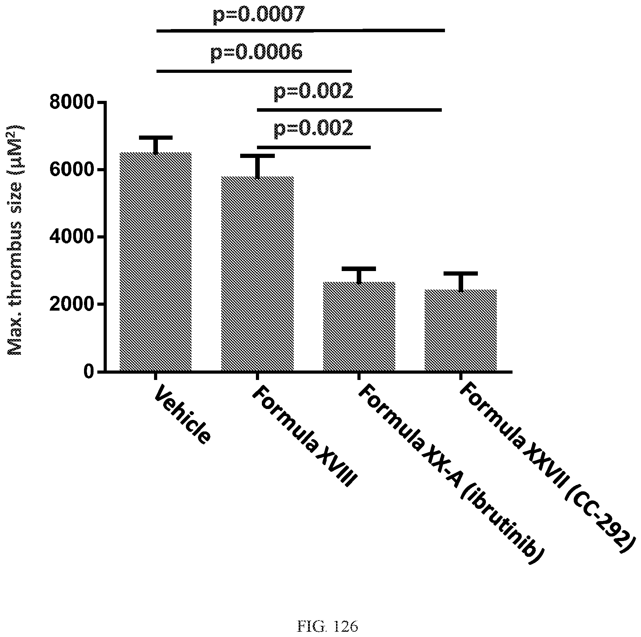

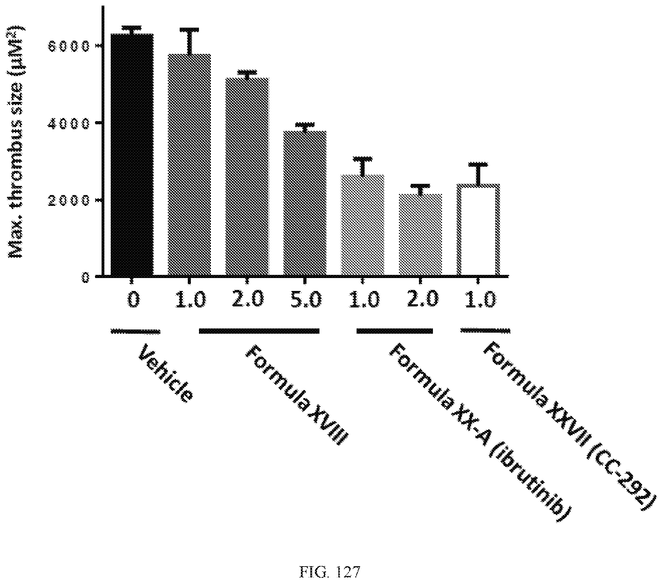

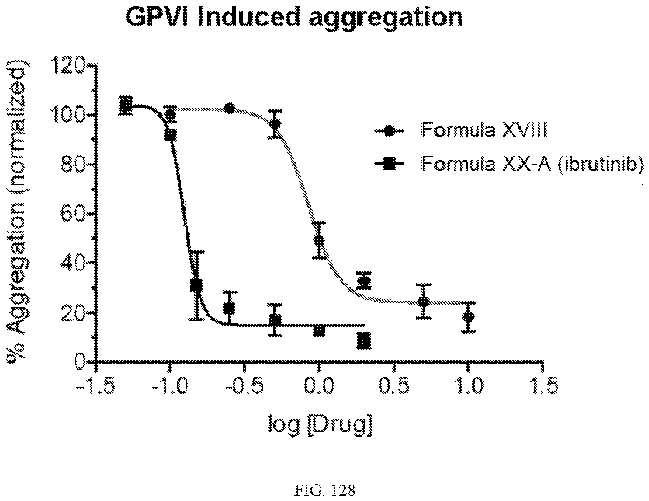

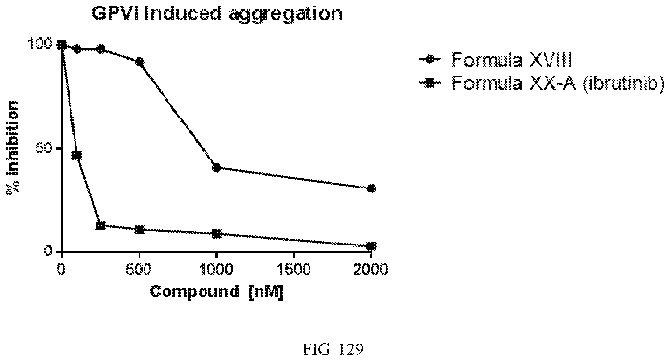

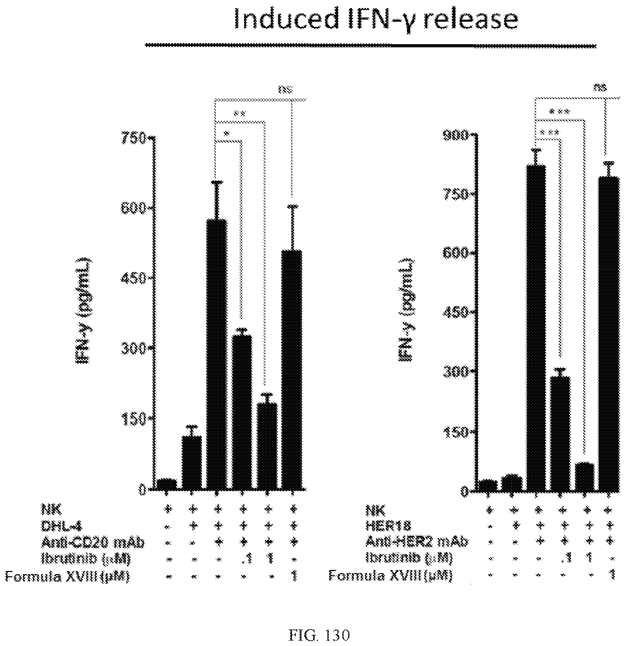

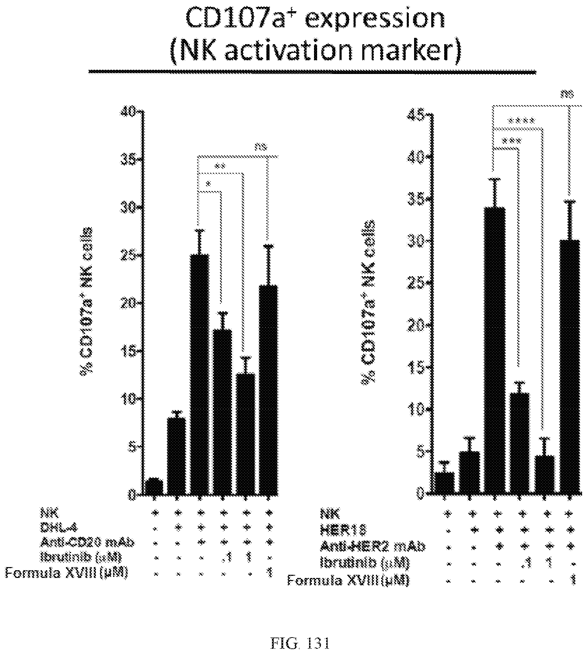

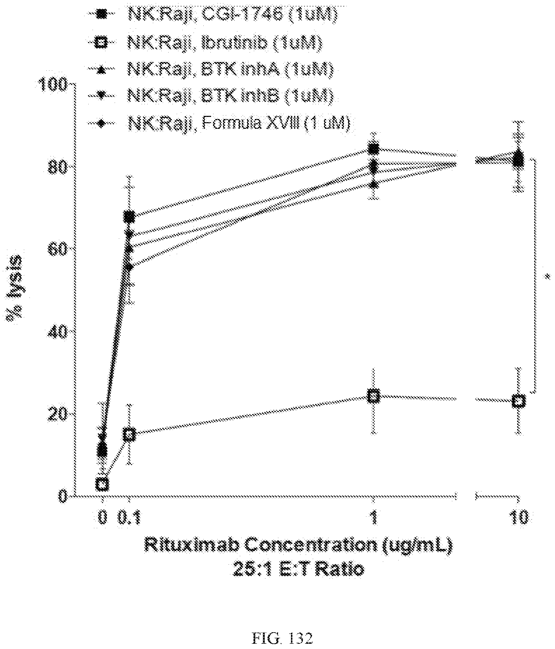

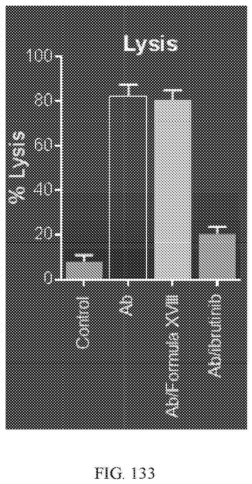

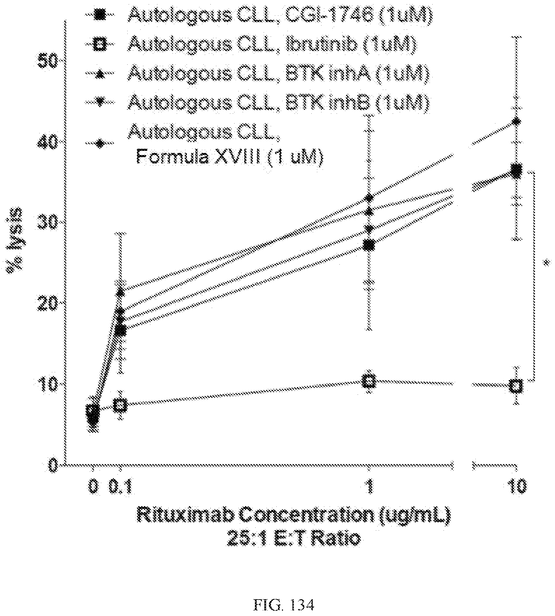

Bioluminescence imaging of mice treated with vehicle, Formula (XVIII), alone, an α-PD-L1 inhibitor (BioXcell InVivoMAb anti-m-PD-L1, clone 10F.9G2), and a combination of Formula (XVIII) and the α-PD-L1 inhibitor are shown in FIG. 108.

### Reference Example 14 - Synergistic Combinations of a BTK Inhibitor, a PI3K-δ Inhibitor, and an α-PD-L1 Inhibitor in the 4T1 Orthotopic Breast Cancer Model

The 4T1 mouse breast cancer model was used to investigate the therapeutic efficacy of various combinations of the BTK inhibitor of Formula (XVIII), the BTK inhibitor ibrutinib (Formula (XX-A)), and the PI3K-δ inhibitor of Formula (IX), with an anti-PD-1L monoclonal antibody (αPD-L1, BioXcell InVivoMAb anti-m-PD-L1, clone 10F.9G2). This model features a tumor derived from the species of origin and allows for study of potential treatments in a human surrogate model with a functional immune system. The 4T1 mouse breast cancer model is described in Pulaski and Ostrand-Rosenberg, Curr Protoc Immunol. 2001, Ch. 20, Unit 20.2, and Chen, et al., Mol. Therapy 2007, 15, 2194-202. The 4T1 model is an accepted experimental animal model for human breast cancer for at least two reasons: tumor cells are transplanted into the mammary gland so that the primary tumor grows in the correct anatomical location, and metastatic disease in this model develops spontaneously from the primary tumor. Also, the progressive spread of 4T1 metastases to lymph nodes and other organs occurs in a similar manner to the stages observed in human mammary cancer. This model also shares many molecular features with human disease and thus represents a model for advanced, resistant human breast cancers, including those resistant to 6-thioguanine and those refractory to most treatments based on stimulation of the immune system.

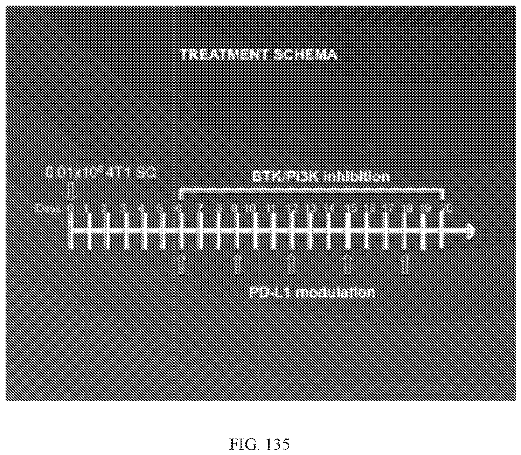

The treatment schema included the following arms: (1) IgG only; (2) the BTK inhibitor of Formula (XVIII) at 15 mg/kg, BID, on days 6 to 20; (3) the PI3K-δ inhibitor of Formula (IX) at 15 mg/kg, BID, on days 6 to 20; (4) the BTK inhibitor of Formula (XVIII) and the PI3K inhibitor of Formula (IX) each at 15 mg/kg, BID, on days 6 to 20; (5) the BTK inhibitor ibrutinib at 6 mg/kg, QD, on days 6 to 20; (6) α-PD-L1 antibody at 150 µg, on days 6, 9, 12, 15, and 18; (7) the BTK inhibitor of Formula (XVIII) at 15 mg/kg, BID, on days 6 to 20, combined with α-PD-L1 antibody at 150 µg, on days 6, 9, 12, 15, and 18; (8) the PI3K-δ inhibitor of Formula (IX) at 15 mg/kg, BID, on days 6 to 20, combined with α-PD-L1 antibody at 150 µg, on days 6, 9, 12, 15, and 18; (9) the PI3K-δ inhibitor ibrutinib at 6 mg/kg, QD, on days 6 to 20, combined with α-PD-L1 antibody at 150 µg, on days 6, 9, 12, 15, and 18; and (10) the BTK inhibitor of Formula (XVIII) and the PI3K inhibitor of Formula (IX) each at 15 mg/kg, BID, on days 6 to 20, further combined with α-PD-L1 antibody at 150 µg, on days 6, 9, 12, 15, and 18. The overall treatment and dosing schema used for the α-PD-L1 inhibitor in combination with the BTK inhibitor of Formula (XVIII), the PI3K inhibitor of Formula (IX), and the BTK inhibitor ibrutinib is depicted in FIG. 135.

The preparation of tumors and rodent species was performed as described in Reference Example 4. A total of 12 mice were used in each arm of the study.

### Reference Example 15 - Synergistic Combinations of a BTK Inhibitor, a PI3K-δ Inhibitor, and an α-PD-L1 inhibitor in the A20 Orthotopic Lymphoma Model

The A20 mouse orthotopic lymphoma cancer model was used to investigate the therapeutic efficacy of various combinations of the BTK inhibitor of Formula (XVIII), the BTK inhibitor ibrutinib (Formula (XX-A)), and the PI3K-δ inhibitor of Formula (IX), with an anti-PD-1L monoclonal antibody (αPD-L1, BioXcell InVivoMAb anti-m-PD-L1, clone 10F.9G2). The A20 lymphoma mouse model is described in Palmieri, et al., Blood 2010,116, 226-38; Kim, et al., J. Immunol. 1979, 122, 549-54. A20 is poorly immunogenic, and A20 cells are injected into BALB/c mice lead to a disseminated leukemia with infiltration of lymph nodes, liver, and spleen and the presence of malignant cells in bone marrow and peripheral blood. This leukemia is highly resistant. Myeloablative doses of irradiation followed by syngeneic bone marrow transplant fail to cure A20 bearing mice. Graner, et al., Clin Cancer Res. 2000, 6, 909-15.

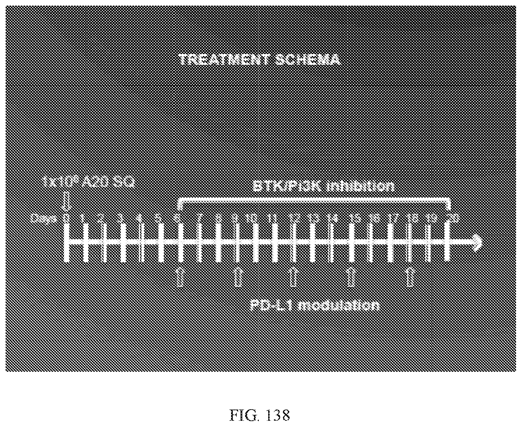

The treatment schema included the following arms: (1) IgG only; (2) the BTK inhibitor of Formula (XVIII) at 15 mg/kg, BID, on days 6 to 20; (3) the PI3K-δ inhibitor of Formula (IX) at 15 mg/kg, BID, on days 6 to 20; (4) the BTK inhibitor of Formula (XVIII) and the PI3K inhibitor of Formula (IX) each at 15 mg/kg, BID, on days 6 to 20; (5) the BTK inhibitor ibrutinib at 6 mg/kg, QD, on days 6 to 20; (6) α-PD-L1 antibody at 150 µg, on days 6, 9, 12, 15, and 18; (7) the BTK inhibitor of Formula (XVIII) at 15 mg/kg, BID, on days 6 to 20, combined with α-PD-L1 antibody at 150 µg, on days 6, 9, 12, 15, and 18; (8) the PI3K-δ inhibitor of Formula (IX) at 15 mg/kg, BID, on days 6 to 20, combined with α-PD-L1 antibody at 150 µg, on days 6, 9, 12, 15, and 18; (9) the PI3K-δ inhibitor ibrutinib at 6 mg/kg, QD, on days 6 to 20, combined with α-PD-L1 antibody at 150 µg, on days 6, 9, 12, 15, and 18; and (10) the BTK inhibitor of Formula (XVIII) and the PI3K inhibitor of Formula (IX) each at 15 mg/kg, BID, on days 6 to 20, further combined with α-PD-L1 antibody at 150 µg, on days 6, 9, 12, 15, and 18. The overall treatment and dosing schema used for the α-PD-L1 inhibitor in combination with the BTK inhibitor of Formula (XVIII), the PI3K inhibitor of Formula (IX), and the BTK inhibitor ibrutinib is depicted in FIG. 138.

The preparation of tumors and rodent species was performed as described in Example 4. A total of 12 mice were used in each arm of the study.

### Example 16 - Preclinical Characteristics of BTK Inhibitors

The BTK inhibitor ibrutinib ((1-[(3*R*)-3-[4-amino-3-(4-phenoxyphenyl)-1*H-*pyrazolo[3,4-*d*]pyrimidin-1-yl]piperidin-1-yl]prop-2-en-1-one) is a first-generation BTK inhibitor. In clinical testing as a monotherapy in subjects with hematologic malignancies, ibrutinib was generally well tolerated at dose levels through 840 mg (the highest dose tested). Advani, et al., J. Clin. Oncol. 2013, 31, 88-94; Byrd, et al., N. Engl. J. Med. 2013, 369, 32-42; Wang, et al., N. Engl. J. Med. 2013, 369, 507-16. No maximum tolerated dose (MTD) was apparent within the tested dose range. Furthermore, subjects typically found the drug tolerable over periods extending to > 2 years. No subject had tumor lysis syndrome. No overt pattern of myelosuppression was associated with ibrutinib treatment. No drug-related reductions in circulating CD4⁺ T cells or serum immunoglobulins were noted. Adverse events with an apparent relationship to study drug included diarrhea and rash.

In subjects with heavily pretreated non-Hodgkin lymphoma (NHL), ibrutinib showed substantial antitumor activity, inducing durable regressions of lymphadenopathy and splenomegaly in most subjects. Improvements in disease-associated anemia and thrombocytopenia were observed. The pattern of changes in subjects with CLL was notable. Single-active pharmaceutical ingredient ibrutinib caused rapid and substantial reductions in lymph node size concomitant with a redistribution of malignant sites into the peripheral blood. An asymptomatic absolute lymphocyte count (ALC) increase was observed that was maximal during the first few months of treatment and generally decreased thereafter but could be persistent in some subjects or could be seen repeatedly in subjects who had interruption and resumption of drug therapy.

Collectively, these data with ibrutinib support the potential benefits of selective BTK inhibition in the treatment of subjects with relapsed lymphoid cancers. However, while highly potent in inhibiting BTK, ibrutinib has also shown *in vitro* activity against other kinases with a cysteine in the same position as Cys481 in BTK to which the drug covalently binds. For example, ibrutinib inhibits epidermal growth factor receptor (EGFR), which may be the cause of ibrutinib-related diarrhea and rash. In addition, it is a substrate for both cytochrome P450 (CYP) enzymes 3A4/5 and 2D6, which increases the possibility of drug-drug interactions. These liabilities support the development of alternative BTK inhibitors for use in the therapy of lymphoid cancer.

The preclinical selectivity and potency characteristics of the second-generation BTK inhibitor of Formula (XVIII) were compared to the first-generation BTK inhibitor ibrutinib. In Table 9, a kinome screen (performed Life Technologies or based on literature data) is shown that compares these compounds.

**TABLE 9. Kinome Screen for BTK Inhibitors (IC₅₀, nM)**

| **3F-Cys Kinase** | **Formula (XVIII)** | **Ibrutinib (Formula (XX-A))** |
|---|---|---|
| Btk | 3.1 | 0.5 |
| Tec | 29 | 78 |
| Bmx | 39 | 0.80 |
| Itk | >1000 | 10.7 |
| Txk | 291 | 2.0 |
| EGFR | >1000 | 5.6 |
| ErbB2 | 912 | 9.4 |
| ErbB4 | 13.2 | 2.7 |
| Blk | >1000 | 0.5 |
| JAK-3 | >1000 | 16.1 |

The results shown in Table 9 are obtained from a 10 point biochemical assay generated from 10 point concentration curves. The BTK inhibitor of Formula (XVIII) shows much greater selectivity for BTK compared to other kinases than ibrutinib.

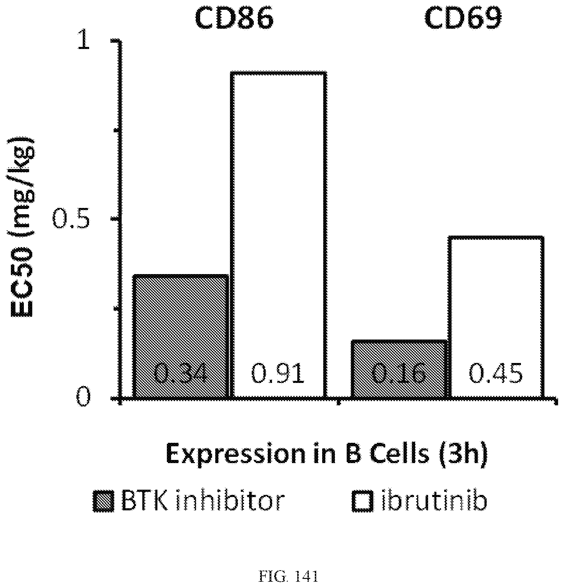

A comparison of the *in vivo* potency results for the BTK inhibitors of Formula (XVIII) and ibrutinib is shown in FIG. 141. CD86 and CD69 are cell surface proteins that are BCR activation markers. To obtain the *in vivo* potency results, mice were gavaged at increasing drug concentration and sacrificed at one time point (3 h post-dose). BCR was stimulated with IgM and the expression of activation marker CD69 and CD86 are monitored by flow cytometry and to determine EC₅₀ values.

*In vitro* and *in vivo* safety pharmacology studies with Formula (XVIII) have demonstrated a favorable nonclinical safety profile. When screened at 10 µM in binding assays evaluating interactions with 80 known pharmacologic targets such as G-proteincoupled receptors, nuclear receptors, proteases, and ion channels, Formula (XVIII) shows significant activity only against the A3 adenosine receptor; follow-up dose-response experiments indicated a IC₅₀ of 2.7 µM, suggesting a low clinical risk of off-target effects. Formula (XVIII) at 10 µM showed no inhibition of *in vitro* EGFR phosphorylation in an A431 human epidermoid cancer cell line whereas ibrutinib had an IC₅₀ of 66 nM. The *in vitro* effect of Formula (XVIII) on human ether-à-go-go-related gene (hERG) channel activity was investigated *in vitro* in human embryonic kidney cells stably transfected with hERG. Formula (XVIII) inhibited hERG channel activity by 25% at 10 µM, suggesting a low clinical risk that Formula (XVIII) would induce clinical QT prolongation as predicted by this assay. Formula (XVIII) was well tolerated in standard *in vivo* Good Laboratory Practices (GLP) studies of pharmacologic safety. A functional observation battery in rats at doses of through 300 mg/kg (the highest dose level) revealed no adverse effects on neurobehavioral effects or body temperature at any dose level. A study of respiratory function in rats also indicated no treatment-related adverse effects at doses through 300 mg/kg (the highest dose level). In a cardiovascular function study in awake telemeterized male beagle dogs, single doses of Formula (XVIII) at dose levels through 30 mg/kg (the highest dose level) induced no meaningful changes in body temperature, cardiovascular, or electrocardiographic (ECG) (including QT interval) parameters. The results suggest that Formula (XVIII) is unlikely to cause serious off-target effects or adverse effects on critical organ systems.

The drug-drug interaction potential of Formula (XVIII) was also evaluated. *In vitro* experiments evaluating loss of parent drug as catalyzed by CYPs indicated that Formula (XVIII) is metabolized by CYP3A4. *In vitro* metabolism studies using mouse, rat, dog, rabbit, monkey, and human hepatocytes incubated with ¹⁴C-labeled Formula (XVIII) indicated two mono-oxidized metabolites and a glutathione conjugate. No unique human metabolite was identified. Preliminary evaluations of metabolism in the plasma, bile, and urine of rats, dogs, and monkeys indicated metabolic processes of oxidation, glutathione binding, and hydrolysis. It was shown that Formula (XVIII) binds to glutathione but does not deplete glutathione *in vitro.* Nonclinical CYP interaction studies data indicate that Formula (XVIII) is very unlikely to cause clinical drug-drug interactions through alteration of the metabolism of drugs that are substrates for CYP enzymes.

### Example 17 - Clinical Study of a BTK Inhibitor in Leukemia/Lymphoma and Effects on Bone Marrow and Lymphoid Microenvironments

Clinical studies have shown that targeting the BCR signaling pathway by inhibiting BTK produces significant clinical benefit in patients with non-Hodgkin's lymphoma (NHL). The second generation BTK inhibitor, Formula (XVIII), achieves significant oral bioavailability and potency, and has favorable preclinical characteristics, as described above. The purpose of this study is to evaluate the safety and efficacy of the second generation BTK inhibitor of Formula (XVIII) in treating subjects with chronic lymphocytic leukemia (CLL) and small lymphocytic lymphoma (SLL).

The design and conduct of this study is supported by an understanding of the history and current therapies for subjects with lymphoid cancers; knowledge of the activity and safety of a first-generation BTK inhibitor, ibrutinib, in subjects with hematologic cancers; and the available nonclinical information regarding Formula (XVIII). The collective data support the following conclusions. BTK expression plays an important role in the biology of lymphoid neoplasms, which represent serious and life-threatening disorders with continuing unmet medical need. Clinical evaluation of Formula (XVIII) as a potential treatment for these disorders has sound scientific rationale based on observations that the compound selectively abrogates BTK activity and shows activity in nonclinical models of lymphoid cancers. These data are supported by clinical documentation that ibrutinib, a first-generation BTK inhibitor, is clinically active in these diseases. Ibrutinib clinical data and Formula (XVIII) nonclinical safety pharmacology and toxicology studies support the safety of testing Formula (XVIII) in subjects with B cell malignancies.

The primary objectives of the clinical study are as follows: (1) establish the safety and the MTD of orally administered Formula (XVIII) in subjects with CLL/SLL; (2) determine pharmacokinetics (PK) of orally administered Formula (XVIII) and identification of its major metabolite(s); and (3) measure pharmacodynamic (PD) parameters including drug occupancy of BTK, the target enzyme, and effect on biologic markers of B cell function.

The secondary objective of the clinical study is to evaluate tumor responses in patients treated with Formula (XVIII).

This study is a multicenter, open-label, nonrandomized, sequential group, dose escalation study. The following dose cohorts will be evaluated:
Cohort 1: 100 mg/day for 28 days (= 1 cycle)
Cohort 2: 175 mg/day for 28 days (= 1 cycle)
Cohort 3: 250 mg/day for 28 days (= 1 cycle)
Cohort 4: 350 mg/day for 28 days (= 1 cycle)
Cohort 5: 450 mg/day for 28 days (= 1 cycle)
Cohort 6: To be determined amount in mg/day for 28 days (= 1 cycle)

Each cohort will be enrolled sequentially with 6 subjects per cohort. If ≤ 1 dose-limiting toxicity (DLT) is observed in the cohort during Cycle 1, escalation to the next cohort will proceed. Subjects may be enrolled in the next cohort if 4 of the 6 subjects enrolled in the cohort completed Cycle 1 without experiencing a DLT, while the remaining 2 subjects are completing evaluation. If ≥ 2 DLTs are observed during Cycle 1, dosing at that dose and higher will be suspended and the MTD will be established as the previous cohort. The MTD is defined as the largest daily dose for which fewer than 33% of the subjects experience a DLT during Cycle 1. Dose escalation will end when either the MTD is achieved or at 3 dose levels above full BTK occupancy, whichever occurs first. Full BTK occupancy is defined as Formula (XVIII) active-site occupancy of > 80% (average of all subjects in cohort) at 24 hours postdose. Should escalation to Cohort 6 be necessary, the dose will be determined based on the aggregate data from Cohorts 1 to 5, which includes safety, efficacy, and PK/PD results. The dose for Cohort 6 will not exceed 900 mg/day.

Treatment with Formula (XVIII) may be continued for > 28 days until disease progression or an unacceptable drug-related toxicity occurs. Subjects with disease progression will be removed from the study. All subjects who discontinue study drug will have a safety follow-up visit 30 (±7) days after the last dose of study drug unless they have started another cancer therapy within that timeframe. Radiologic tumor assessment will be done at screening and at the end of Cycle 2, Cycle 4, and Cycle 12 and at investigator discretion. Confirmation of complete response (CR) will require bone marrow analysis and radiologic tumor assessment. For subjects who remain on study for > 11 months, a mandatory bone marrow aspirate and biopsy is required in Cycle 12 concurrent with the radiologic tumor assessment.

All subjects will have standard hematology, chemistry, and urinalysis safety panels done at screening. This study also includes pancreatic function assessment (serum amylase and serum lipase) due to the pancreatic findings in the 28-day GLP rat toxicity study. Once dosing commences, all subjects will be evaluated for safety once weekly for the first 4 weeks, every other week for Cycle 2, and monthly thereafter. Blood samples will be collected during the first week of treatment for PK/PD assessments. ECGs will be done at screening, and on Day 1-2, 8, 15, 22, 28 of Cycle 1, Day 15 and 28 of Cycle 2, and monthly thereafter through Cycle 6. ECGs are done in triplicate for screening only. Thereafter, single ECG tests are done unless a repeat ECG testing is required.

Dose-limiting toxicity is defined as any of the following events (if not related to disease progression): (1) any Grade ≥ 3 non-hematologic toxicity (except alopecia) persisting despite receipt of a single course of standard outpatient symptomatic therapy (e.g., Grade 3 diarrhea that responds to a single, therapeutic dose of Imodium^{®} would not be considered a DLT); (2) grade ≥ 3 prolongation of the corrected QT interval (QTc), as determined by a central ECG laboratory overread; (3) grade 4 neutropenia (absolute neutrophil count [ANC] < 500/µL) lasting > 7 days after discontinuation of therapy without growth factors or lasting > 5 days after discontinuation of therapy while on growth factors (i.e., Grade 4 neutropenia not lasting as long as specified will not be considered a DLT), (4) grade 4 thrombocytopenia (platelet count < 20,000/µL) lasting > 7 days after discontinuation of therapy or requiring transfusion (*i.e*., Grade 4 thrombocytopenia not lasting as long as specified will not be considered a DLT), and (5) dosing delay due to toxicity for > 7 consecutive days.

The efficacy parameters for the study include overall response rate, duration of response, and progression-free survival (PFS). The safety parameters for the study include DLTs and MTD, frequency, severity, and attribution of adverse events (AEs) based on the Common Terminology Criteria for Adverse Events (CTCAE v4.03) for non-hematologic AEs. Hallek, et al., Blood 2008, 111, 5446-5456.

The schedule of assessments is as follows, with all days stated in the following meaning the given day or +/- 2 days from the given day. A physical examination, including vital signs and weight, are performed at screening, during cycle 1 at 1, 8, 15, 22, and 28 days, during cycle 2 at 15 and 28 days, during cycles 3 to 24 at 28 days, and at follow up (after the last dose). The screening physical examination includes, at a minimum, the general appearance of the subject, height (screening only) and weight, and examination of the skin, eyes, ears, nose, throat, lungs, heart, abdomen, extremities, musculoskeletal system, lymphatic system, and nervous system. Symptom-directed physical exams are done thereafter. Vital signs (blood pressure, pulse, respiratory rate, and temperature) are assessed after the subject has rested in the sitting position. Eastern Cooperative Oncology Group (ECOG) status is assessed at screening, during cycle 1 at 1, 8, 15, 22, and 28 days, during cycle 2 at 15 and 28 days, during cycles 3 to 24 at 28 days, and at follow up, using the published ECOG performance status indications described in Oken, et al., Am. J. Clin. Oncol. 1982, 5, 649-655. ECG testing is performed at screening, during cycle 1 at 1, 2, 8, 15, 22, and 28 days, during cycle 2 at 15 and 28 days, during cycles 3 to 24 at 28 days, and at follow up. The 12-lead ECG test will be done in triplicate (≥ 1 minute apart) at screening. The calculated QTc average of the 3 ECGs must be <480 ms for eligibility. On cycle 1, day 1 and cycle 1, day 8, single ECGs are done predose and at 1, 2, 4, and 6 h postdose. The single ECG on Cycle 1 Day 2 is done predose. On cycle 1, day 15, day 22, and day 28, a single ECG is done 2 hours post-dose. Starting with cycle 2, a single ECG is done per visit. Subjects should be in supine position and resting for at least 10 minutes before study-related ECGs. Two consecutive machine-read QTc > 500 ms or > 60 ms above baseline require central ECG review. Hematology, including complete blood count with differential and platelet and reticulocyte counts, is assessed at screening, during cycle 1 at 1, 8, 15, 22, and 28 days, during cycle 2 at 15 and 28 days, during cycles 3 to 24 at 28 days, and at follow up. Serum chemistry is assesed at screening, during cycle 1 at 1, 8, 15, 22, and 28 days, during cycle 2 at 15 and 28 days, during cycles 3 to 24 at 28 days, and at follow up. Serum chemistry includes albumin, alkaline phosphatase, ALT, AST, bicarbonate, blood urea nitrogen (BUN), calcium, chloride, creatinine, glucose, lactate dehydrogenase (LDH), magnesium, phosphate, potassium, sodium, total bilirubin, total protein, and uric acid. Cell counts and serum immunoglobulin are performed at screening, at cycle 2, day 28, and at every 6 months thereafter until last dose and include T/B/NK/monocyte cell counts (CD3, CD4, CD8, CD14, CD19, CD19, CD16/56, and others as needed) and serum immunoglobulin (IgG, IgM, IgA, and total immunoglobulin). Bone marrow aspirates are performed at cycle 12. Pharmacodynamics samples are drawn during cycle 1 at 1, 2, and 8 days, and at follow up. On days 1 and 8, pharmacodynamic samples are drawn pre-dose and 4 hours (±10 minutes) post-dose, and on day 2, pharmacodynamic samples are drawn pre-dose. Pharmacokinetics samples are drawn during cycle 1 at 1, 2, 8, 15, 22, and 28 days. Pharmacokinetic samples for Cycle 1 Day 1 are drawn pre-dose and at 0.5, 1, 2, 4, 6 and 24 hours (before dose on Day 2) post-dose. Samples for Cycle 1 Day 8 are drawn pre-dose and at 0.5, 1, 2, 4, and 6 hours post-dose. On Cycle 1 Day 15, 22, and 28, a PK sample is drawn pre-dose and the second PK sample must be drawn before (up to 10 minutes before) the ECG acquisition, which is 2 hours postdose. Pretreatment radiologic tumor assessments are performed within 30 days before the first dose. A computed tomography (CT) scan (with contrast unless contraindicated) is required of the chest, abdomen, and pelvis. In addition, a positron emission tomography (PET) or PET/CT must done for subjects with SLL. Radiologic tumor assessments are mandatory at the end of Cycle 2 (-7 days), Cycle 4 (- 7days), and Cycle 12 (-7 days). Otherwise, radiologic tumor assessments are done at investigator discretion. A CT (with contrast unless contraindicated) scan of the chest, abdomen, and pelvis is required for subjects with CLL. In addition, a PET/CT is required in subjects with SLL. Bone marrow and radiologic assessments are both required for confirmation of a complete response (CR). Clinical assessments of tumor response should be done at the end of Cycle 6 and every 3 months thereafter. Molecular markers are measured at screening, and include interphase cytogenetics, stimulated karyotype, IgHV mutational status, Zap-70 methylation, and beta-2 microglobulin levels. Urinalysis is performed at screening, and includes pH, ketones, specific gravity, bilirubin, protein, blood, and glucose. Other assessments, including informed consent, eligibility, medical history, and pregnancy test are done at the time of screening.

The investigator rates the subject's response to treatment based on recent guidelines for CLL, as given in Hallek, et al., Blood 2008, 111, 5446-56, and for SLL, as given in Cheson, et al., J. Clin. Oncol. 2007, 25, 579-586. The response assessment criteria for CLL are summarized in Table 10.

**TABLE 10. Response Assessment Criteria for CLL. Abbreviations: ANC = absolute neutrophil count; CR = complete remission; CRi = CR with incomplete blood count recovery; PR = partial remission.**

| **Response** | **Peripheral Blood** | **Bone Marrow (if performed)** | **Nodes, Liver, and Spleen^{a}** |
|---|---|---|---|
| CR | Lymphocytes < 4 × 10⁹/L ANC >1.5 × 10⁹/L^{b} Platelets > 100 × 10⁹/L^{b} Hemoglobin > 11.0 g/dL (untransfused)^{b} | Normocellular <30% lymphocytes No B-lymphoid nodules | Normal (e.g., no lymph nodes >1.5 cm) |
| CRi | Lymphocytes < 4 × 10⁹/L Persistent anemia, thrombocytopenia, or neutropenia related to drug toxicity | Hypocellular <30% lymphocytes | Normal (e.g., no lymph nodes >1.5 cm) |
| PR | Lymphocytes ≥ 50% decrease from baseline ANC > 1.5 × 10⁹/L or Platelets > 100 × 10⁹/L or 50% improvement over baseline^{b} or Hemoglobin > 11.0 g/dL or 50% improvement over baseline (untransfused)^{b} | Not assessed | ≥50% reduction in lymphadenopathy^{c} and/or in spleen or liver enlargement |

| | | | |
|---|---|---|---|
| a. Computed tomography (CT) scan of abdomen, pelvis, and chest is required for this evaluation b. Without need for exogenous growth factors c. In the sum products of ≤ 6 lymph nodes or in the largest diameter of the enlarged lymph node(s) detected before therapy and no increase in any lymph node or new enlarged lymph nodes | | | |

The response assessment criteria for SLL are summarized in Table 11.

**TABLE 11. Response Assessment Criteria for SLL. Abbreviations: CR = complete remission, CT = computed tomography, FDG = [¹⁸F]fluorodeoxyglucose, PET = positron-emission tomography, PR = partial remission, SD = stable disease, SPD = sum of the product of the diameters.**

| **Response** | **Definition** | **Nodal Masses** | **Spleen**, **Liver** | **Bone Marrow** |
|---|---|---|---|---|
| CR | Disappearance of all evidence of disease | (a) FDG-avid or PET positive prior to therapy; mass of any size permitted if PET negative (b) Variably FDG-avid or PET negative; regression to normal size on CT | Not palpable, nodules disappeared | If infiltrate present at screening, infiltrate cleared on repeat biopsy; if indeterminate by morphology, immunohistochemistry should be negative |
| PR | Regression of measurable disease and no new sites | ≥ 50% decrease in SPD of up to 6 largest dominant masses; no increase in size of other nodes (a) FDG-avid or PET positive prior to therapy; ≥ 1 PET positive at previously involved site (b) Variably FDG-avid or PET negative; regression on CT | ≥ 50% decrease in SPD of nodules (for single nodule in greatest transverse diameter); no increase in size of liver or spleen | Irrelevant if positive prior to therapy; cell type should be specified |
| SD | Failure to attain CR/PR or progressive disease | (a) FDG-avid or PET positive prior to therapy; PET positive at prior sites of disease, and no new sites on CT or PET (b) Variably FDG avid or PET negative; no change in size of previous lesions on CT | | |

The PK parameters of the study are as follows. The plasma PK of Formula (XVIII) and a metabolite is characterized using noncompartmental analysis. The following PK parameters are calculated, whenever possible, from plasma concentrations of Formula (XVIII):
AUC₍₀₋ₜ₎: Area under the plasma concentration-time curve calculated using linear trapezoidal summation from time 0 to time t, where t is the time of the last measurable concentration (Ct),
AUC₍₀₋₂₄₎: Area under the plasma concentration-time curve from 0 to 24 hours, calculated using linear trapezoidal summation,
AUC_{(o-∞)}: Area under the plasma concentration-time curve from 0 to infinity, calculated using the formula: AUC_{(0-∞)} = AUC₍₀₋ₜ₎ + Ct / λz, where λz is the apparent terminal elimination rate constant,
Cmax: Maximum observed plasma concentration,
Tmax: Time of the maximum plasma concentration (obtained without interpolation),
t_{½}: Terminal elimination half-life (whenever possible),
λ_{z}: Terminal elimination rate constant (whenever possible),
Cl/F: Oral clearance.

The PD parameters of the study are as follows. The occupancy of BTK by Formula (XVIII) are measured in peripheral blood mononuclear cells (PBMCs) with the aid of a biotin-tagged Formula (XVIII) analogue probe. The effect of Formula (XVIII) on biologic markers of B cell function will also be evaluated.

The statistical analysis used in the study is as follows. No formal statistical tests of hypotheses are performed. Descriptive statistics (including means, standard deviations, and medians for continuous variables and proportions for discrete variables) are used to summarize data as appropriate.

The following definitions are used for the safety and efficacy analysis sets: Safety analysis set: All enrolled subjects who receive ≥ 1 dose of study drug; Perprotocol (PP) analysis set: All enrolled subjects who receive ≥ 1 dose of study drug and with ≥ 1 tumor response assessment after treatment. The safety analysis set will be used for evaluating the safety parameters in this study. The PP analysis sets will be analyzed for efficacy parameters in this study.

No imputation of values for missing data is performed except for missing or partial start and end dates for adverse events and concomitant medication will be imputed according to prespecified, conservative imputation rules. Subjects lost to follow-up (or drop out) will be included in statistical analyses to the point of their last evaluation.

The safety endpoint analysis was performed as follows. Safety summaries will include summaries in the form of tables and listings. The frequency (number and percentage) of treatment emergent adverse events will be reported in each treatment group by Medical Dictionary for Regulatory Activities (MedDRA) System Organ Class and Preferred Term. Summaries will also be presented by the severity of the adverse event and by relationship to study drug. Laboratory shift tables containing counts and percentages will be prepared by treatment assignment, laboratory parameter, and time. Summary tables will be prepared for each laboratory parameter. Figures of changes in laboratory parameters over time will be generated. Vital signs, ECGs, and physical exams will be tabulated and summarized.

Additional analyses include summaries of subject demographics, baseline characteristics, compliance, and concurrent treatments. Concomitant medications will be coded according to the World Health Organization (WHO) Drug Dictionary and tabulated.

The analysis of efficacy parameters was performed as follows. The point estimate of the overall response rate will be calculated for the PP analysis set. The corresponding 95% confidence interval also will be derived. The duration of overall response is measured from the time measurement criteria are met for CR or PR (whichever is first recorded) until the first date that recurrent or progressive disease is objectively documented (taking as reference for progressive disease the smallest measurements recorded since the treatment started). Kaplan-Meier methodology will be used to estimate event-free curves and corresponding quantiles (including the median). Progression-free survival is measured from the time of first study drug administration until the first date that recurrent or progressive disease is objectively documented (taking as reference for progressive disease the smallest measurements recorded since the treatment started). Kaplan-Meier methodology will be used to estimate the event-free curves and corresponding quantiles (including the median).

The study scheme is a sequential cohort escalation. Each cohort consists of six subjects. The sample size of the study is 24 to 36 subjects, depending on dose escalation into subsequent cohorts. Cohort 1 (N = 6) consists of Formula (XVIII), 100 mg QD for 28 days. Cohort 2 (N = 6) consists of Formula (XVIII), 175 mg QD for 28 days. Cohort 3 (N = 6) consists of Formula (XVIII), 250 mg QD for 28 days. Cohort 4 (N = 6) consists of Formula (XVIII), 350 mg QD for 28 days. Cohort 5 (N = 6) consists of Formula (XVIII), 450 mg QD for 28 days. Cohort 6 (N = 6) consists of Formula (XVIII), at a dose to be determined QD for 28 days. The dose level for Cohort 6 will be determined based on the safety and efficacy of Cohorts 1 to 5, and will not exceed 900 mg/day. Escalation will end with either the MTD cohort or three levels above full BTK occupancy, whichever is observed first. An additional arm of the study will explore 100 mg BID dosing. Treatment with oral Formula (XVIII) may be continued for greater than 28 days until disease progression or an unacceptable drug-related toxicity occurs.

The inclusion criteria for the study are as follows: (1) men and women ≥ 18 years of age with a confirmed diagnosis of CLL/SLL, which has relapsed after, or been refractory to, ≥ 2 previous treatments for CLL/SLL; however, subjects with 17p deletion are eligible if they have relapsed after, or been refractory to, 1 prior treatment for CLL/SLL; (2) body weight ≥ 60 kg, (3) ECOG performance status of ≤ 2; (4) agreement to use contraception during the study and for 30 days after the last dose of study drug if sexually active and able to bear children; (5) willing and able to participate in all required evaluations and procedures in this study protocol including swallowing capsules without difficulty; or (6) ability to understand the purpose and risks of the study and provide signed and dated informed consent and authorization to use protected health information (in accordance with national and local subject privacy regulations).

The dosage form and strength of Formula (XVIII) used in the clinical study is a hard gelatin capsules prepared using standard pharmaceutical grade excipients (microcrystalline cellulose) and containing 25 mg of Formula (XVIII) each. The color of the capsules is Swedish orange. The route of administration is oral (*per os*, or PO). The dose regimen is once daily or twice daily, as defined by the cohort, on an empty stomach (defined as no food 2 hours before and 30 minutes after dosing).

The baseline characteristics for the patients enrolled in the clinical study are given in Table 12.

**TABLE 12. Relapsed/refractory CLL baseline characteristics.**

| **Characteristic** | | **CLL (N=44)** |
|---|---|---|
| Patient Demographics | | |
| | Age (years), median (range) | 62 (45-84) |
| | Sex, men (%) | 33 (75) |
| | Prior therapies, median (range), n | 3 (1-10) |
| | ≥3 prior therapies, n (%) | 26 (59) |
| Clinical Details | | |
| | ECOG performance status ≥1 (%) | 28 (63) |
| | Rai stage III/IV | 16(36) |
| | Bulky disease ≥ 5 cm, n (%) | 15 (34) |
| | Cytopenia at baseline | 33 (75) |
| Cytogenic Status | | |
| | Chromosome 11q22.3 deletion (Del 11q), n (%) | 18 (41) |
| | Chromosome 17p13.1 (Del 17p), n (%) | 19(34) |
| | IgV_{H} status (unmutated), n (%) | 28 (64) |

The results of the clinical study in relapsed/refractory CLL patients are summarized in Table 13.

**TABLE 13. Activity of Formula (XVIII) in relapsed/refractory CLL. (PR = partial response; PR+L = partial response with lymphocytosis; SD = stable disease; PD = progressive disease.)**

| **n (%)** | **All Cohorts (N=31)** | **100 mg QD** (N=8) | **175 mg QD** (N=8) | **250 mg QD** (N=7) | **100 mg BID** (N=3) | **400 mg QD** (N=5) |
|---|---|---|---|---|---|---|
| **PR** | 22 (71) | 7(88) | 5(63) | 5(71) | 3 (100) | 2(40) |
| **PR+L** | 7(23) | 0(0) | 3 (37) | 2(29) | 0(0) | 2(40) |
| **SD** | 2(6) | 1(12) | 0(0) | 0(0) | 0(0) | 1(20) |
| **PD** | 0(0) | 0(0) | 0(0) | 0(0) | 0(0) | 0(0) |
| Median (range) Cycles | | | | | | |
| | **7.3 (3.0-10.8)** | **10.0 (9.0-10.8)** | **8.6 (3.0-8.8)** | **7.0 (7.0-7.3)** | **5.2 (4.7-5.5)** | **5.0 (4.8-5.5)** |

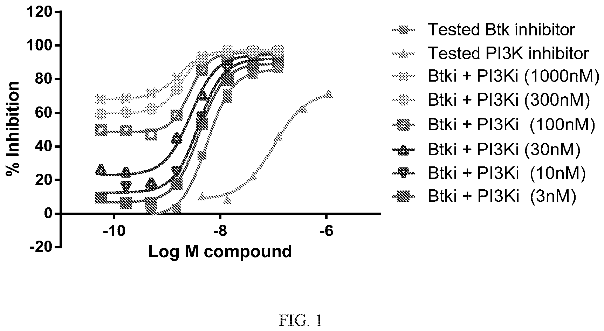

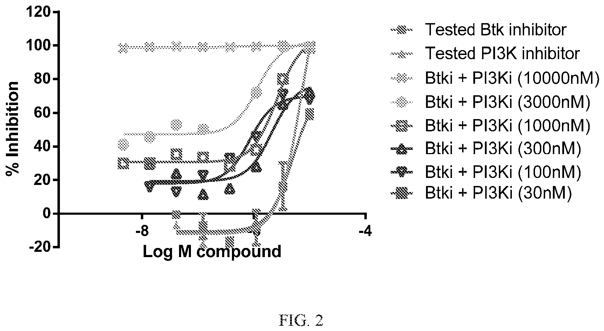

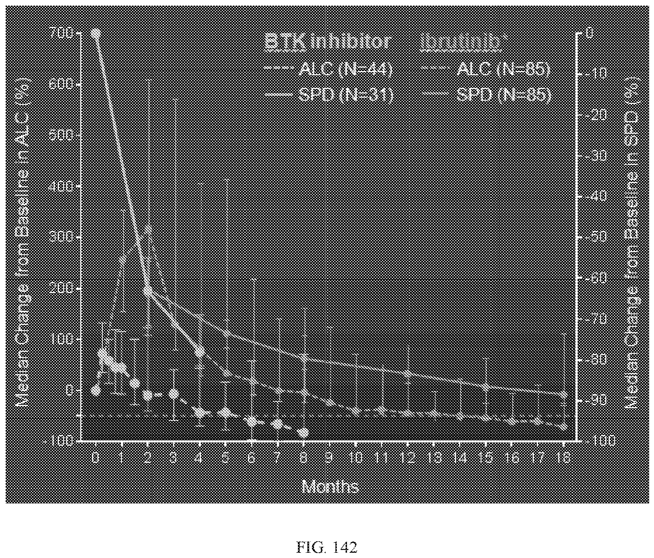

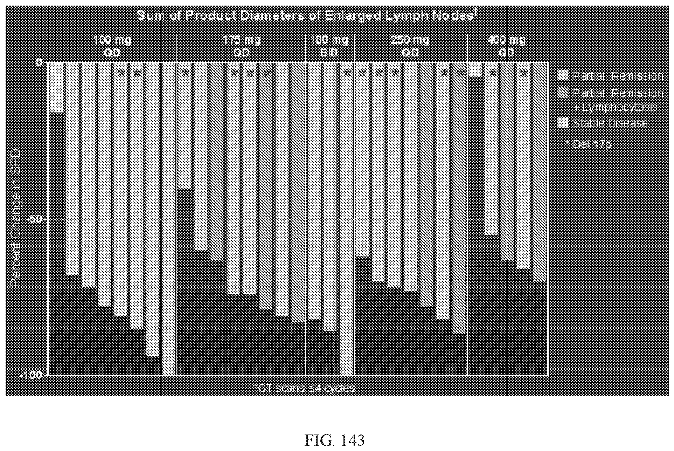

FIG. 142 shows the median % change in ALC and SPD from baseline in the clinical study of Formula (XVIII), plotted in comparison to the results reported for ibrutinib in Figure 1A of Byrd, et al., N. Engl. J. Med. 2013, 369, 32-42. The results show that Formula (XVIII) leads to a more rapid patient response in CLL than corresponding treatment with ibrutinib. This effect is illustrated, for example, by the median % change in SPD, which achieved the same status in the present study at 7 months of treatment with Formula (XVIII) as compared to 18 months for ibrutinib. The % change in SPD observed in the different cohorts (*i.e.* by dose and dosing regimen) is shown in FIG. 143, and in all cases shows significant responses.

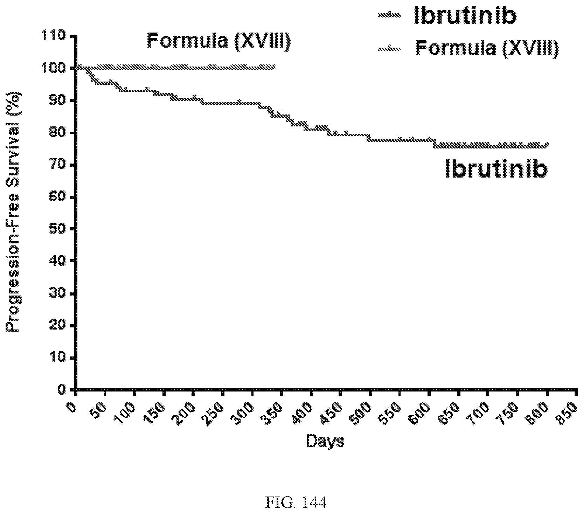

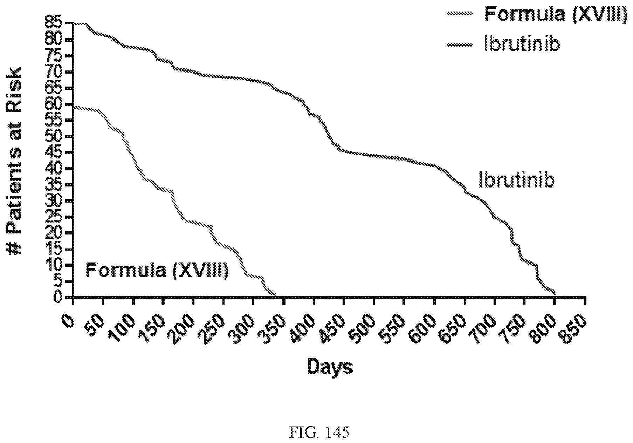

A Kaplan-Meier curve showing PFS from the clinical CLL study of Formula (XVIII) is shown in FIG. 144. A comparison of survival curves was performed using the Log-Rank (Mantle-Cox) test, with a p-value of 0.0206 indicating that the survival curves are different. The number of patients at risk is shown in FIG. 145. Both FIG. 144 and FIG. 145 show the results for Formula (XVIII) in comparison to the results reported for ibrutinib in Byrd, et al., N. Engl. J. Med. 2013, 369, 32-42. An improvement in survival and a reduction in risk are observed in CLL patients treated with Formula (XVIII) in comparison to patients treated with ibrutinib.

Based on the data and comparisons shown in FIG. 142 to FIG. 145, the CLL study with Formula (XVIII) showed that the efficacy of Formula (XVIII) was surprisingly superior to that of ibrutinib.

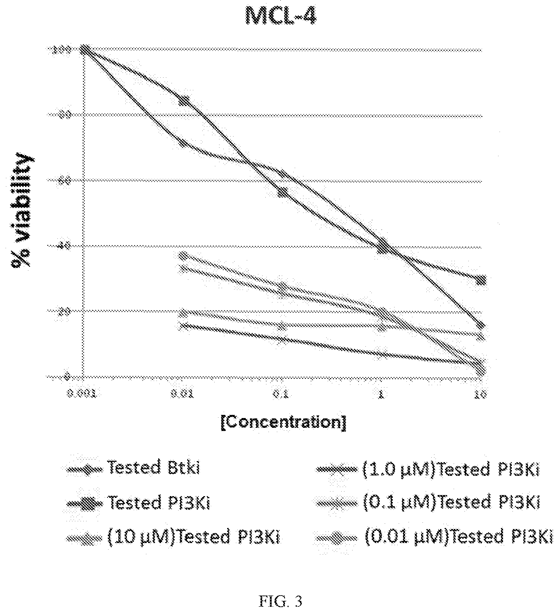

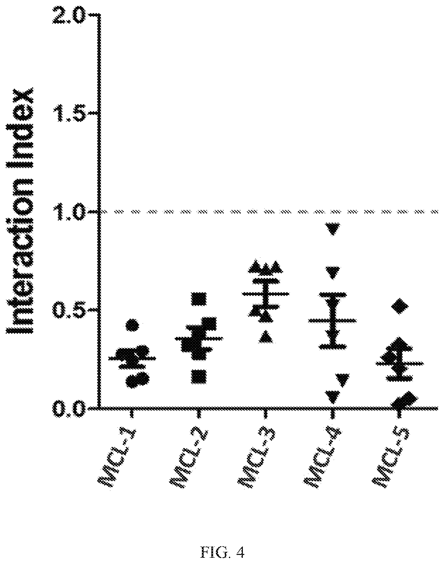

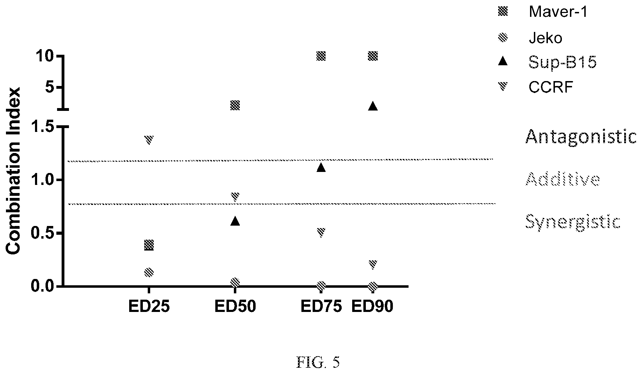

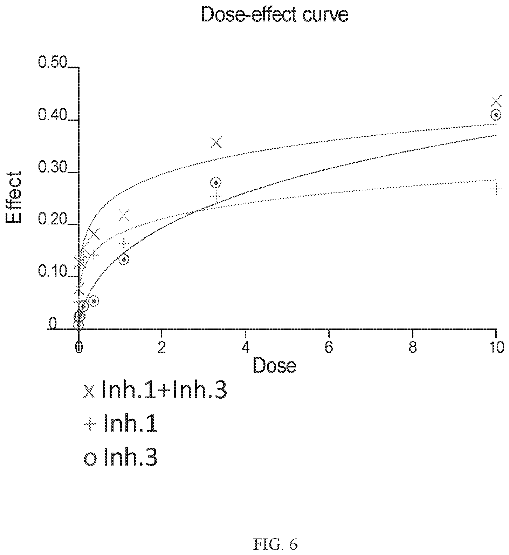

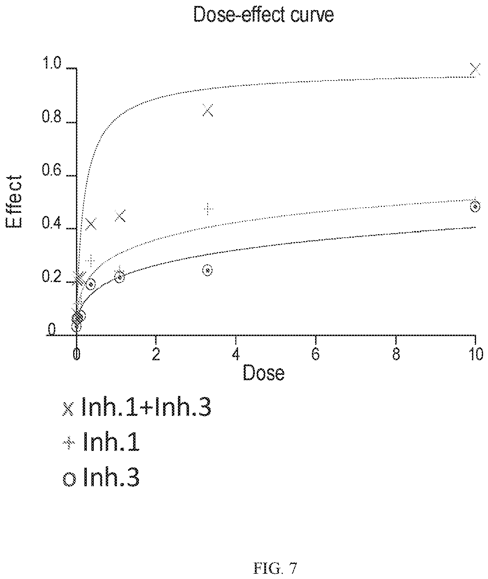

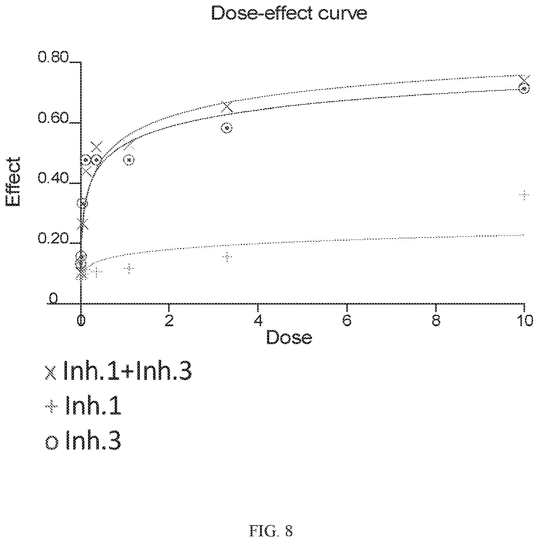

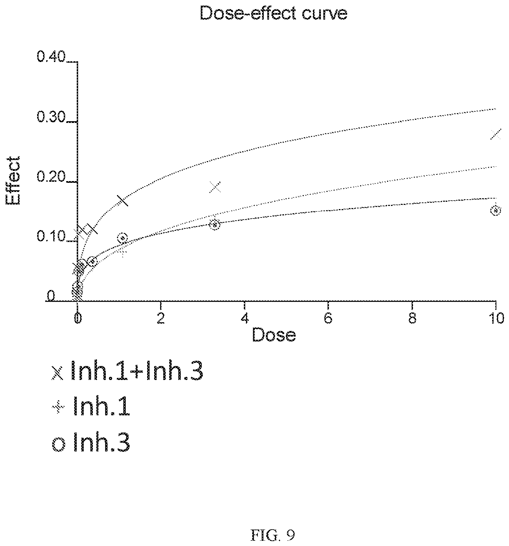

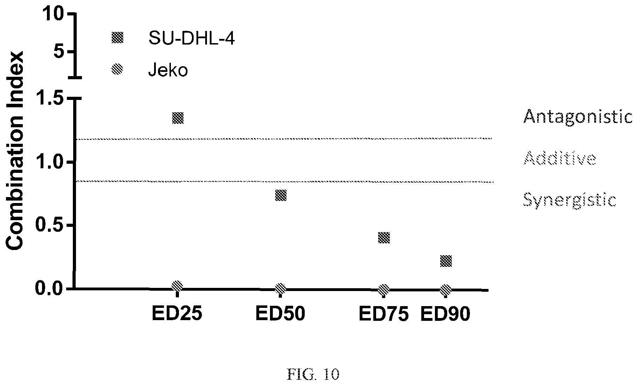

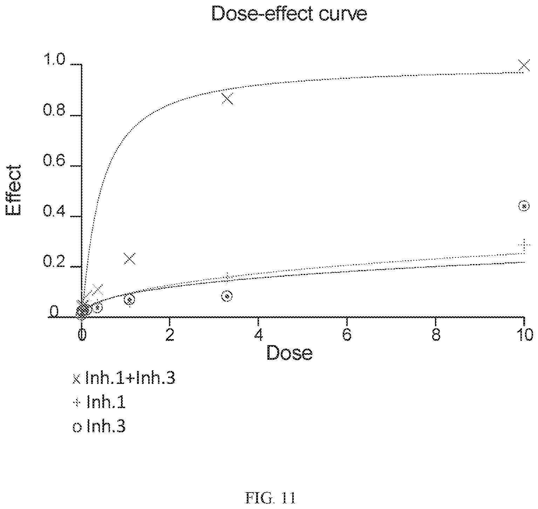

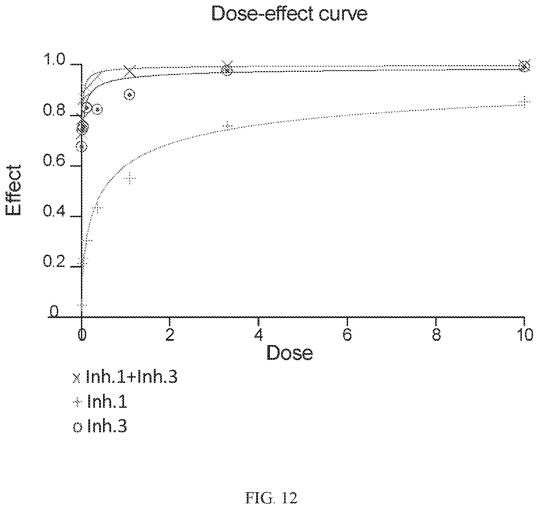

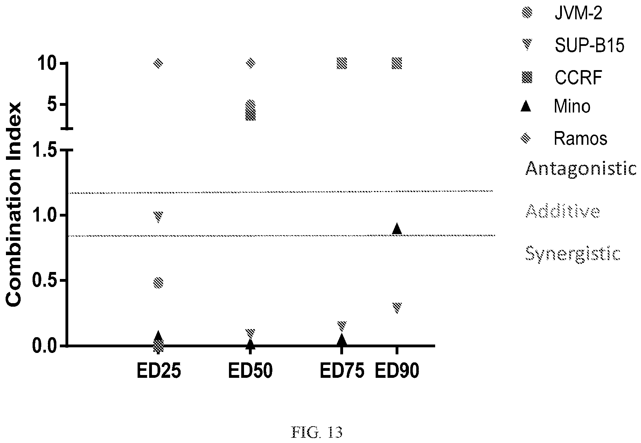

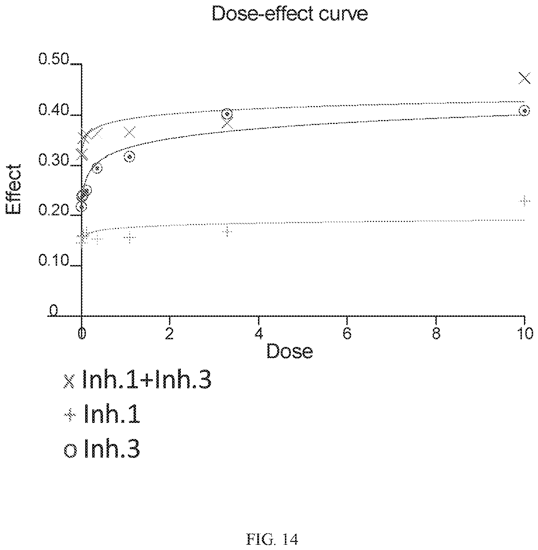

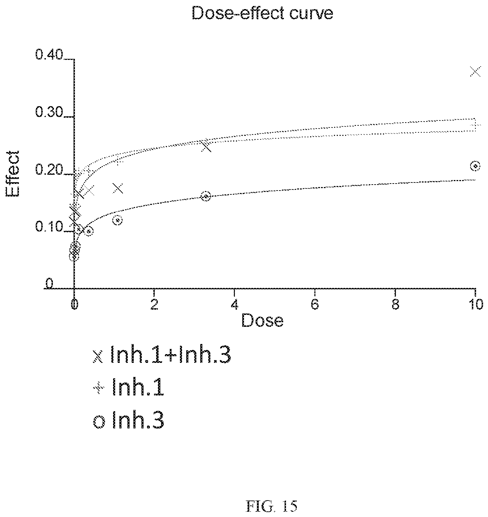

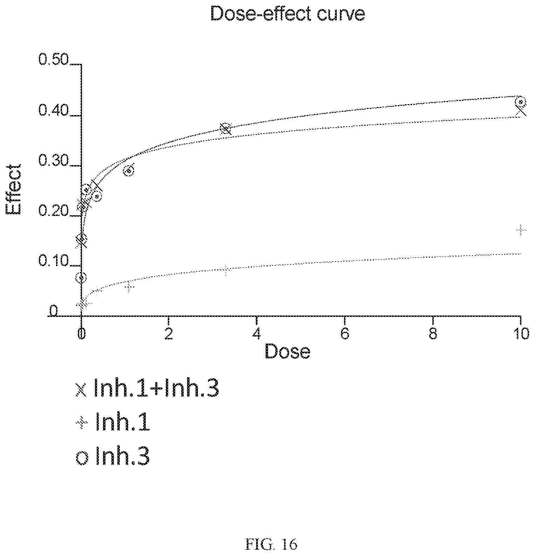

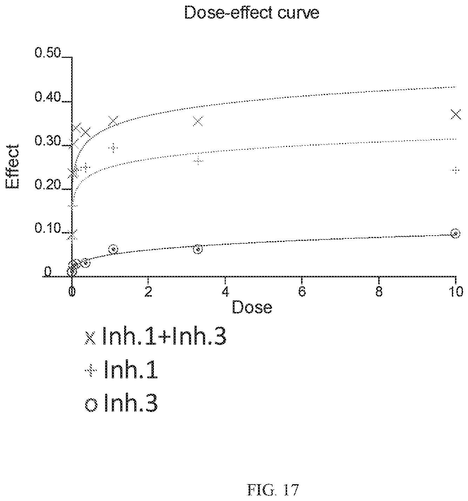

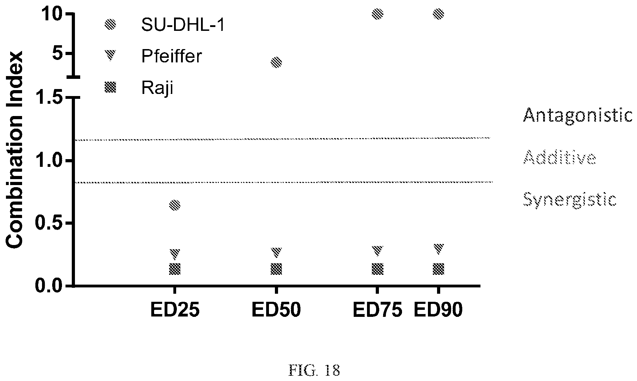

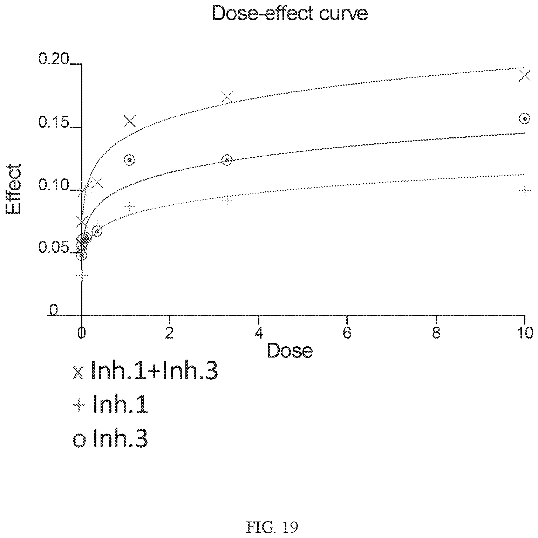

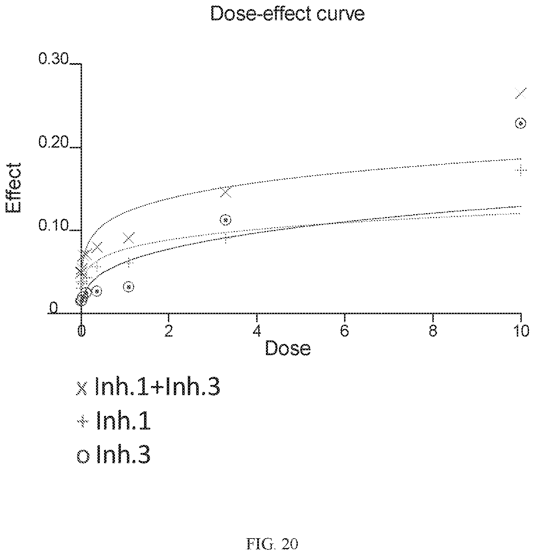

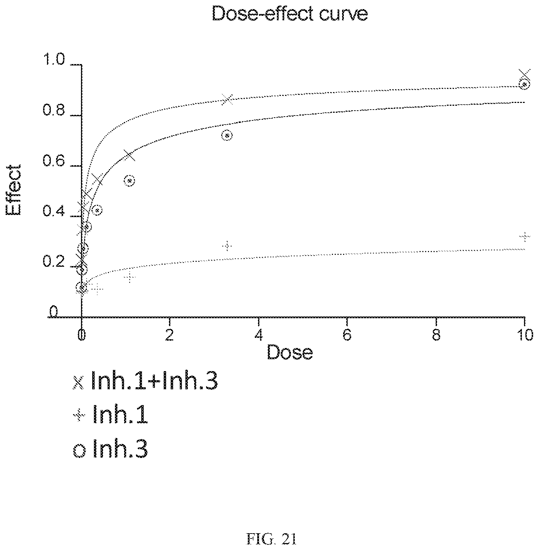

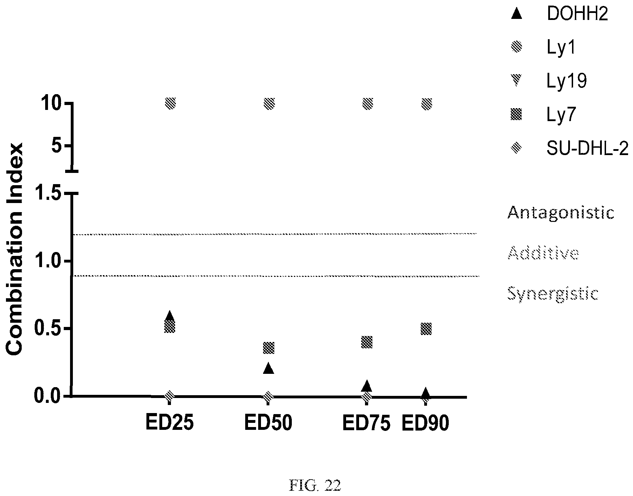

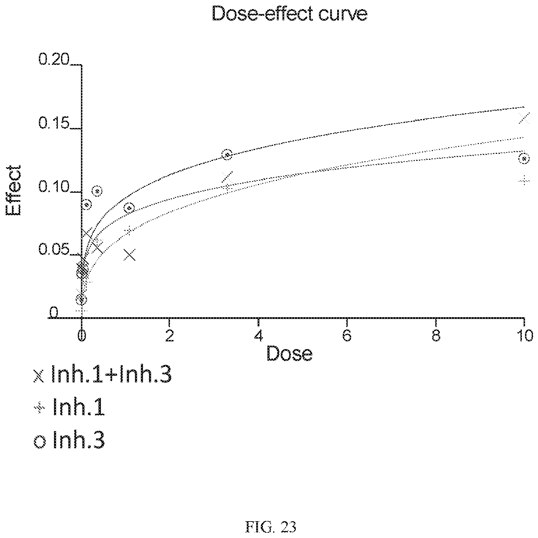

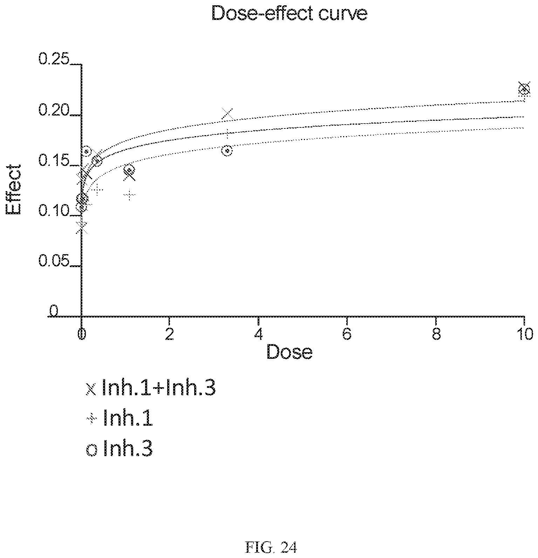

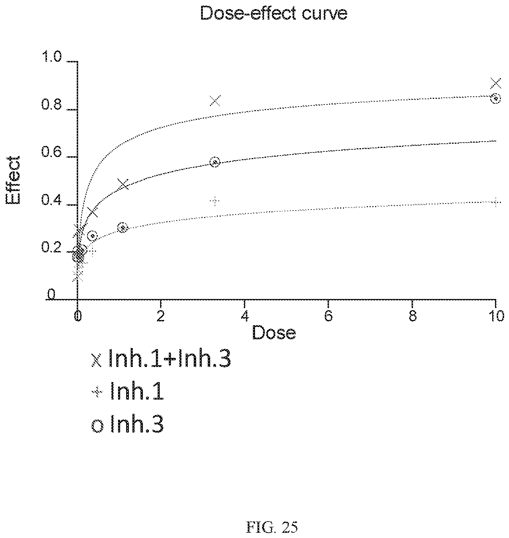

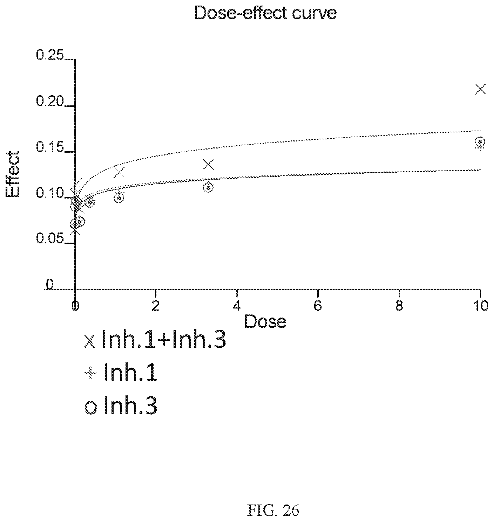

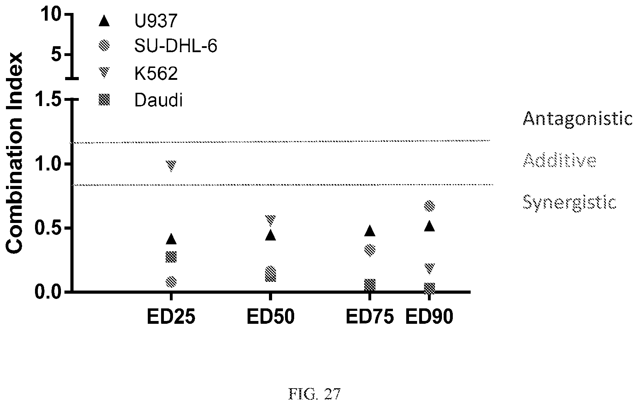

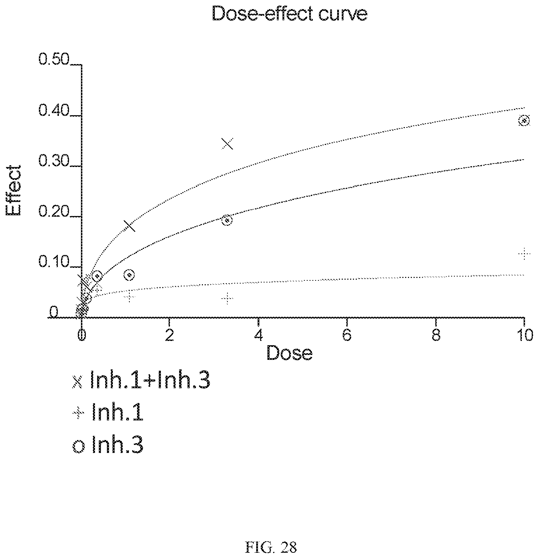

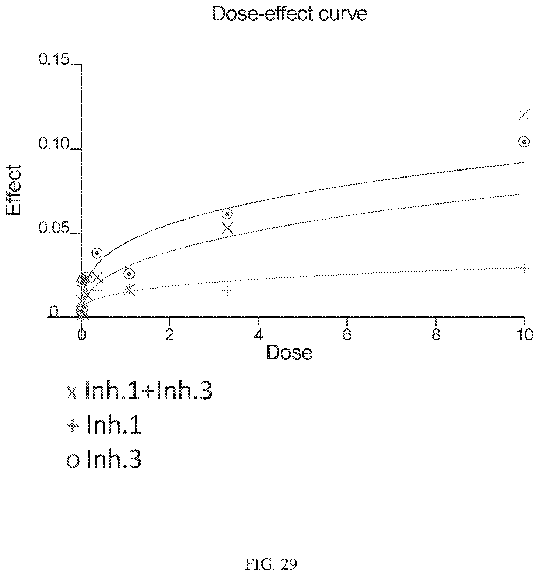

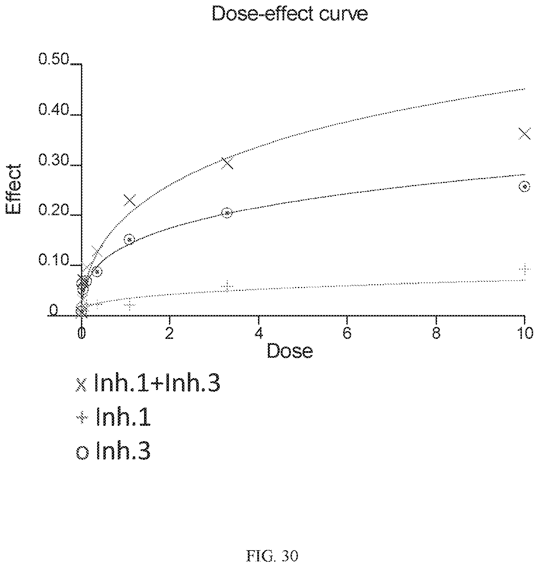

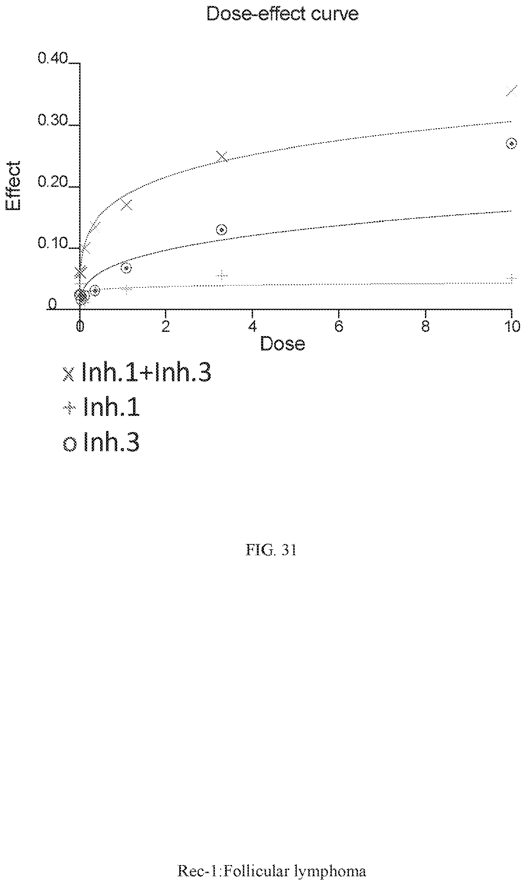

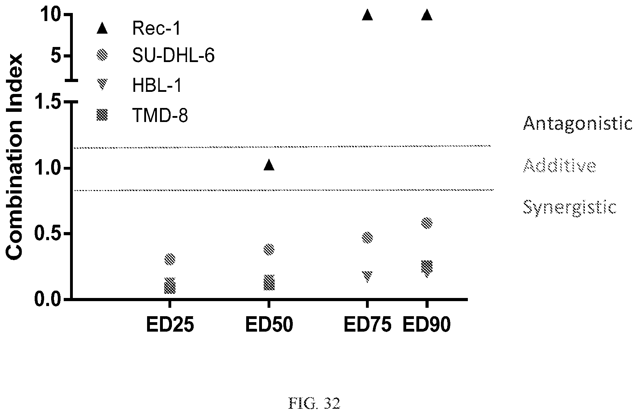

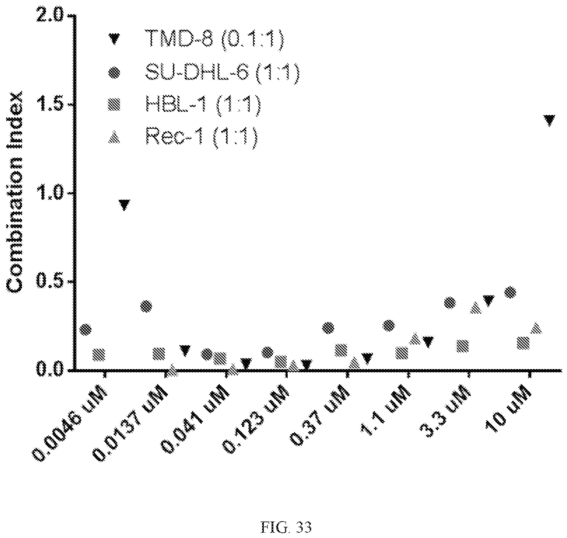

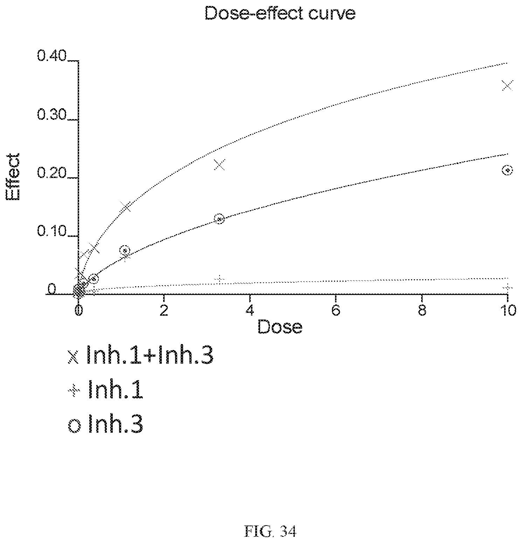

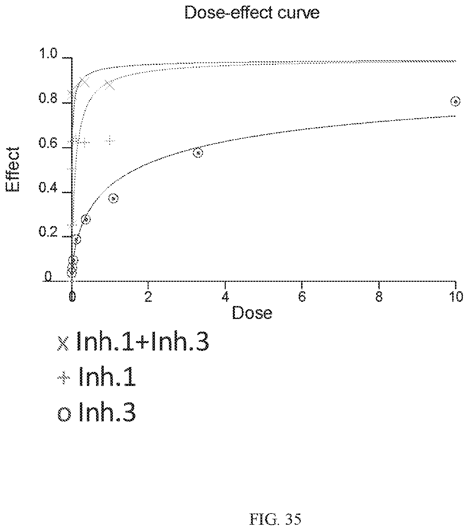

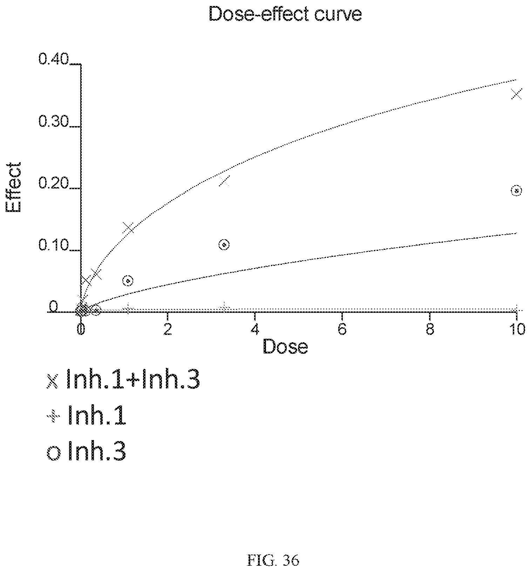

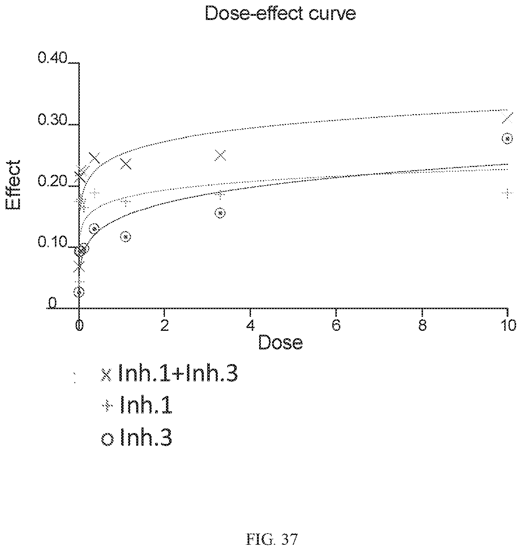

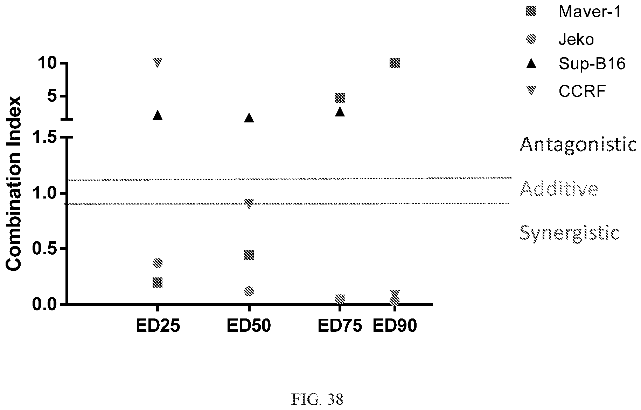

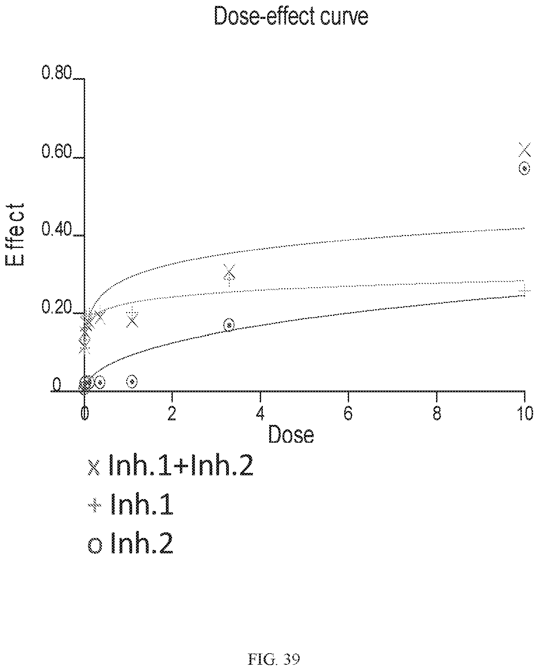

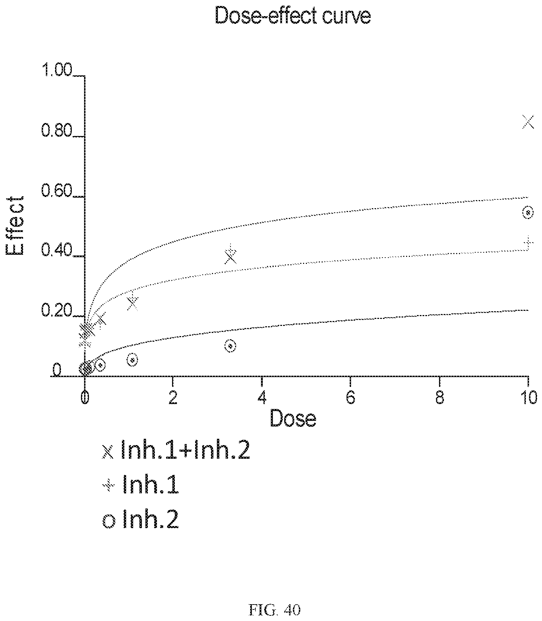

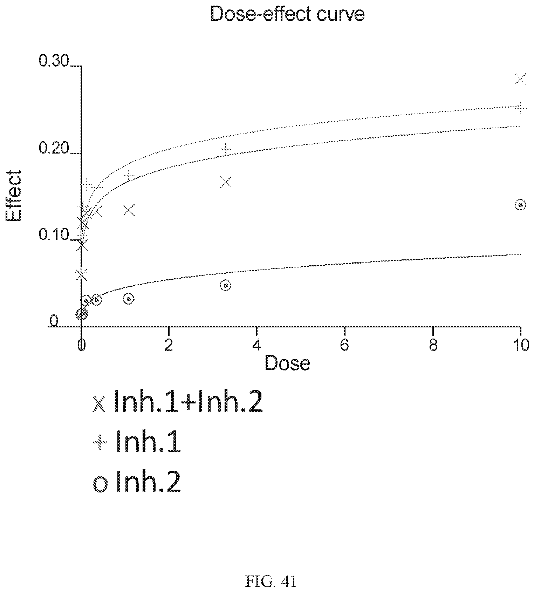

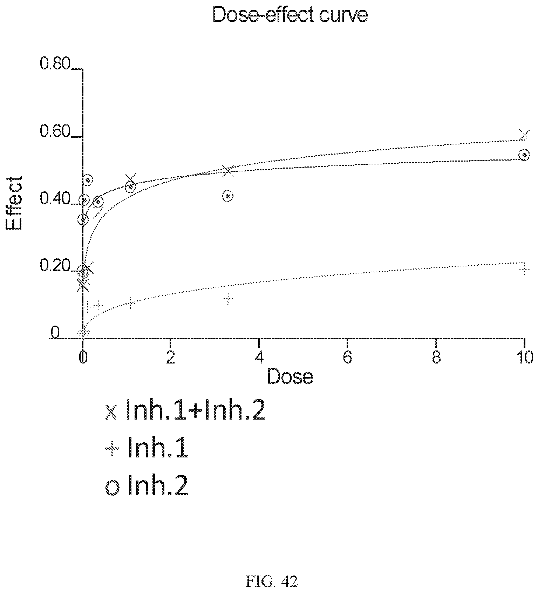

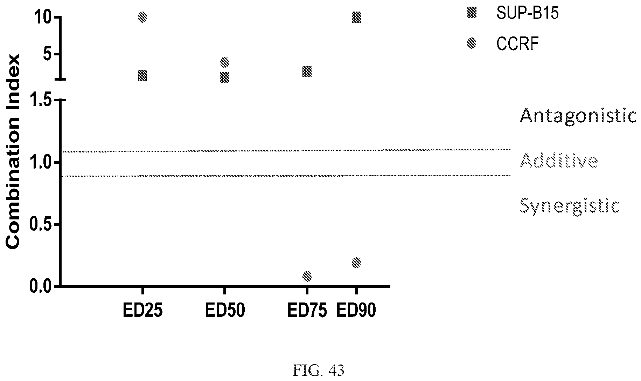

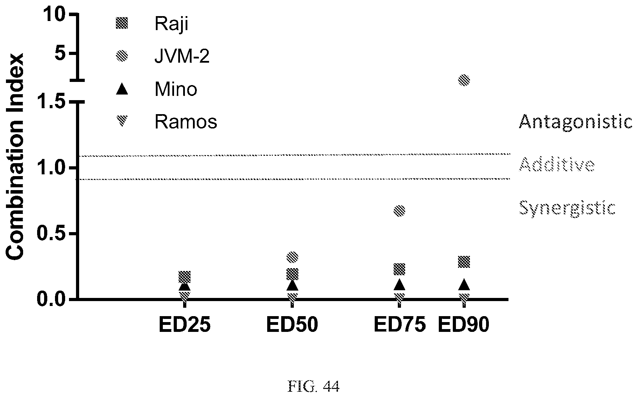

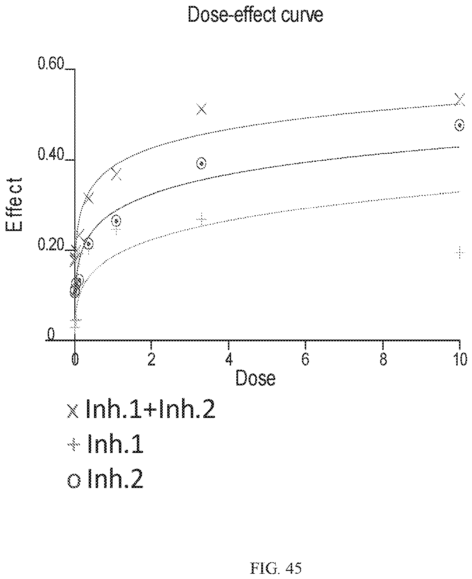

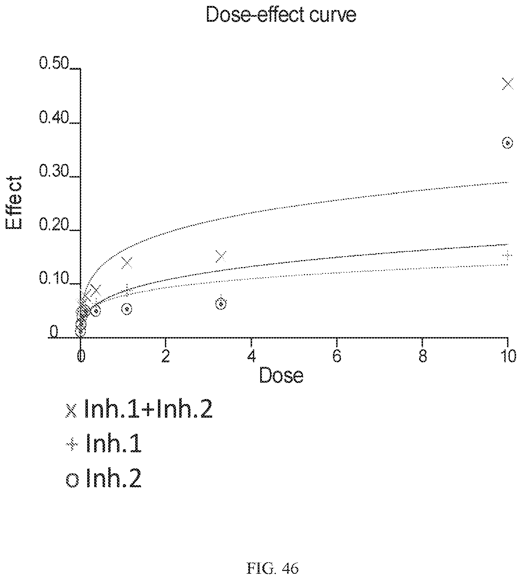

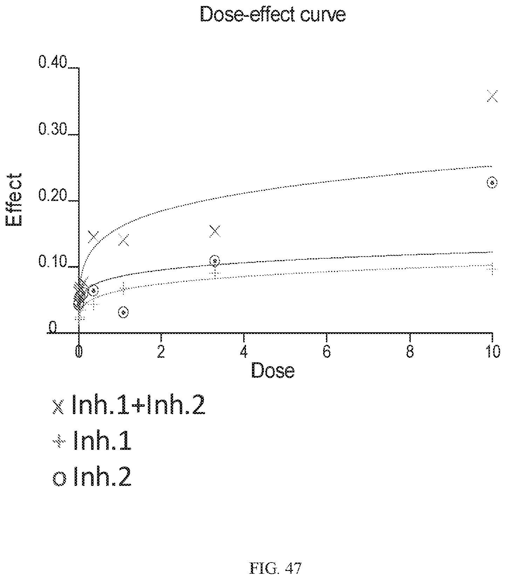

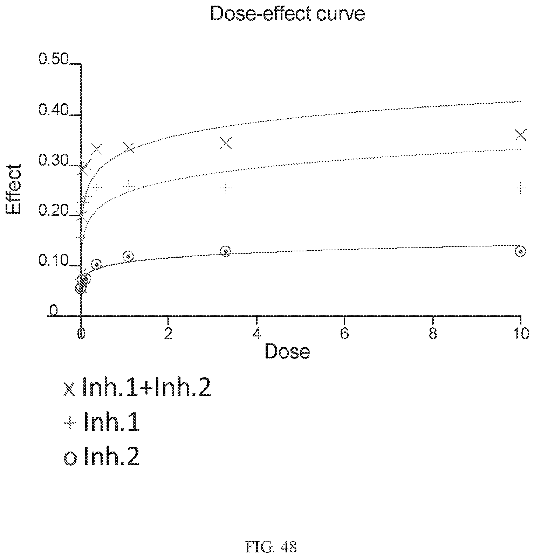

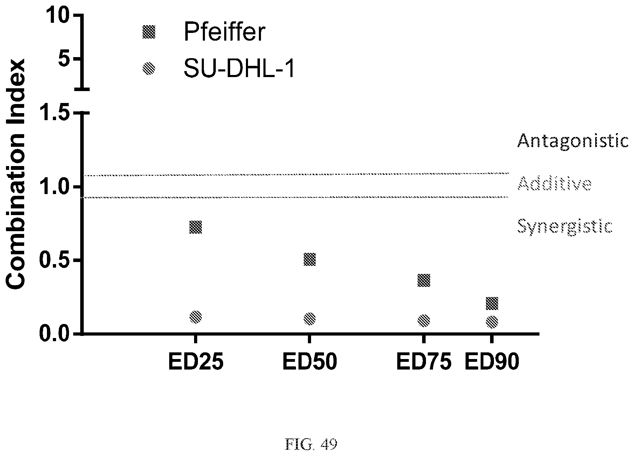

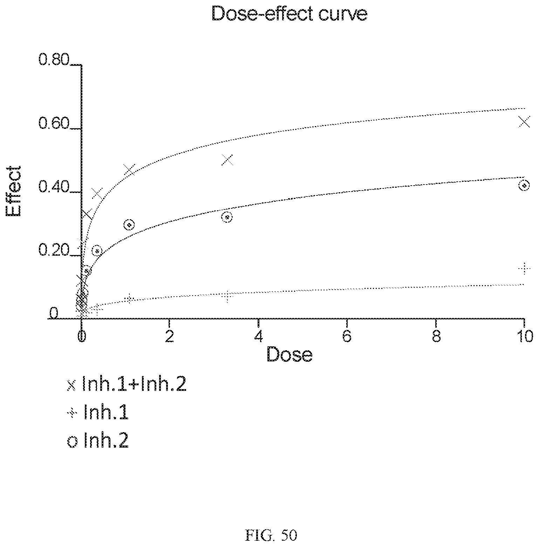

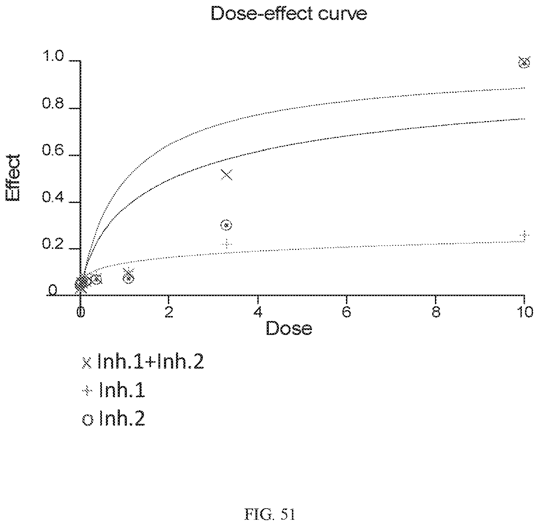

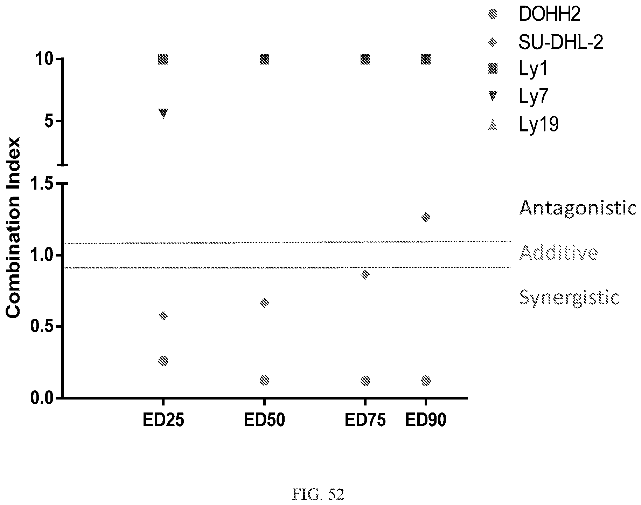

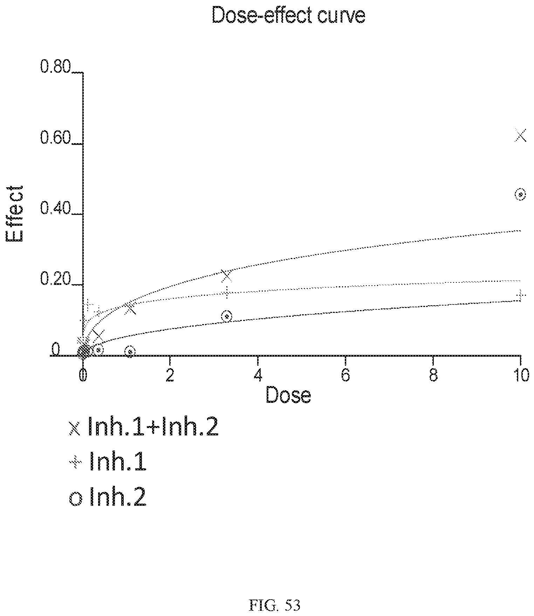

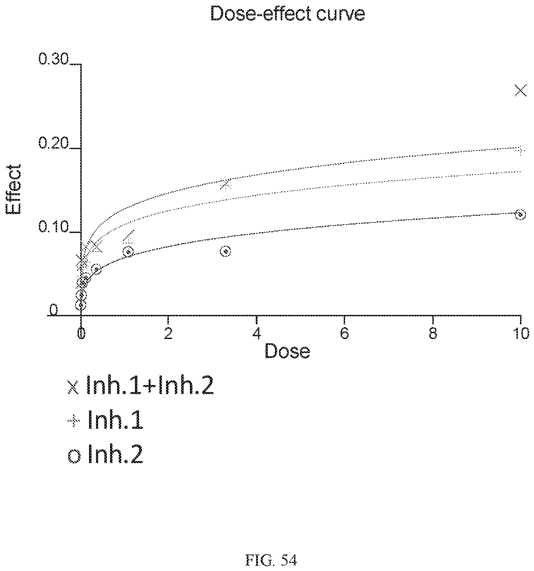

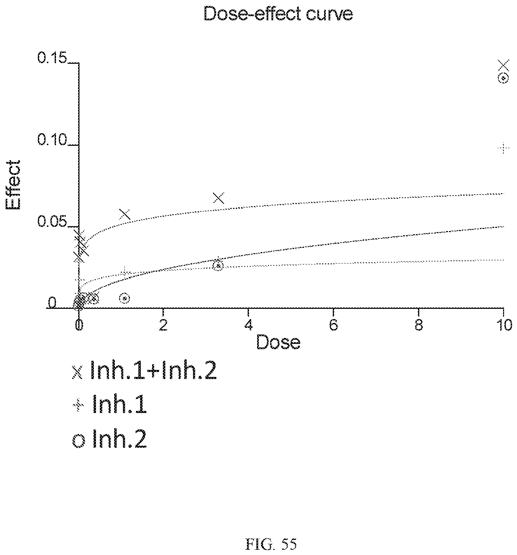

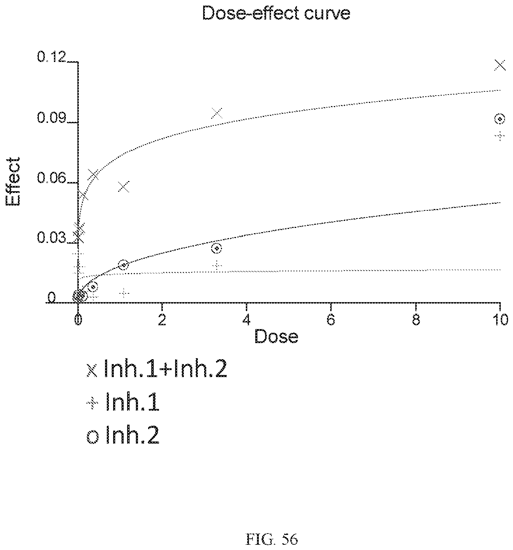

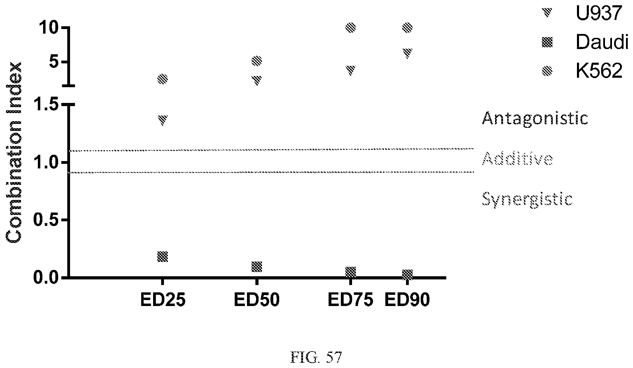

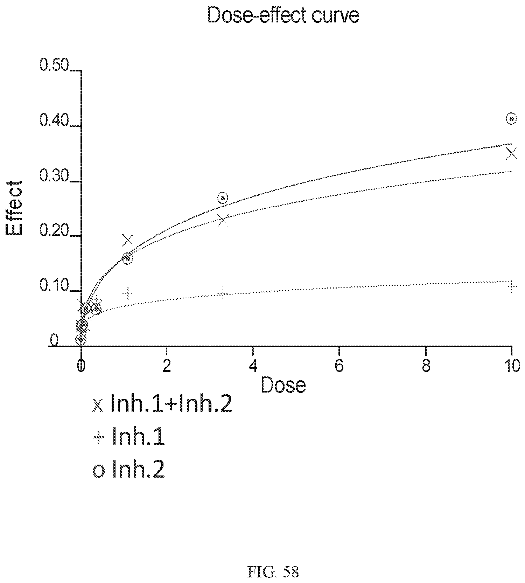

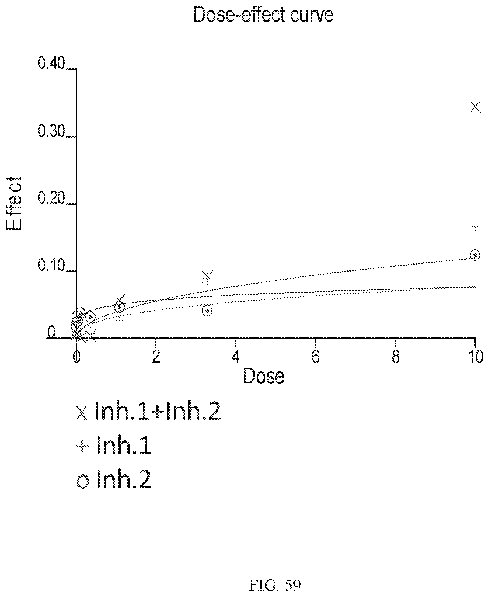

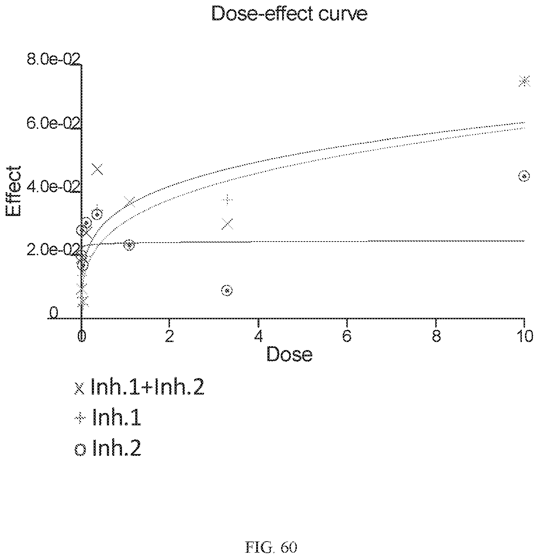

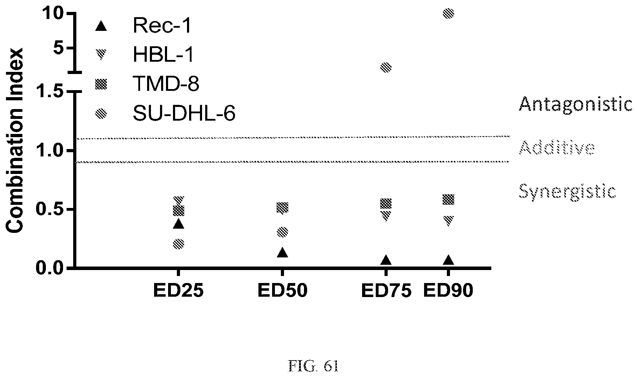

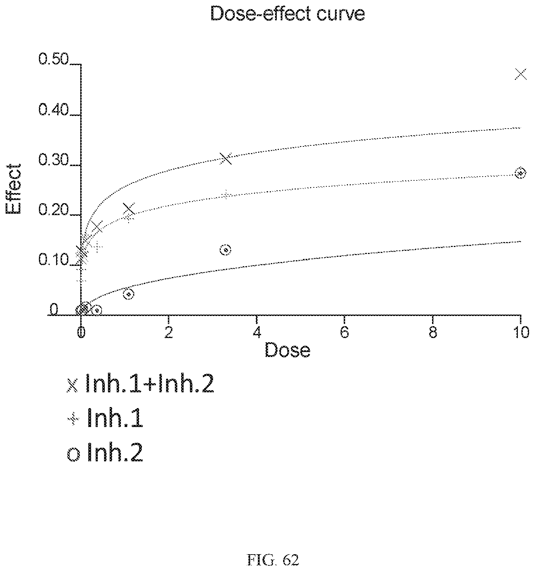

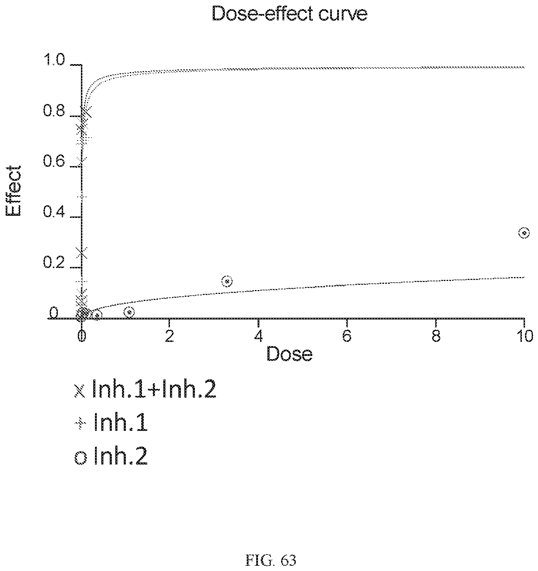

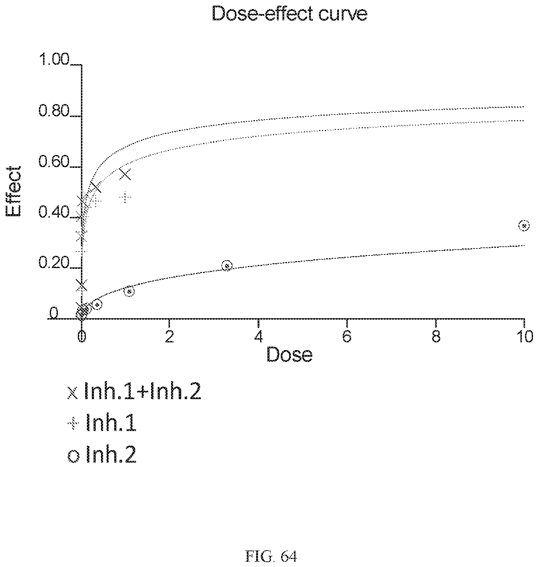

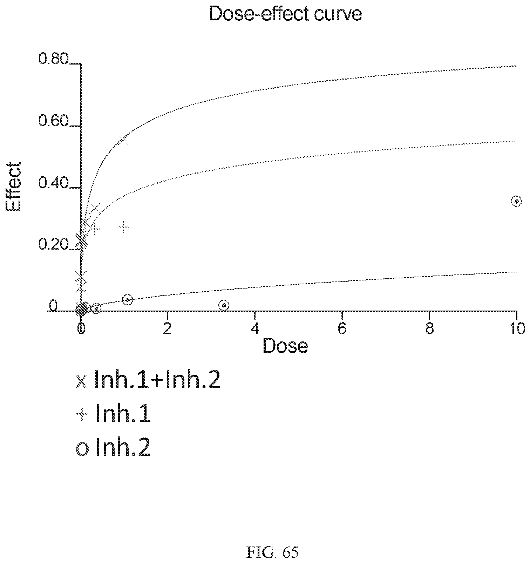

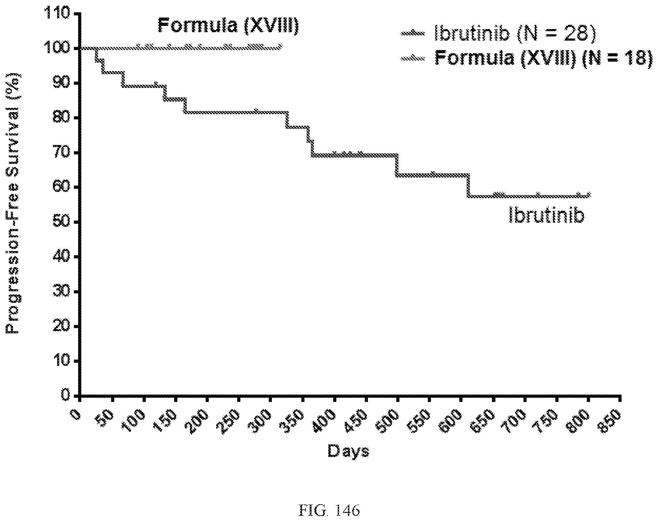

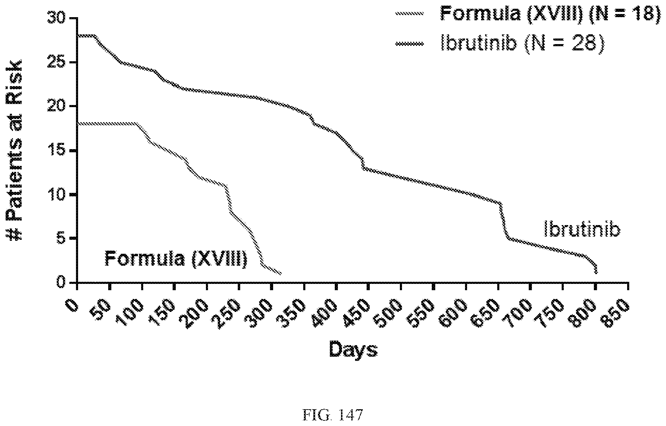

In the literature study of ibrutinib, increased disease progression was associated with patients with high-risk cytogenetic lesions (17p13.1 deletion or 1 1q22.3 deletion), as shown in Figure 3A in Byrd, et al., N. Engl. J. Med. 2013, 369, 32-42, which shows ibrutinib PFS including PFS broken down by genetic abnormality. The 17p and 11q deletions are validated high-risk characteristics of CLL, and the 17p deletion is the highest risk. In FIG. 146, the PFS is shown for Formula (XVIII) in patients with the 17p deletion in comparison to the results obtained for ibrutinib in Byrd, et al., N. Engl. J. Med. 2013, 369, 32-42. A p-value of 0.0696 was obtained. In FIG. 147, the number of patients at risk with the 17p deletion is compared. To date, no 17p patients have progressed on Formula (XVIII).

The adverse events observed in the clinical study in relapsed/refractory CLL are given in Table 14. No DLTs were observed. The MTD was not reached. No treatment-related serious adverse events (SAEs) were observed. No prophylactic antivirals or antibiotics were needed.

**TABLE 14. Treatment-related adverse events reported in the clinical study of Formula (XVIII) in relapsed/refractory CLL. (Reported in ≥ 5% of patients.)**

| **Adverse Events (Treatment-Related), n (%)** | **Grade** | **All (N=44)** |
|---|---|---|
| Headache | 1/2 | 7(16) |
| Increased tendency to bruise | 1 | 6(14) |
| Diarrhea | 1 | 4(9) |
| Petechiae | 1 | 3 (7) |

The clinical study of Formula (XVIII) thus showed other unexpectedly superior results compared to ibrutinib therapy. A lack of lymphocytosis was observed in the study. Furthermore, only grade 1 AEs were observed, and these AEs were attributable to the high BTK selectivity of Formula (XVIII).

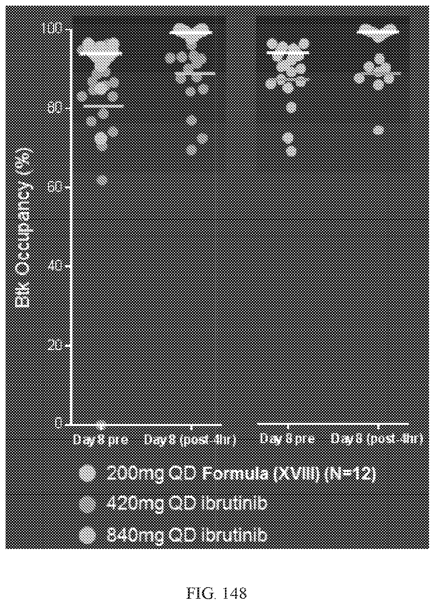

BTK target occupancy was measured for relapsed/refractory CLL patients with the results shown in FIG. 148. For 200 mg QD dosing of the BTK inhibitor of Formula (XVIII), approximately 94% - 99% BTK occupancy was observed, with superior 24 hour coverage and less inter-patient variability also observed. For 420 mg and 840 mg QD of the BTK inhibitor ibrutinib, 80% - 90% BTK occupancy was observed, with more inter-patient variability and capped occupancy. These results indicate that the BTK inhibitor of Formula (XVIII) achieves superior BTK occupancy in CLL patients than ibrutinib.

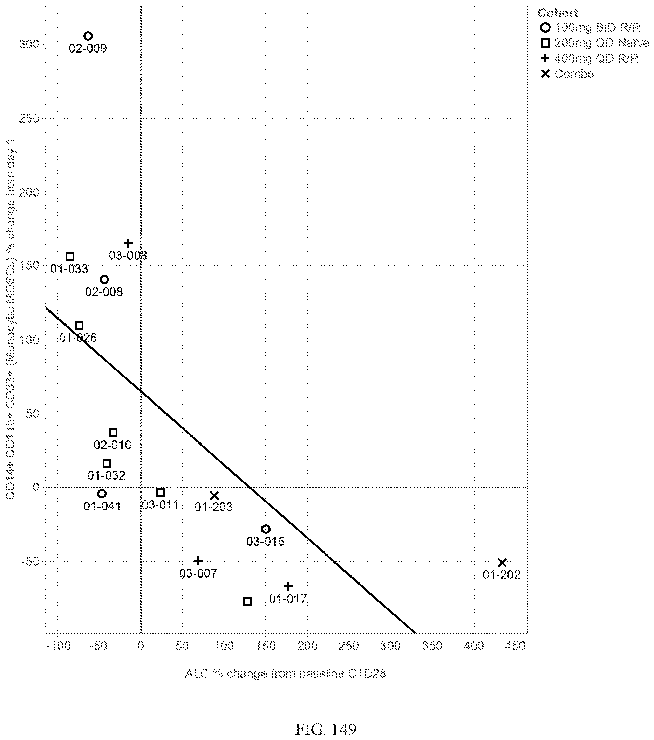

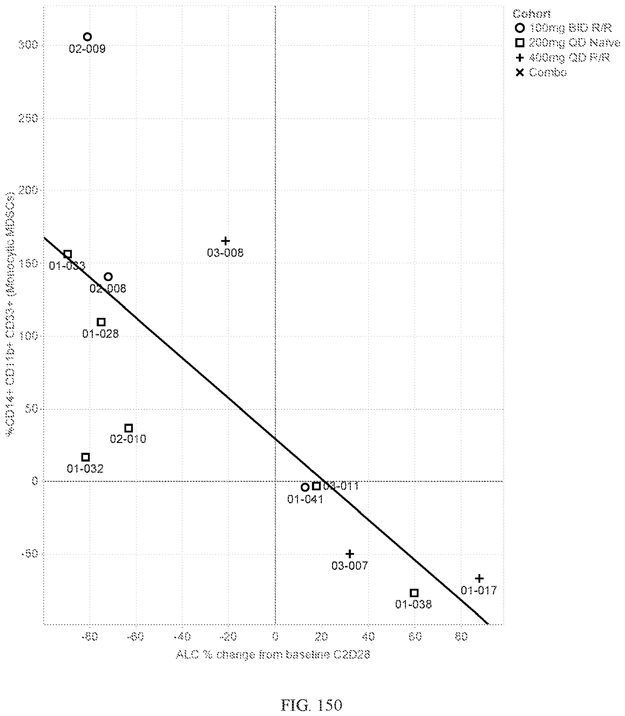

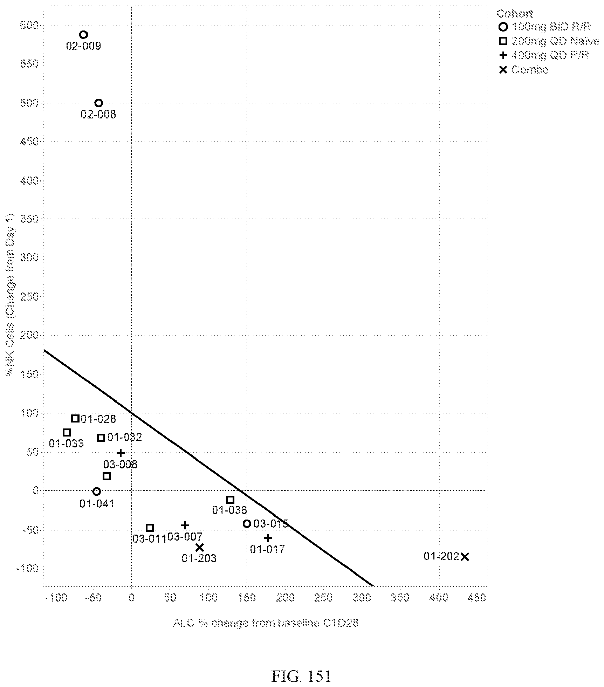

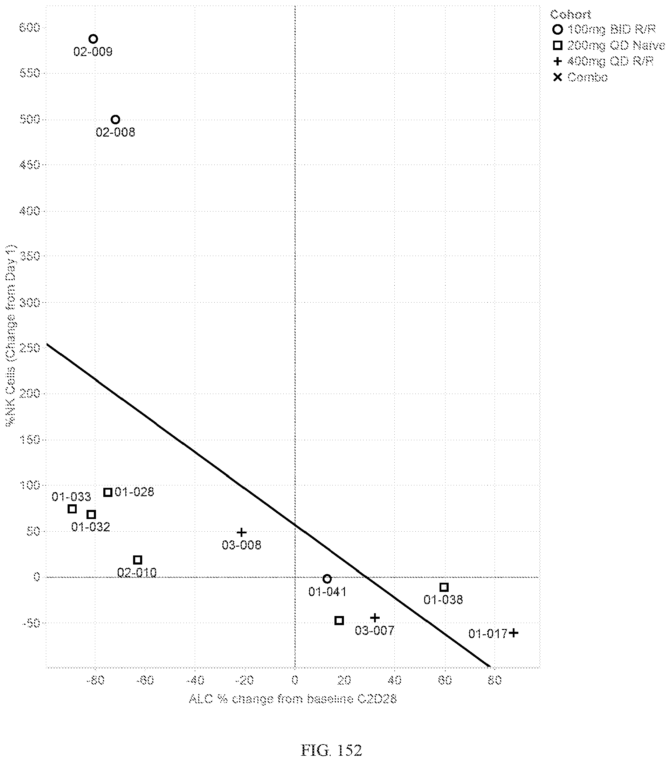

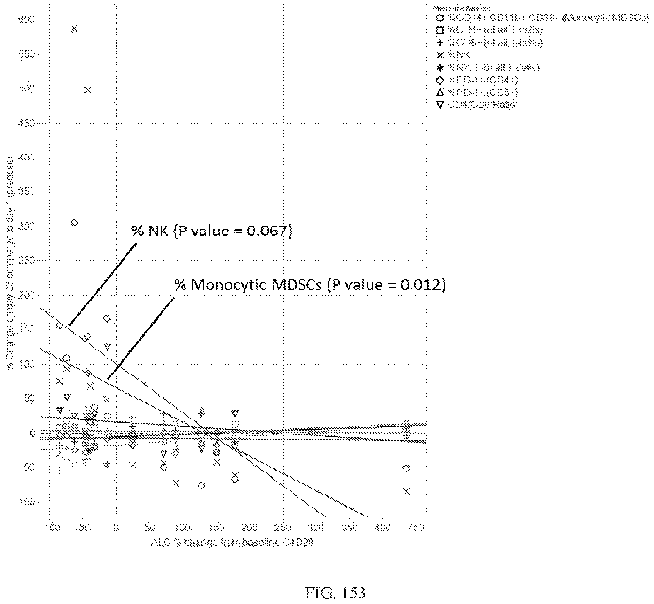

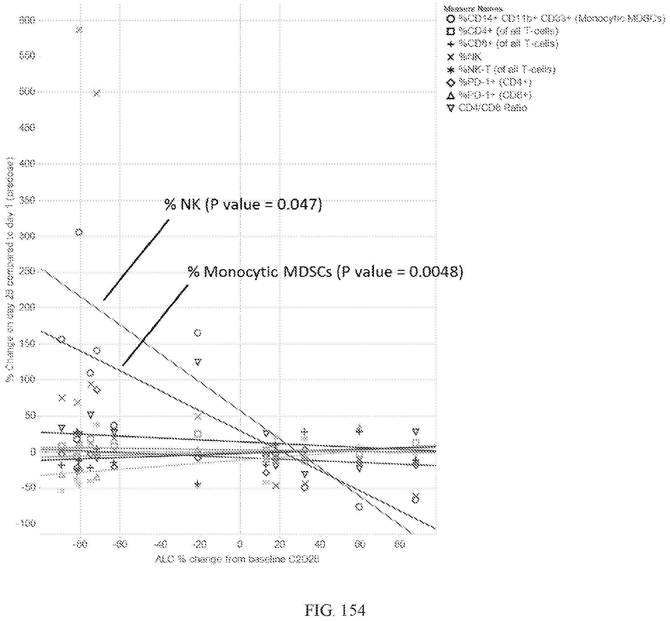

The effects of Formula (XVIII) on cell subset percentages were also evaluated using flow cytometry analysis of peripheral blood, with the results shown in FIG. 149, FIG. 150, FIG. 151, FIG. 152, FIG. 153, and FIG. 154. PBMC samples from CLL patient samples drawn prior to (predose) and after 28 days of dosing with Formula (XVIII) were compared for potential changes in cell subsets. PBMCs were stained with monoclonal antibodies conjugated to fluorescent tags (flourochromes) to identify cell subsets via flow cytometry. Non-viable cells were excluded from the analysis using the dye 7-aminoactinomycin D (7-AAD). To produce the metric of percent change, the following steps were taken. First, each cell subset was defined by hierarchical flow cytometry gating. Then, the change in frequency (between day 1 and day 28) was calculated for each cell subset. MDSC subsets were measured as a % of all myeloid cells. T cell subsets were measured as a % of all CD3⁺ cells, and NK cells were measured as a % of all live CD45⁺ cells. In FIG. 149 and FIG. 150, the results show the % change in MDSC (monocytic) level over 28 days versus % ALC change at cycle 1 day 28 (C1D28) and at cycle 2 day 28 (C2D28). A cycle is 28 days. A trend is observed wherein patients with decreasing ALC % had increasing MDSC (monocytic) %. This may include patients who had quickly resolving lymphocytosis and those with no initial lymphocytosis. This provides evidence that treatment with Formula (XVIII) mobilizes MDSCs and thus affects the CLL tumor microenvironment in marrow and lymph nodes, which is an unexpected indication of superior efficacy. In FIG. 151 and FIG. 152, the results show the % change in NK cell level over 28 days versus % ALC change, measured at C1D28 or C2D28, and similar trends are observed wherein patients with decreasing ALC % had increasing NK cell %. This may include patients who had quickly resolving lymphocytosis and those having no initial lymphocytosis. The effects in FIG. 149 to FIG. 152 are observed in multiple cohorts, at doses including 100 mg BID, 200 mg QD, and 400 mg QD. In FIG. 153 and FIG. 154, the effects on NK cells and MDSC cells are compared to a number of other markers versus % change in ALC at C1D28 and C2D28. These other markers include CD4+ T cells, CD8+ T cells, CD4+/CD8+ T cell ratio, NK-T cells, PD-1+ CD4+ T cells, and PD-1+ CD8+ T cells. The effects on NK cells and MDSC cells are observed to be much more pronounced than on any of these other markers.

These results suggest that after Formula (XVIII) administration, the CLL microenvironment undergoes a change wherein NK cells and monocytic MDSC subsets increase in frequency in the peripheral blood in patients with falling ALC counts, an important clinical parameter in CLL. The NK cell increase may reflect an overall increase in cytolytic activity against B-CLL resulting in the ALC % to drop. The increase in MDSC % in the blood may be due to a movement of these cells out of the lymph nodes, spleen, and bone marrow, which are all possible sites of CLL proliferation. Fewer MDSCs at the CLL proliferation centers would likely result in a reduced immunosuppressive microenvironment leading to an increase in cell-mediated immunity against the tumor, decreased tumor proliferation, and eventually lower ALC% in the circulation.

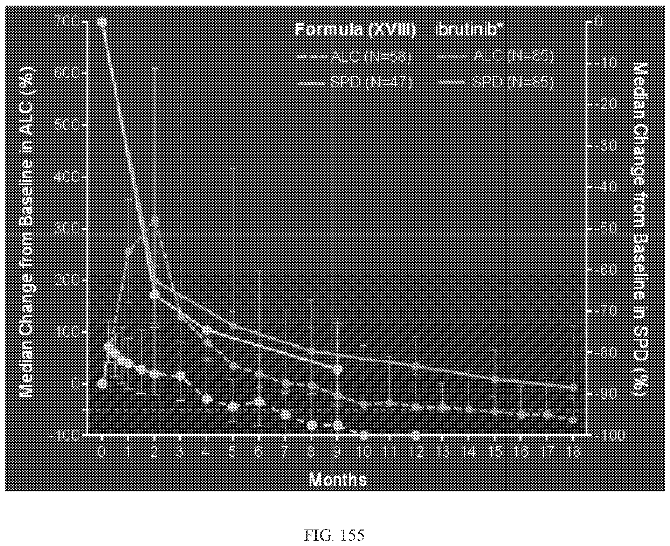

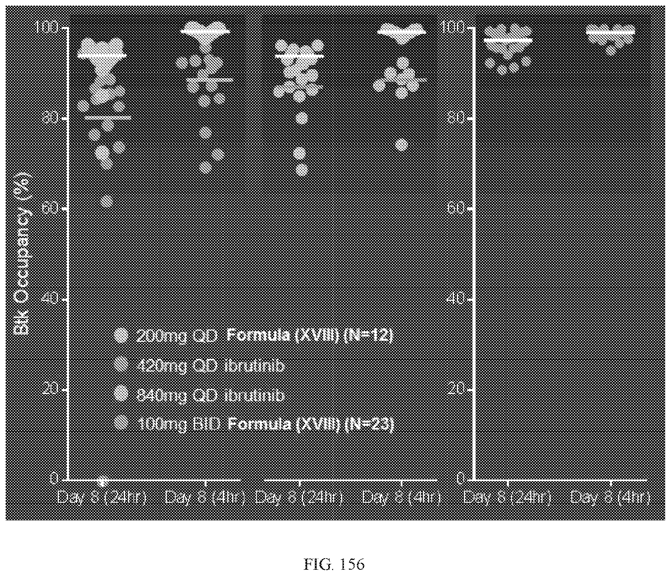

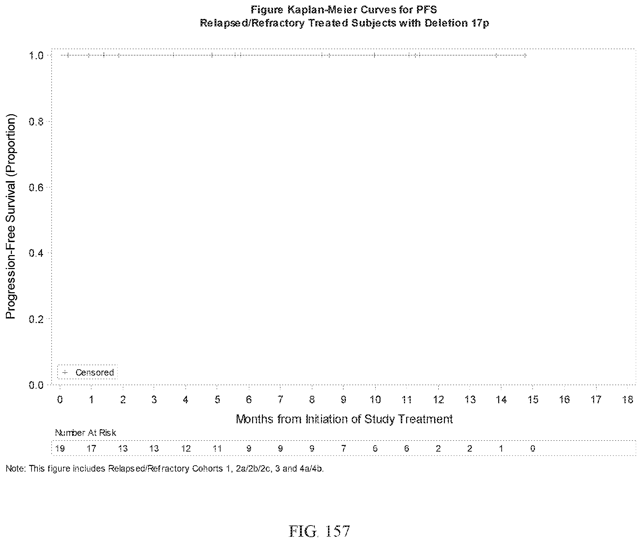

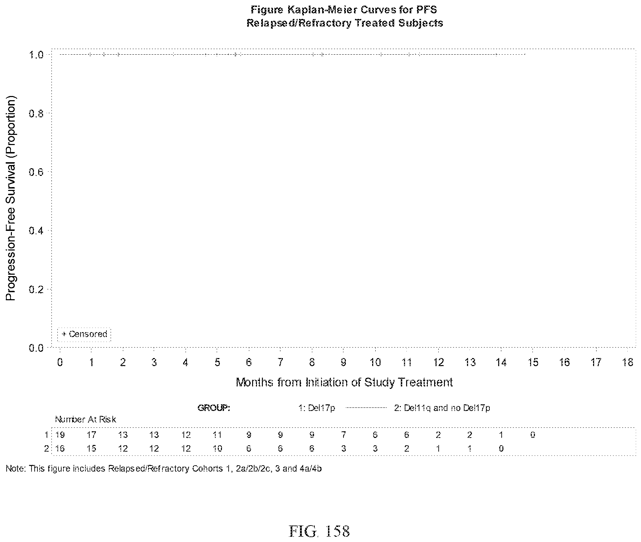

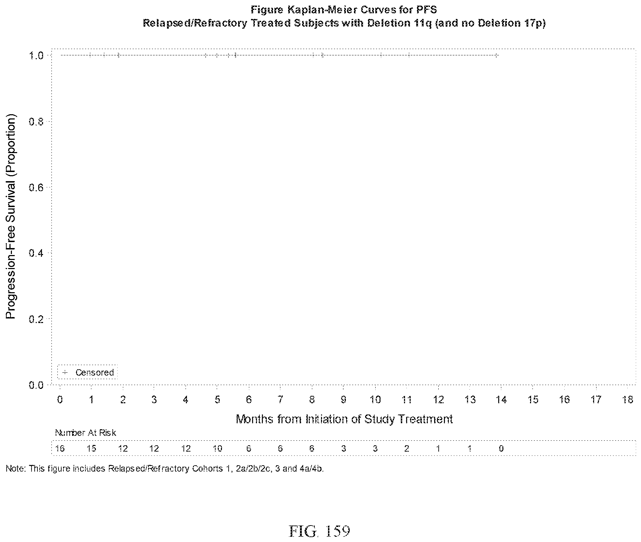

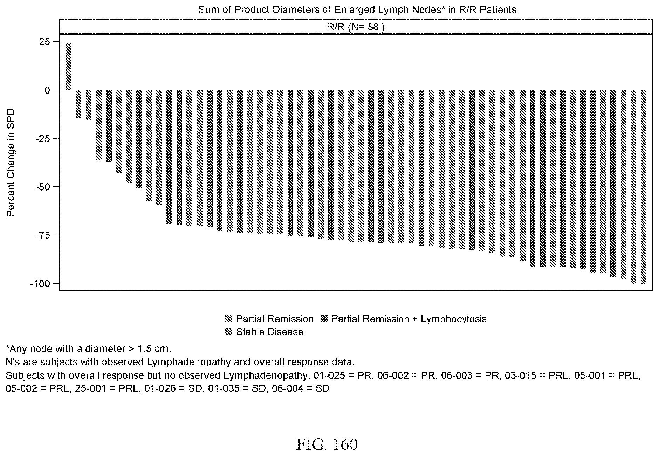

Updated clinical results from the CLL study are shown in FIG. 155 to FIG. 160. FIG. 155 shows an update of the data presented in FIG. 142. FIG. 156 shows an update of the data presented in FIG. 142, and includes BID dosing results. Formula (XVIII) 200 mg QD dosing resulted in 94% - 99% BTK occupancy, 24 hour coverage, and less inter-patient variability. Ibrutinib 420 mg and 840 mg QD dosing resulted in 80% - 90% BTK occupancy, more inter-patient variability, and capped occupancy. Formula (XVIII) 100 mg BID dosing resulted in 97% - 99% BTK occupancy, complete BTK coverage, and less inter-patient variability. The PFS for patients with 11p deletions and 17q deletions are illustrated in FIG. 157, FIG. 158, and FIG. 159. Updated SPD results are illustrated in FIG. 160.

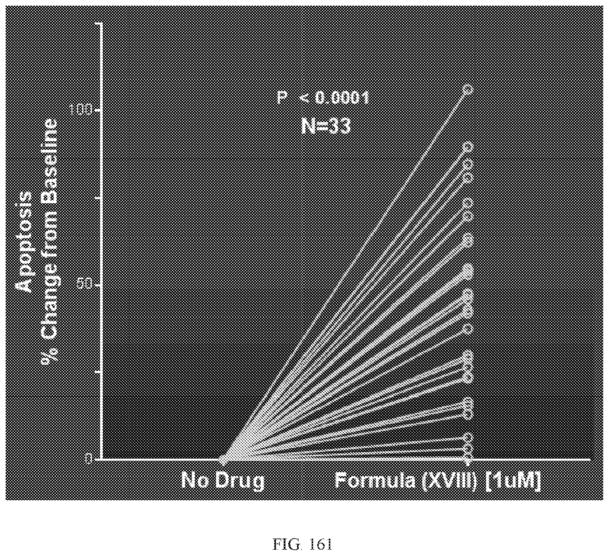

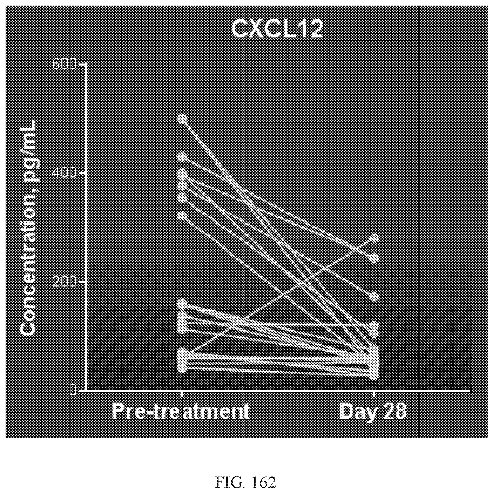

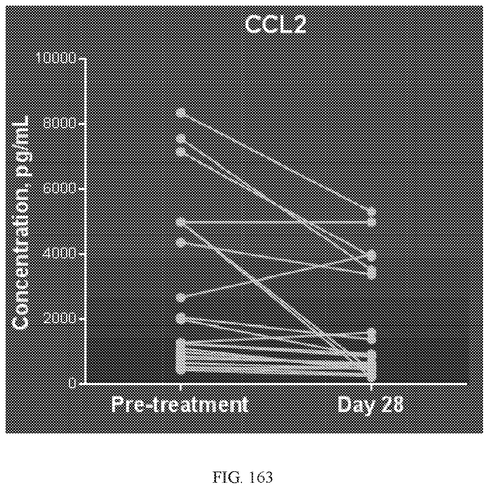

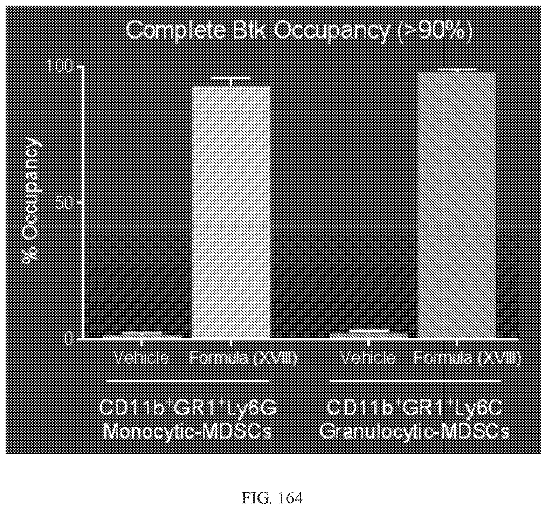

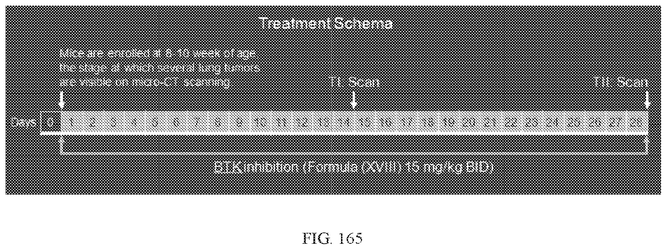

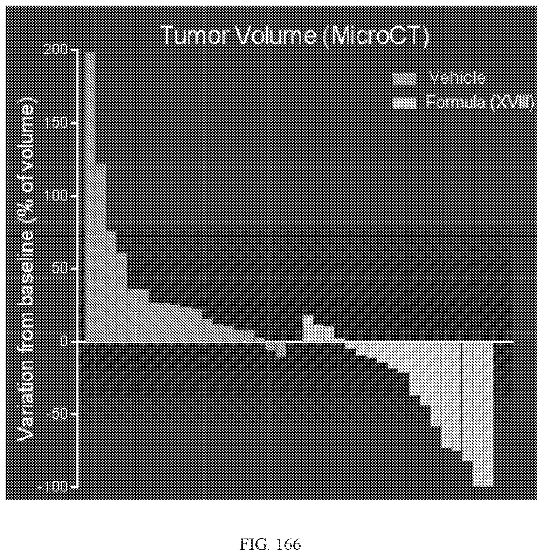

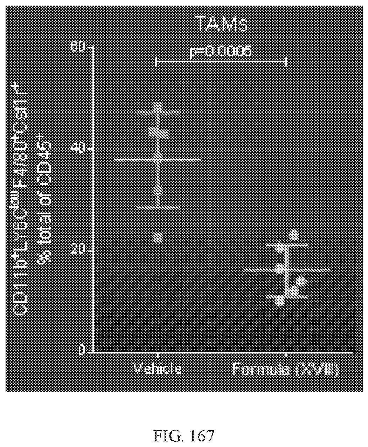

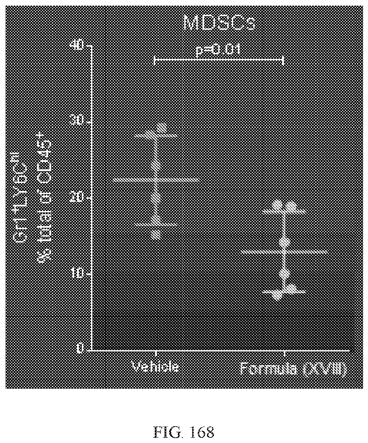

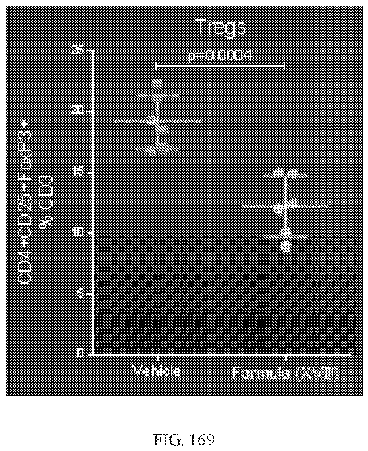

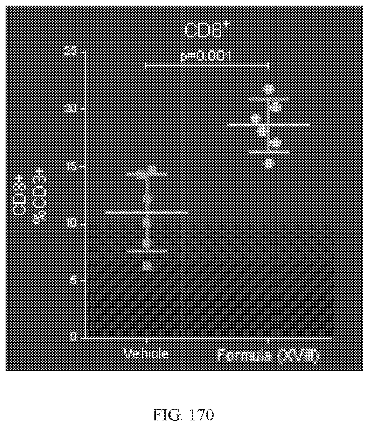

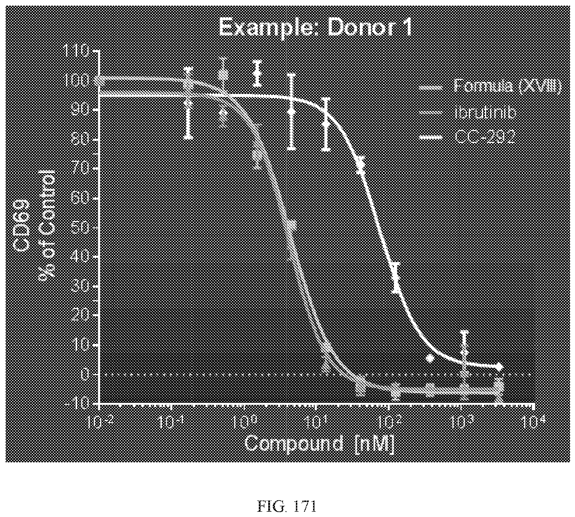

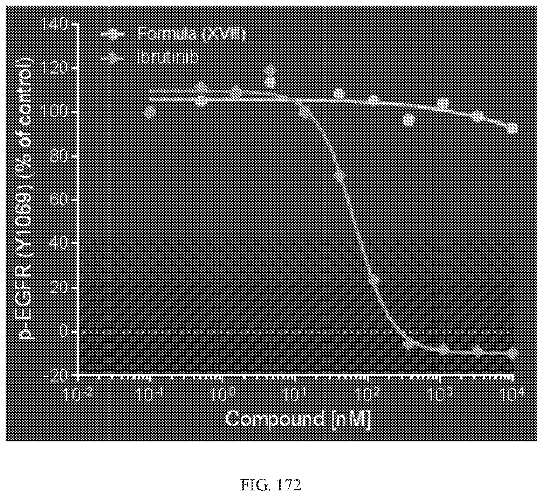

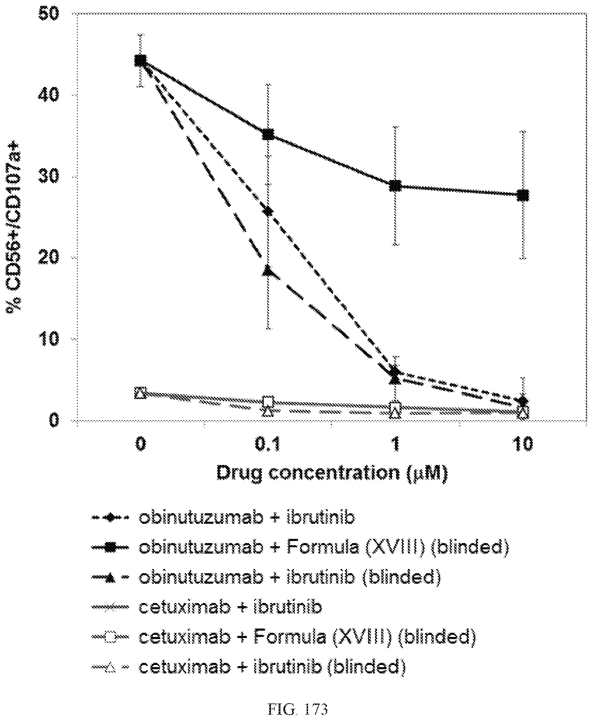

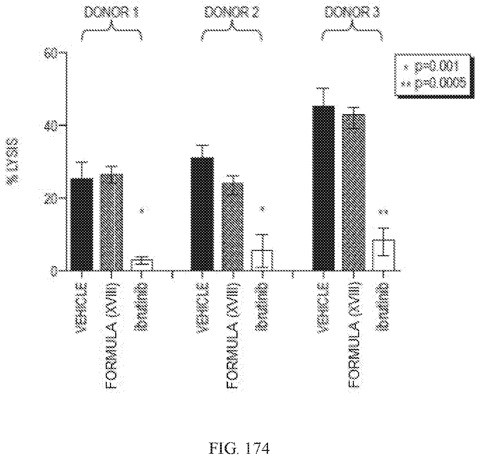

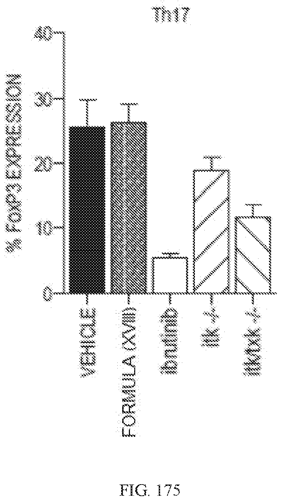

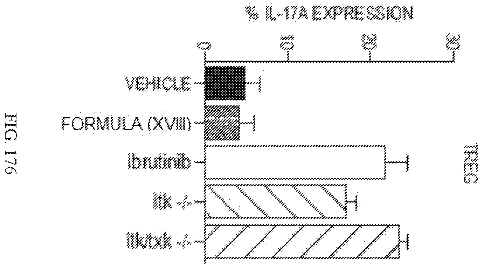

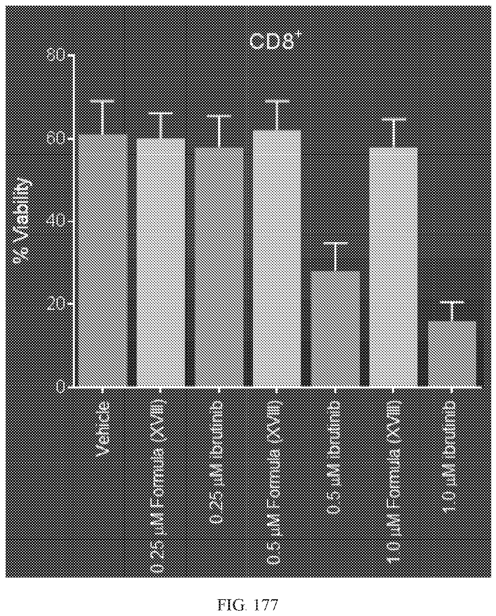

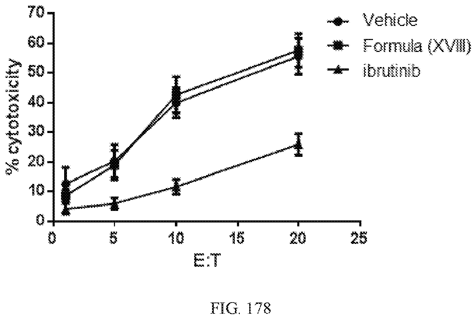

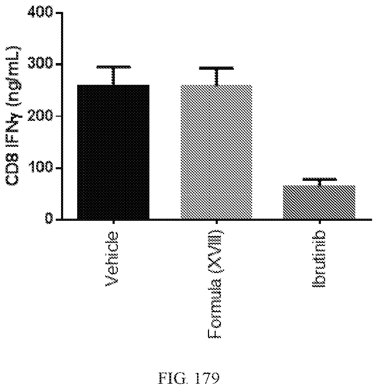

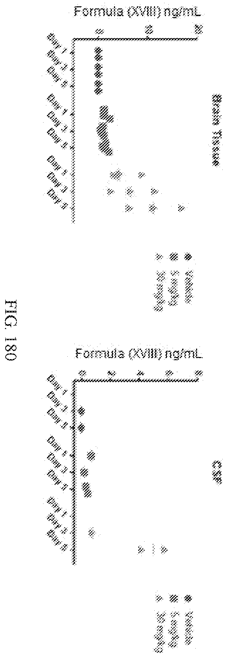

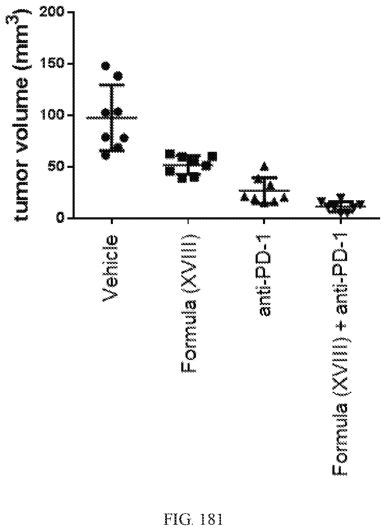

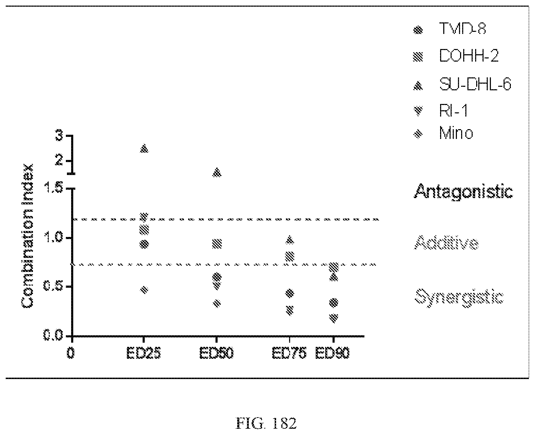

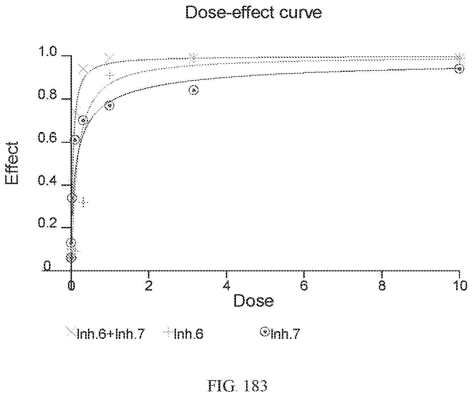

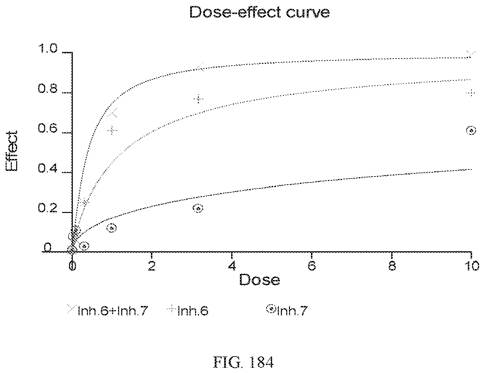

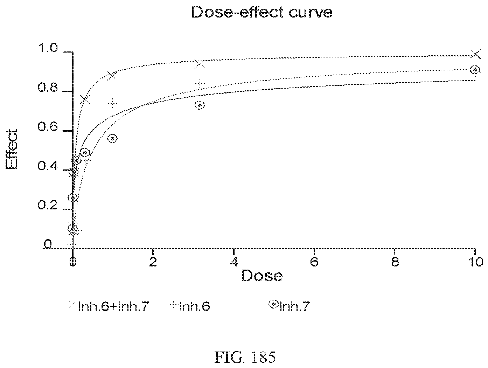

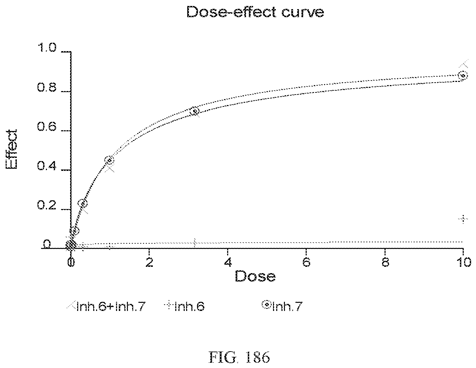

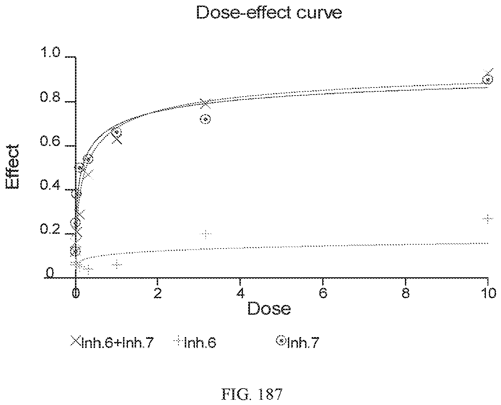

Treatment of CLL patients with Formula (XVIII) also resulted in increased apoptosis, as illustrated in FIG. 161. Apoptotic B-CLL was defined by flow cytometry as having cleaved PARP⁺, Caspase 3⁺, CD19⁺, and CD5⁺ phenotypes. 82% of samples tested had a baseline change greater than 25%. Treatment of CLL patients also showed that Formula (XVIII) decreased plasma chemokines associated with MDSC homing and retention. A significant decrease in CXCL12 and CCL2 levels has been observed in patients treated with Formula (XVIII), as shown in FIG. 162 and FIG. 163, respectively.

Overall, Formula (XVIII) shows superior efficacy to first generation BTK inhibitors such as ibrutinib, or to monotherapy with PI3K-δ inhibitors such as idelalisib. Formula (XVIII) has better target occupancy and better pharmacokinetic and metabolic parameters than ibrutinib, leading to improved B cell apoptosis. Furthermore, unlike treatment with ibrutinib and PI3K-δ inhibitors, treatment with Formula (XVIII) does not affect NK cell function. Finally, treatment with Formula (XVIII) leads to a CLL tumor microenvironmental effect by excluding MDSC cells from the marrow and lymph nodes and reducing their number.

### Reference Example 18 - Clinical Study of a BTK Inhibitor in Leukemia/Lymphoma in Combination with Obinutuzumab (GA-101)

The primary objectives of the study are (1) to determine the overall response rate (ORR) at 12 months with the combination of Formula (XVIII) and obinutuzumab in patients with relapsed or refractory CLL, (2) to determine the ORR at 12 months with the combination of Formula (XVIII) and obinutuzumab in patients with treatment-naive CLL, and (3) to establish the safety and feasibility of the combination of Formula (XVIII) and obinutuzumab.

The secondary objectives of this study are: (1) to determine the complete response (CR) rate and MRD-negative CR rate in previously untreated and relapsed and refractory CLL with this regimen; (2) to determine the progression-free survival (PFS), time to next treatment (TTNT), and overall survival (OS) with this regimen, (3) to perform baseline analysis of patients enrolled on this trial including fluorescence in situ hybridization (FISH), stimulated karyotype, Zap-70 methylation, and IgV_{H} mutational status and describe relationships between these biomarkers and ORR or PFS for patients treated with this regimen; (4) to determine pharmacokinetics (PK) of orally administered Formula (XVIII); (5) to measure pharmacodynamic (PD) parameters including drug occupancy of BTK, change in miR and gene expression on day 8 and 29 of therapy of Formula (XVIII); (6) to determine the influence of Formula (XVIII) on NK cell and T cell function in vivo; (7) to assess for serial development of resistance by baseline and longitudinal assessment of mutations of BTK and PLCG2 at regular follow up intervals and by examining diagnosis to relapse samples by whole exome sequencing; (8) to determine the influence of Formula (XVIII) on emotional distress and quality of life in CLL patients; and (9) to determine trajectory of psychological and behavioral responses to Formula (XVIII) and covariation with response to therapy.

CLL is the most prevalent form of adult leukemia and has a variable clinical course, where many patients do not require treatment for years and have survival equal to age matched controls. Other patients, however, exhibit aggressive disease and have a poor prognosis despite appropriate therapy. Byrd, et al., Chronic lymphocytic leukemia. Hematology Am. Soc. Hematol. Educ. Program. 2004, 163-183. While patients with early disease have not been shown to have a survival advantage with early treatment, most patients will eventually require therapy for their disease with the onset of symptoms or cytopenias, and despite the relatively long life expectancy for early stage disease, CLL remains an incurable disease. Patients diagnosed with or progressing to advanced disease have a mean survival of 18 months to 3 years. Unfortunately these patients with advanced disease are also more refractory to conventional therapy.

The treatment of CLL has progressed significantly over the previous decades. While alkylator therapy was used in the past, randomized trials have demonstrated a higher response rate and longer progression free survival (PFS) with fludarabine and subsequently with fludarabine-and cyclophosphamide-based combinations. O'Brien, et al., Advances in the biology and treatment of B-cell chronic lymphocytic leukemia. Blood 1995, 85, 307-18; Rai, et al., Fludarabine compared with chlorambucil as primary therapy for chronic lymphocytic leukemia. N. Engl. J. Med. 2000, 343, 1750-57; Johnson, et al., Multicentre prospective randomised trial of fludarabine versus cyclophosphamide, doxorubicin, and prednisone (CAP) for treatment of advanced-stage chronic lymphocytic leukemia. The French Cooperative Group on CLL. Lancet 1996, 347, 1432-38; Leporrier, et al., Randomized comparison of fludarabine, CAP, and ChOP in 938 previously untreated stage B and C chronic lymphocytic leukemia patients. Blood 2001, 98, 2319-25; Catovsky, et al., Assessment of fludarabine plus cyclophosphamide for patients with chronic lymphocytic leukemia (the LRF CLL4 Trial): A randomised controlled trial. Lancet 2007, 370, 230-239; Eichhorst, et al., Fludarabine plus cyclophosphamide versus fludarabine alone in first-line therapy of younger patients with chronic lymphocytic leukemia. Blood 2006, 107, 885-91. At the same time, the chimeric anti-CD20 monoclonal antibody rituximab was introduced for the treatment of CLL. At high doses or with dose intensive treatment, single agent rituximab has shown efficacy; however complete responses and extended remissions are very rare. O'Brien, et a/. Rituximab dose-escalation trial in chronic lymphocytic leukemia. J. Clin. Oncol. 2001, 19, 2165-70; Byrd, et al., Rituximab using a thrice weekly dosing schedule in B-cell chronic lymphocytic leukemia and small lymphocytic lymphoma demonstrates clinical activity and acceptable toxicity. Clin. Oncol. 2001, 19, 2153-64. The efficacy of rituximab has been improved by combining it with traditional cytotoxic agents such as fludarabine or fludarabine and cyclophosphamide, which have produced high CR rates and extended progression free survival (PFS) compared to historical controls. Indeed, a large randomized clinical trial reported by the German CLL study group has shown a benefit of the addition of antibody therapy with rituximab to fludarabine and cyclophosphamide in the prolongation of PFS and OS in patients with untreated CLL. Hallek, et a/., Addition of rituximab to fludarabine and cyclophosphamide in patients with chronic lymphocytic leukemia: a randomised, open-label, phase 3 trial. Lancet 2010, 376, 1164-74. This encouraging progress in therapy and our understanding of the disease has resulted in significantly improved response rates and PFS. However, significant improvements in overall survival (OS) and ultimately cure, remain elusive goals.

While fludarabine based chemoimmunotherapy is standard for younger patients, the therapy for older patients is less well defined. In the large Phase 2 and 3 trials outlined previously, median ages were typically in the early-60s, while the average age of patients diagnosed with CLL is 72, which calls into question whether these results are generalizable to the entire CLL population. In fact, the one randomized Phase 3 trial investigating primary CLL therapy in older patients demonstrated that in patients >65 years old, fludarabine is not superior to chlorambucil. Eichhorst, et al., First-line therapy with fludarabine compared with chlorambucil does not result in a major benefit for elderly patients with advanced chronic lymphocytic leukemia. Blood 2009, 114, 3382-91. This finding was corroborated by a large retrospective study of front-line trials performed by the Alliance for Clinical Trials in Oncology, which demonstrated again that fludarabine is not superior to chlorambucil in older patients, but also showed that the addition of rituximab to chemotherapy was beneficial regardless of age. Woyach, et al., Impact of age on outcomes after initial therapy with chemotherapy and different chemoimmunotherapy regimens in patients with chronic lymphocytic leukemia: Results of sequential cancer and leukemia group B studies. J. Clin. Oncol. 2013, 31, 440-7. Two studies have evaluated the combination of rituximab with chlorambucil, showing that this combination is safe and moderately effective. Hillmen, et a/., rituximab plus chlorambucil in patients with CD20-positive B-cell chronic lymphocytic leukemia (CLL): Final response analysis of an open-label Phase II Study, ASH Annual Meeting Abstracts, Blood 2010, 116, 697; Foa, et al., A Phase II study of chlorambucil plus rituximab followed by maintenance versus observation in elderly patients with previously untreated chronic lymphocytic leukemia: Results of the first interim analysis, ASH Annual Meeting Abstracts, Blood 2010, 116, 2462.

Recently, the type II glycoengineered CD20 monoclonal antibody obinutuzumab was introduced. In a Phase 1 trial of previously treated CLL as monotherapy, this antibody has a 62% response rate including 1 MRD-negative complete response, suggesting that alone this antibody may be more active in CLL than rituximab. Morschhauser, et al., Phase I study of R05072759 (GA101) in relapsed/refractory chronic lymphocytic leukemia, ASH Annual Meeting Abstracts. Blood, 2009, 114, 884. The German CLL Study Group (GCLLSG) recently completed a Phase 3 trial of rituximab and chlorambucil or obinutuzumab and chlorambucil versus chlorambucil alone in patients with untreated CLL and significant comorbidities. In this population, obinutuzumab and chlorambucil (but not rituximab and chlorambucil) improved OS over chlorambucil alone (hazard ratio 0.41, p=0.002), and obinutuzumab and chlorambucil improved PFS over rituximab and chlorambucil (median PFS 26.7 months vs 14.9 months, p<0.001). Goede, et al., Obinutuzumab plus chlorambucil in patients with CLL and coexisting conditions, N. Engl. J. Med. 2014, 370, 1101-10. On the basis of these favorable data, the combination of obinutuzumab and chlorambucil is FDA approved as frontline therapy for CLL patients.

Many older patients are also treated with the combination of bendamustine plus rituximab (BR). Although BR has not been compared directly with chlorambucil and rituximab, results of a recent Phase 2 trial show an ORR of 88% with a median event free survival of 33.9 months and 90.5% OS at 27 months. Fischer, et al., Bendamustine in combination with rituximab for previously untreated patients with chronic lymphocytic leukemia: A multicenter phase II trial of the German Chronic Lymphocytic Leukemia Study Group. J. Clin. Oncol. 2012, 30, 3209-16. These results held for patients > 70 years old, and compare favorably with results published for chlorambucil and rituximab. While results with this regimen appear to be improved over historical controls, outcomes are not as good as those observed in younger patients with chemoimmunotherapy. Therefore, the optimal therapy for older patients remains an unmet need in clinical trials.

Additionally, most patients eventually relapse with their disease and are frequently refractory to existing agents. Patients who relapse after combined chemoimmunotherapy have a poor outcome with subsequent standard therapies. While options for these patients include alemtuzumab, bendamustine, high dose corticosteroids, ofatumumab, and combination based approaches, none of these therapies produces durable remissions that exceed that observed with first line chemoimmunotherapy. Keating, et al., Therapeutic role of alemtuzumab (Campath-1H) in patients who have failed fludarabine: results of a large international study. Blood 2002, 99, 3554-61; Bergmann, et al., Efficacy of bendamustine in patients with relapsed or refractory chronic lymphocytic leukemia: results of a phase I/II study of the German CLL Study Group. Haematologica 2005, 90, 1357-64; Thornton PD, Matutes E, Bosanquet AG, et al. High dose methylprednisolone can induce remissions in CLL patients with p53 abnormalities. Ann. Hematology 2003, 82, 759-65; Coiffier, et al., Safety and efficacy of ofatumumab, a fully human monoclonal anti-CD20 antibody, in patients with relapsed or refractory B-cell chronic lymphocytic leukemia: A phase 1-2 study. Blood 2008, 111, 1094-1100; Tsimberidou, et al., Phase I-II study of oxaliplatin, fludarabine, cytarabine, and rituximab combination therapy in patients with Richter's syndrome or fludarabine-refractory chronic lymphocytic leukemia. J. Clin. Oncol. 2008, 26, 196-203. Several of these therapies including alemtuzumab and high dose steroids are also associated with significant toxicities and sustained immunosuppression. Lozanski G, Heerema NA, Flinn 1W, et al. Alemtuzumab is an effective therapy for chronic lymphocytic leukemia with p53 mutations and deletions. Blood 2004,103, 3278-81; Osuji, et al., The efficacy of alemtuzumab for refractory chronic lymphocytic leukemia in relation to cytogenetic abnormalities of p53. Haematologica 2005, 90, 1435-36; Thornton, et al., High dose methyl prednisolone in refractory chronic lymphocytic leukemia. Leuk. Lymphoma 1999, 34, 167-70; Bowen, et al. Methylprednisolone-rituximab is an effective salvage therapy for patients with relapsed chronic lymphocytic leukemia including those with unfavorable cytogenetic features. Leuk Lymphoma 2007, 48, 2412-17; Castro, et al., Rituximab in combination with high-dose methylprednisolone for the treatment of fludarabine refractory high-risk chronic lymphocytic leukemia. Leukemia 2008, 22, 2048-53.

In an ongoing Phase lb/2 study, the BTK inhibitor ibrutinib has shown activity in patients with relapsed or refractory CLL. In patients with relapsed or refractory CLL and measurable lymphadenopathy, the rate of lymph node shrinkage >50% is 89%. With a median follow-up of 4 months, ORR was 48% due to asymptomatic lymphocytosis, and with longer follow-up of 26 months in patients receiving the 420 mg dose, has improved to 71%, with an additional 20% of patients achieving a partial response with lymphocytosis (PR-L). Byrd, et a/., Activity and tolerability of the Bruton's tyrosine kinase (Btk) inhibitor PCI-32765 in patients with chronic lymphocytic leukemia/small lymphocytic lymphoma (CLL/SLL): Interim results of a phase Ib/II study. J. Clin. Oncol. ASCO Annual Meeting Abstracts, 2011, 29, Abstract 6508; Byrd, et al. Targeting BTK with ibrutinib in relapsed chronic lymphocytic leukemia. N. Engl. J. Med. 2013, 369, 32-42. This lymphocytosis is likely related to B cell release from lymph node, spleen and marrow microenvironment due to disruption of homing signals or chemoattractants that are relevant to usual lymphocyte circulation dynamics. Lymphocytosis with ibrutinib is seen within 1-2 weeks of starting therapy, reaches plateau within the first 2-3 cycles, and has resolved over time in virtually all patients. The duration of lymphocytosis does not appear to be related to the depth of eventual response nor to response duration. Woyach, et al., Prolonged lymphocytosis during ibrutinib therapy is associated with distinct molecular characteristics and does not indicate a suboptimal response to therapy. Blood 2014,123, 1810-7. Response to ibrutinib occurs independently of high-risk genomic features including IgV_{H} mutational status and del(17p13.1). Responses to this drug have been durable as well, with an estimated 26 month PFS of 76% and OS of 83% for these relapsed and refractory patients. This study also included a cohort of 31 previously untreated patients. With 16.6 months of follow-up, ORR is 71%, with an additional 10% of patients having persistent lymphocytosis; estimated 22 month PFS is 96%. This agent is currently in Phase 3 trials in treatment-naive disease and is currently FDA approved for the treatment of relapsed CLL. These data with ibrutinib support the potential benefits of selective BTK inhibition in CLL. However, while highly potent in inhibiting BTK, ibrutinib has also shown in vitro activity against other kinases (e.g., epidermal growth factor receptor), which may be the cause of ibrutinib-related diarrhea and rash. Honigberg, et αl., The Bruton tyrosine kinase inhibitor PCI-32765 blocks B-cell activation and is efficacious in models of autoimmune disease and B-cell malignancy. Proc. Natl. Acad. Sci. USA 2010,107, 13075-13080. In addition, it is a substrate for both cytochrome P450 (CYP) enzymes 3A4/5, which increases the possibility of drug-drug interactions. Finally, the inhibition of ITK that is seen with ibrutinib has the potential to abrogate NK cell ADCC, which makes combination with monoclonal antibodies less effective. Kohrt, et αl., Ibrutinib antagonizes rituximab-dependent NK cell-mediated cytotoxicity. Blood 2014, 123, 1957-60. These liabilities support the development of alternative BTK inhibitors for use in the therapy of lymphoid cancers.

In this Phase 1B study, two cohorts (relapsed/refractory and treatment-naive) will be evaluated with slightly staggered enrollment. First, 6 subjects with R/R CLL will be enrolled into Cohort 1. Once the safety has been evaluated, the R/R cohort will be expanded to 26 subjects and enrollment of 6 treatment-naive subjects can begin in Cohort 2. Once safety is established for Cohort 2, then the cohort will be expanded to 19 subjects.

Formula (XVIII) will be administered starting cycle 1 day 1 and will be administered twice daily (100 mg BID) until disease progression. Obinutuzumab will be given in the standard dosing fashion starting on cycle 2 day 1. On cycle 2 day 1, patients will receive 100 mg IV. On cycle 2 day 2, patients will receive 900 mg. On cycle 2 days 8 and 15, patients will receive 1000 mg IV. On cycles 3-7, patients will receive 1000 mg on day 1 of each cycle. For patients treated at dose level -1, 100 mg will be given on Day 1 and 650 mg on Day 2 of Cycle 2. On cycle 2 day 8 and 15, patients will receive 750 mg IV and during cycles 3-7, patients will receive 750 mg on Day 1 of each cycle. It is acceptable for cycles to begin < a 24-hour (1 business day) window before and after the protocol-defined date for Day 1 of a new cycle.

The inclusion criteria for patient eligibility are as follows: (1) Patients with a diagnosis of intermediate or high risk CLL (or variant immunophenotype), SLL, or B-PLL by IWCLL 2008 criteria" who have: (a) COHORT 1: Previously received at least one therapy for their disease; (b) COHORT 2: Previously untreated disease and > 65 years old OR under 65 years old and refuse or are ineligible for chemoimmunotherapy; (2) Patients on Cohort 1 may have received previous ibrutinib (or another BTK inhibitor) as long as discontinuation was for a reason other than "on-treatment" disease progression; (3) All patients must satisfy one of the following criteria for active disease requiring therapy: (a) Evidence of marrow failure as manifested by the development or worsening of anemia or thrombocytopenia (not attributable to autoimmune hemolytic anemia or thrombocytopenia); (b) Massive (> 6 cm below the costal margin), progressive or symptomatic splenomegaly; (c) Massive nodes (> 10 cm) or progressive or symptomatic lymphadenopathy; (d) Constitutional symptoms, which include any of the following: Unintentional weight loss of 10% or more within 6 months, Significant fatigue limiting activity, Fevers > 100.5 degrees F for 2 weeks or more without evidence of infection, Night sweats > 1 month without evidence of infection; (4) Measurable nodal disease by computed tomography (CT). Measurable nodal disease is defined as > 1 lymph node > 1.5 cm in the longest diameter in a site; (5) Patients with a history of Richter's syndrome are eligible if they now have evidence of CLL only, with < 10% large cells in the bone marrow; (6) Subjects must have adequate organ function, defined as creatinine < 2.5 times the upper limit of normal (ULN), ALT and AST < 3.0 × ULN, and bilirubin < 2.5 × ULN; (7) Platelets > 50 × 10⁹/L. In subjects with CLL involvement of the marrow, > 30 × 10⁹/L; (8) ANC > 750/mm³ In subjects with CLL involvement of the marrow, ANC > 500/mm³; (9) Subject must have an ECOG performance status < 2; (10) Subject must not have secondary cancers that result in a life expectancy of < 2 years or that would confound assessment of toxicity in this study; (11) Subjects must be > 18 years of age; (12) Subject must provide written informed consent. A signed copy of the consent form will be retained in the patient's chart; (13) Subject must be able to receive outpatient treatment and follow-up at the treating institution; (14) Subject must have completed all CLL therapies > 4 weeks prior to first study dose. Palliative steroids are allowed, but must be at a dose equivalent of < 20 mg prednisone daily for at least 1 week prior to treatment initiation; (15) Subjects capable of reproduction and male subjects who have partners capable of reproduction must agree to use an effective contraceptive method during the course of the study and for 2 months following the completion of their last treatment. Females of childbearing potential must have a negative β-hCG pregnancy test result within 3 days of first study dose. Female patients who are surgically sterilized or who are > 45 years old and have not experienced menses for > 2 years may have ther3-hCG pregnancy test waived; (16) Subjects must be able to swallow whole capsules.

The exclusion criteria for patient eligibility are as follows: (1) For cohort 1, previous therapy for CLL. Treatment of autoimmune complications of CLL with steroids or rituximab is allowed, however, CD20 must have returned on 10% of the CLL cells if rituximab was recently administered. Palliative steroids are acceptable at doses < 20 mg prednisone equivalent daily; (2) Any life-threatening illness, medical condition, or organ dysfunction which, in the investigator's opinion, could compromise the patients' safety, interfere with the absorption or metabolism of Formula (XVIII), or put the study outcomes at undue risk; (3) Female subjects who are pregnant or breastfeeding; (4) Subjects with active cardiovascular disease not medically controlled or those who have had myocardial infarction in the past 6 months, or QTc > 480 ms; (5) Malabsorption syndrome, disease significantly affecting gastrointestinal function, or resection of the stomach or small bowel or gastric bypass, ulcerative colitis, symptomatic inflammatory bowel disease, or partial or complete bowel obstruction; (6) Grade 2 toxicity (other than alopecia) continuing from prior anticancer therapy including radiation; (7) Major surgery within 4 weeks before first dose of study drug; (8) History of a bleeding diathesis (e.g., hemophilia, von Willebrand disease); (9) Uncontrolled autoimmune hemolytic anemia or idiopathic thrombocytopenia purpura; (10) History of stroke or intracranial hemorrhage within 6 months before the first dose of study drug; (11) Requires or receiving anticoagulation with warfarin or equivalent vitamin K antagonists (eg, phenprocoumon) within 28 days of first dose of study drug; (12) Requires treatment with long-acting proton pump inhibitors (e.g., omeprazole, esomeprazole, lansoprazole, dexlansoprazole, rabeprazole, or pantoprazole); (13) Subjects with active infections requiring IV antibiotic/antiviral therapy are not eligible for entry onto the study until resolution of the infection. Patients on prophylactic antibiotics or antivirals are acceptable; (14) Subjects with history of or ongoing drug-induced pneumonitis; (15) Subjects with human immunodeficiency virus (HIV) or active infection with hepatitis C virus (HCV) or hepatitis B virus (HBV) or any uncontrolled active systemic infection; (16) Subjects who are known to have Hepatitis B infection or who are hepatitis B core antibody or surface antigen positive. Patients receiving prophylactic WIG may have false positive hepatitis serologies. Patients who are on WIG who have positive hepatitis serologies must have a negative hepatitis B DNA to be eligible; (17) Subjects with substance abuse or other medical or psychiatric conditions that, in the opinion of the investigator, would confound study interpretation or affect the patient's ability to tolerate or complete the study; (18) Subjects cannot concurrently participate in another therapeutic clinical trial; (19) Subjects who have received a live virus vaccination within 1 month of starting study drug.

In this study, Formula (XVIII) is administered 100 mg BID, with the second dose 11-13 hours after the first. Obinutuzumab is administered by IV infusion as an absolute (flat) dose. Obinutuzumab is administered in a single day, with the exception of the first administration when patients receive their first dose of obinutuzumab over two consecutive days (split dose) in Cycle 2: 100 mg on Day 1 and 900 mg on Day 2. For patients treated at dose level -1 (750 mg obinutuzumab), - 100 mg will be given on Day 1 and 650 mg on Day 2. On days when both Formula (XVIII) and obinutuzumab are given, the order of study treatment administration will be Formula (XVIII) followed at least 1 hour later by obinutuzumab. The full dosing schedule is given in Table 15.

**TABLE 15. Dosing of obinutuzumab during 6 treatment cycles each of 28 days duration.**

| **Day of Treatment Cycle** | | **Dose of Obinutuzumab** | **Rate of Infusion (In the absence of infusion reactions/ hypersensitivity during previous infusions)** |
|---|---|---|---|
| **Cycle 2 (loading doses)** | Day 1 | 100 mg | Administer at 25 mg/hr over 4 hours. Do not increase the infusion rate. |
| | Day 2 | 900 mg | Administer at 50 mg/hr. The rate of the infusion can be escalated in increments of 50 mg/hr every 30 minutes to a maximum rate of 400 mg/hr. |
| | Day 8 | 1000 mg | Infusions can be started at a rate of 100 mg/hr and increased by 100 mg/hr increments every 30 minutes |
| | Day 15 | 1000 mg | |
| **Cycles 3-7** | Day 1 | 1000 mg | to a maximum of 400 mg/hr. |

Anti-CD20 antibodies have a known safety profile, which include infusion related reactions (IRR). Anti-CD20 antibodies, and in particular obinutuzumab, can cause severe and life threatening infusion reactions. Sequelae of the infusion reactions include patient discontinuations from antibody treatment leading to suboptimal efficacy or increased medical resource utilization, such as hospitalization for hypotension or prolonged antibody infusion time. In the initial study of obinutuzumab in relapsed/refractory CLL patients (Cartron, et al., Blood 2014, 124, 2196), all patients (n=13) in the Phase 1 portion experienced IRRs (15% Grade 3, no Grade 4, and 100% patients experienced all grade AE), with hypotension and pyrexia the most common symptoms. In the Phase 2 portion of the study, 95% of patients developed IRR, with 60% of cases developing symptoms of hypotension; of those, 25% were Grade 3 reactions. In the pivotal trial of obinutuzumab and chlorambucil in previously untreated patients, 69% developed infusion related reactions, of which 21% were grade 3-4.

The results of the Phase 1b study described in this example for Formula (XVIII) in combination with obinutuzumab for patients with relapsed/refractory or untreated CLL/SLL/PLL are as follows. 6 patients have been treated in the study to date with the combination of Formula (XVIII) and obinutuzumab. Patients are first treated with a month run-in of Formula (XVIII) alone, then on cycle 2, day 1, patients are given obinutuzumab. To date, 41 doses of obinutuzumab have been administered to 6 patients. Lymphocyte counts immediately prior to treatment with obinutuzumab have ranged from 8 to 213 × 10⁹/L. No cases of serious or Grade 3-4 IRRs have been reported. Only 2 patients have had obinutuzumab temporarily held for chills and arthralgias/sluured, respectively, and were able to complete the planned infusion. An additional 3 patients had adverse events within 24 hours of the infusion, all grade 1 (terms: flushing, palpitations in one patient, rash, and restlessness and headache). Consequently, there has been a substantial decrease in serious or Grade 3-4 IRRs with the one month lead-in of Formula (XVIII), which could potentially lead to higher efficacy for the combination as well as better tolerability, leading to a decrease in medical resource utilization.

### Example 19 - Clinical Study of a Combination of Pembrolizumab and a BTK Inhibitor in B Cell Malignancies

The use of multidrug regimens has been shown to produce higher CR rates and more durable responses, resulting in improved survival in most oncology indications. However, these benefits are often outweighed with the increased toxicity associated with multidrug regimens. This high risk-benefit ratio limits the use of many effective multidrug regimens in elderly patients or patients with comorbid conditions.

The advent of highly selective, targeted active pharmaceutical ingredients such as BTK inhibitors has changed the risk-benefit paradigm traditionally associated with cytotoxic chemotherapy regimens. For example, ibrutinib, a first-generation oral, small-molecule BTK inhibitor, has been approved for the treatment for CLL and MCL. In addition, ibrutinib has shown clinical efficacy in other NHL histologies, including FL (Advani, et a/., J. Clin. Oncol. 2013, 31, 88-94), ABC-DLBCL (De Vos, et a/., Haematologica 2013, 98(s1), S1180), and in WM (Treon, et a/., Blood 2013, 122, 251). Preliminary data suggests that patients with multiple myeloma (MM) with high BTK activity, as evidenced by phosphorylated Btk, may be particularly responsive to BTK inhibitor therapy (Liu, et al., Leuk Lymphoma 2014, 55, 177-81). In preclinical studies, BTK inhibition significantly reduced MM cell growth and tumor-induced osteolysis in a murine model (Tai, et αl., Blood 2012, 20, 1877-1887). Despite these significant advances, the investigation of additional treatment regimens is essential to improve outcomes in B cell malignances. A low proportion of patients achieve CR when treated with single-active pharmaceutical ingredient BTK inhibitors compared with conventional chemotherapy or chemoimmunotherapy regimens. Moreover, the median duration of response can be brief (< 12 months) in aggressive histologies.

This proof-of-concept clinical study assesses the clinical potential of combined BTK inhibition and checkpoint blockade by evaluating the safety, PD, and efficacy of Formula (XVIII) and pembrolizumab in B-cell malignancies. This is a Phase 1b/2, open-label, nonrandomized study that will be conducted in 2 parts. Part 1 of the study will determine the safety and preliminary efficacy of Formula (XVIII) and pembrolizumab and Part 2 allows for possible expansion cohorts into a wider range of B-cell malignancies.

Part 1: Six subjects will be enrolled to receive Formula (XVIII) in combination with pembrolizumab. If the combination is safe with ≤ 1 dose-limiting toxicity (DLT) (6-week observation period) in the first 6 subjects, the cohort will be expanded to up to 24 subjects to obtain additional safety information and to assess the efficacy of the combination. Part 1 of the study will include adult subjects with the following disease types: Non-germinal center B-cell (non-GCB) diffuse large B-cell lymphoma (DLBCL); Follicular lymphoma (FL); and CLL/small lymphocytic lymphoma (SLL).

Part 2: Part 2 consists of expansion groups of up to 12 subjects per histology provided the safety and efficacy results from Part 1 of the study indicate that further evaluation of the combination is warranted. The possible expansion groups for Part 2 may include adult subjects with the following disease types: non-GCB DLBCL, germinal center B-cell (GCB) DLBCL, Richter's syndrome, mantle cell lymphoma (MCL), indolent non-Hodgkin lymphoma (iNHL), FL, Waldenstrom macroglobulinemia (WM), CLL/SLL, multiple myeloma (MM), other B-cell malignancy (including Hodgkin's lymphoma, Burkitt's lymphoma, marginal zone lymphomas, and hairy cell leukemia).

Treatment with Formula (XVIII) and pembrolizumab, in both Part 1 and Part 2, may be continued until disease progression or an unacceptable drug-related toxicity occurs as defined in the protocol. Combination treatment can end for subjects with confirmed CR (or stringent CR [sCR] for MM) if treatment has been administered for at least 24 weeks and 2 doses of pembrolizumab have been administered after confirmation of CR/sCR. At the end of 52 weeks of treatment, subjects who are tolerating the regimen and deriving clinical benefit may be eligible to roll over into a maintenance protocol under which they could receive Formula (XVIII) and/or pembrolizumab.

All subjects will have hematology, chemistry, and urinalysis safety panels performed at screening. Once dosing commences (Day 1), all subjects will be evaluated for safety, including serum chemistry and hematology, once weekly for the first 8 weeks and monthly thereafter. Radiologic tumor assessments will be done at screening and at 8-to 12-week intervals during the trial.

For Part 1, a DLT will be defined as the occurrence of any of the following study drug-related adverse events (note: adverse events clearly related to disease progression or the subject's current medical history and associated comorbidities will not be considered DLTs): (1) Any Grade ~ 3 non-hematologic toxicity (except Grade 3 nausea, vomiting, or diarrhea that respond to supportive therapy); (2) Any of the following hematologic toxicities: (a) Grade 4 neutropenia lasting > 7 days, (b) Grade 4 thrombocytopenia, or Grade 3 thrombocytopenia with bleeding, or any requirement for platelets transfusion, (c) Grade ~ 3 febrile neutropenia (temperature ≥ 38.5°C), (d) Grade 4 anemia, unexplained by underlying disease; (3) Dosing delay due to toxicity for > 28 consecutive days.

The study objectives are as follows: (1) characterize the safety profile of Formula (XVIII) and pembrolizumab in subjects with relapsed or refractory B-cell malignancies; (2) evaluate the activity of Formula (XVIII) and pembrolizumab as measured by overall response rate (ORR), duration of response, progression-free survival, overall survival, and time-to-next treatment; (3) determine the effects of Formula (XVIII) plus pembrolizumab on peripheral blood T cells and myeloid-derived suppressor cells (MDSCs); (4) determine if any characteristics of peripheral blood T cells and/or MDSC_{S} correlate with immune-mediated toxicities, (5) determine if any characteristics of peripheral blood T cells and/or MDSC_{S} correlate with response to Formula (XVIII) and pembrolizumab, and (6) determine if any baseline tumor characteristics correlate with response to Formula (XVIII) and pembrolizumab.

The safety parameters for the study include type, frequency, severity, timing of onset, duration, and relationship to study drug of any treatment-emergent adverse events (AEs) or abnormalities of laboratory tests; serious adverse events (SAEs); and DLTs or AEs leading to discontinuation of study treatment.

The pharmacodynamic and biomarker parameters for the study are as follows. The occupancy of BTK by Formula (XVIII) will be measured in peripheral blood mononuclear cells (PBMCs) and bone marrow, if available, with the aid of a biotin-tagged Formula (XVIII) analogue probe. The effect of Formula (XVIII) and pembrolizumab on B cells, T cells, and MDSC_{S} will also be evaluated. Tumor tissue, when available, will be evaluated for PD-L1 expression.

The efficacy parameters for the study include ORR, duration of response, progression-free survival, overall survival, and time-to-next treatment.

The sample size for part 1 is up to 24 subjects, and for part 2 is between 12 and 108 subjects.

The inclusion criteria for part 1 are:
(1) Diagnosis of non-GCB DLBCL or iNHL as documented by medical records and with histology based on criteria established by the World Health Organization (WHO) (If a subject has DLBCL, it is characterized as *de novo* non-GCB DLBCL (Choi, et al., Clin. Cancer Res. 2009, 15, 5494-5502; Hans, et al., Blood 2004, 103, 275-282); If the subject has iNHL, the histology shows 1 of the following subtypes: FL Grade 1, 2, or 3a, or CLL/SLL);
(2) Prior treatment for lymphoid malignancy (applies to Part 1 and Part 2): If the subject has DLBCL, there is no curative option with conventional therapy and the prior treatment included ≥ 1 prior combination chemoimmunotherapy regimen (eg, anthracycline based therapy with rituximab); if the subject has MCL or iNHL, the prior treatment comprised any of the following: ~ 1 regimen containing an anti-CD20 antibody administered for ≥ 2 doses and/or ≥ 1 regimen containing ~ 1 cytotoxic active pharmaceutical ingredient (eg, bendamustine, chlorambucil, cyclophosphamide, cytarabine, doxorubicin) administered for ~ 2 cycles, and/or ≥ 1 regimen containing ⁹⁰Y-ibritumomab tiuxetan (ZEVALIN) or ¹³¹1-tositumomab (BEXXAR);
(3) Presence of radiographically measurable lymphadenopathy or extranodal lymphoid malignancy (defined as the presence of a ≥ 2.0 cm lesion, as measured in the longest dimension by computed tomography [CT] scan). Note: not applicable to subjects with WM and MM
(4) Absolute neutrophil count (ANC) ≥ 1.5 × 109/L or platelet count ≥ 100 × 10⁹/L unless due to disease involvement in the bone marrow (Part 1 only).

The inclusion criteria for part 2, which are in addition to the Part 1 criteria, are:
(1) DLBCL (GCB): Confirmed diagnosis of DLBCL with disease characterized as GCB subtype by immunohistochemistry (Choi, et al., Clin. Cancer Res. 2009, 15, 5494-5502; Hans, et al., Blood 2004, 103, 275-282) and meeting the rest of the criteria as defined above.
(2) If the subject has MCL, it is characterized by documentation of monoclonal B-cell that have a chromosome translocation t(11;14)(q13;q32) and/or overexpression of cyclin D1;
(3) Richter's syndrome: Confirmed diagnosis of and biopsy-proven DLBCL due to Richter transformation and meeting the rest of the criteria as defined above;
(4) WM: Confirmed diagnosis of WM, which has relapsed after, or been refractory to ≥ 1 prior therapy for WM, and is progressing at the time of study entry and meeting the rest of the criteria as defined above. Must be able to provide archival or newly obtained bone marrow aspirate/biopsy material for biomarker analysis.
(5) MM: Confirmed diagnosis of MM, which has relapsed after, or been refractory to ≥ 1 prior therapy for MM, and is progressing at the time of study entry and meeting the rest of the criteria as defined above. Must be able to provide archival or newly obtained bone marrow aspirate/biopsy material for biomarker analysis.
(6) Other B-cell malignancy (including: Hodgkin's lymphoma, Burkitt's lymphoma, marginal zone lymphomas, mediastinal large B-cell lymphoma, and hairy cell leukemia): Confirmed diagnosis of previously treated B-cell malignancy and meeting the rest of the criteria as defined above.

Formula (XVIII) is provided as hard gelatin capsules for oral administration. Pembrolizumab is provided as a lyophilized powder in single-use vial for reconstitution and is administered as an intravenous infusion over 30 minutes. The regimen used in the study is: Formula (XVIII) 100 mg twice a day (BID) continuous oral dosing; KEYTRUDA (pembrolizumab) 2 mg/kg by intravenous (IV) infusion every 3 weeks.

Descriptive statistics (including means, standard deviations, and medians for continuous variables and proportions and confidence intervals [CIs] for discrete variables) will be used to summarize data as appropriate. Depending on Part 1 and the number of expansion cohorts opened in Part 2, 6 to 132 evaluable subjects will be enrolled. In Part 1 (DLT review), enrollment of 6 subjects for DLT review is consistent with sample sizes used in oncology studies for determination of maximum tolerated dose (MTD). The trial employs the standard National Cancer Institute definition of MTD (dose associated with DLT in < 33.3% of subjects). Provided ≤ 1 DLT occurs during the DLT review, then expansion will occur in Part 1 to include up to 24 subjects in a select group of histologies. The safety and preliminary efficacy results from Part 1 will be used to determine opening Part 2 of the protocol. In Part 2 (expansion groups), enrollment of 12 subjects per group offers the opportunity to determine if there is sufficient antitumor activity to warrant further development in the selected tumor types. An ORR of ≥ 20% is considered the minimum value of potential interest in each of the selected indications. If 0/12 subjects in a group experience an objective response, the probability is > 0.90 that an ORR of ≥ 20% will be excluded for that cancer (1-sided exact binomial 90% CI upper bound=17.5%).

Results of this study were obtained after enrollment of 24 patients, including: Part 1 DLT: 7 enrolled (4 CLL and 3 folicular lymphoma patients); Part 1 expansion: 2 enrolled (2 CLL); and Part 2:15 enrolled (5 CLL, 2 mantle cell lymphoma, 3 folicular lymphoma, 1 multiple myeloma, 1 non-GCB DLBCL, 1 gray zone lymphoma, 1 Hodgkin's lymphoma, and 1 marginal zone lymphoma patients). With 6 patients progressing past week 8, the following responses were observed: 1 partial response in a folicular lymphoma patient, 2 partial responses plus lymphocytosis in 2 CLL patients, and 3 observations of stable disease in 1 CLL patient and 2 folicular lymphoma patients. No safety concerns were noted during DLT.

### Example 20 - Clinical Study of a BTK Inhibitor Alone and in Combination with Pembrolizumab in Subjects with Advanced or Metastatic Pancreatic Cancer

In 2014, approximately 46,420 people in the United States will be diagnosed with pancreatic cancer (Siegel, et al., CA Cancer J. Clin. 2014, 64, 9-29). Because of the aggressive nature of this cancer, the annual mortality rate almost matches the incidence rate, and it is expected that ~39,590 will die from this disease in the same year. For the 20% of patients with disease involving only the pancreas, surgical resection is the primary therapy. In the ~80% of patients with regional disease extension or metastases at presentation, chemotherapy is the primary treatment (Tempero, et al, J. Natl. Compr. Canc. Netw. 2014, 12, 1083-1093). Among the therapeutic options, polychemotherapy with infusional 5-fluorouracil (5-FU), leucovorin, irinotecan, and oxaliplatin (FOLFIRINOX) or gemcitabine plus albumin-bound paclitaxel (nab-paclitaxel) are commonly employed (Tempero, et al., J. Natl. Compr. Canc. Netw. 2014, 12, 1083-1093). However, because of the inherent chemoresistance of pancreatic cancer, median PFS with these intensive regimens is ≤ 6 months (Conroy, et al., N. Engl. J. Med. 2011, 364, 1817-25, Von Hoff, et al., N. Engl. J. Med. 2013, 369, 1691-1703). Approximately 45% of patients who receive such first-line regimens are alive and sufficiently fit to receive second-line therapy, but no therapies have been approved by the United States Food and Drug Administration (FDA) for such patients. Despite off-label use of existing chemotherapeutic active pharmaceutical ingredients, survival at 2 years is < 10%. Thus, while antitumor benefit has been observed with such regimens, toxicity is substantial and therapeutic options are limited. Novel, less toxic approaches are desperately needed for this lethal cancer, particularly in patients who experience failure of existing first-line therapies.

The importance of pancreatic cancer stroma in the biology of pancreatic cancer has been increasingly recognized (Feig, et al., Clin. Cancer Res. 2012,18, 4266-76, Rucki, et al., World J. Gastroenterol. 2014, 20, 2237-46, Wilson, et al., Front Physiol. 2014, 5, 52). Pancreatic ductal adenocarcinoma exists in a complex desmoplastic microenvironment that provides stromal support for tumor growth and conceals the tumor from immune surveillance. Tumor-associated stroma comprises a mix of fibroblasts (pancreatic stellate cells) and an abundance of mast cells, immunosuppressive Tregs, MDSCs, and TAMs that promote tumor growth and restrain immunologically mediated tumor cell killing (Shibuya, et al., PLoS One 2014, 9, e96565). Pancreatic cancers secrete chemokines that recruit immune cells to the tumor site. These immune cells are then activated either by direct contact or by cancer cell-derived triggers to selectively release "procancer" mediators (Feig, et al., Clin. Cancer Res. 2012, 18, 4266-76, Ma, et al., Cancer Immunol. Immunother. 2014, 63, 247-57). These mediators induce angiogenesis, promote tumor proliferation, inhibit antitumor responses, and alter the surrounding stroma to permit metastases. Treatment of tumor-bearing mice with active pharmaceutical ingredients that block immunocyte migration and function has been shown to decrease the growth of pancreatic cancer (Ma, et al., Cancer Immunol. Immunother. 2014, 63, 247-57; Shibuya, et al., PLoS One 2014, 9, e96565).

Several negative regulatory checkpoint molecules function to check overstimulation of immune responses and contribute to the maintenance of immune tolerance to self-antigens (McDermott and Atkins, Cancer Med. 2013, 2, 662-673). These molecules include cytotoxic T-lymphocyte antigen-4 (CTLA-4), as well as the programmed death (PD)-1 receptor and its ligands (PD-L1 and PD-L2). CTLA-4 acts as a signal dampener, largely within the lymph nodes, to regulate the magnitude of early activation of naive and memory T cells. By contrast, PD-1 is induced on T cells after activation in response to inflammatory signals and limits T-cell function at sites of infection or tumor in peripheral tissues. As the T-cell response progresses, these negative regulatory molecules are induced, limiting the magnitude and duration of the response to prevent healthy tissue damage. Tumors are capable of exploiting the homeostatic mechanisms regulated by these checkpoint molecules, thus limiting immune destruction.

Such checkpoint pathways appear to be operative in pancreatic cancer. Immunohistochemistry analyses have indicated a significantly worse prognosis for patients with PD-L1-positive pancreatic cancer than for those with PD-LI-negative tumors (Nomi, et al., Clin. Cancer Res. 2007, 13, 2151-57; Loos, et al., Cancer Lett. 2008, 268, 98-109). These data have been corroborated in murine pancreatic cancer models in which genetic engineered cells expressing high or low levels of PD-L1 showed hyperproliferative or hypoproliferative phenotypes, respectively (Song, et al., Oncol. Rep. 2014, 31, 1191-98). In human cancers, a positive correlation between PD-L1 expression and Treg infiltration (Loos, et al., Cancer Lett. 2008, 268, 98-109), and an inverse correlation between PD-L1 expression and cytotoxic T lymphocyte infiltration (Nomi, et al., Clin. Cancer Res. 2007, 13, 2151-57) has been reported. Among clinical subjects, PD-L1 tumor expression has been associated with response to therapeutic anti-PD-1 blockade (Taube, etal., Clin. Cancer Res. 2014, 20, 5064-74). Evaluation of intratumoral T cells in pancreatic cancer has shown that the majority expressed PD-1 (Shibuya, et al., PLoS One 2014, 9, e96565), further supporting the concept that pancreatic cancer evades antitumor immunity via PD-L1/PD-1 signaling in the stroma.

BTK is expressed among cells of hematopoietic origin, including B cells, myeloid cells, mast cells and platelets, where it regulates multiple cellular processes including proliferation, differentiation, apoptosis, and cell migration (Khan, Immunol. Res. 2001, 23, 147-156; Mohamed, et al., Immunol. Rev. 2009, 228, 58-73; Bradshaw, Cell Signal. 2010, 22, 1175-84). In addition, BTK-dependent activation of mast cells, myeloid cells and other immunocytes in peritumoral inflammatory stroma has been shown to sustain the complex microenvironment needed for lymphoid and solid tumor maintenance (Soucek, et al., Neoplasia 2011, 13, 1093-1100, Ponader, et al., Blood 2012, 119, 1182-1189, de Rooij, et a/., Blood 2012, 119, 2590-4).

In model systems, *ex vivo* analyses demonstrated BTK inhibition results in macrophages that polarize into M1 macrophages, instead of showing enhanced induction of immunosuppressive M2 macrophages (Ní Gabhann, et al., PLoS One 2014, 9, e85834). These data suggest inhibition of BTK may impair the capacity of tumor-associated macrophages critical for promotion of tumor invasion and metastasis (Mouchemore, et al., FEBSJ. 2013, 280, 5228-5236). Several lines of evidence demonstrate BTK inhibition interferes with cross-talk between malignant cells and their microenvironment, suggesting disruption of intrinsic and extrinsic survival signals may be a critical mechanism for the clinical activity of BTK inhibitors (Ponader, et al., Blood 2012,119, 1182-89, Herman, et al., Leukemia 2013, 27, 2311-21). Furthermore, epithelial derived tumors contain large numbers of TAMs, which are the dominant innate immune cell in mammary cancers of humans (Pollard, Nat. Rev. Immunol. 2009, 9, 259-270).

BTK is also a signaling hub in immature myeloid cells known as MDSCs (Schmidt, et al., Int. Arch. Allergy Immunol. 2004, 134, 65-78). Recent evidence suggests MDSC play an important part in suppression of host immune responses through several mechanisms such as production of arginase 1, release of reactive oxygen species, nitric oxide and secretion of immune-suppressive cytokines. This leads to an immunosuppressive environment necessary for the growth of malignant cells (Wesolowski, et al., J. Immunother. Cancer 2013, 1, 10).

Immune evasion is one of the multiple characteristics of cancer. Monoclonal antibodies that block negative regulators of T cells, such as PD-1, amplify immune responses. Antibodies against PD-1 are showing impressive results in advanced hematologic and solid malignancies (Hamid, et al., N. Engl. J. Med. 2013, 369, 134-44; Westin, et al., Lancet Oncol. 2014, 15, 69-77; Berger, et al., Clin. Cancer Res. 2008,14, 3044-51; Topalian, et al., J. Clin. Oncol. 2014, 32, 1020-30). Studies examining circulating MDSCs in anti-CTL4 and anti-PD-1/PD-L1-treated patients have shown that alterations in the myeloid cell compartment correlate with clinical outcome. Specifically, solid tumor progressors had proportionally higher circulating MDSC levels and a high myeloid gene signature (Powles, et αl., J. Clin. Oncol. 2014, 32, 5s (suppl; abstr 5011); Heery, et al., J. Clin. Oncol. 2014, 32, 5s (suppl; abstr 3064); Weide, et αl., Clin. Cancer Res. 2014, 20, 1601-09). Recent preclinical results show elevated MDSC levels are responsible for this lack of response (Highfill, et al., Sci. Transl. Med. 2014, 6, 237ra67; Kim, et a/., Proc. Nat'l. Acad. Sci. USA, 2014, 111, 11774-79).

The study design is as follows. The clinical trial is a Phase 2, multicenter, open-label, randomized study evaluating Formula (XVIII) monotherapy and the combination of Formula (XVIII) and pembrolizumab in subjects who have advanced or metastatic pancreatic cancer. Subjects meeting the eligibility criteria for the study will be randomized 1:1 to one of the following arms: Arm 1: Formula (XVIII) 100 mg administered orally (PO) twice per day (BID) Arm 2: Formula (XVIII) 100 mg PO BID plus pembrolizumab 200 mg administered as an intravenous (IV) infusion every 3 weeks (Q3W). Although Formula (XVIII) has not demonstrated any dose limiting toxicities (DLTs) to date, the safety of Formula (XVIII) in combination with pembrolizumab in this patient population will be assessed and standard DLT criteria will be applied to Arm 2 of the study. Therefore an interim safety analysis will occur once 12 subjects (6 subjects per arm) have been successfully randomized and have been treated a minimum of 6 weeks. Enrollment will be paused while the safety interim analysis occurs. If ≤ 1 DLT is observed in Arm 2, then randomization will continue to evaluate the objective response rates of Formula (XVIII) monotherapy and the combination of pembrolizumab and Formula (XVIII) (i.e., up to 38 subjects per arm). If ≥ 2 DLTs are observed in Arm 2, then enrollment will continue until an additional 6 subjects are randomized to Arm 2, but with a reduced dose level for Formula (XVIII) (Level -1). If the DLT review is cleared in those additional 6 subjects in Arm 2 then continued enrollment will occur at Level -1 for the combination arm. If ≥ 2 DLTs are observed in Arm 2 at Level -1, then an additional 6 subjects will be randomized at Level -2 and assessed for DLTs. If the DLT review is cleared, continued enrollment will occur at Level -2 for the combination arm. If the DLT review is not cleared, enrollment will be halted in Arm 2. In addition, analyses for futility and toxicity will also be done. Treatment can continue for up to 52 weeks for subjects who are tolerating therapy and not progressing. Subjects who have confirmed progressive disease on the combination of pembrolizumab and Formula (XVIII) will come off study while those in the Formula (XVIII) monotherapy arm with confirmed progressive disease will continue on Formula (XVIII) (dose may be reduced depending on DLT review of combination arm) with the addition of pembrolizumab until a second disease progression. Treatment can end for subjects with confirmed complete response (CR) if treatment has been administered for at least 24 weeks and, for subjects receiving pembrolizumab, 2 doses of pembrolizumab have been administered after confirmation of CR. At the end of 52 weeks of treatment, subjects who are tolerating the regimen and deriving clinical benefit may be eligible to roll over into a maintenance protocol under which they could receive Formula (XVIII) and/or pembrolizumab.

A DLT will be defined as the occurrence of any of the following study drug-related adverse events (AEs) (note: AEs clearly related to disease progression or the subject's current medical history and associated comorbidities will not be considered DLTs): (1) Grade 4 vomiting or diarrhea; (2) Grade 3 nausea, vomiting, or diarrhea lasting for > 72 hours; (3) Other Grade ≥ 3 toxicities; (4) Dosing delay due to toxicity for > 21 consecutive days.

The objectives of the study are as follows: (1) to characterize the safety profile of Formula (XVIII) and pembrolizumab in subjects with advanced or metastatic pancreatic cancer; (2) to evaluate the efficacy of Formula (XVIII) monotherapy and Formula (XVIII) and pembrolizumab combination treatment in subjects with advanced or metastatic pancreatic cancer using standard response criteria; (3) to determine the effects of Formula (XVIII) alone and Formula (XVIII) plus pembrolizumab on peripheral blood T cells and NMSCs; (4) to determine if any characteristics of peripheral blood T cells and/or MDSCs correlate with immune-mediated toxicities; (5) to determine if any characteristics of peripheral blood T cells and/or MDSCs correlate with response to Formula (XVIII) alone or Formula (XVIII) and pembrolizumab; (6) to determine if any baseline tumor characteristics correlate with response to Formula (XVIII) alone or Formula (XVIII) and pembrolizumab; (7) to evaluate the efficacy of adding pembrolizumab to Formula (XVIII) in subjects who progress on Formula (XVIII) monotherapy.

Safety endpoints and pharmacodynamic and biomarker parameters are as in Reference Example 18.

Efficacy endpoints are as follows: (1) disease control rate (DCR) defined as stable disease (SD), partial response (PR) or CR based on modified RECIST 1.1 criteria; (2) ORR, defined as PR or CR based on modified RECIST 1.1 criteria; (3) Duration of response (DOR); (4) Progression-free survival (PFS); (5) Overall survival (OS); (6) Change in serum cancer antigen 19-9 (CA19-9). Exploratory endpoints for efficacy based on immune-related response criteria (irRC) are: (1) Immune-related DCR (irDCR), defined as immune-related SD (irSD), immune-related PR (irPR), and immune-related CR (irCR); (2) irORR, defined as irPR and irCR; (3) irDOR; (4) irPFS.

Sample size is as follows: Interim safety analysis (DLT review): 12 subjects (6 subjects receiving Formula (XVIII) monotherapy and 6 subjects receiving Formula (XVIII) and pembrolizumab combination). Provided the DLT period is cleared in the combination arm and neither arm is stopped early due to futility or toxicity, the study will proceed to full enrollment of 38 subjects per arm for a total enrollment of 76 subjects.

Formula (XVIII) is provided as hard gelatin capsules for oral administration. KEYTRUDA (pembrolizumab) is provided as a lyophilized powder in single-use vial for reconstitution. It is administered as an IV infusion over 30 minutes. The dosing regimen for Arm 1 is 100 mg BID of Formula (XVIII). The dosing regimen for Arm 2 is s starting dose of 100 mg BID Formula (XVIII) and 200 mg of pembrolizumab every three weeks, a Level -1 dose of 100 mg QD Formula (XVIII) and 200 mg of pembrolizumab every three weeks, and a Level -2 dose of 100 mg BID Formula (XVIII) and 200 mg of pembrolizumab every three weeks.

The statistical methods used in the study use the following analysis methods. Descriptive statistics (including means, standard deviations, and medians for continuous variables and proportions and confidence intervals [CIs] for discrete variables) will be used to summarize data as appropriate. The statistical basis for the sample size is as follows. For the safety interim analysis (DLT review), enrollment of 6 subjects in the combination arm for DLT review is consistent with sample sizes used in oncology studies for determination of maximum tolerated dose (MTD). The trial employs the standard National Cancer Institute definition of MTD (dose associated with DLT in ≤ 17% of subjects). Provided ≤ 1 DLT occurs during the DLT review in the combination arm, then up to 32 subjects will be added per arm. The sample size for this study was estimated based on the primary endpoint of DCR (SD, PR, CR). In a Phase 2 trial of oxaliplatin plus capecitabine as second line therapy for patients with advanced pancreatic cancer, a DCR of 28% was observed (Xiong, et al., Cancer 2008, 113, 2046-2052). To reject the null hypothesis of 28% DCR in favor of an alternative hypothesis that the DCR is ≤ 5%, approximately 36 subjects per arm will preserve at least 80% power to detect the difference at a 0.05 level of significance by 2-sided chi-square test. The study will enroll up to 38 subjects per arm to account for up to 2 drop outs per arm. Should the necessary condition of minimum cell counts for a valid chi-square test fail to exist, an exact test will be employed to perform the comparison analysis of the primary endpoint.

### Example 21 - Clinical Study of a Combination of a BTK Inhibitor and Pembrolizumab in Subjects with Platinum-Refractory Metastatic Bladder Cancer

In 2014, approximately 141,610 people in the United States will be diagnosed with urothelial carcinoma of the bladder, renal pelvis, or ureter (Siegel, et al., CA Cancer J. Clin. 2014, 64, 9-29). Although many newly diagnosed patients have localized disease, urothelial carcinoma is often fatal for those diagnosed with metastatic disease. Approximately 30,350 people are anticipated to die from this disease in 2014. In most patients with localized disease, treatment includes localized excision with transurethral resection of bladder tumor (TURBT) and intravesicular Bacillus Calmette Guerin (BCG) infusions (Martyn-Hemphill, et al., Int. J. Surg. 2013, 11, 749-52). In the ~30% of patients who develop metastatic disease, chemotherapy with a platinum-based regimen is the primary treatment (Gartrell and Sonpavde, Expert Opin. Emerg. Drugs, 2013, 18, 477-94). Current standard of care first-line therapy includes combination chemotherapy with cisplatin or carboplatin with gemcitabine, or the combination of methotrexate, vinblastine, adriamycin, and cisplatin (MVAC). However, because of the inherent chemoresistance of bladder cancer, median progression-free survival (PFS) with these chemotherapy regimens is approximately 7.4 months (Von der Maase, et al., J. Clin. Oncol. 2000, 17, 3068-3077). The addition of paclitaxel to gemcitabine/cisplatin has improved PFS to 8.3 months (Bellmunt, et al., J. Clin. Oncol. 2012, 30, 1107-1113). Currently, second-line therapies are limited, and no therapies have been approved by the United States Food and Drug Administration (FDA) for patients who survive to undergo second-line treatment. Survival at 2 years is therefore < 20% (Bellmunt, et αl., J. Clin. Oncol. 2012, 30, 1107-1113). Thus, while antitumor benefit has been observed with such regimens, toxicity is substantial and therapeutic options are limited. Novel, less toxic approaches are needed for metastatic, platinum-refractory urothelial carcinoma.

The importance of the stroma in urothelial carcinoma has been increasingly recognized (Van der Horst, et al., Mol. Cancer Res. 2012, 10, 995-1009), particularly in its role in tumor progression and formation of metastases. Several stromal-changing growth factors, such as FGF2, VEGF, PDGF, EGFR ligands, and TGF-13 are important in mediating tumor progression, supporting tumor associated fibroblasts in urinary bladder tumor specimens (Enkelmann, et a/., J. Cancer Res. Clin. Oncol. 2011, 137, 751-759). Tumor-associated fibroblasts have also shown increased populations in invasive bladder tumors and not in superficial bladder tumors, thus associating with muscle invasion and formation of metastases (Alexa, et al., Rom. J. Morphol. Embryol. 2009, 50, 639-643). Furthermore, tumor-associated macrophages have been shown to mediate (Onita, et a/., Clin. Cancer Res. 2002, 8, 471-480). The TGF-13 mediator is also known to shift macrophages from an M1 antitumor phenotype to an M2 protumor phenotype, leading to remodeling of the microenvironment, angiogenesis, and epithelial plasticity (Fuxe, et αl., Semin. Cancer Biol. 2012, 22, 455-461). In summary, urothelial carcinoma exists in a complex desmoplastic microenvironment providing stromal support for tumor growth, resembling wound healing, thus increasing motility, invasion, and angiogenesis.

Such checkpoint pathways appear to be operative in urothelial carcinoma. Immunohistochemistry analyses have shown PD-L1 positivity is associated with increased staging, high-grade tumors, and tissue-infiltrating mononuclear cells in urothelial carcinoma (Inman, et a/., Cancer 2007, 109, 1499-1505). In a series of 318 patients with urothelial carcinoma, PD-L1 and PD-1 expression were associated with advanced disease, and PD-L1 expression independently predicted for mortality (Boorjian, et αl., Clin. Cancer Res. 2008, 14, 4800-08). PD-L1 expression may protect cancer cells from immune-mediated destruction. In a clinical study of subjects with urothelial carcinoma evaluating the efficacy of an anti-PD-Ll antibody, high expression of PD-L1 in tumor-infiltrating immune cells correlated with a higher overall response rate (ORR, 40% to 50%) compared with an ORR of 13% and 8% for low PD-L1 or no PD-L1 expression (Powles, et αl., J. Clin. Oncol. 2014, 32, 5s(suppl; abstr 5011)). In a clinical study of the anti-PD-1 monoclonal antibody, pembrolizumab, in subjects with recurrent or metastatic urothelial carcinoma, a 24% ORR, including 10% complete response (CR) rate, was observed across the 33 subjects treated. Analysis of the relationship between PD-L1 expression and pembrolizumab efficacy was pending (Plimack, et a/., "A phase IB study of pembrolizumab in patients with advanced urothelial tract cancer," 2014 ESMO Annual Meeting).

The preclinical rationale for use of a combination of a BTK inhibitor and a PD-1 or PD-L1 inhibitor in solid tumor cancers is discussed in Example 19 and in the other examples provided herein.

This proof-of-concept study will assess the clinical potential of a targeted dual inhibition approach by evaluating the safety, pharmacodynamics (PD), and efficacy of Formula (XVIII) and pembrolizumab in subjects with metastatic urothelial carcinoma who have progressed after treatment with cisplatin-based chemotherapy. This clinical trial is a Phase 2, multicenter, open-label, randomized study evaluating pembrolizumab monotherapy and the combination of Formula (XVIII) and pembrolizumab in subjects who have metastatic bladder cancer with disease progression on or after platinum-based chemotherapy. Subjects meeting the eligibility criteria for the study will be randomized 1:1 to one of the following arms: Arm 1: Pembrolizumab 200 mg administered as an intravenous (IV) infusion every 3 weeks (Q3W); Arm 2: Formula (XVIII) 100 mg administered orally (PO) twice per day (BID) plus pembrolizumab 200 mg IV Q3W.

Although Formula (XVIII) has not demonstrated any dose-limiting toxicities (DLTs) to date, the safety of Formula (XVIII) in combination with pembrolizumab in this patient population needs to be assessed. Thus, standard DLT criteria will be applied to Arm 2 of the study. Therefore an interim safety analysis will occur once 12 subjects (6 subjects per arm) have been successfully randomized and have been treated a minimum of 6 weeks. Enrollment will be paused while the safety interim analysis occurs. I f ~ 1 DLT is observed in Arm 2 (*i.e*., DLT review is cleared), then randomization will continue to evaluate the objective response rates of Formula (XVIII) monotherapy and the combination of pembrolizumab and Formula (XVIII) (i.e., up to 37 subjects per arm). If ~ 2 DLTs are observed in Arm 2, then enrollment (1:1) will continue until an additional 6 subjects are randomized to Arm 2, but with a reduced dose level for Formula (XVIII) (Level -1). If the DLT review is cleared in those additional 6 subjects in Arm 2 then continued enrollment will occur at Level -1 for the combination arm. If ~ 2 DLTs are observed in Arm 2 at Level -1, then an additional 6 subjects will be randomized at Level - 2 and assessed for DLTs. If the DLT review is cleared, continued enrollment will occur at Level -2 for the combination arm. If the DLT review is not cleared, enrollment will be halted in Arm 2.

Treatment can continue for up to 52 weeks for subjects who are tolerating therapy and not progressing. Subjects who have confirmed progressive disease on the combination of pembrolizumab and Formula (XVIII) will come off study while those with confirmed progressive disease in the pembrolizumab monotherapy arm will continue on pembrolizumab with the addition of Formula (XVIII) until a second disease progression. Treatment can end for subjects with confirmed complete response (CR) if treatment has been administered for at least 24 weeks and 2 doses of pembrolizumab have been administered after confirmation of CR. At the end of 52 weeks of treatment, subjects who are tolerating the regimen and deriving clinical benefit may be eligible to roll over into a maintenance protocol under which they could receive Formula (XVIII) and/or pembrolizumab.

For assessment of the first 12 subjects randomized, DLT will be defined as the occurrence of any of the following study drug-related adverse events (note: adverse events clearly related to disease progression or the subject's current medical history and associated comorbidities will not be considered DLTs): (1) Any Grade ~ 3 toxicity (except Grade 3 nausea, vomiting, or diarrhea that respond to supportive therapy); (2) Dosing delay due to toxicity for > 21 consecutive days.

The study objectives are as follows: (1) to characterize the safety profile of Formula (XVIII) and pembrolizumab in subjects with metastatic, platinum-refractory bladder cancer; (2) to determine the overall response rate (ORR) of pembrolizumab monotherapy and the combination of Formula (XVIII) and pembrolizumab in subjects with metastatic, platinum-refractory bladder cancer; (3) to determine progression-free survival (PFS) in subjects treated with pembrolizumab monotherapy and the combination of Formula (XVIII) and pembrolizumab; (4) to evaluate the overall survival (OS) in subjects treated with pembrolizumab monotherapy and the combination of Formula (XVIII) and pembrolizumab; (5) to determine the effects of Formula (XVIII) plus pembrolizumab on peripheral blood T cells and myeloid-derived suppressor cells (MDSCs); (6) determine if any characteristics of peripheral blood T cells and/or MDSCs correlate with immune-mediated toxicities; (7) determine if any characteristics of peripheral blood T cells and/or MDSCs correlate with response to Formula (XVIII) and pembrolizumab; (8) determine if any baseline tumor characteristics correlate with response to Formula (XVIII) and pembrolizumab; and (9) to evaluate the efficacy of adding Formula (XVIII) to pembrolizumab in subjects who progress on pembrolizumab monotherapy.

Safety endpoints and pharmacodynamic and biomarker parameters are as in Reference Example 18. Efficacy endpoints are as in Example 19.

The sample size is as follows: Interim safety analysis (DLT review): 12 subjects (6 subjects receiving pembrolizumab monotherapy and 6 subjects receiving Formula (XVIII) and pembrolizumab combination). Provided the DLT period is cleared in the combination arm, the study will proceed to full enrollment of 37 subjects per arm for a total enrollment of 74 subjects.

The dosing regimen and routes of administration for Formula (XVIII) and pembrolizumab are as in Example 19.

The statistical methods are as follows. Descriptive statistics (including means, standard deviations, and medians for continuous variables and proportions and confidence intervals [CIs] for discrete variables) will be used to summarize data as appropriate. The statistical basis for the sample size is as follows. For the safety interim analysis (DLT review), enrollment of 6 subjects in the combination arm for DLT review is consistent with sample sizes used in oncology studies for determination of maximum tolerated dose (MTD). The trial employs the standard National Cancer Institute definition of MTD (dose associated with DLT in ~ 17% of subjects). Provided ~ 1 DLT occurs during the DLT review in the combination arm, then up to 31 subjects will be added per arm. A sample size for a 2-arm pick-the-winner, non-comparative trial was determined by a Z-test for normal approximation of binomial distribution, based on one-sided a = 0.05, 80% power, with projected response rates of 40% in pembrolizumab/Formula (XVIII) arm and 18% in pembrolizumab arm. Accounting for 10% drop-out rate (3 in each arm), final sample size is 37 in each arm.

### Example 22 - Clinical Study of a Combination of a BTK Inhibitor, a PI3K-δ Inhibitor, and Pembrolizumab in Subjects with Advanced Head and Neck Squamous Cell Carcinoma

Head and neck squamous cell carcinoma (HNSCC) is the sixth most common cancer worldwide; approximately 600,000 new cases are diagnosed per year worldwide (International Agency for Research on Cancer. World Health Organisation. Globocan 2012: Estimated cancer incidence, mortality and prevalence worldwide in 2012). While early stage disease may be treated with a single modality such as surgery or RT, most patients (60%) present with Stage III/IV poor prognosis disease (Seiwert, et al., Nat. Clin. Pract. Oncol. 2007, 4, 156-171). These patients are generally treated with a combination of RT, surgery, and cytotoxic or targeted chemotherapy. Despite this aggressive multimodal treatment, most patients will relapse. The survival rates for all patients with HNSCC are approximately 40% to 60% at 5 years (Grégoire, et al., Ann. Oncol. 2010, 21, 184-186). While the addition of cetuximab, an immunoglobulin G1 (IgG1) monoclonal antibody targeting the epidermal growth factor receptor (EGFR), improves OS when combined with RT or chemotherapy (median OS is 10.1 months for the combination of cisplatin or carboplatin with 5-fluorouracil and cetuximab), few patients will benefit from anti-EGFR monoclonal antibodies, and the objective response rate in monotherapy is between 6% and 13% (Bonner, et al., Lancet Oncol. 2010, 11, 21-28; Machiels, et al., Lancet Oncol. 2011,12 ,333-343; Vermorken, et al., N. Engl. J. Med. 2008, 359, 1116-1127; Vermorken, et al, Cancer 2008,112, 2710-2709). Based on recent developments in HNSCC molecular biology, new compounds are currently being investigated including immune checkpoint inhibitors (Schmitz, et al., Cancer Treat. Rev. 2014, 40, 390-404).

Over half of solid tumors (including HNSCC, lung cancer, melanoma, renal cell carcinoma, pancreatic cancer, ovarian cancer, and others) express PD-L1 in the tumor microenvironment which results in immune tolerance and impaired immune response against the tumor (Zou and Chen, Nat. Rev. Immunol. 2008, 8, 467-77). Additionally, PD-L1 expression in the tumor microenvironment has been shown to be a poor prognostic factor in several cancers (Pardoll, Nat. Rev. Cancer 2012, 12, 252-264). In a clinical study of the anti-PD-1 monoclonal antibody, pembrolizumab, in subjects with advanced unresectable HNSCC (N=60), a 20% ORR was observed with response lasting up to 41 weeks (Chow, et a/., ESMO 2014 Abstract LBA31). Pembrolizumab was well tolerated with few serious drug-related AEs (Seiwert, et al., Nat. Clin. Pract. Oncol. 2007, 4, 156-171).

The clinical study is a Phase 2, multicenter, open-label, randomized study evaluating pembrolizumab monotherapy and the combination of Formula (XVIII) and pembrolizumab in subjects who have recurrent, metastatic or unresectable HNSCC. Subjects meeting the eligibility criteria for the study will be randomized 1:1 to one of the following arms: Arm 1: Pembrolizumab 200 mg administered as an intravenous (IV) infusion every 3 weeks (Q3W); Arm 2: Formula (XVIII) 100 mg administered orally (PO) twice per day (BID) plus pembrolizumab 200 mg IV Q3W. Although Formula (XVIII) has not demonstrated any dose-limiting toxicity (DLT) to date, the safety of Formula (XVIII) in combination with pembrolizumab in this patient population needs to be assessed. Thus, standard DLT criteria will be applied to Arm 2 of the study. An interim safety analysis will occur once 12 subjects (6 subjects per arm) have been successfully randomized and have been treated a minimum of 6 weeks. Enrollment will be paused while the safety interim analysis occurs. If ≤ 1 DLT is observed in Arm 2 (i.e., DLT review is cleared), then randomization will continue to evaluate the objective response rates of pembrolizumab monotherapy and the combination of pembrolizumab and Formula (XVIII) (i.e., up to 37 subjects per arm). If ≥ 2 DLTs are observed in Arm 2, then enrollment (1:1) will continue until an additional 6 subjects are randomized to Arm 2, but with a reduced dose level for Formula (XVIII) (Level -1). If the DLT review is cleared in those additional 6 subjects in Arm 2 then continued enrollment will occur at Level -1 for the combination arm. If ≥ 2 DLTs are observed in Arm 2 at Level -1, then an additional 6 subjects will be randomized at Level -2 and assessed for DLTs. If the DLT review is cleared, continued enrollment will occur at Level -2 for the combination arm. If the DLT review is not cleared, enrollment will be halted in Arm 2. In addition, analyses for futility and toxicity will also be done as outlined in 5.5. Treatment can continue for up to 52 weeks for subjects who are tolerating therapy and have not developed disease progression. Subjects who have confirmed progressive disease on the combination of pembrolizumab and Formula (XVIII) will come off study while those with confirmed progressive disease in the pembrolizumab monotherapy arm will continue on pembrolizumab with the addition of Formula (XVIII) until a second disease progression is observed. Treatment will end for subjects with confirmed complete response (CR) once treatment has been administered for at least 24 weeks and 2 doses of pembrolizumab have been administered after confirmation of CR.

DLT will be defined as the occurrence of any of the following study drug-related adverse events (AEs): (1) any Grade ≥ 3 toxicity (note: AEs clearly related to disease progression or the subject's current medical history and associated comorbidities will not be considered DLTs); (2) dosing delay due to toxicity for > 28 consecutive days.

The objectives of the study are as follows: (1) to characterize the safety and tolerability of Formula (XVIII) in combination with pembrolizumab in subjects with recurrent, metastatic or unresectable HNSCC; (2) to determine the overall response rate (ORR) of pembrolizumab monotherapy and the combination of Formula (XVIII) and pembrolizumab in subjects with recurrent, metastatic or unresectable HNSCC; (3) to determine progression-free survival (PFS) in subjects treated with pembrolizumab monotherapy and the combination of Formula (XVIII) and pembrolizumab; (4) to evaluate the overall survival (OS) in subjects treated with pembrolizumab monotherapy and the combination of Formula (XVIII) and pembrolizumab; (5) to determine the effects of Formula (XVIII) plus pembrolizumab on peripheral blood T cells and myeloid-derived suppressor cells (MDSCs); (6) to determine if any characteristics of peripheral blood T cells and/or MDSCs correlate with immune-mediated toxicities; (7) to determine if any characteristics of peripheral blood T cells and/or MDSCs correlate with response to Formula (XVIII) and pembrolizumab; (8) to determine if any baseline tumor characteristics correlate with response to Formula (XVIII) and pembrolizumab; and (9) to evaluate the efficacy of adding Formula (XVIII) to pembrolizumab in subjects who progress on pembrolizumab monotherapy.

Safety endpoints and pharmacodynamic and biomarker parameters are as in Reference Example 18.

Efficacy endpoints are as follows: (1) ORR, defined as partial response (PR) and CR, based on modified RECIST 1.1 criteria; (2) duration of response (DOR); (3) PFS; (4) OS. Exploratory endpoints for efficacy based on immune-related response criteria (irRC) are: (1) Immune-related ORR (irORR), defined as immune-related partial response (irPR) and immune-related complete response (irCR); (2) Immune-related duration of response (irDOR); (3) Immune-related progression-free survival (irPFS).

Interim safety analysis (DLT review): 12 subjects (6 subjects receiving pembrolizumab monotherapy and 6 subjects receiving Formula (XVIII) and pembrolizumab combination). Provided the DLT period is cleared in the combination arm and neither arm is stopped early due to futility or toxicity, the study will proceed to full enrollment of 37 subjects per arm for a total enrollment of 74 subjects.

The dosing regimen and routes of administration for Formula (XVIII) and pembrolizumab are as in Example 19.

The statistical methods are as follows. Descriptive statistics (including means, standard deviation [SD], and medians for continuous variables and proportions and confidence intervals [CIs] for discrete variables) will be used to summarize data as appropriate. For the safety interim analysis (DLT review), enrollment of 6 subjects in the combination arm for DLT review is consistent with sample sizes used in oncology studies for determination of maximum tolerated dose (MTD). The trial employs the standard National Cancer Institute definition of MTD (dose associated with DLT in ≤ 17% of subjects). Provided ≤ 1 DLT occurs during the DLT review in the combination arm and the study is not stopped early due to futility or toxicity, then up to 31 subjects will be added per arm. A sample size for a 2-arm pick-the-winner, non-comparative trial was determined by a Z-test for normal approximation of binomial distribution, based on one-sided α = 0.05, 80% power, with projected response rates of 40% in the pembrolizumab and Formula (XVIII) arm and 18% in the pembrolizumab arm. Accounting for 10% drop-out rate (3 in each arm), the final sample size is 37 in each arm.

### Example 30 - Synergistic Combination of a BTK Inhibitor and an α-PD-1 Inhibitor in the ID8 Ovarian Cancer Model

The ID8 ovarian cancer model of Examples 6 and 7 was also used to investigate potential synergistic effects between the BTK inhibitor of Formula (XVIII) and an α-PD-1 inhibitor. Doses of 15 mg/kg BID of Formula (XVIII) and 150 µg of α-PD-1 antibody (anti-mouse PD-1 antibody Clone J43, BE0033-2) or anti-mouse PD-1 antibody Clone RMP1-14 (BioXcell, BE0146)). The experiments were performed as described in Reference Example 7.

## Claims

1. A pharmaceutical combination comprising (1) a programmed death 1 (PD-1) inhibitor or a programmed death ligand 1 (PD-L1) inhibitor and (2) a Bruton's tyrosine kinase (BTK) inhibitor or a pharmaceutically acceptable salt thereof for use in treatment of cancer in a human subject, wherein the BTK inhibitor is selected from the group consisting of: 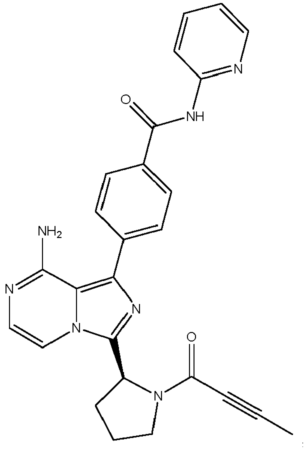 the PD-1 inhibitor is pembrolizumab, and the PD-L1 inhibitor is durvalumab.

2. The pharmaceutical combination for use according to claim 1, wherein the combination comprises the PD-1 inhibitor pembrolizumab.

3. The pharmaceutical combination for use according to claim 1, wherein the combination comprises the PD-L1 inhibitor durvalumab.

4. The pharmaceutical combination for use according to any preceding claim, wherein the combination further comprises an anti-CD20 antibody selected from the group consisting of rituximab, obinutuzumab, ofatumumab, veltuzumab, tositumomab, ¹³¹I-tositumomab, ibritumomab, ⁹⁰Y-ibritumomab, ¹¹¹In-ibritumomab and ibritumomab tiuxetan.

5. The pharmaceutical combination for use according to any preceding claim, wherein the cancer is a B-cell hematological malignancy selected from the group consisting of chronic lymphocytic leukemia (CLL), small lymphocytic leukemia (SLL), non-Hodgkin's lymphoma (NHL), diffuse large B cell lymphoma (DLBCL), follicular lymphoma (FL), mantle cell lymphoma (MCL), Hodgkin's lymphoma, B cell acute lymphoblastic leukemia (B-ALL), Burkitt's lymphoma, Waldenström's macroglobulinemia (WM), multiple myeloma (MM), myelodysplastic syndrome, and myelofibrosis.

6. The pharmaceutical combination for use according to any preceding claim, wherein the cancer is chronic lymphocytic leukemia (CLL).

7. The pharmaceutical combination for use according to any preceding claim, wherein the cancer is diffuse large B cell lymphoma (DLBCL).

8. The pharmaceutical combination for use according to any preceding claim, wherein the cancer is mantle cell lymphoma (MCL).

9. The pharmaceutical combination for use according to any one of claims 1 to 8, wherein the use comprises administering the PD-1 or PD-L1 inhibitor and the BTK inhibitor simultaneously in separate compositions.

10. The pharmaceutical combination for use according to any one of claims 1 to 8, wherein the use comprises administering the PD-1 or PD-L1 inhibitor and the BTK inhibitor at different times in separate compositions.

11. The pharmaceutical combination for use according to any one of claims 1 to 8, wherein the use comprises administering the PD-1 or PD-L1 inhibitor and the BTK inhibitor in a composition in which two or more active pharmaceutical ingredients are present.

## Patentansprüche

1. Pharmazeutische Kombination, umfassend (1) einen Programmed Death 1 (PD-1)-Inhibitor oder einen Programmed Death Ligand 1 (PD-L1)-Inhibitor und (2) einen Bruton-Tyrosinkinase (BTK)-Inhibitor oder ein pharmazeutisch unbedenkliches Salz davon zur Verwendung bei der Behandlung von Krebs in einem menschlichen Subjekt, wobei der BTK-Inhibitor aus der aus 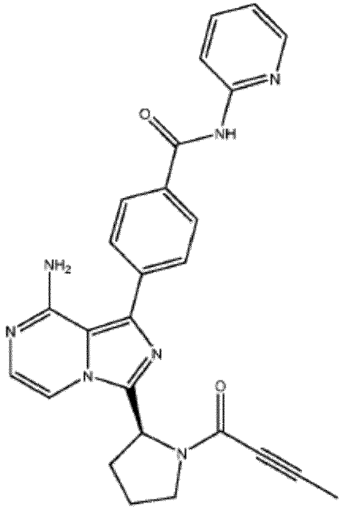 bestehenden Gruppe ausgewählt ist, es sich bei dem PD-1-Inhibitor um Pembrolizumab handelt und es sich bei dem PD-L1-Inhibitor um Durvalumab handelt.

2. Pharmazeutische Kombination zur Verwendung nach Anspruch 1, wobei die Kombination den PD-1-Inhibitor Pembrolizumab umfasst.

3. Pharmazeutische Kombination zur Verwendung nach Anspruch 1, wobei die Kombination den PD-L1-Inhibitor Durvalumab umfasst.

4. Pharmazeutische Kombination zur Verwendung nach einem der vorhergehenden Ansprüche, wobei die Kombination weiterhin einen aus der aus Rituximab, Obinutuzumab, Ofatumumab, Veltuzumab, Tositumomab, ¹³¹I-Tositumomab, Ibritumomab, ⁹⁰Y-Ibritumomab, ¹¹¹In-Ibritumomab und Ibritumomab Tiuxetan bestehenden Gruppe ausgewählten Anti-CD20-Antikörper umfasst.

5. Pharmazeutische Kombination zur Verwendung nach einem der vorhergehenden Ansprüche, wobei es sich bei dem Krebs um eine hämatologische B-Zell-Malignität ausgewählt aus der Gruppe bestehend aus chronischer lymphozytischer Leukämie (Chronic, Lymphocytic Leukemia, CLL), kleinzelliger lymphozytischer Leukämie (Small Lymphocytic Leukemia, SLL), Non-Hodgkin-Lymphom (NHL), diffusem großzelligem B-Zell-Lymphom (Diffuse Large B Cell Lymphoma, DLBCL), follikulärem Lymphom (FL), Mantelzelllymphom (Mantle Cell Lymphoma, MCL), Hodgkin-Lymphom, akuter lymphoblastischer B-Zell-Leukämie (B Cell Acute Lymphoblastic Leukemia, B-ALL), Burkitt-Lymphom, Waldenström-Macroglobulinämie (WM), multiplem Myelom (MM), myelodysplastischem Syndrom und Myelofibrose handelt.

6. Pharmazeutische Kombination zur Verwendung nach einem der vorhergehenden Ansprüche, wobei es sich bei dem Krebs um chronische lymphozytische Leukämie (Chronic Lymphocytic Leukemia, CLL) handelt.

7. Pharmazeutische Kombination zur Verwendung nach einem der vorhergehenden Ansprüche, wobei es sich bei dem Krebs um diffuses großzelliges B-Zell-Lymphom (Diffuse Large B Cell Lymphoma, DLBCL) handelt.

8. Pharmazeutische Kombination zur Verwendung nach einem der vorhergehenden Ansprüche, wobei es sich bei dem Krebs um Mantelzelllymphom (Mantle Cell Lymphoma, MCL) handelt.

9. Pharmazeutische Kombination zur Verwendung nach einem der Ansprüche 1 bis 8, wobei die Verwendung die Verabreichung des PD-1- oder PD-L1-Inhibitors und des BTK-Inhibitors gleichzeitig in getrennten Zusammensetzungen umfasst.

10. Pharmazeutische Kombination zur Verwendung nach einem der Ansprüche 1 bis 8, wobei die Verwendung die Verabreichung des PD-1- oder PD-L1-Inhibitors und des BTK-Inhibitors zu verschiedenen Zeitpunkten in getrennten Zusammensetzungen umfasst.

11. Pharmazeutische Kombination zur Verwendung nach einem der Ansprüche 1 bis 8, wobei die Verwendung die Verabreichung des PD-1- oder PD-L1-Inhibitors und des BTK-Inhibitors in einer Zusammensetzung, in der zwei oder mehr pharmazeutische Wirkstoffe vorliegen, umfasst.

## Revendications

1. Combinaison pharmaceutique, comprenant (1) un inhibiteur de mort programmée 1 (PD-1) ou un inhibiteur du ligand de mort programmée 1 (PD-L1) et (2) un inhibiteur d'une tyrosine kinase de Bruton (BTK) ou un sel de celui-ci acceptable d'un point de vue pharmaceutique, destinée à être utilisée dans le traitement d'un cancer chez un sujet humain, dans laquelle l'inhibiteur de BTK est choisi dans le groupe constitué par : 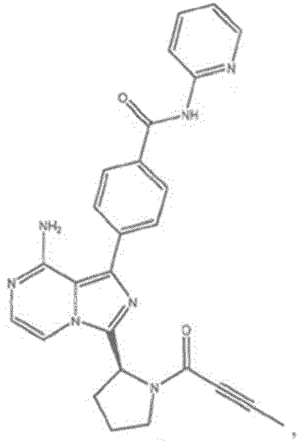 l'inhibiteur de PD-1 est le pembrolizumab et l'inhibiteur de PD-L1 est le durvalumab.

2. Combinaison pharmaceutique destinée à être utilisée selon la revendication 1, la combinaison comprenant l'inhibiteur de PD-1 pembrolizumab.

3. Combinaison pharmaceutique destinée à être utilisée selon la revendication 1, la combinaison comprenant l'inhibiteur de PD-L1 durvalumab.

4. Combinaison pharmaceutique destinée à être utilisée selon une quelconque revendication précédente, la combinaison comprenant en outre un anticorps anti-CD20 choisi dans le groupe constitué par les rituximab, obinutuzumab, ofatumumab, veltuzumab, tositumomab, ¹³¹I-tositumomab, ibritumomab, ⁹⁰Y-ibritumomab, ¹¹¹In-ibritumomab et ibritumomab tiuxétan.

5. Combinaison pharmaceutique destinée à être utilisée selon une quelconque revendication précédente, dans laquelle le cancer est une affection hématologique maligne à cellules B choisie dans le groupe constitué par la leucémie lymphoïde chronique (LLC), le lymphome lymphocytaire à petits lymphocytes (LPL), un lymphome non Hodgkinien (LNH), le lymphome diffus à grandes cellules B (LDGCB), un lymphome folliculaire (LF), le lymphome à cellules du manteau (LCM), un lymphome Hodgkinien, la leucémie lymphoblastique aiguë à cellules B (LLA-B), le lymphome de Burkitt, la macroglobulinémie de Waldenström (MW), un myélome multiple (MM), un syndrome myélodysplasique et une myélofibrose.

6. Combinaison pharmaceutique destinée à être utilisée selon une quelconque revendication précédente, dans laquelle le cancer est la leucémie lymphoïde chronique (LLC).

7. Combinaison pharmaceutique destinée à être utilisée selon une quelconque revendication précédente, dans laquelle le cancer est le lymphome diffus à grandes cellules B (LDGCB).

8. Combinaison pharmaceutique destinée à être utilisée selon une quelconque revendication précédente, dans laquelle le cancer est le lymphome à cellules du manteau (LCM).

9. Combinaison pharmaceutique destinée à être utilisée selon l'une quelconque des revendications 1 à 8, dans laquelle l'utilisation comprend une administration de l'inhibiteur de PD-1 ou de PD-L1 et l'inhibiteur de BTK simultanément dans des compositions séparées.

10. Combinaison pharmaceutique destinée à être utilisée selon l'une quelconque des revendications 1 à 8, dans laquelle l'utilisation comprend une administration de l'inhibiteur de PD-1 ou de PD-L1 et l'inhibiteur de BTK à différents moments dans des compositions séparées.

11. Combinaison pharmaceutique destinée à être utilisée selon l'une quelconque des revendications 1 à 8, dans laquelle l'utilisation comprend une administration de l'inhibiteur de PD-1 ou de PD-L1 et l'inhibiteur de BTK dans une composition dans laquelle deux ingrédients pharmaceutiques actifs ou plus sont présents.
